# EUROPEAN PATENT APPLICATION

(11) **EP 4 818 174 A1**
(43) Date of publication of application: **07.10.2026**
(21) Application number: 25797346.1
(22) Date of filing: 21.04.2025
(51) Int. Cl.: C07D 493/22, C07K 16/28, C07K 16/30, A61K 47/68, A61K 47/64, A61P 35/00

(54) **ERIBULIN DERIVATIVE AND ANTIBODY-DRUG CONJUGATE THEREOF, AND MEDICAL USE THEREOF**

(30) Priority: 28.04.2024 CN 202410530458
(71) Applicant: PHRONTLINE BIOPHARMA CO., LTD., Shanghai 200032 (CN); Phrontline Biopharma (Hangzhou) Co., Ltd, Hangzhou, Zhejiang 311103 (CN)
(72) Inventor: MAO, Yanli, Shanghai 201210 (CN); CHEN, Zhaoyuan, Shanghai 201210 (CN)
(74) Representative: Lorenz Seidler Gossel Part. mbB
(86) International application number: PCT/CN2025/090154
(87) International publication number: WO 2025/228172

(57) **Abstract**

The present application relates to the technical field of medicines, and in particular to an eribulin derivative and an antibody-drug conjugate (ADC) thereof, and a medical use thereof. In the present application, a series of derivatives with good tumor suppression effects are synthesized by modifying eribulin, and have lower toxicity and a larger therapeutic window than that of eribulin; and then the eribulin derivative and an antibody are made into an ADC by means of an appropriate linker, and the therapeutic efficiency of the eribulin derivative is improved using the targeting specificity of the antibody.

## Description

### TECHNICAL FIELD

The present application relates to the field of medical technology, and specifically, to an eribulin derivative and an antibody-drug conjugate thereof, and a medical use thereof.

### BACKGROUND

Antibody-Drug Conjugates (ADCs) are therapeutic agents formed by conjugating, with small-molecule toxins (payloads) via linkers, antibodies specifically targeting relevant antigens, and have both a potent cytotoxic effect of small-molecule chemotherapeutic agents and a tumor-targeting property of antibody drugs. Once inside the body, ADC molecules can bind to antigens on surfaces of target cells via a targeting function of the antibodies, and release toxin molecules through chemical or enzymatic action to eliminate target cells. By virtue of their targeting property and intracellular drug release in tumor cells, the ADCs can bind precisely to tumors and reduce their impact on normal cells, thereby increasing local drug concentration, decreasing toxic and side effects on normal cells, and expanding a therapeutic window. The ADCs have broad application prospects in tumor therapy and have achieved some encouraging results in clinical research.

Early failures of the ADCs were mainly attributed to their toxicity. Some side effects of certain ADCs are similar to those caused by conventional cytotoxins used in chemotherapy, and sometimes, these side effects may be severe. First, these side effects are caused by the uncontrolled release of cytotoxic agents due to linker instability during systemic circulation. Second, these side effects are caused because immunoglobulin G1 (IgG1) isotypes of some ADCs may interact with Fc-γ receptors (FcγR), which may trigger target-independent, FcγR-mediated internalization in FcγR-positive cells. With such non-specific endocytosis (off-target toxicities), the antibodies exert toxic effects on these healthy cells, which are not intended targets of the ADCs. Other side effects are related to specificity (on-target toxicity) of the ADCs. According to statistical analysis, dose-limiting toxicity of ADCs is almost consistent with that of chemotherapeutic agents used as toxins (Cancer Cell 40, 14, 2022, 1257). The development of cytotoxicities for the ADCs becomes a complex task due to these factors.

Eribulin belongs to a class of inhibitors featuring tubulin polymerization and has a unique and novel use. Eribulin is a synthetic analog of halichondrin B. In 1986, Japanese scientists Hirata and Uemura isolated a polyether macrolide compound containing only C, H, and O atoms from a sponge (Halichondria okadai). They named this natural product with an extremely complex structure halichondrin B. Eribulin inhibits microtubule elongation and mitotic spindle formation to exert its antitumor activity, thereby inducing apoptosis. In addition, studies have revealed that eribulin has subnanomolar effects on both mitotic and non-mitotic processes in tumors and the tumor microenvironment. In November 2010, eribulin was first approved for use in treatment of patients with metastatic breast cancer in the United States. Currently, eribulin is approved for the treatment of breast cancer in more than 65 countries and regions in Europe, the Americas, and Asia. In addition, eribulin was first approved in the United States in January 2016 for the treatment of soft tissue sarcoma, and has been approved in 55 countries and regions ever since. In addition, in the United States and Japan, eribulin has been designated as an orphan drug for the treatment of soft tissue sarcoma. Five most common adverse reactions of eribulin are: decreased white blood cell count, decreased neutrophil count, elevated aspartate aminotransferase, elevated alanine aminotransferase, and anemia.

In the present application, a series of derivatives with good tumor suppression effects are synthesized by modifying eribulin, and have lower toxicity and a larger therapeutic window than that of eribulin; and then the eribulin derivative and an antibody are made into an ADC by means of an appropriate linker, and the therapeutic efficiency of the eribulin derivative is improved using the targeting specificity of the antibody.

### SUMMARY

To achieve the inventive objective of the present application, a first aspect of the present application provides an eribulin derivative, which is a compound of formula (I) or (II), or an isotope-labeled compound, an optical isomer, a geometric isomer, a tautomer, an isomer mixture, or a pharmaceutically acceptable salt thereof; 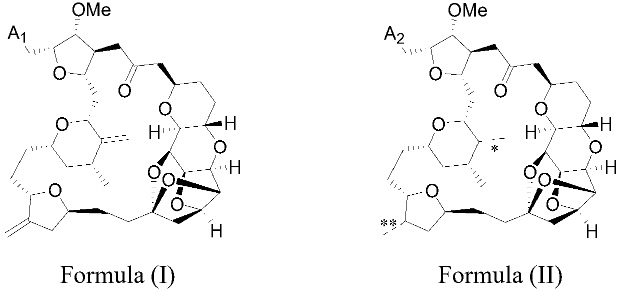 where:
A₁ in formula (I) is selected from 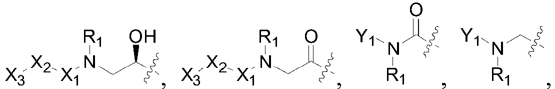 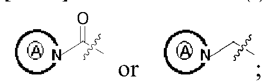
A₂ in formula (II) is selected from 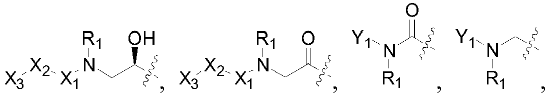 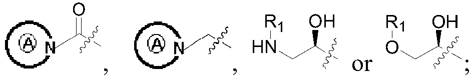 each of 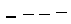 marked by * and ** is independently a single bond, a double bond, or an oxo bond (=O), and at least one of 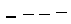 is not a double bond;
R₁ is selected from H, an alkyl group, a hydroxyl-substituted alkyl group, an alkyloxy-substituted alkyl group, an amino-substituted alkyl group, a haloalkyl group or a cyano-substituted alkyl group;
X₁ is selected from CO-, -CH₂, -S(O)-, or -S(O)₂-;
X₂ is selected from the direct bond, -O-, or -NR₁-;
X₃ is selected from -(CR₂R₃)ₘ₁-NH₂, -(CR₂R₃)ₘ₁-OH, -R₄-(CR₂R₃)ₘ₂-NH₂, -R₄-(CR₂R₃)ₘ₂-OH, or 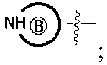
Yi is selected from -(CR₂R₃)ₘ₁-NH₂, -(CR₂R₃)ₘ₁-OH, -R₄-(CR₂R₃)ₘ₂-NH₂, -R₄-(CR₂R₃)ₘ₂-OH, or 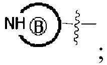
R₂ and R₃ are each independently selected from H, an alkyl group, a hydroxyl group, an alkyloxy group, an amino group, halogen, a cyano group, a hydroxyl-substituted alkyl group, an alkyloxy-substituted alkyl group, an amino-substituted alkyl group, a haloalkyl group, a cyano-substituted alkyl group, an alkyl group substituted by a substituted or unsubstituted carbon ring, an alkyl group substituted by a substituted or unsubstituted heterocyclic substituent, a substituted or unsubstituted carbon ring group or a substituted or unsubstituted heterocyclic group; and the substituted or unsubstituted carbon ring group and the substituted or unsubstituted heterocyclic group are optionally substituted by one or more substituents selected from an oxo group, the alkyl group, the hydroxyl group, the alkyloxy group, the amino group, halogen, the cyano group, the hydroxyl-substituted alkyl group, the alkyloxy-substituted alkyl group, the amino-substituted alkyl group, a haloalkyl group, and the cyano-substituted alkyl group;
R₄ is selected from a substituted or unsubstituted carbon ring group or a substituted or unsubstituted heterocyclic group; and the substituted or unsubstituted carbon ring group and the substituted or unsubstituted heterocyclic group are optionally substituted by one or more substituents selected from an oxo group, the alkyl group, the hydroxyl group, the alkyloxy group, the amino group, halogen, the cyano group, the hydroxyl-substituted alkyl group, the alkyloxy-substituted alkyl group, the amino-substituted alkyl group, a haloalkyl group, and the cyano-substituted alkyl group;
Ⓑ ring is a substituted or unsubstituted heterocyclic group; and a ring of the heterocyclic group contains at least one -NH-, and the substitution refers to substitution with one or more substituents selected from an oxo group, the alkyl group, the alkyloxy group, halogen, the cyano group, the alkyloxy-substituted alkyl group, a haloalkyl group, and the cyano-substituted alkyl group;
Ⓐ ring is the heterocyclic group; and a ring of the heterocyclic group contains at least one -NH-, and/or the heterocyclic group is substituted by the amino group, the hydroxyl group, the amino-substituted alkyl group, or the hydroxyl-substituted alkyl group, and the heterocyclic group is optionally substituted by one or more substituents selected from an oxo group, the alkyl group, the alkyloxy group, halogen, the cyano group, the alkyloxy-substituted alkyl group, a haloalkyl group, and the cyano-substituted alkyl group;
m1 is selected from 1, 2, 3, 4, 5, 6, 7, 8, 9, or 10; and
m2 is selected from 0, 1, 2, 3, 4, 5, 6, 7, 8, 9, or 10.

Specifically, the alkyl group is a C₁-C₁₂ linear or branched alkyl group (for example, the number of carbon atoms is 1, 2, 3, 4, 5, 6, 7, 8, 9, 10, 11, or 12). The carbon ring group may be a saturated, partially unsaturated or completely unsaturated carbon ring group such as a cycloalkyl group, an aryl group, a cycloalkenyl group, a cycloalkynyl group, and an enyne ring group, and the number of carbon atoms may be 3 to 20 (for example, 3, 4, 5, 6, 7, 8, 9, 10, 11, 12, 13, 14, 15, 16, 17, 18, 19, or 20). The heterocyclic group may be a saturated, partially unsaturated or completely unsaturated heterocyclic group such as a heterocycloalkyl group or a heteroaryl group, the number of carbon atoms may be 3 to 20 (for example, 3, 4, 5, 6, 7, 8, 9, 10, 11, 12, 13, 14, 15, 16, 17, 18, 19, or 20), and may contain 1, 2, 3, or 4 heteroatoms selected from N, O, and S.

In a group of embodiments, R₁ is selected from H, the alkyl group, or a hydroxyl-substituted alkyl group, or preferably, is selected from H, a C₁-C₆ alkyl group, or a hydroxy-substituted C₁-C₆ alkyl group, or is more preferably selected from H, a C₁-C₄ alkyl group, or a hydroxy-substituted C₁-C₄ alkyl group; or specifically, can be selected from H, a methyl group, an ethyl group, an n-propyl group, an isopropyl group, a hydroxymethyl group, a hydroxyethyl group, a hydroxy-n-propyl group, and a hydroxyisopropyl group.

In a group of embodiments, X₁ is selected from -CO- or -CH₂-.

In a group of embodiments, m1 is selected from 1, 2, 3, 4, 5, or 6; and m2 is selected from 0, 1, 2, 3, or 4.

In a group of embodiments, R₂ and R₃ are each independently selected from H, a C₁-C₆ alkyl group, a hydroxy group, a C₁-C₆ alkyloxy group, a hydroxy-substituted C₁-C₆ alkyl group, a C₁-C₆ alkyloxy-substituted C₁-C₆ alkyl group, a C₁-C₆ alkyl group substituted by a substituted or unsubstituted C₃-C₆ cycloalkyl group, a C₁-C₆ alkyl group substituted by a substituted or unsubstituted 3- to 6-membered heterocycloalkyl group containing 1 or 2 heteroatoms of N or O, a substituted or unsubstituted C₃-C₆ cycloalkyl group, and a substituted or unsubstituted 3- to 6-membered heterocycloalkyl group containing 1 or 2 heteroatoms of N or O; and the substituted or unsubstituted C₃-C₆ cycloalkyl group and the substituted or unsubstituted 3- to 6-membered heterocycloalkyl group containing 1 or 2 heteroatoms of N or O are optionally substituted with one or more substituents selected from oxo, a C₁-C₆ alkyl group, a hydroxy group, a C₁-C₆ alkyloxy group, a hydroxy-substituted C₁-C₆ alkyl group, and a C₁-C₆ alkyloxy-substituted C₁-C₆ alkyl group. Preferably, R₂ and R₃ are each independently selected from H, a C₁-C₆ alkyl group, a hydroxy group, a hydroxy-substituted C₁-C₆ alkyl group, a C₁-C₆ alkyl group substituted by a substituted or unsubstituted C₃-C₆ cycloalkyl group, a C₁-C₆ alkyl group substituted by a substituted or unsubstituted 3- to 6-membered heterocycloalkyl group containing 1 or 2 heteroatoms of N or O, a substituted or unsubstituted C₃-C₆ cycloalkyl group, and a substituted or unsubstituted 3- to 6-membered heterocycloalkyl group containing 1 or 2 heteroatoms of N or O; and the substituted or unsubstituted C₃-C₆ cycloalkyl group and the substituted or unsubstituted 3- to 6-membered heterocycloalkyl group containing 1 or 2 heteroatoms of N or O are optionally substituted with one or more substituents selected from oxo, a C₁-C₆ alkyl group, a hydroxy group, and a hydroxy-substituted C₁-C₆ alkyl group.

In a group of embodiments, R₄ is selected from a substituted or unsubstituted C₃-C₁₂ monocyclic, fused, bicyclic, spiro or bridged cycloalkyl group, a substituted or unsubstituted 3- to 12-membered monocyclic, fused, bicyclic, spiro or bridged heterocycloalkyl group containing 1, 2, or 3 heteroatoms of N or O, a substituted or unsubstituted C₆-C₁₀ aryl group, and a substituted or unsubstituted 5- to 10-membered heteroaryl group containing 1, 2, or 3 heteroatoms of N or O; and substitution means being substituted with one or more substituents selected from oxo, a C₁-C₆ alkyl group, a hydroxy group, a C₁-C₆ alkyloxy group, a hydroxy-substituted C₁-C₆ alkyl group, and a C₁-C₆ alkyloxy-substituted C₁-C₆ alkyl group. Preferably, R₄ is selected from a substituted or unsubstituted C₄-C₁₂ monocyclic, fused, bicyclic, spiro or bridged cycloalkyl group, a substituted or unsubstituted 4- to 12-membered monocyclic, fused, bicyclic, spiro or bridged heterocycloalkyl group containing 1, 2, or 3 heteroatoms of N or O, a substituted or unsubstituted phenyl group, and a substituted or unsubstituted 5- to 10-membered heteroaryl group containing 1, 2, or 3 heteroatoms of N or O; and substitution means being substituted with one or more substituents selected from oxo and a C₁-C₆ alkyl group.

In a group of embodiments, Ⓑ ring is a substituted or unsubstituted 3- to 12-membered monocyclic, fused, bicyclic, spiro or bridged heterocycloalkyl group containing 1, 2, or 3 heteroatoms of N or O, the heterocycloalkyl ring contains at least one -NH-, and substitution means being substituted with one or more substituents selected from oxo, a C₁-C₆ alkyl group, a C₁-C₆ alkyloxy group, and a C₁-C₆ alkyloxy-substituted C₁-C₆ alkyl group.

In a group of embodiments, X₃ is selected from -(CR₂R₃)ₘ₁-NH₂, -(CR₂R₃)ₘ₁-OH, -R₄-(CR₂R₃)ₘ₂-NH₂, -R₄-(CR₂R₃)ₘ₂-OH, or 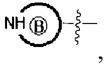 Ⓑ ring is a substituted or unsubstituted 4-to 12-membered monocyclic, fused, bicyclic, spiro or bridged heterocycloalkyl group containing 1, 2, or 3 heteroatoms of N or O, the substitution means being substituted with one or more substituents selected from oxo and a C₁-C₆ alkyl group; m1 is selected from 1, 2, 3, 4, 5, or 6; m2 is selected from 0, 1, 2, 3, 4, 5, or 6; R₂ and R₃ are each independently selected from H, a C₁-C₆ alkyl group, a hydroxy group, a hydroxy-substituted C₁-C₆ alkyl group, a C₁-C₆ alkyl group substituted by a substituted or unsubstituted C₃-C₆ cycloalkyl group, and a substituted or unsubstituted C₃-C₆ cycloalkyl group, where the substituted or unsubstituted C₃-C₆ cycloalkyl group is optionally substituted with one or more substituents selected from oxo and a C₁-C₆ alkyl group; and R₄ is selected from a C₄-C₁₂ monocyclic, fused, bicyclic, spiro or bridged cycloalkyl group, a 4- to 12-membered monocyclic, fused, bicyclic, spiro or bridged heterocycloalkyl group containing 1, 2, or 3 heteroatoms of N or O, and a phenyl group.

More preferably, X₃ is selected from the following groups:

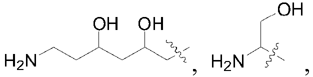

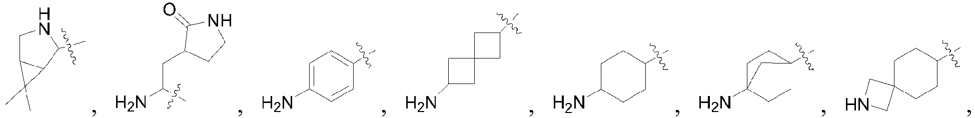

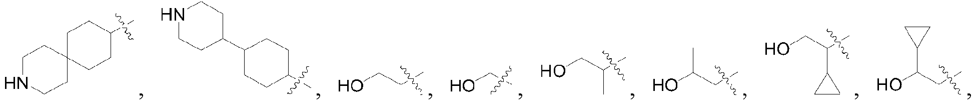

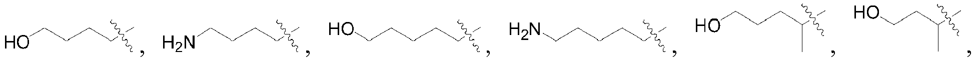

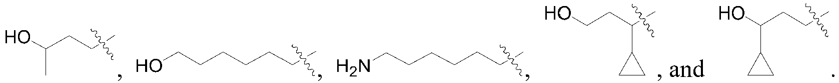

Most preferably, X₃ is selected from the following groups:

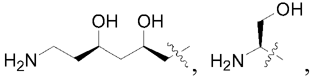

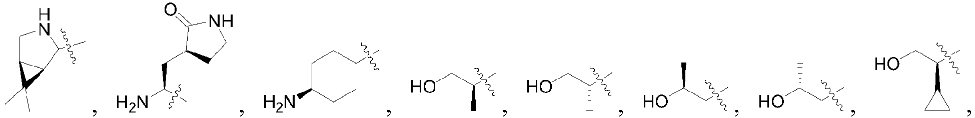

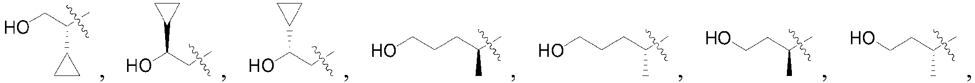

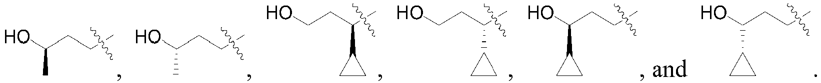

In a group of embodiments, Yi is selected from -(CR₂R₃)ₘ₁-NH₂, -(CR₂R₃)ₘ₁-OH, -R₄-(CR₂R₃)ₘ₂-NH₂, -R₄-(CR₂R₃)ₘ₂-OH, or 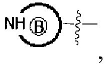 Ⓑ ring is a substituted or unsubstituted 4-to 12-membered monocyclic, fused, bicyclic, spiro or bridged heterocycloalkyl group containing 1, 2, or 3 heteroatoms of N or O, the substitution means being substituted with one or more substituents selected from oxo and a C₁-C₆ alkyl group; m1 is selected from 1, 2, 3, 4, 5, or 6; m2 is selected from 0, 1, 2, 3, 4, 5, or 6; R₂ and R₃ are each independently selected from H, a C₁-C₆ alkyl group, a hydroxy group, a hydroxy-substituted C₁-C₆ alkyl group, a C₁-C₆ alkyl group substituted by a substituted or unsubstituted C₃-C₆ cycloalkyl group, and a substituted or unsubstituted C₃-C₆ cycloalkyl group, where the substituted or unsubstituted C₃-C₆ cycloalkyl group is optionally substituted with one or more substituents selected from oxo and a C₁-C₆ alkyl group; and R₄ is selected from a C₄-C₁₂ monocyclic, fused, bicyclic, spiro or bridged cycloalkyl group, a 4- to 12-membered monocyclic, fused, bicyclic, spiro or bridged heterocycloalkyl group containing 1, 2, or 3 heteroatoms of N or O, and a phenyl group.

Preferably, Y₁ is selected from -(CR₂R₃)ₘ₁-NH₂ and -(CR₂R₃)ₘ₁-OH, m1 is selected from 1, 2, 3, 4, 5, or 6, and R₂ and R₃ are each independently selected from H, a C₁-C₆ alkyl group, a hydroxy group, or a hydroxy-substituted C₁-C₆ alkyl group.

More preferably, Y₁ is selected from the following group:

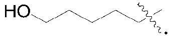

In a group of embodiments, Ⓐ ring is a 3- to 12-membered monocyclic, fused, bicyclic, spiro or bridged heterocycloalkyl group containing 1, 2, or 3 heteroatoms of N or O, the heterocycloalkyl ring contains at least one -NH- and/or the heterocycloalkyl group is substituted with an amino group, a hydroxy group, an amino-substituted C₁-C₆ alkyl group, or a hydroxy-substituted C₁-C₆ alkyl group, and the heterocycloalkyl group is optionally substituted with one or more substituents selected from oxo, a C₁-C₆ alkyl group, a C₁-C₆ alkyloxy group, or a C₁-C₆ alkyloxy-substituted C₁-C₆ alkyl group.

In a group of embodiments, Ⓐ ring is a 4- to 12-membered monocyclic, fused, bicyclic, spiro or bridged heterocycloalkyl group containing 1, 2, or 3 heteroatoms of N or O, and the heterocycloalkyl ring contains at least one -NH- and/or the heterocycloalkyl group is substituted with an amino group, a hydroxy group, an amino-substituted C₁-C₆ alkyl group, or a hydroxy-substituted C₁-C₆ alkyl group.

More preferably, Ⓐ is selected from the following groups:

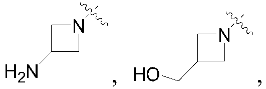

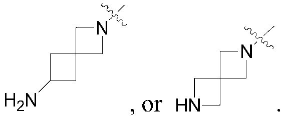

In a group of embodiments, A₁ and A₂ are selected from the following groups:

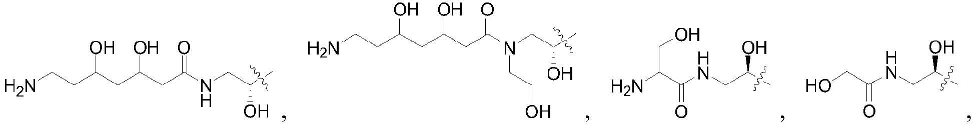

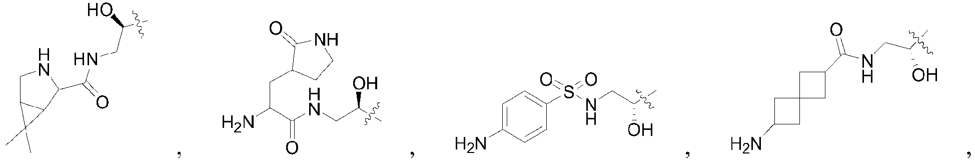

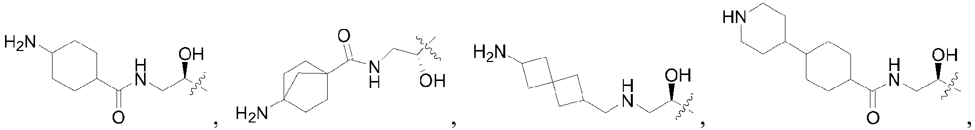

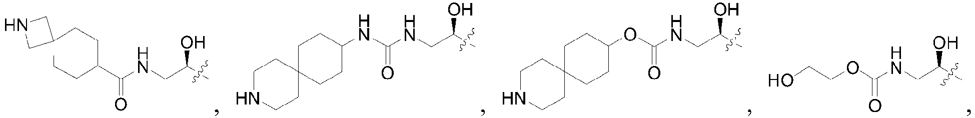

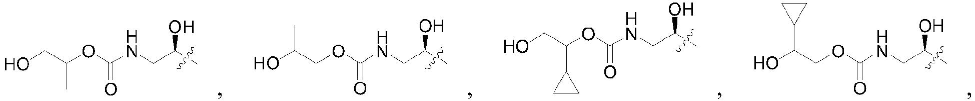

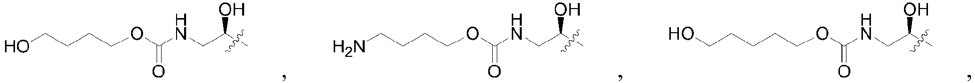

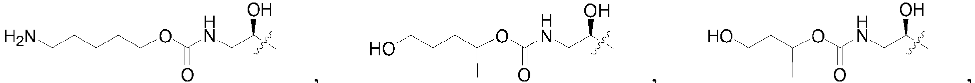

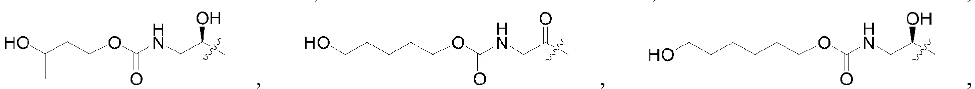

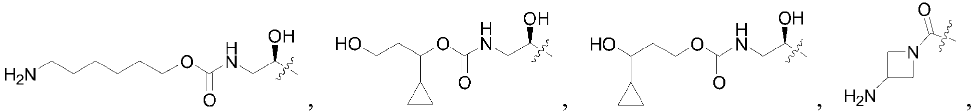

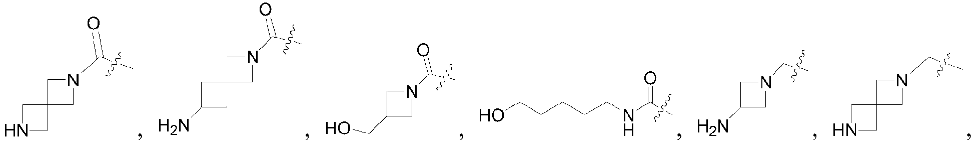

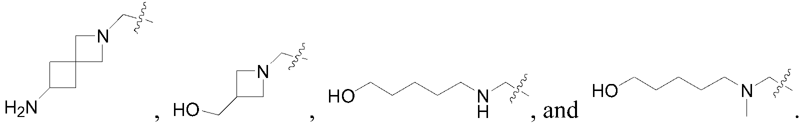

A₂ in formula (II) is alternatively selected from

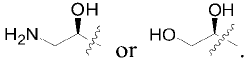

Preferably, A₁ and A₂ are selected from the following groups:

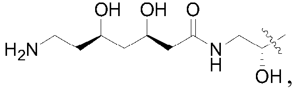

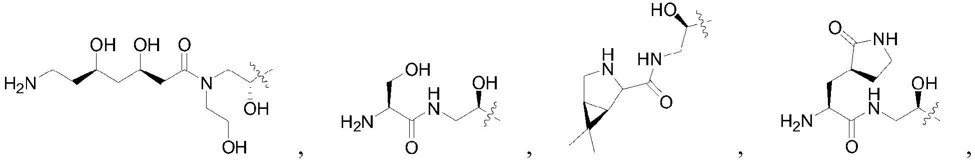

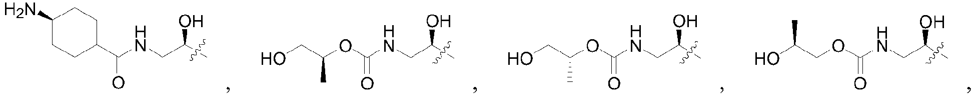

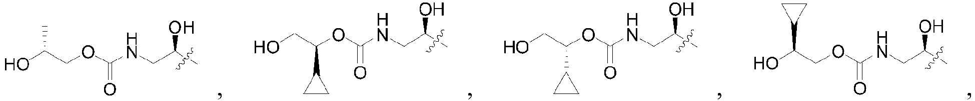

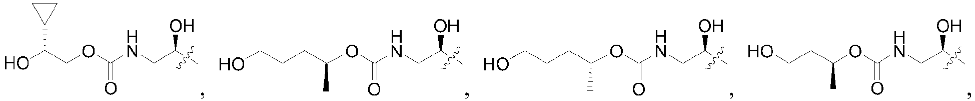

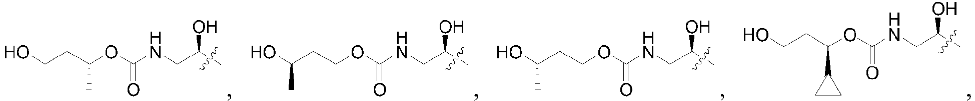

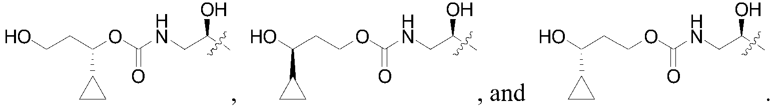

Specifically, the compound of formula (I) is one of the following: 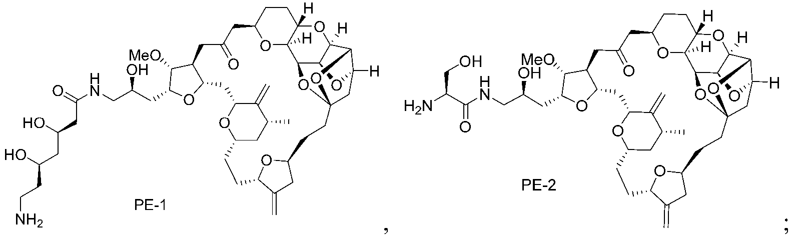 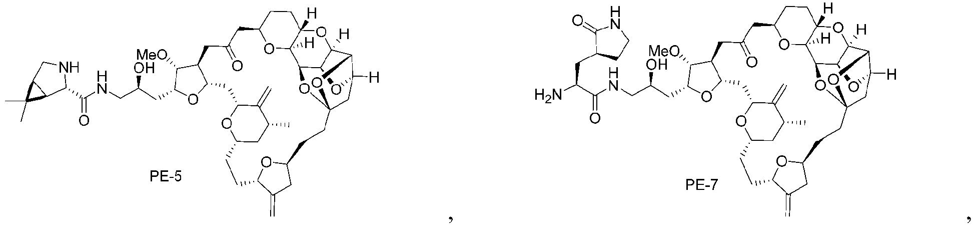 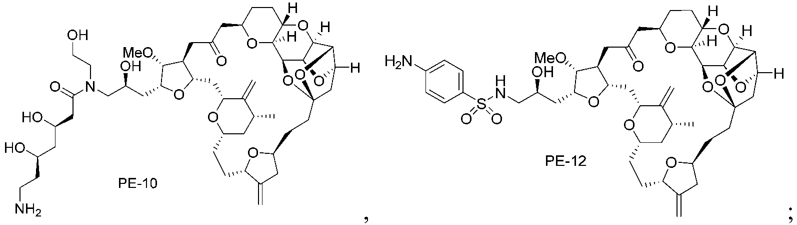 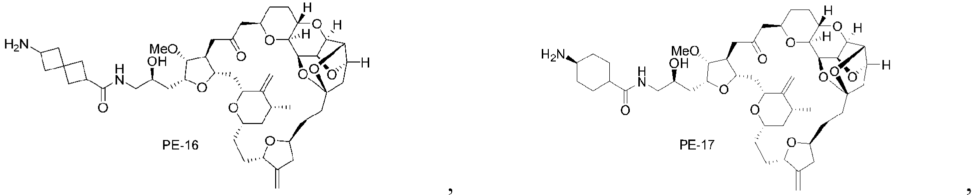 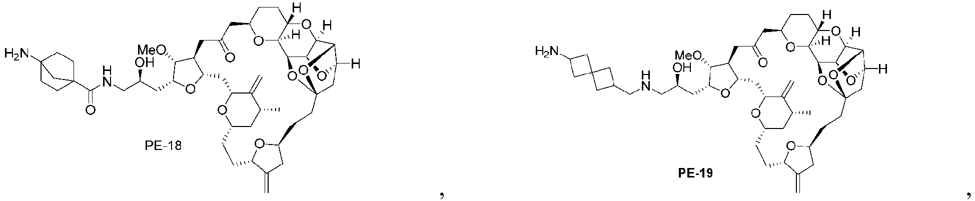 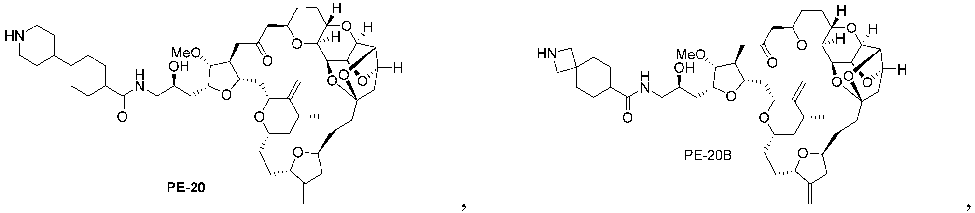 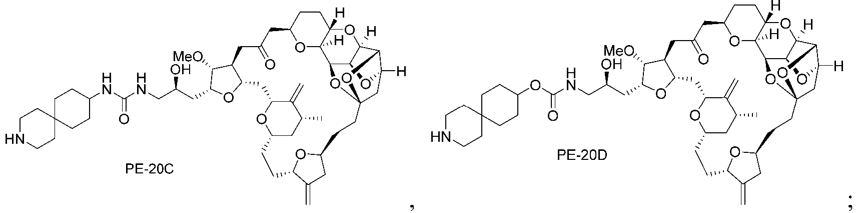 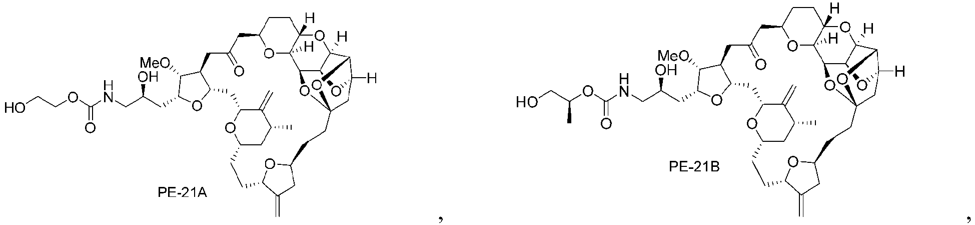 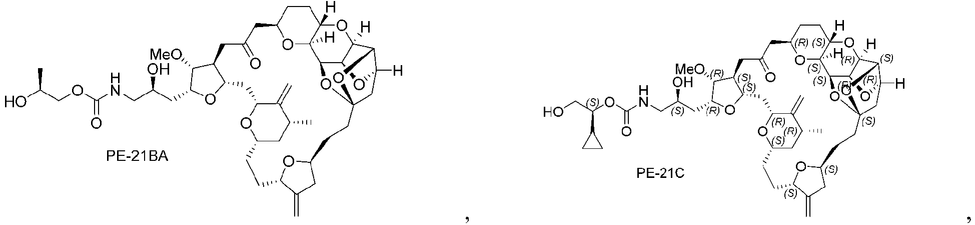 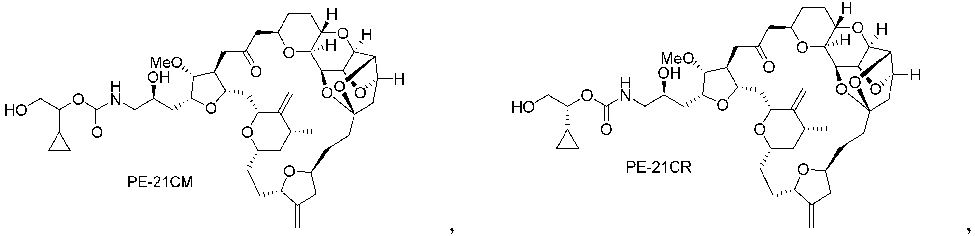 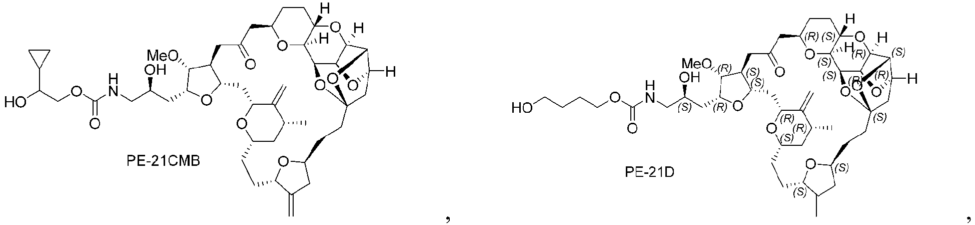 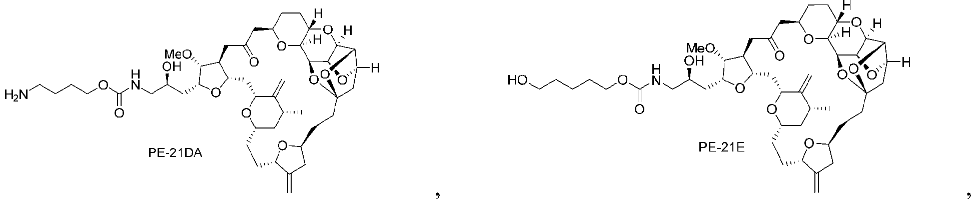 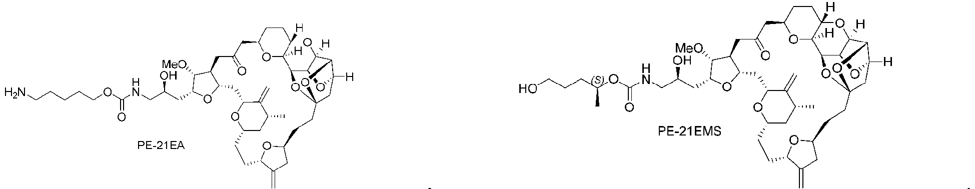 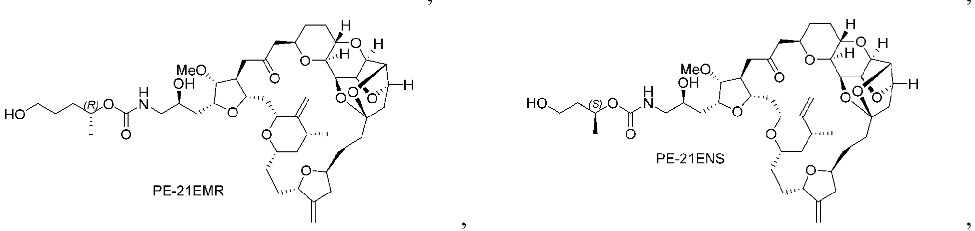 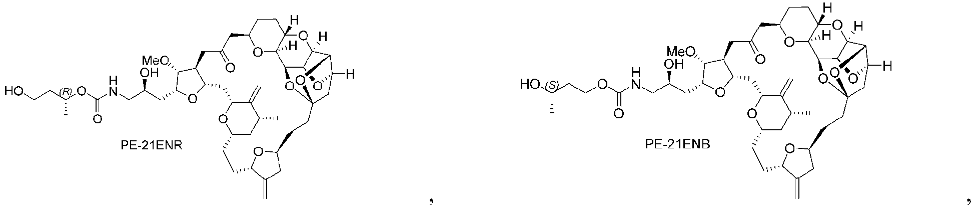 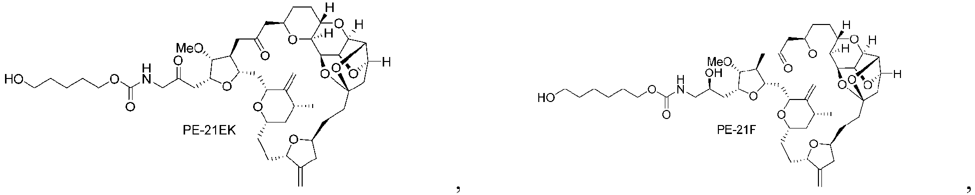 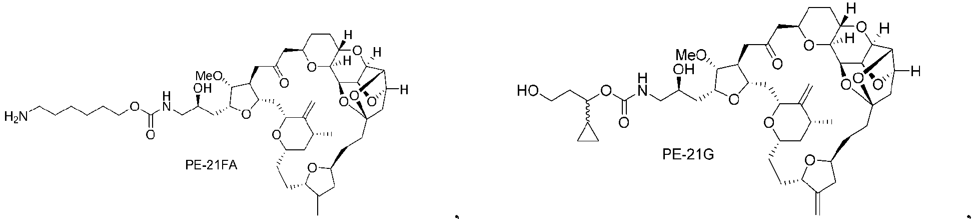 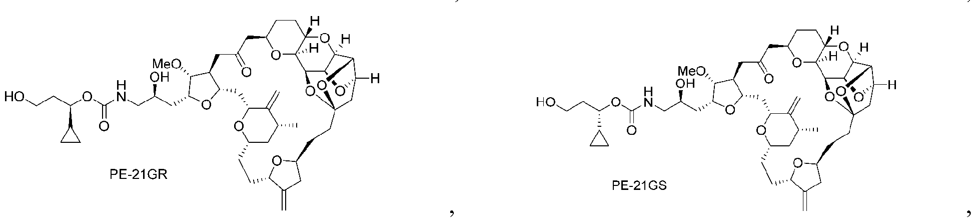 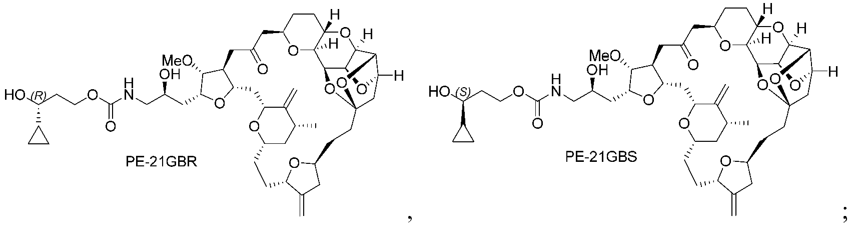 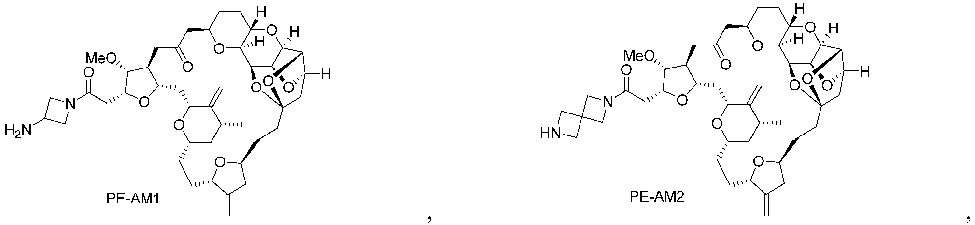 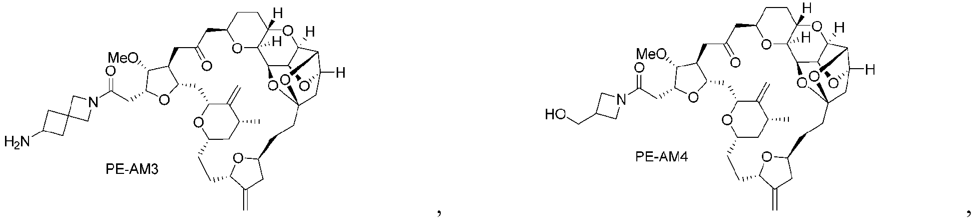 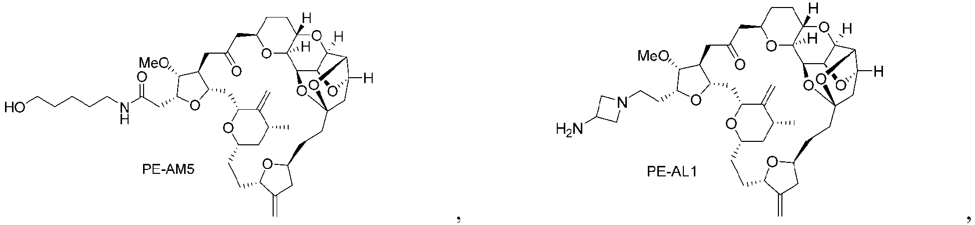 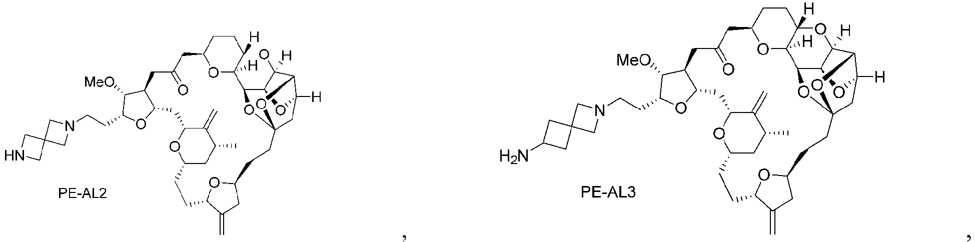 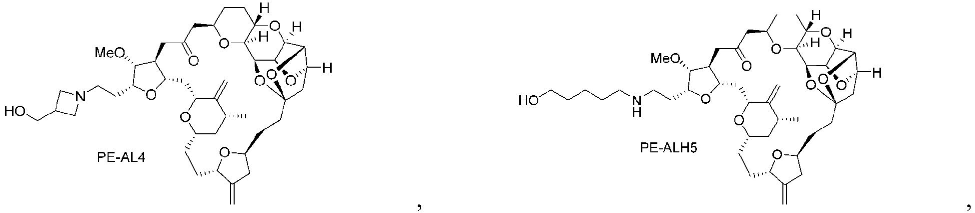 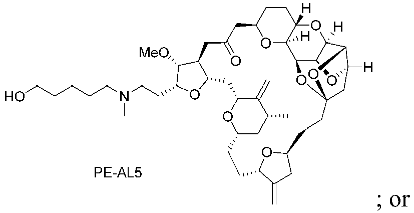 specifically, the compound of formula (II) is one of the following:

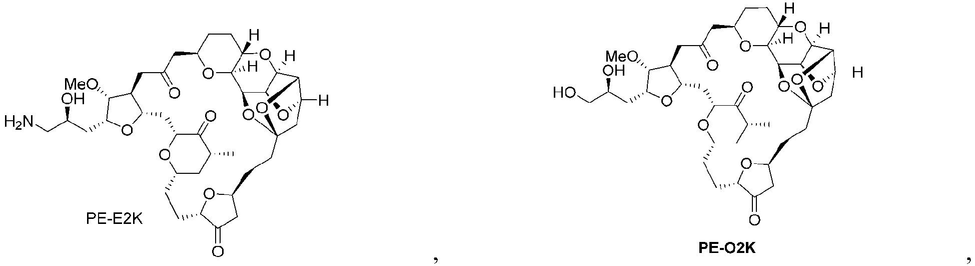

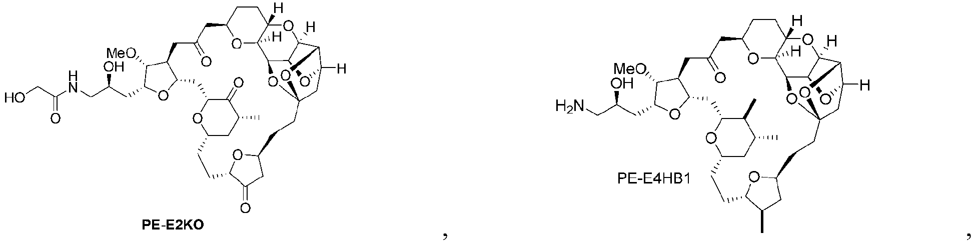

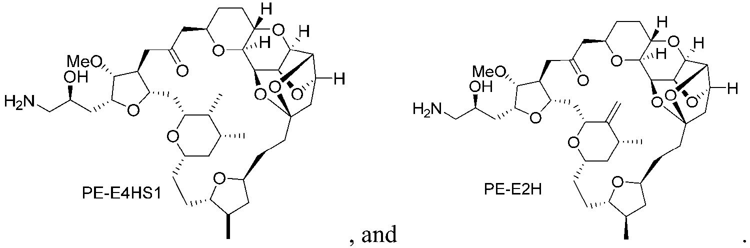

A second aspect of the present application provides an eribulin derivative, which is a compound of formula (I') or (II'), or an isotope-labeled compound, an optical isomer, a geometric isomer, a tautomer, an isomer mixture, or a pharmaceutically acceptable salt thereof; 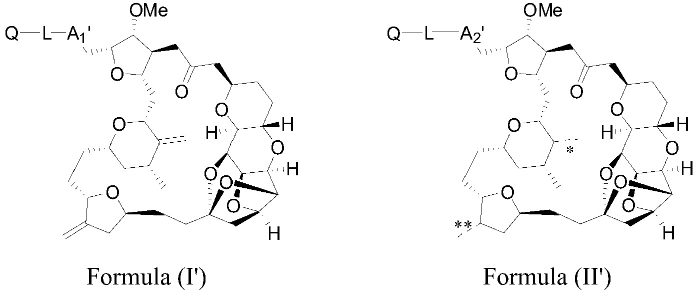 where:
Q is a linker group capable of conjugating to an antibody or antigen-binding fragment;
L is a linker group that connects Q to A₁' or Q to A₂';
A₁' is a group formed by connecting A₁ to L after removal of a hydrogen atom from -NH₂, -OH, -NH- on the chain, or -NH- on the ring of A₁;
A₂' is a group formed by connecting A2 to L after removal of a hydrogen atom from -NH2, -OH, -NH- on the chain, or -NH- on the ring of A2; and
definitions of A₁, A₂, *, and ** are the same as those in the foregoing formula (I) and formula (II).

In a group of embodiments, when X₃ and Y₁ are each independently selected from -(CR₂R₃)ₘ₁-NH₂, -(CR₂R₃)ₘ₁-OH, -R4-(CR2R3)m2-NH2, and -R4-(CR2R3)m2-OH, A₁' is a group formed by connecting A₁ to L after removal of the hydrogen atom from terminal -NH₂ or terminal -OH of X₃ or Y₁; and A₂' is a group formed by connecting A₂ to L after removal of the hydrogen atom from the terminal -NH₂ or terminal -OH of X₃ or Y₁;
when X₃ and Y₁ are each independently 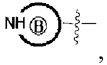 A₁' represents a group formed by connecting A₁ to L after removal of the hydrogen atom of -NH- on the Ⓑ ring, and A2' represents a group formed by connecting A₂ to L after removal of the hydrogen atom of -NH- on the Ⓑ ring; and
when A₁ and A₂ are each independently 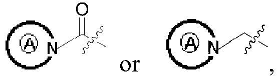
A₁' is a group formed by connecting A₁ to L after removal of the hydrogen atom of -NH- on the Ⓐ ring, or the hydrogen atom of -NH₂ or -OH on the Ⓐ ring substituent, and A₂' is a group formed by connecting A₂ to L after removal of the hydrogen atom of -NH- on the Ⓐ ring, or the hydrogen atom of -NH₂ or -OH on the Ⓐ ring substituent.

Definitions of R₁, X₁, X₂, R₂, R₃, R₄, Ⓑ ring, Ⓐ ring, m1, and m2 are the same as those in the foregoing formula (I) and formula (II).

In a group of embodiments, R₁ is H, A₁' is a group formed by connecting A₁ to L after removal of R₁ from -NR₁-, and A₂' is a group formed by connecting A₂ to L after removal of R₁ from -NR₁-.

In a group of embodiments, L is -L₄-L₃-L₂-L₁-, and Li is connected to A₁' or A₂', and L₄ is connected to Q;
Li is selected from the direct bond, 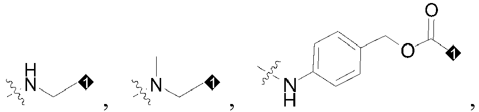 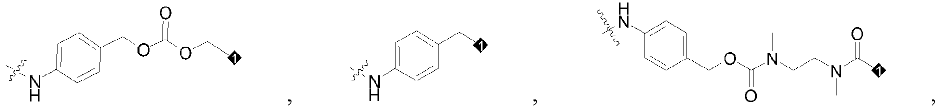 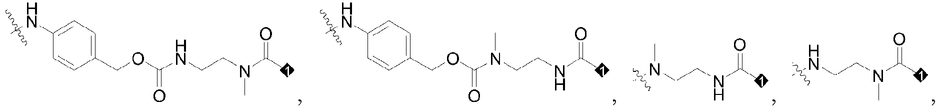 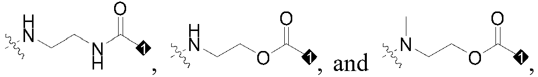 where 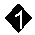 is a ligation site for ligating A₁' or A₂' and 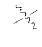 is a ligation site for ligating L₂;
L₂ is a direct bond, an amino acid residue, or a short peptide chain formed by two or more amino acid residues (for example, 2 to 10 amino acid residues, optionally, a short peptide chain formed by 2, 3, 4, 5, 6, 7, 8, 9, and 10 amino acid residues), the amino acid residues are selected from natural or unnatural amino acid residues, the amino acid residue or short peptide chain has N-terminus for ligating L₃ and C-terminus for ligating L₁, and L₂ optionally contains one or more structures selected from formula (III-1) to formula (III-9): 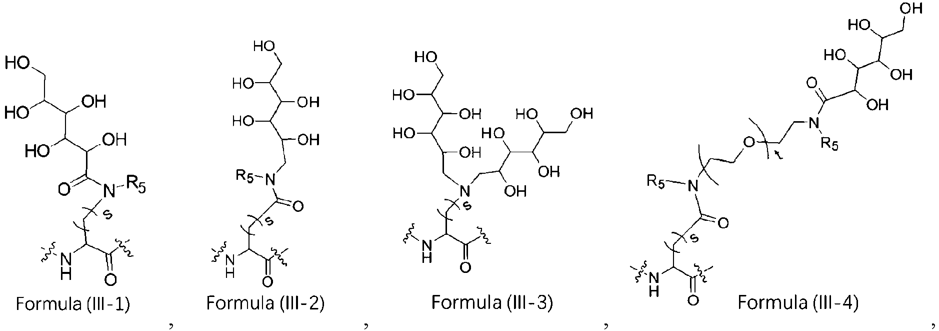 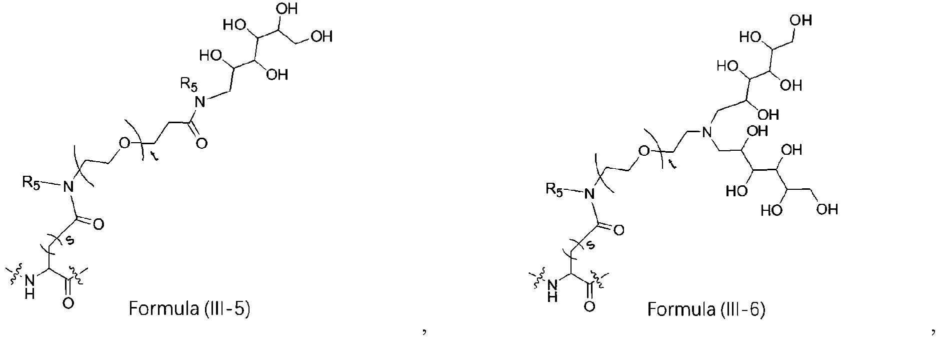 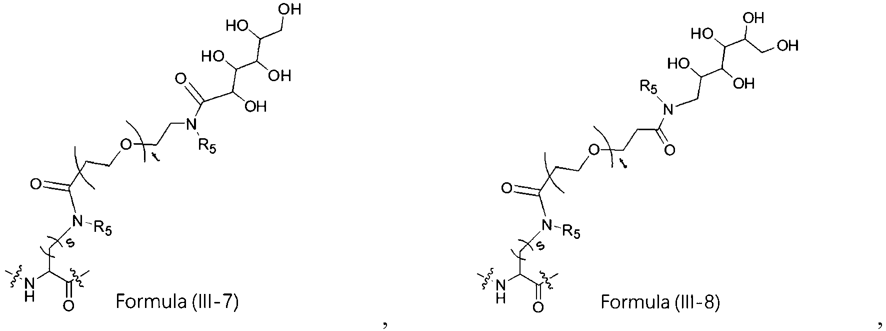 and 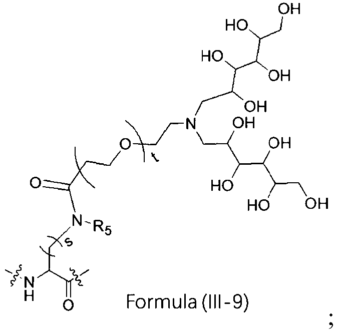
s is selected from 1, 2, 3, 4, 5, 6, 7, and 8, t is selected from 1, 2, 3, 4, 5, 6, 7, 8, 9, 10, 11, and 12, and R₅ is selected from H or a C₁-C₈ alkyl group; or preferably, s is selected from 2, 3, 4, 5, and 6, t is selected from 4, 5, 6, 7, and 8, and R₅s is selected from H or a C₁-C₆ alkyl group;
L₃ is selected from a direct bond, -NR₆-(CH₂)ᵣ₁-(CH₂CH₂O)ᵣ₂-(CH₂)ᵣ₃-CO- 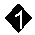 , -O-(CH₂)ᵣ₁-(CH₂CH₂O)ᵣ₂-(CH₂)ᵣ₃-CO- 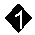 , -NR₆-(CH₂)ᵣ₄-(OCH₂CH₂)ᵣ₅-(CH₂)ᵣ₆-CO- 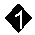 , or -O-(CH₂)ᵣ₄-(OCH₂CH₂)ᵣ₅-(CH₂)ᵣ₆-CO- 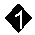 ; or L₃ is selected from: 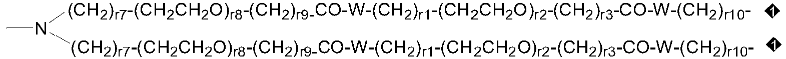 or 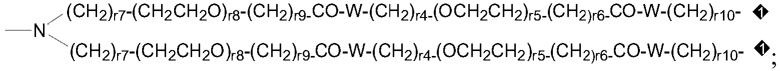 r1 is selected from 0, 1, 2, 3, 4, 5, or 6, r2 is selected from 1, 2, 3, 4, 5, 6, 7, 8, 9, 10, 11, or 12, r3 is selected from 1, 2, 3, 4, 5, or 6, r4 is selected from 1, 2, 3, 4, 5, or 6, r5 is selected from 1, 2, 3, 4, 5, 6, 7, 8, 9, 10, 11, or 12, r6 is selected from 0, 1, 2, 3, 4, 5, or 6, r7 is selected from 0, 1, 2, 3, 4, 5, or 6, r8 is selected from 1, 2, 3, 4, 5, 6, 7, 8, 9, 10, 11, or 12, r9 is selected from 1, 2, 3, 4, 5, or 6, r10 is selected from 1, 2, 3, 4, 5, or 6, W is selected from O or NR₆, R₆ is selected from H or a C₁-C₈ alkyl group, where 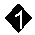 represents an attachment site to L2, and when 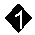 appears twice in the same L₃ group, it means that L₂ is attached at both sites; and preferably, r1 is selected from 0, 1, 2, 3, or 4; r2 is selected from 4, 5, 6, 7, or 8; r3 is selected from 1, 2, 3, or 4; r4 is selected from 1, 2, 3, or 4; r5 is selected from 4, 5, 6, 7, or 8; r6 is selected from 0, 1, 2, 3, or 4; r7 is selected from 0, 1, 2, 3, or 4; r8 is selected from 1, 2, 3, 4, 5, or 6; r9 is selected from 1, 2, 3, or 4; r10 is selected from 1, 2, 3, or 4; W is selected from O or NR₆; and R₆ is selected from H or C₁-C₆ alkyl group; and
L₄ is selected from a direct bond, -CO-, -(CH₂)ₚ₁-CO-, -(CH₂CH₂O)ₚ₂-(CH₂)ₚ₃-CO-, -(CH₂)ₚ₄-O-(CH₂)ₚ₅-C(O)-, -NR₇-(CH₂)ₚ₁-CO-, -NR₇-(CH₂CH₂O)ₚ₂-(CH₂)ₚ₃-CO-, -NR₇-(CH₂)ₚ₄-O-(CH₂)ₚ₅-C(O)-, -O-(CH₂)ₚ₁-CO-, -O-(CH₂CH₂O)ₚ₂-(CH₂)ₚ₃-CO-, and -O-(CH₂)ₚ₄-O-(CH₂)ₚ₅-C(O)-, p1, p2, p3, p4, and p5 are each independently selected from 1, 2, 3, 4, 5, 6, 7, and 8, and R₇ is selected from H or a C₁-C₈ alkyl group, where -CO- is ligated to L₃; and preferably, p1 is selected from 1, 2, 3, 4, 5, and 6, p2 is selected from 1, 2, 3, and 4, p3 is selected from 1, 2, 3, and 4, p4 is selected from 1, 2, 3, and 4, p5 is selected from 1, 2, 3, and 4, and R₇ is selected from H or a C₁-C₆ alkyl group.

In a group of embodiments, A₁' represents a group formed by connecting A₁ to L after removal of the hydrogen atom from -NH₂, -NH- on the chain, or -NH- on the ring, and A₂' represents a group formed by connecting A₂ to L after removal of the hydrogen atom from -NH₂, -NH- on the chain, or -NH- on the ring, and L₁ is selected from a direct bond, 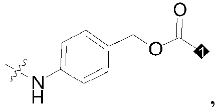 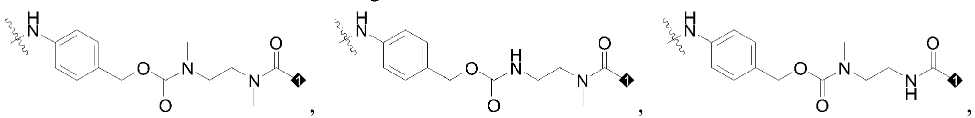 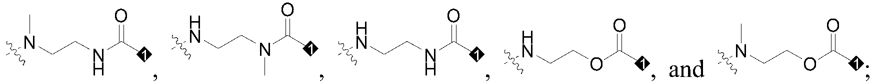 or when A₁' represents a group formed by connecting A₁ to L after removal of the hydrogen atom of -OH-, or A2' represents a group formed by connecting A₂ to L after removal of the hydrogen atom of -OH-, L₁ is selected from

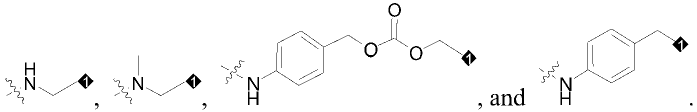

In a group of embodiments, L₁ is selected from a direct bond, 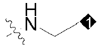 and

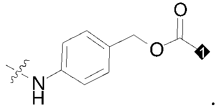

In a group of embodiments, L₂ is an amino acid residue or a short peptide chain formed by 2 to 8 amino acid residues, and preferably, the amino acid residues are amino acid residues formed by amino acids selected from phenylalanine, alanine, glycine, valine, citrulline, leucine, isoleucine, tryptophan, tyrosine, histidine, lysine, serine, threonine, cysteine, glutamic acid, glutamine, aspartic acid, asparagine, methionine, and arginine, or more preferably, the amino acid residues are amino acid residues formed by amino acids selected from phenylalanine, alanine, glycine, valine, and citrulline.

In a group of embodiments, L₂ optionally includes one or more structures selected from formula (III-1) and formula (III-9), or preferably, includes one or more structures selected from the following groups: 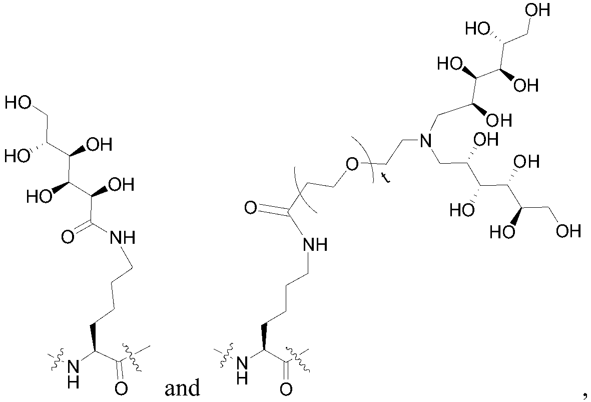 where t=8.

In a group of embodiments, L₂ is selected from -^{NH}Gly^{CO}-, or selected from the following short peptide chains: -^{NH}Gly-Gly-Phe-Gly^{CO}-, -^{NH}Val-Cit^{CO}-, -^{NH}Val-Ala^{CO}-, -^{NH}Gly-Gly-Val-Ala^{CO}-, -^{NH} Gly-Gly-Val-Cit^{CO}-, -^{NH}Lys-Gly-Val-Ala^{CO}-, -^{NH}Lys-Gly-Val-Cit^{CO}-, and -^{NH}Gly-Val-Cit^{CO}-. In addition, L₂ optionally contains one or more structures selected from formula (III-1) to formula (III-9); or preferably, L₂ optionally contains one or more structures selected from formula (III-1) and formula (III-9).

Preferably, L₂ is selected from the following groups: 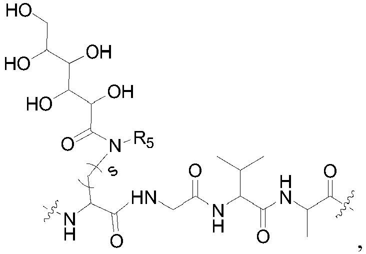 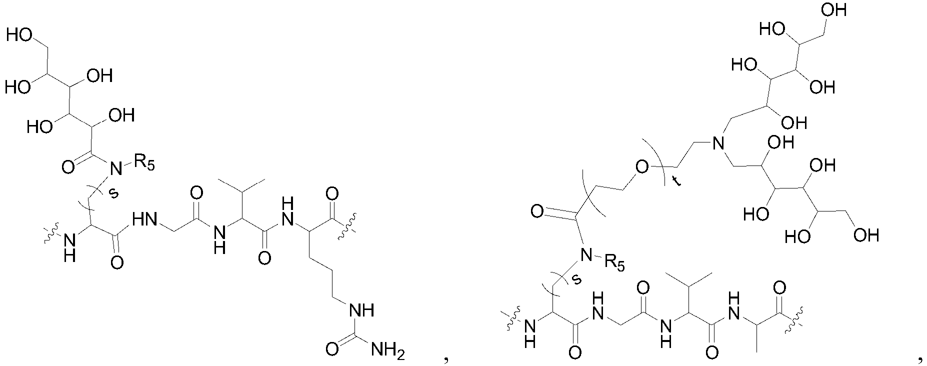 and 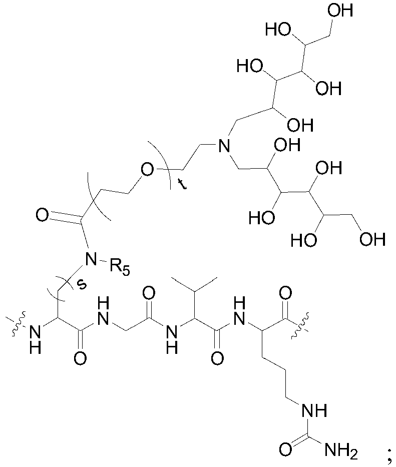 and

preferably, L₂ is selected from the following groups: 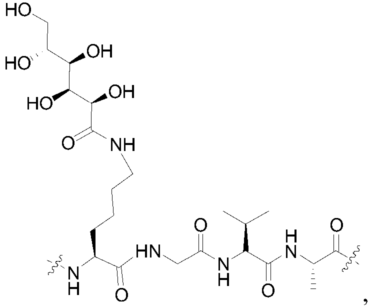 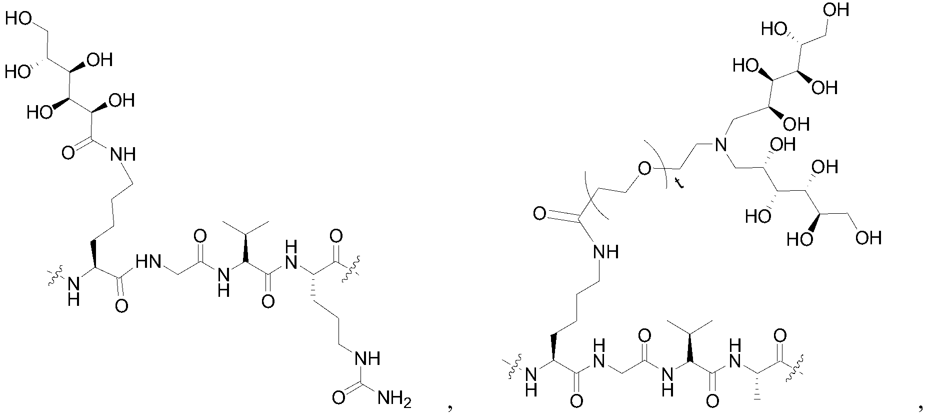 and 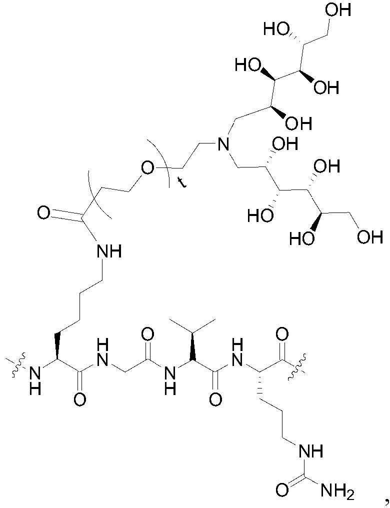 where t=8.

In a group of embodiments, L₃ is selected from a direct bond, -NH-(CH₂CH₂O)ᵣ₂- (CH₂)ᵣ₃-CO- 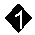 , -NH-(CH₂)ᵣ₄-(OCH₂CH₂)ᵣ₅-CO- 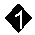 , -O-(CH₂CH₂O)ᵣ₂-(CH₂)ᵣ₃-CO- 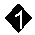 or -O-(CH₂)ᵣ₄- (OCH₂CH₂)ᵣ₅-CO- 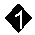 ; L₃ is selected from 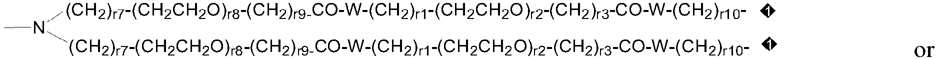 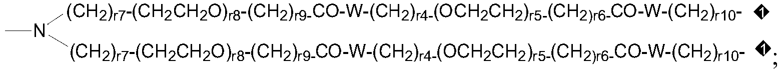 r1, r6, or r7 is 0, r2 is selected from 4, 5, 6, 7, or 8, r3 is selected from 1, 2, 3, or 4, r4 is selected from 1, 2, 3, or 4, r5 is selected from 4, 5, 6, 7, or 8, r8 is selected from 1, 2, 3, or 4, r9 is selected from 1, 2, 3, or 4, r10 is selected from 1, 2, 3, or 4, and W is NH; or preferably, L₃ is selected from a direct bond, -O-(CH₂CH₂O)ᵣ₂-(CH₂)ᵣ₃-CO- 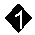 , -NH-(CH₂CH₂O)ᵣ₂-(CH₂)ᵣ₃-CO- 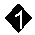 , and 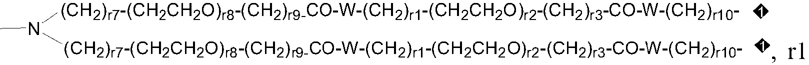 and r7 are 0, r2 is selected from 4, 5, 6, 7, or 8, r3 is 2, r8 is selected from 2, 3, or 4, r9 is 2, and r10 is 2.

In a group of embodiments, L₄ is selected from the direct bond, -(CH₂)ₚ₁-CO-, -(CH₂CH₂O)ₚ₂-(CH₂)ₚ₃-CO-, -(CH₂)ₚ₄-O-(CH₂)ₚ₅-C(O)-, -NR₇-(CH₂)ₚ₁-CO-, -NR₇-(CH₂CH₂O)ₚ₂-(CH₂)ₚ₃-CO-, or -NR₇-(CH₂)ₚ₄-O-(CH₂)ₚ₅-C(O)-, p1 is selected from 1, 2, 3, 4, 5, or 6, p2, p3, p4, and p5 are each independently selected from 1, 2, 3, or 4, and R₇ is selected from H or a C₁-C₆ alkyl group. Preferably, L₄ is selected from the following groups: the direct bond, -(CH₂)ₚ₁-CO-, -(CH₂CH₂O)ₚ₂-(CH₂)ₚ₃-CO-, -(CH₂)ₚ₄-O-(CH₂)ₚ₅-C(O)-, -NR₇-(CH₂)ₚ₁-CO-, -NR₇-(CH₂CH₂O)ₚ₂-(CH₂)ₚ₃-CO-, or -NR₇-(CH₂)ₚ₄-O-(CH₂)ₚ₅-C(O)-, p1 is selected from 3, 4, 5, or 6, p2, p3, p4, and p5 are each independently selected from 2 and 3, and R₇ is H.

In a group of embodiments, Q is selected from a group containing a maleimide linker, a group containing a methanesulfonylpyrimidine linker, a group containing a methanesulfonylpyrimidinylthiazole linker, a group containing a methanesulfonylpyrimidinothiazole linker, a group containing a cycloenyne linker, or a group containing an oxime bond linker.

In a group of embodiments, Q is selected from

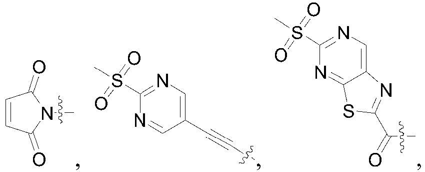

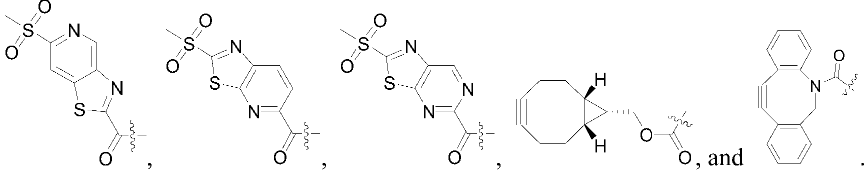

In a group of embodiments, Q-L₄ is selected from 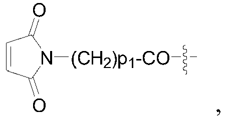 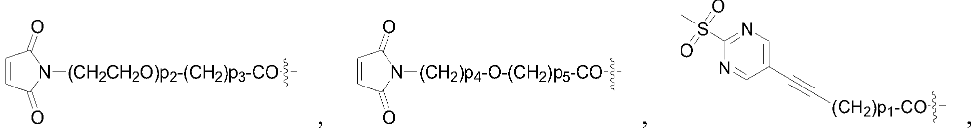 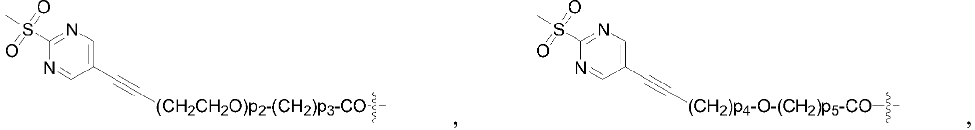 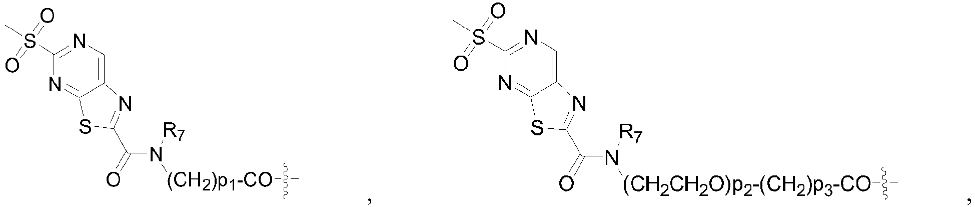 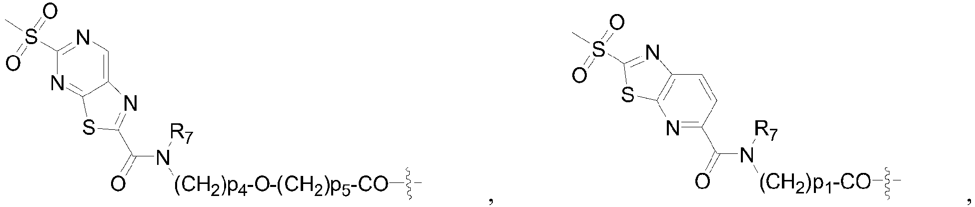 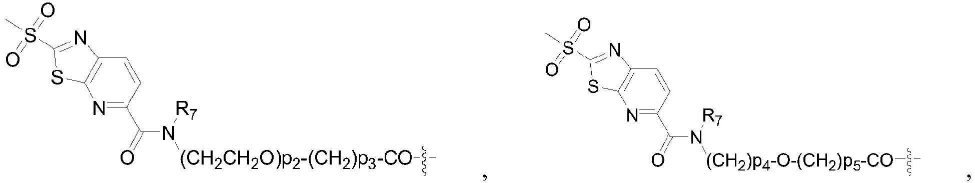 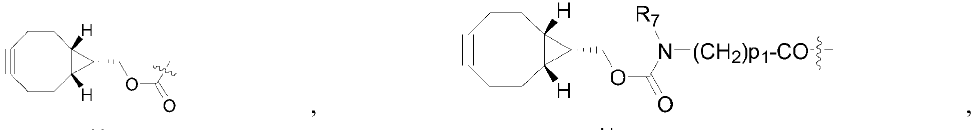 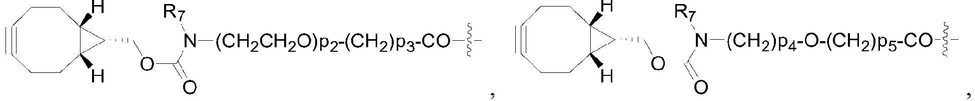 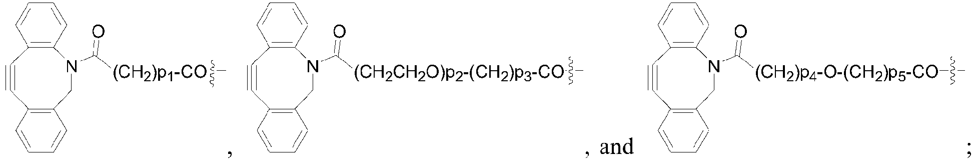 and
p1 is selected from 1, 2, 3, 4, 5, and 6, p2 is selected from 1, 2, 3, and 4, p3 is selected from 1, 2, 3, and 4, p4 is selected from 1, 2, 3, and 4, p5 is selected from 1, 2, 3, and 4, and R₇ is selected from H or a C₁-C₆ alkyl group. Preferably, p1 is selected from 3, 4, 5, and 6; p2 is selected from 2 and 3; p3 is selected from 2 and 3; p4 is selected from 2 and 3; p5 is selected from 2 and 3; and R₇ is selected from H.

Preferably, Q-L₄ is selected from

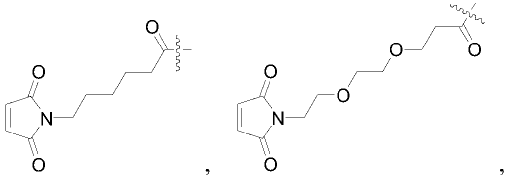

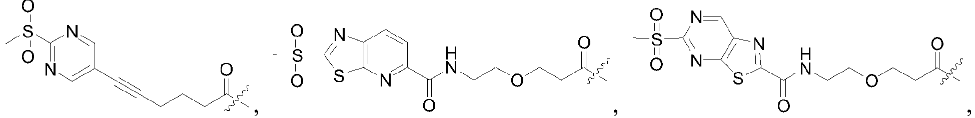

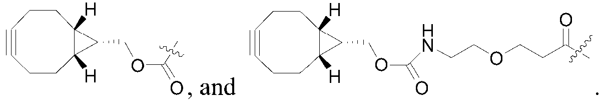

Specifically, the formula (I') is one of the following:

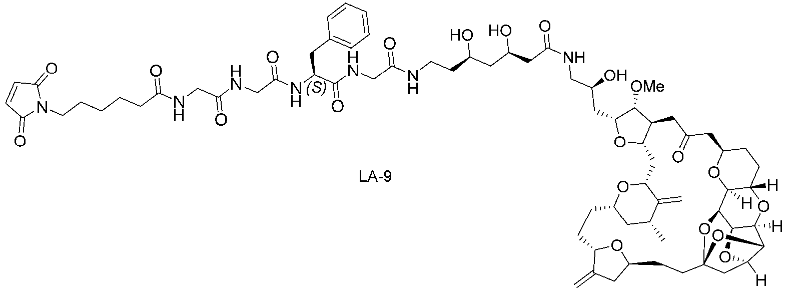

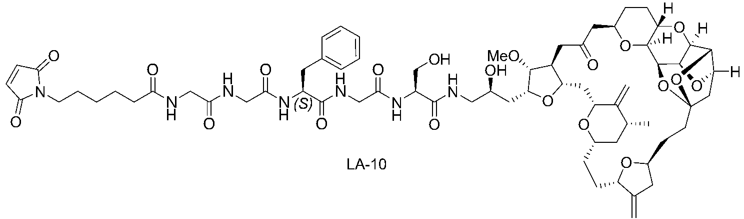

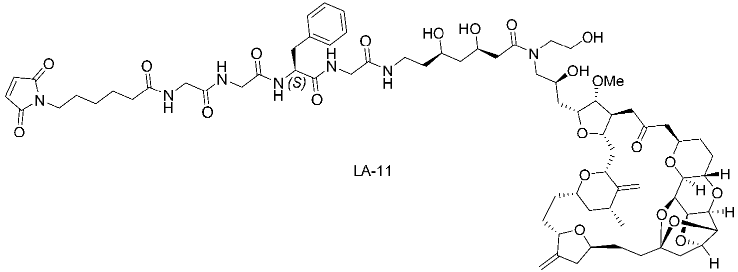

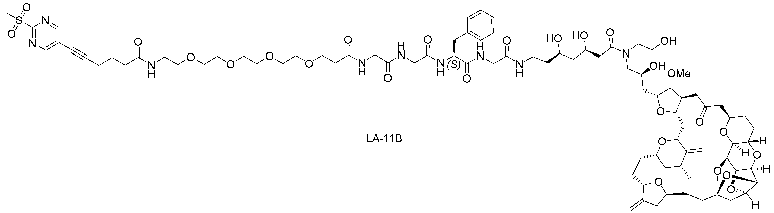

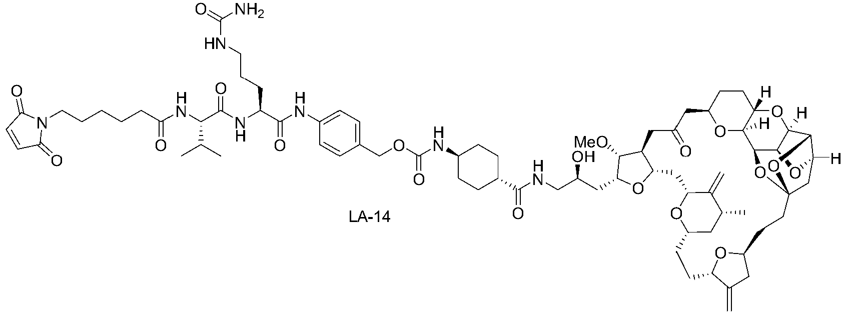

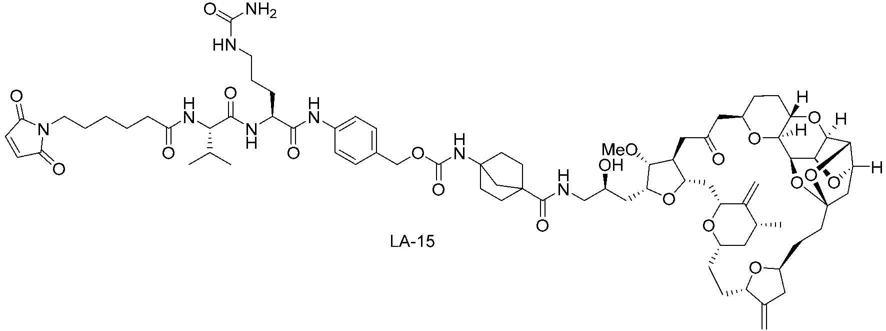

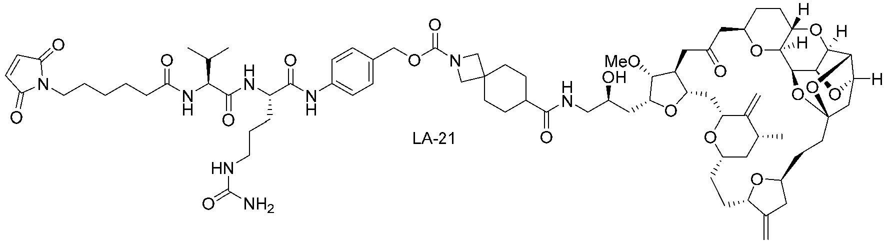

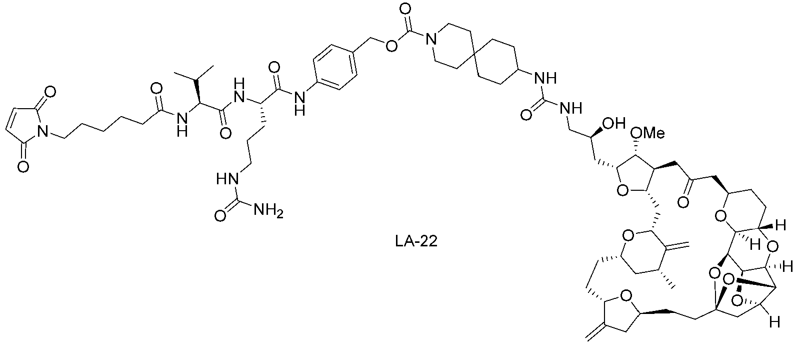

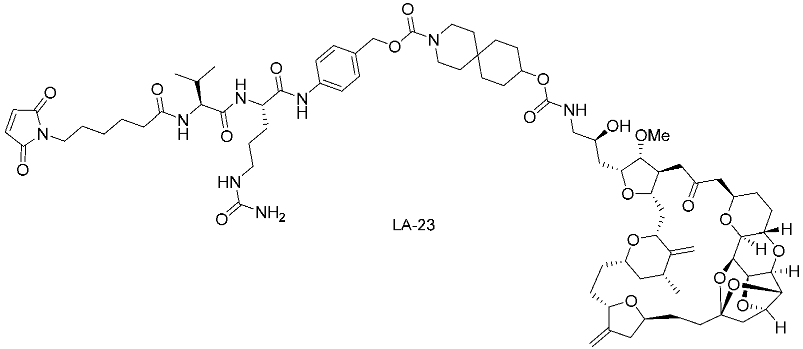

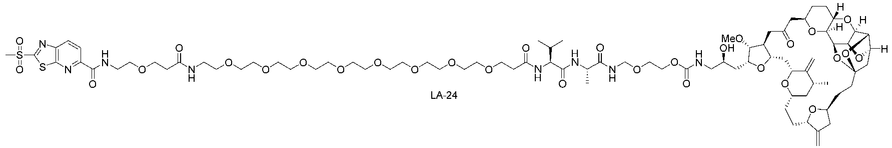

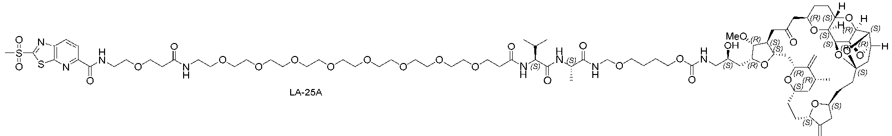

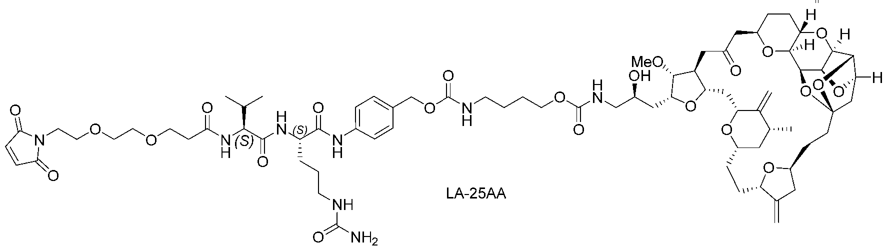

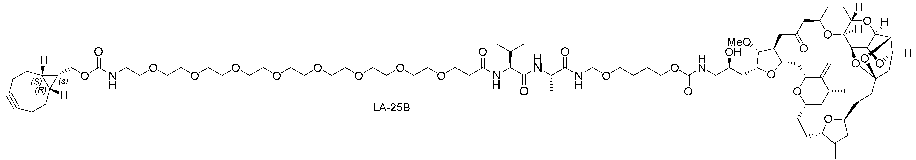

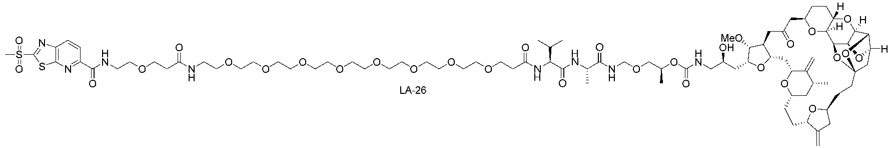

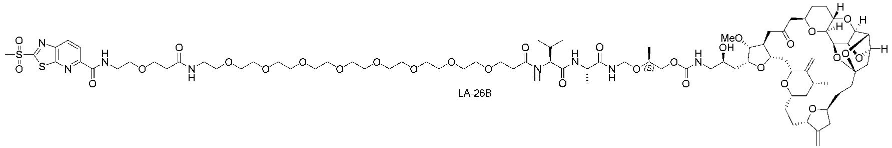

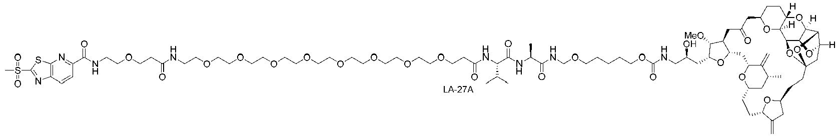

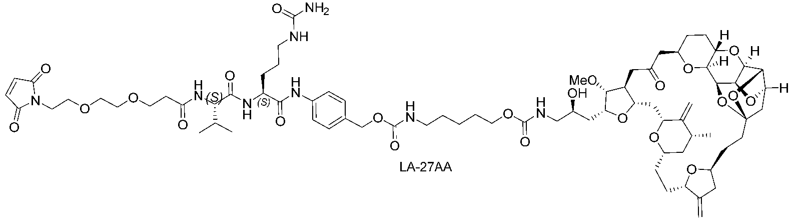

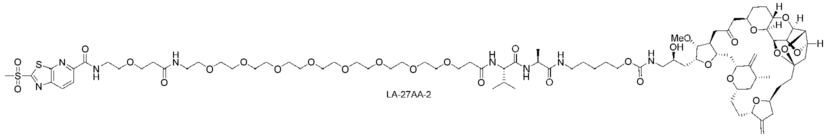

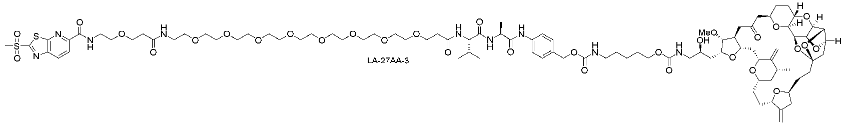

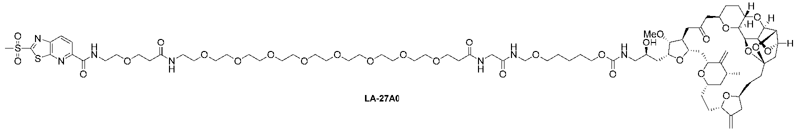

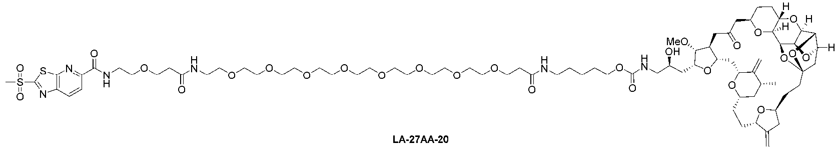

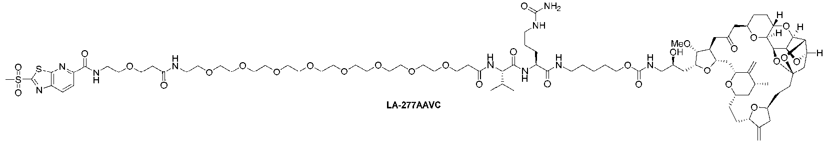

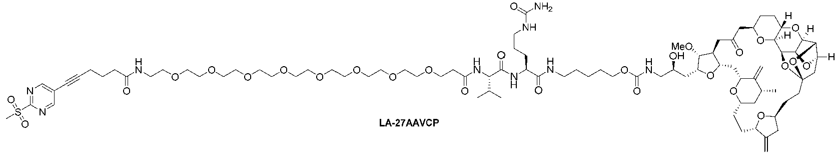

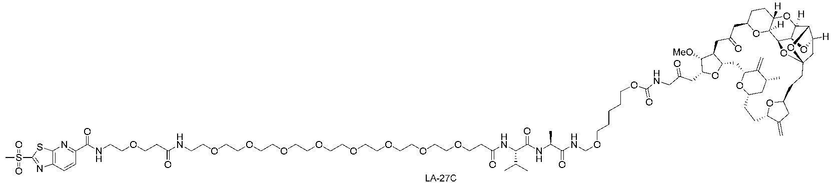

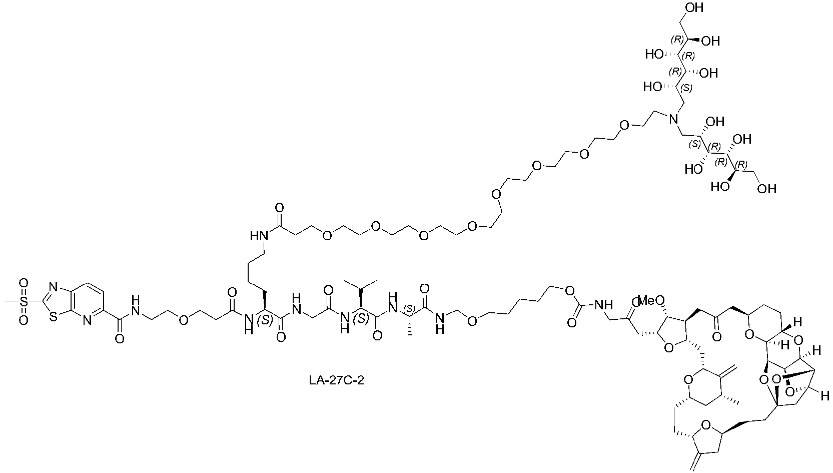

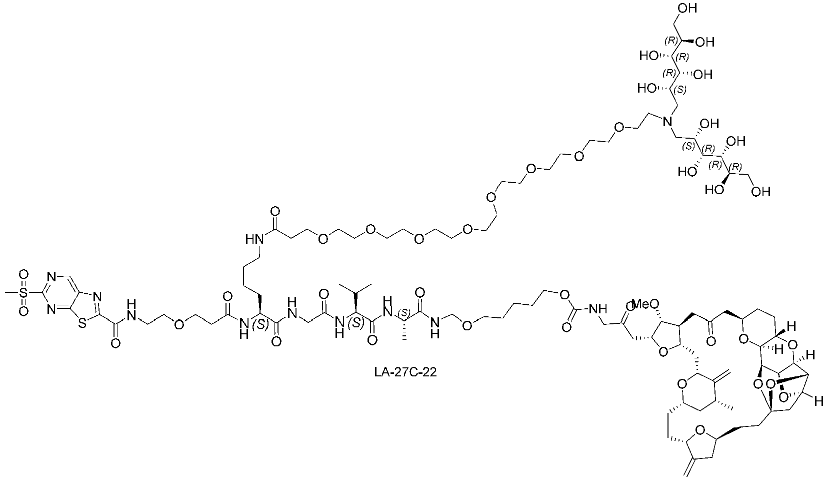

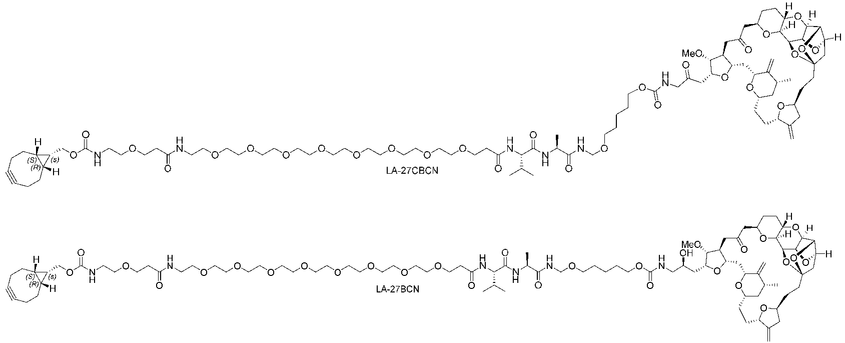

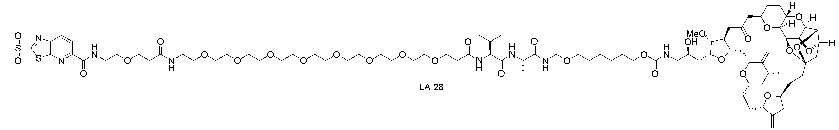

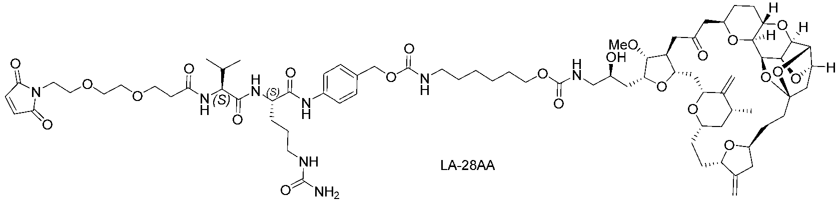

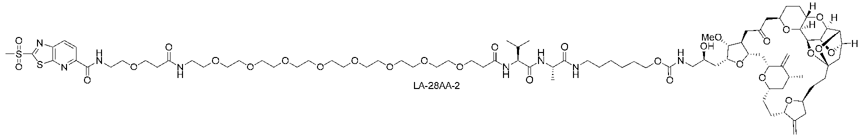

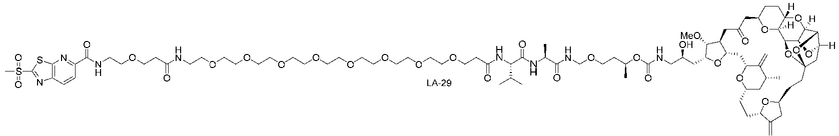

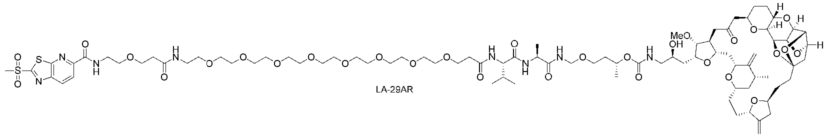

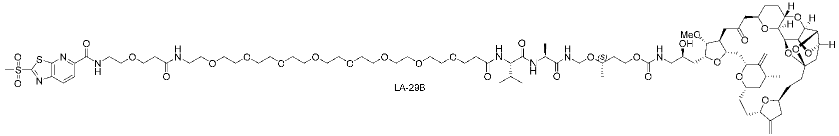

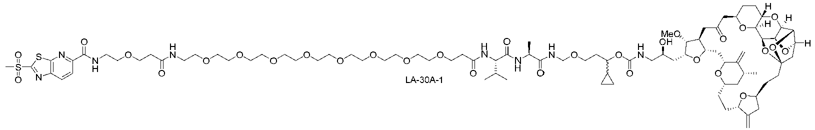

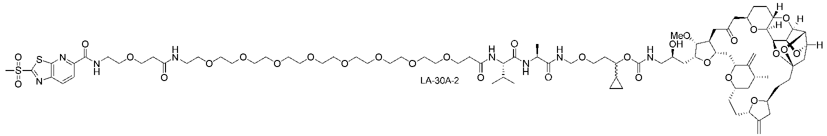

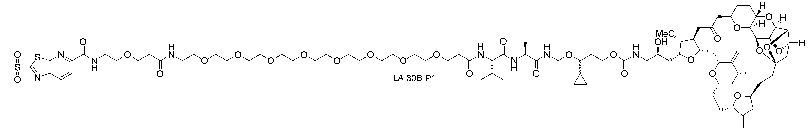

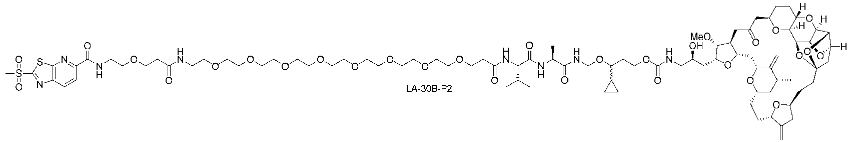

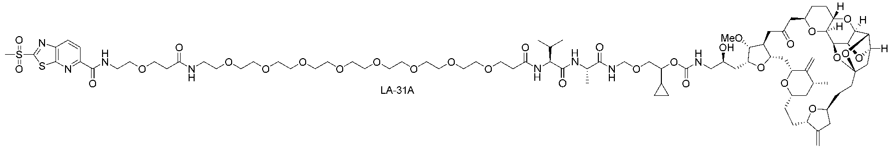

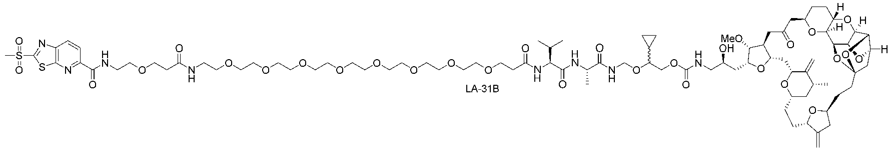

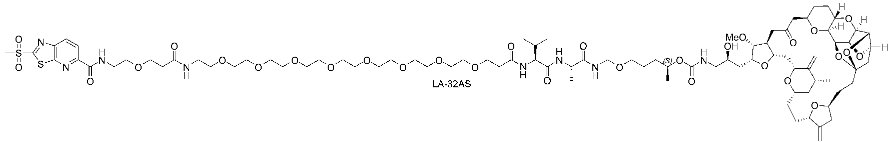

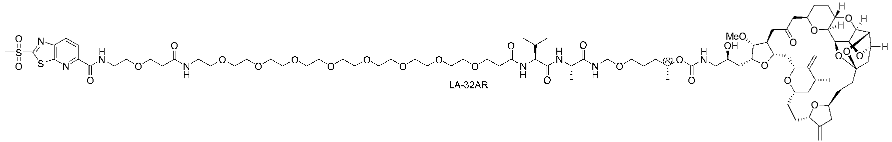

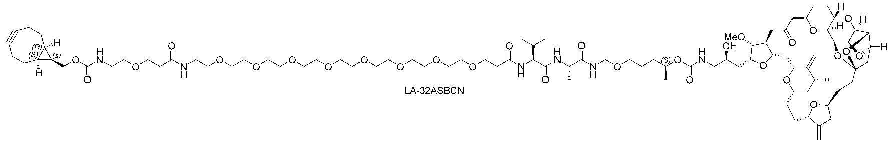

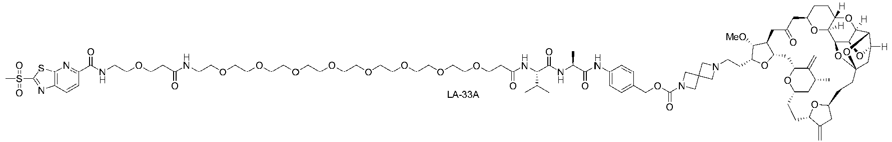

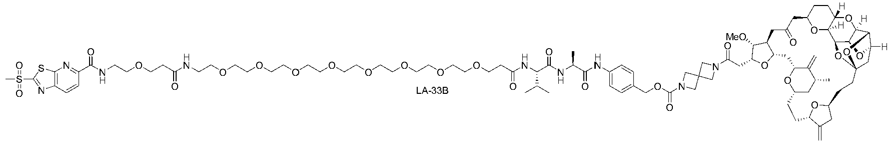

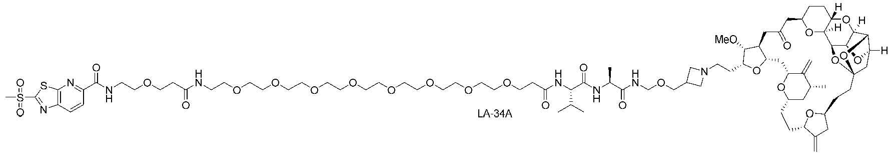

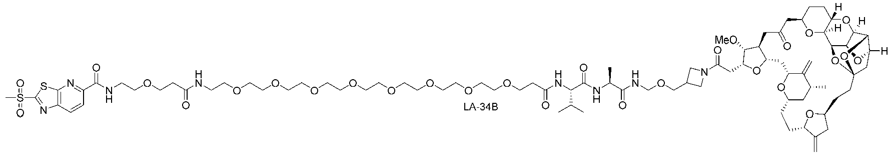

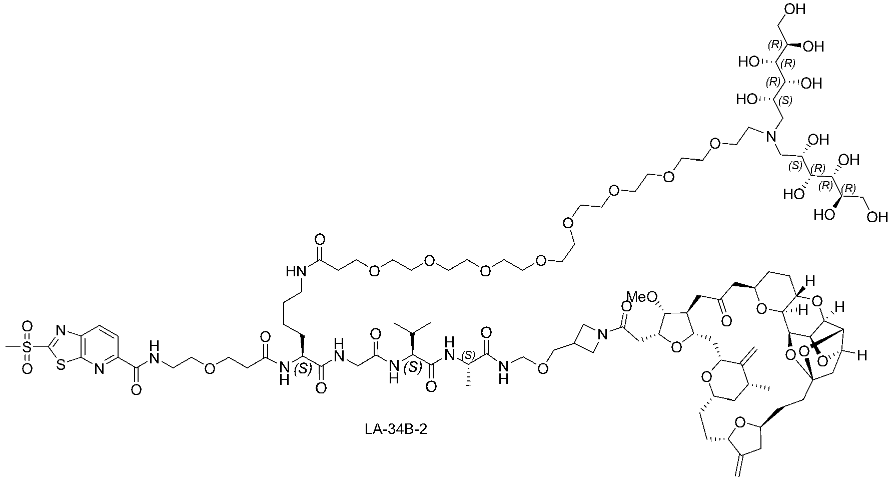

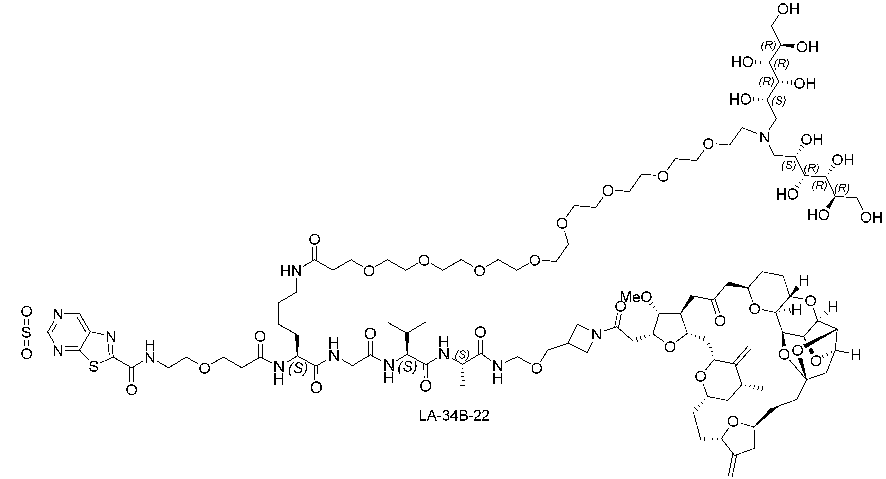

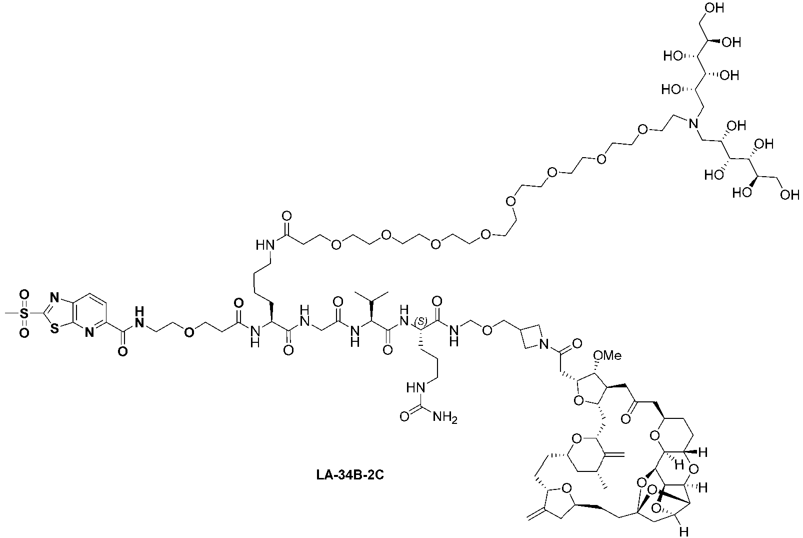

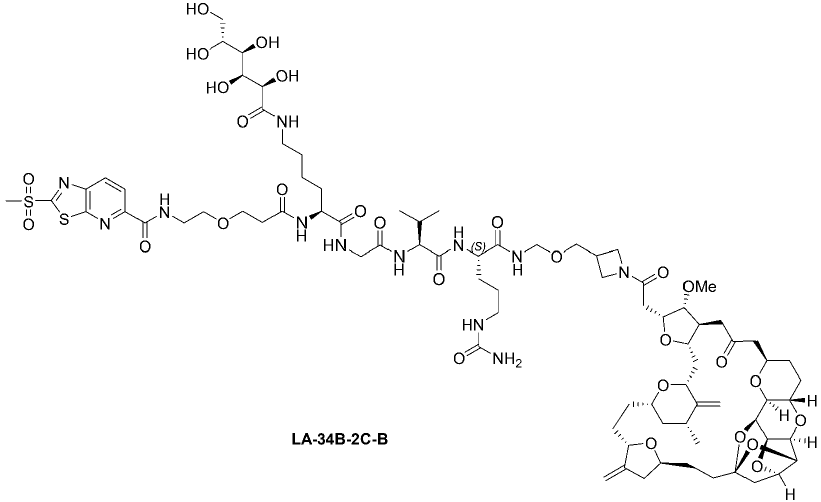

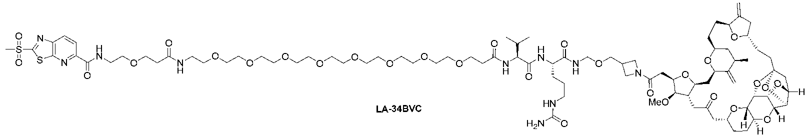

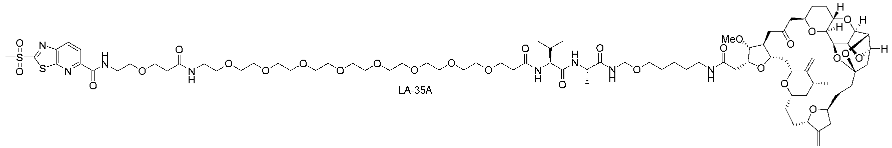

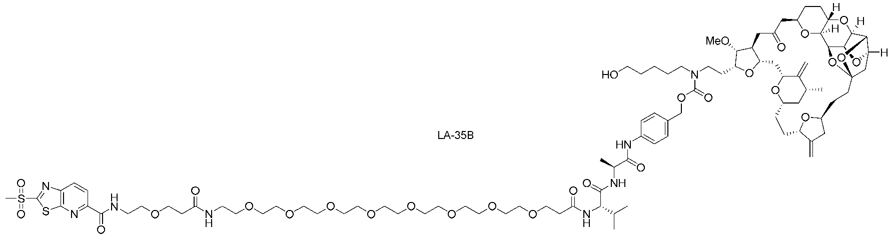

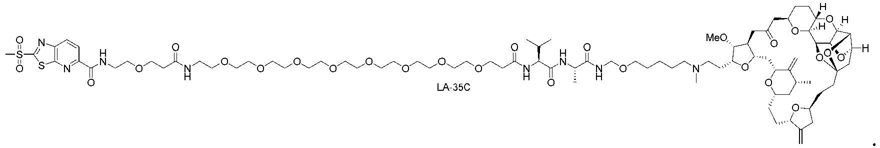

Specifically, the formula (II') is one of the following: 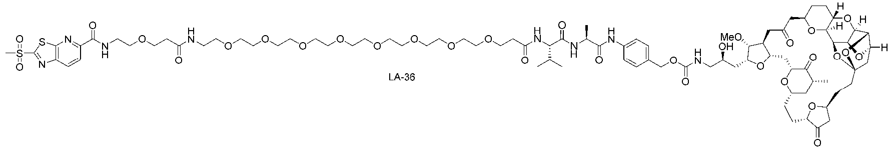 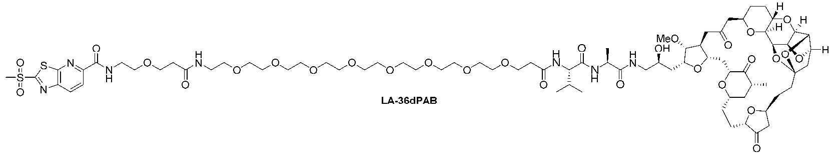 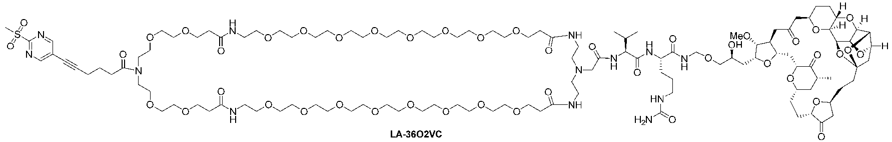 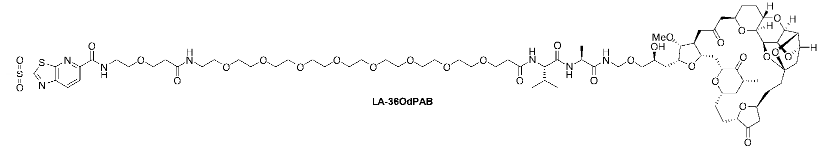 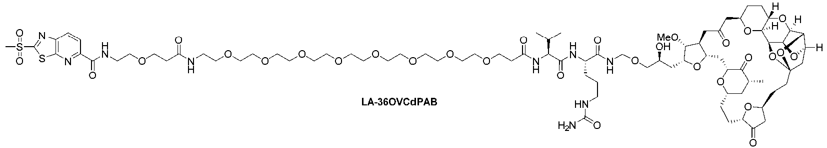 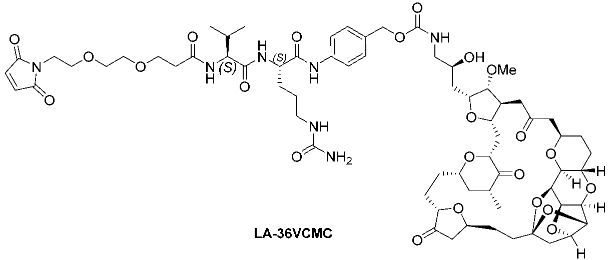 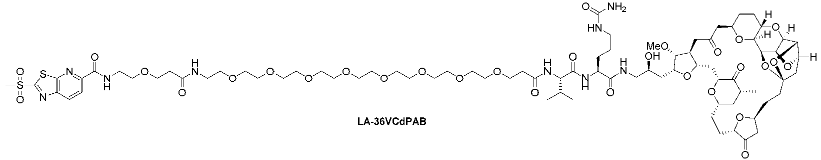 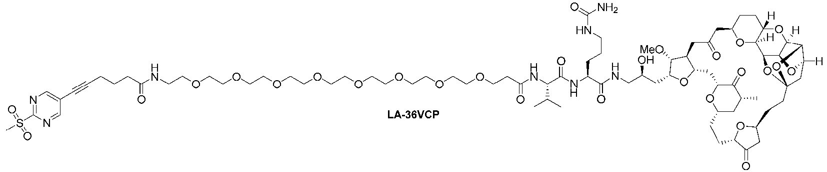 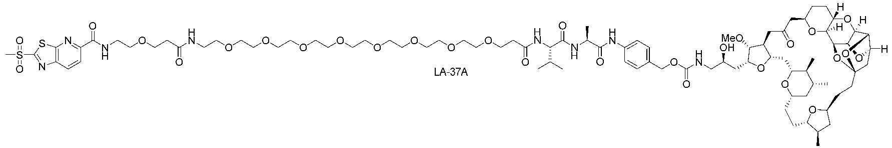 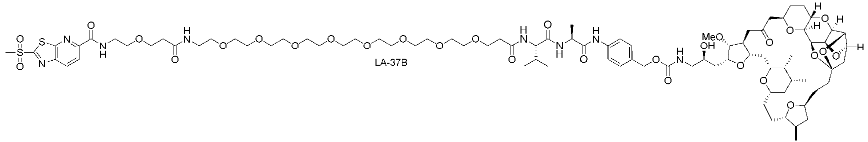 and

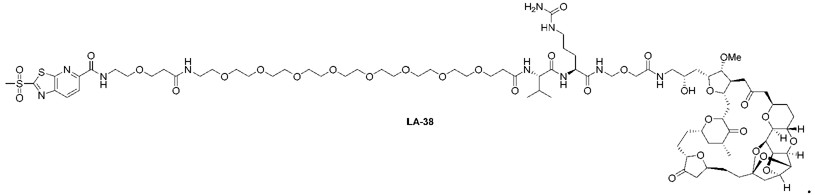

A third aspect of the present application provides an antibody-drug conjugate, which is a compound of formula (I") or (II"), or an isotope-labeled compound, an optical isomer, a geometric isomer, a tautomer, an isomer mixture, or a pharmaceutically acceptable salt thereof; 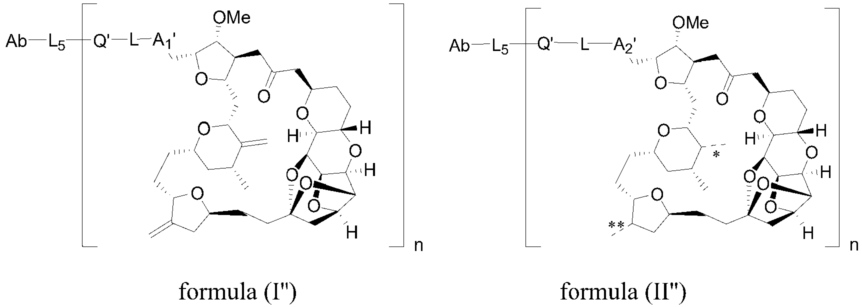 where:
Ab is an antibody or an antigen-binding fragment;
L₅ is a direct bond or a linking group connecting Ab and Q';
Q' is a group formed after Q is attached to Ab via L₅;
n is an integer or a decimal between 0 and 20, for example, 1, 2, 3, 4, 5, 6, 7, 8, 9, 10, 11, 12, 13, 14, 15, 16, 17, 18, 19, 20, or 0 and a decimal between the foregoing integers; and
definitions of Q, L, A₁, A₂, *, and ** are the same as those in the foregoing formula (I') and formula (II').

In a group of embodiments, n is an integer or a decimal between 0 and 8.

In a group of embodiments, L₅ is the direct bond or 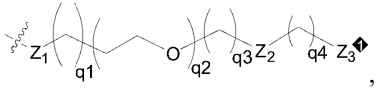 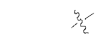 represents a ligation site for ligating Ab, and 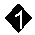 indicates that Z₃ is ligated to Q'; and Z₃ is a click probe or a sulfhydryl group or a precursor thereof that can be ligated to Q' after reaction such as a metal-free click reaction, and preferably, Z₃ is an azide group;
Z₁ and Z₂ are each independently selected from -C(O)NH-, -C(O)O-, -C(O)-, -OC(O)-, -NHC(O)-, -NH-, -O-, -OC(O)NH-, or -NHC(O)O-;
q₁ is 0, 1, 2, 3, 4, 5, 6, 7, or 8;
q₂ is 1, 2, 3, 4, 5, 6, 7, or 8;
q₃ is 1, 2, 3, 4, 5, 6, 7, or 8; and
q₄ is 1, 2, 3, 4, 5, 6, 7, or 8.

Preferably, L₅ is the direct bond or 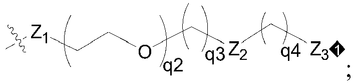 or more preferably, L₅ is the direct bond or

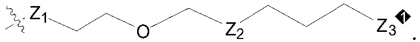

Preferably, Z₁ and Z₂ are each independently -C(O)NH-.

In a group of embodiments, Q' is selected from: 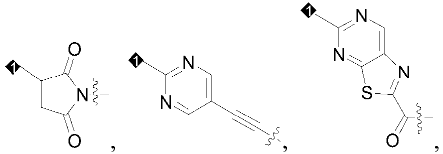 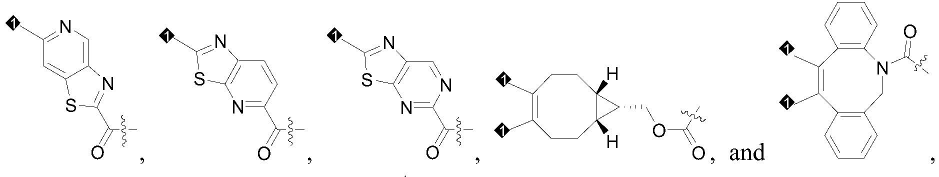 where 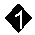 is a ligation site for ligating L₅, and 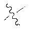 represents a ligation site for ligating L₄.

In a group of embodiments, L₅-Q' is selected from 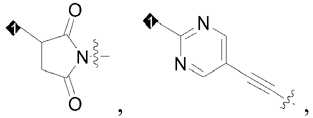 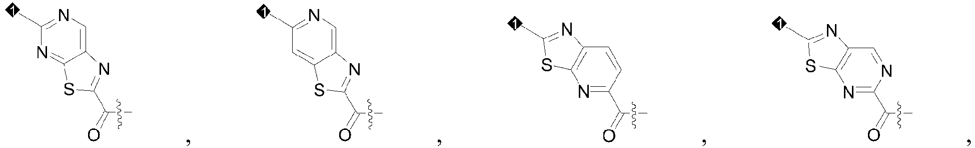 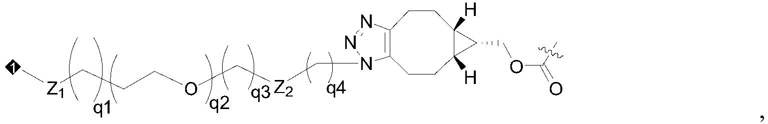 and 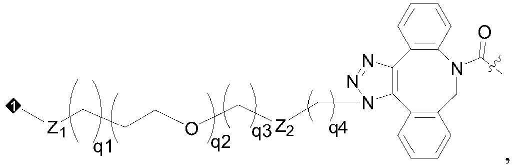 where 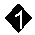 is a ligation site for ligating Ab, and 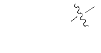 represents a ligation site for ligating L₄.

Preferably, L₅-Q' is selected from 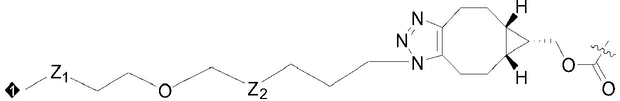 and 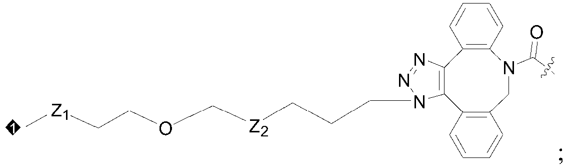 or more preferably, Z₁ and Z₂ are each independently -C(O)NH-.

In a group of embodiments, Ab is selected from a mouse-derived antibody, a chimeric antibody, a humanized antibody, a fully human antibody, or an antigen-binding fragment thereof; and preferably, Ab is selected from anti-HER2 (ErbB2) antibody, anti-EGFR antibody, anti-B7-H3 antibody, anti-c-Met antibody, anti-HER3 (ErbB3) antibody, anti-HER4 (ErbB4) antibody, anti-ROR1 antibody, anti-CLDN6 antibody, anti-CLDN9 antibody, anti-CLDN18.2 antibody, anti-NaPi-2b antibody, anti-TNF-α antibody, anti-ENPP3 antibody, anti-DLL3 antibody, anti-CD20 antibody, anti-CD22 antibody, anti-CD28 antibody, anti-CD30 antibody, anti-CD33 antibody, anti-CD37 antibody, anti-CD38 antibody, anti-CD44 antibody, anti-CD45 antibody, anti-CD47 antibody, anti-CD48 antibody, anti-CD56 antibody, anti-CD70 antibody, anti-CD73 antibody, anti-CD98 antibody, anti-CD105 antibody, anti-CEA antibody, anti-EphA2 antibody, anti-MUCI antibody, anti-Lewis Y antibody, anti-VEGFR antibody, anti-GPNMB antibody, anti-Integrin antibody, anti-PSMA antibody, anti-Tenascin-C antibody, anti-SLC44A4 antibody, anti-CD79 antibody, anti-TROP-2 antibody, anti-CD79B antibody, anti-Mesothelin antibody, anti-Nectin-4 antibody, anti-TPBG antibody, or an antigen-binding fragment thereof.

Preferably, Ab is the anti-HER2 (ErbB2) antibody or the antigen-binding fragment thereof.

Preferably, Ab is selected from the monoclonal antibody or the antigen-binding fragment thereof, or preferably, selected from Trastuzumab, Cetuximab, Pertuzumab, Nimotuzumab, Enoblituzumab, Emibetuzumab, Inotuzumab, Pinatuzumab Vedotin, Brentuximab Vedotin, Gemtuzumab, Bivatuzumab, Lorvotuzumab, or an antigen-binding fragment thereof, or more preferably, Ab is selected from Trastuzumab or an antigen-binding fragment thereof.

In a group of embodiments, the formula (I") is one of the following: 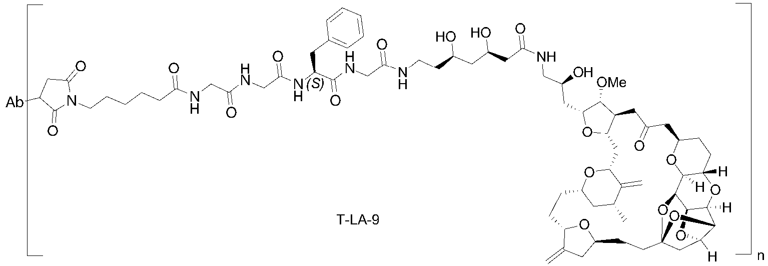 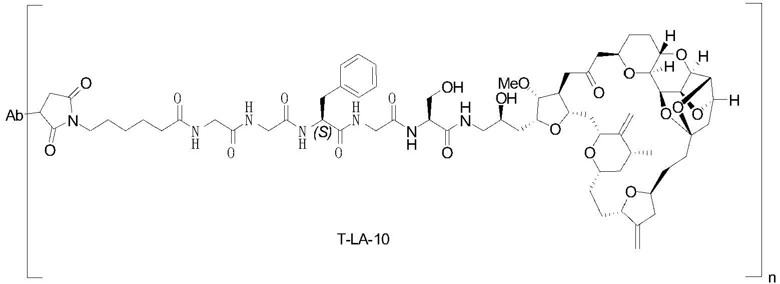 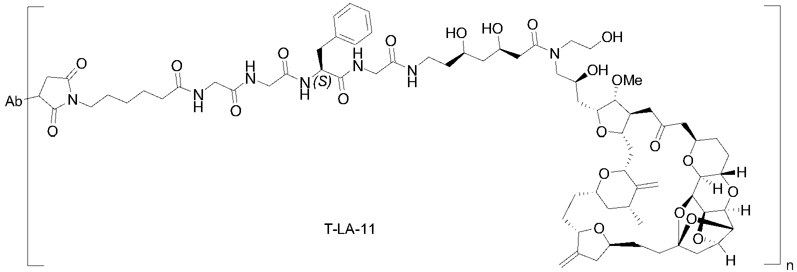 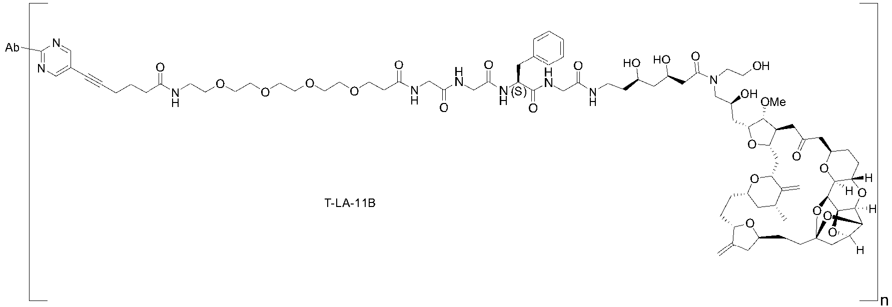 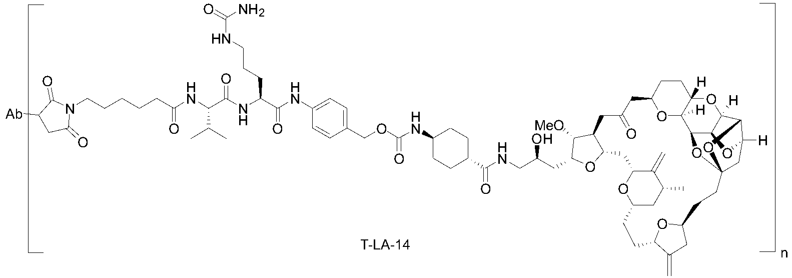 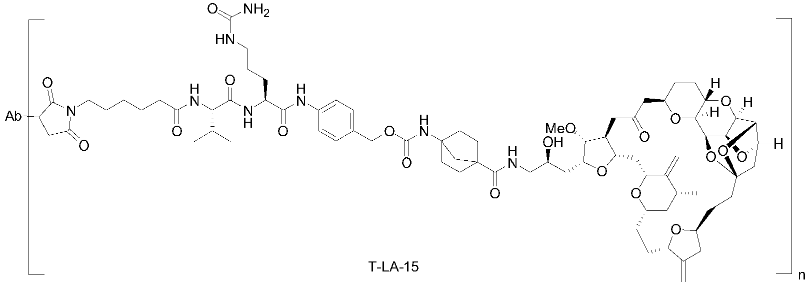 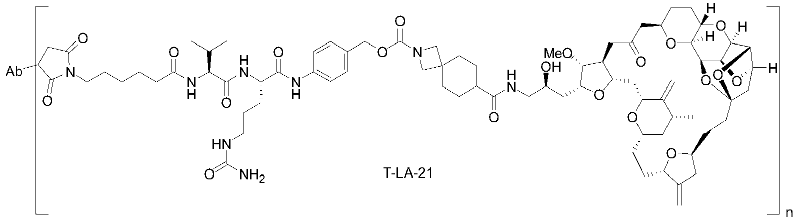 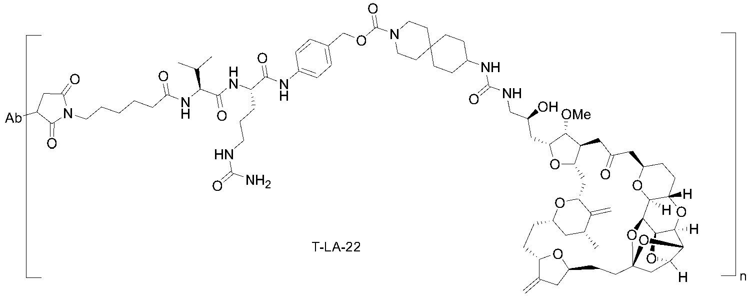 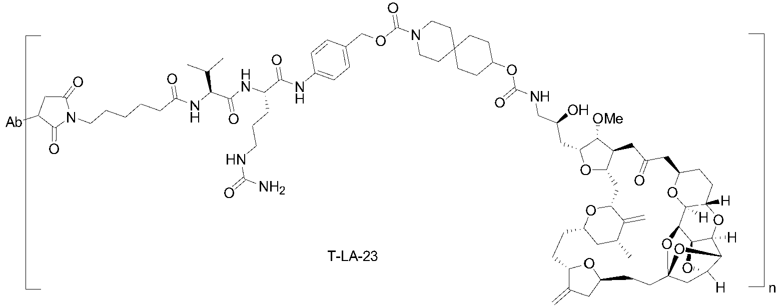 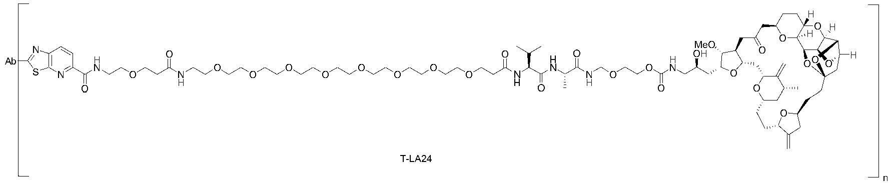 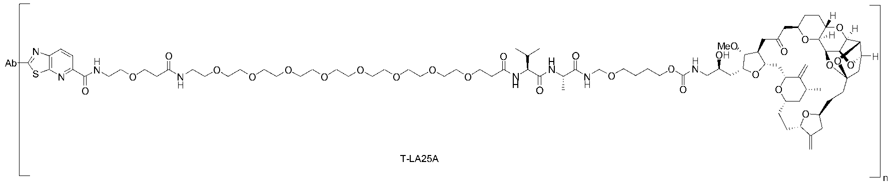 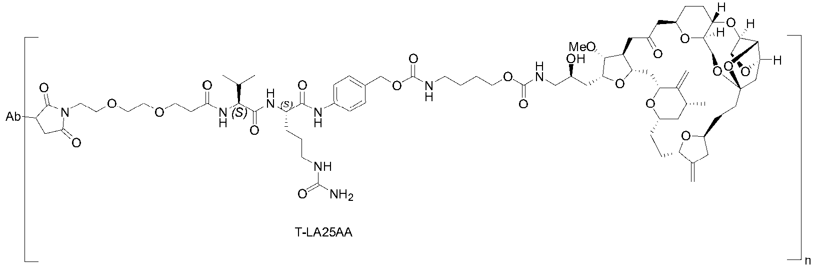 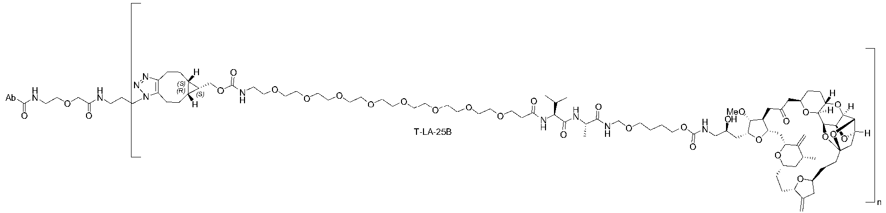 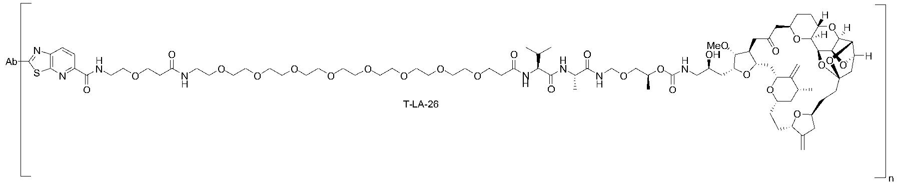 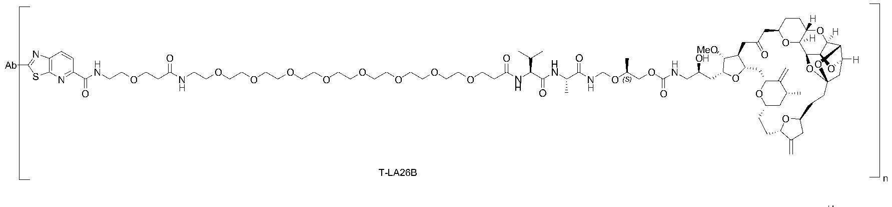 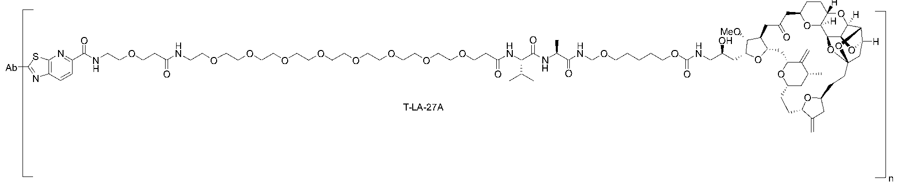 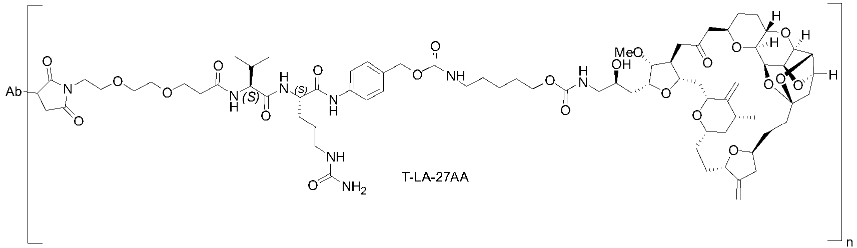 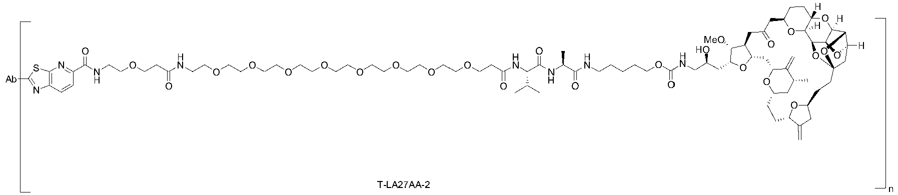 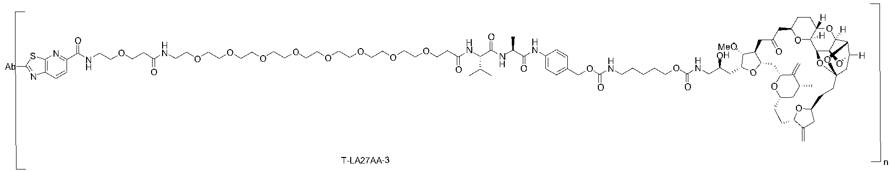 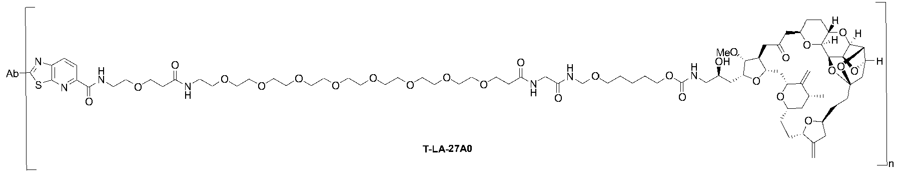 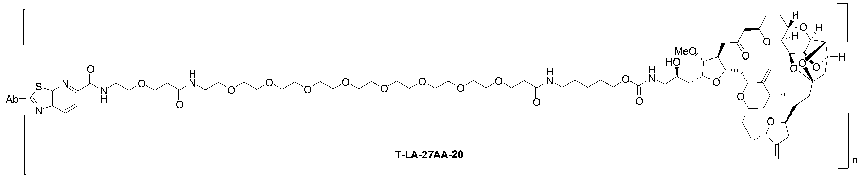 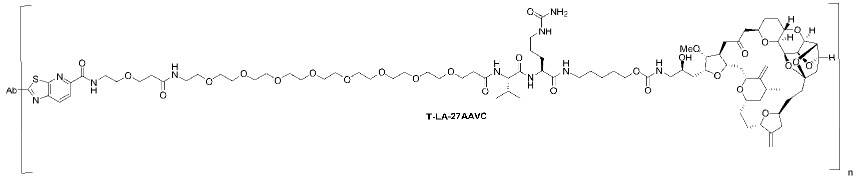 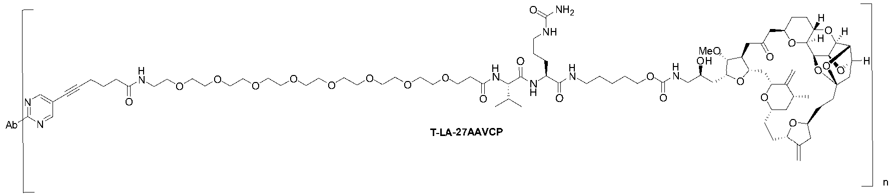 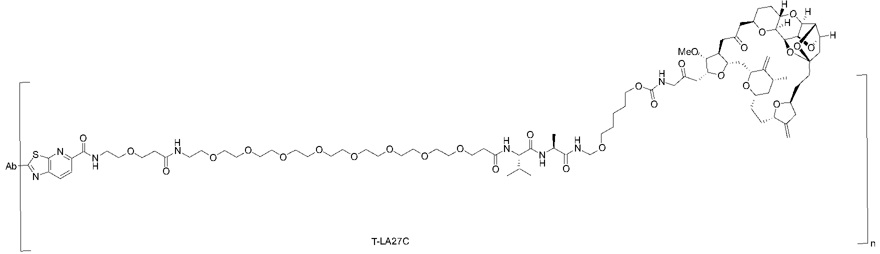 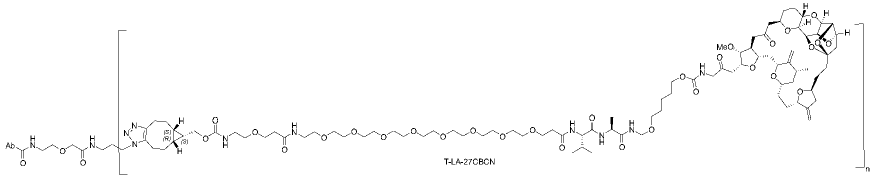 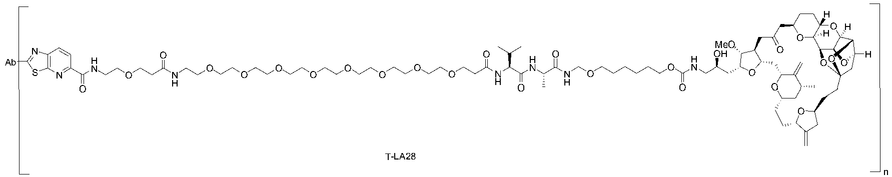 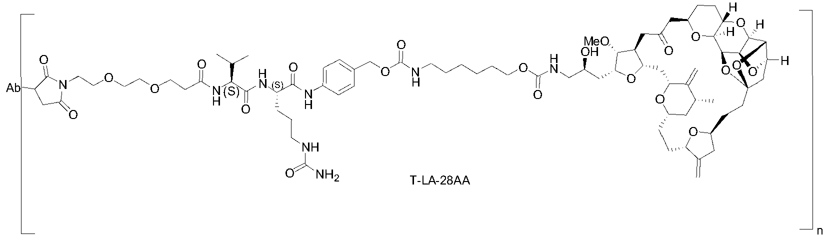 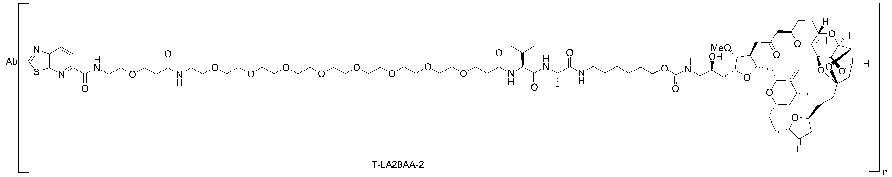 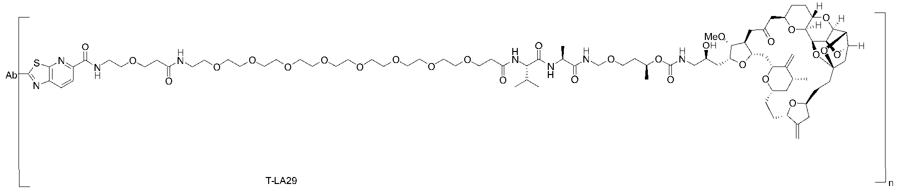 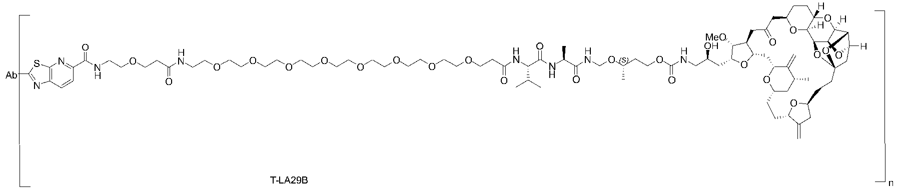 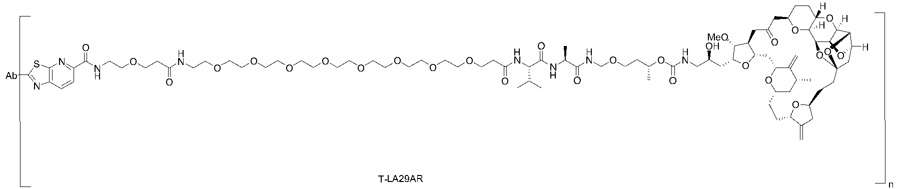 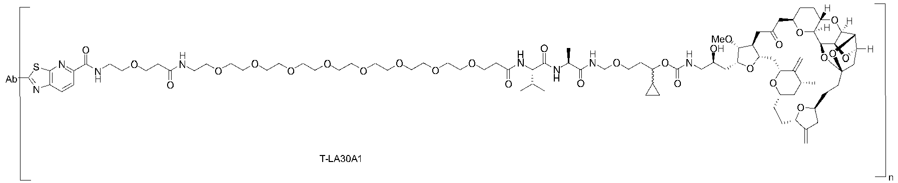 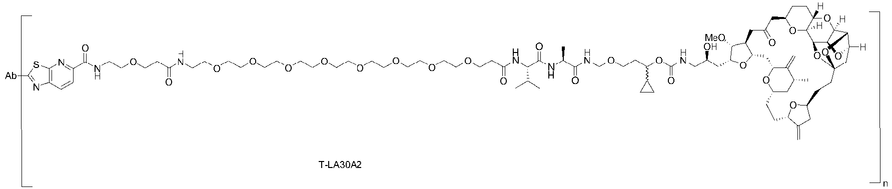 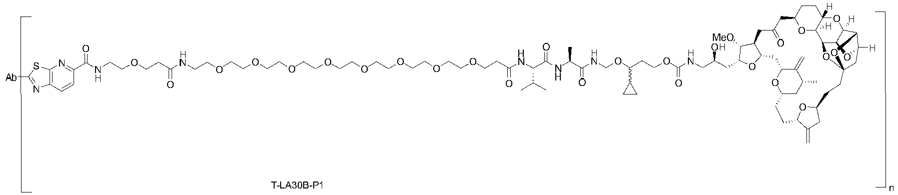 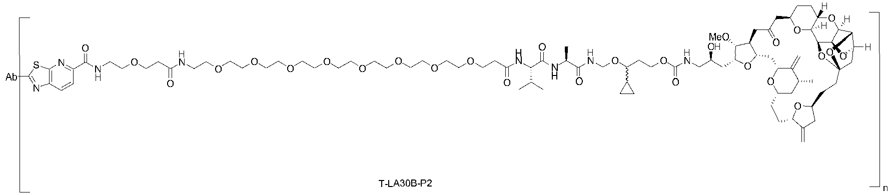 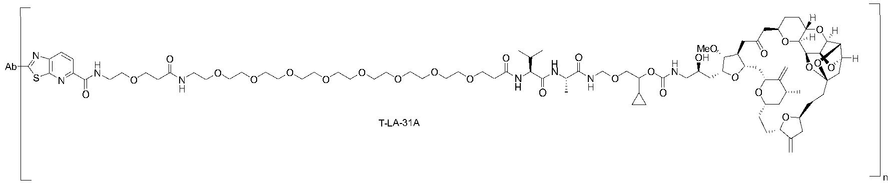 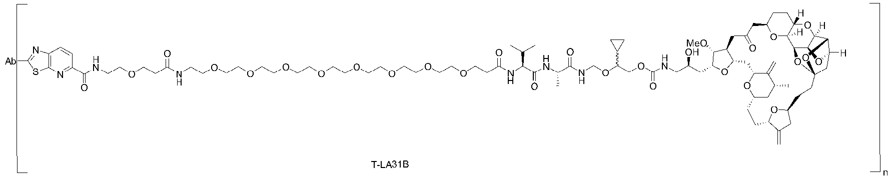 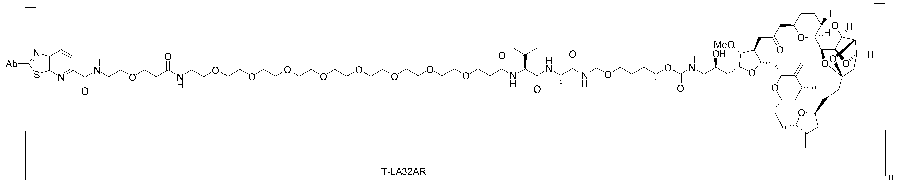 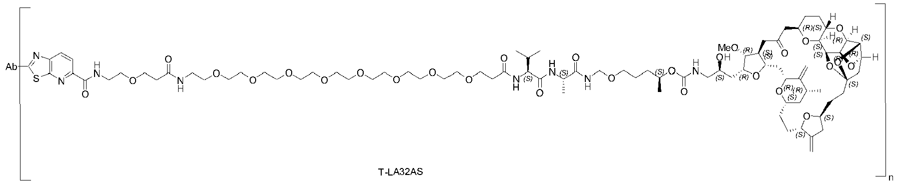 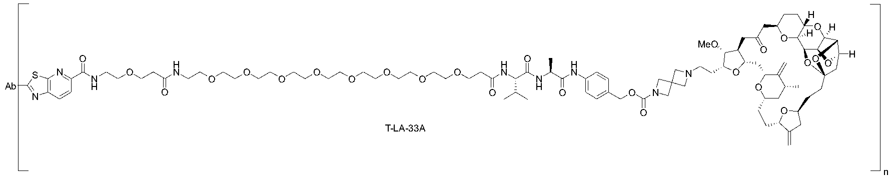 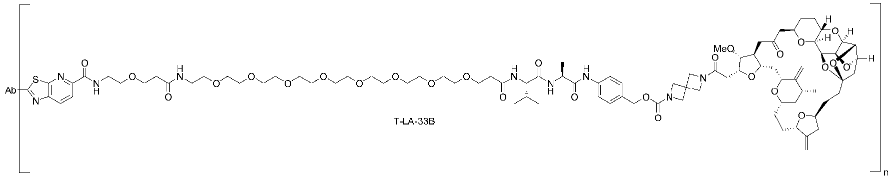 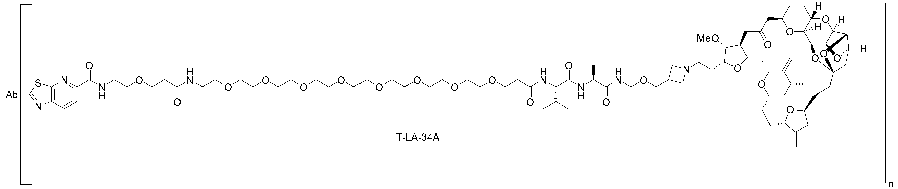 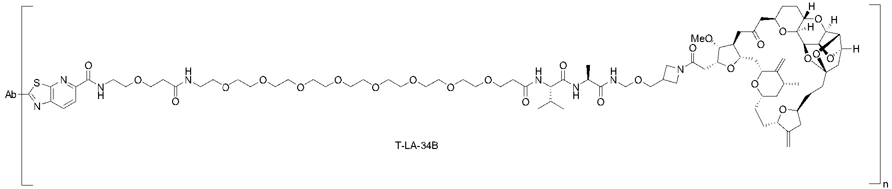 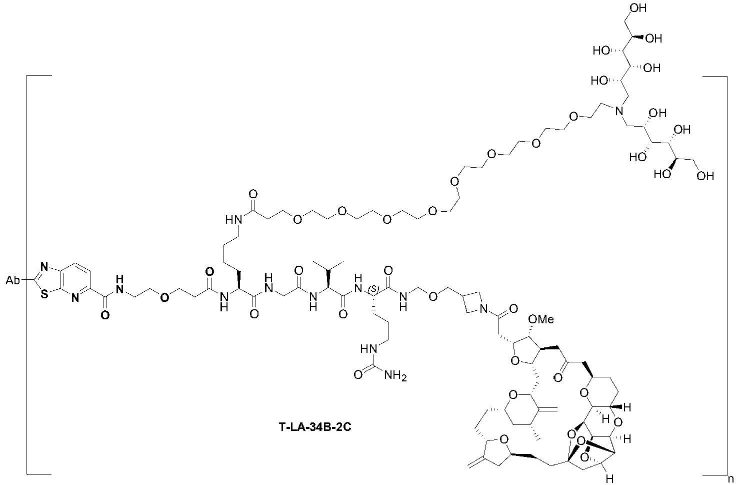 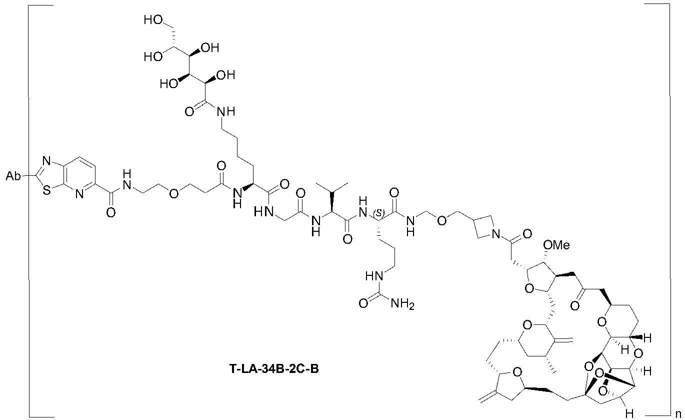 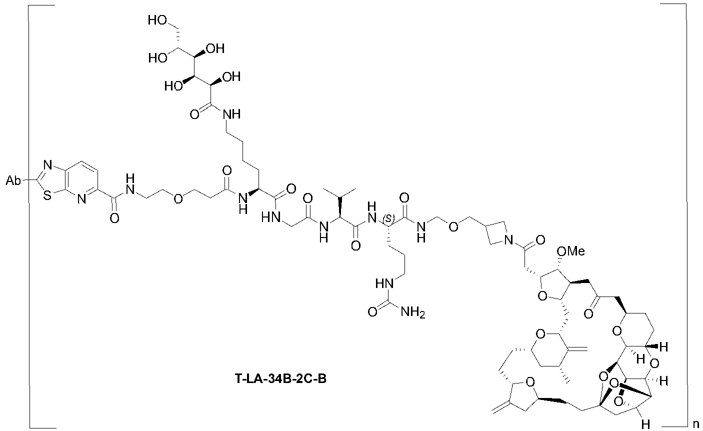 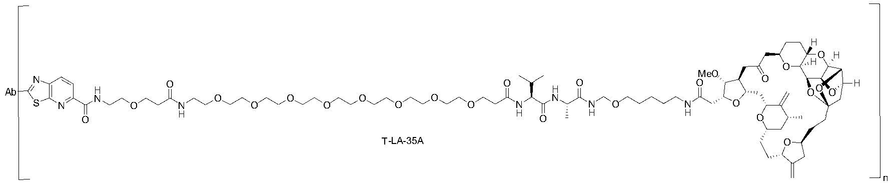 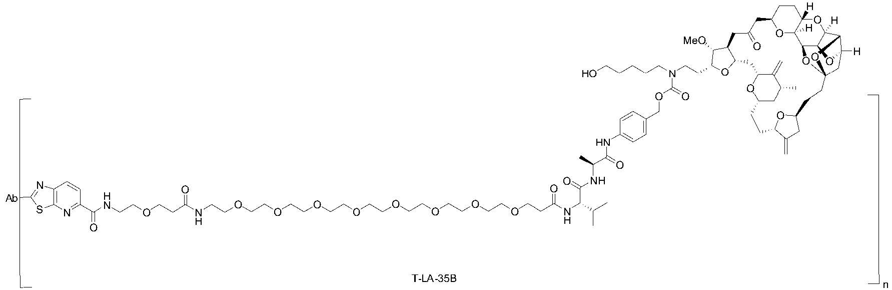 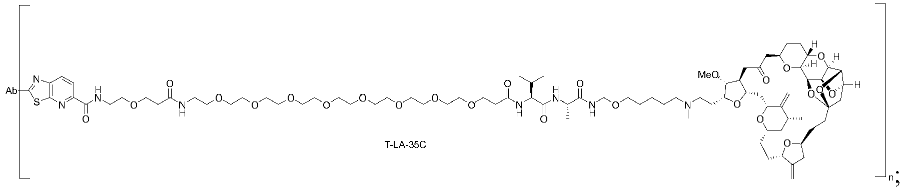 preferably, Ab is the anti-HER2 (ErbB2) antibody or the antigen-binding fragment thereof, and more preferably, Ab is Trastuzumab or an antigen-binding fragment thereof.

In a group of embodiments, the formula (II") is one of the following: 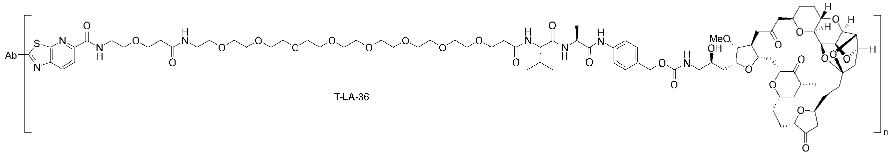 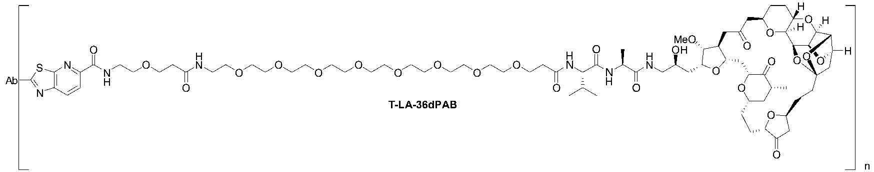 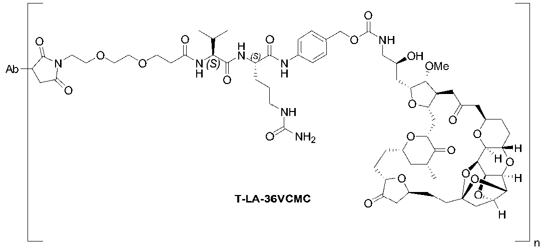 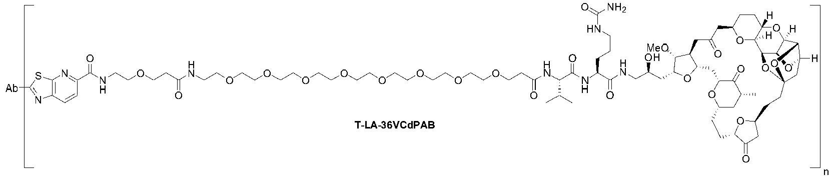 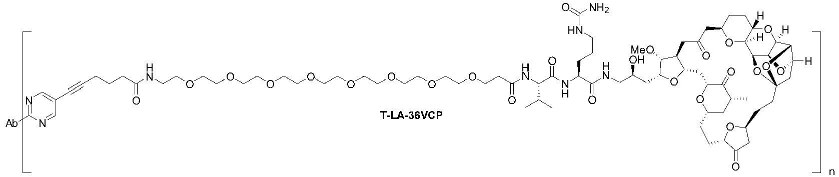 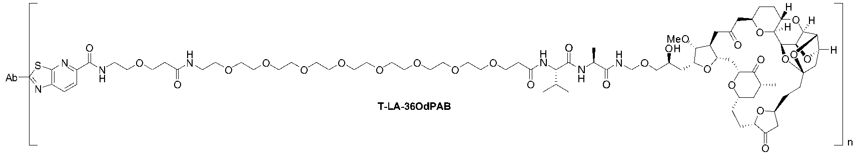 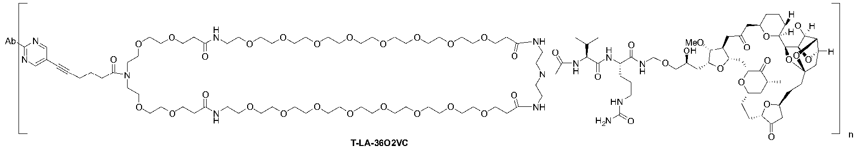 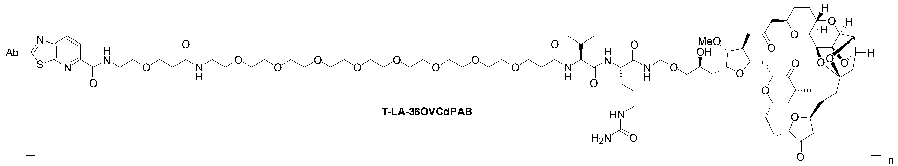 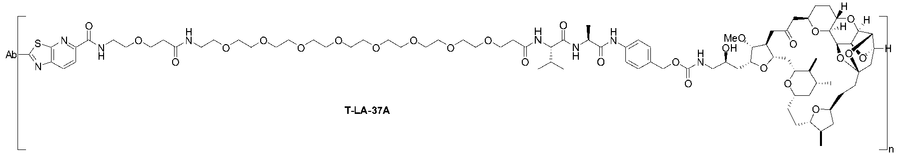 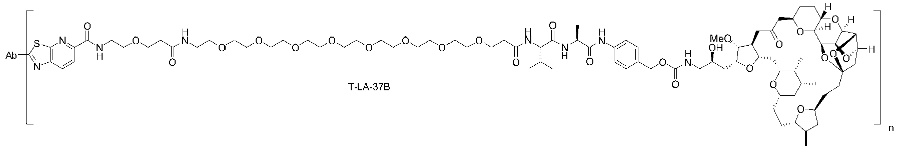 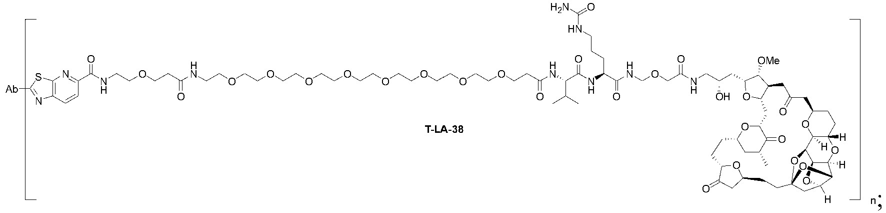 preferably, Ab is the anti-HER2 (ErbB2) antibody or the antigen-binding fragment thereof, and more preferably, Ab is Trastuzumab or an antigen-binding fragment thereof.

A fourth aspect of the present application provides an eribulin derivative, where the eribulin derivative is a compound shown below, or an isotope-labeled compound, an optical isomer, a geometric isomer, a tautomer, an isomer mixture, or a pharmaceutically acceptable salt thereof;

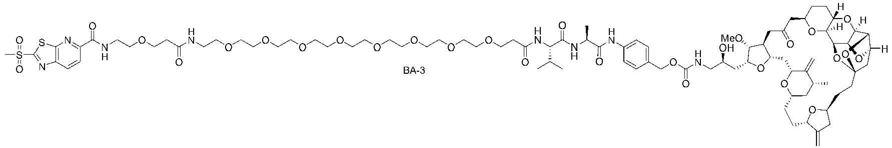

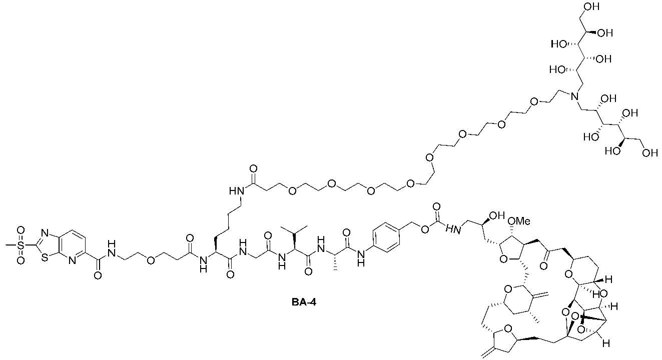

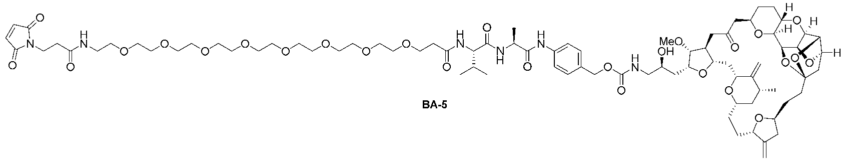

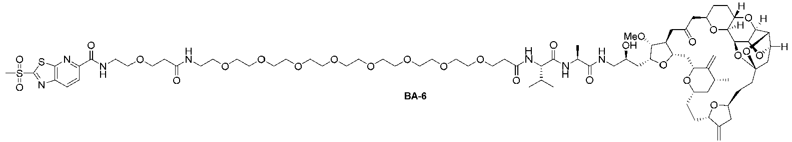

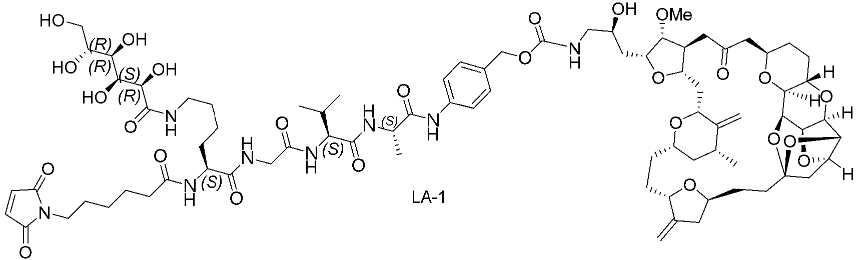

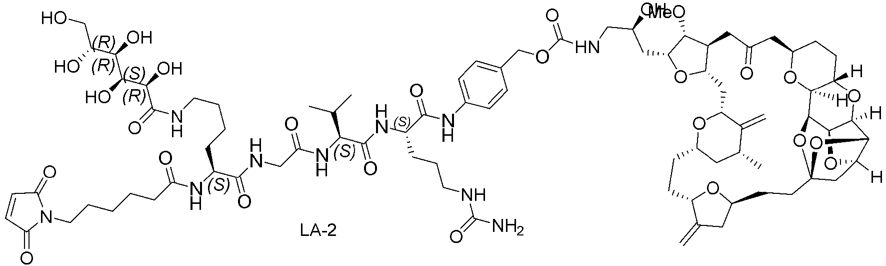

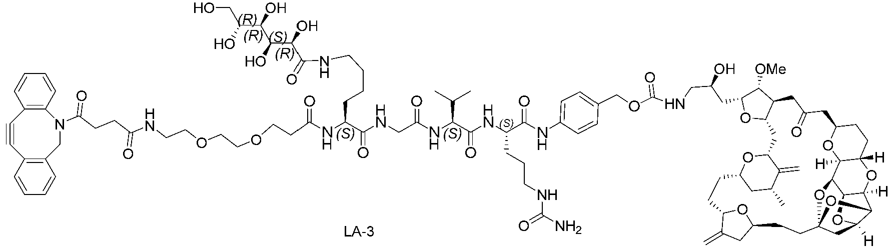

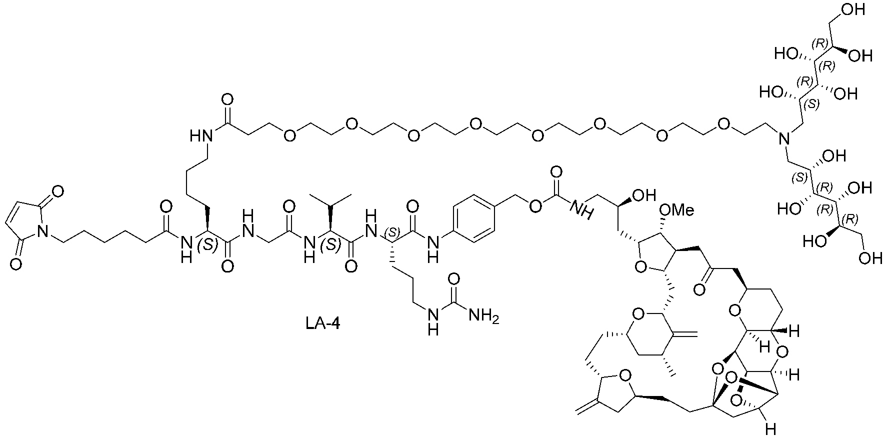

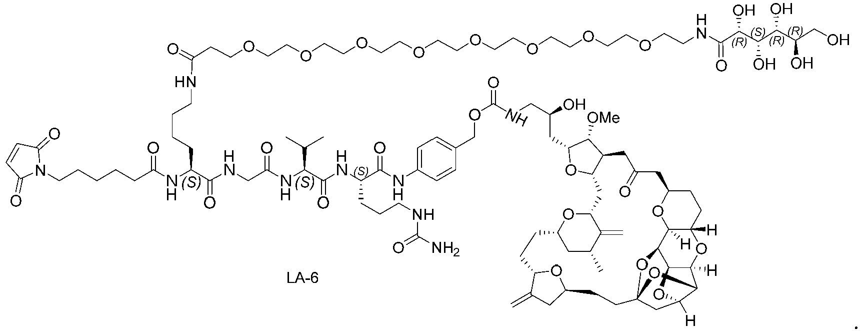

A fifth aspect of the present application provides an antibody-drug conjugate, where the antibody-drug conjugate is a compound shown below, or an isotope-labeled compound, an optical isomer, a geometric isomer, a tautomer, an isomer mixture, or a pharmaceutically acceptable salt thereof; 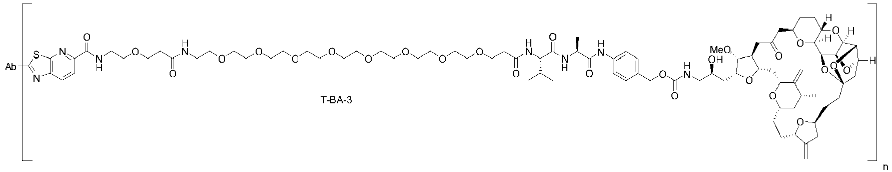 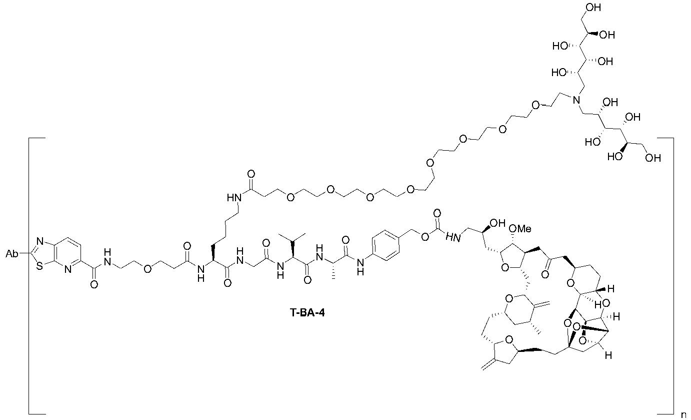 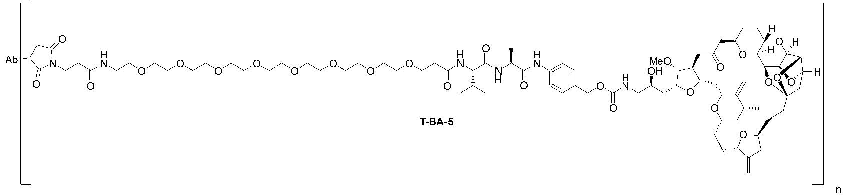 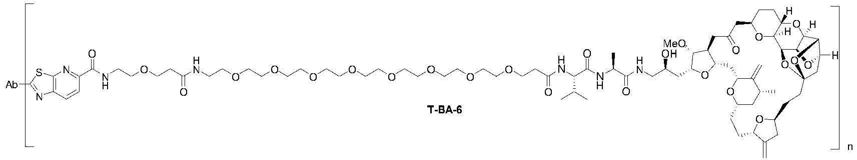 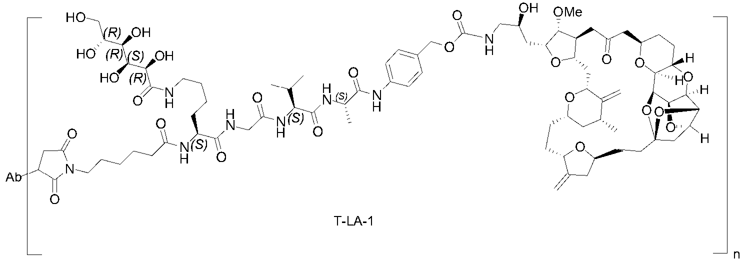 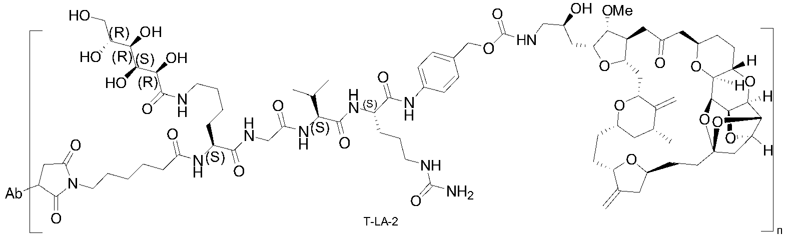 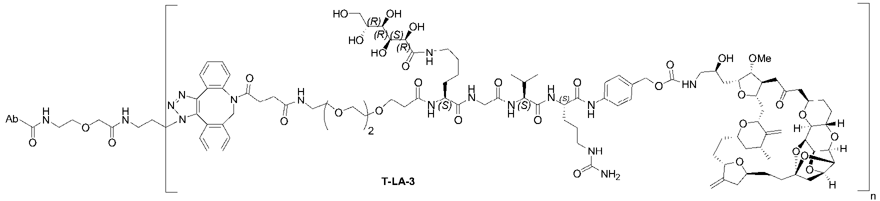 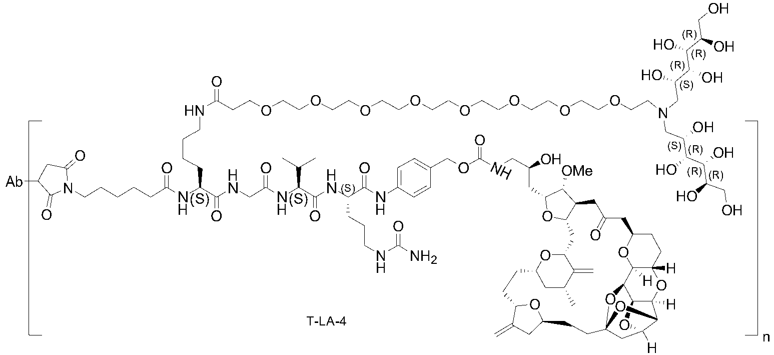 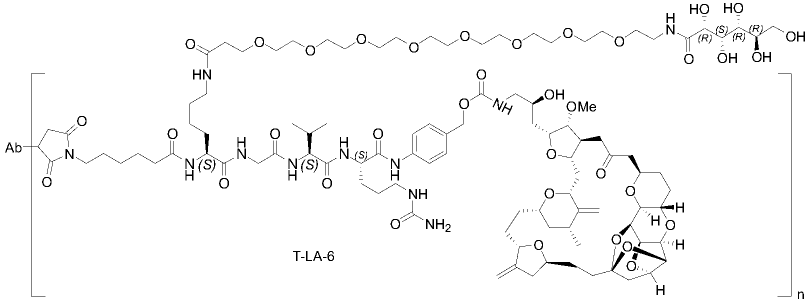 definitions of Ab and n are the same as those in the foregoing formula (I") and formula (II"). Preferably, Ab is the anti-HER2 (ErbB2) antibody or the antigen-binding fragment thereof, and more preferably, Ab is Trastuzumab or an antigen-binding fragment thereof.

A sixth aspect of the present application provides a pharmaceutical composition, including the foregoing eribulin derivative, antibody-drug conjugate, and one or more pharmaceutically acceptable carriers or excipients according to the present application.

A seventh aspect of the present application provides use of the foregoing eribulin derivative according to the present application in preparation of an antibody-drug conjugate.

An eighth aspect of the present application provides use of the foregoing eribulin derivative, antibody-drug conjugate, and pharmaceutical composition according to the present application in preparation of a medicament for treating tumors or cancers.

A ninth aspect of the present application provides a method for treating a tumor or cancer, including administering an effective dose of the foregoing eribulin derivative, antibody-drug conjugate, and pharmaceutical composition according to the present application to a subject in need thereof.

In a group of embodiments, the tumor or cancer is selected from a solid tumor or hematological tumor; preferably, the tumor or cancer is selected from a metastatic and recurrent tumor or cancer; and preferably, the tumor or cancer is selected from breast cancer, gastric cancer, colon cancer, pancreatic cancer, kidney cancer, ovarian cancer, lung cancer, soft tissue sarcoma, liposarcoma, non-small cell lung cancer, melanoma, head and neck cancer, cervical cancer, and prostate cancer, and may be specifically HER-2-positive breast cancer, triple-negative breast cancer, HER-2-positive triple-negative breast cancer, clear cell renal cell carcinoma, small cell lung cancer, non-small cell lung cancer, and large cell lung cancer.

In a group of embodiments, the tumor or cancer is selected from a cancer or tumor with high expression of HER2 (ErbB2) antibody.

In the present application, a series of derivatives with good tumor suppression effects are synthesized by modifying eribulin, and have lower toxicity and a larger therapeutic window than that of eribulin; and then the eribulin derivative and an antibody are made into an ADC by means of an appropriate linker, and the therapeutic efficiency of the eribulin derivative may be further improved using the targeting specificity of the antibody.

### BRIEF DESCRIPTION OF DRAWINGS

FIG. 1 is a tumor volume change curve 1 in a JIMT-1 breast cancer xenograft mouse model.
FIG. 2 is a weight change curve of rats after administration.
FIG. 3 is a tumor volume change curve 2 in a JIMT-1 breast cancer xenograft mouse model.

### DETAILED DESCRIPTION

Unless otherwise specified, all terms (including technical and scientific terms) used to disclose the present application have the same meanings as commonly understood by a person of ordinary skills in the art to which the present application belongs. The following definitions are used to better understand instructions in the present application. Herein, interpretation of the terms used in the present application is only intended to describe specific examples rather than limit the present application.

Features illustrated or described as a part of a groups of embodiments in the present application can be used in another embodiment to create still another embodiment.

The term "and/or" as used herein refers to a selection range that includes any one of the listed items among two or more related items, as well as any and all combinations of those listed items. These combinations include any two of the related items, any greater number of the related items, or all of the related items. In the present application, it should be understood that when at least three items are connected by "and/or", the technical solutions undoubtedly include technical solutions connected by logical "AND", and also undoubtedly include technical solutions connected by logical "OR". For example, "A and/or B" includes three parallel solutions: A, B, and both A and B. For another example, technical solutions of "A, B, C and/or D" include any one of A, B, C or D (namely, a technical solution connected by logical "OR"), and also include a combination of any one and all of A, B, C and D, that is, a combination of any two or three of A, B, C and D and a combination of four of A, B, C and D (namely, technical solutions connected by logical "AND").

The terms "comprise", "include", and "contain", as well as their variations, as used herein are synonymous, and are inclusive or open-ended, and do not exclude additional uncited members, elements, or method steps.

In the present application, a numerical range defined by endpoints includes all values and fractions within the range, as well as the cited endpoints.

In the present application, concentration values refer to values that may fluctuate within a certain range. For example, fluctuations may occur within the corresponding precision range. For instance, a value of 2% may allow fluctuations within 0.1%. For larger values or values that do not require precise control, the meaning may include greater fluctuations. For example, a value of 100 mM may allow fluctuations within ±1%, ±2%, ±5%, etc. For molecular weight, the meaning may include fluctuations within ±10%.

In the present application, descriptions such as "multiple" and "various" refer to two or more than two, unless otherwise specified.

In the present application, technical features described in an open-ended manner encompass both closed technical solutions consisting of the listed features and open technical solutions that include the listed features.

In the present application, expressions such as "preferably", "more preferably", "most preferably", "specific", or "desirably" are used solely to describe embodiments or examples with better effects, and shall not be construed as limiting the scope of protection of the present application. In present application, "optionally", "optional" and "option" refer to "dispensable" or any one of two parallel solutions of "with" and "without". If "optional" appears multiple times in a technical solution, "optional" at each location is independent unless specific explanation is provided and there are contradictions or mutual constraints.

Eribulin derivatives in the present application include a compound of formula (I), a compound of formula (II), a compound of formula (I'), a compound of formula (II'), the compound of claim 44, isotope-labeled compounds of the compound of formula (I), the compound of formula (II), the compound of formula (I'), the compound of formula (II'), or the compound of claim 44, and optical isomers, geometric isomers, tautomers, or isomeric mixtures of the compound of formula (I), the compound of formula (II), the compound of formula (I'), the compound of formula (II'), or the compound of claim 44, or the isotope-labeled compounds thereof, as well as pharmaceutically acceptable salts of the various compounds mentioned above.

The antibody-drug conjugate in the present application include a compound of formula (I"), a compound of formula (II"), and the compound of claims 45 to 48, isotope-labeled compounds of the compound of formula (I"), the compound of formula (II"), or the compound of claims 45 to 48, and optical isomers, geometric isomers, tautomers, or isomeric mixtures of the compound of formula (I"), the compound of formula (II"), or the compound of claims 45 to 48, or the isotope-labeled compounds thereof, as well as pharmaceutically acceptable salts of the various compounds mentioned above.

The term "isotope-labeled compound" refers to a compound that is labeled with an isotope and that is obtained by substituting any atom in the compound with its isotopic atom. The present invention includes all pharmaceutically acceptable isotope-labeled compounds of the compound, where one or more atoms are substituted by an atom having the same atomic number as an atom usually found in nature but a different atomic mass or mass quantity. Examples of isotopes suitable for inclusion in the compound in the present application include isotopes of hydrogen, such as ²H(D) and ³H(T); isotopes of carbon, such as ¹¹C, ¹³C, and ¹⁴C; isotopes of chlorine, such as ³⁷Cl; isotopes of fluorine, such as ¹⁸F; isotopes of iodine such as ¹²³I and ¹²⁵I; isotopes of nitrogen such as ¹³N and ¹⁵N; isotopes of oxygen, such as ¹⁵O, ¹⁷O, and ¹⁸O; and isotopes of sulfur such as ³⁵S. The isotope-labeled compound can generally be prepared by conventional techniques known to a person skilled in the art or by using a suitable isotope-labeled reagent in place of a previously used non-labeled reagent in a method analogous to that described in examples and preparations appended herein.

The term "optical isomer" means that when a compound has one or more chiral centers, each chiral center can have an R configuration or an S configuration, and various isomers formed by them are optical isomers. The optical isomers include all diastereoisomers, enantiomers, mesoforms, racemates, or mixtures thereof. For example, the optical isomer can be separated via a chiral chromatographic column or chiral synthesis.

The term "geometric isomer" means that when there is a double bond in the compound, the compound may have a cis isomer, a trans isomer, an E isomer, and a Z isomer. The geometric isomer includes a cis isomer, a trans isomer, an E isomer, a Z isomer, or a mixture thereof.

The term "tautomer" refers to an isomer generated due to rapid movement of a specific atom in a molecule between two positions. A person skilled in the art can understand that tautomers can transform into each other, and may is present together when an equilibrium state is reached in a specific state.

The compound in the present application may be present in a form of a pharmaceutically acceptable salt such as an acid addition salt and/or base addition salt of the compound of formula (I). Unless otherwise clarified, the "pharmaceutically acceptable salt" used herein includes acid addition salt or base addition salt that may be present in the compound in the present application. A suitable acid addition salt is formed by an acid that forms a non-toxic salt. Examples of the acid addition salt include, but are not limited to: acetate, adipate, aspartate, benzoate, benzenesulfonate, bicarbonate/carbonate, bisulfate/sulfate, borate, camphorsulfonate, citrate, cyclamate, edisylate, formate, fumarate, glucoheptonate, gluconate, glucuronic acid salt, hexafluorophosphate, 2-(4-hydroxybenzyl)benzoate, hydrochloride/chloride, hydrobromide/bromide, hydroiodide/iodide, 2-isethionate, lactate, malate, maleate, malonate, methanesulfonate, sulfate, methylsulfate, naphthalate, 2-naphthalenesulfonate, nicotinate, nitrate, orotate, oxalate, palmitate, phosphate/hydrogen phosphate/dihydrogen phosphate, pyroglutamate, glucarate, stearate, salicylate, tannate, tartrate, tosylate, and trifluoroacetate. A suitable acid addition base is formed by a base that forms a non-toxic salt. Examples of the base addition salt include, but are not limited to, aluminum, arginine, calcium, choline, diethylamine, diethanolamine, glycine, lysine, magnesium, meglumine, ethanolamine, potassium, sodium, tromethamine, and zinc salt. Half-salts of acids and bases such as hemisulfate and hemicalcium salts may also be formed. For a review of suitable salts, refer to Handbook of Pharmaceutical Salts: Properties, Selection and Use (Stahl and Wermuth, Wiley-VCH, 2002). A method for preparing the pharmaceutically acceptable salt of the compound in the present application is known to a person skilled in the art.

Some compounds in the present application may is present in different crystalline or amorphous forms, and fall within the scope of the present application regardless of which form the compounds are in.

In the present application, the term "antibody" refers to an immunoglobulin, which is a tetrapeptide chain structure formed by bonding two identical heavy chains with two identical light chains through interchain disulfide bonds. Immunoglobulins have heavy chain constant regions with different amino acid compositions and sequences, and therefore, also have different antigenicities. Therefore, immunoglobulins can be divided into five categories, or isoforms of immunoglobulins, namely IgM, IgD, IgG, IgA, and IgE, and corresponding heavy chains thereof are µ chain, δ chain, γ chain, α chain, and ε chain respectively. The same type of Ig can be further divided into different subtypes based on a difference in amino acid compositions in hinge regions and the numbers and positions of disulfide bonds in heavy chains. For example, Ig can be divided into IgG1, IgG2, IgG3, and IgG4. Light chains are divided into κ chains or λ chains as per the difference in the constant regions. Each of the five types of Ig can include either the κ chains or λ chains. The sequences of about 110 amino acids near the N-termini of the heavy chain and light chain of the antibody may vary greatly and are referred to as variable regions (Fv regions). The rest amino acid sequences near the C-termini are relatively stable and are referred to as the constant regions. The variable region includes 3 hypervariable regions (HVRs) and 4 framework regions (FRs) with relatively conserved sequences. Specificity of the antibody is determined by the 3 hypervariable regions, also referred to as complementary-determining regions (CDRs). Each of a light chain variable region (LCVR) and a heavy chain variable region (HCVR) is composed of 3 CDR regions and 4 FR regions, and a sequence from the N-terminus to the C-terminus is as follows: FR1, CDR1, FR2, CDR2, FR3, CDR3, and FR4. 3 CDR regions of the light chain are LCDR1, LCDR2, and LCDR3. 3 CDR regions of the heavy chain are HCDR1, HCDR2, and HCDR3. The number and positions of CDR amino acid residues in the LCVR region and HCVR region of the antibody or antigen-binding fragment in the present application conform to the known Kabat numbering rule (LCDR1 to LCDR3 and HCDR2 and HCDR3) or the Kabat and Chothia numbering rules (HCDR1).

The term "antigen-binding fragment" refers to one or more fragments of an antibody that maintain an ability to bind specifically to the antigen. The antigen-binding ability of the antibody has been demonstrated by using a fragment of a full-length antibody. Examples of binding fragments included in the "antigen-binding fragment" include: (i) Fab fragment, a monovalent fragment consisting of VL, VH, CL, and CH domains; (ii) F(ab')2 fragments, including bivalent fragments of two Fab fragments bound by a disulfide bridge in the hinge region; (iii) F fragment consisting of VH and CH domains; (iv) Fv fragment consisting of VH and VL domains on a single arm of the antibody; (v) a single-domain or dAb fragment consisting of a VH domain; and (vi) a separated complementary-determining region (CDR) or (vii) a combination of two or more separated CDRs that can optionally be bound by a synthesized linker. In addition, although both VL and VH domains of the Fv fragment are encoded by separate genes, the VL and VH domains can be bound in a recombinant method through synthesized linkers, so that the VL and VH domains can generate a single protein chain (referred to as single-stranded Fv (scFv)) for pairing the VL and VH regions to form monovalent molecules. Such single-chain antibodies are incorporated into the term "antigen-binding fragment". Such antibody fragments are obtained through conventional techniques known to persons skilled in the art, and fragments are sifted out as per functions in the same manner as intact antibodies. Antigen-binding sites can be generated by recombinant DNA techniques, enzymatic reactions or chemical fragmentation of intact immunoglobulins. Antibodies can be different types of antibodies, for example, IgG (for example, IgG1, IgG2, IgG3, or IgG4 subtype), IgA1, IgA2, IgD, IgE, or IgM antibody.

The term "linker group" refers to a chemical structure fragment or bond that has one end linked to an antibody and another end linked to a toxin drug, which can also be linked to the antibody or toxin drug after linked to another linking group.

The term "antibody-drug conjugate (ADC)" means that an antibody or an antigen-binding fragment is linked to a biologically active toxin drug such as the eribulin derivative through the stable linking unit.

The term "short peptide" has a common meaning in the art and can refer to a compound formed by covalent linking of an amide bond (peptide bond) formed by condensation and dehydration of a carboxyl group of one amino acid molecule and an amino group of another amino acid molecule. The nomenclature for defining peptides is commonly used in the art, where an amino group at the N-terminus is on the left side and a carboxyl group at the C-terminus is on the right side. The short peptide chain in the present application refers to a peptide chain obtained by condensation and dehydration of two or more (for example, 2, 3, 4, 5, 6, 7, 8, 9, 10, 11 or 12) amino acids, with -NH- formed by the amino group at the N-terminus on the left side and -CO- formed by the carboxyl group at the C-terminus on the right side. For example, "-^{NH}Gly-Gly-Phe-Gly^{CO}-" represents a tetrapeptide chain formed by condensation of Gly, Gly, Phe, and Gly in sequence, with -NH- formed by the amino group of Gly at the N-terminus on the left side and -CO- formed by the carboxyl group of Gly at the C-terminus on the right side.

The term "amino acid residue" has a common meaning in the art and refers to a group formed by reaction of an amino acid (as a single amino acid or part of a peptide) and a peptide, another amino acid, or an amino acid residue. Usually, when the amino acid is ligated with another amino acid or the amino acid residue, water is eliminated, and remaining part of the amino acid is referred to as the amino acid residue.

The term "amino acid" has a common meaning in the art and can include both natural and unnatural amino acids. An abbreviation of the amino acid residue in the present invention is a standard 3-letter and/or 1-letter code for denoting one of 20 common amino acids in the art.

The amino acid residue or amino acid has isomeric forms, including L-type and D-type.

Examples of three-letter and single-letter codes of amino acids used in the present application and structures thereof are as follows:

| Brief name | (Abbreviation) | Name | Structure |
|---|---|---|---|
| G | Gly | Glycine (Glycine) | 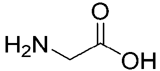 |
| A | Ala | Alanine (Alanine) | 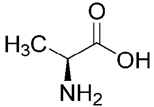 |
| V | Val | Valine (Valine) | 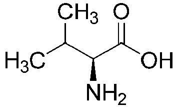 |
| F | Phe | Phenylalanine (Phenylalanine) | 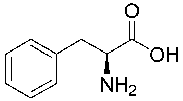 |
| C | Cit | Citrulline (Citrulline) | 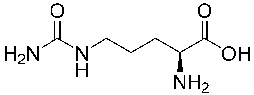 |

In some substituents in the present application, 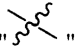 represents a linking site and **"** 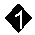 **"** represents a linking site. For example, for 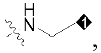 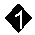 represents a linking site linked to A₁' or A₂', and 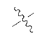 represents a linking site linked to L₂.The expression "-NH-CH₂-" means that the left side is connected to L₂ via -NH-, and the right side is connected to A₁' or A₂' via -CH₂-.

Unless otherwise specified, "optional substitution" means that hydrogen on the substituted group is not substituted or one or more substitutable sites of the substituted group are independently subjected to substitution with a substituent. The substituent is independently selected from one or more of deuterium, an oxo group, halogen, an amino group, a hydroxyl group, a nitro group, a cyano group, a sulfhydryl group, an optionally substituted alkyl group, an optionally substituted alkyloxy group, an optionally substituted alkylthio group, an optionally substituted alkyl amino group, an optionally substituted carbon ring group, and an optionally substituted heterocyclic group; and the substituent on the alkyl group, the carbon ring group, and the heterocyclic group is selected from one or more of an oxo group, deuterium, halogen, an amino group, a hydroxyl group, a nitro group, a cyano group, a sulfhydryl group, an alkyl group, a haloalkyl group, an alkyloxy group, a hydroxyl-substituted alkyl group, an alkyloxyalkyl group, and the like. When the substituent is "oxo", this indicates that two hydrogen atoms on carbon at the same substitution site are substituted by oxygen atoms, or a heteroatom is bonded to oxygen, to form, for example, a nitrogen oxide with nitrogen bonded to oxygen or a sulfinyl group or sulfonyl group with sulfur bonded to oxygen. "Optional oxo" means that the hydrogen on the substituted group is not substituted, or two hydrogen atoms on carbon at the same substitution site are substituted by oxygen atoms, or a heteroatom is bonded to oxygen, to form, for example, a nitrogen oxide with nitrogen bonded to oxygen or a sulfinyl group or sulfonyl group with sulfur bonded to oxygen. The term "independently" means that when the number of substituents is greater than one, these substituents may be the same or different.

The term "oxygen bond" (=O) means that two hydrogen atoms on the bonded carbon atom are substituted by an oxygen atom to form a carbonyl group.

In the present application, 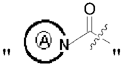 means that N forms a Ⓐ ring with another group bonded thereto; and 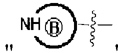 means that X₃ or Y₁ is a Ⓑ ring that contains at least one -NH-.

The term "alkyl group" refers to a saturated aliphatic hydrocarbonyl group, which is a linear or branched group including 1 to 20 carbon atoms, or preferably, an alkyl group including 1 to 12 carbon atoms (the number of carbon atoms is between 1 and 12, and is specifically 1, 2, 3, 4, 5, 6, 7, 8, 9, 10, 11, or 12), or more preferably, an alkyl group including 1 to 10 carbon atoms or most preferably 1-8 carbon atoms (namely, a C₁-C₈ alkyl group, where the number of carbon atoms is 1-8, and is specifically 1, 2, 3, 4, 5, 6, 7, or 8) or 1-6 carbon atoms (namely, a C₁-C₆ alkyl group, where the number of carbon atoms is 1-6, and is specifically 1, 2, 3, 4, 5, or 6). Examples include, but are not limited to, a methyl group, an ethyl group, an n-propyl group, an isopropyl group, an n-butyl group, an isobutyl group, a tert-butyl group, a sec-butyl group, an n-amyl group, a neopentyl group, a 1,1-dimethylpropyl group, 1,2-dimethylpropyl group, 2,2-dimethylpropyl group, 1-ethylpropyl group, 2-methylbutyl group, 3-methylbutyl group, an n-hexyl group, an n-heptyl group, an n-octyl group, 3-hexyl group, and 2-pentyl group.

The terms "alkyloxy group" refers to an alkyl group with -O-, and a definition of the alkyl group is the same as that in the foregoing description. Representative examples include, but are not limited to, a methoxy group, an ethoxy group, a propoxy group, an isopropoxy group, a butoxy group, 1-methyl butoxy group, 2-methyl butoxy group, or a tert-butoxy group.

The term "halogen" refers to F, Cl, Br, and I.

The terms "hydroxyl-substituted alkyl group", "alkyloxy-substituted alkyl group", "amino-substituted alkyl group", "haloalkyl group", "cyano-substituted alkyl group", "carbon ring-substituted alkyl group", and "heterocyclyl-substituted alkyl group" refer to substitution on an alkyl group with one or more hydroxyl groups, substitution on an alkyl group with one or more alkyloxy groups, substitution on an alkyl group with one or more amino groups, substitution on an alkyl group with one or more halogen atoms, substitution on an alkyl group with one or more cyano groups, substitution on an alkyl group with one or more carbon ring groups, and substitution on an alkyl group with one or more heterocyclic groups.

The term "carbon ring group" refers to a saturated, partially unsaturated or completely unsaturated carbon ring group such as a cycloalkyl group, an aryl group, a cycloalkenyl group, a cycloalkynyl group, and an enyne ring group, and may be monocyclic or polycyclic (for example, bicyclic or tricyclic), for example, single-ring, fused-ring, integrated-ring, spiro-ring, or bridged-ring. The number of carbon atoms may be 3 to 20 (for example, 3, 4, 5, 6, 7, 8, 9, 10, 11, 12, 13, 14, 15, 16, 17, 18, 19, or 20).

The term "cycloalkyl group" refers to a saturated monocyclic or polycyclic (for example, bicyclic or tricyclic) cyclic hydrocarbon substituent, including a single ring, a fused ring, an integrated ring, a spiro ring, a bridged ring, or the like. The cycloalkyl group can contain 3 to 20 carbon atoms (for example, 3, 4, 5, 6, 7, 8, 9, 10, 11, 12, 13, 14, 15, 16, 17, 18, 19, or 20), preferably 3 to 15 carbon atoms, and more preferably, 3 to 12 carbon atoms. Non-limiting examples of the cycloalkyl group include a cyclopropyl group, a cyclobutyl group, a cyclopentyl group, a cyclohexyl group, a cycloheptyl group, a cyclooctyl group, 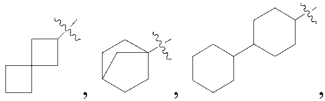 and the like.

The term "aryl group" represents a monocyclic or bicyclic aromatic carbon ring system containing 6-10 carbon atoms, and examples of aryl groups include a phenyl group and a naphthalene group, for example, 1-naphthalene group, 2-naphthalene group, 3-naphthalene group, and 4-naphthalene group.

The term "heterocyclic group" refers to a saturated, partially unsaturated or completely unsaturated heterocyclic group such as a heterocycloalkyl group and heteroaryl group, and may be monocyclic or polycyclic (for example, bicyclic or tricyclic), for example, single-ring, fused-ring, integrated-ring, spiro-ring, or bridged-ring. The number of atoms can be 3 to 20 (for example, 3, 4, 5, 6, 7, 8, 9, 10, 11, 12, 13, 14, 15, 16, 17, 18, 19, or 20), and can contain 1, 2, 3, or 4 heteroatoms selected from N, O, or S, for example, 1, 2, or 3 heteroatoms selected from N or O.

The term "heterocycloalkyl group" refers to a saturated monocyclic or polycyclic (for example, bicyclic or tricyclic) hydrocarbon substituent, including, a single-ring, fused-ring, integrated-ring, spiro-ring, or bridged-ring hydrocarbon substituent, which contains 3 to 20 ring atoms (for example, 3, 4, 5, 6, 7, 8, 9, 10, 11, 12, 13, 14, 15, 16, 17, 18, 19, or 20), where one or more (for example, 1, 2, 3, or 4) ring atoms are heteroatoms selected from N, O, or S. Preferably, the hydrocarbon substituent contains 3 to 12 ring atoms (including 3, 4, 5, 6, 7, 8, 9, 10, 11 or 12 ring atoms), where 1 to 3 ring atoms are heteroatoms (1, 2, or 3 heteroatoms), and the heteroatoms are selected from N, O or S. The heterocycloalkyl group can be 3- to 11-membered single-ring, fused-ring, integrated-ring, spiro-ring, or bridged-ring heterocycloalkyl group with selectively 1 or 2 N atoms, for example, 3- to 6-membered single-ring heterocycloalkyl group, 6- to 12-membered fused-ring heterocycloalkyl group, 6- to 12-membered integrated-ring heterocycloalkyl group, 6- to 12-membered spiro-ring heterocycloalkyl group, and 6- to 12-membered bridged-ring heterocycloalkyl group. Inclusive examples include a pyrrolidinyl group, an imidazolidinyl group, a tetrahydrofuran group, a piperidinyl group, a piperazine group, a tetrahydropyranyl group, an azacyclopropyl group, an oxacyclopropyl group, a thiacyclopropyl group, an azacyclobutyl group, an oxacyclobutyl group, a thiacyclobutyl group, oxacyclohexane, a morpholinyl group, a thiomorpholinyl group, a dioxane group, a dithiacyclohexyl group, an oxazolidinyl group, a thiazolidinyl group, a pyrazolidinyl group, imidazoline, tetrahydrofuran-1-yl, tetrahydrofuran-2-yl, tetrahydrofuran-3-yl, tetrahydropyran-1-yl, tetrahydropyran-2-yl, tetrahydropyran-3-yl, tetrahydropyran-4-yl, piperidine-1-yl, piperidine-4-yl,

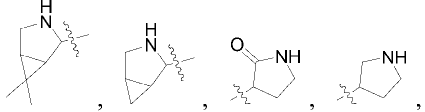

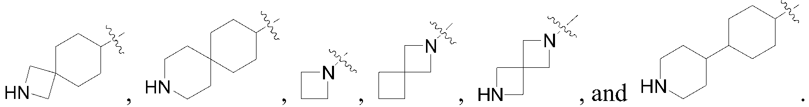

The term "heteroaryl group" represents an aromatic monocyclic or multi-cyclic system containing a 5- to 10-membered structure, or preferably, a 5- to 8-membered structure or a 6- to 10-membered structure, or more preferably, a 5- to 6-membered structure, where one or more ring atoms are heteroatoms and other atoms are carbon atoms, heteroatoms are independently selected from O, N or S, and the number of heteroatoms is preferably 1, 2, 3 or 4. The heteroaryl group can be 5- or 6-membered aromatic monocyclic structure containing selectively 1 or 2 N, S, or O atoms, and can be 8- to 10-membered aromatic fused-ring structure containing selectively 1, 2, 3 or 4 N, S, or O atoms. Examples of heteroaryl groups include, but are not limited to, a furanyl group, a thienyl group, an oxazolyl group, a thiazolyl group, an isooxazolyl group, an oxadiazolyl group, a thiadiazolyl group, a pyrrolyl group, a pyrazolyl group, an imidazol group, a triazolyl group, a tetrazolyl group, a pyridinyl group, a pyrimidyl group, a pyrazinyl group, a pyridazinyl group, a thiodiazolyl group, a triazinyl group, a phtharazinyl group, a quinolinyl group, an isoquinolinyl group, a pteridinyl group, a purinyl group, an indolyl group, an isoindolyl group, an inazolyl group, a benzo-furanyl group, a benzo-thiophenyl group, a benzo-pyridinyl group, a benzo-pyrimidinyl group, a benzo-pyrazinyl group, a benzo-imidazol group, a benzo-phtharazinyl group, a pyrrolo [2,3-b]pyridinyl group, an imidazo[1,2-a]pyridinyl group, a pyrazolo[1,5-a]pyridinyl group, a pyrazolo[1,5-a]pyrimidinyl group, an imidazo[1,2-b]pyridazinyl group, a [1,2,4]triazolo[4,3-b] pyridazinyl group, a [1,2,4]triazolo[1,5-a]pyrimidinyl group and a [1,2,4]triazolo[1,5-a] pyridyl group, a pyridin-1-yl group, a pyridin-2-yl group, a pyridin-3-yl group, a pyridin-4-yl group, a quinolin-2-yl group, a quinolin-3-yl group, a quinolin-4-yl group, a quinolin-5-yl group, a quinolin-6-yl group, a quinolin-7-yl group, and a quinolin-8-yl group.

The term "fused-ring" means that two rings share two bonded ring atoms. For example, the "fused-ring" is 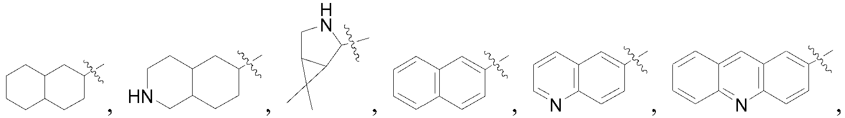 and the like.

The term "integrated-ring" means that two rings are bonded by a single bond. For example, the "integrated-ring" is a biphenyl group, a terphenyl group, 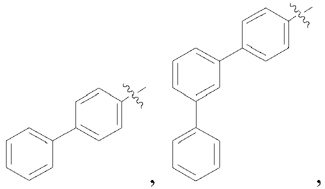 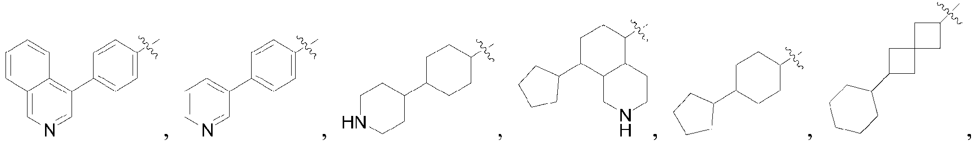 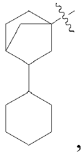 and the like.

The term "spiro-ring" means that two rings share one ring atom. For example, the "spiro-ring" is 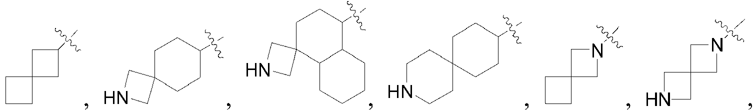 and the like.

The term "bridged-ring" means that two rings share two indirectly linked ring atoms. For example, the "bridged-ring" is 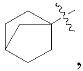 and the like.

In the present application, number ranges related to the number of substituents, the number of carbon atoms, the number of ring atoms, the number of heteroatoms, and the number of groups represent enumerations of all integers within the range, and the range is only used as a simplified representation. For example, "1-4 substituents" represent 1, 2, 3, or 4 substituents; and "3-8 ring atoms" represent 3, 4, 5, 6, 7, or 8 ring atoms. Therefore, the number ranges related to the number of substituents, the number of carbon atoms, the number of ring atoms, the number of heteroatoms, and the number of groups also cover any one of its sub-ranges, and each sub-range is also construed as being disclosed in the present application.

The term "pharmaceutical composition" refers to a mixture containing one or more of the compounds in the present application and one or more pharmaceutically acceptable carriers or excipients. The pharmaceutical composition may not contain other active ingredients, or may further contain one or more other active ingredients. An objective of the pharmaceutical composition is to facilitate administration to the organism and facilitate absorption of the active components, thereby exerting biological activity.

The term "pharmaceutically acceptable carrier" or "pharmaceutically acceptable excipient" includes, but is not limited to, any glidant, sweetener, diluent, preservative, dye/colorant, flavoring agent, surfactant, wetting agent, dispersant, suspending agent, stabilizer, isotonic agent, solvent, emulsifier, disintegrant, binder, filler, antioxidant, fragrance, co-solvent, solubilizer, osmotic pressure regulator, and matrix fraction in semi-solid formulations of ointments and creams approved for use in humans or livestock by a relevant regulatory authority.

The pharmaceutical composition in the present application can be prepared into a liquid dosage form, a solid dosage form, or a semisolid dosage form. The liquid dosage form can be a solution (including a true solution and a colloid solution), an emulsion (including o/w type, w/o type and double emulsions), a suspension, an injection (including a water injection, a powder injection and an infusion), eye drops, nasal drops, a lotion, a liniments, and the like; the solid dosage form can be a tablet (including an ordinary tablet, an enteric-coated tablet, a buccal tablet, a dispersible tablet, a chewable tablet, an effervescent tablet and an orally disintegrating tablet), a capsule (including a hard capsule, a soft capsule and an enteric-coated capsule), granules, powder, pills, a suppository, films, patches, an aerosol, a spray, and the like; and the semisolid dosage form can be an ointment, a gel, a paste, and the like. The drug composition in the present application can be prepared into a common formulation or a sustained-release formulation, a controlled-release formulation, a targeted formulation and various particle delivery systems.

The term "subject" refers to any human or non-human organism that could potentially benefit from treatment with the compound in the present application. Exemplarily, a subject includes a human or a mammal at any age. Preferably, the subject is a human.

The term "treatment" includes treatment of a disease or a symptom in the mammal, especially the human, and includes: (a) inhibition of an infection, a disease or a symptom, that is, inhibition or delaying of development of the infection, the disease or the symptom; (b) relief of the infection, the disease or the symptom, that is, improvement of the disease or the symptom; and/or (c) cure of the infection, the disease or the symptom.

The term "prevention" includes a prophylactic therapy in the mammal, especially the human, to reduce occurrence possibility of the infection, the disease or the symptom. A patient can be selected for the prophylactic therapy based on a factor of an increased risk of infection or having a disease or a symptom compared to the general population. The "prevention" can include treatment of a subject who has not yet exhibited an infection or a clinical condition, and prevention of a second occurrence of the same or similar infection or clinical condition.

The "effective dose" refers to the amount of compound in the present application that is effective in treating or preventing cancers, inhibiting cancer cell proliferation, and/or reducing tumor volumes when administered alone or in combination.

The embodiments of the present application are described in detail below with reference to examples. It should be understood that these embodiments are merely used to describe the present application but not to limit the scope of the present application. A person skilled in the art can make various modifications and changes to the present application without departing from the scope or spirit of the present application. In the following examples, for a test method for which a specific condition is not specified, preferably, refer to the guidance provided in the present application, a test manual or a conventional condition in the art, another test method known in the art, or a condition recommended by a manufacturer.

In the following specific examples, a measurement parameter related to an ingredient may have a slight deviation within a weighing accuracy range unless otherwise specified. Parameters involving temperature and time may allow acceptable deviations resulting from instrument testing accuracy or operational precision.

In addition, some protective groups may be used in the present application to protect some functional groups from unnecessary reactions. Protective groups suitable for various functional groups and protection or deprotection conditions thereof have been widely known to persons skilled in the art. For example, Protective Groups in Organic Synthesis (T. W. Greene and G. M. Wuts, 3rd edition, Wiley, New York, 1999) and references in the book describe in detail protection or deprotection of a large number of protective groups.

Isolation and purification of compounds and intermediates are implemented through suitable methods and steps, for example, filtration, extraction, distillation, crystallization, column chromatography, preparative thin-layer chromatography, preparative high-performance liquid chromatography, or a combination of these methods based on specific needs. For specific use method, refer to examples described in the present application. Certainly, other similar isolation and purification methods can also be used. Conventional methods (including physical constants and spectral data) can be used for characterization.

### Preparation Examples

### Preparation Example 1: Preparation of PE-1

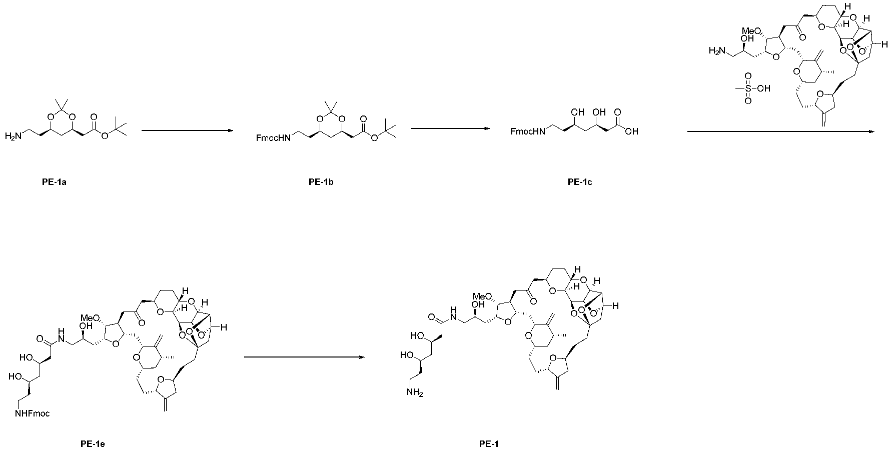

### Step 1: Preparation of tert-butyl 2-((4R,6R)-6-(2-((((9H-fluoren-9-yl)methoxy)carbonyl) amino)ethyl)-2,2-dimethyl-1,3-dioxan-4-yl)acetate

Sodium bicarbonate (307 mg, 3.66 mmol, 2 eq) (dissolved in 3 mL of water) was added to a solution of tert-butyl 2-((4R,6R)-6-(2-aminoethyl)-2,2-dimethyl-1,3-dioxan-4-yl)acetate (500 mg, 1.83 mmol, 1.0 eq) and 2,5-dioxocyclopentyl (9H-fluoren-9-yl)methyl carbonate (615.17 mg, 1.83 mmol, 1.0 eq) in tetrahydrofuran (6 mL) at 0°C. After the addition, the mixture was allowed to react at 25°C for 16 hours. After LCMS showed the reaction ended, the reaction solution was diluted with water and extracted with ethyl acetate three times. The combined organic phases were washed with brine once, dried over anhydrous sodium sulfate, and filtered, and the filtrate was concentrated under reduced pressure to obtain the compound tert-butyl 2-((4R,6R)-6-(2-((((9H-fluoren-9-yl) methoxy)carbonyl)amino)ethyl)-2,2-dimethyl-1,3-dioxan-4-yl)acetate as a yellow solid (988 mg, crude product, where the yield was not calculated).

### Step 2: Preparation of (3R,5R)-7-((((9H-fluoren-9-yl)methoxy)carbonyl)amino)-3,5-dihydroxyheptanoic acid

10% hydrochloric acid (2 mL) was added to a solution of the compound tert-butyl 2-((4R,6R)-6-(2-((((9H-fluoren-9-yl)methoxy)carbonyl)amino)ethyl)-2,2-dimethyl-1,3-dioxan-4-yl) acetate (111 mg, 90%, 0.20 mmol, 1 eq) in tetrahydrofuran (10 mL). After the addition, the reaction solution was allowed to react at 25°C for 2 hours, and the LCMS showed approximately 10% product. The reaction solution was purified by preparative high-performance liquid chromatography (preparative chromatograph manufacturer: ISCO, model: ISCO-R2, column: YMC-Triart Prep C18 250 × 30 mm × 10 µm, mobile phase: water (0.225% FA, formic acid)-acetonitrile, with an elution ratio of acetonitrile increased from 39% to 69%), to afford the compound (3R,5R)-7-((((9H-fluoren-9-yl)methoxy)carbonyl)amino)-3,5-dihydroxyheptanoic acid (17 mg, 21% yield).

LCMS (ESI): m/z, 422[M+Na]⁺. Rt = 1.662 min. ¹H NMR (400 MHz, Chloroform-d) δ 7.77 (d, *J =* 7.6 Hz, 2H), 7.59 (d, *J =* 8.0 Hz, 2H), 7.41 (t, *J =* 7.4 Hz, 2H), 7.36-7.29 (m, 2H), 5.09 (s, 2H), 4.48 (d, *J =* 6.6 Hz, 2H), 4.34-4.14 (m, 2H), 3.88 (t, *J =* 10.2 Hz, 1H), 3.63-3.47 (m, 1H), 3.17-3.06 (m, 1H), 2.52-2.55 (m, 3H).

### Step 3: Preparation of (9H-fluoren-9-yl)methyl (3R,5R)-3,5-dihydroxy-7-(S)-2-hydroxy-3-(2S,5S,8S,11S,13R,15R,16aS,18R,19R,19aS,23R,24aS,25S,26aR,27S,28R,29aR,29bS)-19-methox y-13-methyl-7,14-dimethylene-21-oxooctahydro-25H-2,27:5,8,15-trioxa-23,25-ethano-2,28-methan o[3,2',3':5,6]pyran[4]

A solution of compound (3R,5R)-7-((((9H-fluoren-9-yl)methoxy)carbonyl)amino)-3,5-dihydroxyheptanoic acid (2.9 mg, 7.26 µmol, 1.2 eq), eribulin mesylate (5 mg, 6.05 µmol, 1 eq), N,N,N',N'-tetramethyl-O-(7-azabenzotriazol-1-yl)uronium hexafluorophosphate (HATU, 2.76 mg, 7.26 µmol, 1.2 eq), and N,N-diisopropylethylamine (2 µL, 12.11 µmol, 2 eq) in N,N-dimethylformamide (1 mL) was stirred in an ice bath for 5 minutes, then warmed to room temperature and stirred for 2 hours. LCMS showed the starting materials were completely consumed. The mixture was concentrated via an oil pump to afford (9H-fluoren-9-yl)methyl (3R,5R)-3,5-dihydroxy-7-(S)-2-hydroxy-3-(2S,5S,8S,11S,13R,15R,16aS,18R,19R,19aS,23R,24aS, 25S,26aR,27S,28R,29aR,29bS)-19-methoxy-13-methyl-7,14-dimethylene-21-oxooctahydro-25H-2, 27:5,8,15-trioxa-23,25-ethano-2,28-methano[3,2',3':5,6]pyran[4] (10.4 mg, crude product, where the yield was not calculated).

LCMS (ESI): m/z, 1133[M+Na]⁺.

### Step 4: Preparation of (3R,5R)-7-amino-3,5-dihydroxy-N-((S)-2-hydroxy-3-((2S,5S,8S,11S, 13R,15R,16aS,18R,19R,19aS,23R,24aS,25S,26aR,27S,28R,29aR,29bS)-19-methoxy-13-methyl-7, 14-dimethylene-21-oxooctahydro-25H-2,27,5,8,15-trioxa-23,25-ethoxy-2,28-methoxy[3,2-i]furo[2', 3':5,6]pyrano[4,3-b][1,4]dioxepin-18-yl)propyl)heptanamide (PE-1)

1,8-diazabicyclo[5.4.0]undec-7-ene (1.75 µL, 11.69 µmol, 3 eq) was added to a solution of (9H-fluoren-9-yl)methyl ((3R,5R)-3,5-dihydroxy-7-(((S)-2-hydroxy-3-((2S,5S,8S,11S,13R,15R, 16aS,18R,19R,19aS,23R,24aS,25S,26aR,27S,28R,29aR,29bS)-19-methoxy-13-methyl-7,14-dimeth ylene-21-oxooctahydro-25H-2,27:5,8,15-trioxa-23,25-ethano-2,28-methano[3,2',3':5,6]pyran[4] (10.4 mg, crude) in N,N-dimethylformamide (1 mL). The mixture was stirred in an ice bath for 5 minutes, then allowed to warm to room temperature and stirred for 2 hours. LCMS indicated that the starting materials were completely consumed. The resulting solution was purified by preparative high-performance liquid chromatography (preparative chromatograph manufacturer: Shimadzu, model LC-20AP, column: GS-120-10-C18AP, mobile phase: water (0.225% HCOOH)-acetonitrile, with an elution ratio of water increased from 10% to 45%), to afford (3R,5R)-7-amino-3,5-dihydroxy-N-((S)-2-hydroxy-3-((2S,5S,8S,11S,13R,15R,16aS,18R,19R,19aS,23R,24aS,25S,26aR,2 7S,28R,29aR,29bS)-19-methoxy-13-methyl-7,14-dimethylene-21-oxooctahydro-25H-2,27,5,8,15-tr ioxa-23,25-ethoxy-2,28-methoxy[3,2-i]furo[2',3':5,6]pyrano[4,3-b][1,4]dioxepin-18-yl)propyl)hepta namide (1.05 mg, 24.2% yield).

LCMS (ESI): m/z, 889[M+H]⁺.

¹H NMR (400 MHz, DMSO-*d*₆) δ 8.40 (s, 1H), 7.80 (s, 1H), 5.08-4.97 (m, 2H), 4.79 (d, *J* = 30.8 Hz, 2H), 4.64 (dt, *J* = 4.6, 2.6 Hz, 1H), 4.55 (t, *J* = 4.2 Hz, 1H), 4.26 (d, *J* = 10.8 Hz, 1H), 4.17 (td, *J =* 10.2, 4.2 Hz, 1H), 4.10 (d, *J =* 4.4 Hz, 2H), 4.02 (s, 1H), 3.93 (d, *J =* 5.6 Hz, 1H), 3.84-3.67 (m, 4H), 3.52 (d, *J =* 10.0 Hz, 4H), 3.05 (q, *J =* 6.8 Hz, 4H), 2.86-2.75 (m, 4H), 2.28-2.20 (m, 6H), 2.13 (t, *J* = 11.6 Hz, 3H), 2.06-1.82 (m, 10H), 1.74-1.57 (m, 8H), 1.55-1.39 (m, 6H), 1.36-1.14 (m, 6H), 1.04 (d, *J =* 6.4 Hz, 3H).

### Preparation Example 2: Preparation of Compound PE-2

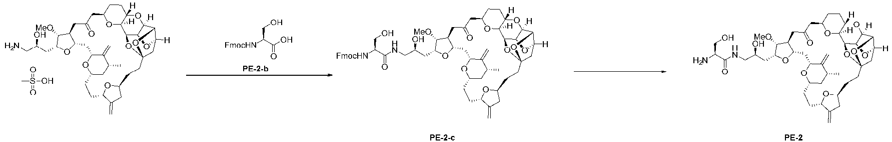

### Step 1: Preparation of compound PE-2-c

A solution of eribulin mesylate (8 mg, 9.69 µmol, 1 eq), (((9H-fluoren-9-yl)methoxy) carbonyl)-L-serine (3.8 mg, 11.69 µmol, 1.2 eq), N,N,N',N'-tetramethyl-O-(7-azabenzotriazol-1-yl)uronium hexafluorophosphate (4.42 mg, 11.62 µmol, 1.2 eq), and N,N-diisopropylethylamine (3.2 µL, 19.37 µmol, 2 eq) in N,N-dimethylformamide (1 mL) was stirred in an ice bath for 5 minutes, then allowed to warm to room temperature and stirred for 2 hours. LCMS indicated that the starting materials were completely consumed. The mixture was concentrated via an oil pump to afford the crude product PE-2-c (19.4 mg, crude product, where the yield was not calculated).

LCMS(ESI): m/z, 1039[M+H]⁺.

### Step 2: Preparation of compound PE-2

1,8-diazabicyclo[5.4.0]undec-7-ene (8.37 µL) was added to a solution of compound PE-2-c (crude, 19.4 mg) in N,N-dimethylformamide (1 mL) in an ice bath. The mixture was stirred in an ice bath for 5 minutes, then allowed to warm to room temperature and stirred for 2 hours. LCMS showed that the starting materials were completely consumed. The resulting mixture was purified by preparative reversed-phase HPLC (preparative chromatograph manufacturer: Shimadzu, model: LC-20AP, column: GS-120-10-C18AP, mobile phase: water (0.225% HCOOH)-acetonitrile, with an elution ratio of acetonitrile increased from 30% to 60%), to afford compound PE-2 (3.11 mg, 39.4% yield, white solid).

LCMS(ESI): m/z, 817[M+H]⁺.

¹H NMR (400 MHz, DMSO-*d*₆) δ 8.25 (s, 1H), 7.92 (s, 1H), 5.03 (dd, *J =* 21.6, 2.6 Hz, 2H), 4.84-4.74 (m, 2H), 4.63 (dt, *J =* 4.2, 2.4 Hz, 1H), 4.55 (t, *J =* 4.2 Hz, 1H), 4.26 (d, *J =* 10.8 Hz, 1H), 4.17 (td, *J* = 10.2, 4.2 Hz, 1H), 4.10 (q, *J* = 4.8, 4.0 Hz, 3H), 4.06-3.99 (m, 1H), 3.83-3.67 (m, 4H), 3.58-3.49 (m, 6H), 3.19-2.99 (m, 6H), 2.84 (dd, *J =* 9.4, 1.8 Hz, 1H), 2.80-2.70 (m, 2H), 2.33-2.17 (m, 6H), 2.17-2.07 (m, 2H), 2.05-1.95 (m, 2H), 1.92 (q, *J* = 6.4, 5.8 Hz, 4H), 1.75-1.58 (m, 6H), 1.48 (dtd, *J =* 19.8, 11.2, 9.8, 5.2 Hz, 3H), 1.31 (ddt, *J =* 15.8, 11.4, 5.0 Hz, 3H), 1.26-1.10 (m, 2H), 1.06-0.98 (m, 3H).

### Preparation Example 3: Preparation of Compound PE-E2H

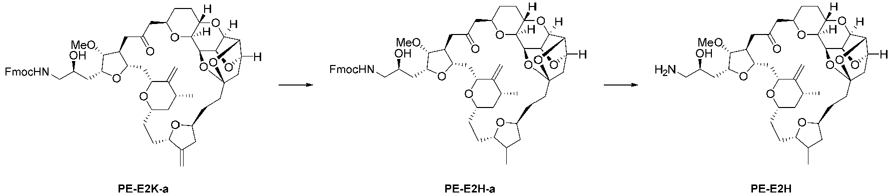

### Step 1: Preparation of compound PE-E2H-a

Wet Pd/C (10 mg) was added to a solution of compound PE-E2K-a (for synthesis of the compound, refer to the preparation example of PE-E2K) (10 mg, 10.50 µmol, 1 eq) in MeOH (2 mL). The reaction solution was stirred at 25°C under hydrogen for 3 hours. LCMS analysis showed that the reaction ended. The mixture was filtered through Celite and concentrated under reduced pressure, then purified by preparative high-performance liquid chromatography (preparative chromatograph manufacturer: Orienda, model: BRIX-2860, column: Welch Xtimate C18 150 × 30 mm × 10 µm, mobile phase: water (0.225% FA)-acetonitrile, with an elution ratio of acetonitrile increased from 50% to 80%), to obtain the compound PE-E2H-a (7 mg, 69.85% yield) as a white solid. LCMS (ESI): m/z, 954.4[M+H]⁺.

### Step 2: Preparation of compound PE-E2H

Et₂NH (5.42 mg 73.36 µmol, 10 eq) was added to the solution of PE-E2H-a (7 mg, 7.34 µmol, 1 eq) in DMF (2 mL), the reaction solution was stirred at 25°C for 2 hours, and the LCMS showed that the starting materials were completely consumed. The mixture was purified by preparative high-performance liquid chromatography (preparative chromatograph manufacturer: Orienda, model: BRIX-2860, column: YMC-Triart Prep C18 150 × 30 mm × 10 µm, mobile phase: water (0.225% FA)-acetonitrile, with an elution ratio of acetonitrile increased from 30% to 60%), to obtain PE-E2H (3.70 mg, 52.03% yield) as a white solid. LCMS (ESI): m/z, 732.4[M+H]⁺.

¹H NMR (400 MHz, DMSO-*d*₆) δ 8.43 (s, 2H), 5.58 (d, *J =* 8.0 Hz, 3H), 4.81 (s, 1H), 4.74 (s, 1H), 4.62 (t, *J =* 4.4 Hz, 1H), 4.56 (t, *J* = 4.4 Hz, 1H), 4.26-4.15 (m, 2H), 4.14-4.07 (m, 3H), 3.89-3.79 (m, 3H), 3.75-3.68 (m, 2H), 3.56 (s, 4H), 3.27 (d, *J =* 3.2 Hz, 6H), 2.89-2.81 (m, 2H), 2.70 (d, *J =* 7.2 Hz, 1H), 2.26-2.14 (m, 4H), 2.05-1.84 (m, 7H), 1.73-1.61 (m, 6H), 1.51-1.44 (m, 2H), 1.30-1.20 (m, 4H), 1.11-1.01 (m, 5H), 0.96-0.86 (m, 3H).

### Preparation Example 4: Preparation of Compound PE-E2K

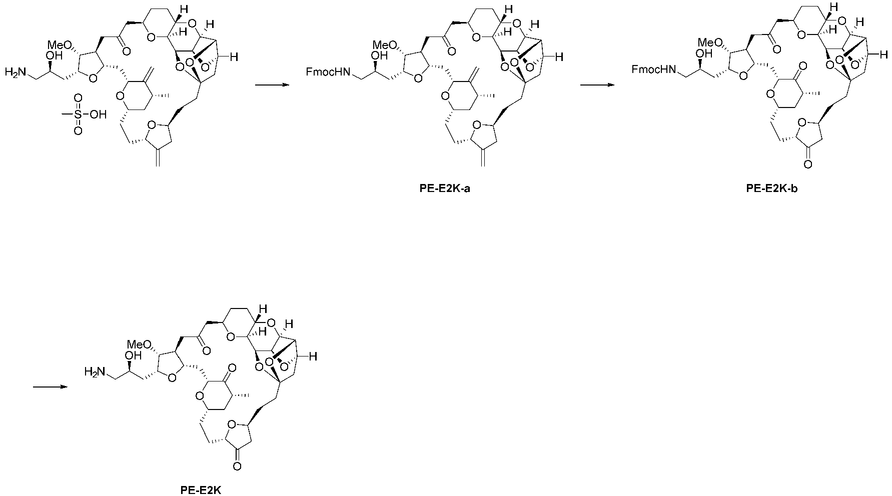

### Step 1: Preparation of compound PE-E2K-a

NaHCO₃ (6.13 mg, 72.64 µmol, 2 eq) and Fmoc-Osu (fluorenylmethyloxycarbonyl succinimide, 13.75 mg, 39.95 µmol, 1.1 eq) were added to the solution of the compound eribulin mesylate (30 mg, 36.32 µmol, 1 eq) in THF (2 mL) at 0°C and the reaction solution was stirred at 25°C for 2 hours. LCMS analysis showed that the reaction ended. The residue was purified by silica gel column chromatography (C18, ISCO, R-40g SepaFlash Silica Flash Column, eluent of 10%-75% water/CH₃CN, 100 mL/min) to afford PE-E2K-a (30 mg, 86.75% yield) as a colorless oil. LCMS (ESI): m/z, 952.6[M+H]⁺.

### Step 2: Preparation of compound PE-E2K-b

Potassium osmate dihydrate (490.97 µg, 1.58 µmol, 0.1 eq) and NaIO₄ (67.73 mg, 315.08 µmol, 20 eq) were added to the solution of the compound PE-E2K-a (15 mg, 15.75 µmol, 1 eq) in THF (tetrahydrofuran, 3 mL) and water (1 mL) at 0°C and the reaction solution was stirred at 25°C for 2 hours. NaIO₄ (67.73 mg, 315.08 µmol, 20 eq) was added to the reaction solution, the reaction solution was stirred overnight at room temperature, and the LCMS analysis showed that the reaction ended. The mixture (combined with the previous batch, 5 mg, small-scale test) was subjected to preparative high-performance liquid chromatography (preparative chromatograph manufacturer: Orienda, model: BRIX-2860, column: Synergi Max-RP 200 × 30mm × 10 µm, mobile phase: water (0.1% TFA)-acetonitrile, with an elution ratio of acetonitrile increased from 40% to 70%), to afford the product PE-E2K-b (16.60 mg, 82.68% yield) as a white solid. LCMS (ESI): m/z, 956.4[M+H]⁺.

### Step 3: Preparation of compound PE-E2K

Et₂NH (6.41 mg, 86.81 µmol, 5 eq) was added to the solution of PE-E2K-b (16.6 mg, 17.36 µmol, 1 eq) in DMF (2 mL), the reaction solution was stirred at 25°C for 1 hour, and the LCMS showed that the starting materials were completely consumed. The mixture was purified by preparative high-performance liquid chromatography (preparative chromatograph manufacturer: Orienda, model: BRIX-2860, column: Welch Xtimate C18 250 × 30 mm × 10 µm, mobile phase: water (0.1% TFA, trifluoroacetic acid)-acetonitrile, with an elution ratio of acetonitrile increased from 10% to 40%), to afford PE-E2K (7.50 mg, 58.86% yield) as a white solid.

LCMS (ESI): m/z, 734.3[M+H]⁺. ¹H NMR (400 MHz, DMSO-*d*₆) δ 7.67 (s, 2H), 5.23 (s, 1H), 4.70-4.53 (m, 2H), 4.46-4.28 (m, 2H), 4.19-4.05 (m, 3H), 3.94-3.67 (m, 5H), 3.61-3.47 (m, 1H), 3.33-3.21 (m, 4H), 3.01-2.70 (m, 4H), 2.65-2.52 (m, 2H), 2.41-2.34 (m, 2H), 2.30-1.87 (m, 8H), 1.85-1.10 (m, 14H), 0.97-0.90 (m, 2H).

### Preparation Example 5: Preparation of Compounds PE-E4HB1 and PE-E4HS1

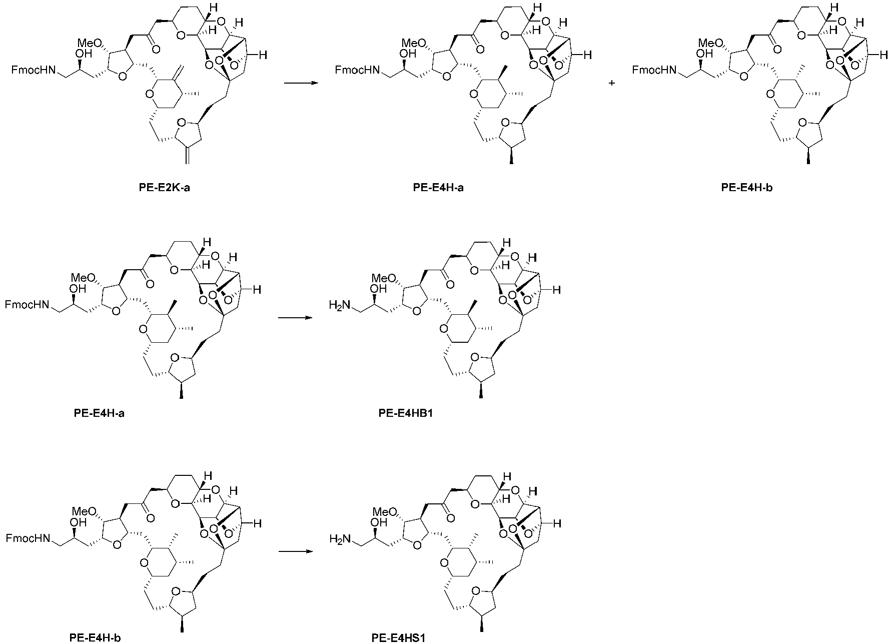

### Step 1: Preparation of compounds PE-E4H-a and PE-E4H-b

Wet Pd/C (10 mg) was added to a solution of compound PE-E2K-a (for synthesis of the compound, refer to the preparation example of PE-E2K) (10 mg, 10.50 µmol, 1 eq) in MeOH (2 mL). The reaction solution was stirred at 25°C under hydrogen overnight. LCMS analysis showed that the reaction ended. The mixture was filtered through Celite and concentrated under reduced pressure, then purified by preparative high-performance liquid chromatography (preparative chromatograph manufacturer: Orienda, model: BRIX-2860, column: YMC-Triart Prep C18 150 × 30 mm × 10 µm, mobile phase: water (0.225% FA)-acetonitrile, with an elution ratio of acetonitrile increased from 60% to 90%), to obtain products PE-E4H-a (7 mg, 69.7% yield, where LCMS (ESI): m/z, 954.4[M+H]⁺) and PE-E4H-b (2.50 mg, 24.89% yield, where LCMS (ESI): m/z, 954.4[M+H]⁺) as white solids.

### Step 2: Preparation of compound PE-E4HB1

Et₂NH (5.41 mg 73.21 µmol, 10 eq) was added to the solution of PE-E4H-a (7 mg, 7.32 µmol, 1 eq) in DMF (2 mL), the reaction solution was stirred at 25°C for 2 hours, and the LCMS showed that the starting materials were completely consumed. The mixture was purified by preparative high-performance liquid chromatography (preparative chromatograph manufacturer: Orienda, model: BRIX-2860, column: YMC-Triart Prep C18 150 × 30 mm × 10 µm, mobile phase: water (0.225% FA)-acetonitrile, with an elution ratio of acetonitrile increased from 20% to 50%), to obtain PE-E4HB1 (4.90 mg, 40.52% yield, dimethanesulfonate) as a white solid. LCMS (ESI): m/z, 734.4[M+H]⁺.

¹H NMR (400 MHz, DMSO-*d*₆) *δ* 8.41 (s, 6H), 5.42 (s, 1H), 5.26 (s, 1H), 4.95 (t, *J* = 9.2 Hz, 1H), 4.62 (s, 1H), 4.11 (s, 2H), 3.65 (t, *J =* 9.0 Hz, 3H), 3.28 (d, *J =* 3.0 Hz, 3H), 2.79 (s, 14H), 2.56 (d, *J=* 15.9 Hz, 1H), 2.29 (s, 3H), 1.99-1.70 (m, 4H), 1.47 (s, 3H), 1.33-1.21 (m, 2H), 1.12 (t, *J=* 7.2 Hz, 19H), 1.07 (d, *J=* 10.4 Hz, 1H), 0.96-0.72 (m, 4H).

### Step 3: Preparation of compound PE-E4HS1

Et₂NH (1.93 mg 26.15 µmol, 10 eq) was added to the solution of PE-E4H-b (2.50 mg, 2.61 µmol, 1 eq) in DMF (2 mL), the reaction solution was stirred at 25°C for 2 hours, and the LCMS showed that the starting materials were completely consumed. The mixture was purified by preparative high-performance liquid chromatography (preparative chromatograph manufacturer: Orienda, model: BRIX-2860, column: YMC-Triart Prep C18 150 × 30 mm × 10 µm, mobile phase: water (0.225% FA)-acetonitrile, with an elution ratio of acetonitrile increased from 20% to 50%), to obtain PE-E4HS1 (1.90 mg, 99.01% yield) as a white solid. LCMS (ESI): m/z, 734.4[M+H]⁺.

### Preparation Example 6: Preparation of Compound PE-5

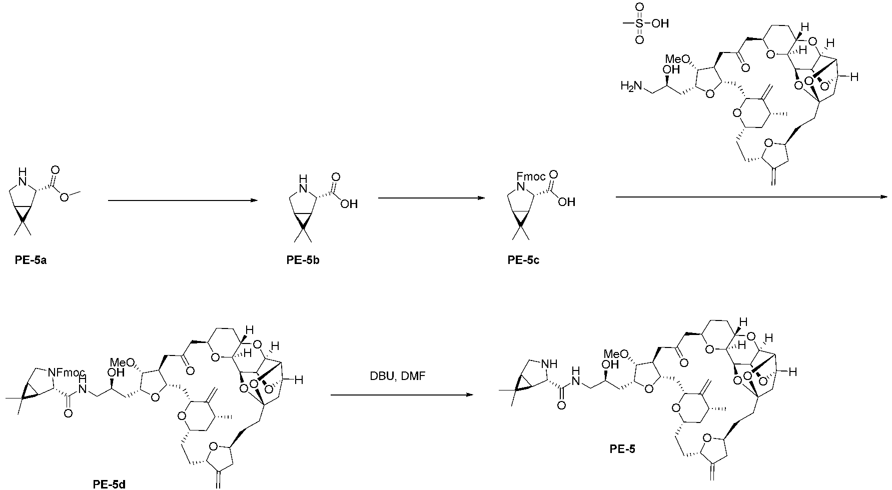

### Step 1: Preparation of (1R,2S,SS)-6,6-dimethyl-3-azabicyclo[3.1.0]hexane-2-carboxylic acid

LiOH.H₂O (163.36 mg, 108.18 µL, 3.89 mmol, 4 eq) was added to a solution of (1R,2S, 5S)-methyl 6,6-dimethyl-3-azabicyclo[3.1.0]hexane-2-carboxylate hydrochloride (200 mg, 972.37 µmol, 1 eq) in THF (4 mL) and H₂O (4 mL), and the mixture was stirred at 25°C for 16 hours. The reaction was monitored by LCMS. The starting materials were completely consumed and a major product was generated. The pH was adjusted to 3-4 with HCl to afford a crude product 6,6-dimethyl-3-(1R,2S,5S)-azabicyclo[3.1.0]hexane-2-carboxylic acid, to be used directly in the next step (140 mg, where the yield was not calculated for the crude product). LCMS (ESI): m/z, 156[M+H]⁺.

### Step 2: Preparation of (1R,2S,5S)-3-(((9H-fluoren-9-yl)methoxy)carbonyl)-6,6-dimethyl-3-azabicyclo[3.1.0]hexane-2-carboxylic acid

Fmoc-OSu (304.29 mg, 217.35 µL, 902.06 µmol, 1 eq) was added to a NaHCO₃ solution (1 mL, adjusted to pH ≥ 7) of 6,6-dimethyl-3-(1R,2S,5S)-azabicyclo[3.1.0]hexane-2-carboxylic acid (140 mg, 902.06 µmol, 1 eq). The mixture was stirred at 25°C for 5 hours. The reaction was monitored by LCMS. The starting material was completely consumed and the major product was formed. The mixture was extracted with ethyl acetate (EA, 5 mL × 3) and H₂O (5 mL × 2). The organic phases were then combined. The pH of the aqueous phase was adjusted to 3-4 with HCl, and the water layer was then extracted with EA (10 mL × 3). The organic layers were then combined and evaporated to dryness. The residue was purified by preparative high-performance liquid chromatography (preparative chromatograph manufacturer: Orienda, model: Lab311-DJ-R7, column: Welch Xtimate C18 250 × 30mm × 10 µm, mobile phase: water (0.225% HCOOH)-acetonitrile, with an elution ratio of acetonitrile increased from 39% to 69%). The product was lyophilized to afford the white solid product (1R,2S,5S)-3-(((9H-fluoren-9-yl) methoxy)carbonyl)-6,6-dimethyl-3-azabicyclo[3.1.0]hexane-2-carboxylic acid (80 mg, 23.50% yield). LCMS (ESI): m/z, 400[M+Na]⁺.

### Step 3: Preparation of compound PE-5d

HATU (6.55 mg, 17.22 µmol, 1.3 eq) and DIPEA (N,N-diisopropylethylamine, 5.14 mg, 6.57 µL, 39.75 µmol, 3 eq) were added to a solution of eribulin mesylate (9.67 mg, 13.25 µmol, 1 eq) and (1R,2S,5S)-3-(((9H-fluoren-9-yl)methoxy)carbonyl)-6,6-dimethyl-3-azabicyclo[3.1.0] hexane-2-carboxylic acid (5 mg, 13.25 µmol, 1 eq) in DMF (5 mL) at 0°C. The mixture was stirred at 25°C for 16 hours. The reaction was monitored by LCMS. The starting material was completely consumed and the major product was formed. The residue was purified preparative high-performance liquid chromatography (preparative chromatograph manufacturer: ISCO, model: Lab311-DJ-R5, column: Phenomenex Luna C18 250 × 30mm × 10 µm, mobile phase: water (0.225% HCOOH)-acetonitrile, with an elution ratio of acetonitrile increased from 57% to 87%) and lyophilized to afford a compound PE-5d (12.50 mg, 86.61% yield) as a white solid. LCMS (ESI): m/z, 1011[M+Na]⁺.

### Step 4: Preparation of compound PE-5

DBU (1,8-diazabicyclo[5.4.0]undec-7-ene, 146.74 µg, 1.44e⁻¹ µL, 963.90 nmol, 0.1 eq) was added to a solution of compound PE-5d (10.50 mg, 9.64 µmol, 1 eq) in DMF (2 mL). The mixture was stirred at 25°C for 1 hour. The reaction was monitored by LCMS. The starting material was completely consumed and the major product was formed. The residue was purified preparative high-performance liquid chromatography (preparative chromatograph manufacturer: ISCO, model: Lab311-DJ-R5, column: Phenomenex Luna C18 250 × 30mm × 10 µm, mobile phase: water (0.225% HCOOH)-acetonitrile, with an elution ratio of acetonitrile increased from 30% to 60%) and lyophilized to obtain a compound PE-5 (1 mg, 11.96% yield, 100% purity) as a white solid.

LCMS (ESI): m/z, 867[M+H]⁺. ¹H NMR (400 MHz, DMSO-d6) δ 8.25 (s, 1H), 8.02 (t, *J =* 5.9 Hz, 1H), 5.03 (d, *J =* 21.3 Hz, 2H), 4.83 (s, 1H), 4.76 (s, 2H), 4.64 (t, *J =* 4.0 Hz, 1H), 4.55 (t, *J* = 4.3 Hz, 1H), 4.26 (d, *J* = 10.8 Hz, 1H), 4.17 (td, *J* = 10.2, 4.1 Hz, 1H), 4.13-4.06 (m, 3H), 4.01 (d, *J =* 9.4 Hz, 1H), 3.77 (ddd, *J =* 13.5, 10.1, 6.7 Hz, 2H), 3.69 (ddd, *J =* 9.3, 5.6, 2.9 Hz, 1H), 3.52 (q, *J =* 11.1, 9.9 Hz, 6H), 3.26 (s, 6H), 3.12 (dt, *J* = 11.4, 5.4 Hz, 2H), 3.04 (dt, *J* = 10.4, 6.2 Hz, 2H), 2.89 - 2.81 (m, 2H), 2.61-2.55 (m, 1H), 2.33 (d, *J* = 14.7 Hz, 2H), 2.14 (d, *J* = 12.0 Hz, 1H), 2.07-1.96 (m, 2H), 1.92 (q, *J =* 6.2, 5.7 Hz, 4H), 1.66 (tq, *J =* 13.4, 9.2, 6.5 Hz, 6H), 1.56-1.44 (m, 2H), 1.41 (d, *J* = 7.1 Hz, 1H), 1.38-1.26 (m, 3H), 1.24-1.14 (m, 2H), 1.03 (d, *J* = 6.5 Hz, 3H), 0.98 (s, 6H).

### Preparation Example 7: Preparation of Compound PE-7

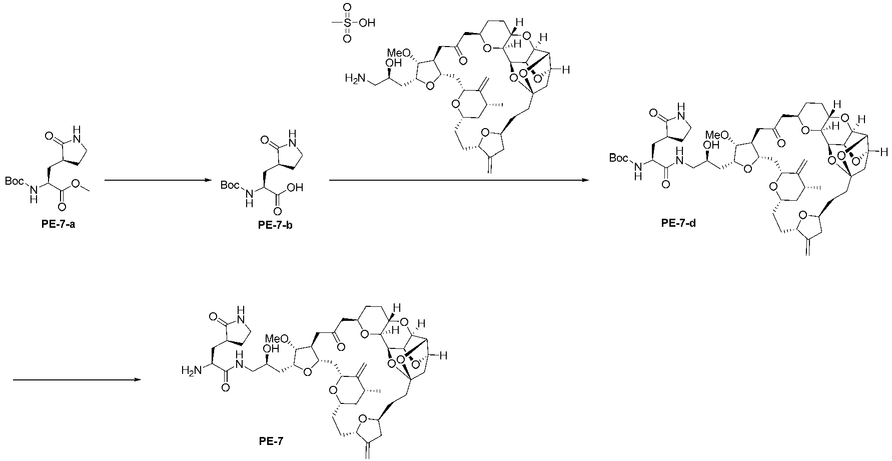

### Step 1: Preparation of (S)-2-((tert-butoxycarbonyl)amino)-3-((S)-2-oxopyrrolidin-3-yl) propanoic acid

Methyl (S)-2-((tert-butoxycarbonyl)amino)-3-((S)-3-oxopyrrolidin-3-yl)propanoate (300 mg, 1.0 eq) was dissolved in a mixture of tetrahydrofuran (3 mL) and water (3 mL). Lithium hydroxide monohydrate (132 mg, 3.0 eq) was added, and then the reaction mixture was stirred and allowed to react at 25°C for 3 hours. LCMS analysis showed that the reaction ended. Aftertreatment: The reaction solution was diluted with purified water (10 mL), neutralized with 1 M dilute hydrochloric acid to pH of 5, and extracted twice with a mixture of dichloromethane and methanol (10:1 by volume). The organic phases were combined, washed with saturated brine, dried, and then concentrated to obtain the crude product (S)-2-((tert-butoxycarbonyl)amino)-3-((S)-2-oxopyrrolidin -3-yl)propanoic acid, to be used directly in the next step.

LCMS: m/z = 295.2 [M+Na]⁺; Rt = 0.761min.

### Step 2: Preparation of compound PE-7-d

Eribulin mesylate (10 mg, 1.0 eq, ChemExpress) and (S)-2-((tert-butoxycarbonyl)amino)-3-((S)-2-oxopyrrolidin-3-yl)propanoic acid (3.73 mg, 1.0 eq) were dissolved in DMF (2 mL). DIPEA (6.79 µL, 3.0 eq) and HATU (6.77 mg, 1.3 eq) were added, and the reaction mixture was then stirred and allowed to react at 25°C for 16 hours. LCMS analysis showed that the starting material was completely consumed. Aftertreatment: The reaction solution was filtered, and the filtrate was subjected to preparative high-performance liquid chromatography (preparative chromatograph manufacturer: Orienda, model: BRIX-2860, column: Phenomenex Luna C18 250 × 30mm × 10 µm, mobile phase: water (0.225% HCOOH),-acetonitrile, with an elution ratio of water increased from 30% to 60%), to obtain compound PE-7-d (10 mg, 74% yield) as a white solid.

LCMS: m/z = 984.5 [M+H]⁺; Rt = 2.040 min.

### Step 3: Preparation of compound PE-7

Compound PE-7-d (10 mg, 1.0 eq) was dissolved in anhydrous dichloromethane (2 mL). Trifluoroacetic acid (280 µL) was added while cooled under an ice bath, and the mixture was warmed naturally to 25°C, stirred, and allowed to react for 16 hours. LCMS analysis showed that the starting material was completely consumed. Aftertreatment: The reaction solution was concentrated to dryness, and the residue was dissolved in DMF. The resulting mixture was then purified by preparative high-performance liquid chromatography (preparative chromatograph manufacturer: Orienda, model: BRIX-2860, column: Phenomenex Luna C18 250 × 50 mm × 10 µm, mobile phase: water (0.225% HCOOH)-acetonitrile, with an elution ratio of acetonitrile increased from 30% to 60%), to obtain compound PE-7 (5.68 mg, 79% yield) as a white solid. LCMS : m/z = 884.5 [M+H]⁺; Rt = 2.040 min.

¹H NMR (400 MHz, DMSO-*d⁶*) *δ* 8.05 (t, *J =* 5.9 Hz, 1H), 7.66 (s, 2H), 6.20 (d, *J =* 3.0 Hz, 1H), 5.95 (d, *J* = 3.0 Hz, 1H), 4.97-4.83 (m, 3H), 4.81 (s, 2H), 4.75 (s, 3H), 4.67-4.63 (m, 2H), 4.32 (dd, *J =* 19.2, 7.8 Hz, 3H), 4.06 (q, *J* = 6.8, 6.3 Hz, 1H), 4.01 (d, *J* = 3.0 Hz, 1H), 3.88-3.75 (m, 3H), 3.70 (dd, *J* = 8.4, 3.9 Hz, 3H), 3.61 (d, *J* = 4.6 Hz, 1H), 3.21 (s, 4H), 3.17-3.07 (m, 4H), 2.98 (dd, *J* = 9.3, 2.1 Hz, 1H), 2.76 (dt, *J =* 14.5, 5.7 Hz, 3H), 2.35 (s, 1H), 2.31-2.16 (m, 3H), 2.13 (td, *J =* 15.9, 15.4, 6.8 Hz, 1H), 1.96 (dd, *J* = 5.8, 3.1 Hz, 1H), 1.96-1.89 (m, 1H), 1.89-1.81 (m, 2H), 1.76 (dt, *J =* 8.4, 4.2 Hz, 2H), 1.74-1.66 (m, 7H), 1.69-1.61 (m, 1H), 1.60 (d, *J =* 8.2 Hz, 3H), 1.59-1.43 (m, 1H), 1.42 (dd, *J* = 11.1, 5.1 Hz, 2H), 1.31 (s, 2H), 1.04 (d, *J =* 6.3 Hz, 4H), 0.94 (q, *J =* 12.0 Hz, 1H).

### Preparation Example 8: Preparation of Compound PE-10

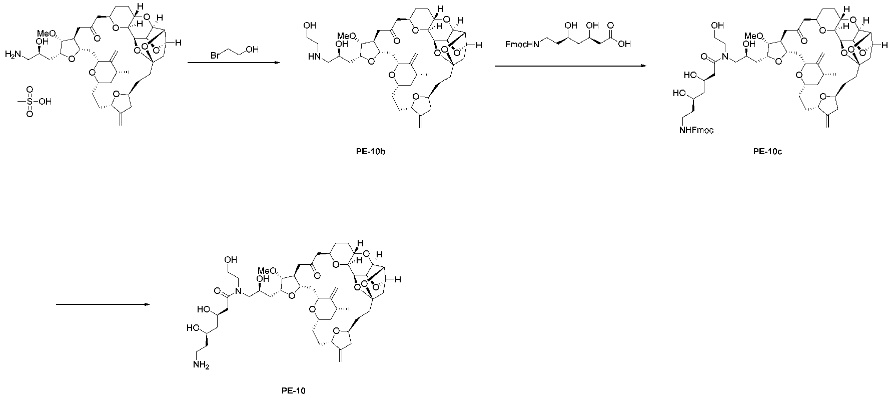

### Step 1: Preparation of compound PE-10b

Potassium carbonate (5.02 mg, 36.3 µmol, 3 eq) was added to a solution of eribulin mesylate (10 mg, 12.1 µmol, 1 eq) and bromoethanol (2.27 mg, 18.2 µmol, 1.5 eq) in N,N-dimethyl formamide (1.5 mL). The mixture was maintained in an oil bath at 25°C for 4 hours. LCMS indicated formation of the product. The mixture was filtered and the residue was purified by preparative high-performance liquid chromatography (preparative chromatograph manufacturer: Shimadzu, model: LC-20AP, column: Welch Xtimate C18 250 × 30mm × 10 µm, mobile phase: water (0.225% HCOOH)-acetonitrile, with an elution ratio of acetonitrile increased from 20% to 50%), to obtain PE-10b (7.8 mg, 73.5% yield). LCMS (ESI): m/z, 774[M+H]⁺.

### Step 2: Preparation of compound PE-10c

N,N,N',N'-tetramethyl-O-(7-azabenzotriazol-1-yl)uronium hexafluorophosphate (5.75 mg, 15.12 µmol, 1.5 eq) and N,N-diisopropylethylamine (3.33 µL, 20.16 µmol, 2 eq) were added to a solution of compound PE-10b (7.8 mg, 10.08 µmol, 1 eq) and (3R,5R)-7-((((9H-fluoren-9-yl) methoxy)carbonyl)amino)-3,5-dihydroxyheptanoic acid (4.83 mg, 12.09 µmol, 1.2 eq; for the synthesis of this intermediate, refer to the preparation example of PE-1) in N,N-dimethylformamide (2 mL). The mixture was stirred at room temperature for 2 hours. LCMS analysis showed complete consumption of the starting material. The mixture was concentrated under an oil pump to dryness to afford the crude product (22.5 mg, the yield not calculated for the crude product). LCMS (ESI): m/z, 1155[M+H]⁺.

### Step 3: Preparation of compound PE-10

1,8-diazabicyclo[5.4.0]undec-7-ene (4.5 µL, 30.24 µmol, 3 eq) was added to a solution of compound PE-10c (22.5 mg, 51.7% purity, 10.08 µmol, 1 eq) in N,N-dimethylformamide (2 mL). The mixture was stirred at room temperature for 1 hour. LCMS analysis showed complete consumption of the starting material. After filtration, the residue was purified by preparative high-performance liquid chromatography (preparative chromatograph manufacturer: Shimadzu, model: LC-20AP, column: GS-120-10-C18AP, mobile phase: water (0.225% HCOOH)-acetonitrile, with an elution ratio of acetonitrile increased from 20% to 50%), to afford compound PE-10 (7.8 mg, 73.5% yield) as a white solid.

LCMS (ESI): m/z, 933[M+H]⁺. ¹H NMR (400 MHz, DMSO-*d*₆) δ 8.42 (s, 1H), 7.81 (s, 1H), 5.06 (s, 1H), 5.00 (s, 1H), 4.84 (s, 1H), 4.76 (s, 1H), 4.64 (s, 1H), 4.56 (t, *J* = 4.2 Hz, 1H), 4.26 (d, *J* = 10.4 Hz, 1H), 4.19 (d, *J* = 13.2 Hz, 1H), 4.10 (d, *J* = 4.8 Hz, 3H), 4.06-3.93 (m, 2H), 3.85-3.64 (m, 5H), 3.51 (s, 6H), 3.27 (d, *J =* 7.2 Hz, 8H), 3.06 (s, 2H), 2.87-2.70 (m, 5H), 2.58 (d, *J* = 16.2 Hz, 1H), 2.34 (s, 1H), 2.24 (dd, *J* = 16.8, 9.8 Hz, 5H), 2.14 (d, *J* = 12.0 Hz, 2H), 2.06-1.96 (m, 2H), 1.93 (s, 4H), 1.67 (t, *J* = 12.2 Hz, 6H), 1.50 (s, 6H), 1.35 (d, *J =* 18.2 Hz, 3H), 1.26-1.12 (m, 2H), 1.04 (d, *J* = 6.4 Hz, 3H).

### Preparation Example 9: Preparation of Compound PE-12

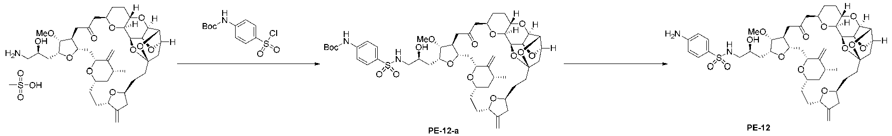

### Step 1: Preparation of compound PE-12-a

N,N-diisopropylethylamine (1.5 µL, 9.08 µmol, 1.5 eq) and N-BOC-4-aminobenzene sulfonyl chloride (1.94 mg, 6.66 µmol, 1.1 eq) were added to a solution of eribulin mesylate (5 mg, 6.05 µmol, 1 eq) in dichloromethane (1 mL) under an ice bath. The mixture was stirred at room temperature for 2 hours. LCMS analysis showed complete consumption of the starting material. The mixture was concentrated under an oil pump to afford the crude product PE-12-a (13 mg, where the yield was not calculated for the crude product, and a theoretical mass was 9.6 mg).

LCMS (ESI): m/z, 1007[M+Na]⁺.

### Step 2: Preparation of compound PE-12

Trifluoroacetic acid (0.1 mL) was added to a solution of compound PE-12-a (7.68 mg, 4.84 µmol, 1 eq) in dichloromethane (1 mL) under an ice bath and the resulting mixture was stirred for 2 hours at room temperature. LCMS indicated that the starting materials were completely consumed. After being concentrated, the reaction solution was dissolved in N,N-dimethylformamide, the mixture was filtered, and the filtrate was purified by high-performance liquid chromatography (preparative chromatograph manufacturer: Orienda, model: BRIX-2860, column: Synergi Max-RP 250 × 40 mm × 10 µm, mobile phase: water (0.225% HCOOH)-acetonitrile, with an elution ratio of acetonitrile increased from 30% to 60%), to afford compound PE-12 (0.52 mg, 12.14% yield) as a white solid.

LCMS (ESI): m/z, 907[M+Na]⁺.

### Preparation Example 10: Preparation of Compound PE-16

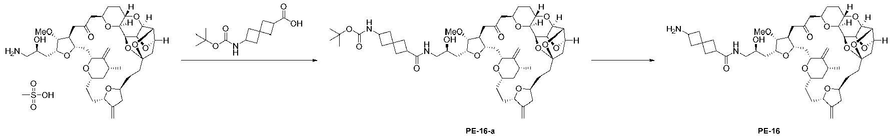

### Step 1: Preparation of compound PE-16-a

N,N-diisopropylethylamine (2 µL, 12.11 µmol, 2 eq) and N,N,N',N'-tetramethyl-O-(7-azabenzotriazol-1-yl)uronium hexafluorophosphate (2.76 mg, 7.26 µmol, 1.2 eq) were added to the solution of eribulin mesylate (5 mg, 6.05 µmol, 1 eq) and 6-((tert-butoxycarbonyl)amino)spiro [3.3]heptane-2-carboxylic acid (1.85 mg, 7.26 µmol, 1.2 eq) in N,N-dimethylformamide (1 mL) under an ice bath, and then the reaction solution was stirred at room temperature for 2 hours. LCMS analysis showed complete consumption of the starting material. The reaction solution was purified by preparative high-performance liquid chromatography (preparative chromatograph manufacturer: Orienda, model: BRIX-2860, column: YMC-Triart Prep C18 150 × 30 mm × 10 µm, mobile phase: water (0.225% HCOOH)-acetonitrile, with an elution ratio of acetonitrile increased from 40% to 70%), to afford a product PE-16-a (4.60 mg, 78.5% yield) as a white solid.

LCMS (ESI): m/z, 967[M+H]⁺.

### Step 2: Preparation of compound PE-16

Trifluoroacetic acid (0.1 mL) was added to a solution of compound PE-16-a (4.60 mg, 4.76 µmol, 1 eq) in dichloromethane (1 mL) under an ice bath. The mixture was then stirred at room temperature for 2 hours. LCMS analysis showed complete consumption of the starting material. The mixture was concentrated via an oil pump. The residue was purified by preparative high-performance liquid chromatography (preparative chromatograph manufacturer: Orienda, model: BRIX-2860, column: Welch Xtimate C18 250 × 30mm × 10 µm, mobile phase: water (0.225% HCOOH)-acetonitrile, with an elution ratio of acetonitrile increased from 30% to 60%), to obtain product PE-16 (1.66 mg, 39.3% yield) as a white solid.

LCMS (ESI): m/z, 867[M+H]⁺.

### Preparation Example 11: Preparation of Compound PE-18

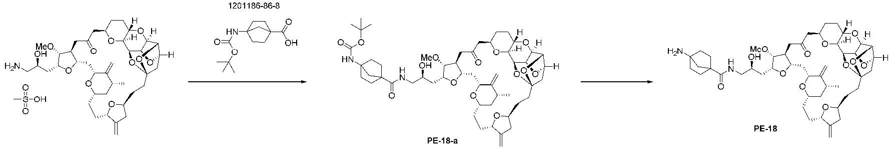

### Step 1: Preparation of compound PE-18-a

N,N-diisopropylethylamine (4.69 mg, 6 µL, 36.32 µmol, 3 eq), 2-(7-azabenzotriazol)-N,N,N',N'-tetramethyluronium hexafluorophosphate (5.52 mg, 14.53 µmol, 1.2 eq) and 4-((tert-butoxycarbonyl)amino)bicyclo[2.2.1]heptane-1-carboxylic acid (2.55 mg, 10 µmol, 1 eq) were added sequentially to a solution of eribulin mesylate (10 mg, 12.11 µmol, 1 eq) in N,N-dimethylformamide (1 mL) at room temperature. The mixture was stirred at 20°C for 3 hours. LCMS analysis showed complete consumption of the starting material. The reaction solution was purified by preparative high-performance liquid chromatography (preparative chromatograph manufacturer: Orienda, model: BRIX-2860 (R1, 4, 5, 6, and 7), column: Phenomenex Luna C18 250 × 50 mm × 10 µm, mobile phase: water (0.225% HCOOH)-acetonitrile, with an elution ratio of acetonitrile increased from 30% to 60%) and lyophilized, to afford a product PE-18-a (6 mg, 6.20 µmol, 51% yield) as a white solid.

LCMS (ESI): m/z, 967.6[M+H]⁺.

### Step 2: Preparation of compound PE-18

At room temperature, PE-18-a (6 mg, 6.20 µmol, 1 eq) was added to dichloromethane (1 mL) and trifluoroacetic acid (0.1 mL), and the mixture was stirred at 20°C for 1 hour. LCMS analysis showed that the reaction ended. The reaction solution was purified by preparative high-performance liquid chromatography (preparative chromatograph manufacturer: Orienda, model: BRIX-2860, column: Phenomenex Luna C18 250×50 mm×10 µm, mobile phase: water (0.225% HCOOH)-acetonitrile, with an elution ratio of water increased from 30% to 60%) and lyophilized, to afford a product PE-18 (3.09 mg, 3.38 µmol, 55% yield) as a white solid.

LCMS (ESI): m/z, 867.6[M+H]⁺.

### Preparation Example 12: Preparation of Compound PE-21A

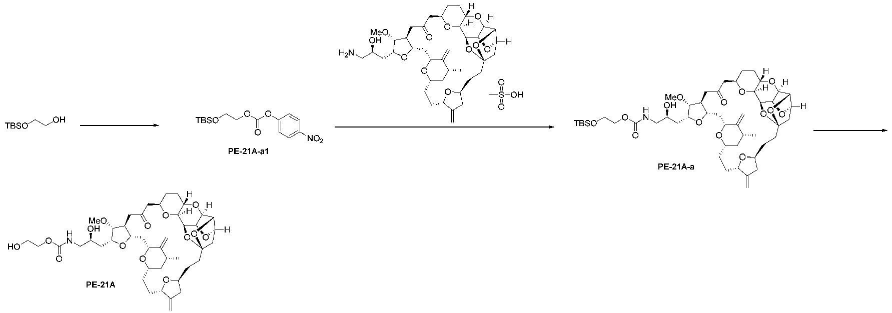

### Step 1: Preparation of compound 2-[(tert-butyldimethylsilyl)oxy]ethyl 4-nitrophenyl carbonate

DIPEA (6.60g, 8.44 mL, 51.04 mmol, 3 eq) was added to a solution of tert-butyldimethylsilyl ethanol (3g, 17.01 mmol, 1 eq) and bis(4-nitrophenyl) carbonate (10.35g, 6.90 mL, 34.03 mmol, 2 eq) in DMF (20 mL). The reaction solution was allowed to react at 25°C for 15 hours. LCMS analysis showed complete consumption of the starting material. The mixture was extracted with dichloromethane (10 mL × 3). The organic phases were combined and washed with water (20 mL × 2) and saturated brine (10 mL), dried over anhydrous sodium sulfate, filtered, and concentrated. The residue was purified by silica gel column chromatography (eluents: petroleum ether: ethyl acetate = 5:1), to afford the compound 2-[(tert-butyldimethylsilyl)oxy]ethyl 4-nitrophenyl carbonate (4.52g, 77.73% yield) as a yellow oil. LCMS (ESI): m/z, 342[M+H]⁺.

### Step 2: Preparation of compound PE-21A-a

DIPEA (N,N-diisopropylethylamine, 1.42 mg, 1.81 µL, 10.96 µmol, 2 eq) was added to a solution of 2-((tert-butyldimethylsilyl)oxy)ethyl 4-nitrophenyl carbonate (2.25 mg, 6.58 µmol, 1.2 eq) and eribulin mesylate (4 mg, 5.48 µmol, 1 eq) in DMF (1 mL) at 0°C. The reaction mixture was allowed to react at 25°C for 15 hours. TLC indicated complete consumption of the starting material. The mixture was evaporated to dryness and used directly in the next step to afford the compound PE-21A-a (5.11 mg, where the yield was not calculated for the crude product) as a brown oil. LCMS (ESI): m/z, 945[M+H]⁺.

### Step 3: Preparation of compound PE-21A

TFA (625 µg, 80 µL, 5.48 µmol, 1 eq) was added to a solution of PE-21A-a (5.11 mg, 5.48 µmol, 1 eq) in DCM (dichloromethane, 800 µL) at 0°C. The mixture was allowed to react at 0°C to 25°C for 2 hours. LCMS analysis showed complete consumption of the starting material. The reaction solution was purified by preparative high-performance liquid chromatography (preparative chromatograph manufacturer: Orienda, model: BRIX-2860, column: Welch Xtimate C18 150 × 30 mm × 10 µm, mobile phase: water (0.225% HCOOH)-acetonitrile, with an elution ratio of acetonitrile increased from 30% to 60%), to afford compound PE-21A (600 µg, 13.38% yield, 100% purity) as a white solid. LCMS (ESI): m/z, 840[M+Na]⁺.

### Preparation Example 13: Preparation of Compound PE-21B

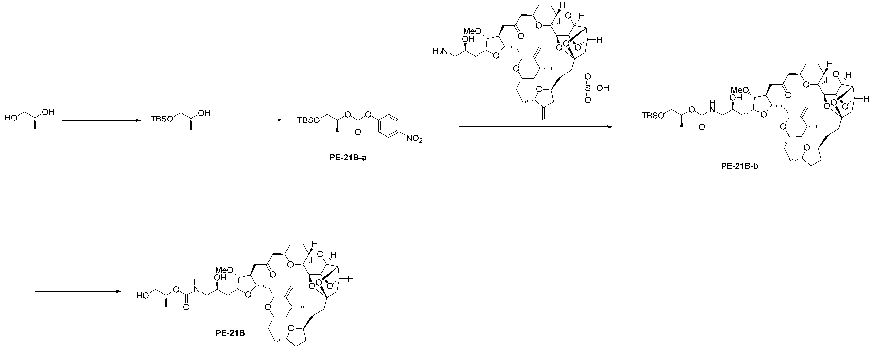

### Step 1: Preparation of compound (S)-1-((tert-butyldimethylsilyl)oxy)propan-2-ol

TBSCl (tert-butyldimethylsilyl chloride, 2.18g, 2.50 mL, 14.46 mmol, 1.1 eq) and imidazole (894.73 mg, 813.39 µL, 13.14 mmol, 1 eq) were added to a solution of (S)-propane-1,2-diol (1g, 13.14 mmol, 1 eq) in DCM (10 mL) at 0°C. After the addition, the reaction mixture was allowed to react at 25°C for 16 hours. The reaction solution was added into ice-cold aqueous sodium bicarbonate solution (60 mL) and the mixture was then stirred for 2 minutes and extracted with dichloromethane (60 mL × 3). The organic phases were combined, washed with brine, dried over anhydrous sodium sulfate, and filtered. The filtrate was concentrated to afford the compound (S)-1-((tert-butyldimethylsilyl)oxy)propan-2-ol (2.30g, 91.94% yield) as a colorless oil.

¹H NMR (400 MHz, Chloroform-*d*) δ 3.82-3.78 (m, 1H), 3.59-3.56 (m, 1H), 3.36-3.31 (m, 1H), 2.52 (brs, 1H), 1.11 (d, *J* = 8.0 Hz, 3H), 0.90 (s, 9H), 0.06 (s, 6H).

### Step 2: Preparation of compound PE-21B-a

Bis(4-nitrophenyl) carbonate (2.43g, 1.62 mL, 8 mmol, 2 eq) and DIPEA (1.55g, 1.98 mL, 12 mmol, 3 eq) were added to a solution of (S)-1-((tert-butyldimethylsilyl)oxy)propan-2-ol (761.40 mg, 4 mmol, 1 eq) in DMF (10 mL) at 0°C. The reaction solution was allowed to react at 25°C for 16 hours. The LCMS showed that a new compound was generated. The reaction solution was evaporated to dryness, to afford the residue. The residue was purified by silica gel column chromatography (eluents: ethyl acetate/hexane = 1:30 to 1:15) to afford PE-21B-a (900 mg, 63.30% yield) as a pale yellow oil.

¹H NMR (400 MHz, DMSO-*d⁶*) δ 8.32 (d, *J* = 8.0 Hz, 2H), 7.51 (d, *J* = 8.0 Hz, 2H), 4.88-4.86 (m, 1H), 3.78-3.66 (m, 2H), 1.28 (d, *J* = 8.0 Hz, 3H), 0.86 (s, 9H), 0.06 (s, 6H).

### Step 3: Preparation of compound PE-21B-b

Eribulin mesylate (5.66 mg, 6.85 µmol, 1 eq), DIPEA (2.66 mg, 3.40 µL, 20.55 µmol, 3 eq) and HOBt (1-hydroxybenzotriazole, 925.61 µg, 6.85 µmol, 1 eq) were added to a solution of compound PE-21B-a (2.43 mg, 6.85 µmol, 1 eq) in DMF (1 mL) at 0°C. The reaction solution was allowed to react at 25°C for 1 hour. The LCMS analysis showed that the reaction ended. The reaction solution was concentrated to afford the crude product PE-21B-b (7 mg, crude) as a yellow oil.

LCMS (ESI): m/z, 968.7 [M+Na] ⁺, Rt = 2.46 min.

### Step 4: Preparation of compound PE-21B

Trifluoroacetic acid (0.1 mL) was added to a solution of compound PE-21B-b (6.48 mg, 6.85 µmol, 1 eq) in DCM (1 mL) at room temperature. The reaction solution was allowed to react at 25°C for 1 hour. The LCMS analysis showed that the reaction ended. The reaction solution was concentrated, and the residue was purified by preparative high-performance liquid chromatography (preparative chromatograph manufacturer: Orienda, model: BRIX-2860, column: Welch Xtimate C18 150 × 30 mm × 10 µm, mobile phase: water (0.225% HCOOH)-acetonitrile, with an elution ratio of acetonitrile increased from 30% to 60%) and lyophilized, to afford PE-21B (360 µg, 6.32% yield, 96.6% purity) as a white solid.

LCMS (ESI): m/z, 854.5 [M+Na]⁺, Rt = 2.153 min. HPLC: Rt = 5.683 min.

### Preparation Example 14: Preparation of Compound PE-21C

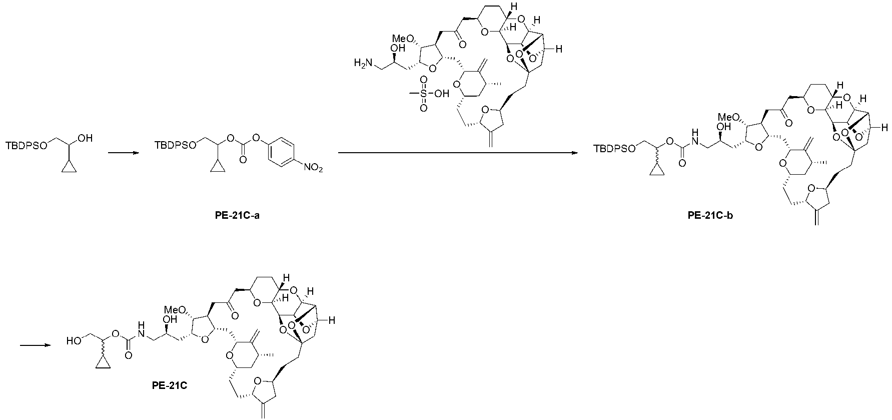

### Step 1: Preparation of compound PE-21C-a

NPC (4-nitrophenyl chloroformate, 2.68g, 8.81 mmol, 6 eq) and DIPEA (1.14g, 8.81 mmol, 6 eq) were added to a solution of 2-((tert-butyldiphenylsilyl)oxy)-1-cyclopropylethan-1-ol (500 mg, 1.47 mmol, 1 eq; for synthesis of the compound, refer to the Example of LA-31B) in DMF (20 mL). The reaction solution was stirred at room temperature for 2 hours. LCMS analysis showed that the reaction ended. The reaction solution was evaporated to dryness under reduced pressure. DCM (50 mL) and water (100 mL) were added to the mixture. The mixture was extracted with DCM (50 mL × 3). The organic phases were combined and washed with saturated brine (100 mL), dried over anhydrous Na₂SO₄, filtered, and evaporated to dryness. The residue was purified by silica gel column chromatography (eluents: ethyl acetate/petroleum ether = 0:1 to 1:5) to afford the product PE-21C-a (426 mg, 57.38% yield) as a colorless oil.

¹H NMR (400 MHz, CDCl₃) δ 8.29-8.25 (m, 2H), 7.72-7.68 (m, 4H), 7.46-7.37 (m, 6H), 7.36-7.31 (m, 2H), 4.34-4.27 (m, 1H), 3.91 (d, *J* = 5.6 Hz, 2H), 1.09-1.05 (m, 10H), 0.64-0.49 (m, 3H), 0.29-0.21 (m, 1H).

### Step 2: Preparation of compound PE-21C-b

DIPEA (4.69 mg, 36.32 µmol, 3 eq) and HOBT (1.64 mg, 12.11 µmol, 1 eq) were added to the solution of eribulin mesylate (10 mg, 12.11 µmol, 1 eq) and PE-21C-a (7.35 mg, 14.53 µmol, 1.2 eq) in DMF (2 mL), and the reaction solution was stirred for 2 hours at room temperature. LCMS analysis showed that the reaction ended. The reaction solution was evaporated to dryness under reduced pressure to afford the product PE-21C-b (13.27 mg, where the yield was not calculated for the crude product) as a colorless oil. LCMS (ESI): m/z, 1118.8[M+Na]⁺, Rt=3.950 min.

### Step 3: Preparation of compound PE-21C

Triethylamine trihydrofluoride (0.04 mL) was added to a solution of compound PE-21C-b (12.52 mg, crude) in DMF (2 mL) at 0°C. The reaction solution was stirred at 65°C for 2.5 hours. LCMS analysis showed complete consumption of the starting material. The mixture was purified by preparative high-performance liquid chromatography (preparative chromatograph manufacturer: Orienda, model: BRIX-2860-R6, column: Welch Xtimate C18 250 × 30mm × 10 µm, mobile phase: water (0.225% HCOOH)-acetonitrile, with an elution ratio of water increased from 30% to 60%), to afford PE-21C (4.2 mg, 42.87% yield) as a white solid. LCMS (ESI): m/z, 880.7[M+Na]⁺, Rt=1.985 min.

¹H NMR (400 MHz, DMSO-*d*₆) δ 6.93-6.79 (m, 1H), 5.10-4.96 (m, 2H), 4.87-4.49 (m, 5H), 4.33-3.98 (m, 6H), 3.86-3.64 (m, 3H), 3.57-3.42 (m, 4H), 3.26 (s, 6H), 3.02-2.54 (m, 7H), 2.35-1.82 (m, 12H), 1.78-0.86 (m, 17H), 0.51-0.18 (m, 4H).

### Preparation Example 15: Preparation of Compound PE-21D

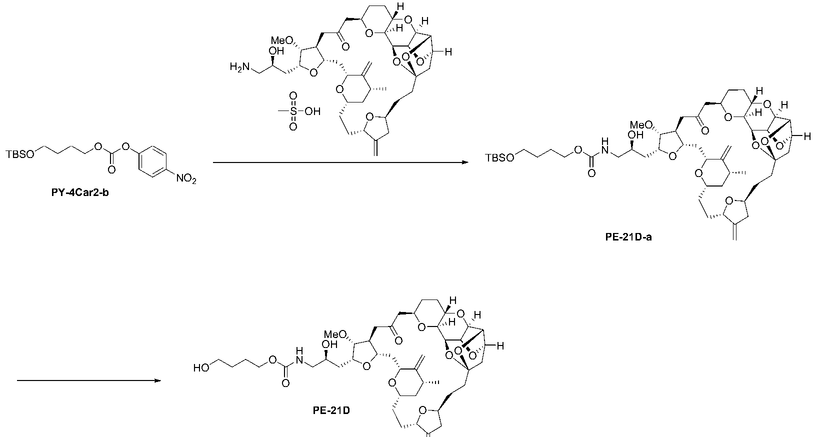

### Step 1: Preparation of compound PE-21D-a

DIPEA (4.69 mg, 36.32 µmol, 3 eq) and HOBt (1.64 mg, 12.11 µmol, 1 eq) were added to the solution of eribulin mesylate (10 mg, 12.11 µmol, 1 eq) and PY-4Car2-b (5.82 mg, 15.74 µmol, 1.3 eq) in THF (20 mL), and the reaction solution was stirred for 2.5 hours at room temperature. LCMS analysis showed that the reaction ended. The reaction solution was evaporated to dryness under reduced pressure to afford the product PE-21D-a (11.60 mg, where the yield was not calculated for the crude product) as a colorless oil. LCMS (ESI): m/z, 960.9[M+H]⁺, Rt=1.128min.

### Step 2: Preparation of compound PE-21D

TFA (0.4 mL) was added to a solution of compound PE-21D-a (11.60 mg, crude) in DCM (2 mL) at 0°C. The reaction solution was stirred at room temperature for 2 hours. LCMS analysis showed complete consumption of the starting material. The reaction solution was evaporated to dryness under reduced pressure. The mixture was purified by preparative high-performance liquid chromatography (preparative chromatograph manufacturer: Orienda, model: BRIX-2860, column: Welch Xtimate C18 200 × 30 mm × 10 µm, mobile phase: water (0.225% HCOOH)-acetonitrile, with an elution ratio of acetonitrile increased from 30% to 60%), to afford PE-21D (1.40 mg, 13.70% yield) as a white solid. LCMS (ESI): m/z, 808.4[M+H]⁺, Rt=2.128min.

### Preparation Example 16: Preparation of Compound PE-21DA

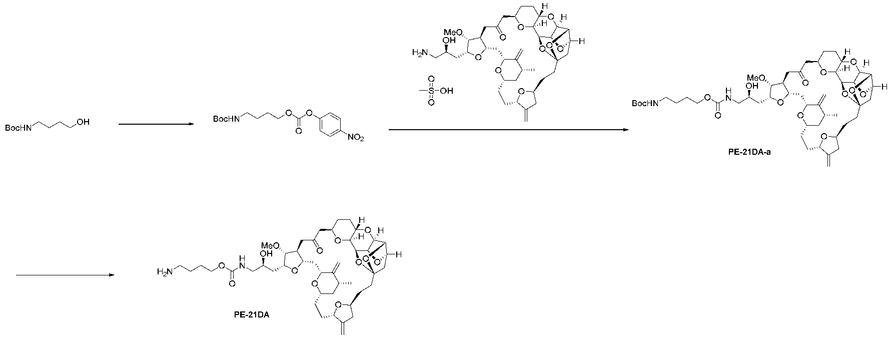

### Step 1: Preparation of 1-{[(4-{[(tert-butoxy)carbonyl]amino}butoxy)carbonyl]oxy}-4-nitrobenzene

Bis(4-nitrophenyl) carbonate (642.98 mg, 428.65 µL, 2.11 mmol, 4 eq) and DIPEA (409.74 mg, 523.97 µL, 3.17 mmol, 6 eq) were added to a solution of 4-(Boc-amino)-1-butanol (100 mg, 528.40 µmol, 1 eq) in DMF (5 mL). The reaction solution was allowed to react at 33°C for 1 hour. LCMS analysis indicated the reaction ended. The residue was purified by silica gel column chromatography (C18, ISCO, R-120g SepaFlash Silica Flash Column, eluents: 10%-60% water/CH₃CN, 60 mL/min) to afford the compound 1-{[(4-{[(tert-butoxy)carbonyl]amino}butoxy) carbonyl]oxy}-4-nitrobenzene as a white solid (131 mg, 69.96% yield).

LCMS (ESI): m/z, 255[M-100+H]⁺. ¹H NMR (400 MHz, Chloroform-*d*) δ 8.29 (d, *J* = 9.1 Hz, 2H), 7.39 (d, *J* = 9.2 Hz, 2H), 4.58 (s, 1H), 4.31 (t, *J* = 6.5 Hz, 2H), 3.19 (t, *J* = 6.7 Hz, 2H), 1.79 (dt, *J* = 8.8, 6.6 Hz, 2H), 1.66 (d, *J* = 7.3 Hz, 2H), 1.45 (s, 9H).

### Step 2: Preparation of compound PE-21DA-a

DIPEA (5.31 mg, 6.79 µL, 41.10 µmol, 3 eq) and HOBT (1.85 mg, 1.23 µL, 13.70 µmol, 1 eq) were added to a solution of 1-{[(4-{[(tert-butoxy)carbonyl]amino}butoxy)carbonyl]oxy}-4-nitrobenzene (9.71 mg, 27.40 µmol, 2 eq) and eribulin mesylate (10 mg, 13.70 µmol, 1 eq) in THF (1 mL) at 0°C. The reaction solution was allowed to react at 35°C for 1 hour. LCMS then indicated complete consumption of the starting material. The mixture was evaporated to dryness and used directly in the next step. LCMS (ESI): m/z, 945[M+H]⁺.

### Step 3: Preparation of compound PE-21DA

At 0°C, TFA (614 mg, 400 µL, 5.39 mmol, 393.0275 eq) was added to a solution of PE-21DA-a (12.95 mg, 13.70 µmol, 1 eq) in DCM (1 mL). The reaction solution was allowed to react at 0°C for 90 minutes. LCMS indicated complete consumption of the starting material. The reaction solution was evaporated to dryness to afford the crude product. The crude product was purified by preparative high-performance liquid chromatography (preparative chromatograph manufacturer: Shimadzu, model: LC-20AP, column: Phenomenex Luna C18 150 × 25 mm × 10 µm, mobile phase: water (0.225% HCOOH)-acetonitrile, with an elution ratio of acetonitrile increased from 20% to 50%) and lyophilized, to afford compound PE-21DA (4 mg, 34.55% yield, 95% purity) as a white solid.

LCMS (ESI): m/z, 845 [M+H]⁺. ¹H NMR (400 MHz, DMSO-*d*6) δ 8.42 (s, 1H), 6.97 (s, 1H), 6.20 (d, *J* = 3.0 Hz, 1H), 5.95 (d, *J* = 3.0 Hz, 1H), 4.95 (s, 1H), 4.87 (s, 1H), 4.81 (s, 1H), 4.75 (s, 1H), 4.65 (s, 1H), 4.36-4.27 (m, 2H), 4.07-3.99 (m, 2H), 3.93 (t, *J* = 6.4 Hz, 2H), 3.84-3.77 (m, 2H), 3.70 (s, 3H), 3.60 (d, *J* = 4.1 Hz, 3H), 3.52 (s, 5H), 3.21 (s, 5H), 3.02-2.91 (m, 4H), 2.78 (d, *J* = 6.8 Hz, 1H), 2.72 (q, *J =* 7.6, 6.7 Hz, 3H), 2.67 (q, *J =* 1.9 Hz, 1H), 2.33 (dt, *J =* 3.8, 1.9 Hz, 2H), 2.11 (dd, *J =* 15.8, 7.4 Hz, 1H), 1.90 (dd, *J =* 35.4, 11.5 Hz, 3H), 1.77-1.67 (m, 4H), 1.66-1.45 (m, 8H), 1.31 (t, *J* = 9.1 Hz, 2H), 1.04 (d, *J* = 6.4 Hz, 3H).

### Preparation Example 17: Preparation of Compound PE-21E

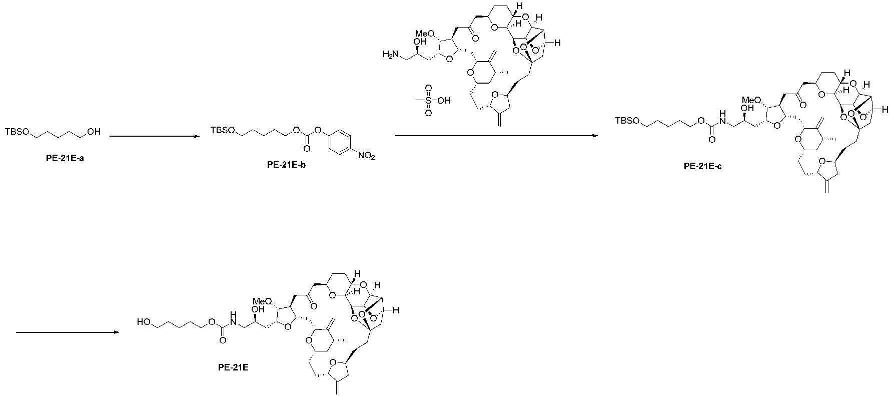

### Step 1: Preparation of 5-[(tert-butyldimethylsilyl)oxy]pentyl 4-nitrophenyl carbonate

5-[(tert-butyldimethylsilyl)oxy]-1-pentanol (218.41 mg, 1 mmol, 1 eq) was dissolved in DMF (3 mL). Then bis(4-nitrophenyl) carbonate (912.63 mg, 608.42 µL, 3 mmol, 3 eq) and DIPEA (387.72 mg, 495.81 µL, 3 mmol, 3 eq) were added. The reaction solution was allowed to react at 35°C for 3 hours. The LCMS showed that a new compound was generated. The reaction solution was diluted with water (50 mL) and extracted with dichloromethane (50 mL × 3). The organic phases were combined, washed with water (100 mL), dried over sodium sulfate, filtered, and concentrated. The residue was purified by silica gel column chromatography (PE/EA (petroleum ether/ethyl acetate) = 93%/7%) to afford 5-[(tert-butyldimethylsilyl)oxy]pentyl 4-nitrophenyl carbonate (330 mg, 86.05% yield) as a pale yellow oil.

¹H NMR (400 MHz, DMSO-*d*₆) δ 8.39-8.25 (m, 2H), 7.60-7.45 (m, 2H), 4.24 (t, J = 6.4 Hz, 2H), 3.58 (t, J = 6.4 Hz, 2H), 1.77-1.59 (m, 2H), 1.60-1.45 (m, 2H), 1.46-1.33 (m, 2H), 0.85 (s, 9H), 0.02 (s, 6H).

### Step 2: Preparation of compound PE-21E-c

5-[(tert-butyldimethylsilyl)oxy]pentyl 4-nitrophenyl carbonate (5.57 mg, 14.53 µmol, 2 eq) was dissolved in THF (500 µL), the mixture was cooled to 0°C, and then eribulin mesylate (6 mg, 7.26 µmol, 1 eq) and DIPEA (2.82 mg, 3.60 µL, 21.79 µmol, 3 eq) were added. The reaction solution was allowed to react at 25°C for 36 hours. The LCMS analysis showed that the reaction ended. The reaction solution was concentrated to afford the crude product PE-21E-c (8 mg) as a yellow oil.

LCMS (ESI): m/z, 974.6 [M+H] ⁺, Rt = 3.83 min.

### Step 3: Preparation of compound PE-21E

PE-21E-c (8 mg, 8.21 µmol, 1 eq) was dissolved in DCM (500 µL), the mixture was cooled to 0°C, and then TFA (153.50 mg, 100 µL) was added. The reaction solution was allowed to react at 0°C for 30 minutes. The LCMS analysis showed that the reaction ended. The reaction solution was concentrated, and the residue was purified by preparative high-performance liquid chromatography (preparative chromatograph manufacturer: Orienda, model: BRIX-2860, column: Welch Xtimate C18 150 × 30mm × 10 µm, mobile phase: water (0.225% HCOOH)-acetonitrile, with an elution ratio of acetonitrile increased from 30% to 60%), to afford a white solid PE-21E (1.03 mg, 14.59% yield, 97.91% purity).

LCMS (ESI): m/z, 882.6 [M+Na]⁺, Rt = 2.208 min.

### Preparation Example 18: Preparation of Compound PE-21EA

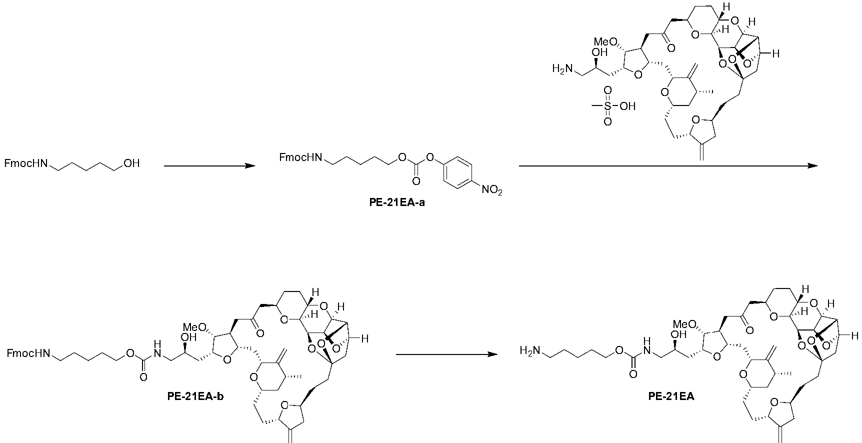

### Step 1: Preparation of compound PE-21EA-a

Bis(4-nitrophenyl) carbonate (2.59g, 8.25 mmol, 3 eq) and DIPEA (2.15g, 16.51 mmol, 6 eq) were added to a solution of (9H-fluoren-9-yl)methyl (5-hydroxypentyl)carbamate (895 mg, 2.75 mmol, 1 eq) in DMF (10 mL). The reaction solution was stirred at 25°C for 1 hour. The LCMS showed that the product was generated. After concentration, the reaction solution was purified by silica gel column chromatography (eluents: petroleum ether/ethyl acetate = 0:100 to 40:60) and reversed-phase silica gel column chromatography (C18, ISCO, R-220g SepaFlash Silica Flash Column, eluents: water/CH₃CN, with an elution ratio of acetonitrile increased from 5% to 80%, flow rate: 80 mL/min) to afford PE-21EA-a (1.07g, 79.29% yield) as a white solid.

LCMS (ESI): m/z, 491.3[M+H]⁺.

### Step 2: Preparation of compound PE-21EA-b

A solution of compound PE-21EA-a (32.07 mg, 65.37 µmol, 1.2 eq) in DMF (1 mL) was added to a solution of eribulin mesylate (45 mg, 54.48 µmol, 1 eq) and DIPEA (21.34 mg, 163.44 µmol, 3 eq) in DMF (1 mL) at 0°C. After the addition, the reaction solution was stirred at 25°C for 1 hour. LCMS analysis showed that the reaction ended. The reaction solution was filtered and the residue was then purified by preparative high-performance liquid chromatography (preparative chromatograph manufacturer: Oriento; model: BRIX-2860; column: Synergi Max-RP 200 × 30 mm × 10 µm; mobile phase: water (0.225% FA)-acetonitrile, with an elution ratio of acetonitrile increased from 50% to 80%) and silica gel column chromatography (eluents: dichloromethane/ tetrahydrofuran = 0:100 to 30:70) to afford PE-21EA-b (56.10 mg, 95.23% yield) as a white solid.

LCMS (ESI): m/z, 1103.8[M+Na]⁺.

### Step 3: Preparation of compound PE-21EA

Diethylamine (29 mg, 388.42 µmol, 10 eq) was added to the solution of compound PE-21EA-b (42 mg, 38.84 µmol, 1 eq) in DMF (3 mL) at 0°C. The reaction solution was stirred at 25°C for 1 hour. LCMS analysis showed that the reaction ended. The reaction solution was filtered and the residue was then purified by high-performance liquid chromatography (preparative chromatograph manufacturer: Oriento; model: BRIX-2860; column: Synergi Max-RP 200 × 30 mm × 10 µm; mobile phase: water (0.225% FA)-acetonitrile, with an elution ratio of acetonitrile increased from 20% to 50%) to afford PE-21EA (26.06 mg, 78.1% yield, 95% purity) as a white solid.

LCMS (ESI): m/z, 859.6[M+H]⁺.

¹H NMR (400 MHz, DMSO-*d*₆) δ 8.42 (s, 1H), 6.94 (t, *J* = 5.9 Hz, 1H), 5.05 (s, 1H), 4.99 (s, 1H), 4.83 (s, 1H), 4.75 (s, 1H), 4.63 (d, *J =* 3.9 Hz, 1H), 4.55 (t, *J =* 4.2 Hz, 1H), 4.26 (d, *J =* 10.9 Hz, 1H), 4.18 (dd, *J* = 10.3, 4.2 Hz, 1H), 4.09 (d, *J =* 5.0 Hz, 2H), 4.02 (t, *J =* 9.6 Hz, 1H), 3.91 (t, *J =* 6.6 Hz, 2H), 3.80 (s, 1H), 3.77 - 3.73 (m, 1H), 3.70 (s, 1H), 3.56 (s, 2H), 3.49 (d, *J =* 10.7 Hz, 5H), 3.25 (s, 4H), 2.94 (t, *J* = 6.0 Hz, 2H), 2.84 (d, *J* = 9.6 Hz, 1H), 2.79 - 2.73 (m, 1H), 2.69 (q, *J =* 6.5, 5.6 Hz, 3H), 2.60 - 2.54 (m, 1H), 2.34 - 2.30 (m, 1H), 2.24 (t, *J =* 14.7 Hz, 3H), 2.14 (d, *J =* 12.0 Hz, 1H), 2.04 - 1.95 (m, 2H), 1.91 (d, *J =* 9.0 Hz, 4H), 1.71 (d, *J =* 11.4 Hz, 2H), 1.65 (d, *J* = 12.6 Hz, 2H), 1.50 (dd, *J* = 14.7, 7.5 Hz, 5H), 1.35 - 1.30 (m, 3H), 1.26 (d, *J* = 12.9 Hz, 2H), 1.19 - 1.12 (m, 1H), 1.03 (d, *J =* 6.6 Hz, 3H), 0.94 (d, *J =* 6.8 Hz, 1H), 0.86 (d, *J =* 6.9 Hz, 1H), 0.84 - 0.80 (m, 2H).

### Preparation Example 19: Preparation of Compound PE-21EMS

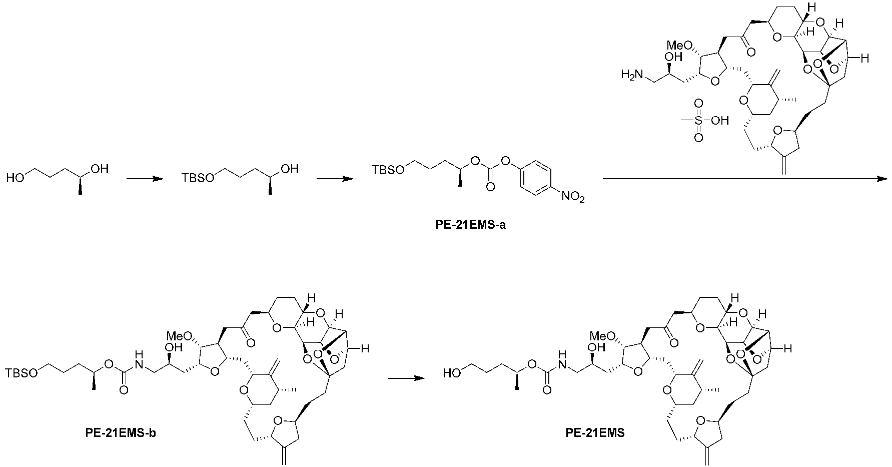

### Step 1: Preparation of (S)-tert-butyldimethylsilyloxy-2-methylbutanol

Imidazole (65.37 mg, 960.15 µmol, 1 eq) and tert-butyldimethylsilyl chloride (159.19 mg, 1.06 mmol, 1.1 eq) were added to a solution of (4S)-1,4-pentanediol (100 mg, 960.15 µmol, 1 eq) in DCM (2 mL) at 0°C. The reaction solution was stirred at room temperature overnight. TLC analysis showed that the reaction ended. DCM (10 mL) and water (20 mL) were added to the mixture. The mixture was extracted with DCM (20 mL × 3). The organic phases were combined and washed with saturated brine (10 mL), dried over anhydrous Na₂SO₄, filtered, and evaporated to dryness, to afford (S)-tert-butyldimethylsilyloxy-2-methylbutanol (131 mg, 62.47% yield) as a colorless oil.

¹H NMR (400 MHz, CDCl₃) 3.93-3.58 (m, 3H), 1.59-1.42 (m, 4H), 1.30-1.09 (m, 3H), 0.95-0.86 (m, 10H), 0.11-0.05 (m, 6H).

### Step 2: Preparation of compound PE-21EMS-a

NPC (547.39 mg, 1.80 mmol, 3 eq) and DIPEA (465.10 mg, 594.76 µL, 3.60 mmol, 6 eq) were added to a solution of (S)-tert-butyldimethylsilyloxy-2-methylbutanol (131 mg, 599.79 µmol, 1 eq) in DMF (2 mL). The reaction solution was stirred at 35°C for 2 hours. LCMS analysis showed that the reaction ended. The reaction solution was evaporated to dryness under reduced pressure. The residue was purified by silica gel column chromatography (eluents: ethyl acetate/petroleum ether = 0:1 to 1:20) to afford the product PE-21EMS-a (112 mg, 48.69% yield) as a colorless oil.

¹H NMR (400 MHz, CDCl₃) δ 8.29 (d, *J =* 9.2 Hz, 2H), 7.42-7.36 (m, 2H), 5.03 - 4.89 (m, 1H), 3.74-3.60 (m, 2H), 1.85-1.58 (m, 4H), 1.45-1.33 (m, 3H), 0.95-0.86 (m, 9H), 0.10-0.02 (m, 6H).

### Step 3: Preparation of compound PE-21EMS-b

DIPEA (4.69 mg, 36.32 µmol, 3 eq) and HOBT (1.64 mg, 12.11 µmol, 1 eq) were added to the solution of eribulin mesylate (10 mg, 12.11 µmol, 1 eq) and PE-21EMS-a (5.57 mg, 14.53 µmol, 1.2 eq) in DMF (2 mL), and the reaction solution was stirred for 2 hours at room temperature. LCMS analysis showed that the reaction ended. The reaction solution was evaporated to dryness under reduced pressure to afford the product PE-21EMS-b (11.80 mg, where the yield was not calculated for the crude product) as a colorless oil. LCMS (ESI): m/z, 1014.6[M+Na]⁺, Rt=2.968 min.

### Step 4: Preparation of compound PE-21EMS

Triethylamine trihydrofluoride (0.1 mL) was added to a solution of compound PE-21EMS-b (11.4 mg, crude) in DMF (2 mL). The reaction solution was stirred at 65°C for 2.5 hours. LCMS analysis showed complete consumption of the starting material. The mixture was purified by preparative high-performance liquid chromatography (preparative chromatograph manufacturer: Orienda, model: BRIX-2860-R6, column: Welch Xtimate C18 250 × 30 mm × 10 µm, mobile phase: water (0.225% HCOOH)-acetonitrile, with an elution ratio of acetonitrile increased from 30% to 60%), to afford PE-21EMS (5.10 mg, 50.68% yield) as a white solid. LCMS (ESI): m/z, 860.6[M+H]⁺, Rt=2.038min.

¹H NMR (400 MHz, DMSO-*d*₆) δ 6.96-6.80 (m, 1H), 5.02 (d, *J* = 22.8 Hz, 2H), 4.79 (d, *J* = 31.2 Hz, 2H), 4.67-4.50 (m, 3H), 4.44-4.36 (m, 1H), 4.29-3.64 (m, 10H), 3.61-3.39 (m, 5H), 3.25 (s, 6H), 2.99-2.53 (m, 7H), 2.36-1.88 (m, 11H), 1.78-1.10 (m, 17H), 1.06-0.92 (m, 4H).

### Preparation Example 20: Preparation of Compound PE-21EMR

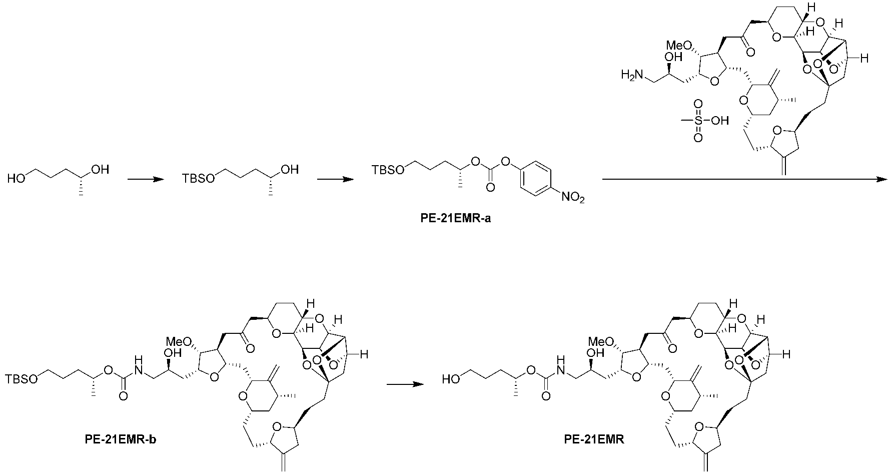

### Step 1: Preparation of (R)-tert-butyldimethylsilyloxy-2-methylbutanol

Imidazole (32.68 mg, 480.08 µmol, 1 eq) and tert-butyldimethylsilyl chloride (79.59 mg, 528.08 µmol, 1.1 eq) were added to a solution of (4R)-1,4-pentanediol (50 mg, 480.08 µmol, 1 eq) in DCM (2 mL) at 0°C. The reaction solution was stirred at room temperature overnight. TLC analysis showed that the reaction ended. DCM (10 mL) and water (20 mL) were added to the mixture. The mixture was extracted with DCM (20 mL × 3). The organic phases were combined and washed with saturated brine (10 mL), dried over anhydrous Na₂SO₄, filtered, and evaporated to dryness, to afford (R)-tert-butyldimethylsilyloxy-2-methylbutanol (84.40 mg, 80.49% yield) as a colorless oil.

¹H NMR (400 MHz, CDCl₃) 3.93-3.56 (m, 3H), 1.70-1.60 (m, 2H), 1.53-1.42 (m, 2H), 1.27-1.11 (m, 3H), 0.94-0.82 (m, 10H), 0.11 (s, 3H), 0.08 (s, 3H).

### Step 2: Preparation of compound PE-21EMR-a

NPC (352.67 mg, 1.16 mmol, 3 eq) and DIPEA (299.65 mg, 2.32 mmol, 6 eq) were added to a solution of (R)-tert-butyldimethylsilyloxy-2-methylbutanol (84.40 mg, 386.43 µmol, 1 eq) in DMF (2 mL). The reaction solution was stirred at 35°C for 2 hours. LCMS analysis showed that the reaction ended. The reaction solution was evaporated to dryness under reduced pressure. The residue was purified by silica gel column chromatography (eluents: ethyl acetate/petroleum ether = 0:1 to 1:20) to afford the product PE-21EMR-a (40 mg, 26.99% yield) as a colorless oil.

¹H NMR (400 MHz, CDCl₃) δ 8.31-8.26 (m, 2H), 7.42-7.37 (m, 2H), 4.98-4.89 (m, 1H), 3.69-3.62 (m, 2H), 1.80-1.59 (m, 4H), 1.45-1.38 (m, 3H), 0.93-0.87 (m, 9H), 0.07 (s, 6H).

### Step 3: Preparation of compound PE-21EMR-b

DIPEA (4.69 mg, 36.32 µmol, 3 eq) and HOBT (1.64 mg, 12.11 µmol, 1 eq) were added to the solution of eribulin mesylate (10 mg, 12.11 µmol, 1 eq) and PE-21EMR-a (6 mg, 15.64 µmol, 1.29 eq) in DMF (2 mL), and the reaction solution was stirred for 1 hour at room temperature. LCMS analysis showed that the reaction ended. The reaction solution was evaporated to dryness under reduced pressure to afford the product PE-21EMR-b (11.80 mg, where the yield was not calculated for the crude product) as a colorless oil. LCMS (ESI): m/z, 1014.6[M+Na]⁺.

### Step 4: Preparation of compound PE-21EMR

Triethylamine trihydrofluoride (0.1 mL) was added to a solution of compound PE-21EMR -b (11.8 mg, crude) in DMF (2 mL). The reaction solution was stirred at 60°C for 4 hours. LCMS analysis showed complete consumption of the starting material. The mixture was purified by preparative high-performance liquid chromatography (preparative chromatograph manufacturer: Orienda, model: BRIX-2860-R6, column: Welch Xtimate C18 250 × 30 mm × 10 µm, mobile phase: water (0.225% HCOOH)-acetonitrile, with an elution ratio of acetonitrile increased from 30% to 60%), to afford PE-21EMR (3.90 mg, 37.44% yield) as a white solid. LCMS (ESI): m/z, 860.6[M+H]⁺.

¹H NMR (400 MHz, DMSO-*d*₆) δ 6.93-6.81 (m, 1H), 5.02 (d, *J* = 22.8 Hz, 2H), 4.79 (d, *J* = 31.6 Hz, 2H), 4.68-4.49 (m, 3H), 4.43-4.35 (m, 1H), 4.31-3.63 (m, 9H), 3.62-3.37 (m, 6H), 3.30-3.23 (m, 6H), 2.96-2.55 (m, 7H), 2.35-1.89 (m, 11H), 1.77-1.08 (m, 17H), 1.07-0.92 (m, 4H).

### Preparation Example 21: Preparation of Compound PE-21EK

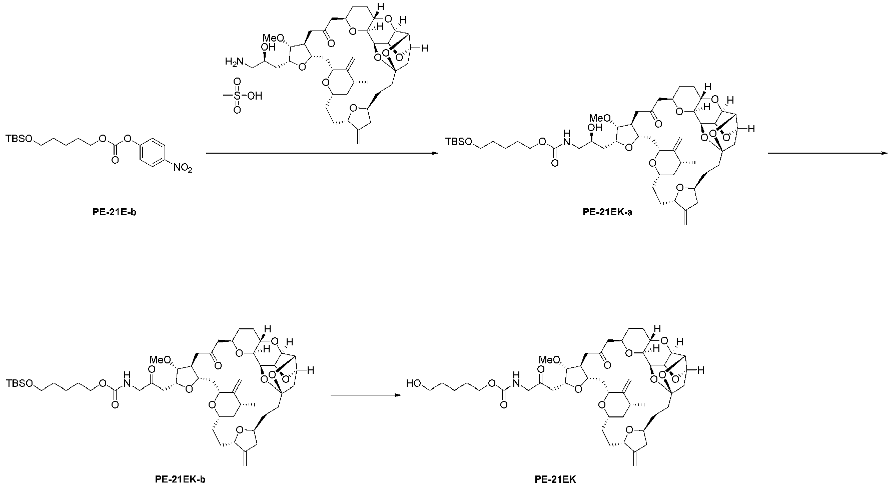

### Step 1: Preparation of compound PE-21EK-a

5-[(tert-butyldimethylsilyl)oxy]pentyl 4-nitrophenyl carbonate (10 mg, 26.07 µmol, 1 eq; for the synthesis of the compound, refer to the preparation example of PE-21E) was dissolved in DMF (2 mL). Eribulin mesylate (80 mg, 289.38 µmol, 1 eq), DIPEA (10.11 mg, 99%, 12.93 µL, 78.22 µmol, 3 eq) and HOBt (3.52 mg, 99.96%, 2.35 µL, 26.07 µmol, 1 eq) were added. The reaction solution was stirred at 25°C for 2 hours. The LCMS showed that the product was generated. The reaction solution was subjected to preparative high-performance liquid chromatography to obtain a white solid product (15 mg, 59.04% yield).

Conditions of the preparative high-performance liquid chromatography (preparative chromatograph manufacturer: Orienda, model: BRIX-2860, column: Welch Xtimate C18 250 × 30 mm × 10 µm, mobile phase: water (0.225% HCOOH)-acetonitrile, with an elution ratio of water increased from 65% to 95%.

LCMS (ESI): m/z, 974.7[M+H]⁺.

### Step 2: Preparation of compound PE-21EK-b

Compound PE-22EK-a (10 mg, 10.26 µmol, 1 eq) was dissolved in DCM (3 mL), DMP (26.12 mg, 98%, 19.18 µL, 61.58 µmol, 6 eq) was added at 0°C, and finally, a catalytic amount of water (50 µL) was added. The reaction mixture was stirred at 25°C for 12 hours. LCMS analysis showed that the reaction ended. The reaction solution was evaporated to dryness, and the residue was dissolved in DMF (1 mL). The mixture was subjected to preparative high-performance liquid chromatography to afford a white solid product (3 mg, 30.06% yield).

The conditions of the preparative high-performance liquid chromatography were as follows:
preparative chromatograph manufacturer: Orienda, model: BRIX-2860, column: Welch Xtimate C18 150 × 30 mm × 10 µm, mobile phase: water (0.225% HCOOH)-acetonitrile, with an elution ratio of water increased from 65% to 95%.

LCMS (ESI): m/z, 972.54[M+H]⁺.

### Step 3: Preparation of compound PE-21EK

Et3N.3HF (98.90 mg, 97%, 100 µL, 595.08 µmol, 192.8653 eq) was added to the solution of compound PE-21EK-b (3 mg, 3.09 µmol, 1 eq) in THF (1 mL) at 25°C, and the reaction solution was stirred at 25°C for 2 hours. LCMS analysis showed complete consumption of the starting material. The reaction solution was purified by high-performance liquid chromatography to afford the product (1.32 mg, 49.86% yield, 96% purity) as a white solid.

The conditions of the preparative high-performance liquid chromatography were as follows:
preparative chromatograph manufacturer: Orienda, model: BRIX-2860, column: YMC-Triart Prep C18 150 × 30 mm × 10 µm, mobile phase: water (0.225% HCOOH)-acetonitrile, with an elution ratio of acetonitrile increased from 30% to 60%.

LCMS (ESI): m/z, 880.4[M+Na]⁺.

¹H NMR (400 MHz, DMSO-*d*₆) δ 7.29 (t, *J* = 6.0 Hz, 1H), 5.04 (d, *J =* 11.1 Hz, 2H), 4.83 (s, 1H), 4.77-4.73 (m, 1H), 4.63 (d, *J =* 2.8 Hz, 1H), 4.55 (t, *J =* 4.2 Hz, 1H), 4.37 (t, *J* = 5.1 Hz, 1H), 4.26 (d, *J* = 10.8 Hz, 1H), 4.17 (td, *J =* 10.2, 4.2 Hz, 1H), 4.10 (t, *J* = 3.2 Hz, 2H), 4.06-3.98 (m, 2H), 3.93 (t, *J* = 6.7 Hz, 2H), 3.87-3.75 (m, 3H), 3.71 (s, 1H), 3.57-3.47 (m, 2H), 3.23 (s, 3H), 2.84 (d, *J* = 9.2 Hz, 1H), 2.82-2.72 (m, 2H), 2.71-2.65 (m, 2H), 2.60 (dd, *J* = 18.0, 8.0 Hz, 2H), 2.29 (q, *J* = 18.7, 17.8 Hz, 5H), 2.18-1.84 (m, 8H), 1.68 (q, *J* = 12.5 Hz, 4H), 1.58-1.38 (m, 6H), 1.38-1.15 (m, 6H), 1.00 (dd, *J* = 22.6, 9.1 Hz, 4H).

### Preparation Example 22: Preparation of Compound E2KO

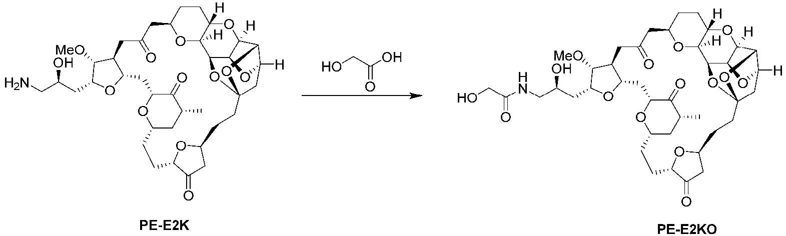

### Step 1: Preparation of compound PE-E2KO

Compound PE-E2K (30 mg, 0.04 mmol) was dissolved in N,N-dimethylformamide (2 mL), glycolic acid (7 mg, 0.08 mmol), 1-hydroxybenzotriazole (16 mg, 0.12 mmol) and 1-(3-dimethylaminopropyl)-3-ethylcarbodiimide (23 mg, 0.12 mmol) were added at room temperature. The reaction solution was stirred at 30°C for 2 hours. The reaction solution was purified by preparative high-performance liquid chromatography (preparative chromatograph manufacturer: Oriento, model: BRIX-2860, column: phenomenex Luna C18 250×50 mm×10 µm, mobile phase: water (0.5% TFA)-acetonitrile, with an elution ratio of acetonitrile increased from 12% to 42%) and the eluate was lyophilized, to afford the product PE-E2KO (8.1 mg, 24.7%) as a white solid.

LCMS (ESI): m/z, 792.5 [M+H]⁺, Rt = 0.97 min.

¹H NMR (400 MHz, DMSO-*d*_{*6*)} δ 7.51 (s, 1H), 4.64 (t, *J* = 4.4 Hz, 1H), 4.56 (s, 1H), 4.39 (s, 1H), 4.11 (d, *J* = 1.6 Hz, 4H), 3.79 (s, 6H), 3.55 (d, *J* = 5.2 Hz, 3H), 3.27 (d, *J =* 3.2 Hz, 2H), 3.25 (s, 3H), 3.21 - 3.01 (m, 3H), 2.89 - 2.61 (m, 4H), 2.43 - 2.12 (m, 6H), 1.95 (d, *J* = 17.2 Hz, 5H), 1.71 - 1.43 (m, 9H), 1.23 (s, 4H), 0.94 (d, *J* = 6.4 Hz, 3H).

### Preparation Example 23: Preparation of Compound PE-21F

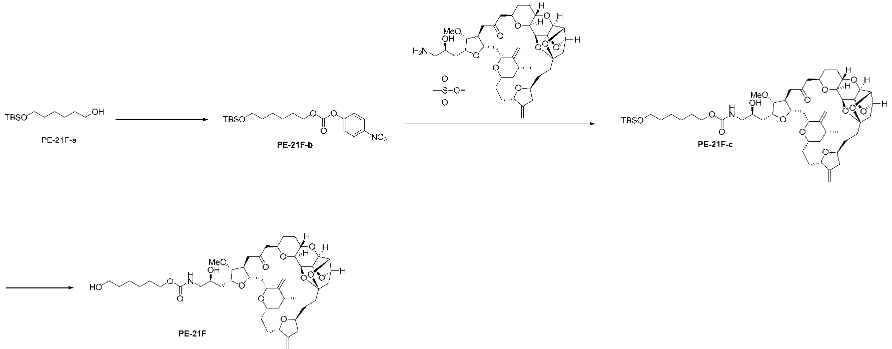

### Step 1: Preparation of 6-((tert-butyldimethylsilyl)oxy)hexyl 4-nitrophenyl carbonate

6-((tert-butyldimethylsilyl)oxy)hexan-1-ol (232.43 mg, 1 mmol, 1 eq) was dissolved in DMF (3 mL). Then bis(4-nitrophenyl) carbonate (912.63 mg, 608.42 µL, 3 mmol, 3 eq) and DIPEA (387.72 mg, 495.81 µL, 3 mmol, 3 eq) were added. The reaction solution was allowed to react at 35°C for 2 hours. The LCMS showed that a new compound was generated. The reaction solution was diluted with water (50 mL) and extracted with dichloromethane (50 mL × 3). The organic phases were combined, washed with brine (100 mL), dried over sodium sulfate, filtered, and concentrated. The residue was purified by silica gel column chromatography (eluents: PE/EA = 95%/5%), to afford 6-((tert-butyldimethylsilyl)oxy)hexyl 4-nitrophenyl carbonate (370 mg, 93.07% yield) as a yellow oil.

¹H NMR (400 MHz, DMSO-*d*₆) δ 8.37-8.24 (m, 2H), 7.54 (dd, *J* = 9.2, 1.6 Hz, 2H), 4.23 (t, *J =* 6.4 Hz, 2H), 3.56 (t, *J* = 6.4 Hz, 2H), 1.67 (t, *J* = 7.2 Hz, 2H), 1.46 (t, *J* = 6.4 Hz, 2H), 1.34 (dt, *J* = 8.0, 4.8 Hz, 4H), 0.84 (d, *J =* 1.2 Hz, 9H), 0.01 (d, *J* = 1.2 Hz, 6H).

### Step 2: Preparation of compound PE-21F-c

6-((tert-butyldimethylsilyl)oxy)hexyl 4-nitrophenyl carbonate (9.63 mg, 24.21 µmol, 2 eq) was dissolved in THF (500 µL), the mixture was cooled to 0°C, and then eribulin mesylate (10 mg, 12.11 µmol, 1 eq) and DIPEA (4.69 mg, 6 µL, 36.32 µmol, 3 eq) were added. The reaction solution was allowed to react at 25°C for 16 hours. LCMS analysis showed that the reaction ended. The reaction solution was concentrated to afford the crude product of PE-21F-c (12 mg) as a yellow oil.

LCMS (ESI): m/z, 988.7 [M+H]⁺, Rt = 3.93 min.

### Step 3: Preparation of compound PE-21F

Compound PE-21F-c (12 mg, 12.14 µmol, 1 eq) was dissolved in DCM (500 µL), the mixture was cooled to 0°C, and then TFA (153.50 mg, 100 µL) was added. The reaction solution was allowed to react at 0°C for 30 minutes. The LCMS analysis showed that the reaction ended. The reaction solution was concentrated, and the residue was purified by preparative high-performance liquid chromatography (preparative chromatograph manufacturer: Shimadzu, model: LC-20AP, column: Welch Xtimate C18 250 × 40mm × 10 µm, mobile phase: water (0.225% HCOOH)-acetonitrile, with an elution ratio of acetonitrile increased from 30% to 60%), to afford a white solid PE-21F (2.46 mg, 23.18% yield, 100% purity).

LCMS (ESI): m/z, 874.6 [M+H]⁺, Rt = 2.298 min.

### Preparation Example 24: Preparation of Compound PE-21G

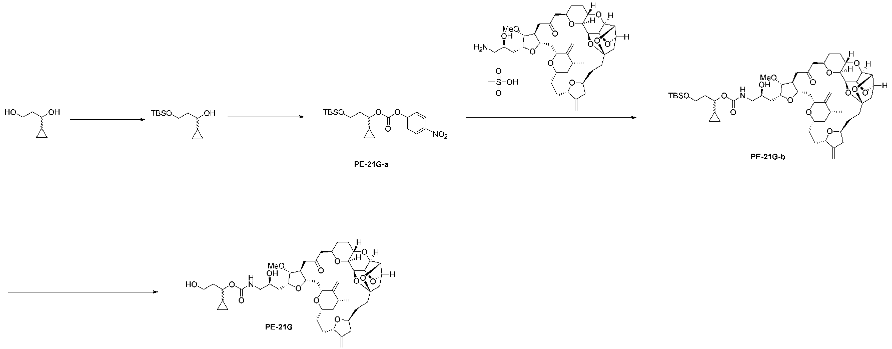

### Step 1: Preparation of 3-((tert-butyldimethylsilyl)oxy)-1-cyclopropylpropan-1-ol

TBSCl (135.59 mg, 155.85 µL, 899.62 µmol, 1.1 eq) and imidazole (55.68 mg, 50.62 µL, 817.84 µmol, 1 eq) were added to a solution of 1-cyclopropylpropane-1,3-diol (95 mg, 817.84 µmol, 1 eq) in DCM (8 mL) at 0°C. After the addition, the mixture was allowed to react at 25°C for 16 hours. The reaction ended as indicated by NMR. The reaction solution was added into ice-cold aqueous sodium bicarbonate solution (60 mL) and the mixture was then stirred for 2 minutes and extracted with dichloromethane (60 mL × 3). The organic phases were combined, washed with brine, dried over anhydrous sodium sulfate, and filtered. The filtrate was concentrated to afford the compound 3-((tert-butyldimethylsilyl)oxy)-1-cyclopropylpropan-1-ol (147 mg, 78.01% yield) as a colorless oil.

¹H NMR (400 MHz, Chloroform-*d*) δ 3.96-3.87 (m, 1H), 3.80 (ddd, *J =* 10.1, 7.0, 6.0 Hz, 1H), 3.11 (dt, *J* = 8.3, 5.8 Hz, 1H), 1.85-1.78 (m, 2H), 0.89 (s, 9H), 0.89-0.86 (m, 1H), 0.57-0.41 (m, 2H), 0.41-0.33 (m, 1H), 0.21-0.13 (m, 1H), 0.07 (d, *J* = 2.9 Hz, 6H).

### Step 2: Preparation of compound PE-21G-a

Bis(4-nitrophenyl) carbonate (1.61g, 1.07 mL, 5.28 mmol, 4 eq) and DIPEA (1.02g, 1.31 mL, 7.92 mmol, 6 eq) were added to a solution of 3-((tert-butyldimethylsilyl)oxy)-1-cyclopropyl propan-1-ol (304 mg, 1.32 mmol, 1 eq) in DMF (10 mL) at 0°C. The reaction solution was allowed to react at 33°C for 2 hours. The LCMS showed that a new compound was generated. The reaction solution was evaporated to dryness, to afford the residue. The residue was purified by silica gel column chromatography (eluents: ethyl acetate/hexane = 1:30 to 1:20) to afford PE-21G-a (257 mg, 49.25% yield) as a pale yellow oil.

¹H NMR (400 MHz, Chloroform-*d*) δ 8.29-8.26 (m, 2H), 7.41-7.36 (m, 2H), 4.38 (ddd, *J* = 9.3, 7.8, 5.1 Hz, 1H), 3.81-3.69 (m, 2H), 2.11-1.92 (m, 2H), 1.18-1.06 (m, 1H), 0.88 (s, 9H), 0.67-0.55 (m, 3H), 0.40-0.31 (m, 1H), 0.05 (d, *J* = 1.1 Hz, 6H). The structure of the compound was further confirmed by 2D NMR spectroscopy.

### Step 3: Preparation of compound PE-21G-b

Eribulin mesylate (10 mg, 12.11 µmol, 1 eq), DIPEA (4.69 mg, 6 µL, 36.32 µmol, 3 eq) and HOBt (1.64 mg, 1.09 µL, 12.11 µmol, 1 eq) were added to a solution of compound PE-21G-a (5.57 mg, 14.53 µmol, 2 eq) in DMF (1 mL) at 0°C. The reaction solution was allowed to react at 25°C for 16 hours. The LCMS analysis showed that the reaction ended. The reaction solution was concentrated to afford the crude product PE-21G-b (12 mg, crude) as a yellow oil.

LCMS (ESI): m/z, 1008.6 [M+Na] ⁺, Rt = 2.430 min.

### Step 4: Preparation of compound PE-21G

Triethylamine trihydrofluoride (78.45 mg, 79.33 µL, 486.65 µmol, 40 eq) was added to a solution of compound PE-21G-b (12 mg, 12.17 µmol, 1 eq) in THF (3 mL) at room temperature. The reaction solution was allowed to react at 60°C for 2 hours. The LCMS analysis showed that the reaction ended. The reaction solution was concentrated, and the residue was purified by preparative high-performance liquid chromatography (preparative chromatograph manufacturer: Orienda, model: BRIX-2860, column: Welch Xtimate C18 150 × 30 mm × 10 µm, mobile phase: water (0.225% HCOOH)-acetonitrile, with an elution ratio of acetonitrile increased from 30% to 60%) and lyophilized, to afford PE-21G (3.77 mg, 35.53% yield, 95% purity) as a white solid.

LCMS (ESI): m/z, 894.5 [M+Na]⁺, Rt = 2.168 min.

¹H NMR (400 MHz, DMSO-*d*₆) δ 6.86 (t, *J* = 5.8 Hz, 1H), 5.05 (s, 1H), 4.99 (s, 1H), 4.83 (s, 1H), 4.75 (s, 1H), 4.63 (dt, *J =* 4.3, 2.4 Hz, 1H), 4.58-4.47 (m, 2H), 4.39 (td, *J* = 5.1, 3.3 Hz, 1H), 4.26 (d, *J* = 10.9 Hz, 1H), 4.22-4.05 (m, 5H), 4.02 (s, 1H), 3.84-3.65 (m, 3H), 3.59-3.42 (m, 5H), 3.26 (s, 4H), 3.01-2.89 (m, 2H), 2.84 (d, *J* = 9.8 Hz, 1H), 2.72 (td, *J* = 20.0, 18.2, 8.7 Hz, 2H), 2.61-2.53 (m, 1H), 2.23 (tt, *J =* 29.1, 12.9 Hz, 6H), 1.96 (d, *J =* 34.1 Hz, 6H), 1.81-1.58 (m, 7H), 1.56-1.41 (m, 3H), 1.38-1.12 (m, 4H), 1.07-0.83 (m, 5H), 0.51-0.34 (m, 2H), 0.34-0.19 (m, 2H).

### Preparation Example 25: Preparation of Compound PE-AL1

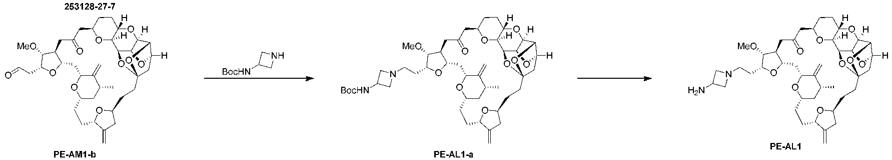

### Step 1: Preparation of compound PE-AL1-a

Acetic acid (2.76 mg, 99.5%, 2.64 µL, 45.79 µmol, 2 eq) was added to a solution of compound PE-AM1-b (for the synthesis of the compound, refer to the preparation example of PE-AM1) (16 mg, 22.89 µmol, 1 eq) and 3-N-(tert-butoxycarbonyl)aminocyclobutylamine (4.88 mg, 97%, 27.47 µmol, 1.2 eq) in 1,2-dichloroethane (1 mL). After stirring at 25°C for 20 minutes, sodium triacetoxyborohydride (9.90 mg, 98%, 45.79 µmol, 2 eq) was added to the reaction solution, and the mixture was stirred at 25°C for 40 minutes. LCMS analysis indicated the reaction ended. The reaction solution was added to water (20 mL) and extracted with ethyl acetate (10 mL×3). The organic phases were combined and washed with saturated brine (30 mL), anhydrous sodium sulfate was added for drying, and a resulting mixture was evaporated to dryness under reduced pressure to afford the compound PE-AL1-a (26 mg, crude product) as a white solid.

LCMS (ESI): m/z, 855.6[M+H]⁺.

### Step 2: Preparation of compound PE-AL1

Trifluoroacetic acid (0.2 mL) was added to a solution of compound PE-AL1-a (19.58 mg, 22.90 µmol, 1 eq) in dichloromethane (1 mL) at 0°C. The mixture was stirred at 0°C for 1 hour. LCMS analysis indicated the reaction ended. The reaction solution was concentrated. The residue was then purified by preparative high-performance liquid chromatography (preparative chromatograph manufacturer: Shimadzu, model: LC-20AP, column: YMC-Triart Prep C18 150 × 30 mm × 10 µm, mobile phase: water (0.225% HCOOH)-acetonitrile, with an elution ratio of acetonitrile increased from 30% to 60%) and lyophilized, to afford compound PE-AL1 (0.93 mg, 5.38% yield) as a white solid.

LCMS (ESI): m/z, 755.5[M+H]⁺, 777.5[M+Na]⁺.

¹H NMR (400 MHz, DMSO-*d*₆) δ 6.20 (d, *J* = 3.0 Hz, 1H), 5.95 (d, *J* = 3.0 Hz, 1H), 4.96-4.94 (m, 1H), 4.87 (s, 1H), 4.81 (s, 1H), 4.75 (s, 1H), 4.34 (d, *J* = 6.1 Hz, 1H), 4.30 (d, *J* = 9.6 Hz, 1H), 4.10-4.04 (m, 1H), 4.01 (d, *J* = 2.6 Hz, 1H), 3.78 (dd, *J* = 9.7, 2.7 Hz, 1H), 3.68 (q, *J* = 4.7, 3.7 Hz, 3H), 3.59 (dd, *J* = 9.0, 4.6 Hz, 3H), 3.54-3.49 (m, 6H), 3.22 (s, 4H), 2.97 (d, *J* = 7.1 Hz, 1H), 2.80-2.74 (m, 2H), 2.67-2.62 (m, 3H), 2.58-2.55 (m, 1H), 2.43 (d, *J* = 9.4 Hz, 3H), 2.32 (d, *J* = 4.6 Hz, 1H), 2.25 (t, *J* = 6.5 Hz, 1H), 2.14-2.08 (m, 1H), 2.01-1.94 (m, 3H), 1.85 (dd, *J* = 11.9, 4.3 Hz, 1H), 1.70 (dd, *J =* 11.0, 6.4 Hz, 3H), 1.62 (dd, *J =* 10.4, 5.0 Hz, 2H), 1.53 (d, *J* = 7.1 Hz, 2H), 1.48-1.40 (m, 3H), 1.34-1.28 (m, 3H), 1.04 (d, *J* = 6.5 Hz, 3H), 0.97-0.91 (m, 1H), 0.85 (t, *J* = 6.7 Hz, 1H).

### Preparation Example 26: Preparation of Compound PE-AL2

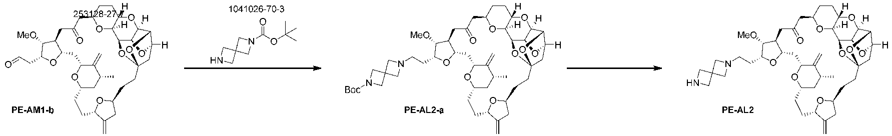

### Step 1: Preparation of compound PE-AL2-a

Acetic acid (2.76 mg, 99.5%, 2.64 µL, 45.79 µmol, 2 eq) was added to a solution of compound PE-AM1-b (for the synthesis of the compound, refer to the preparation example of PE-AM1) (16 mg, 22.89 µmol, 1 eq) and tert-butyl 2,6-diazaspiro[3.3]heptane-2-carboxylate (4.78 mg, 95%, 22.89 µmol, 1 eq) in 1,2-dichloroethane (1 mL). After stirring at 25°C for 20 minutes, sodium triacetoxyborohydride (9.90 mg, 98%, 45.79 µmol, 2 eq) was added to the reaction solution, and the mixture was stirred at 25°C for 40 minutes. LCMS analysis indicated the reaction ended. The reaction solution was added to water (20 mL) and extracted with ethyl acetate (10 mL×3). The organic phases were combined and washed with saturated brine (30 mL), anhydrous sodium sulfate was added for drying, and a resulting mixture was evaporated to dryness under reduced pressure to afford the compound PE-AL2-a (24 mg, crude product) as a white solid.

LCMS (ESI): m/z, 881.6[M+H]⁺, 903.6[M+Na]⁺.

### Step 2: Preparation of compound PE-AL2

Trifluoroacetic acid (0.2 mL) was added to a solution of compound PE-AL2-a (20.17 mg, 22.89 µmol, 1 eq) in dichloromethane (1 mL) at 0°C. The mixture was stirred at 25°C for 1 hour. LCMS analysis indicated the reaction ended. The reaction solution was concentrated. The residue was then purified by preparative high-performance liquid chromatography (preparative chromatograph manufacturer: Shimadzu, model: LC-20AP, column: Welch Xtimate C18 250 × 30mm × 10 µm, mobile phase: water (0.225% HCOOH)-acetonitrile, with an elution ratio of acetonitrile increased from 30% to 60%) and lyophilized, to afford compound PE-AL2 (2.32 mg, 12.98% yield) as a white solid.

LCMS (ESI): m/z, 781.4[M+H]⁺, 803.4[M+Na]⁺.

¹H NMR (400 MHz, DMSO-*d*₆) δ 6.20 (d, *J* = 3.0 Hz, 1H), 5.95 (d, *J* = 3.0 Hz, 1H), 4.95 (d, *J* = 2.1 Hz, 1H), 4.88-4.85 (m, 1H), 4.80 (s, 1H), 4.75 (s, 1H), 4.34 (d, *J =* 6.3 Hz, 1H), 4.29 (d, *J* = 9.7 Hz, 1H), 4.06 (t, *J* = 6.6 Hz, 1H), 4.00 (t, *J* = 2.5 Hz, 1H), 3.87 (d, *J* = 9.7 Hz, 4H), 3.78 (dd, *J =* 9.7, 2.7 Hz, 1H), 3.68 (dd, *J =* 9.8, 5.9 Hz, 4H), 3.57 (dt, *J =* 9.3, 5.0 Hz, 6H), 3.21 (s, 5H), 3.00-2.95 (m, 2H), 2.80-2.73 (m, 2H), 2.66 (dt, *J* = 6.8, 1.9 Hz, 1H), 2.63-2.57 (m, 2H), 2.56-2.54 (m, 1H), 2.44 (d, *J* = 3.4 Hz, 1H), 2.33 (ddd, *J =* 7.8, 5.6, 3.7 Hz, 3H), 2.24 (t, *J =* 7.6 Hz, 1H), 2.14-2.08 (m, 1H), 1.97-1.90 (m, 2H), 1.85 (dd, *J =* 12.0, 3.9 Hz, 1H), 1.71 (ddd, *J =* 14.0, 10.3, 6.5 Hz, 4H), 1.65-1.57 (m, 3H), 1.52-1.47 (m, 2H), 1.45-1.39 (m, 2H), 1.34-1.27 (m, 2H), 1.04 (d, *J =* 6.5 Hz, 3H), 0.98-0.90 (m, 1H).

### Preparation Example 27: Preparation of Compound PE-AL2H5

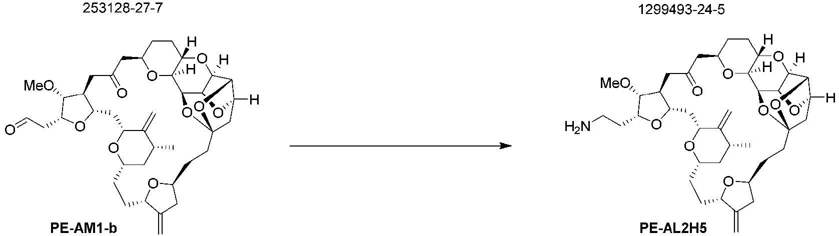

Acetic acid (2.76 mg, 99.5%, 2.64 µL, 45.79 µmol, 2 eq) was added to a solution of compound PE-AM1-b (for the synthesis of the compound, refer to the preparation example of PE-AM1) (16 mg, 22.89 µmol, 1 eq) and ammonia solution (3.85 mg, 25%, 27.47 µmol, 1.2 eq) in 1,2-dichloroethane (1 mL). After stirring at 25°C for 20 minutes, sodium triacetoxyborohydride (9.90 mg, 98%, 45.79 µmol, 2 eq) was added to the reaction solution, and the mixture was stirred at 25°C for 40 minutes. LCMS analysis indicated the reaction ended. The reaction solution was concentrated. The residue was then purified by preparative high-performance liquid chromatography (preparative chromatograph manufacturer: Shimadzu, model: LC-20AP, column: Welch Xtimate C18 250 × 30mm × 10 µm, mobile phase: water (0.225% HCOOH)-acetonitrile, with an elution ratio of acetonitrile increased from 30% to 60%) and lyophilized, to afford compound PE-AL2H5 (0.42 mg, 2.62% yield) as a white solid.

LCMS (ESI): m/z, 700.2[M+H]⁺, 722.2[M+Na]⁺.

¹H NMR (400 MHz, DMSO-d6) δ 5.06 (s, 1H), 4.98 (s, 1H), 4.83 (s, 1H), 4.76 (s, 1H), 4.63 (dt, J = 4.6, 2.4 Hz, 1H), 4.55 (t, J = 4.2 Hz, 1H), 4.26 (d, J = 10.8 Hz, 1H), 4.18 (dd, J = 10.5, 4.0 Hz, 1H), 4.10 (d, J = 4.8 Hz, 3H), 4.02 (s, 1H), 3.80 (t, J = 10.1 Hz, 1H), 3.74-3.67 (m, 2H), 3.48 (s, 5H), 3.27 (s, 2H), 3.26 (s, 1H), 2.84 (dd, J = 9.5, 1.9 Hz, 1H), 2.74 (q, J = 7.8, 6.9 Hz, 2H), 2.67 (h, J = 2.3, 1.9 Hz, 1H), 2.60-2.54 (m, 1H), 2.36-2.06 (m, 6H), 2.04-1.97 (m, 1H), 1.96-1.87 (m, 3H), 1.86-1.76 (m, 1H), 1.76-1.61 (m, 4H), 1.56-1.43 (m, 2H), 1.33 (dd, J = 29.9, 18.2 Hz, 3H), 1.23 (s, 2H), 1.22-1.05 (m, 2H), 1.03 (d, J = 6.5 Hz, 2H), 1.00-0.93 (m, 1H), 0.89-0.80 (m, 2H).

### Preparation Example 28: Preparation of Compound PE-AL3

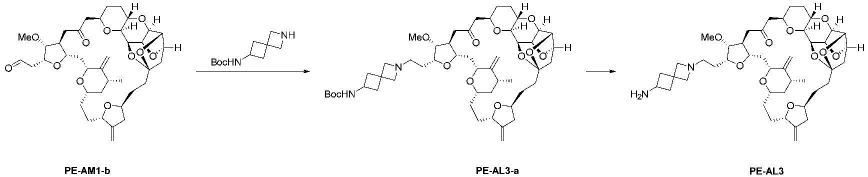

### Step 1: Preparation of compound PE-AL3-a

Acetic acid (2.76 mg, 45.79 µmol, 2 eq) was added to a solution of compound PE-AM1-b (16 mg, crude; for the synthesis of the compound, refer to the preparation example of PE-AM1) and tert-butyl N-2-azaspiro[3.3]hept-6-ylcarbamate (5.01 mg, 22.89 µmol, 1 eq) in DCE (1,2-dichloro ethane, 1 mL). The reaction solution was stirred at room temperature for 20 minutes. Sodium triacetoxyborohydride (9.90 mg, 45.79 µmol, 2 eq) was added to the reaction solution, and the reaction solution was stirred at room temperature for 40 minutes. LCMS analysis showed that the reaction ended. DCM (10 mL) and water (20 mL) were added to the mixture. The mixture was extracted with DCM (20 mL × 3). The organic phases were combined and washed with saturated brine (10 mL), dried over anhydrous Na₂SO₄, filtered, and evaporated to dryness, to afford the product PE-AL3-a (20.59 mg, where the yield was not calculated for the crude product) as a colorless oil. LCMS (ESI): m/z, 895.6[M+H]⁺.

### Step 2: Preparation of compound PE-AL3

TFA (0.4 mL) was added to a solution of compound PE-AL3-a (20.49 mg, crude) in DCM (2 mL) at 0°C. The reaction solution was stirred at room temperature for half an hour. LCMS analysis showed complete consumption of the starting material. The mixture was evaporated to dryness under reduced pressure. The mixture was purified by preparative high-performance liquid chromatography (preparative chromatograph manufacturer: Orienda, model: BRIX-2860-R6, column: Welch Xtimate C18 250 × 30 mm × 10 µm, mobile phase: water (0.225% HCOOH)-acetonitrile, with an elution ratio of acetonitrile increased from 20% to 50%), to obtain PE-AL3 (2.70 mg, 12.37% yield) as a white solid. LCMS (ESI): m/z, 795.6[M+H]⁺.

¹H NMR (400 MHz, DMSO-*d*₆) δ 8.33 (s, 2H), 6.20 (d, *J* = 3.2 Hz, 1H), 5.95 (d, *J* = 3.2 Hz, 1H), 4.95 (s, 1H), 4.89-4.72 (m, 4H), 4.34 (d, *J* = 6.4 Hz, 1H), 4.29 (d, *J =* 9.6 Hz, 1H), 4.11-3.94 (m, 4H), 3.80-3.75 (m, 3H), 3.70 (d, *J* = 4.0 Hz, 3H), 3.33 (d, *J* = 3.6 Hz, 3H), 3.21 (s, 2H), 3.18 (s, 1H), 3.16-3.11 (m, 3H), 3.04 (d, *J* = 9.2 Hz, 2H), 2.99-2.92 (m, 2H), 2.80-2.72 (m, 2H), 2.69-2.58 (m, 3H), 2.39-2.27 (m, 6H), 2.15-2.06 (m, 2H), 1.97-1.81 (m, 4H), 1.75-1.54 (m, 7H), 1.52-1.39 (m, 4H), 1.35-1.27 (m, 2H), 1.04 (d, *J =* 6.4 Hz, 2H), 1.01-0.88 (m, 2H).

### Preparation Example 29: Preparation of Compound PE-AL4

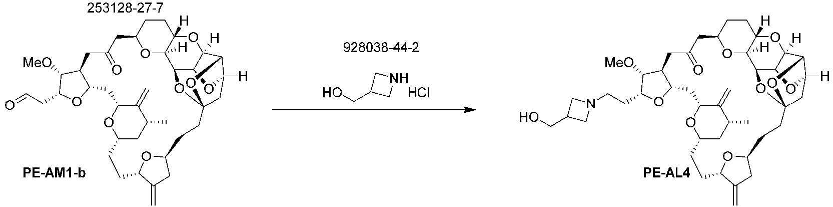

Acetic acid (2.76 mg, 99.5%, 2.64 µL, 45.79 µmol, 2 eq) was added to a solution of compound PE-AM1-b (for the synthesis of the compound, refer to the preparation example of PE-AM1) (16 mg, 22.89 µmol, 1 eq) and 3-(hydroxymethyl)azetidine hydrochloride (3.50 mg, 97%, 27.47 µmol, 1.2 eq) in 1,2-dichloroethane (1 mL). After stirring at 25°C for 20 minutes, sodium triacetoxyborohydride (9.90 mg, 98%, 45.79 µmol, 2 eq) was added to the reaction solution, and the mixture was stirred at 25°C for 40 minutes. LCMS analysis indicated the reaction ended. The reaction solution was added to water (20 mL) and extracted with ethyl acetate (10 mL×3). The organic phases were combined and washed with saturated brine (30 mL), anhydrous sodium sulfate was added for drying, and a resulting mixture was evaporated to dryness under reduced pressure to afford the crude product. The crude product was purified by preparative high-performance liquid chromatography (preparative chromatograph manufacturer: Shimadzu, model: LC-20AP, column: Synergi Max-RP 200 × 30mm × 10 µm, mobile phase: water (0.225% HCOOH)-acetonitrile, with an elution ratio of acetonitrile increased from 20% to 50%) and lyophilized, to afford compound PE-AL4 (0.59 mg, 3.35% yield) as a white solid.

LCMS (ESI): m/z, 770.5[M+H]⁺.

¹H NMR (400 MHz, DMSO-*d*₆) δ 5.03 (dd, *J* = 18.1, 2.4 Hz, 2H), 4.83 (s, 1H), 4.75 (d, *J* = 1.7 Hz, 1H), 4.63 (dt, *J* = 4.4, 2.5 Hz, 1H), 4.55 (t, *J* = 4.3 Hz, 1H), 4.26 (d, *J* = 10.8 Hz, 1H), 4.17 (td, *J =* 10.3, 4.2 Hz, 1H), 4.10 (t, *J =* 3.1 Hz, 2H), 4.03 (d, *J =* 10.3 Hz, 1H), 3.80 (t, *J =* 10.4 Hz, 1H), 3.67 (td, *J* = 6.6, 2.7 Hz, 1H), 3.61 (td, *J* = 6.7, 3.3 Hz, 1H), 3.55-3.49 (m, 2H), 3.45 (d, *J* = 6.7 Hz, 2H), 3.26 (s, 3H), 3.22 (d, *J* = 3.5 Hz, 1H), 3.15 (s, 2H), 2.85-2.76 (m, 3H), 2.75-2.65 (m, 2H), 2.60-2.52 (m, 1H), 2.42 (q, *J* = 6.8 Hz, 2H), 2.35-2.31 (m, 3H), 2.27-2.22 (m, 2H), 2.19-2.09 (m, 2H), 2.04-1.97 (m, 2H), 1.92 (d, *J* = 2.9 Hz, 3H), 1.70-1.62 (m, 3H), 1.54-1.44 (m, 4H), 1.35-1.27 (m, 3H), 1.24 (d, *J =* 3.3 Hz, 3H), 1.20-1.13 (m, 1H), 1.03 (d, *J* = 6.5 Hz, 3H), 1.00-0.93 (m, 1H), 0.85 (t, *J =* 6.7 Hz, 1H).

### Preparation Example 30: Preparation of Compound PE-AL5

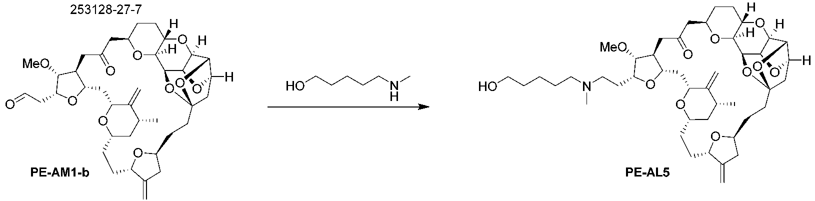

Acetic acid (2.76 mg, 99.5%, 2.64 µL, 45.79 µmol, 2 eq) was added to a solution of compound PE-AM1-b (for the synthesis of the compound, refer to the preparation example of PE-AM1) (16 mg, 22.89 µmol, 1 eq) and 5-(methylamino)pentan-1-ol (3.39 mg, 95%, 27.47 µmol, 1.2 eq) in 1,2-dichloroethane (1 mL). After stirring at 25°C for 20 minutes, sodium triacetoxy borohydride (9.90 mg, 98%, 45.79 µmol, 2 eq) was added to the reaction solution, and the mixture was stirred at 25°C for 40 minutes. LCMS analysis indicated the reaction ended. The reaction solution was concentrated. The residue was then purified by preparative high-performance liquid chromatography (preparative chromatograph manufacturer: Shimadzu, model: LC-20AP, column: Synergi Max-RP 200 × 30mm × 10 µm, mobile phase: water (0.225% HCOOH)-acetonitrile, with an elution ratio of acetonitrile increased from 20% to 50%) and lyophilized, to afford compound PE-AL5 (5.02 mg, 26.86% yield) as a white solid.

LCMS (ESI): m/z, 800.5[M+H]⁺, 822.5[M+Na]⁺.

¹H NMR (400 MHz, DMSO-*d*₆) δ 5.02 (d, *J* = 23.5 Hz, 2H), 4.83 (d, *J* = 6.3 Hz, 1H), 4.75 (s, 1H), 4.66-4.61 (m, 1H), 4.55 (t, *J* = 4.2 Hz, 1H), 4.26 (d, *J* = 10.5 Hz, 1H), 4.17 (td, *J* = 10.3, 4.1 Hz, 1H), 4.10 (q, *J* = 4.2 Hz, 3H), 4.03 (s, 1H), 3.80 (t, *J* = 10.8 Hz, 1H), 3.67 (t, *J* = 7.5 Hz, 1H), 3.64-3.59 (m, 1H), 3.55-3.47 (m, 3H), 3.27 (s, 4H), 3.24-3.22 (m, 2H), 3.19 (s, 1H), 2.84 (d, *J* = 9.6 Hz, 1H), 2.78-2.67 (m, 2H), 2.60-2.54 (m, 1H), 2.38 (d, *J* = 6.4 Hz, 1H), 2.34-2.22 (m, 7H), 2.17 (d, *J* = 11.9 Hz, 1H), 2.11 (s, 3H), 2.05-1.95 (m, 2H), 1.92 (d, *J =* 3.0 Hz, 3H), 1.84 (d, *J =* 14.3 Hz, 1H), 1.67 (dt, *J* = 15.4, 6.3 Hz, 5H), 1.57-1.45 (m, 2H), 1.39 (td, *J* = 13.2, 11.8, 6.6 Hz, 5H), 1.33-1.12 (m, 6H), 1.03 (d, *J* = 6.4 Hz, 3H), 1.01-0.84 (m, 1H).

### Preparation Example 31: Preparation of Compound PE-ALH5

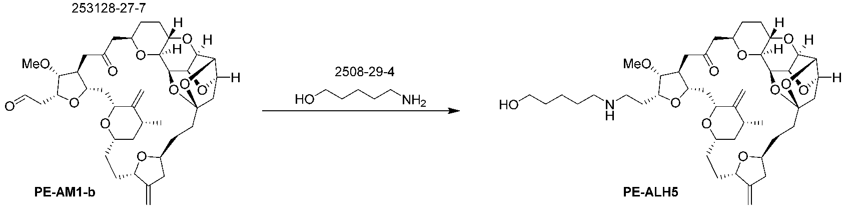

Acetic acid (5.53 mg, 99.5%, 5.27 µL, 91.58 µmol, 2 eq) was added to a solution of compound PE-AM1-b (for the synthesis of the compound, refer to the preparation example of PE-AM1) (32 mg, 45.79 µmol, 1 eq) and 5-aminopentan-1-ol (5.97 mg, 95%, 54.95 µmol, 1.2 eq) in 1,2-dichloroethane (2 mL). After stirring at 25°C for 20 minutes, sodium triacetoxyborohydride (19.81 mg, 98%, 91.58 µmol, 2 eq) was added to the reaction solution, and the mixture was stirred at 25°C for 40 minutes. LCMS analysis indicated the reaction ended. The reaction solution was added to water (20 mL) and extracted with ethyl acetate (10 mL×3). The organic phases were combined and washed with saturated brine (30 mL), anhydrous sodium sulfate was added for drying, and a resulting mixture was evaporated to dryness under reduced pressure to afford the crude product. The crude product was purified by preparative high-performance liquid chromatography (preparative chromatograph manufacturer: Shimadzu, model: LC-20AP, column: Welch Xtimate C18 250 × 30mm × 10 µm, mobile phase: water (0.225% HCOOH)-acetonitrile, with an elution ratio of acetonitrile increased from 20% to 50%) and lyophilized, to afford compound PE-ALH5 (2.37 mg, 6.58% yield) as a white solid.

LCMS (ESI): m/z, 786.5[M+H]⁺, 808.4[M+Na]⁺.

¹H NMR (400 MHz, DMSO-*d⁶*) δ 5.02 (d, *J* = 24.8 Hz, 2H), 4.83 (s, 1H), 4.76 (s, 1H), 4.63 (q, *J* = 3.2, 2.0 Hz, 1H), 4.55 (t, *J* = 4.2 Hz, 1H), 4.26 (d, *J* = 10.6 Hz, 1H), 4.20-4.14 (m, 1H), 4.10 (d, *J* = 5.0 Hz, 3H), 4.02 (s, 1H), 3.78 (d, *J* = 10.0 Hz, 1H), 3.68 (s, 2H), 3.50 (t, *J* = 10.0 Hz, 4H), 3.27 (s, 4H), 3.20 (s, 1H), 2.84 (d, *J* = 9.6 Hz, 1H), 2.78-2.55 (m, 7H), 2.34-2.12 (m, 6H), 2.04-1.86 (m, 6H), 1.68 (dd, *J* = 27.8, 13.1 Hz, 6H), 1.49-1.38 (m, 5H), 1.34-1.27 (m, 4H), 1.26-1.11 (m, 2H), 1.03 (d, *J* = 6.4 Hz, 3H), 0.96 (t, *J* = 11.8 Hz, 1H).

### Preparation Example 32: Preparation of Compound PE-ALOH

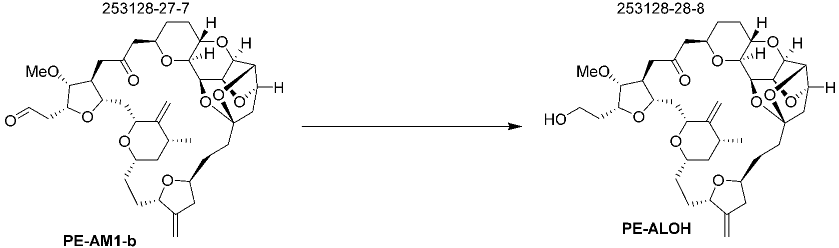

Sodium borohydride (883.78 µg, 98%, 22.89 µmol, 1 eq) was added to a solution of compound PE-AM1-b (for the synthesis of the compound, refer to the preparation example of PE-AM1) (16 mg, 22.89 µmol, 1 eq) in methanol (1 mL) and dichloromethane (0.25 mL) at -78°C. The mixture was stirred at -78°C to 25°C for 1 hour. LCMS analysis indicated the reaction ended. The reaction solution was concentrated. The residue was then purified by preparative high-performance liquid chromatography (preparative chromatograph manufacturer: Shimadzu, model: LC-20AP, column: Welch Xtimate C18 150 × 30mm × 10 µm, mobile phase: water (0.225% HCOOH)-acetonitrile, with an elution ratio of acetonitrile increased from 30% to 60%) and lyophilized, to afford compound PE-ALOH (11.48 mg, 71.54% yield) as a white solid.

LCMS (ESI): m/z, 723.4[M+Na]⁺.

¹H NMR (400 MHz, DMSO-*d*₆) δ 5.02 (d, *J =* 21.9 Hz, 2H), 4.84 (s, 1H), 4.75 (s, 1H), 4.63 (d, *J =* 3.2 Hz, 1H), 4.55 (t, *J =* 4.2 Hz, 1H), 4.41 (t, *J* = 5.3 Hz, 1H), 4.26 (d, *J =* 10.9 Hz, 1H), 4.17 (dt, *J* = 10.1, 5.0 Hz, 1H), 4.10 (t, *J* = 3.4 Hz, 3H), 4.02 (s, 1H), 3.80 (t, *J* = 10.5 Hz, 1H), 3.70 (t, *J* = 5.4 Hz, 2H), 3.55-3.49 (m, 1H), 3.48-3.36 (m, 3H), 3.26 (s, 2H), 3.23 (d, *J* = 3.4 Hz, 1H), 2.84 (d, *J* = 10.1 Hz, 1H), 2.73 (td, *J* = 19.1, 18.3, 7.5 Hz, 2H), 2.60-2.52 (m, 1H), 2.23 (ddd, *J* = 49.8, 24.5, 13.0 Hz, 6H), 1.96 (d, *J* = 33.1 Hz, 6H), 1.74-1.59 (m, 6H), 1.56-1.43 (m, 2H), 1.42-1.08 (m, 5H), 1.03 (d, *J* = 6.4 Hz, 3H), 1.00- 0.90 (m, 1H).

### Preparation Example 33: Preparation of Compound PE-AM1

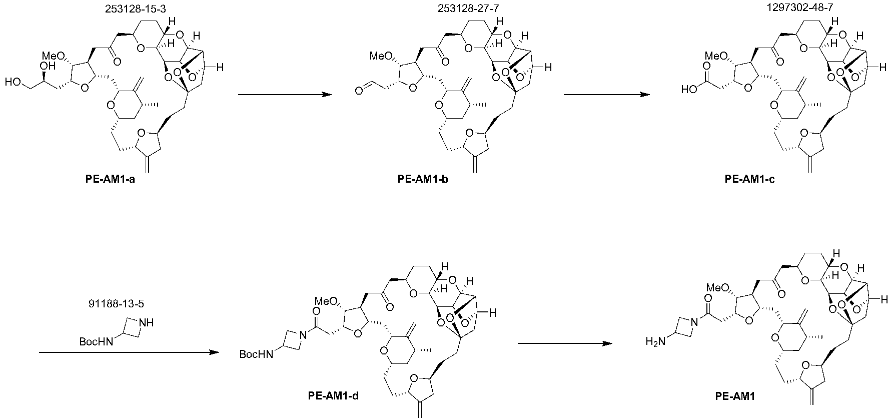

### Step 1: Preparation of compound PE-AM1-b

Sodium periodate (11.76 mg, 99.5%, 3.04 µL, 54.73 µmol, 2 eq) was added to the solution of the compound PE-AM1-a (20 mg, 27.36 µmol, 1 eq, purchased from ChemExpress) in tetrahydrofuran (1 mL) and water (0.5 mL), a resulting mixture was stirred at 25°C for 4 hours, and LCMS analysis indicated complete consumption of the starting material. The reaction solution was added to water (15 mL) and extracted with ethyl acetate (10 mL×3). The organic phases were combined and washed with saturated brine (30 mL), anhydrous sodium sulfate was added for drying, and a resulting mixture was evaporated to dryness under reduced pressure to obtain a white solid compound PE-AM1-b (27 mg, crude product). LCMS (ESI): m/z, 721.5[M+Na]⁺.

### Step 2: Preparation of compound PE-AM1-c

The solution of sodium chlorite (18.44 mg, 80%, 7.38 µL, 163.13 µmol, 6 eq) and sodium dihydrogen phosphate (6.59 mg, 99%, 54.38 µmol, 2 eq) in water (1 mL) were added to the solution of compound PE-AM1-b (19 mg, 27.19 µmol, 1 eq) and 2-methyl-2-butene (21.29 mg, 90%, 32.10 µL, 271.88 µmol, 10 eq) in tert-butanol (1 mL) at 0°C, then the reaction solution was stirred at 25°C for 1 hour, and LCMS analysis **showed** complete consumption of the starting material. The reaction solution was added to water (15 mL) and extracted with ethyl acetate (10 mL×3). The organic phases were combined and washed with saturated brine (30 mL), anhydrous sodium sulfate was added for drying, and a resulting mixture was evaporated to dryness under reduced pressure to afford compound PE-AM1-c (24 mg, crude product) as a white solid.

LCMS (ESI): m/z, 715.4[M+H]⁺, 737.4[M+Na]⁺.

### Step 3: Preparation of compound PE-AM1-d

DIPEA (10.65 mg, 99%, 13.62 µL, 81.58 µmol, 3 eq) and HATU (15.99 mg, 97%, 40.79 µmol, 1.5 eq) were added to a solution of compound PE-AM1-c (19.44 mg, 27.19 µmol, 1 eq) and 3-N-(tert-butoxycarbonyl)aminocyclobutylamine (5.79 mg, 97%, 32.63 µmol, 1.2 eq) in DMF (1 mL). The mixture was stirred at 25°C for 1 hour. LCMS analysis indicated the reaction ended. The reaction solution was added to water (20 mL) and extracted with ethyl acetate (10 mL×3). The organic phases were combined and washed with saturated brine (30 mL), anhydrous sodium sulfate was added for drying, and a resulting mixture was evaporated to dryness under reduced pressure to afford the compound PE-AM1-d (36 mg, crude product) as a yellow solid.

LCMS (ESI): m/z, 869.6[M+H]⁺, 891.6[M+Na]⁺.

### Step 4: Preparation of compound PE-AM1

Trifluoroacetic acid (0.2 mL) was added to a solution of compound PE-AM1-d (23.60 mg, 27.16 µmol, 1 eq) in dichloromethane (1 mL) at 0°C. The mixture was stirred at 0°C for 2 hours. LCMS analysis indicated the reaction ended. The reaction solution was concentrated. The residue was then purified by preparative high-performance liquid chromatography (preparative chromatograph manufacturer: Shimadzu, model: LC-20AP, column: Welch Xtimate C18 250 × 30 mm × 10 µm, mobile phase: water (0.225% HCOOH)-acetonitrile, with an elution ratio of acetonitrile increased from 30% to 60%) and lyophilized, to afford compound PE-AM1 (2.41 mg, 11.54% yield, 98.81% purity) as a white solid.

### LCMS (ESI): m/z, 769.5[M+H]⁺, 791.5[M+Na]⁺.

¹H NMR (400 MHz, DMSO-*d*₆) δ 8.33 (s, 1H), 6.19 (d, *J=* 3.0 Hz, 1H), 5.94 (d, *J =* 3.0 Hz, 1H), 4.94 (d, *J* = 2.2 Hz, 1H), 4.88-4.85 (m, 1H), 4.80 (s, 1H), 4.75 (s, 1H), 4.57 (s, 1H), 4.34 (d, *J* = 6.7 Hz, 1H), 4.29 (d, *J* = 9.7 Hz, 1H), 4.22 (dd, *J* = 13.9, 6.6 Hz, 1H), 4.08-4.00 (m, 3H), 3.95 (dd, *J* = 9.7, 7.2 Hz, 1H), 3.77 (dd, *J* = 9.8, 2.7 Hz, 1H), 3.71-3.66 (m, 3H), 3.61 (ddd, *J* = 12.7, 7.8, 4.4 Hz, 3H), 3.54-3.50 (m, 1H), 3.43 (s, 2H), 3.21 (d, *J* = 6.5 Hz, 4H), 2.97 (dd, *J* = 9.3, 2.1 Hz, 1H), 2.81-2.73 (m, 2H), 2.66 (tt, *J* = 4.4, 1.8 Hz, 1H), 2.62-2.54 (m, 3H), 2.44-2.40 (m, 1H), 2.34-2.29 (m, 3H), 2.28-2.23 (m, 1H), 2.11 (ddd, *J* = 16.1, 6.1, 3.6 Hz, 1H), 2.02-1.89 (m, 3H), 1.87-1.82 (m, 1H), 1.74-1.67 (m, 3H), 1.60 (ddd, *J* = 17.3, 8.4, 3.6 Hz, 3H), 1.44 (tt, *J* = 7.7, 4.5 Hz, 2H), 1.34-1.28 (m, 2H), 1.23 (d, *J* = 3.6 Hz, 2H), 1.03 (d, *J* = 6.4 Hz, 3H), 0.94 (d, *J* = 11.9 Hz, 1H).

### Preparation Example 34: Preparation of Compound PE-AM2

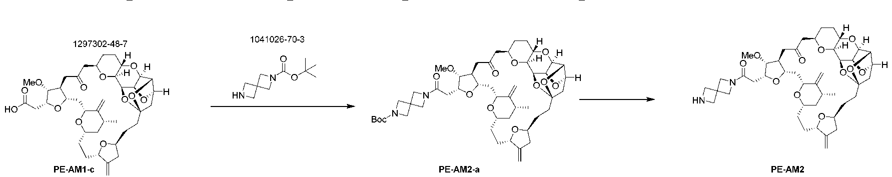

### Step 1: Preparation of compound PE-AM2-a

DIPEA (8.77 mg, 99%, 11.21 L, 67.15 µmol, 3 eq) and HATU (13.16 mg, 97%, 33.57 µmol, 1.5 eq) were added to a solution of compound PE-AM1-c (for the synthesis of the compound, refer to the Preparation Example of PE-AM1) (16 mg, 22.38 µmol, 1 eq) and tert-butyl 2,6-diazaspiro[3.3]heptane-2-carboxylate (5.61 mg, 95%, 26.86 µmol, 1.2 eq) in DMF (1 mL). The mixture was stirred at 25°C for 1 hour. LCMS analysis indicated the reaction ended. The reaction solution was added to water (20 mL) and extracted with ethyl acetate (10 mL×3). The organic phases were combined and washed with saturated brine (30 mL), anhydrous sodium sulfate was added for drying, and a resulting mixture was evaporated to dryness under reduced pressure to afford the crude product. The crude product was purified by preparative high-performance liquid chromatography (preparative chromatograph manufacturer: Shimadzu, model: LC-20AP, column: Welch Xtimate C18 250 × 30 mm × 10 µm, mobile phase: water (0.225% HCOOH)-acetonitrile, with an elution ratio of acetonitrile increased from 30% to 60%) and lyophilized, to afford compound PE-AM2-a (6 mg, 29.96% yield) as a white solid.

LCMS (ESI): m/z, 895.4[M+H]⁺, 917.4[M+Na]⁺.

### Step 2: Preparation of compound PE-AM2

Trifluoroacetic acid (0.2 mL) was added to a solution of compound PE-AM2-a (6 mg, 6.70 µmol, 1 eq) in dichloromethane (1 mL) at 0°C. The mixture was stirred at 0°C for 2 hours. LCMS analysis indicated the reaction ended. The reaction solution was concentrated. The residue was then purified by preparative high-performance liquid chromatography (preparative chromatograph manufacturer: Shimadzu, model: LC-20AP, column: Synergi Max-RP 200 × 30mm × 10 µm, mobile phase: water (0.225% HCOOH)-acetonitrile, with an elution ratio of acetonitrile increased from 30% to 60%) and lyophilized, to afford compound PE-AM2 (1.46 mg, 27.40% yield, 95% purity) as a white solid.

LCMS (ESI): m/z, 795.4[M+H]⁺, 817.4[M+Na]⁺.

¹H NMR (400 MHz, DMSO-*d*₆) δ 6.19 (d, *J* = 3.0 Hz, 1H), 5.94 (d, *J* = 3.0 Hz, 1H), 4.95 (s, 1H), 4.87 (s, 1H), 4.80 (s, 1H), 4.76 (s, 1H), 4.57 (s, 1H), 4.33 (s, 1H), 4.29 (d, *J* = 9.7 Hz, 1H), 4.19 (t, *J* = 7.9 Hz, 2H), 4.02 (dd, *J* = 10.1, 6.0 Hz, 2H), 3.91 (s, 2H), 3.77 (d, *J* = 9.3 Hz, 1H), 3.70 (d, *J* = 7.1 Hz, 4H), 3.61 (d, *J =* 6.4 Hz, 2H), 3.51 (s, 2H), 3.43 (d, *J =* 3.8 Hz, 2H), 3.20 (s, 2H), 2.97 (dd, *J* = 9.4, 2.1 Hz, 1H), 2.78 (dd, *J* = 17.3, 6.5 Hz, 2H), 2.67 (p, *J* = 1.9 Hz, 1H), 2.60 (dd, *J* = 11.7, 6.5 Hz, 2H), 2.56 (s, 1H), 2.45-2.41 (m, 2H), 2.33-2.29 (m, 3H), 2.26 (s, 2H), 2.12 (d, *J* = 8.7 Hz, 1H), 1.95 (s, 2H), 1.85 (d, *J =* 12.1 Hz, 1H), 1.70 (dd, *J =* 12.9, 6.3 Hz, 3H), 1.62 (dd, *J* = 10.0, 5.3 Hz, 3H), 1.43 (s, 2H), 1.31 (t, *J =* 9.3 Hz, 2H), 1.24 (d, *J =* 3.5 Hz, 2H), 1.04 (d, *J* = 6.5 Hz, 3H), 0.95 (d, *J =* 11.9 Hz, 1H).

### Preparation Example 35: Preparation of Compound PE-AM3

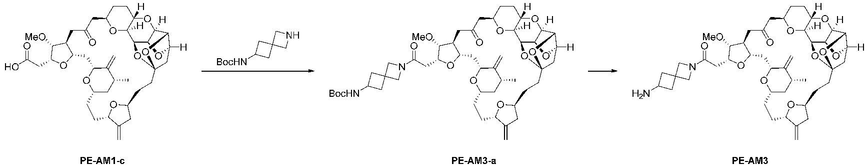

### Step 1: Preparation of compound PE-AM3-a

HATU (12.77 mg, 33.57 µmol, 1.2 eq) and DIPEA (10.96 mg, 83.93 µmol, 3 eq) were added to a solution of compound PE-AM1-c (20 mg, crude; for the synthesis of the compound, refer to the preparation example of PE-AM1) and tert-butyl N-2-azaspiro[3.3]hept-6-ylcarbamate (6.74 mg, 30.78 µmol, 1.1 eq) in DMF (2 mL). The reaction solution was stirred overnight at room temperature. LCMS analysis showed that the reaction ended. The mixture was purified by preparative high-performance liquid chromatography (preparative chromatograph manufacturer: Orienda, model: BRIX-2860-R6, column: Welch Xtimate C18 150 × 30 mm × 10 µm, mobile phase: water (0.225% HCOOH)-acetonitrile, with an elution ratio of acetonitrile increased from 40% to 70%), to afford the compound PE-AM3-a (11.8 mg, 46.39% yield) as a colorless oil. LCMS (ESI): m/z, 909.5[M+H]⁺.

### Step 2: Preparation of compound PE-AM3

TFA (0.4 mL) was added to a solution of compound PE-AM3-a (11.8 mg, 12.98 µmol, 1 eq) in DCM (2 mL) at 0°C. The reaction solution was stirred at room temperature for half an hour. LCMS analysis showed complete consumption of the starting material. The mixture was evaporated to dryness under reduced pressure. The mixture was purified by preparative high-performance liquid chromatography (preparative chromatograph manufacturer: Orienda, model: BRIX-2860-R6, column: Welch Xtimate C18 250 × 30 mm × 10 µm, mobile phase: water (0.225% HCOOH)-acetonitrile, with an elution ratio of acetonitrile increased from 30% to 60%), to afford the compound PE-AM3 (3.98 mg, 35.74% yield) as a white solid. LCMS (ESI): m/z, 809.5[M+H]⁺.

¹H NMR (400 MHz, DMSO-*d*₆) δ 8.34 (d, *J* = 2.0 Hz, 1H), 6.19 (d, *J =* 3.2 Hz, 1H), 5.94 (d, *J =* 3.2 Hz, 1H), 4.96-4.74 (m, 5H), 4.36-4.26 (m, 2H), 4.14-3.98 (m, 5H), 3.83-3.72 (m, 4H), 3.71-3.67 (m, 2H), 3.61-3.57 (m, 2H), 3.20 (d, *J* = 1.6 Hz, 4H), 3.00-2.94 (m, 1H), 2.81-2.72 (m, 2H), 2.69-2.53 (m, 4H), 2.46-2.20 (m, 9H), 2.16-2.07 (m, 1H), 2.02-1.81 (m, 6H), 1.75-1.55 (m, 6H), 1.43 (d, *J* = 5.6 Hz, 2H), 1.35-1.27 (m, 2H), 1.09-0.88 (m, 5H).

### Preparation Example 36: Preparation of Compound PE-AM4

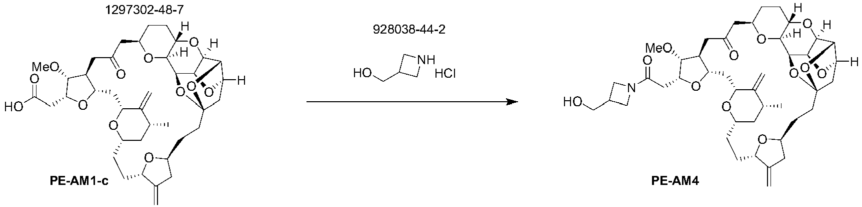

DIPEA (8.77 mg, 99%, 11.21 L, 67.15 µmol, 3 eq) and HATU (10.53 mg, 97%, 26.86 µmol, 1.2 eq) were added to a solution of compound PE-AM1-c (for the synthesis of the compound, refer to the Preparation Example of PE-AM1) (16 mg, 22.38 µmol, 1 eq) and 3-(hydroxymethyl)azetidine hydrochloride (3.42 mg, 97%, 26.86 µmol, 1.2 eq) in DMF (1 mL). The mixture was stirred at 25°C for 1 hour. LCMS analysis indicated the reaction ended. The reaction solution was purified by preparative high-performance liquid chromatography (preparative chromatograph manufacturer: Shimadzu, model: LC-20AP, column: YMC-Triart Prep C18 150 × 30 mm × 10 µm, mobile phase: water (0.225% HCOOH)-acetonitrile, with an elution ratio of acetonitrile increased from 30% to 60%) and lyophilized, to afford compound PE-AM4 (11.14 mg, 63.49% yield) as a white solid.

LCMS (ESI): m/z, 784.4[M+H]⁺, 806.4[M+Na]⁺.

¹H NMR (400 MHz, DMSO-*d*₆) δ 5.11-4.96 (m, 2H), 4.90-4.70 (m, 3H), 4.63 (dt, *J* = 4.6, 2.6 Hz, 1H), 4.55 (t, *J =* 4.2 Hz, 1H), 4.26 (d, *J =* 10.8 Hz, 1H), 4.17 (dt, *J =* 10.2, 5.0 Hz, 1H), 4.14-4.06 (m, 4H), 4.05-3.96 (m, 2H), 3.84-3.75 (m, 3H), 3.70 (ddd, *J* = 9.5, 5.7, 2.8 Hz, 1H), 3.51 (td, *J =* 11.5, 10.4, 4.8 Hz, 5H), 3.31 (t, *J=* 3.9 Hz, 1H), 3.26 (s, 3H), 2.86-2.71 (m, 3H), 2.64-2.53 (m, 2H), 2.36-2.18 (m, 7H), 2.12 (t, *J* = 11.9 Hz, 1H), 2.04-1.86 (m, 6H), 1.74-1.59 (m, 4H), 1.54-1.41 (m, 2H), 1.35-1.13 (m, 4H), 1.03 (d, *J =* 6.4 Hz, 3H), 1.00-0.91 (m, 1H).

### Preparation Example 37: Preparation of Compound PE-AM5

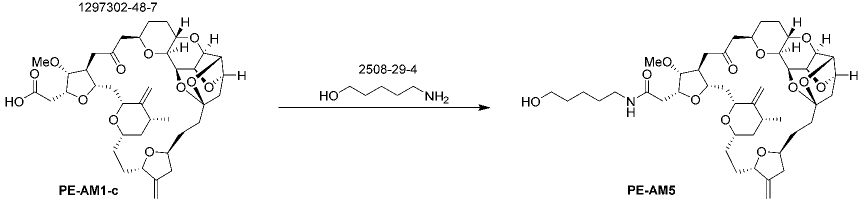

DIPEA (8.77 mg, 99%, 11.21 L, 67.15 µmol, 3 eq) and HATU (13.16 mg, 97%, 33.57 µmol, 1.5 eq) were added to a solution of compound PE-AM1-c (for the synthesis of the compound, refer to the Preparation Example of PE-AM1) (16 mg, 22.38 µmol, 1 eq) and 5-aminopentan-1-ol (2.92 mg, 95%, 26.86 µmol, 1.2 eq) in DMF (1 mL). The mixture was stirred at 25°C for 1 hour. LCMS analysis indicated the reaction ended. The reaction solution was purified by preparative high-performance liquid chromatography (preparative chromatograph manufacturer: Shimadzu, model: LC-20AP, column: Welch Xtimate C18 150 × 30 mm × 10 µm, mobile phase: water (0.225% HCOOH)-acetonitrile, with an elution ratio of acetonitrile increased from 20% to 50%) and lyophilized, to afford compound PE-AM5 (10.86 mg, 60.65% yield) as a white solid.

LCMS (ESI): m/z, 800.5[M+H]⁺, 822.4[M+Na]⁺.

¹H NMR (400 MHz, DMSO-*d*₆) δ 7.78 (t, *J* = 5.6 Hz, 1H), 5.09-4.99 (m, 2H), 4.78 (d, *J=* 25.4 Hz, 2H), 4.63 (dt, *J* = 4.6, 2.5 Hz, 1H), 4.55 (t, *J* = 4.2 Hz, 1H), 4.34 (t, *J* = 5.1 Hz, 1H), 4.26 (d, *J* = 10.9 Hz, 1H), 4.17 (td, *J* = 11.0, 10.6, 4.5 Hz, 1H), 4.10 (t, *J =* 3.3 Hz, 2H), 3.99 (dt, *J* = 8.2, 4.8 Hz, 2H), 3.79 (t, *J* = 10.4 Hz, 1H), 3.68 (s, 1H), 3.56 - 3.47 (m, 2H), 3.38 (s, 2H), 3.29 (d, *J* = 3.5 Hz, 1H), 3.25 (s, 2H), 3.00 (dp, *J =* 19.6, 6.3 Hz, 2H), 2.85-2.72 (m, 3H), 2.56 (dd, *J =* 16.6, 10.0 Hz, 1H), 2.43-2.37 (m, 1H), 2.35-2.19 (m, 6H), 2.12 (t, *J =* 11.9 Hz, 1H), 2.01-1.84 (m, 6H), 1.67 (q, *J* = 11.6 Hz, 4H), 1.56-1.46 (m, 2H), 1.40-1.34 (m, 4H), 1.29-1.22 (m, 4H), 1.20-1.12 (m, 1H), 1.03 (d, *J =* 6.4 Hz, 3H), 0.94 (d, *J =* 6.6 Hz, 1H), 0.87-0.81 (m, 3H).

### Preparation Example 38: Preparation of Compound PE-O2K

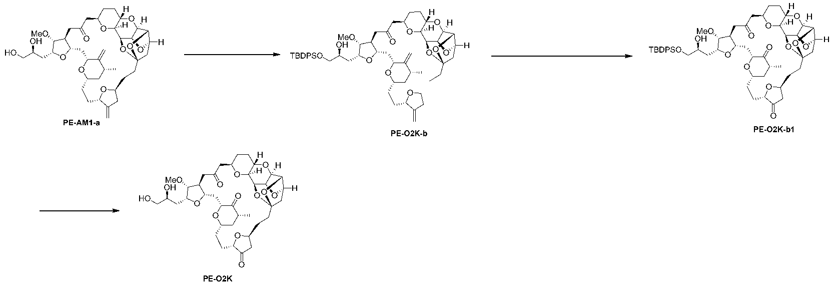

### Step 1: Preparation of compound PE-O2K-b

Compound TBDPSCl (378 mg, 1.373 mmol, 6.7 eq) and imidazole (97.8 mg, 1.437 mmol, 7 eq) were added to a solution of compound PE-AM1-a (purchased from ChemExpress) (150 mg, 0.205 mmol, 1 eq) in DMF (3 mL). After the addition, the mixture was stirred at 25°C for 1 hour. LCMS analysis showed that the reaction ended. The reaction solution was filtered. Water (30 mL) was added to the filtrate, and the mixture was then extracted with EtOAc (50 mL × 3). The organic phases were combined and washed with brine (30 mL), dried over anhydrous sodium sulfate, and filtered to obtain the filtrate. The filtrate was concentrated under reduced pressure to afford a crude product. The residue was purified by reversed-phase column chromatography (ACN: H₂O = 0:100 to 100:0) to afford the product as a yellow liquid (98.0 mg, 49% yield).

LCMS (ESI): m/z, 991.3 [M+Na]⁺, Rt=2.075 min.

### Step 2: Preparation of compound PE-O2K-b1

Potassium osmate dihydrate (4 mg, 0.010 mmol, 0.1 eq) and NaIO₄ (433 mg, 2.022 mmol, 20 eq) were added to the solution of the compound PE-O2K-b (98 mg 0.101 mmol, 1 eq) in THF (4 mL) and water (1 mL) at 0°C and the reaction solution was stirred at 25°C for 2 hours. NaIO₄ (433 mg, 2.022 mmol, 20 eq) was added during the reaction, the reaction solution was stirred overnight at room temperature, and the LCMS showed that the reaction completed. The reaction solution was filtered. Water (30 mL) was added to the filtrate, and the mixture was then extracted with EtOAc (50 mL × 3). The organic phases were combined and washed with brine (30 mL), dried over anhydrous sodium sulfate, and filtered to obtain the filtrate. The filtrate was concentrated under reduced pressure to afford the product PE-O2K-b1 as a yellow liquid (130 mg, crude).

LCMS (ESI): m/z, 994.7[M+Na]⁺, Rt=1.822 min.

### Step 3: Preparation of compound PE-O2K

Triethylamine trihydrofluoride (0.2 mL) was added to a solution of compound PE-O2K-b1 (90.0 mg, 0.092 mmol, 1 eq) in DMF (4 mL) at room temperature. The reaction solution was stirred at 65°C for 2.5 hours. LCMS analysis showed nearly complete consumption of the starting material. The reaction solution was concentrated, and the residue was subjected to preparative high-performance liquid chromatography to obtain the product PE-O2K (9.28 mg, 14% yield) as a white solid.

The conditions of the preparative high-performance liquid chromatography were as follows:
Preparative chromatograph manufacturer: Shimadzu, model: JB-C235-04, column: Prime C18 30 × 150 mm × 5 µm, mobile phase: water (0.1% TFA)-ACN, with an elution ratio of acetonitrile increased from 23% to 43%.

LCMS (ESI): m/z, 735.2[M+H]⁺, Rt=1.140 min.

¹H NMR (400 MHz, DMSO-*d*₆) δ 4.65 (t, *J* = 4.4 Hz, 1H), 4.56 (d, *J* = 4.2 Hz, 1H), 4.39 (s, 1H), 4.12 (dd, *J* = 12.0, 10.6 Hz, 4H), 3.86 (d, *J* = 8.8 Hz, 1H), 3.77 (ddd, *J* = 14.2, 9.4, 5.2 Hz, 4H), 3.56 - 3.48 (m, 2H), 3.26 (d, *J* = 7.8 Hz, 8H), 2.85 (ddd, *J* = 13.8, 8.0, 5.1 Hz, 2H), 2.79 - 2.72 (m, 1H), 2.68 - 2.53 (m, 2H), 2.39 - 1.89 (m, 10H), 1.74 (ddd, *J =* 14.6, 12.2, 8.2 Hz, 4H), 1.67 - 1.38 (m, 8H), 1.31 - 1.20 (m, 2H), 0.94 (d, *J* = 6.6 Hz, 2H).

### Preparation Example 39: Preparation of Compound PY-4car2-b

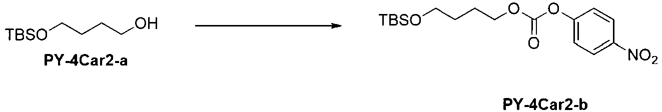

N,N-diisopropylethylamine (2.43 mL, 14.68 mmol, 3 eq) was added to a solution of 4-((tert-butyldimethylsilyl)oxy)but-1-ol (1g, 4.89 mmol, 1 eq) and bis(p-nitrobenzene)carbonate (2.98g, 9.79 mmol, 2 eq) in N,N-dimethylformamide (15 mL), and a resulting mixture was stirred overnight at room temperature. The reaction solution was concentrated, diluted with dichloromethane (10 mL), the organic phase was washed with water (10 mL) 3 times and washed with saturated brine (10 mL) once, and the organic phase was dried with anhydrous sodium sulfate, filtered, and concentrated. The residue was purified by silica gel column chromatography (eluent: ethyl acetate/petroleum ether = 1:10, dichloromethane/petroleum ether = 40%) to obtain a product 4-((tert-butyldimethylsilyl)oxy)butyl(4-nitrophenyl)carbonate (PY-4car2-b) (1.63g, yield = 90.2%).

### Preparation Example 40: Preparation of Compound LY-46F-c

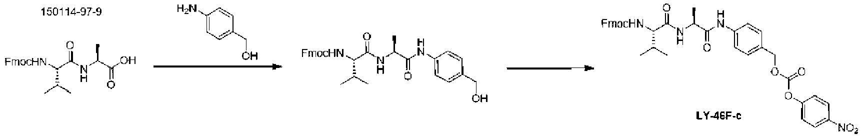

### Step 1: Preparation of (9H-fluoren-9-yl)methyl((S)-1-(((S)-1-(4-(hydroxymethyl)phenyl) amino)-1-oxopropane-2-yl)amino)-3-methyl-1-oxobutane-2-yl)carbamate

The compound (((9H-fluoren-9-yl)methoxy)carbonyl)-L-valine-L-alanine (1g, 2.44 mmol, 1 eq) was dissolved in DCM (20 mL)/MeOH (10 mL), and (4-aminophenyl)methanol (360.04 mg, 2.92 mmol, 1.2 eq) and EEDQ (2-ethoxy-1-ethoxycarbonyl-1,2-dihydroquinoline, 903.71 mg, 753.09 µL, 3.65 mmol, 1.5 eq) were added. The reaction solution was stirred for 12 hours at 25°C. The reaction solution was concentrated under reduced pressure, the obtained solid was triturated with petroleum ether/ethyl acetate (ratio: 3:1, total volume: 30 mL), and the filter cake was washed with petroleum ether/ethyl acetate (ratio: 3:1, total volume: 50 mL), to obtain the yellow solid product (9H-fluoren-9-yl)methyl((S)-1-(((S)-1-(4-(hydroxymethyl)phenyl)amino)-1-oxopropane-2-yl)amino)-3-methyl-1-oxobutane-2-yl)carbamate (1.10g, 87.57% yield).

LCMS(ESI): m/z, 538.2 [M+Na]⁺.

### Step 2: Preparation of compound LY-46F-c

(9H-fluoren-9-yl)methyl((S)-1-(((S)-1-(4-(hydroxymethyl)phenyl)amino)-1-oxopropane-2-yl)amino)-3-methyl-1-oxobutane-2-yl)carbamate (500 mg, 969.74 µmol, 1 eq) was dissolved in DMF (6 mL), and bis(4-nitrophenyl)carbonate (590.01 mg, 393.34 µL, 1.94 mmol, 2 eq) and DIPEA (375.99 mg, 480.80 µL, 2.91 mmol, 3 eq) were added to the solution. The reaction solution allowed to react for 16 hours at 25°C. The reaction solution was concentrated under reduced pressure, and the residue was purified by silica gel column chromatography (eluents: ethyl acetate/petroleum ether = 1:30-1:1), to obtain a yellow solid compound LY-46F-c (380 mg, 57.57% yield).

LCMS(ESI): m/z, 703.3 [M+Na]⁺.

### Preparation Example 41: Preparation of Compound LY-22CD-3-d

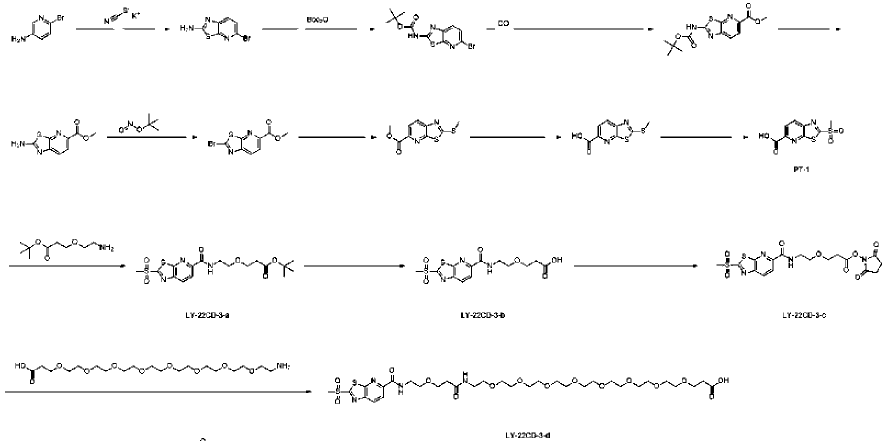

### Step 1: Preparation of 5-bromothiazolo[5,4-b]pyridine-2-amine

A solution (20 mL) of liquid bromine in acetic acid was added to a solution (100 mL) of 5-amino-2-bromopyridine (10g, 57.80 mmol, 1 eq) and potassium thiocyanate (22.47g, 11.91 mL, 231.20 mmol, 4 eq) in acetic acid at 0°C and the reaction solution was stirred for 3 hours at 20°C. The reaction solution was quenched with water (50 mL) and filtered to obtain a filtrate, and the filtrate was concentrated under reduced pressure to obtain a crude product. The crude product was alkalized with the aqueous solution of sodium bicarbonate until pH reached 8, a resulting solution was extracted with ethyl acetate (50 mL × 4), and organic phases were combined, dried with anhydrous sodium sulfate, and concentrated under reduced pressure, to obtain 5-bromothiazolo [5,4-b]pyridine-2-amine (9.80g) as a yellow solid.

LCMS(ESI): m/z, 230.0[M+H]⁺.

¹H NMR (400 MHz, DMSO-*d⁶*) δ 7.96 (s, 2H), 7.57 (d, *J* = 8.4 Hz, 1H), 7.43 (d, *J* = 8.4 Hz, 1H).

### Step 2: Preparation of (5-bromothiazolo[5,4-b]pyridin-2-yl)tert-butyl carbamate

5-bromothiazolo[5,4-b]pyridine-2-amine (7.80g, 33.90 mmol, 1 eq)/triethylamine (4.46g, 6.13 mL, 44.07 mmol, 1.3 eq)/4-dimethylaminopyridine (414.15 mg, 414.15 µL, 3.39 mmol, 0.1 eq) and di-t-butyl dicarbonate (9.62g, 9.43 mL, 44.07 mmol, 1.3 eq) were added to a solution of tetrahydrofuran (320 mL) at room temperature and the solution was stirred at 25°C for 16 hours. After the reaction solution was concentrated, the residue was purified by silica gel column chromatography (eluents: ethyl acetate/petroleum ether = 40%-50%), to obtain the product (5-bromothiazolo[5,4-b]pyridin-2-yl)tert-butyl carbamate (7.0g, 21.20 mmol, two-step yield = 26%).

LCMS(ESI): m/z, 330.0[M+H]⁺.

¹H NMR (400 MHz, DMSO-*d*₆) δ 12.12 (s, 1H), 7.99 (d, *J* = 8.4 Hz, 1H), 7.64 (d, *J* = 8.4 Hz, 1H), 1.52 (s, 9H).

### Step 3: Preparation of methyl 2-((tert-butoxycarbonyl)amino)thiazolo[5,4-b]pyridin-5-carboxylate

(5-bromothiazolo[5,4-b]pyridin-2-yl)tert-butyl carbamate (6g, 18.17 mmol, 1 eq), potassium carbonate (7.53g, 3.10 mL, 54.51 mmol, 3 eq), palladium acetate (1.22g, 5.45 mmol, 0.3 eq), and 1,3-bis(diphenylphosphino)propane (1.80g, 4.36 mmol, 0.24 eq) were sequentially added to a solution of methanol (120 mL) and N, N-dimethylformamide (60 mL) at room temperature. The reaction solution was stirred at 60°C for 16 hours in a carbon monoxide atmosphere. After the reaction solution was concentrated, the residue was purified by silica gel column chromatography (eluents: ethyl acetate/petroleum ether = 40%-80%), to obtain the product methyl 2-((tert-butoxy carbonyl)amino)thiazolo[5,4-b]pyridin-5-carboxylate (2.20g, 7.11 mmol, 39% yield).

LCMS(ESI): m/z, 310.1[M+H]⁺.

¹H NMR (400 MHz, DMSO-*d*₆) δ 12.26 (s, 1H), 8.25-8.04 (m, 2H), 3.90 (s, 3H), 1.53 (s, 9H).

### Step 4: Preparation of methyl 2-aminothiazolo[5,4-b]pyridin-5-carboxylate

Trifluoroacetic acid (40 mL) was added to a solution of methyl 2-((tert-butoxycarbonyl) amino)thiazolo[5,4-b]pyridin-5-carboxylate (2.20g, 7.11 mmol, 1 eq) in dichloromethane (80 mL) at 0°C and the reaction solution was stirred for 2 hours at 20°C. After the reaction solution was concentrated, the aqueous solution of saturated sodium bicarbonate was added to the residue, then the reaction solution was filtered, and the filter cake was concentrated under reduced pressure to obtain methyl 2-aminothiazolo[5,4-b]pyridin-5-carboxylate (1.38g, 6.60 mmol, 39% yield).

LCMS(ESI): m/z, 210.1[M+H]⁺.

¹H NMR (400 MHz, DMSO-*d*₆) δ 8.27 (s, 2H), 7.95 (d, *J* = 8.4 Hz, 1H), 7.68 (d, *J* = 8.4 Hz, 1H), 3.85 (s, 3H).

### Step 5: Preparation of methyl 2-bromothiazolo[5,4-b]pyridin-5-carboxylate

Tert-butyl nitrite (1.22 g, 1.42 mL, 11.87 mmol, 1.8 eq) and methyl 2-aminothiazolo[5,4-b] pyridin-5-carboxylate (1.38g, 6.60 mmol, 1 eq) were added to the solution of copper bromide (1.84 g, 386.06 µL, 8.24 mmol, 1.25 eq) in acetonitrile (60 mL) at 0°C, and the reaction solution was stirred at 25°C for 16 hours. After the reaction solution was concentrated, the residue was purified by silica gel column chromatography (eluents: ethyl acetate/petroleum ether = 20%-40%), to obtain the product methyl 2-bromothiazolo[5,4-b]pyridin-5-carboxylate (1.45g, 5.31 mmol, 81% yield).

LCMS(ESI): m/z, 273.1[M+H]⁺.

¹H NMR (400 MHz, Chloroform-*d*) δ 8.37-8.27 (m, 2H), 4.07 (s, 3H).

### Step 6: Preparation of methyl 2-(methylthio)thiazolo[5,4-b]pyridin-5-carboxylate

A solution of methyl 2-bromo-[1,3]thiazolo[5,4-b]pyridin-5-carboxylate (375 mg, 1.37 mmol, 1 eq) and sodium methyl mercaptan (240.61 mg, 3.43 mmol, 2.5 eq) in ethylene glycol dimethyl ether (10 mL) was stirred for 2 hours at -15°C. The aqueous solution of saturated ammonium chloride (5 mL) was added to the reactant, a resulting solution was concentrated, tetrahydrofuran (30 mL) was added, the resulting solution was filtered, and the filter cake was collected and purified by preparative high-performance liquid chromatography (preparative chromatograph manufacturer: Orienda, model: BRIX-2860-R1, column: Welch Xtimate C18 150 × 30 mm × 10 µm, mobile phase: water (0.225% HCOOH)-acetonitrile, with an elution ratio of acetonitrile increased from 30% to 60%), to obtain white solids of methyl 2-(methylthio)thiazolo [5,4-b]pyridin-5-carboxylate (180 mg, 25.89% yield) and methyl 2-(methylthio)thiazolo[5,4-b] pyridin-5-carboxylate (100 mg, 15.28% yield).

LCMS (ESI): m/z, 241.1[M+H]⁺.

### Step 7: Preparation of 2-(methylsulfanyl)thiazolo[5,4-b]pyridine-5-carboxylic acid

A solution of compound methyl 2-(methylthio)thiazolo[5,4-b]pyridin-5-carboxylate (180 mg, 749.06 µmol, 1 eq) and lithium hydroxide monohydrate (62.92 mg, 41.67 µL, 1.50 mmol, 2 eq) in tetrahydrofuran (2 mL) was mixed with water (2 mL) at room temperature, the solution was stirred at 20°C for 12 hours, the reaction solution was concentrated, water (50 mL) and aqueous hydrochloric acid (2M) were added until pH was below 5, the reaction solution was filtered and washed with water (3 mL × 2), and the filter cake was collected to obtain the product 2-(methyl sulfanyl)thiazolo[5,4-b]pyridine-5-carboxylic acid (169 mg, crude product).

LCMS(ESI): m/z, 227.1[M+H]⁺.

### Step 8: Preparation of 2-methylsulfonylthiazolo[5,4-b]pyridine-5-carboxylic acid

Potassium peroxymonosulfate sulfate (3.29g, 9.51 mmol, 8 eq) was added to the solution of 2-(methylsulfanyl)thiazolo[5,4-b]pyridine-5-carboxylic acid (269 mg, 1.19 mmol, 1 eq) in tetrahydrofuran (10 mL) and water (10 mL) and the mixture was stirred at 20°C for 2 hours. Ethyl acetate (30 mL) and H₂O (20 mL) were added to the mixture, and the aqueous phase was extracted with ethyl acetate (20 mL × 3). The combined organic layers were washed with saturated brine (10 mL), dried with anhydrous sodium sulfate, filtered and concentrated, and the residue was purified by preparative high-performance liquid chromatography (preparative chromatograph manufacturer: Orienda, model: BRIX-2860, column: YMC-Triart Prep C18 150 × 30 mm × 10 µm, mobile phase: water (0.225% HCOOH)-acetonitrile, with an elution ratio of acetonitrile increased from 10% to 40%), to obtain the product 2-methylsulfonylthiazolo[5,4-b]pyridine-5-carboxylic acid (220 mg, 71.65% yield).

LCMS(ESI): m/z, 259.1 [M+H]⁺.

### Step 9: Preparation of compound LY-22CD-3-a

N,N-diisopropylethylamine (80.07 mg, 102.38 µL, 619.51 µmol, 2 eq) and N,N,N',N'-tetramethyl-O-(7-azabenzotriazole-1-yl)hexafluorophosphate (141.34 mg, 371.70 µmol, 1.2 eq) were added to the solution of compounds tert-butyl 3-(2-aminoethoxy)propionate (70.34 mg, 371.70 µmol, 1.2 eq) and 2-methylsulfonylthiazolo[5,4-b]pyridine-5-carboxylic acid (80 mg, 309.75 µmol, 1 eq) in N,N-dimethylformamide (4 mL), and the reaction solution was stirred at room temperature for 1 hour. The reaction solution was filtered and then purified by preparative high-performance liquid chromatography (preparative chromatograph manufacturer: Orienda, model: BRIX-2860-R6, column: Synergi Max-RP 250 × 40 mm × 10 µm, mobile phase: water (0.225% HCOOH)-acetonitrile, with an elution ratio of acetonitrile increased from 30% to 60%), to obtain compound LY-22CD-3-a (114.3 mg, 85.91% yield).

LCMS(ESI): m/z, 452[M+Na]⁺.

### Step 10: Preparation of compound LY-22CD-3-b

Trifluoroacetic acid (1 mL) was added to a solution of compound LY-22CD-3-a (114.30 mg, 266.12 µmol, 1 eq) in dichloromethane (5 mL) in the ice bath and then the reaction solution was stirred for 2 hours at room temperature. The reaction solution was concentrated under reduced pressure to obtain a crude product (150 mg).

LCMS(ESI): m/z, 396[M+Na]⁺.

### Step 11: Preparation of compound LY-22CD-3-c

N-hydroxysuccinimide (183.77 mg, 1.60 mmol, 6 eq) and N,N'-diisopropylcarbodiimide (201.51 mg, 248.77 µL, 1.60 mmol, 6 eq) were added to the solution of compound LY-22CD-3-b (99.37 mg, 266.12 µmol, 1 eq) in N,N-dimethylformamide (4 mL) in the ice bath and then the reaction solution was stirred at 50°C for 2 hours. The reaction solution was filtered and then purified by preparative high-performance liquid chromatography (preparative chromatograph manufacturer: Orienda, model: BRIX-2860, column: YMC-Triart Prep C18 150 × 30 mm × 10 µm, mobile phase: water (0.225% HCOOH)-acetonitrile, with an elution ratio of acetonitrile increased from 10% to 40%), to obtain compound LY-22CD-3-c (100 mg, 79.87% yield).

LCMS(ESI): m/z, 493[M+Na]⁺.

### Step 12: Preparation of compound LY-22CD-3-d

N,N-diisopropylethylamine (54.94 mg, 70.26 µL, 425.11 µmol, 2 eq) was added to a solution of compound LY-22CD-3-c (100 mg, 212.55 µmol, 1 eq) and 27-amino-4,7,10,13,16,19, 22,25-octaoxaheptacosanoic acid (112.61 mg, 102.37 µL, 255.06 µmol, 1.2 eq) in N,N-dimethyl formamide (3 mL). The reaction solution was stirred at room temperature for 1 hour. The reaction solution was filtered and then purified by preparative high-performance liquid chromatography (preparative chromatograph manufacturer: Shimadzu, model: LC-20AP, column: Welch Xtimate C18 150 × 30mm × 10 µm, mobile phase: water (0.225% HCOOH)-acetonitrile, with an elution ratio of acetonitrile increased from 10% to 40%), to obtain compound LY-22CD-3-d (48.4 mg, 28.57% yield).

LCMS(ESI): m/z, 797[M+H]⁺.

### Preparation Example 42: Preparation of Compound LY-5a

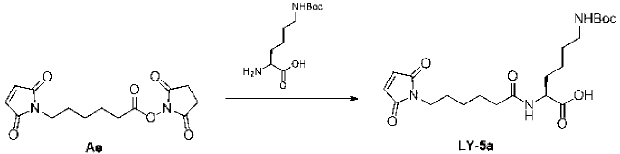

### Preparation of N⁶-(tert-butoxycarbonyl)-N²-(6-(2,5-dioxo-2,5-dihydro-1H-pyrrole-1-yl) hexanoyl)-L-lysine (LY -5a)

N⁶-(tert-butoxycarbonyl)-L-lysine (300 mg, 1.0 eq), 2,5-dioxopyrrolidin-1-yl-6-(2,5-dioxo-2,5-dihydro-1H-pyrrole-1-yl)hexanoate (Ae) (375 mg, 1.0 eq) and DIPEA (315 mg, 2 eq) were added to the reaction flask in the nitrogen atmosphere, then 10 mL of anhydrous DMF was added, nitrogen displacement was performed three times, and the reaction solution allowed to react at room temperature for 16 hours. The reaction solution was isolated by preparative high-performance liquid chromatography (Orienda, BRIX-2860; column: Phenomenex Luna C18 250 × 50 mm × 10 µm; mobile phase: water (0.225% trifluoroacetic acid)-acetonitrile, eluted from 45% to 85%), to obtain 400 mg of a colorless colloidal compound LY-5a.

LCMS(ESI): m/z, 340.2 [M-100+1]⁺, 462.3 [M+Na]⁺.

### Preparation Example 43: Preparation of compound LY-82

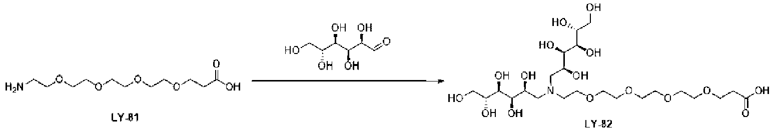

### Preparation of (18S, 19R,20R,21R)-18, 19,20,21,22-pentahydroxy-16-(2S,3R,4R,5R)-2,3,4, 5,6-pentahydroxyhexyl)-4,7,10,13-tetraoxa-16-azadocosanoic acid (LY-82)

(2R,3S,4R,5R)-2,3,4,5,6-pentahydroxyhexanal (D-glucose) (1.36g, 7.54 mmol, 10 eq) and NaBH₃CN (237 mg, 3.77 mmol, 5 eq) were added to a solution of 1-amino-3,6,9,12-tetraoxaicosan-15-acetic acid (**LY-81**) (200 mg, 0.75 mmol, 1.0 eq) in anhydrous MeOH (15 mL) and HOAc (1.5 mL), and after addition, the reaction solution allowed to react at 40°C-45°C for 24 hours in a nitrogen atmosphere. The reaction solution was concentrated under reduced pressure, approximately 8 mL of water and 2 mL of methanol were added to the residue, and the residue was purified by preparative high-performance liquid chromatography (Orienda, BRIX-2860; column: Phenomenex Luna C18 250 × 50 mm × 10 µm; mobile phase: water (0.225% HCOOH)-acetonitrile, with an elution ratio of acetonitrile increased from 45% to 85%) and lyophilized, to obtain the compound LY-82 (384 mg, 72% yield, TFA salt) (colorless/off-white gel-like substance).

LCMS(ESI): m/z, 594.5[M+1]⁺.

### Preparation Example 44: Preparation of compound LY-27a

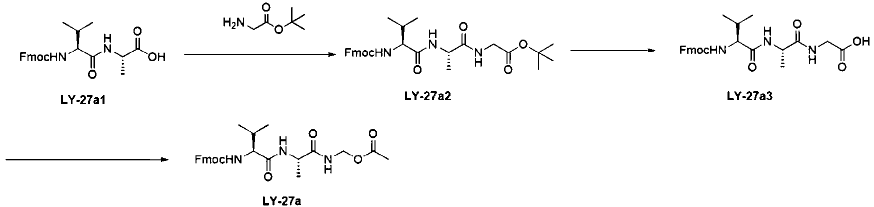

### Step 1: Preparation of compound LY-27a2

2-ethoxy-1-ethoxycarbonyl-1,2-dihydroquinoline (7.68g, 31.06 mmol, 1.5 eq) was added to a solution of (((9H-fluoren-9-yl)methoxy)carbonyl)-L-valine-L-alanine (ChemExpress, LY-27a1) (8.5g, 20.71 mmol, 1 eq) and tert-butyl glycinate (3.26g, 24.85 mmol, 1.2 eq) in methanol/dichloro methane (50 mL/100 mL) in the ice bath, and the reaction solution was stirred for 10 minutes and then stirred at 40°C overnight. After concentration, the residue was triturated with petroleum ether/ethyl acetate (100 mL/50 mL), and the resulting solution was stirred for 20 minutes and then filtered to obtain a white solid (10.6g, 97.76% yield).

LCMS(ESI): m/z, 546[M+Na]⁺.

### Step 2: Preparation of compound LY-27a3

Trifluoroacetic acid (20 mL) was added to the solution of compound **LY-27a2** (10.6g, 20.24 mmol, 1.0 eq) in dichloromethane (60 mL) in the ice bath, and the reaction solution was naturally warmed to room temperature and stirred overnight. The reaction solution was concentrated with dichloromethane (10 mL) 3 times to obtain a light yellow sticky crude product (15.24g).

LCMS(ESI): m/z, 468[M+H]⁺.

### Step 3: Preparation of compound LY-27a

Lead tetraacetate (23.3g, 52.52 mmol, 2.6 eq), acetic acid (4.6 mL, 80.8 mmol, 4 eq), and copper acetate (1.46g, 8.08 mmol, 0.4 eq) were added to a solution of compound **LY-27a3** (15.24g, 20.2 mmol, 1.0 eq, purity: 62.1%) in N,N-dimethylformamide (100 mL) under a nitrogen atmosphere, and the reaction solution was stirred at 60°C for 1 hour and warmed to room temperature. The reaction solution was concentrated under reduced pressure, water was added to the residue, a large amount of insoluble substances were generated and dissolved in water (100 mL) and acetonitrile (100 mL), the resulting solution was extracted with ethyl acetate (100 mL), washed with brine (50 mL × 2), dried with anhydrous sodium sulfate, filtered, and concentrated to obtain compound **LY-27a** (8g, 82.2% yield, 80% purity).

LCMS(ESI): m/z, 504[M+Na]⁺.

¹H NMR (400 MHz, Methanol-d4) δ 7.81 (d, J = 7.6 Hz, 2H), 7.69 (t, J = 7.0 Hz, 2H), 7.40 (t, J = 7.6 Hz, 2H), 7.32 (t, J = 7.4 Hz, 2H), 5.20 (d, J = 6.2 Hz, 2H), 4.48-4.30 (m, 3H), 4.24 (t, J = 6.8 Hz, 1H), 3.94 (d, J = 6.8 Hz, 1H), 2.01 (s, 3H), 1.36 (d, J = 7.2 Hz, 3H), 0.97 (dd, J = 9.6, 6.6 Hz, 6H).

### Preparation Example 45: Preparation of Compound LY-22CC-c

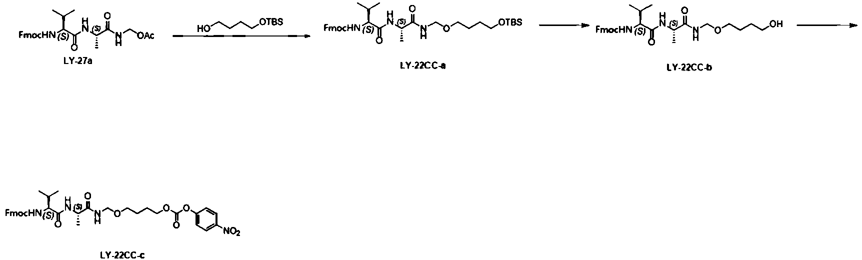

### Step 1: Preparation of compound LY-22CC-a

The compound LY-27a (500 mg, 1.04 mmol, 1 eq) was dissolved in THF (10 mL), then compound 4-((tert-butyldimethylsilyl)oxy)but-1-ol (424.42 mg, 2.08 mmol, 2 eq) and TsOH (p-toluenesulfonic acid, 16.46 mg, 95.59 µmol, 1 eq) were added, and the reaction solution was stirred at 30°C for 4 hours. The reaction solution was directly used for the next step.

LCMS(ESI): m/z, 648.4 [M+Na]⁺.

### Step 2: Preparation of compound LY-22CC-b

The compound LY-22CC-a (500 mg, 798.88 µmol, 1 eq) was dissolved in THF (10 mL), and 4M hydrogen chloride 1,4 dioxane solution (29.13 mg, 199.72 µL, 798.88 µmol, 1 eq) was added to the solution, and the solution was stirred at 15°C for 3 hours. The reaction solution was concentrated under reduced pressure, and the residue was purified by high-performance liquid chromatography (preparative chromatograph manufacturer: Shimadzu, model: LC-20AP, column: XT-15×30-10, mobile phase: water (0.225% HCOOH)-acetonitrile, with an elution ratio of acetonitrile increased from 40% to 70%), to afford the compound LY-22CC-b (70 mg, 17.13% yield) as a white solid.

LCMS(ESI): m/z, 534.3 [M+Na]⁺.

### Step 3: Preparation of compound LY-22CC-c

The compound LY-22CC-b (70 mg, 136.82 µmol, 1 eq) was dissolved in a solution of DMF (5 mL), and bis(4-nitrophenyl)carbonate (83.24 mg, 55.50 µL, 273.64 µmol, 2 eq) and DIPEA (53.05 mg, 67.84 µL, 410.46 µmol, 3 eq) were added. The reaction solution was stirred at 15°C for 12 hours. The reaction solution was concentrated to remove most of DMF, DCM (30 mL) was added, the reaction solution was washed with water (30 mL), the organic phase was dried, filtered, and concentrated under reduced pressure, and the residue was purified by silica gel column chromatography (eluents: DCM: MeOH = 0:1-5:1) to obtain the yellow solid product LY-22CC-c (70 mg, 75.60% yield).

LCMS(ESI): m/z, 699.3 [M+Na]⁺.

### Preparation Example 46: Preparation of Compound LY-29C-c

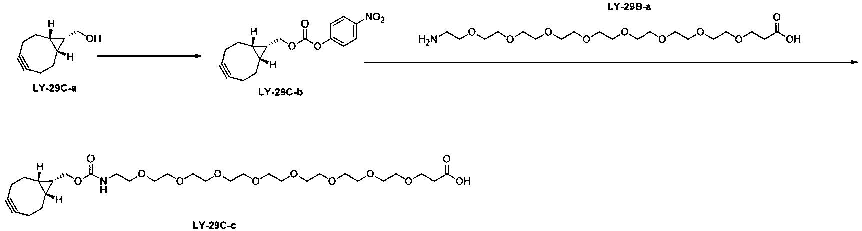

### Step 1: Preparation of ((1R,8S,9s)-bicyclo[6.1.0]non-4-yn-9-yl)methyl (4-nitrophenyl) carbonate (LY-29C-b)

Pyridine (105.31 mg, 107.68 µL, 1.33 mmol, 2.5 eq) and 4-nitrophenyl chloroformate (134.18 mg, 665.69 µmol, 1.25 eq) were added to the solution of compound (1R, 8S, 9S)-bicyclo[6.1.0]non-4-yn-9-ylmethanol (**LY-29C-a**) (80 mg, 532.55 µmol, 1 eq) (Shandong Xingzhi Biopharmaceutical) in anhydrous DCM (5 mL) at 0°C in the nitrogen atmosphere, and after addition, the reaction solution allowed to react at room temperature for half an hour. The reaction solution was quenched with saturated ammonium chloride (10 mL) at 0°C and extracted with DCM (40 mL × 3) three times. The organic phases were combined, washed with brine, dried with anhydrous sodium sulfate, and filtered, the filtrate was concentrated under reduced pressure, and the residue was purified by silica gel column chromatography (eluents: ethyl acetate/n-hexane = 1:20-1:9) to obtain anhydrous oily compound **LY-29C-b** (75 mg, 44.66% yield).

### Step 2: Preparation of compound LY-29C-c

**LY-29C-b** (112 mg, 355.18 µmol, 1 eq) and DIPEA (114.77 mg, 146.76 µL, 887.96 µmol, 2.5 eq) were added to the solution of compound **LY-29B-a** (156.82 mg, 355.18 µmol, 1 eq) in anhydrous DMF (5 mL) at 0°C in the nitrogen atmosphere. After addition, the reaction solution allowed to react at room temperature for 16 hours, and concentrated under reduced pressure at no more than 40°C, and the residue was purified by preparative high-performance liquid chromatography (preparative chromatograph manufacturer: ISCO, model: ISCO-R1, column: Synergi Max-RP 250 × 40 mm × 10 µm, mobile phase: water (0.225% HCOOH)-acetonitrile, with an elution ratio of acetonitrile increased from 21% to 51%), to obtain anhydrous oily compound LY-29C-c (148 mg, 67.45% yield).

### Examples

### Example 1: Preparation of Compound BA-1

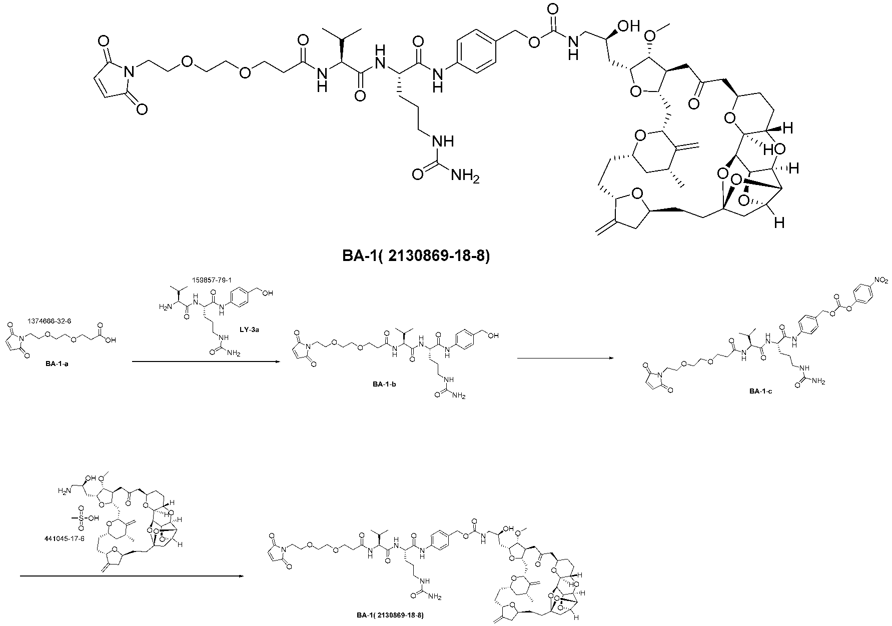

### Step 1: Preparation of compound BA-1-b

DMTMM (4-(4,6-dimethoxy-1,3,5-triazin-2-yl)-4-methylmorpholinium chloride, 87.51 mg, 316.25 µmol, 2.4 eq) was added to a mixture of compound BA-1-a (purchased from Bide Pharmatech Co., Ltd., 40.68 mg, 158.12 µmol, 1.2 eq) and compound LY-3a (purchased from Bide Pharmatech Co., Ltd., 50 mg, 131.77 µmol, 1 eq) in THF (1 mL) and MeOH (1 mL) at 0°C. After the addition, the mixture was allowed to react at 25°C for 2 hours. The LCMS analysis showed that the reaction ended. The reaction solution was evaporated to dryness to afford a crude product, and the crude product was purified by trituration (3 mL of acetonitrile) to afford the compound BA-1-b (75 mg, 92% yield) as a white solid.

LCMS (ESI): m/z, 619.4[M+H]⁺. Rt = 1.530 min.

### Step 2: Preparation of compound BA-1-c

DIPEA (47 mg, 60.10 µL, 363.67 µmol, 3 eq) and NPC (184.39 mg, 122.93 µL, 606.12 µmol, 5 eq) was added to a solution of compound BA-1-b (75 mg, 121.22 µmol, 1 eq) in DMF (5 mL) at 0°C. After addition, the reaction solution was allowed to react at 33°C for 2 hours. The LCMS analysis showed that the reaction ended. The reaction solution was concentrated to afford the crude product. The crude product was purified by silica gel column chromatography (eluents: methanol/dichloromethane = 1:20 to 1:10) to afford the compound BA-1-c (83 mg, 87.36% yield) as a white solid.

LCMS (ESI): m/z, 784.5[M+H]⁺. Rt = 1.778 min.

### Step 3: Preparation of compound BA-1

DIPEA (4.69 mg, 6 µL, 36.32 µmol, 3 eq) was added to a solution of compound BA-1-c (12.34 mg, 15.74 µmol, 1.3 eq) and eribulin mesylate (10 mg, 12.11 µmol, 1 eq) in DMF (3 mL) at 0°C. After addition, the reaction solution allowed to react at 25°C for 16 hours, and the LCMS analysis showed that the reaction ended. The reaction solution was purified by preparative high-performance liquid chromatography (preparative chromatograph manufacturer: Orienda, model: BRIX-2860, column: Synergi Max-RP 200 × 30mm × 10 µm, mobile phase: water (0.225% HCOOH)-acetonitrile, with an elution ratio of water increased from 30% to 60%) and lyophilized, to afford compound BA-1 (2130869-18-8, 10.38 mg, 62.37% yield, 98.24% purity) as a white solid.

LCMS (ESI): m/z, 1374.9[M+H]⁺, 1397.9[M+Na]⁺. Rt = 2.213 min.

### Example 2: Preparation of Compound BA-2

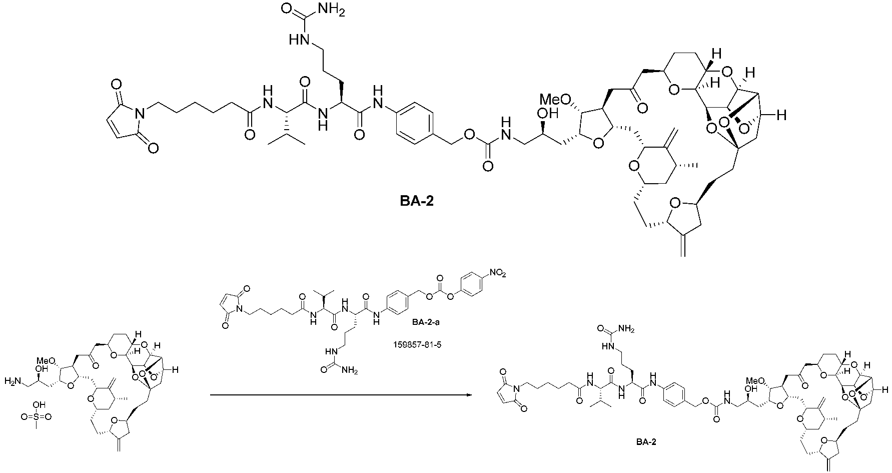

DIPEA (1.41 mg, 1.80 µL, 10.90 µmol, 3 eq) was added to a solution of eribulin mesylate (3 mg, 3.63 µmol, 1 eq) and BA-2-a (4.02 mg, 5.45 µmol, 1.5 eq) in DMF (1 mL) at 0°C After addition, the reaction solution allowed to react at 25°C for 16 hours, and the LCMS analysis showed that the reaction ended. The reaction solution was purified by preparative high-performance liquid chromatography (preparative chromatograph manufacturer: Shimadzu, model: LC-20AP, column: Welch Xtimate C18 150 × 21.2 mm × 5 µm, mobile phase: water (0.225% FA)-acetonitrile, with an elution ratio of acetonitrile increased from 40% to 70%) and lyophilized, to afford compound LA-B2 (2.39 mg, 49.53% yield, 99% purity) as a white solid.

LCMS (ESI): m/z, 1351.6[M+Na]⁺. Rt = 2.222 min.

### Example 3: Preparation of Compound BA-3

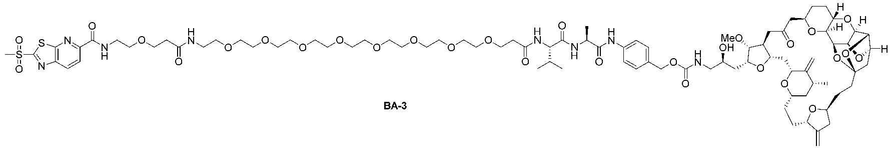

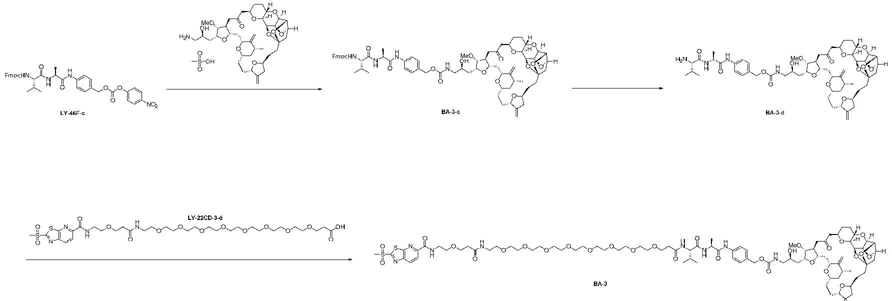

### Step 1: Preparation of compound BA-3-c

DIPEA (4.69 mg, 6 µL, 36.32 µmol, 3 eq) and HOBT (1.64 mg, 12.11 µmol, 1 eq) were added to the solution of compound LY-46F-c (10.42 mg, 87%, 13.32 µmol, 1.1 eq) and eribulin mesylate (10 mg, 12.11 µmol, 1 eq) in DMF (1 mL), and after a resulting mixture allowed to react for 2 hours at room temperature, the LCMS analysis indicated complete consumption of the starting material, and the reaction solution was purified by preparative high-performance liquid chromatography (preparative chromatograph manufacturer: ISCO, model: ISCO, column: Welch Xtimate C18 250 × 30mm × 10 µm, mobile phase: water (0.225% HCOOH)-acetonitrile, with an elution ratio of acetonitrile increased from 60% to 90%), to afford the product BA-3-c (5.50 mg, 35.73% yield) as a white solid. LCMS (ESI): m/z, 1293[M+Na]⁺.

### Step 2: Preparation of compound BA-3-d

DBU (1.98 mg, 1.94 µL, 12.98 µmol, 3 eq) was added to a solution of compound BA-3-c (5.50 mg, 4.33 µmol, 1 eq) in DMF (1 mL). After the mixture was allowed to react at room temperature for 40 minutes, the LCMS analysis indicated complete consumption of the starting material. The reaction was quenched by addition of TsOH (2.23 mg, 12.98 µmol, 3 eq) and the mixture was then used directly in the next step. LCMS (ESI): m/z, 1071[M+Na]⁺.

### Step 3: Preparation of compound BA-3

DIPEA (1.12 mg, 1.43 µL, 8.65 µmol, 2 eq) and HATU (1.97 mg, 5.19 µmol, 1.2 eq) were added to a solution of compound BA-3-d (3.45 mg, 4.33 µmol, 1 eq) and LY-22CD-3-d (3.45 mg, 4.33 µmol, 1 eq) in DMF (1 mL) at 0°C. After the mixture was allowed to react at room temperature for 1 hour, the LCMS analysis indicated complete consumption of the starting material. The reaction solution was purified by preparative high-performance liquid chromatography (preparative chromatograph manufacturer: ISCO, model: ISCO, column: Welch Xtimate C18 250 × 30 mm × 10 µm, mobile phase: water (0.225% HCOOH)-acetonitrile, with an elution ratio of acetonitrile increased from 30% to 60%), to afford product BA-3 (3.45 mg, 43.62% yield, 97% purity) as a white solid. LCMS (ESI): m/z, 1849[M+Na]⁺.

### Example 4: Preparation of Compound BA-4

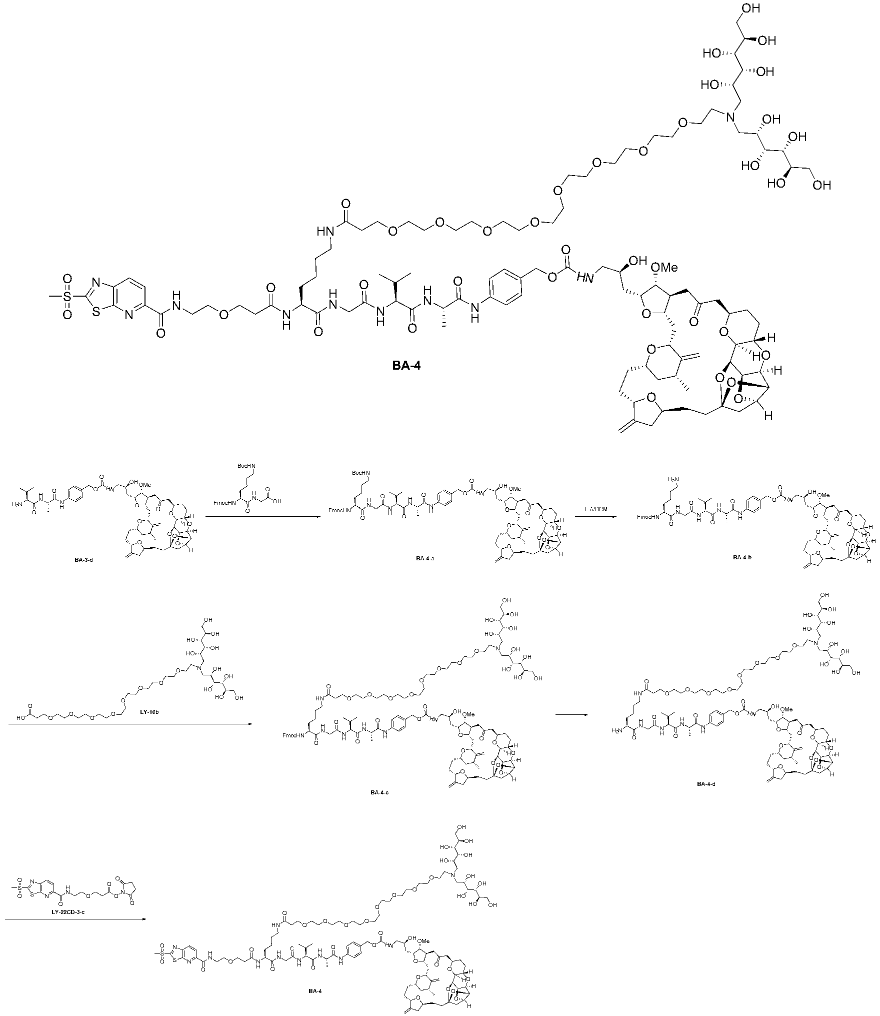

### Step 1: Preparation of compound BA-4-a

DIPEA (12.09 µL, 72.43 µmol, 2 eq) and HATU (16.52 mg, 43.46 µmol, 1.2 eq) were added to a solution of compound BA-3-d (38 mg, 36.22 µmol, 1 eq; for synthesis of the compound, refer to the Preparation Example of BA-3) and 2-[(2S)-6-[(tert-butoxycarbonyl)amino]-2-([(9H-fluoren-9-ylmethoxy)carbonyl]amino)hexanamido]acetic acid (21.15 mg, 39.84 µmol, 1.1 eq; for synthesis of the compound, refer to patent application WO2024175069A1) in DMF (1.5 mL) at 0°C. The mixture was stirred at 25°C for 1 hour, and the LCMS analysis indicated complete consumption of the starting material. The residue was purified by preparative high-performance liquid chromatography (preparative chromatograph manufacturer: Shimadzu, model: LC-20AP, column: Synergi Max-RP 250 × 40 mm × 10 µm, mobile phase: water (0.225% HCOOH)-acetonitrile, with an elution ratio of acetonitrile increased from 50% to 80%), to afford BA-4-a (29.60 mg, 52.50% yield) as a white solid.

LCMS (ESI): m/z, 1578 [M+Na]⁺, Rt=3.068 min.

### Step 2: Preparation of compound BA-4-b

TFA (0.5 mL) was added to a solution of compound BA-4-a (29.6 mg, 18.8 µmol, 1 eq) in DCM (3 mL) at 0°C. The mixture was stirred for 30 minutes. The LCMS analysis indicated complete consumption of the starting material. The mixture was concentrated and used directly in the next step. A theoretical mass of BA-4-b was 27 mg.

LCMS (ESI): m/z, 1478 [M+Na]⁺, Rt=2.443 min.

### Step 3: Preparation of compound BA-4-c

DIPEA (12.25 µL, 74.14 µmol, 6 eq) and HATU (5.64 mg, 14.83 µmol, 1.2 eq) were added to a solution of compound BA-4-b (18 mg, 12.36 µmol, 1 eq) and LY-10b (11.41 mg, 14.83 µmol, 1.2 eq; for synthesis of the compound, refer to Preparation Example of LA-27C-2) in DMF (1 mL) at 0°C. The mixture was stirred at 25°C for 1 hour. The LCMS analysis indicated complete consumption of the starting material. The reaction solutions from two batches of the same scale were combined and purified by preparative high-performance liquid chromatography (preparative chromatograph manufacturer: Orienda, model: BRIX-2860, column: YMC-Triart Prep C18 150 × 30 mm × 10 µm, mobile phase: water (0.225% HCOOH)-acetonitrile, with an elution ratio of acetonitrile increased from 30% to 60 %), to afford BA-4-c (35.60 mg, 66% yield) as a white solid.

LCMS (ESI): m/z, 1126[1/2M+Na]⁺, Rt=2.262 min.

### Step 4: Preparation of compound BA-4-d

Et₂NH (6 mg, 80.60 µmol, 5 eq) was added to the solution of the compound BA-4-c (35.60 mg, 16.12 µmol, 1 eq) in DMF (1.5 mL) at 0°C, the reaction solution was stirred at 25 C for 1 hour, and the LCMS analysis indicated complete consumption of the starting material. The mixture was concentrated by an oil pump and used directly in the next step. A theoretical mass of BA-4-d was 32 mg.

LCMS (ESI): m/z, 993 [1/2M+H]⁺, Rt=1.739 min.

### Step 5: Preparation of compound BA-4

DIPEA (5.38 µL, 32.22 µmol, 2 eq) was added to the solution of compounds BA-4-d (32 mg, 16.11 µmol, 1 eq) and LY-22CD-3-c (for synthesis of the compound, refer to Preparation Example 41) (8.34 mg, 17.72 µmol, 1.1 eq) in DMF (1.5 mL) at 0°C. After addition, the reaction solution was allowed to react at 25°C for 1 hour. LCMS analysis showed that the reaction ended. The reaction solution was purified by preparative high-performance liquid chromatography (preparative chromatograph manufacturer: Orienda, model: BRIX-2860, column: YMC-Triart Prep C18 150 × 30 mm × 10 µm, mobile phase: water (0.225% HCOOH)-acetonitrile, with an elution ratio of acetonitrile increased from 30% to 60%) and lyophilized, to afford compound BA-4 (5.74 mg, 14.50% yield, 95.29% purity) as a white solid.

LCMS (ESI): m/z, 1193[1/2M+Na]⁺. Rt=1.871 min.

¹H NMR (400 MHz, DMSO-*d*₆) δ 9.90 (s, 1H), 9.09 (s, 1H), 8.86 (d, *J =* 8.6 Hz, 1H), 8.38 (d, *J* = 8.6 Hz, 1H), 8.22 (d, *J* = 6.8 Hz, 1H), 8.19 - 8.11 (m, 2H), 8.03 (d, *J* = 7.6 Hz, 1H), 7.81 - 7.72 (m, 2H), 7.57 (d, *J =* 8.4 Hz, 2H), 7.28 (d, *J =* 8.4 Hz, 2H), 7.09 (s, 1H), 6.20 (d, *J =* 3.0 Hz, 1H), 5.94 (d, *J =* 3.0 Hz, 1H), 5.42 (s, 2H), 4.94 (s, 2H), 4.87 (s, 1H), 4.81 (s, 1H), 4.75 (s, 1H), 4.54 (s, 5H), 4.42 - 4.26 (m, 5H), 4.19 (d, *J =* 7.6 Hz, 3H), 4.05 (s, 1H), 4.00 (s, 3H), 3.86 - 3.45 (m, 63H), 3.19 (s, 3H), 2.96 (d, *J =* 17.0 Hz, 5H), 2.75 (d, *J =* 12.8 Hz, 2H), 2.46 - 2.36 (m, 4H), 2.28 (t, *J* = 6.6 Hz, 2H), 2.13 (s, 2H), 1.95 (s, 4H), 1.84 (d, *J* = 11.8 Hz, 2H), 1.70 (d, *J* = 16.4 Hz, 5H), 1.62 (s, 4H), 1.45 (s, 5H), 1.30 (d, *J =* 7.2 Hz, 6H), 1.24 (s, 3H), 1.04 (d, *J =* 6.4 Hz, 3H), 0.86 (d, *J* = 6.8 Hz, 3H), 0.81 (d, *J =* 6.8 Hz, 3H).

### Example 5: Preparation of Compound BA-5

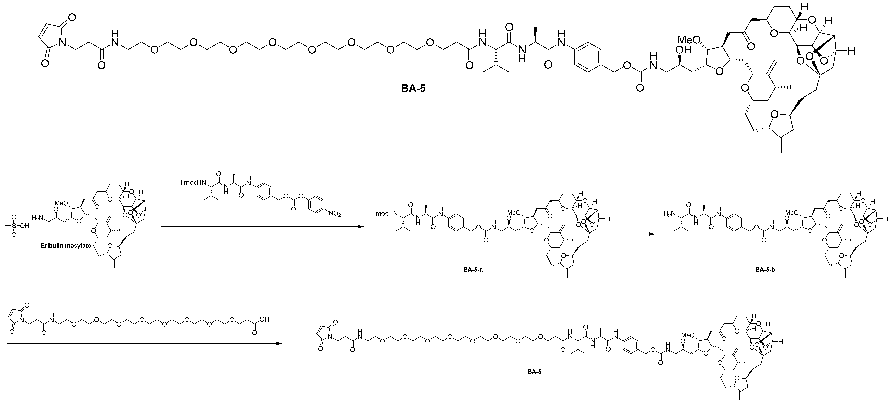

### Step 1: Preparation of compound BA-5-a

Fmoc-Val-Ala-PAB-PNP (9.89 mg, 0.015 mmol, 1.2 eq), DIPEA (0.006 mL, 0.036 mmol, 3 eq) and HOBt (1.64 mg, 0.012 mmol, 1 eq) were added to a solution of eribulin mesylate (10 mg, 0.012 mmol, 1 eq) in DMF (1 mL) at 20°C. The mixture was stirred at room temperature for 1 hour, and the LCMS analysis indicated complete consumption of the starting material. The reaction solution was purified by reverse-phase C18 column (H₂O (0.1% TFA): ACN = 17%: 83%) and lyophilized to afford BA-5-a (11 mg, 71.47% yield) as a white solid.

LCMS (ESI): m/z, 1293.2[(M+Na)]⁺.

### Step 2: Preparation of compound BA-5-b

Diethylamine (0.004 mL, 0.043 mmol, 5 eq) was added to the solution of compound BA-5-a (11 mg, 0.009 mmol, 1 eq) in DMF (1 mL) at 0°C, the reaction solution was stirred at 20°C for 1 hour, and the LCMS analysis indicated complete consumption of the starting material. The reaction solution was purified by silica gel column chromatography (C18, H₂O (0.1% TFA): ACN = 40%: 60%) to afford the product BA-5-b (7 mg, 77.09% yield) as a white solid. LCMS (ESI): m/z, 1048.7 [(M-1)]⁺.

### Step 3: Preparation of compound BA-5

DIEA (0.002 mL, 0.013 mmol, 2 eq), 1-maleimido-3-oxo-7,10,13,16,19,22,25,28-octaoxa-4-aza (4.74 mg, 0.008 mmol, 1.2 eq) and HATU (3.04 mg, 0.008 mmol, 1.2 eq) were added to a solution of compound BA-5-b (7 mg, 0.007 mmol, 1 eq) in DMF (1 mL) at 0°C. The mixture was stirred at 25°C for 1 hour, and the LCMS analysis indicated complete consumption of the starting material. The reaction solution was purified by silica gel column chromatography (C18, H₂O (0.1% TFA): MeCN = 34%: 66%), to afford the product BA-5 (5.2 mg, 48.01% yield) as a white solid.

LCMS (ESI): m/z, 1645.4[(M+Na)]⁺.

¹H NMR (400 MHz, DMSO-*d6*) δ 9.92 (s, 1H), 8.18 (d, *J* = 6.8 Hz, 1H), 8.01 (s, 1H), 7.88 (d, *J =* 8.8 Hz, 1H), 7.58 (d, *J =* 8.4 Hz, 2H), 7.28 (d, *J* = 8.8 Hz, 2H), 7.09 (s, 1H), 7.00 (s, 2H), 5.03 (d, *J* = 22.8 Hz, 2H), 4.94 (s, 2H), 4.84 (s, 1H), 4.76 (s, 1H), 4.64 (s, 1H), 4.56 (t, *J =* 4.0 Hz, 1H), 4.44 - 4.36 (m, 1H), 4.27 (d, *J* = 10.8 Hz, 1H), 4.24 - 4.15 (m, 2H), 4.11 (s, 2H), 4.03 (s, 1H), 3.84 - 3.67 (m, 4H), 3.59 (dd, *J =* 12.0, 6.8 Hz, 5H), 3.49 (dd, *J =* 6.8, 4.4 Hz, 36H), 3.25 (s, 3H), 3.15 (dd, *J =* 11.6, 6.0 Hz, 3H), 2.97 (s, 2H), 2.84 (d, *J =* 10.4 Hz, 1H), 2.81 - 2.64 (m, 3H), 2.36 - 2.17 (m, 8H), 2.05 - 1.84 (m, 7H), 1.69 (dd, *J =* 25.2, 12.0 Hz, 5H), 1.51 (s, 3H), 1.31 (d, *J* = 7.2 Hz, 5H), 1.12 - 0.93 (m, 5H), 0.86 (dd, *J =* 16.0, 6.8 Hz, 7H).

### Example 6: Preparation of Compound BA-6

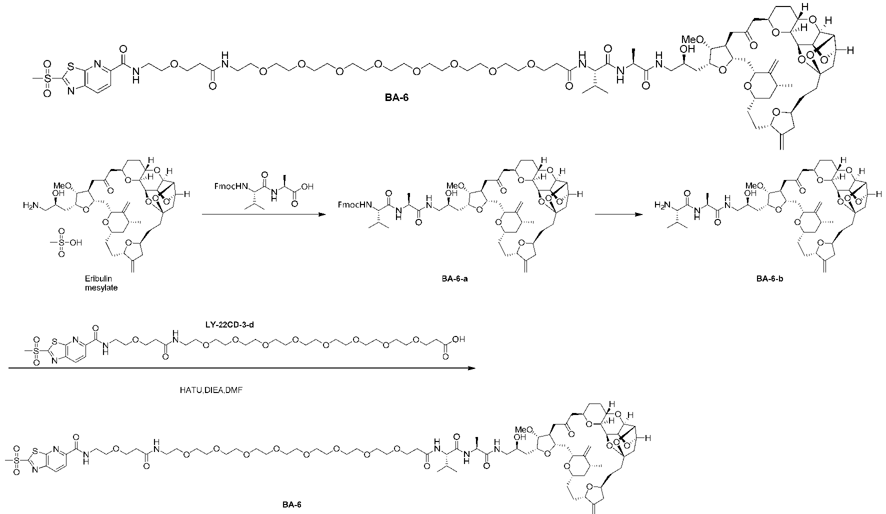

### Step 1: Preparation of compound BA-6-a

Fmoc-L-valyl-L-alanine (8.94 mg, 0.022 mmol, 1.2 eq), DIPEA (0.009 mL, 0.054 mmol, 3 eq) and HATU (8.29 mg, 0.022 mmol, 1.2 eq) were added to a solution of eribulin mesylate (15 mg, 0.018 mmol, 1 eq) in DMF (2 mL) at 0°C. The mixture was stirred at room temperature for 1 hour, and the LCMS analysis indicated complete consumption of the starting material. The reaction solution was purified by preparative silica gel column chromatography (C18, H₂O (0.1% TFA): ACN = 13%: 87%) to afford the product BA-6-a (16 mg, 78.51% yield) as a white solid.

LCMS (ESI): m/z, 1121.7[(M-1)]⁺.

### Step 2: Preparation of compound BA-6-b

Diethylamine (5 mg, 0.07 mmol, 5 eq) was added to the solution of compound BA-6-a (16 mg, 0.014 mmol, 1 eq) in DMF (1 mL) at 0°C, the reaction solution was stirred at 20°C for 1 hour, and the LCMS analysis indicated complete consumption of the starting material. The reaction solution was concentrated under reduced pressure to afford the crude product (12.83 mg, crude) to be used directly in the next step without further purification.

LCMS (ESI): m/z, 900.2 [(M)]⁺.

### Step 3: Preparation of compound BA-6

DIEA (0.007 mL, 0.043 mmol, 3 eq), LY-22CD-3-d (for synthesis of the compound, refer to Preparation Example 41) (12.49 mg, 0.016 mmol, 1.1 eq) and HATU (7.05 mg, 0.019 mmol, 1.3 eq) were added to the solution of the compound BA-6-b (12.83 mg, 0.014 mmol, 1 eq) in DMF (2 mL) at 0°C, the reaction solution was stirred for 1 hour at 25°C, and the LCMS analysis indicated complete consumption of the starting material. The reaction solution was purified by preparative high-performance liquid chromatography (model: JB-C235-04, column: Bonnasil-BS C18, 20 × 250 mm × 8 µm; mobile phase: water (0.1% TFA)-acetonitrile, with an elution ratio of acetonitrile increased from 30% to 60%), to afford the product BA-6 (7.44 mg, 31.09% yield) as a white solid.

LCMS (ESI): m/z, 1678.4[(M+1)]⁺.

¹H NMR (400 MHz, DMSO-*d₆*) δ 9.06 (t, *J =* 5.6 Hz, 1H), 8.87 (d, *J* = 8.4 Hz, 1H), 8.39 (d, *J =* 8.4 Hz, 1H), 7.96 (d, *J =* 7.3 Hz, 1H), 7.92 - 7.83 (m, 2H), 7.71 (t, *J =* 5.5 Hz, 1H), 5.03 (d, *J* = 21.5 Hz, 2H), 4.84 (s, 1H), 4.76 (s, 1H), 4.64 (s, 1H), 4.56 (t, *J =* 4.1 Hz, 1H), 4.30 - 4.24 (m, 2H), 4.20 - 4.14 (m, 2H), 4.11 (s, 3H), 4.02 (s, 1H), 3.84 - 3.71 (m, 3H), 3.65 (d, *J =* 3.5 Hz, 4H), 3.63 (s, 1H), 3.62 - 3.58 (m, 3H), 3.56 (dd, *J* = 10.2, 4.2 Hz, 5H), 3.51 - 3.48 (m, 26H), 3.25 (s, 4H), 3.19 (dd, *J =* 11.6, 5.8 Hz, 3H), 3.15 - 3.07 (m, 2H), 3.00 (d, *J =* 13.4 Hz, 2H), 2.88 - 2.66 (m, 5H), 2.37 - 2.18 (m, 10H), 2.02 - 1.89 (m, 7H), 1.76 - 1.45 (m, 9H), 1.24 (dd, *J =* 34.3, 17.4 Hz, 8H), 1.01 (dd, *J* = 23.2, 9.3 Hz, 4H), 0.83 (dd, *J* = 11.2, 6.8 Hz, 7H).

### Example 7: Preparation of Compound LA-1

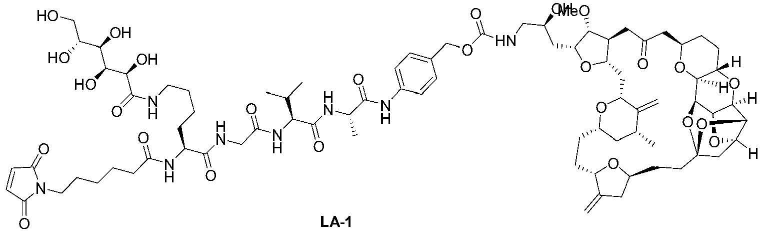

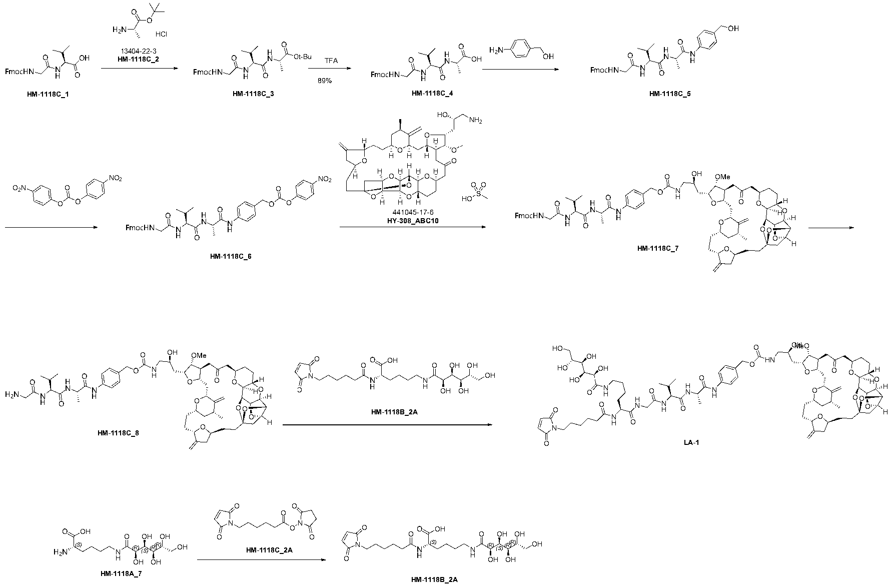

### Step 1: Preparation of compound HM-1118C_3

HM-1118C_1 (8g, 20.2 mmol) and HM-1118C_2 (3.66g, 20.2 mmol) were added to a 250 mL three-necked flask, and DMF (80 mL) was added. Under the nitrogen atmosphere, DIPEA (7.8g, 60.6 mmol) was added dropwise. After stirring for 5 minutes, a solution of HATU (9.2g, 24.2 mmol) in DMF (30 mL) was added dropwise. After addition, the mixture was stirred at room temperature for 1 hour, and the LCMS analysis indicated the reaction ended. The reaction solution was slowly added into water (800 mL), a resulting solution was stirred for 30 minutes and filtered, the solid was dissolved with DCM (200 mL), the layers were separated, the resulting solution was washed with water once, and the organic phase was dried over anhydrous sodium sulfate, and evaporated to dryness to afford 9.5g of yellow oily substance with yield of 90%.

LCMS(ESI):m/z, 524.2[M+H]⁺, 546.2[M+Na]⁺.

¹H NMR (400 MHz, DMSO) δ 8.32 (d, *J =* 6.7 Hz, 1H), 7.88 (d, *J* = 7.5 Hz, 2H), 7.70 (d, *J* = 7.3 Hz, 3H), 7.51 (t, *J* = 6.0 Hz, 1H), 7.41 (t, *J =* 7.4 Hz, 2H), 7.32 (t, *J* = 7.4 Hz, 2H), 4.19 (m , 5H), 3.66 (d, *J =* 5.1 Hz, 2H), 1.95 (m, 1H), 1.37 (s, 9H), 1.23 (d, *J =* 7.2 Hz, 3H), 0.85 (dd, *J =* 18.7, 6.8 Hz, 6H).

### Step 2: Preparation of compound HM-1118C_4

HM-1118C_3 (6g, 11.47 mmol) was dissolved in DCM (30 mL). Under the nitrogen atmosphere, TFA (20 mL) was added dropwise. After the addition, the mixture was allowed to react at room temperature for 2 hours, and the LCMS analysis indicated the reaction ended. The reaction solution was concentrated until the volume was small, saturated sodium bicarbonate solution was added to adjust pH to 7-8, DCM (100 mL) was added to extract impurities, pH was adjusted to 1-2 with 1N HCl, the product was extracted with EA (200 mL × 2), the organic phase was washed with water (100 mL × 3), the organic phase was dried over anhydrous sodium sulfate and evaporated to dryness to afford 4.8g of white solid with yield of 89%.

LCMS(ESI):m/z, 468.2[M+H]⁺, 490.2[M+Na]⁺.

¹H NMR (400 MHz, DMSO) δ 7.88 (d, *J* = 7.5 Hz, 2H), 7.70 (d, *J =* 7.4 Hz, 2H), 7.41 (t, *J* = 7.4 Hz, 2H), 7.32 (t, *J =* 7.3 Hz, 2H), 4.21 (m, 5H), 3.65 (s, 2H), 1.92 (m, 1H), 1.26 (d, *J* = 7.3 Hz, 3H), 0.84 (dd, *J =* 20.8, 6.7 Hz, 6H).

### Step 3: Preparation of compound HM-1118C_5

HM-1118C_4 (2.5g, 5.35 mmol) and (4-aminophenyl)methanol (645 mg, 5.24 mmol) were added to a reaction flask, and DMF (25 mL) was added for dissolution. Under the nitrogen atmosphere, 2,6-lutidine (1.72g, 16 mmol) was added, and the mixture was then stirred for 5 minutes. A solution of HATU (2.44g, 6.4 mmol) in DMF (8 mL) was added dropwise. After the addition, the mixture was allowed to react at room temperature for 1 hour. The LCMS analysis indicated the reaction ended. The reaction solution was added to water (200 mL), the product was extracted with EA (200 mL × 2), the organic phase was washed with dilute hydrochloric acid and sodium bicarbonate, dried over anhydrous sodium sulfate, and evaporated to dryness to obtain 2.4g of an off-white solid with yield of 80%.

LCMS(ESI):m/z, 595.2[M+Na]⁺.

¹H NMR (400 MHz, DMSO) δ 7.90 (d, *J =* 7.5 Hz, 2H), 7.70 (d, *J =* 7.5 Hz, 2H), 7.55 (d, *J* = 8.3 Hz, 2H), 7.43 (t, *J =* 7.4 Hz, 2H), 7.33 (t, *J =* 7.4 Hz, 2H), 7.24 (d, *J =* 8.3 Hz, 2H), 4.48-4.35 (m, 3H), 4.25 (dd, *J =* 21.4, 6.6 Hz, 4H), 3.70 (d, *J =* 2.4 Hz, 2H), 2.00-1.95 (m, 1H), 1.33 (d, *J* = 7.1 Hz, 3H), 0.87 (dd, *J =* 19.4, 6.8 Hz, 6H).

### Step 4: Preparation of compound HM-1118C_6

HM-1118C_5 (1g, 1.74 mmol) and HM-1118C_1A (0.8g, 2.62 mmol) were dissolved in DMF (10 mL). Under the nitrogen atmosphere, DIPEA (563 mg, 4.37 mmol) was added dropwise. After the addition, the mixture was allowed to react at room temperature for 2 hours, and the LCMS analysis indicated the reaction ended. The reaction solution was added to water (50 mL), and the product was extracted with EA (100 mL × 2). The EA layers were dried over anhydrous sodium sulfate and concentrated. The residue was mixed with silica gel and subjected to column chromatography by using a PE:EA system. The product was eluted with pure EA, and the solvent was evaporated to dryness to afford 1g of an off-white solid with yield of 78%.

LCMS(ESI):m/z, 760.2[M+Na]⁺.

¹H NMR (400 MHz, DMSO) δ 9.96 (s, 1H), 8.35 - 8.23 (m, 3H), 7.87 (m, 3H), 7.62 (m, 7H), 7.41 (t, *J* = 6.8 Hz, 4H), 7.32 (t, *J* = 7.0 Hz, 2H), 5.23 (s, 2H), 4.45-4.33 (m, 1H), 4.22 (m, 4H), 3.70 (s, 2H), 1.99 (m, 1H), 1.33 (m, 3H), 0.86 (dd, *J =* 19.9, 6.8 Hz, 6H).

### Step 5: Preparation of compound HM-1118C_7

HM-1118C_6 (100 mg, 0.135 mmol) and HY-308_ABC10 (110 mg, 0.133 mmol) were added to a reaction flask. Under the nitrogen atmosphere, DMF (2 mL) was added for dissolution. HOBT (18.3 mg, 0.135 mmol) and DIPEA (70 mg, 0.542 mmol) were added. The mixture was allowed to react at room temperature for 1 hour, and LC-MS indicated the reaction ended. The reaction solution was added into ice water, and EA (100 mL) was added to extract the product. The organic phase was washed with water (50 mL × 3), dried over anhydrous sodium sulfate, and evaporated to dryness. The residue was triturated with PE/EA (10:1), and the mixture was then filtered to afford 150 mg of an off-white solid with yield of 83%.

LCMS(ESI):m/z, 1350.4[M+Na]⁺.

¹H NMR (400 MHz, DMSO) δ 7.87 (d, *J* = 7.7 Hz, 2H), 7.67 (d, 2H), 7.56 (d, *J =* 8.3 Hz, 2H), 7.40 (t, *J =* 7.4 Hz, 2H), 7.32 (t, *J =* 6.0 Hz, 2H), 7.25 (d, *J =* 8.5 Hz, 2H), 5.04 (s, 1H), 4.98 (s, 1H), 4.92 (d, 2H), 4.81 (s, 1H), 4.74 (s, 1H), 4.63 (s, 1H), 4.54 (t, 1H), 4.36 (d, *J =* 7.5 Hz, 1H), 4.24 (m, 3H), 4.20 (m, 3H), 4.09 (s, 3H), 4.01 (m, 1H), 3.77 (m, 2H), 3.68 (d, 2H), 3.23 (s, 4H), 2.95 (s, 2H), 2.83 (m, 4H), 2.30-2.09 (m, 6H), 2.01-1.88 (m, 7H), 1.72 (m, 6H), 1.52 (s, 3H), 1.33-1.21 (m, 9H), 1.01 (m, 4H), 0.84 (m, 7H).

### Step 6: Preparation of compound HM-1118C_8

HM-1118C_7 (170 mg, 0.128 mmol) was dissolved in DCM (3 mL). Piperidine (0.3 mL) was added dropwise. After the addition, the mixture was allowed to react at room temperature for 1 hour, and the LCMS analysis indicated the reaction ended. PE (150 mL) was added to the reaction solution, and an oil precipitated and adhered to the flask wall. After settling for 10 minutes, the supernatant was decanted, and the residue was dried under an oil pump. The residue was dissolved in a solvent and the mixture was purified by preparative reversed-phase HPLC (preparative chromatograph manufacturer: Orienda, model: BRIX-2860, column: YMC-Triart Prep C18 150 × 30 mm × 10 µm, mobile phase: water (0.1% TFA)-acetonitrile, with an elution ratio of acetonitrile increased from 10% to 40%) (TFA system) and lyophilized, to afford 90 mg of a white solid with yield of 63%.

LCMS(ESI):m/z, 1106.5[M+H]⁺.

¹H NMR (400 MHz, DMSO) δ 10.00 (s, 1H), 8.40 (t, *J =* 7.3 Hz, 2H), 7.94 (t, 3H), 7.56 (d, *J* = 8.6 Hz, 2H), 7.28 (d, *J* = 8.6 Hz, 2H), 7.07 (t, *J* = 5.5 Hz, 1H), 5.05 (s, 1H), 5.00 (s, 1H), 4.98-4.90 (m, 2H), 4.83 (s, 1H), 4.75 (s, 1H), 4.64 (m, 1H), 4.55 (t, *J =* 4.1 Hz, 1H), 4.43-4.32 (m, 2H), 4.26 (d, *J =* 10.4 Hz, 1H), 4.22-3.97 (m, 6H), 3.85-3.73 (m, 3H), 3.67 (m, 1H), 3.62 (m, 2H), 3.27-3.19 (m, 4H), 2.98 (t, *J =* 5.6 Hz, 2H), 2.84 (d, *J =* 9.7 Hz, 1H), 2.73 (m, 2H), 2.60-2.53 (m, 1H), 2.33-2.11 (m, 6H), 2.03-1.88 (m, 7H), 1.68 (m, 5H), 1.51 (m, 3H), 1.26 (m, 7H), 1.01 (m, 4H), 0.91-0.83 (m, 6H).

### Step 7: Preparation compound HM-1118B_2A

HM-1118C_2A (300 mg, 0.97 mmol) and HM-1118A_7 (for the synthesis of the compound, refer to the example of LA-3) (789 mg, 2.43 mmol) were added to THF (5 mL), and water (5 mL) and NaHCO₃ (409 mg, 48.7 mmol) were then added. The mixture was allowed to react at room temperature for 1 hour, and the LCMS analysis indicated the reaction ended. The reaction solution was concentrated to a small volume, lyophilized, and purified by preparative reversed-phase HPLC (preparative chromatograph manufacturer: Orienda, model: BRIX-2860, column: YMC-Triart Prep C18 150 × 30 mm × 10 µm, mobile phase: water (0.1% TFA)-acetonitrile, with an elution ratio of acetonitrile increased from 20% to 40%) (TFA system), to afford 65 mg of a white solid with yield of 13%.

LCMS(ESI):m/z, 518.2[M+H]⁺.

¹H NMR (400 MHz, DMSO) δ 12.42 (s, 1H), 7.98 (d, *J =* 7.7 Hz, 1H), 7.63 (t, *J =* 5.9 Hz, 1H), 7.00 (s, 2H), 4.11 (m, 1H), 3.97 (d, *J =* 3.7 Hz, 1H), 3.90 (d, *J* = 1.9 Hz, 1H), 3.60-3.54 (m, 2H), 3.46 (m, 3H), 3.39-3.35 (m, 6H), 3.10-3.02 (m, 2H), 2.09 (t, *J =* 7.2 Hz, 2H), 1.53 (m, 8H), 1.31-1.13 (m, 4H).

### Step 8: Preparation of compound LA-1

HM-1118C_8 (72 mg, 0.065 mmol) and HM-1118B_2A (40 mg, 0.065 mmol) were dissolved in DMF (2 mL). DIPEA (25 mg, 0.19 mmol) was added, and a solution of HATU (25 mg, 0.065 mmol) in DMF (0.1 mL) was added dropwise. After addition, the mixture was stirred at room temperature for 30 minutes, and the LCMS analysis indicated the reaction ended. The reaction solution was directly purified by preparative reversed-phase HPLC (preparative chromatograph manufacturer: Orienda, model: BRIX-2860, column: YMC-Triart Prep C18 150 × 30 mm × 10 µm, mobile phase: water (0.1% TFA) - acetonitrile, with an elution ratio of acetonitrile increased from 30% to 60%. After preparative purification under the TFA system, the preparative solution was subjected to a neutral column (preparative chromatograph manufacturer: Oriento, model: BRIX-2860, column: YMC-Triart Prep C18 150 × 30 mm × 10 µm, mobile phase: water (10 mM NH₄HCO₃) - acetonitrile, with an elution ratio of acetonitrile increased from 30% to 60%. The product was subjected to preparative purification again to remove TFA, and lyophilized to afford 46 mg of a white solid with yield of 44%.

LCMS(ESI):m/z, 1605.7[M+H]⁺.

¹H NMR (400 MHz, DMSO) δ 9.89 (s, 1H), 8.24-8.11 (m, 2H), 7.92 (d, *J =* 7.5 Hz, 1H), 7.72 (d, *J =* 8.5 Hz, 1H), 7.63-7.54 (m, 3H), 7.27 (d, *J* = 8.4 Hz, 2H), 7.08 (t, 1H), 7.00 (s, 2H), 5.35 (d, *J* = 5.0 Hz, 1H), 5.05 (s, 1H), 5.00 (s, 1H), 4.93 (s, 2H), 4.83 (s, 1H), 4.75 (s, 1H), 4.63 (s, 1H), 4.55 (m, 3H), 4.47 (d, *J* = 5.2 Hz, 1H), 4.38 (m, 2H), 4.31 (t, 1H), 4.24 (d, 1H), 4.19 (m, 3H), 4.10 (s, 3H), 4.03-3.96 (m, 2H), 3.91 (m, 1H), 3.77 (m, 5H), 3.49 (m, 6H), 3.39 (s, 2H), 3.24 (s, 1H), 3.05 (m, 2H), 2.97 (m, 2H), 2.84 (d, *J* = 10.2 Hz, 1H), 2.74 (m, 2H), 2.31-2.07 (m, 8H), 1.95 (m, 7H), 1.68 (m, 6H), 1.54 -1.44 (m, 8H), 1.31 (m, 9H), 1.18 (m, 4H), 1.00 (m, 5H), 0.84 (dd, *J =* 21.0, 6.7 Hz, 7H).

### Example 8: Preparation of Compound LA-2

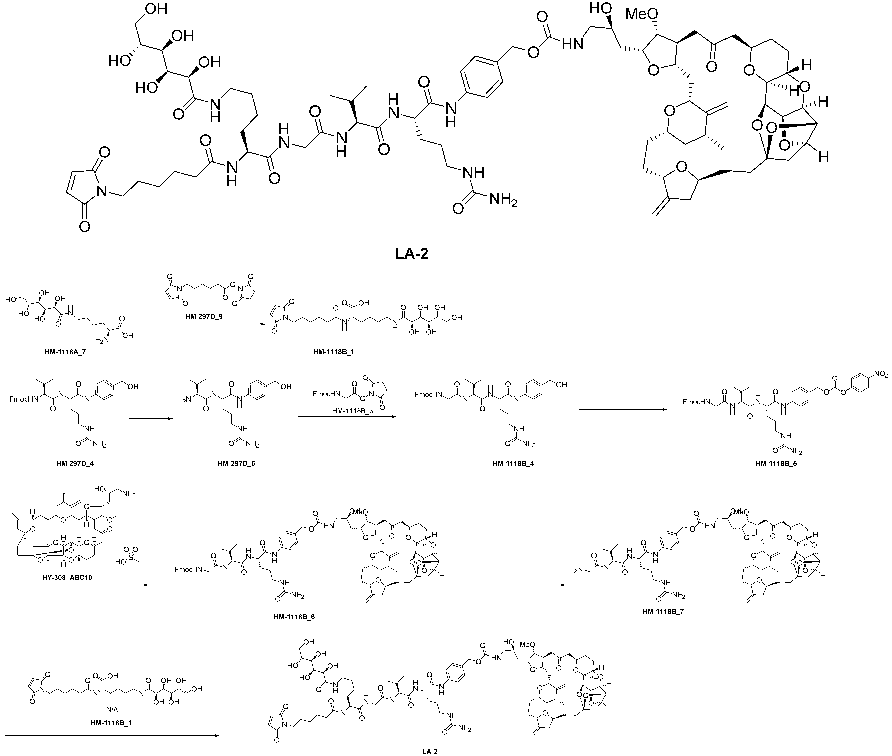

### Step 1: Preparation of compound HM-1118B_1

A starting material HM-297D_9 (1g, 3.24 mmol, 1 eq) was dissolved in DMF (10 mL, 10 V). HM-1118A_7 (1.58g, 4.87 mmol, 1.5 eq) and DIPEA (1.26g, 9.73 mmol, 3 eq) were added. After the addition, the mixture was allowed to react at 10°C-20°C for 16 hours. The LCMS analysis showed that some starting material remained. The reaction solution was purified by preparative high-performance liquid chromatography (preparative chromatograph manufacturer: Orienda, model: BRIX-2860, column: YMC-Triart Prep C18 150 × 30 mm × 10 µm, mobile phase: water (0.1% TFA)-acetonitrile, with an elution ratio of acetonitrile increased from 30% to 60%) (TFA system) and isolated, to afford 320 mg of the product as a white solid with yield of 19%.

¹H NMR (400 MHz, D₂O) δ 6.76 (s, 2H), 4.32-4.17 (m, 2H), 4.07-3.95 (m, 1H), 3.78-3.63 (m, 3H), 3.58 (dd, *J* = 11.6, 6.0 Hz, 1H), 3.43 (t, *J* = 6.9 Hz, 2H), 3.19 (t, *J* = 6.9 Hz, 2H), 2.22 (t, *J* = 7.2 Hz, 2H), 1.87-1.75 (m, 1H), 1.68 (ddd, *J* = 14.2, 11.9, 7.3 Hz, 1H), 1.61-1.47 (m, 6H), 1.43-1.26 (m, 2H), 1.26-1.14 (m, 2H). LCMS 518.2[M+1]⁺.

### Step 2: Preparation of compound HM-297D_5

A starting material HM-297D_4 (5.0g, 8.3 mmol) was suspended in anhydrous DCM (50 mL). DEA (diethylamine, 25 mL) was added, and the starting material gradually dissolved. The mixture was allowed to react at room temperature (10°C-20°C) for 2 hours. The LCMS analysis indicated complete conversion of the starting material. The reaction solution was evaporated to dryness at 30°C-40°C. The crude product was redissolved in dichloromethane (50 mL), and the mixture was evaporated to dryness twice and then recrystallized from isopropanol (20 V) to afford HM-297D_5 (3.0g, 95% yield) as a white powdery solid.

¹H NMR (400 MHz, DMSO) δ 10.02 (s, 1H), 8.11 (d, *J =* 6.8 Hz, 1H), 7.54 (d, *J =* 8.5 Hz, 2H), 7.24 (d, *J =* 8.5 Hz, 2H), 5.96 (t, *J =* 5.7 Hz, 1H), 5.40 (s, 2H), 5.09 (t, *J =* 5.7 Hz, 1H), 4.45 (dd, *J* = 16.0, 5.4 Hz, 3H), 3.16-2.80 (m, 3H), 2.07-1.85 (m, 1H), 1.79-1.50 (m, 4H), 1.50-1.25 (m, 2H), 0.89 (d, *J =* 6.9 Hz, 3H), 0.78 (d, *J* = 6.8 Hz, 3H). LCMS 380.3[M+1]⁺.

### Step 3: Preparation of compound HM-1118B_4

A starting material HM-297D_5 (1.5g, 3.96 mmol, 1 eq) was dissolved in anhydrous DMF (30 mL). HM-1118B_3 (1.59g, 4.0 mmol, 1.02 eq) was added, and DIPEA (765.3 mg, 5.9 mmol, 1.5 eq) was added dropwise to the reaction solution. The reaction solution became clear, and was allowed to react in an ice bath (0°C-5°C) for 1 hour. The LCMS analysis indicated complete conversion of the starting material. A solid precipitated from the reaction solution at this time. EA (150 mL) was added dropwise to the reaction solution. After the addition, the reaction solution was further stirred for 30 minutes, and filtered. The solid was collected to afford the product HM-1118B_4 (2.5g, 96.1% yield) as a white powdery solid.

¹H NMR (400 MHz, DMSO) δ 9.88 (s, 1H), 8.20 (d, *J =* 7.3 Hz, 1H), 7.88 (dd, *J =* 21.5, 8.0 Hz, 3H), 7.71 (d, *J* = 6.9 Hz, 2H), 7.66-7.51 (m, 3H), 7.43 (t, *J* = 7.4 Hz, 2H), 7.34 (t, *J* = 7.4 Hz, 2H), 7.24 (d, *J =* 8.3 Hz, 2H), 6.01 (s, 1H), 5.44 (s, 2H), 5.14 (t, *J =* 5.6 Hz, 1H), 4.51-4.34 (m, 3H), 4.25 (dt, *J* = 20.7, 7.0 Hz, 4H), 3.72 (d, *J* = 6.0 Hz, 2H), 3.09-2.93 (m, 2H), 2.11-1.92 (m, 1H), 1.80-1.56 (m, 2H), 1.54-1.31 (m, 2H), 0.86 (dd, *J* = 17.5, 6.7 Hz, 6H). LCMS 659.4[M+1]⁺.

### Step 4: Preparation of compound HM-1118B_5

The starting material HM-1118B_4 (450 mg, 0.68 mmol, 1 eq) was suspended in DMAC (N,N-dimethylacetamide, 13.5 mL, 30 V). NPC (517.2 mg, 1.7 mmol, 2.5 eq) was added, and DIPEA (132 mg, 1.0 mmol, 1.5 eq) was then added dropwise to the reaction solution. The reaction solution became turbid and was allowed to react at 20°C-30°C for 48 hours. After the reaction ended, the reaction solution was added dropwise to MTBE (methyl tert-butyl ether, 67.5 mL). A flocculent precipitate formed, and the reaction solution was filtered to collect the product.

¹H NMR (400 MHz, DMSO) δ 10.05 (s, 1H), 8.29 (dd, *J =* 30.8, 8.2 Hz, 3H), 7.88 (dd, *J=* 14.6, 8.1 Hz, 3H), 7.77-7.50 (m, 7H), 7.39 (ddd, *J =* 35.2, 10.3, 4.9 Hz, 6H), 6.02 (d, *J =* 5.1 Hz, 1H), 5.46 (s, 2H), 5.26 (s, 2H), 4.42 (dd, *J =* 13.1, 7.7 Hz, 1H), 4.26 (dq, *J =* 13.7, 6.9 Hz, 4H), 3.73 (d, *J =* 5.9 Hz, 2H), 3.14-2.97 (m, 2H), 2.07-1.99 (m, 1H), 1.84-1.57 (m, 2H), 1.44 (dd, *J =* 15.8, 8.7 Hz, 2H), 0.88 (dd, *J =* 18.0, 6.7 Hz, 6H). LCMS 824.3[M+1]⁺.

### Step 5: Preparation of compound HM-1118B_6

The starting material eribulin mesylate (30 mg, 36.33 µmol, 1 eq) was dissolved in DMAC (N,N-dimethylacetamide, 0.3 mL, 10 V). DIPEA (4.69 mg, 36.33 µmol, 1 eq, diluted in 0.1 mL of DMAC) was added, and the mixture was stirred for 10 minutes. Then HM-1118B_5 (41.89 mg, 51 µmol, 1.4 eq), HOBt (2.43 mg, 18 µmol, diluted in 0.1 mL of DMAC), and pyridine (5.74 mg, 72.7 µmol, 2 eq, diluted in 0.1 mL of DMAC) were added sequentially. After the addition, the reaction solution was allowed to react at 5°C-10°C for 16 hours. The LCMS analysis showed that the reaction ended. The reaction solution was then added dropwise to MTBE (5 mL). A flocculent precipitate formed, and the reaction solution was filtered to collect the product.

¹H NMR (400 MHz, DMSO) δ 9.95 (s, 1H), 8.37 (s, 1H), 8.19 (d, *J =* 7.2 Hz, 1H), 7.85 (dd, *J =* 26.8, 8.0 Hz, 3H), 7.73-7.66 (m, 2H), 7.64-7.49 (m, 3H), 7.41 (t, *J =* 7.3 Hz, 2H), 7.28 (dt, *J* = 24.3, 11.6 Hz, 4H), 7.07 (t, *J =* 5.6 Hz, 1H), 5.99 (t, *J =* 5.4 Hz, 1H), 5.41 (s, 2H), 5.10-4.87 (m, 4H), 4.83 (s, 1H), 4.75 (s, 1H), 4.63 (s, 1H), 4.55 (t, *J =* 4.2 Hz, 2H), 4.38 (dd, *J =* 13.3, 7.8 Hz, 1H), 4.31-4.13 (m, 6H), 4.02 (s, 4H), 3.86-3.45 (m, 10H), 3.25 (d, *J* = 7.5 Hz, 3H), 3.13 (d, *J =* 7.0 Hz, 1H), 3.02 (d, *J* = 6.8 Hz, 1H), 2.84 (d, *J* = 9.7 Hz, 1H), 2.73-2.65 (m, 1H), 2.62-2.55 (m, 1H), 2.28-2.09 (m, 4H), 2.00 (d, *J =* 7.4 Hz, 2H), 1.92 (s, 3H), 1.80-1.61 (m, 6H), 1.59-1.41 (m, 4H), 1.27 (t, *J =* 12.5 Hz, 13H), 1.00 (dd, *J =* 21.3, 9.0 Hz, 4H), 0.84 (dd, *J =* 17.8, 6.7 Hz, 6H). LCMS 1414.6[M+1]⁺.

### Step 6: Preparation of compound HM-1118B_7

The starting material HM-1118B_6 (45 mg, 31.8 µmol, 1 eq) was dissolved in DMAC/DEA (N,N-dimethylacetamide/triethylamine, 5:1, 0.67 mL, 15 V). The reaction solution was allowed to react at 5°C-10°C for 2 hours. The LCMS analysis showed that the reaction ended. The reaction solution was purified by preparative high-performance liquid chromatography (preparative chromatograph manufacturer: Orienda, model: BRIX-2860, column: YMC-Triart Prep C18 150 × 30 mm × 10 µm, mobile phase: water (0.1% TFA)-acetonitrile, with an elution ratio of acetonitrile increased from 30% to 60%) (TFA system) and isolated, to afford 14 mg of the product.

¹H NMR (400 MHz, DMSO) δ 10.12 (s, 1H), 8.43 (dd, *J =* 31.1, 8.2 Hz, 2H), 8.05 (s, 3H), 7.63 (d, *J =* 8.5 Hz, 2H), 7.34 (d, *J* = 8.5 Hz, 2H), 7.13 (t, *J* = 5.7 Hz, 1H), 6.11 (s, 1H), 5.52 (s, 2H), 5.05 (dd, *J =* 27.2, 17.9 Hz, 4H), 4.89 (s, 1H), 4.81 (s, 1H), 4.70 (s, 1H), 4.61 (d, *J =* 4.2 Hz, 1H), 4.51-4.38 (m, 2H), 4.32 (d, *J* = 10.4 Hz, 1H), 4.28 - 4.01 (m, 5H), 3.93-3.57 (m, 9H), 3.31 (d, *J* = 4.7 Hz, 3H), 3.12-2.96 (m, 4H), 2.93-2.63 (m, 4H), 2.48-2.14 (m, 6H), 2.13-1.90 (m, 7H), 1.86-1.31 (m, 15H), 1.30-1.18 (m, 2H), 1.07 (dd, *J* = 23.1, 9.0 Hz, 4H), 0.93 (dd, *J* = 18.2, 6.8 Hz, 6H). LCMS 1192.5[M+1]⁺, 597[1/2M+1].

### Step 7: Preparation of compound LA-2

The starting material HM-1118B_7 (40 mg, 33.56 µmol, 1 eq) was dissolved in DMF (2 mL). HM-1118B_1 (20.82 mg, 40.27 µmol, 1.2 eq) was added, and the mixture was cooled to 0°C in an ice bath. HATU (15.3 mg, 40.27 µmol, 1.2 eq) and DIPEA (13 mg, 100.68 µmol, 3 eq) were added. The reaction solution was allowed to react at 0°C-5°C for 2 hours and then purified by preparative high-performance liquid chromatography (preparative chromatograph manufacturer: Orienda, model: BRIX-2860, column: YMC-Triart Prep C18 150 × 30 mm × 10 µm, mobile phase: water (0.1% TFA)-acetonitrile, with an elution ratio of acetonitrile increased from 20% to 60%) and isolated to afford the product (24 mg, 42% yield) as a white solid.

¹H NMR (400 MHz, DMSO) δ 9.96 (s, 1H), 8.19 (s, 2H), 7.95 (d, *J* = 7.3 Hz, 1H), 7.74 (d, *J* = 8.7 Hz, 1H), 7.62 (dd, *J =* 18.0, 7.1 Hz, 3H), 7.29 (d, *J* = 8.5 Hz, 2H), 7.11 (d, *J* = 8.1 Hz, 1H), 7.01 (d, *J =* 9.6 Hz, 2H), 6.01 (s, 1H), 5.47-5.34 (m, 3H), 4.99 (d, *J =* 26.0 Hz, 4H), 4.81 (d, *J =* 31.9 Hz, 2H), 4.51 (dd, *J* = 50.5, 22.6 Hz, 7H), 4.28-4.12 (m, 7H), 4.02 (d, *J =* 17.5 Hz, 2H), 3.80 (dd, *J =* 32.0, 7.9 Hz, 5H), 3.59-3.50 (m, 5H), 3.27 (s, 4H), 3.15-2.61 (m, 12H), 2.34-1.93 (m, 14H), 1.82-1.62 (m, 8H), 1.61-1.19 (m, 25H), 1.03 (dd, *J =* 22.6, 8.9 Hz, 4H), 0.85 (dd, *J =* 18.0, 6.7 Hz, 6H).

LCMS 1691.7[M+1]⁺, 846.6[1/2M+1]⁺.

### Example 9: Preparation of Compound LA-3

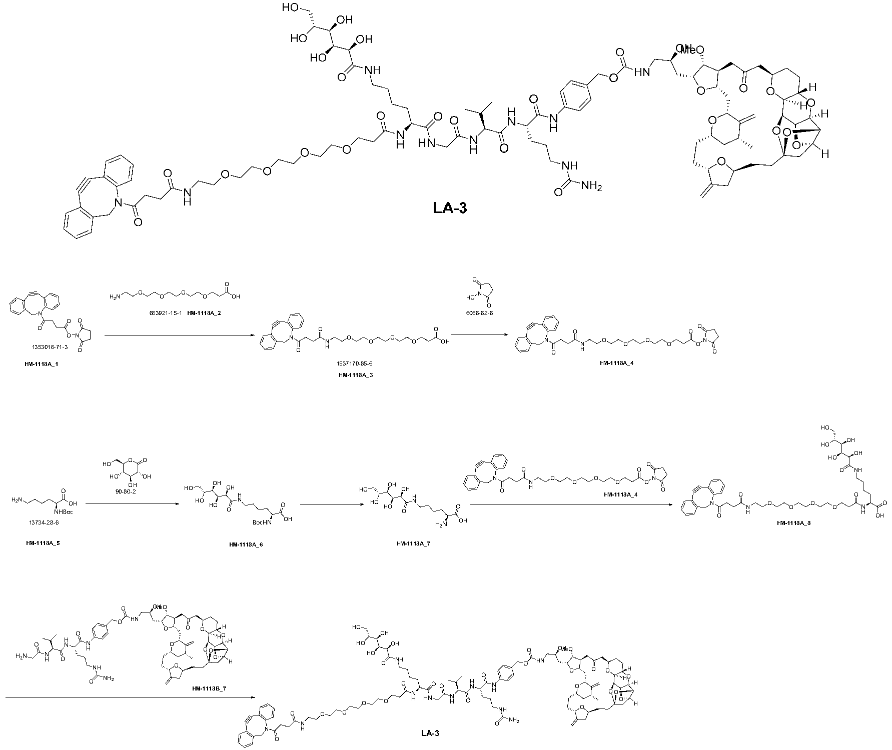

### Step 1: Preparation of compound HM-1118A_3

Starting materials TEA (triethylamine, 753 mg, 7.4 mmol) and HM-1118A_2 (659 mg, 2.4 mmol) were dissolved in anhydrous DMF (15 mL). HM-1118A_1 (1.0g, 2.4 mmol) was added, and the reaction solution was allowed to react at room temperature (10°C-20°C) for 17 hours. The LCMS analysis indicated complete conversion of the starting material. The residue was purified by silica gel column chromatography (C18) (ISCO, R-330 g SepaFlash Silica Flash Column, eluent of 5%-75% water/CH₃CN at 100 mL/min), and lyophilized to afford 1.2g of the product with yield of 87%.

LCMS(ESI):m/z, 553.2[M+H]⁺, 575.2[M+Na]⁺. ¹H NMR (400 MHz, DMSO) δ 7.71-7.65 (m, 1H), 7.62 (d, J = 7.0 Hz, 1H), 7.54-7.44 (m, 3H), 7.41-7.28 (m, 3H), 5.04 (d, J = 14.0 Hz, 1H), 3.65-3.56 (m, 3H), 3.50-3.40 (m, 9H), 3.30 (t, J = 5.7 Hz, 2H), 3.13-3.03 (m, 2H), 2.62-2.54 (m, 4H), 2.40 (t, J = 6.4 Hz, 2H), 2.29-2.18 (m, 1H), 2.05-1.95 (m, 1H), 1.87-1.71 (m, 1H).

### Step 2: Preparation of compound HM-1118A_4

HM-1118A_3 (2.3g, 4.16 mmol) and HOSu (N-hydroxysuccinimide, 0.718g, 6.24 mmol) were dissolved in DCM (40 mL). EDCI (1-ethyl-3-(3-dimethylaminopropyl)carbodiimide hydrochloride, 1.19g, 6.24 mmol) was added during stirring, and the reaction solution was allowed to react at 10°C-15°C for 16 hours. The LCMS analysis indicated the reaction ended. Water (20 mL) was added to the reaction solution and the layers were separated. The aqueous phase was extracted with DCM (40 mL). The organic phases were combined twice, washed with saturated brine, dried over anhydrous sodium sulfate, and concentrated to afford 2.1g of a pale yellow foam with yield of 77%.

LCMS(ESI):m/z, 650.2[M+H]⁺, 672.2[M+Na]⁺. ¹H NMR (400 MHz, DMSO) δ 7.81 (t, *J=* 5.5 Hz, 1H), 7.77-7.72 (m, 1H), 7.70-7.66 (m, 1H), 7.59-7.49 (m, 3H), 7.48-7.35 (m, 3H), 5.09 (d, *J* = 14.0 Hz, 1H), 3.77 (t, *J =* 6.0 Hz, 2H), 3.68 (dd, *J =* 12.5, 5.4 Hz, 1H), 3.64-3.43 (m, 14H), 3.35 (d, *J =* 6.2 Hz, 2H), 3.19-3.10 (m, 2H), 2.97 (t, *J =* 6.0 Hz, 2H), 2.69-2.60 (m, 1H), 2.35-2.25 (m, 1H), 2.11-2.02 (m, 1H), 1.89-1.75 (m, 1H).

### Step 3: Preparation of compound HM-1118A_6

The starting material HM-1118A_5 (5g, 0.02 mol) and the compound (CAS: 90-80-2, 3.6g, 0.02 mol) were dissolved in MeOH (80 mL). TEA (4.1g, 0.04 mol) was added, and the reaction solution was allowed to react at 70°C for 16 hours. The LCMS analysis indicated complete conversion of the starting material. The reaction solution was concentrated, and 50 mL of MTBE was added three times, and the mixture was concentrated to dryness each time. The residue was triturated with petroleum ether to afford 8g of the crude product as a white foam (the product was an acidic compound and formed a salt with TEA). The yield of the crude product was not calculated.

LCMS(ESI):m/z, 425.2[M+H]⁺, 447.2[M+Na]⁺. ¹H NMR (400 MHz, DMSO) δ 7.50 (brs, 1H), 5.61 (d, *J* = 4.0 Hz, 1H), 4.27 (s, 1H), 4.13 (s, 1H), 4.10-3.85 (m,1H), 3.85-3.66 (m, 4H), 3.28-3.15 (m, 2H), 1.80-1.55 (m, 2H),1.55-1.48 (m, 2H), 1.50-1.21 (m, 11H).

### Step 4: Preparation of compound LA-1118A_7

The starting material HM-1118A_6 (7g) was dissolved in DCM (35 mL). TFA (35 mL) was added, and the reaction solution was allowed to react at 10°C-15°C for 16 hours. The LCMS analysis showed complete consumption of the starting material. The reaction solution was concentrated, and DCM (50 mL × 3) was added and concentrated three times. The residue was dissolved in water and lyophilized to afford 8g of a yellow sticky crude product. The yield was not calculated.

¹H NMR (400 MHz, DMSO) δ 9.26 (s, 1H), 8.26 (d, *J* = 25.1 Hz, 3H), 7.69 (dd, *J =* 37.2, 31.4 Hz, 1H), 3.99 (d, *J* = 3.7 Hz, 1H), 3.94-3.84 (m, 2H), 3.58 (dd, *J* = 13.3, 3.7 Hz, 1H), 3.51-3.45 (m, 2H), 3.40-3.34 (m, 1H), 3.09 (qt, *J =* 15.7, 7.9 Hz, 2H), 1.84-1.67 (m, 2H), 1.60-1.27 (m, 4H), 1.18 (t, *J* = 7.3 Hz, 5H).

### Step 5: Preparation of compound HM-1118A_8

HM-1118A_7 (3g, 9.6 mmol) was dissolved in DMF (40 mL). TEA (1.6g, 16 mmol) was added to make the reaction mixture basic (pH > 9). Then a solution of HM-1118A_4 (2.1g, 3.2 mmol) in DMF (10 mL) was added, and the mixture was allowed to react at 25°C-30°C for 16 hours. The LCMS analysis indicated the reaction ended. The residue was purified by silica gel column chromatography (C18, ISCO, R-330 g SepaFlash Silica Flash Column, eluent of 5%-75% water/CH₃CN at 100 mL/min) and lyophilized to afford 600 mg of the product with yield of 22%.

LCMS(ESI):m/z, 859.3[M+H]⁺, 882.3[M+Na]⁺. ¹H NMR (400 MHz, DMSO) δ 7.75 (s, 2H), 7.71-7.55 (m, 3H), 7.55-7.42 (m, 3H), 7.40-7.23 (m, 3H), 5.03 (d, *J* = 14.0 Hz, 1H), 4.12-3.89 (m, 3H), 3.66-3.57 (m, 4H), 3.52-3.42 (m, 12H), 3.29-3.25 (m, 5H), 3.15-3.00 (m, 4H), 2.75-2.65 (m, 5H), 2.34-2.22 (m, 4H), 2.05-1.95 (m, 1H), 1.81-1.73 (m, 1H), 1.67-1.47 (m, 2H), 1.42-1.32 (m, 2H), 1.30-1.20 (m, 2H).

### Step 6: Preparation of compound LA-3

HM-1118A_8 (34 mg, 0.04 mmol) and HM-1118B_7 (for the synthesis of the compound, refer to the Example of LA-2) (40 mg, 0.03 mmol) were dissolved in DMF (2 mL). The mixture was cooled to 0-5°C in an ice bath. HATU (15 mg, 0.04 mmol) and DIPEA (10.8 mg, 0.08 mmol) were added sequentially, and the mixture was maintained at 0-5°C for 1 hour. The LCMS analysis showed that the reaction ended. The reaction solution was purified by preparative high-performance liquid chromatography (preparative chromatograph manufacturer: Orienda, model: BRIX-2860, column: YMC-Triart Prep C18 150 × 30 mm × 10 µm, mobile phase: water (0.1% TFA)-acetonitrile, with an elution ratio of acetonitrile increased from 30% to 60%) and lyophilized, to afford 29 mg of the product with yield of 42%.

LCMS(ESI):m/z, 1017.2[M/2+H]⁺. ¹H NMR (400 MHz, DMSO) δ 9.94 (s, 1H), 8.77 (d, *J* = 3.8 Hz, 1H), 8.54 (d, *J =* 7.7 Hz, 1H), 8.16 (s, 2H), 8.03 (d, *J =* 7.3 Hz, 1H), 7.78-7.66 (m, 3H), 7.60 (dd, *J =* 15.3, 7.8 Hz, 4H), 7.53-7.44 (m, 3H), 7.40-7.32 (m, 2H), 7.29 (t, *J =* 8.4 Hz, 3H), 7.08 (d, *J =* 4.9 Hz, 1H), 5.99 (s, 1H), 5.42 (s, 3H), 5.15-4.71 (m, 8H), 4.69-4.50 (m, 4H), 4.37 (d, *J* = 6.0 Hz, 2H), 4.29-3.98 (m, 11H), 3.96-3.64 (m, 9H), 3.61-3.54 (m, 6H), 3.48 (s, 11H), 3.16-2.80 (m, 13H), 2.40 (dd, *J =* 13.8, 7.1 Hz, 4H), 2.32-2.08 (m, 8H), 2.06-1.85 (m, 9H), 1.82-1.12 (m, 26H), 1.10-0.95 (m, 4H), 0.84 (dd, *J* = 17.4, 6.7 Hz, 6H).

### Example 10: Preparation of Compound LA-4

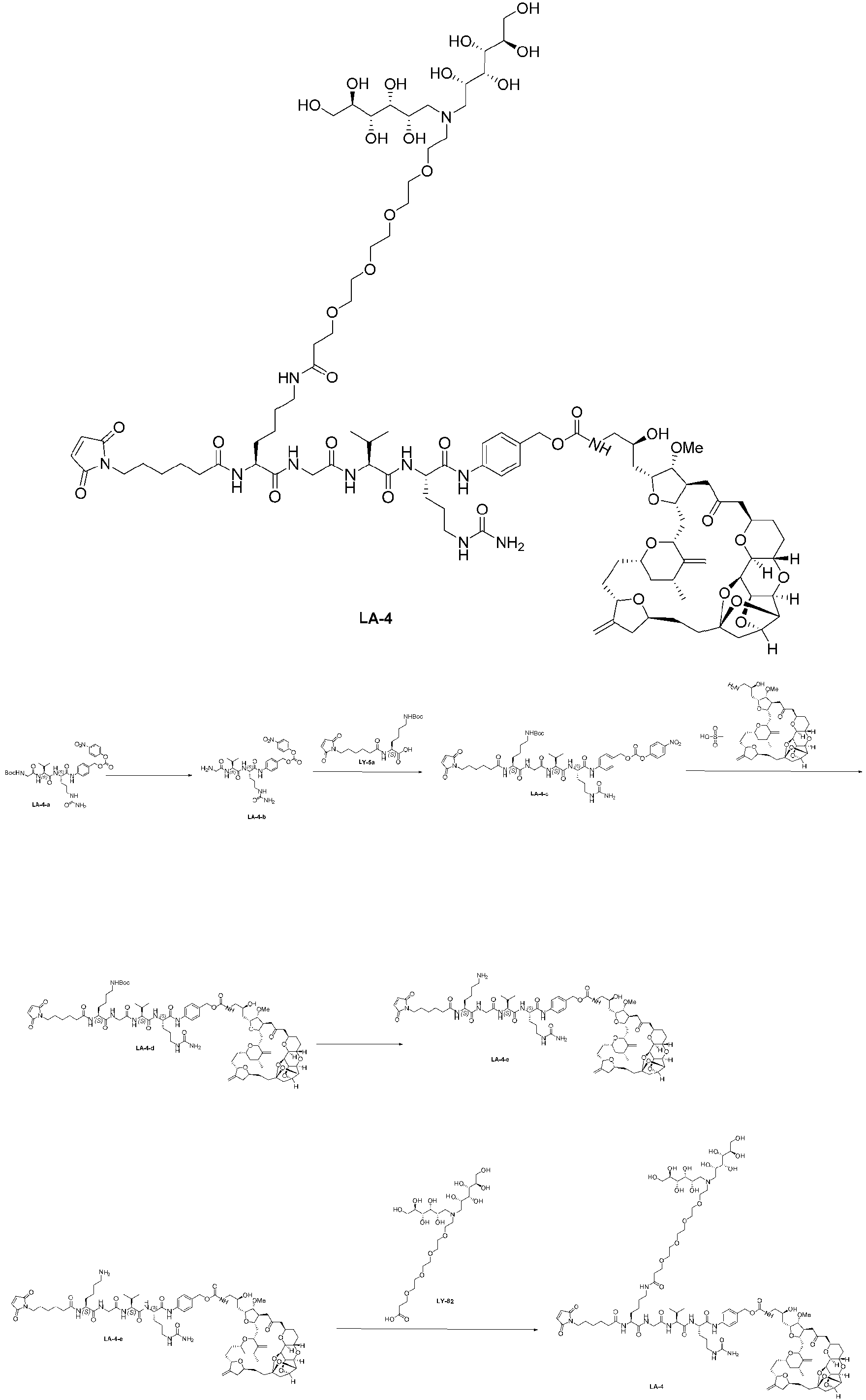

### Step 1: Preparation of compound LA-4-b

Trifluoroacetic acid (0.5 mL) was added to a solution of compound LA-4-a (purchased from ChemExpress, 10.60 mg, 8.27 µmol, 1 eq) in DCM (5 mL) at 0°C. After the addition, the mixture was allowed to react at 0-5°C for 5 hours. The LCMS analysis showed that the reaction ended. The reaction solution was evaporated to dryness to afford LA-4-b (47 mg, 90.00% yield) as a yellow oil.

LCMS (ESI): m/z, 602.2[M+H]⁺. Rt = 0.74 min.

### Step 2: Preparation of compound LA-4-c

DIPEA (7 eq, 74.64 mg, 577.50 µmol) and HATU (1.5 eq, 47.05 mg, 123.80 µmol) were added to a solution of compound LA-4-b (1 eq, 72 mg, 82.50 µmol, 82%) and LY-5a (1.6 eq, 73.07 mg, 132 µmol) in DMF (3 mL) at 0°C. After the addition, the reaction solution was allowed to react at 25°C for 12 hours. The LCMS analysis showed that the reaction ended. The reaction solution was purified by high-performance liquid chromatography (preparative chromatograph manufacturer: Shimadzu, model: LC-20AP, column: YMC-Triart Prep C18 250 × 50 mm × 7 µm, mobile phase: water (0.225% HCOOH)-acetonitrile, with an elution ratio of acetonitrile increased from 30% to 60%) and lyophilized, to afford compound LA-4-c (34.60 mg, 36.88% yield) as a white solid.

LCMS (ESI): m/z, 1045.6[M+Na]⁺.

### Step 3: Preparation of compound LA-4-d

DIPEA (17.39 mg, 134.55 µmol, 5 eq) and HOBt (1.82 mg, 13.46 µmol, 0.5 eq) were added to a solution of compound LA-4-c (30.60 mg, 26.91 µmol, 1 eq) and eribulin mesylate (23.90 mg, 26.91 µmol, 1 eq, purchased from Shandong Xingzhi Biopharmaceutical) in DMF (2 mL) at 0°C. After addition, the reaction solution allowed to react at 25°C for 12 hours, and the LCMS analysis showed that the reaction ended. The reaction solution was purified by preparative high-performance liquid chromatography (preparative chromatograph manufacturer: Orienda, model: BRIX-2860, column: YMC-Triart Prep C18 250 × 50 mm × 7 µm, mobile phase: water (0.225% HCOOH)-acetonitrile, with an elution ratio of acetonitrile increased from 48% to 78%) and lyophilized, to afford compound LA-4-d (1 mg, 25.34% yield) as a white solid.

LCMS (ESI): m/z, 1636.0[M+Na]⁺. Rt = 2.82 min.

### Step 4: Preparation of compound LA-4-e

Trifluoroacetic acid (0.5 mL) was added to a solution of compound LA-4-d (11 mg, 6.82 µmol, 1 eq) in DCM (5 mL) at 0°C. After the addition, the mixture was allowed to react at 0-10°C for 3 hours. The LCMS analysis showed that the reaction ended. The reaction solution was evaporated to dryness to afford LA-4-e (11 mg, crude product) as a yellow oil.

LCMS (ESI): m/z, 1537.1[M+H]⁺. Rt = 2.44 min.

### Step 5: Preparation of compound LA-4

DIPEA (5.56 mg, 43.00 µmol, 7 eq) and HATU (7.01 mg, 18.43 µmol, 3 eq) were added to a solution of compound LA-4-e (10 mg, 6.14 µmol, 1 eq) and LY-82 (13.04 mg, 18.43 µmol, 3 eq) in DMF (3 mL) at 0°C, and after addition, the reaction solution was allowed to react at 25°C for 2 hours, and the LCMS analysis showed that the reaction ended. The reaction solution was purified by preparative high-performance liquid chromatography (preparative chromatograph manufacturer: Orienda, model: BRIX-2860, column: Welch Xtimate C18 150 × 21.2 mm × 5 µm, mobile phase: water (0.225% HCOOH)-acetonitrile, with an elution ratio of acetonitrile increased from 21% to 51%) and lyophilized, to afford compound LA-4 (4.80 mg, 37.40% yield) as a white solid.

LCMS (ESI): m/z, 1045.8[1/2M+Na]⁺.

¹H NMR (400 MHz, DMSO-*d*₆) δ 9.95 (s, 1H), 8.17 (d, *J =* 8.0 Hz, 2H), 7.94 (d, *J =* 7.5 Hz, 1H), 7.80 (s, 1H), 7.72 (d, *J =* 8.6 Hz, 1H), 7.58 (d, *J =* 8.3 Hz, 2H), 7.27 (d, *J =* 8.3 Hz, 2H), 7.09 (s, 1H), 7.00 (s, 2H), 6.20 (d, *J =* 3.0 Hz, 1H), 6.01-5.92 (m, 2H), 5.42 (s, 2H), 4.94 (s, 3H), 4.87 (d, *J=* 3.3 Hz, 2H), 4.81 (s, 1H), 4.75 (s, 1H), 4.61 (t, *J =* 6.9 Hz, 2H), 4.48 (d, *J* = 5.5 Hz, 2H), 4.32 (dd, *J* = 11.8, 6.1 Hz, 6H), 4.28-4.14 (m, 4H), 4.00 (s, 2H), 3.88-3.65 (m, 8H), 3.58 (q, *J =* 5.8, 4.7 Hz, 7H), 3.48 (d, *J* = 8.1 Hz, 14H), 3.41-3.35 (m, 15H), 3.19 (s, 5H), 2.97 (dd, *J* = 12.6, 6.7 Hz, 8H), 2.81-2.72 (m, 3H), 2.68-2.57 (m, 5H), 2.29 (t, *J =* 6.6 Hz, 3H), 2.16-2.04 (m, 4H), 2.01-1.88 (m, 4H), 1.76-1.56 (m, 10H), 1.47 (h, *J =* 7.3 Hz, 9H), 1.33 (dt, *J =* 16.7, 8.3 Hz, 7H), 1.22-1.04 (m, 5H), 1.03 (s, 3H), 0.83 (dd, *J =* 18.6, 6.7 Hz, 6H).

### Example 11: Preparation of Compound LA-6

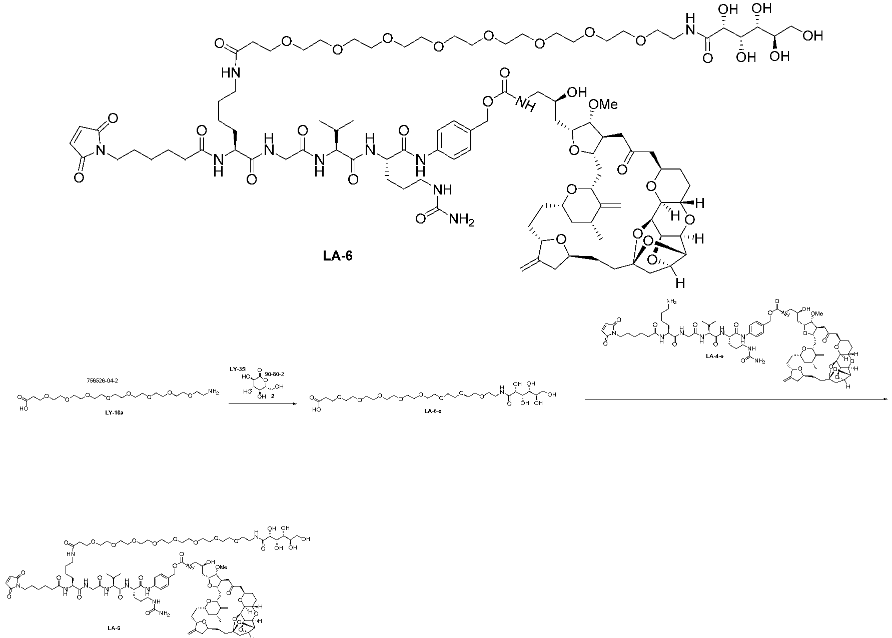

### Step 1: Preparation of compound LA-6-a

LY-35i (1.94g, 16 eq) and triethylamine (411 mg, 6 eq) were added to a solution of compound LY-10a (purchased from Bide Pharmatech Co., Ltd., 300 mg, 679.47 µmol, 1 eq) in MeOH (10 mL) at room temperature. After the addition, the reaction solution was allowed to react at 70°C for 21 hours. The LCMS analysis showed that the reaction ended. The reaction solution was evaporated to dryness to afford a crude product, and the crude product was purified by preparative high-performance liquid chromatography (preparative chromatograph manufacturer: Orienda, model: BRIX-2860, column: Welch Xtimate C18 150 × 21.2 mm × 5 µm, mobile phase: water (0.225% HCOOH)-acetonitrile, with an elution ratio of acetonitrile increased from 11% to 41%) and lyophilized, to afford LA-6-a (354 mg, 84.08% yield) as a colorless oil.

LCMS (ESI): m/z, 620.4[M+H]⁺. Rt = 2.187 min.

### Step 2: Preparation of compound LA-6

DIPEA (7.38 mg, 6 eq) and HATU (6.77 mg, 9 eq) were added to a solution of compound LA-6-a (3 mg, 1.98 µmol, 1 eq) and LA-4-e (13.04 mg, 18.43 µmol, 3 eq; for the synthesis of the compound, refer to the Example of LA-4) in DMF (3 mL) at 0°C. After the addition, the reaction solution was allowed to react at 25°C for 2 hours, and the LCMS analysis showed that the reaction ended. The reaction solution was purified by preparative high-performance liquid chromatography (preparative chromatograph manufacturer: Orienda, model: BRIX-2860, column: Welch Xtimate C18 150 × 21.2 mm × 5 µm, mobile phase: water (0.225% HCOOH)-acetonitrile, with an elution ratio of acetonitrile increased from 12% to 42%) and lyophilized, to afford compound LA-6 (0.360 mg, yield: 8.59%).

LCMS (ESI): m/z, 1058.3[1/2M+Na]⁺.

¹H NMR (400 MHz, DMSO-*d*₆) δ 9.95 (s, 1H), 8.45 (s, 1H), 8.18 (s, 2H), 7.94 (d, *J =* 7.5 Hz, 1H), 7.80 (s, 1H), 7.72 (d, *J =* 8.5 Hz, 1H), 7.58 (d, *J =* 8.2 Hz, 3H), 7.27 (d, *J =* 8.2 Hz, 2H), 7.09 (s, 1H), 7.00 (s, 2H), 6.19 (d, *J =* 2.9 Hz, 1H), 5.99 (s, 1H), 5.94 (d, *J* = 2.9 Hz, 1H), 5.43 (d, *J* = 4.4 Hz, 3H), 4.94 (s, 3H), 4.87 (d, *J =* 3.4 Hz, 2H), 4.78 (d, *J =* 25.9 Hz, 3H), 4.61 (t, *J =* 6.4 Hz, 2H), 4.55 (d, *J =* 3.9 Hz, 1H), 4.48 (s, 1H), 4.41 (d, *J =* 7.2 Hz, 1H), 4.33 (dd, *J =* 13.1, 7.6 Hz, 4H), 4.25-4.14 (m, 3H), 4.08-3.97 (m, 3H), 3.87 (d, *J =* 26.7 Hz, 4H), 3.73 (d, *J =* 5.5 Hz, 4H), 3.57 (t, *J* = 6.6 Hz, 5H), 3.53-3.45 (m, 28H), 3.19 (s, 5H), 3.06-2.89 (m, 10H), 2.82-2.71 (m, 4H), 2.28 (t, *J* = 6.6 Hz, 5H), 2.10 (dt, *J =* 12.3, 6.6 Hz, 4H), 1.97 (d, *J =* 15.5 Hz, 4H), 1.84 (d, *J =* 11.3 Hz, 2H), 1.70 (d, *J* = 13.3 Hz, 11H), 1.53-1.40 (m, 9H), 1.38-1.26 (m, 7H), 1.18 (q, *J* = 7.7 Hz, 3H), 1.08-0.99 (m, 4H), 0.83 (dd, *J =* 18.4, 6.7 Hz, 6H).

### Example 12: Preparation of Compound LA-9

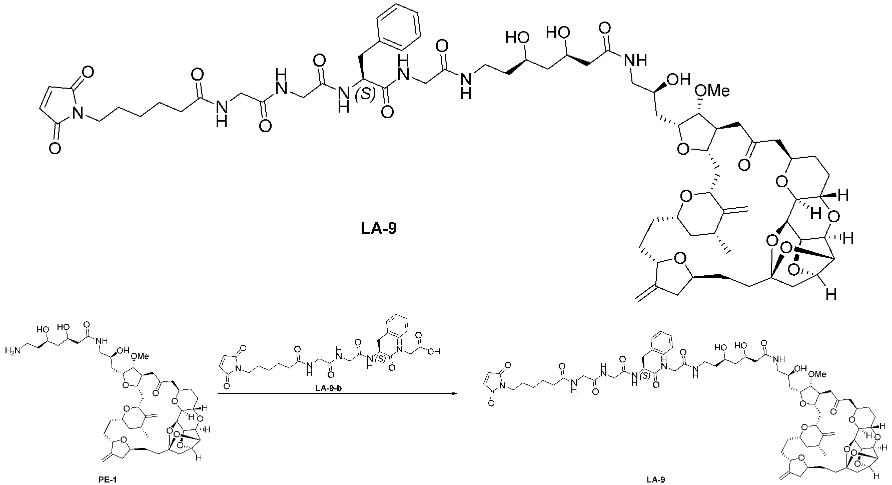

N,N-diisopropylethylamine (2.83 µL, 17.1 µmol, 2 eq) and N,N,N',N'-tetramethyl-O-(7-azabenzotriazol-1-yl)uronium hexafluorophosphate (3.9 mg, 10.26 µmol, 1.2 eq) were added to a solution of compound PE-1 (8 mg, 8.55 mmol, 1 eq) and (6-(2,5-dioxo-2,5-dihydro-1H-pyrrol-1-yl)hexanoyl)glycyl-L-phenylalanylglycine (LA-9-b, 4.53 mg, 8.55 mmol, 1 eq, purchased from ChemExpress) in N,N-dimethylformamide (2 mL) under an ice bath. After stirring for 5 minutes, the mixture was warmed to room temperature and stirred for 2 hours. LCMS analysis showed complete consumption of the starting material. The reaction solution was purified by preparative high-performance liquid chromatography (preparative chromatograph manufacturer: Orienda, model: LC-20 AP, column: GS-120-10-C18AP, mobile phase: water (0.225% HCOOH)-acetonitrile, with an elution ratio of acetonitrile increased from 30% to 60%), to afford the product LA-9 (0.89 mg, 7.43% yield). LCMS (ESI): m/z, 1400[M+H]⁺.

### Example 13: Preparation of Compound LA-10

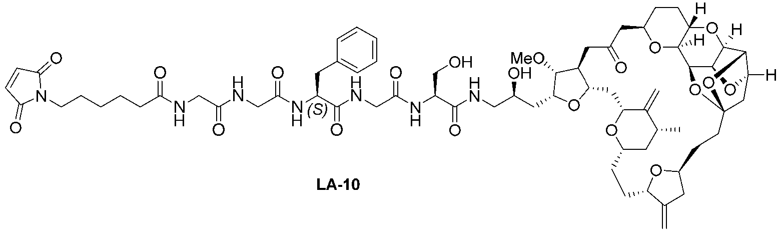

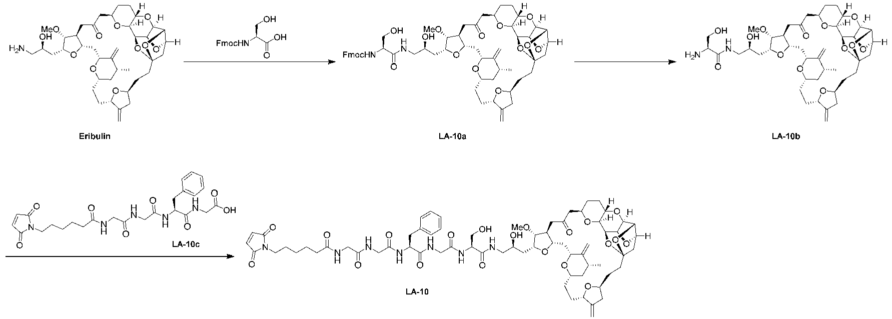

### Step 1: Preparation of compound LA-10a

Eribulin mesylate (40 mg, 48.43 µmol, 1 eq) and (((9H-fluoren-9-yl)methoxy)carbonyl)-L-serine (19.02 mg, 58.11 µmol, 1.2 eq) were dissolved in DMF (3 mL). N,N-diisopropylethylamine (12.52 mg, 16.01 µL, 96.85 µmol, 2 eq) and N,N,N',N'-tetramethyl-O-(7-azabenzotriazol-1-yl) uronium hexafluorophosphate (22.10 mg, 58.11 µmol, 1.2 eq) were added sequentially at 0°C. The solution was stirred at 10°C for 2 hours. The LCMS analysis indicated complete consumption of the starting material. The mixture was concentrated via an oil pump to afford the product LA-10a (50 mg, where the yield was not calculated for the crude product).

LCMS(ESI): m/z, 1039.4[M+H]⁺.

### Step 2: Preparation of compound LA-10b

Compound LA-10a (crude, 50 mg, 48.11 µmol, 1 eq) was dissolved in N,N-dimethyl formamide (2 mL). 1,8-diazabicyclo[5.4.0]undec-7-ene (21.97 mg, 21.56 µL, 144.34 µmol, 3 eq) was added, and the mixture was stirred at 10°C for 2 hours. LCMS analysis showed complete consumption of the starting material. The reaction solution was directly used for the next step without treatment.

LCMS(ESI): m/z, 817.4[M+H]⁺.

### Step 3: Preparation of compound LA-10

Compound LA-10b was dissolved in N,N-dimethylformamide (2 mL). (6-(2,5-dioxo-2,5-dihydro-1H-pyrrol-1-yl)hexanoyl)glycylglycyl-L-phenylalanylglycine (27.22 mg, 51.41 µmol, 1.2 eq) and 4-hydroxybenzotriazole (8.68 mg, 5.79 µL, 64.26 µmol, 1.5 eq) were added, and 1-(3-dimethylaminopropyl)-3-ethylcarbodiimide hydrochloride (12.32 mg, 14.05 µL, 64.26 µmol, 1.5 eq) was then added. The reaction solution was stirred at 10°C for 1 hour. LCMS analysis showed that the reaction ended. The reaction solution was subjected to preparative high-performance liquid chromatography to afford the compound LA-10 (40.14 mg, 69.52% yield, 98.57% purity).

The conditions of the preparative high-performance liquid chromatography were as follows:
preparative chromatograph manufacturer: Orienda, model: BRIX-2860, column: Phenomenex Luna C18 250 × 50 mm × 10 µm, mobile phase: water (0.225% HCOOH)-acetonitrile, with an elution ratio of acetonitrile increased from 30% to 60%.

LCMS(ESI): m/z, 1328.5[M+H]⁺.

¹H NMR (400 MHz, DMSO-*d*₆) δ 8.28 (t, *J =* 5.7 Hz, 1H), 8.15 (d, *J =* 7.9 Hz, 1H), 8.06 (t, *J =* 5.7 Hz, 1H), 7.99 (t, *J =* 5.7 Hz, 1H), 7.87 (d, *J =* 7.8 Hz, 1H), 7.79 (t, *J =* 5.7 Hz, 1H), 7.25 (d, *J* = 5.3 Hz, 4H), 7.21-7.14 (m, 1H), 7.00 (s, 2H), 5.05 (s, 1H), 5.00 (s, 1H), 4.89 (t, *J =* 5.6 Hz, 1H), 4.83 (s, 1H), 4.75 (s, 1H), 4.66-4.59 (m, 2H), 4.55 (t, *J =* 4.2 Hz, 1H), 4.48 (td, *J =* 9.4, 8.9, 4.3 Hz, 1H), 4.27 (t, *J =* 10.7 Hz, 2H), 4.17 (td, *J =* 10.3, 4.4 Hz, 1H), 4.10 (s, 3H), 4.02 (s, 1H), 3.84-3.64 (m, 8H), 3.62-3.46 (m, 6H), 3.26 (s, 4H), 3.11-3.00 (m, 3H), 2.87-2.63 (m, 5H), 2.60-2.54 (m, 1H), 2.36-2.17 (m, 6H), 2.16-2.07 (m, 3H), 1.97 (d, *J =* 33.5 Hz, 6H), 1.76-1.53 (m, 7H), 1.47 (h, *J =* 6.8 Hz, 6H), 1.29 (d, *J* = 13.1 Hz, 3H), 1.20 (d, *J =* 7.4 Hz, 3H), 1.00 (dd, *J* = 22.7, 9.1 Hz, 4H).

### Example 14: Preparation of Compound LA-11

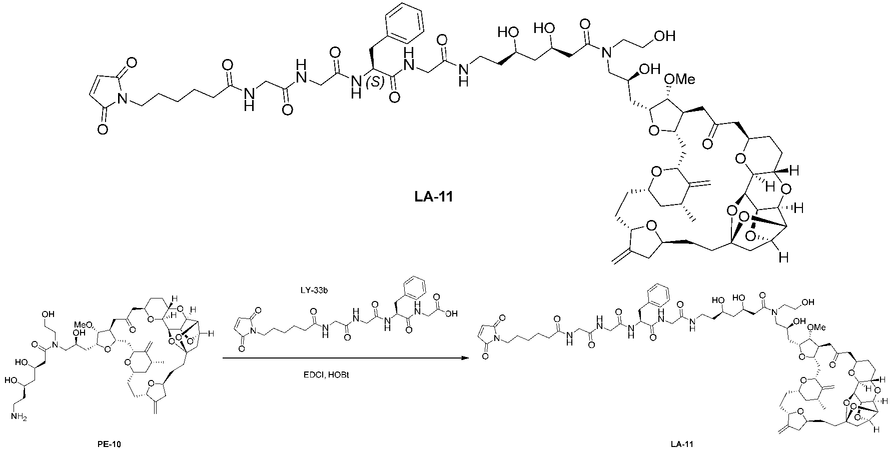

Compound PE-10 (4 mg, 4.29 µmol, 1 eq) was dissolved in DMF (1 mL). (6-(2,5-dioxo-2,5-dihydro-1H-pyrrol-1-yl)hexanoyl)glycylglycyl-L-phenylalanylglycine (LY-33b, purchased from ChemExpress, 2.27 mg, 4.29 µmol, 1 eq) and HOBT (1-hydroxybenzotriazole, 868.80 µg, 5.79e⁻¹ µL, 6.43 µmol, 1.5 eq) were added, and EDCI·HCl (1.23 mg, 1.41 µL, 6.43 µmol, 1.5 eq) was then added. The mixture was stirred at 15°C for 1 hour. The LCMS showed that the product was generated. The reaction solution was subjected to preparative high-performance liquid chromatography to afford the product (8.20 mg, 70.91% yield) as a white solid.

The conditions of the preparative high-performance liquid chromatography were as follows:
preparative chromatograph manufacturer: Orienda, model: BRIX-2860, column: Phenomenex Luna C18 250 × 30 mm × 10 µm, mobile phase: water (0.225% HCOOH)-acetonitrile, with an elution ratio of acetonitrile increased from 30% to 60%.

LCMS (ESI): m/z, 1444.7[M+H]⁺.

### Example 15: Preparation of Compound LA-14

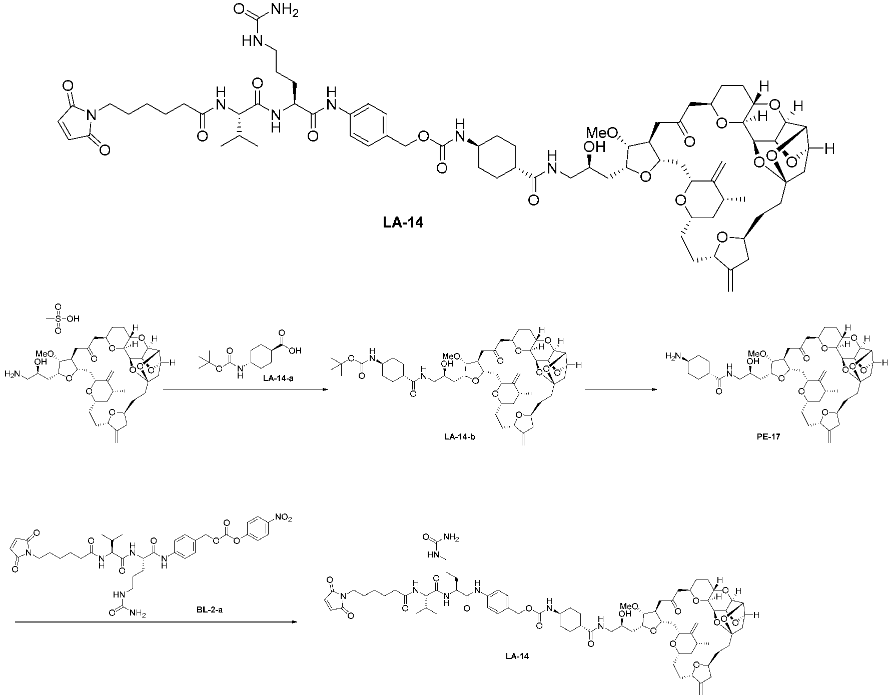

### Step 1: Preparation of compound LA-14-b

Eribulin mesylate (10 mg, 12.11 µmol, 1 eq) was dissolved in DMF (2 mL). (1R,4R)-4-((tert-butoxycarbonyl)amino)cyclohexane-1-carboxylic acid (purchased from ChemExpress, 3.53 mg, 14.53 µmol, 1.2 eq) and DIPEA (3.13 mg, 4 µL, 24.21 µmol, 2 eq) were added. HATU (5.52 mg, 14.53 µmol, 1.2 eq) was added at 0°C, and the mixture was stirred at 25°C for 1 hour. The LCMS showed that the product was generated. The reaction solution was subjected to preparative high-performance liquid chromatography to afford the product (8.20 mg, 70.91% yield) as a white solid.

The conditions of the preparative high-performance liquid chromatography were as follows:
preparative chromatograph manufacturer: Orienda, model: BRIX-2860, column: Welch Xtimate C18 150 × 30 mm × 10 µm, mobile phase: water (0.225% HCOOH)-acetonitrile, with an elution ratio of acetonitrile increased from 30% to 60%.

LCMS (ESI): m/z, 977.6[M+Na]⁺.

### Step 2: Preparation of compound PE-17

The compound LA-14-b (8.20 mg, 8.58 µmol, 1 eq) was dissolved in DCM (2 mL), TFA (307 mg, 200 µL, 2.69 mmol, 313.6 eq) was added in an ice bath, and the reaction solution was stirred at 0-25°C for 12 hours. LCMS analysis showed that the reaction ended. The reaction solution was directly evaporated to dryness to afford the product (7 mg, 95.36% yield) as a white oil to be directly used in the next step.

LCMS (ESI): m/z, 855.5[M+H]⁺.

### Step 3: Preparation of compound LA-14

Compound PE-17 (5 mg, 5.85 µmol, 1 eq) was dissolved in DMF (1 mL). DIPEA (2.27 mg, 2.90 µL, 17.54 µmol, 3 eq) and compound BL-2-a (purchased from ChemExpress, 5.18 mg, 7.02 µmol, 1.2 eq) were added at 0°C. The reaction solution was stirred at 25°C, and the LCMS analysis showed complete consumption of the starting material. The reaction solution was purified by high-performance liquid chromatography to afford the product LA-14 (8 mg, 86.81% yield) as a white solid.

The conditions of the preparative high-performance liquid chromatography were as follows:
Preparative chromatograph manufacturer: Shimadzu, model: LC-20AP, column: Welch Xtimate C18 250 × 30mm × 10 µm, mobile phase: water (0.225% HCOOH)-acetonitrile, with an elution ratio of acetonitrile increased from 30% to 60%.

LCMS (ESI): m/z, 1476.8[(M+H)]⁺.

### Example 16: Preparation of Compound LA-15

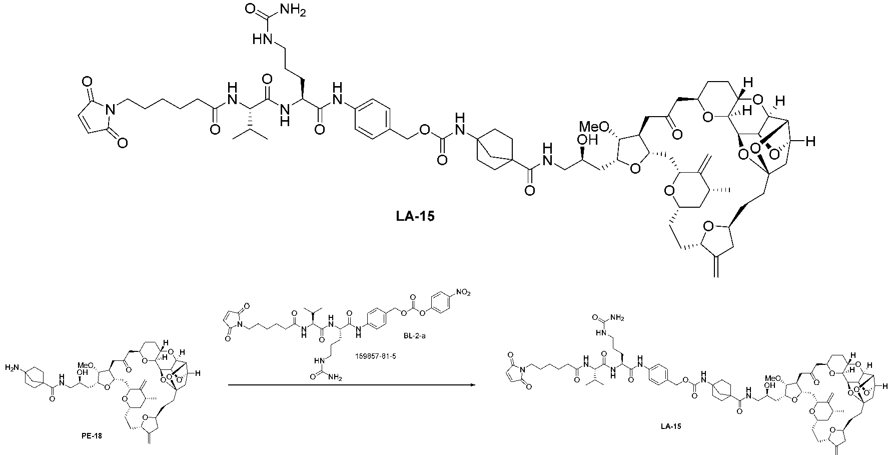

BL-2-a (purchased from ChemExpress, 2.09 mg, 2.84 µmol, 1.2 eq) was added to a solution of PE-18 (2.05 mg, 100%, 2.36 µmol, 1 eq) and N,N-diisopropylethylamine (916.66 µg, 1.17 µL, 7.09 µmol, 3 eq) in N,N-dimethylformamide (0.5 mL) at 0°C. The reaction solution was stirred at 20°C for 1 hour, and the LCMS analysis showed complete consumption of the starting material. The reaction solution was purified by preparative high-performance liquid chromatography (preparative chromatograph manufacturer: Orienda, model: BRIX-2860 (R1, 4, 5, 6, 7), column: Phenomenex Luna C18 250 × 50 mm × 10 µm, mobile phase: water (0.1% TFA)-acetonitrile, with an elution ratio of acetonitrile increased from 30% to 60%) and lyophilized, to afford the compound LA-15 (700 µg, 0.46 µmol, 19.42% yield, 96.15% purity) as a white solid.

LCMS (ESI): m/z, 733.6[1/2M+H]⁺.

### Example 17: Preparation of Compound LA-21

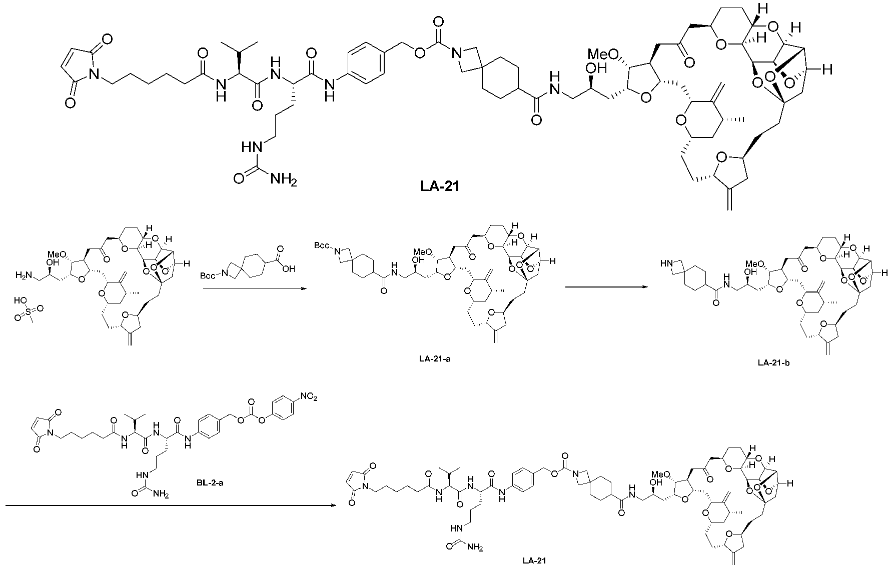

### Step 1: Preparation of compound LA-21-a

Eribulin mesylate (10 mg, 12.11 µmol, 1 eq) was dissolved in DMF (1 mL). 2-(tert-butoxy carbonyl)-2-azaspiro[3.5]nonane-7-carboxylic acid (3.69 mg, 13.70 µmol, 1 eq) and DIPEA (3.54 mg, 4.53 µL, 27.40 µmol, 2 eq) were added. HATU (6.25 mg, 16.44 µmol, 1.2 eq) was added at 0°C, and the mixture was stirred at 0-25°C for 2 hours. The LCMS showed that the product was generated. The reaction solution was subjected to preparative high-performance liquid chromatography to afford the product LA-21-a (7 mg, 52.07% yield) as a white solid.

The conditions of the preparative high-performance liquid chromatography were as follows:
Preparative chromatograph manufacturer: Shimadzu, model: LC-20AP, column: Welch Xtimate C18 250 × 30 mm × 10 µm, mobile phase: water (0.225% HCOOH)-acetonitrile, with an elution ratio of acetonitrile increased from 50% to 80%.

LCMS (ESI): m/z, 981.6[M+H]⁺.

### Step 2: Preparation of compound LA-21-b

LA-21-a (7 mg, 7.13 µmol, 1 eq) was dissolved in DCM (2 mL), TFA (307 mg, 200 µL, 2.69 mmol, 377.4261 eq) was added at 0°C, and the reaction solution was stirred at 25°C for 0.5 hours. LCMS analysis showed that the reaction ended. The reaction solution was directly evaporated to dryness to afford the product LA-21-b (5 mg, 79.54% yield) as a yellow oil.

LCMS (ESI): m/z, 881.6[M+H]⁺.

### Step 3: Preparation of compound LA-21

LA-21-b (6 mg, 1 eq) was dissolved in DMF (2 mL) at room temperature. DIPEA (1 mg, 1.28 µL, 7.74 µmol, 3 eq) was added, and BL-2-a (8 mg, 10.84 µmol, 1.2 eq, purchased from ChemExpress) was then added at 0°C. The reaction solution was stirred at 25°C for 1 hour, and the LCMS analysis showed complete consumption of the starting material. The reaction solution was purified by high-performance liquid chromatography to obtain the product LA-21 (880 µg, 80% purity) as a white solid.

The conditions of the preparative high-performance liquid chromatography were as follows:
Preparative chromatograph manufacturer: Shimadzu, model: LC-20AP, column: Welch Xtimate C18 250 × 30 mm × 10 µm, mobile phase: water (0.225% HCOOH)-acetonitrile, with an elution ratio of water increased from 30% to 60%.

LCMS (ESI): m/z, 1502.9[(M+H)]⁺.

### Example 18: Preparation of Compound LA-22

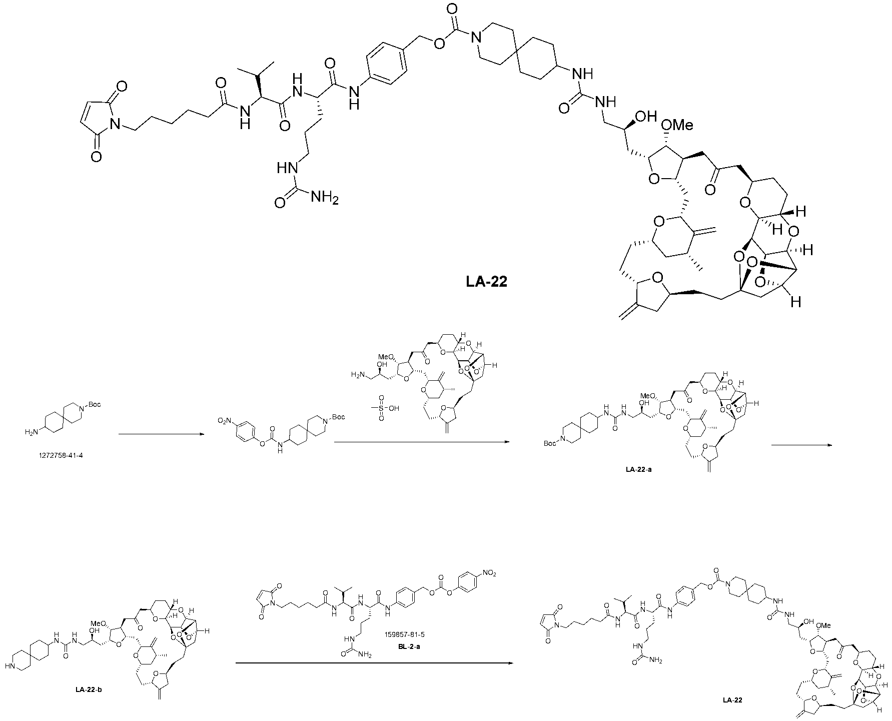

### Step 1: Preparation of tert-butyl 9-(((4-nitrophenoxy)carbonyl)amino)-3-azaspiro[5.5] undecane-3-carboxylate

Bis(4-nitrophenyl) carbonate (102.01 mg, 68.01 µL, 335.33 µmol, 3 eq) and DIPEA (28.89 mg, 36.95 µL, 223.56 µmol, 2 eq) were added to a solution of tert-butyl 9-amino-3-azaspiro[5.5]undecane-3-carboxylate (purchased from BirdoTech, 30 mg, 111.78 µmol, 1 eq) in DMF (1 mL) at 0°C. After the addition, the reaction solution was allowed to react at 25°C for 2 hours. The LCMS analysis indicated the reaction ended. The reaction solution was purified by preparative high-performance liquid chromatography (preparative chromatograph manufacturer: Orienda, model: BRIX-2860, column: Phenomenex Luna C18 150 × 25 mm × 10 µm, mobile phase: water (0.225% HCOOH) - acetonitrile, with an elution ratio of acetonitrile increased from 50% to 80%) and lyophilized, to afford tert-butyl 9-(((4-nitrophenoxy)carbonyl)amino)-3-azaspiro [5.5]undecane-3-carboxylate (19.50 mg, 40.24% yield).

LCMS (ESI): m/z, 334[M-100+H]⁺. Rt = 1.082 min.

### Step 2: Preparation of compound LA-22-a

HOBT (817.91 µg, 5.45e⁻¹ µL, 6.05 µmol, 0.5 eq) and DIPEA (4.69 mg, 6 µL, 36.32 µmol, 3 eq) were added to a solution of eribulin mesylate (10 mg, 12.11 µmol, 1 eq) and tert-butyl 9-(((4-nitrophenoxy)carbonyl)amino)-3-azaspiro[5.5]undecane-3-carboxylate (7.35 mg, 16.95 µmol, 1.4 eq) in DMF (1 mL) at 20°C. After addition, the reaction solution allowed to react at 25°C for 1 hour, and the LCMS analysis showed that the reaction ended. The reaction solution was combined with another small-scale batch and purified by preparative high-performance liquid chromatography (preparative chromatograph manufacturer: Orienda, model: BRIX-2860, column: Welch Xtimate C18 250 × 30 mm × 10 µm, mobile phase: water (0.225% HCOOH) - acetonitrile, with an elution ratio of water increased from 60% to 90%) and lyophilized, to afford compound LA-22-a (14 mg, 94.08% yield, batch 1.2; one-fifth scale trial product was combined with the current batch for purification) as a white solid.

LCMS (ESI): m/z, 1024.7[M+H]⁺. Rt = 3.55 min.

### Step 3: Preparation of compound LA-22-b

TFA (0.2 mL) was added to the solution of compound LA-22-a (14 mg, 13.67 µmol, 1 eq) in DCM (2 mL) at 0°C. After addition, the reaction solution was allowed to react at 0-10°C for 1 hour, the LCMS analysis showed that the reaction ended, and the reaction solution was directly evaporated to dryness to afford the compound LA-22-b (15 mg, crude product) as a yellow solid.

LCMS (ESI): m/z, 946.7[M+Na]⁺. Rt = 2.49 min.

### Step 4: Preparation of compound LA-22

DIPEA (6.29 mg, 8.05 µL, 48.69 µmol, 3 eq) was added to a solution of compound LA-22 -b (15 mg, 16.23 µmol, 1 eq) and BL-2-a (11.97 mg, 16.23 µmol, 1 eq) in DMF (2 mL) at 0°C, and after addition, the reaction solution was allowed to react at 25°C for 1 hour. The LCMS analysis showed that the reaction ended. The reaction solution was purified by preparative high-performance liquid chromatography (preparative chromatograph manufacturer: Orienda, model: BRIX-2860, column: Welch Xtimate C18 150 × 30 mm × 10 µm, mobile phase: water (10 mM NH₄HCO₃)-acetonitrile, with an elution ratio of acetonitrile increased from 50% to 80%) and lyophilized, to afford the compound LA-22 (2.96 mg, 11.98% yield, 84% purity) as a white solid.

LCMS (ESI): m/z, 1544.9[M+Na]⁺, 761.9[1/2M+1]⁺. Rt = 2.44 min.

### Example 19: Preparation of Compound LA-23

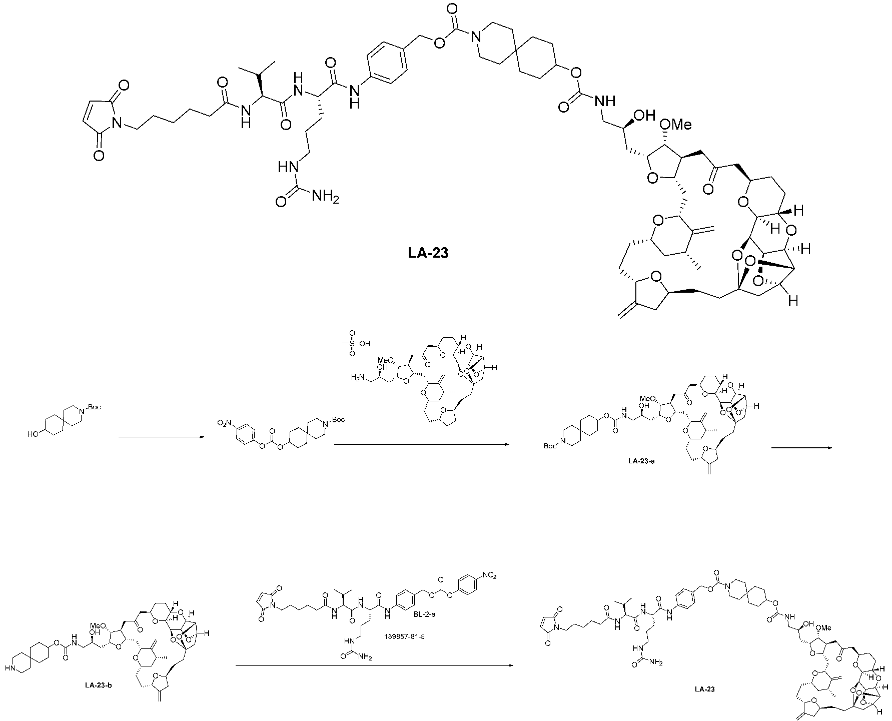

### Step 1: Preparation of tert-butyl 9-(((4-nitrophenoxy)carbonyl) oxy)-3-azaspiro[5.5] undecane-3-carboxylate

Bis(4-nitrophenyl) carbonate (67.76 mg, 45.17 µL, 222.73 µmol, 1.2 eq) and DIPEA (47.98 mg, 61.35 µL, 371.22 µmol, 2 eq) were added to a solution of tert-butyl 9-hydroxy-3-azaspiro[5.5] undecane-3-carboxylate (purchased from BirdoTech, 50 mg, 185.61 µmol, 1 eq) in DMF (3 mL) at 0°C. After the addition, the reaction solution was allowed to react at 30°C for 1 hour. The LCMS analysis indicated the reaction ended. The reaction solution was purified by preparative high-performance liquid chromatography (preparative chromatograph manufacturer: Orienda, model: BRIX-2860, column: Welch Xtimate C18 250 × 30 mm × 10 µm, mobile phase: water (0.225% HCOOH) - acetonitrile, with an elution ratio of water increased from 60% to 90%) and lyophilized, to afford tert-butyl 9-(((4-nitrophenoxy)carbonyl) oxy)-3-azaspiro[5.5]undecane-3-carboxylate (26.90 mg, 33.36% yield) as a bright yellow solid.

LCMS (ESI): m/z, 335[M-100+H]⁺. Rt = 3.459 min.

### Step 2: Preparation of compound LA-23-a

HOBT (925.61 µg, 6.17e⁻¹ µL, 6.85 µmol, 0.5 eq) and DIPEA (5.31 mg, 6.79 µL, 41.10 µmol, 3 eq) were added to a solution of eribulin mesylate (10 mg, 13.70 µmol, 1 eq) and tert-butyl 9-(((4-nitrophenoxy)carbonyl) oxy)-3-azaspiro[5.5]undecane-3-carboxylate (8.33 mg, 19.18 µmol, 1.4 eq) in DMF (3 mL) at 20°C. After addition, the reaction solution allowed to react at 25°C for 3 hours, and the LCMS analysis showed that the reaction ended. The reaction solution was purified by preparative high-performance liquid chromatography (preparative chromatograph manufacturer: Orienda, model: BRIX-2860, column: Welch Xtimate C18 250 × 30mm × 10 µm, mobile phase: water (0.225% HCOOH)-acetonitrile, with an elution ratio of acetonitrile increased from 60% to 90%) and lyophilized, to afford the product LA-23-a (12 mg, 85.43% yield) as a white solid.

LCMS (ESI): m/z, 1047.6[M+Na]⁺. Rt = 3.206 min.

### Step 3: Preparation of compound LA-23-b

TFA (0.6 mL) was added to the solution of compound LA-23-a (12 mg, 11.70 µmol, 1 eq) in DCM (6 mL) at 0°C. After addition, the reaction solution was allowed to react at 0-10°C for 30 minutes, the LCMS analysis showed that the reaction ended, and the reaction solution was directly evaporated to dryness to afford the compound LA-23-b (11 mg, crude product) as a yellow solid.

LCMS (ESI): m/z, 947.6[M+Na]⁺. Rt = 1.947 min.

### Step 4: Preparation of compound LA-23

DIPEA (4.61 mg, 5.89 µL, 35.67 µmol, 3 eq) was added to a solution of LA-23-b (11 mg, 11.89 µmol, 1 eq) and BL-2-a (11.97 mg, 16.23 µmol, 1 eq) in DMF (2 mL) at 0°C After addition, the reaction solution allowed to react at 25°C for 2 hours, and the LCMS analysis showed that the reaction ended. The reaction solution was purified by preparative high-performance liquid chromatography (preparative chromatograph manufacturer: Orienda, model: BRIX-2860, column: Welch Xtimate C18 150 × 30 mm × 10 µm, mobile phase: water (0.225% FA)-acetonitrile, with an elution ratio of acetonitrile increased from 30% to 60%) to afford compound LA-23 (8.84 mg, 48.79% yield, 84% purity) as a white solid.

LCMS (ESI): m/z, 1546.8[M+Na]⁺, 785.2[1/2M+Na]⁺. Rt = 2.564 min.

### Example 20: Preparation of Compound LA-24

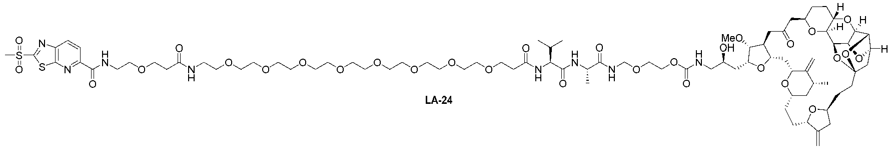

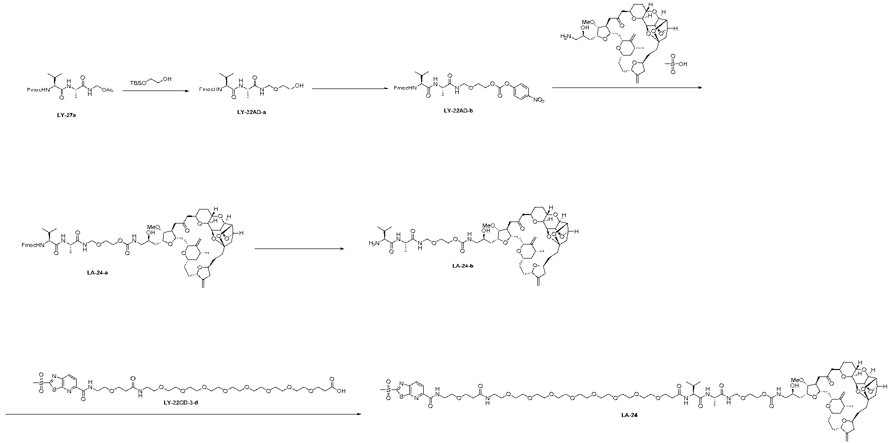

### Step 1: Preparation of (9H-fluoren-9-yl)methyl (((S)-1-(((S)-1-((2-hydroxyethoxy)methyl) amino)-1-oxopropan-2-yl)amino)-3-methyl-1-oxobutan-2-yl)carbamate

2-((tert-butyldimethylsilyl)oxy)ethan-1-ol (183.09 mg, 1.04 mmol, 2 eq) and TsOH (26.82 mg, 25.07 µL, 155.75 µmol, 0.3 eq) were added to a solution of compound LY-27a (250 mg, 519.16 µmol, 1 eq) in THF (10 mL) at room temperature. After the addition, the mixture was allowed to react at 33°C for 3 hours. The LCMS indicated the reaction ended. The reaction mixture was concentrated to afford the crude product, which was purified by conventional reversed-phase silica gel column (ISCO, 220 g SepaFlash Silica Flash Column, eluents: 0%-47% water/CH₃CN, 100 mL/min) and lyophilized to afford compound LY-22AD-a (98 mg, 39.04% yield) as a yellow solid.

LCMS (ESI): m/z, 506.3[M+H]⁺. Rt = 1.813 **min.**

### Step 2: Preparation of compound LY-22AD-b

Bis(p-nitrobenzene)carbonate (123.30 mg, 82.20 µL, 405.32 µmol, 2 eq) and DIPEA (78.58 mg, 100.48 µL, 607.98 µmol, 3 eq) were added to the solution of compound LY-22AD-a (98 mg, 202.66 µmol, 1 eq) in DMF (3 mL) at 0°C, and then the reaction solution allowed to react at 33°C for 2 hours. The LCMS analysis showed that the reaction ended. The reaction solution was concentrated to afford the crude product. The crude product was purified by silica gel column chromatography (eluents: methanol/dichloromethane = 1:20 to 1:10) to afford the compound LY-22AD-b (111 mg, crude, 75.99% yield, 90% purity) as a yellow solid.

### Step 3: Preparation of compound LA-24-a

DIPEA (1.56 mg, 2 µL, 12.11 µmol, 1 eq) was added to a solution of the compound Eribulin mesylate (10 mg, 12.11 µmol, 1 eq) in DMAC (3 mL) at 0°C, then a resulting mixture was stirred at 25°C for 10 minutes, and then LY-22AD-b (8.73 mg, 90%, 12.11 µmol, 1 eq), HOBt (817.91 µg, 6.05 µmol, 0.5 eq), and pyridine (4.79 mg, 4.90 µL, 60.53 µmol, 5 eq) were added to the reaction solution. After addition, the reaction solution allowed to react at 25°C for 2 hours, and the LCMS analysis showed that the reaction ended. The reaction solution was purified by preparative high-performance liquid chromatography (preparative chromatograph manufacturer: Orienda, model: BRIX-2860, column: Phenomenex Luna C18 250 × 30 mm × 10 µm, mobile phase: water (0.225% HCOOH)-acetonitrile, with an elution ratio of acetonitrile increased from 50% to 80%) and lyophilized, to afford the product LA-24-a (9.10 mg, 60.64% yield) as a yellow solid.

LCMS (ESI): m/z, 1261.7[M+Na]⁺. Rt = 2.909 min.

### Step 4: Preparation of compound LA-24-b

DBU (3.35 mg, 3.29 µL, 22.03 µmol, 3 eq) was added to the solution of compound LA-24-a (9.10 mg, 7.34 µmol, 1 eq) in DMF (2 mL) at 0°C, and then the reaction solution allowed to react at 25°C for half an hour. The LCMS showed that the reaction ended, TsOH (3.79 mg, 22.03 µmol, 3eq) was added to the reaction solution, and then the reaction solution was directly used for the next step.

LCMS (ESI): m/z, 1017.7[M+H]⁺. Rt = 1.629 min.

### Step 5: Preparation of compound LA-24

DIPEA (3.81 mg, 4.87 µL, 29.49 µmol, 4 eq) and HATU (3.36 mg, 8.85 µmol, 1.2 eq) were added to the solution of compounds LA-24-b (7.50 mg, 7.37 µmol, 1 eq) and LY-22CD-3-d (5.88 mg, 7.37 µmol, 1 eq) in DMF (2 mL) at 0°C. After addition, the reaction solution allowed to react at 25°C for half an hour, and the LCMS analysis showed that the reaction ended. The reaction solution was purified by preparative high-performance liquid chromatography (preparative chromatograph manufacturer: Orienda, model: BRIX-2860, column: Phenomenex Luna C18 250 × 30 mm × 10 µm, mobile phase: water (0.225% HCOOH)-acetonitrile, with an elution ratio of acetonitrile increased from 30% to 60%) and lyophilized, to afford compound LA-24 (3.55 mg, 26.81% yield, 100% purity) as a white solid.

LCMS (ESI): m/z, 1819.0[M+Na]⁺, 899.4[1/2M+1]⁺. Rt = 2.069 min.

### Example 21: Preparation of Compound LA-25A

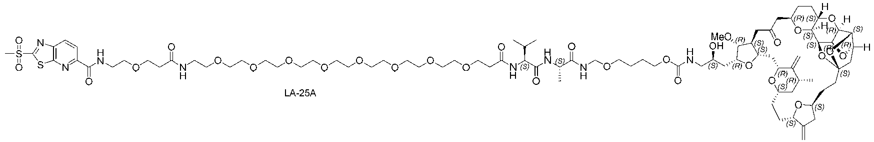

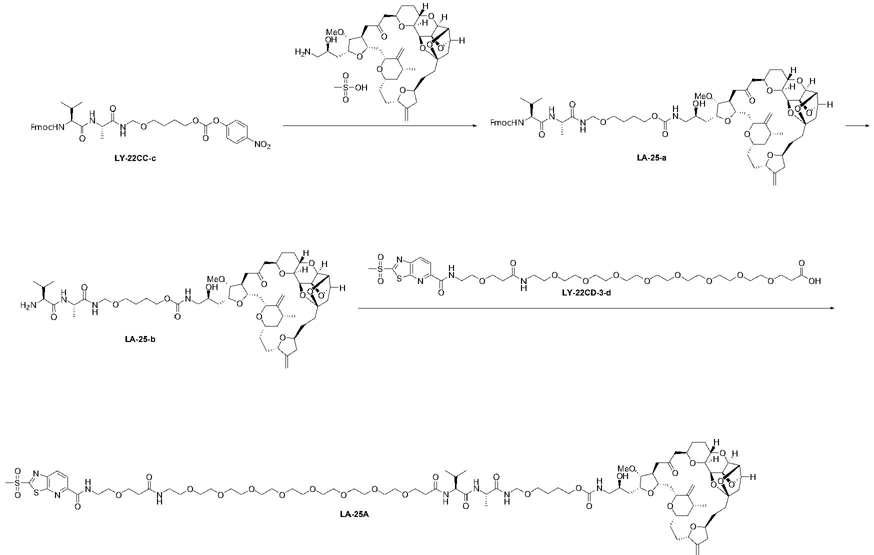

### Step 1: Preparation of compound LA-25-a

The compound LY-22CC-c (12 mg, 18 µmol, 1.5 eq), HOBT (0.8 mg, 6 µmol, 0.5 eq) and pyridine (4.90 µL, 60 µmol, 5 eq) was added to a solution of eribulin mesylate (10 mg, 12.11 µmol, 1 eq) and DIPEA (2 µL, 12 µmol, 1 eq) in DMAC (2 mL) at room temperature, and the reaction solution was stirred at 25°C for 12 hours. LCMS analysis showed that the reaction ended. The mixture was purified by preparative high-performance liquid chromatography (preparative chromatograph manufacturer: Orienda, model: BRIX-2860-R1, column: Phenomenex Luna C18 250 × 30 mm × 10 µm, mobile phase: water (0.225% HCOOH)-acetonitrile, with an elution ratio of acetonitrile increased from 50% to 80%), to afford LA-25-a (15 mg, 97.75% yield) as a white solid.

### Step 2: Preparation of compound LA-25-b

The compound LA-25-a (2.40g, 4.65 mmol, 1 eq) was dissolved in DMF (2 mL), DBU (15 mg, 12 µmol, 1 eq) was then added, and the reaction solution was stirred at 25°C for half an hour. LCMS analysis showed that the reaction ended. 15 mg of TsOH was added to the reaction solution to quench the reaction, and then the reaction solution was used directly for the next step (12.37 mg, a crude product in 2 mL of DMF).

### Step 3: Preparation of compound LA-25A

LY-22CD-3-d (9.43 mg, 11.83 µmol, 1 eq), N,N-diisopropylethylamine (7.82 µL, 47 µmol, 4 eq), and HATU (5.40 mg, 14.20 µmol, 1.2 eq) were added to a solution of compound LA-25-b (12.37 mg) in DMF (2 mL) at 0°C. The mixture was stirred at room temperature for half an hour, and the LCMS analysis indicated complete consumption of the starting material. The mixture was purified by preparative high-performance liquid chromatography (preparative chromatograph manufacturer: Orienda, model: BRIX-2860-R1, column: Welch Xtimate C18 150 × 30 mm × 10 µm, mobile phase: water (0.225% HCOOH)-acetonitrile, with an elution ratio of acetonitrile increased from 30% to 60%), to obtain LA-25A (2.50 mg, 11.58% yield) as a white solid.

LCMS (ESI): m/z, 865.4[M+H]⁺.

### Example 22: Preparation of Compound LA-25AA

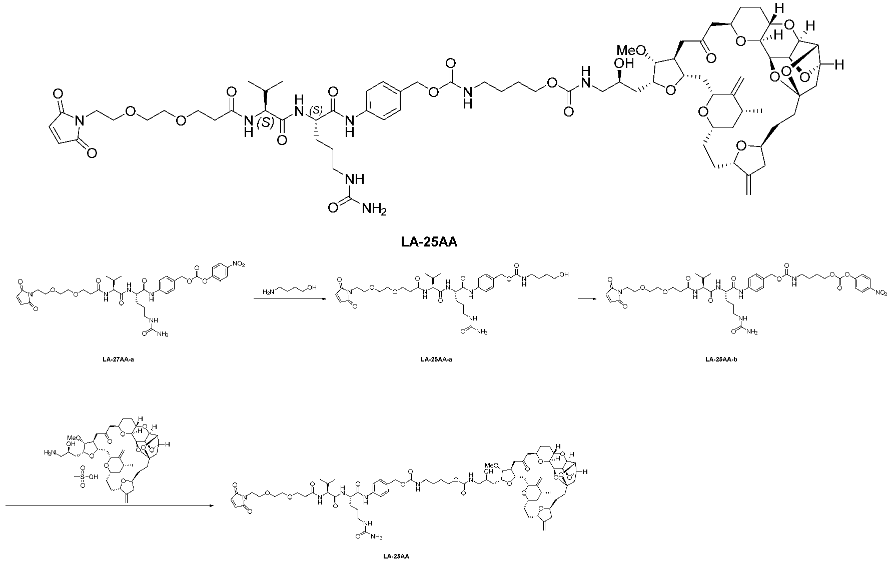

### Step 1: Preparation of compound LA-25AA-a

Compound DIPEA (16.49 mg, 127.59 µmol, 2 eq) was added to a solution of compound LA-27AA-a (50 mg, 63.79 µmol, 1 eq; for the synthesis of the compound, refer to the preparation of BA-1-c in Example 1) and 5-amino-1-butanol (5.69 mg, 63.79 µmol, 1 eq) in DMF (2 mL) at 0°C. The reaction solution was stirred at room temperature for 2 hours. LCMS analysis showed that the reaction ended. The mixture was purified by preparative high-performance liquid chromatography (preparative chromatograph manufacturer: Orienda, model: BRIX-2860-R1, column: Welch Xtimate C18 250 × 30 mm × 10 µm, mobile phase: water (0.225% HCOOH)-acetonitrile, with an elution ratio of acetonitrile increased from 10% to 40%), to afford LA-25AA-a (33 mg, 70.49% yield) as a white solid.

### Step 2: Preparation of compound LA-25AA-b

NPC (82.08 mg, 269.82 µmol, 6 eq) and DIPEA (34.87 mg, 269.82 µmol, 6 eq) were added to the solution of the compound LA-25AA-a (33 mg, 44.97 µmol, 1 eq) in DMF (2 mL), and the reaction solution was stirred at 25°C for 1 hour. LCMS analysis showed that the reaction ended. The reaction solution was evaporated to dryness under reduced pressure. The residue was purified by silica gel column chromatography (eluents: methanol/dichloromethane = 0:1 to 1:10) to afford the product LA-25AA-b (31 mg, 76.69% yield) as a white solid.

### Step 3: Preparation of compound LA-25AA

N,N-diisopropylethylamine (2.82 mg, 21.79 µmol, 3 eq) was added to a solution of compound LA-25AA-b (7.18 mg, 7.99 µmol, 1.1 eq) and eribulin mesylate (6 mg, 7.26 µmol, 1 eq) in DMF (2 mL) at 0°C. The mixture was stirred for 2 hours at room temperature, and the LCMS analysis indicated complete consumption of the starting material. The mixture was purified by preparative high-performance liquid chromatography (preparative chromatograph manufacturer: Orienda, model: BRIX-2860, column: Welch Xtimate C18 150 × 30mm × 10 µm, mobile phase: water (0.225% HCOOH)-acetonitrile, with an elution ratio of acetonitrile increased from 30% to 60%), to obtain LA-25AA (5.80 mg, 53.60% yield) as a white solid.

LCMS (ESI): m/z, 1512.8[M+Na]⁺.

### Example 23: Preparation of Compound LA-25B

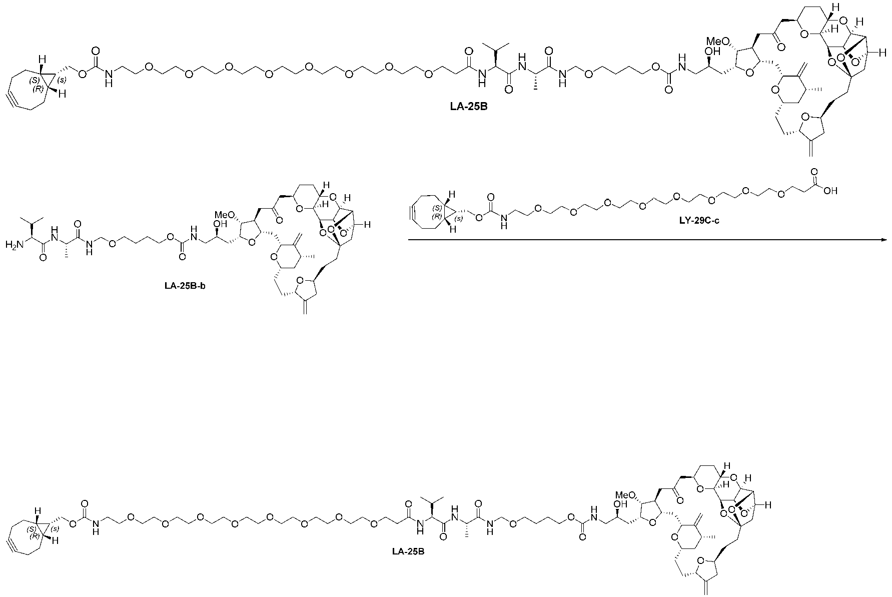

Compound LY-29C-c (4.73 mg, 7.65 µmol, 1 eq) was dissolved in DMF (500 µL) and the mixture was cooled to 0°C. Then compound LA-25B-b (8 mg, 7.65 µmol, 1 eq), HATU (2.91 mg, 7.65 µmol, 1 eq) and DIPEA (2.97 mg, 3.79 µL, 22.96 µmol, 3 eq) were added. The reaction solution was allowed to react at 25°C for 1 hour. LCMS analysis showed that the reaction ended. The reaction solution was purified by preparative high-performance liquid chromatography (preparative chromatograph manufacturer: Orienda, model: BRIX-2860, column: Phenomenex Luna C18 250 × 50 mm × 10 µm, mobile phase: water (0.225% HCOOH)-acetonitrile, with an elution ratio of acetonitrile increased from 50% to 80%), to obtain LA-25B (1.98 mg, 100% purity, 15.73% yield) as a white solid.

LCMS (ESI): m/z, 1667.2 [M+Na]⁺, Rt = 2.576 min.

### Example 24: Preparation of Compound LA-26

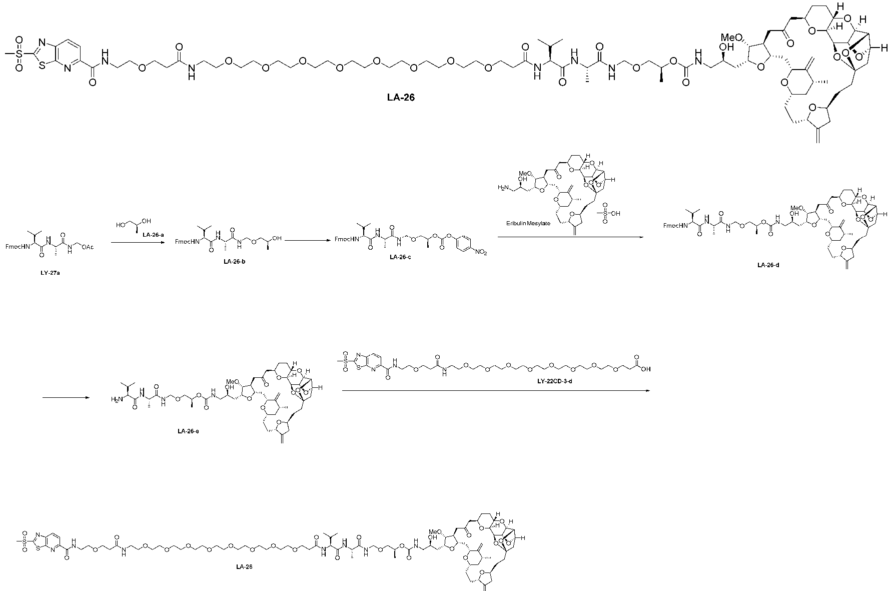

### Step 1: Preparation of compound LA-26-b

Compound LY-27a (250 mg, 519 µmol, 1 eq) was dissolved in THF (3 mL) and the mixture was cooled to 0°C. Then S-1,2-propanediol (118.5 mg, 1.56 mmol, 3 eq) and TsOH (89.4 mg, 519 µmol, 1 eq) were added. The reaction solution was allowed to react at 35°C for 3 hours. LCMS analysis showed that the reaction ended. The reaction solution was concentrated to afford 500 mg of white solid, and the white solid was then purified by preparative high-performance liquid chromatography (preparative chromatograph manufacturer: Orienda, model: BRIX-2860, column: Phenomenex Luna C18 250 × 50 mm × 10 µm, mobile phase: water (0.225% HCOOH)-acetonitrile, with an elution ratio of acetonitrile increased from 30% to 60%), to afford LA-26-b (120 mg, 46.45% yield) as a white solid.

LCMS (ESI): m/z, 520.4 [M+Na]⁺, Rt = 1.914 min.

### Step 2: Preparation of compound LA-26-c

Compound LA-26-b (99.5 mg, 200 µmol, 1 eq) was dissolved in DMF (2 mL), the mixture was cooled to 0°C, and then NPC (121.6 mg, 400 µmol, 2 eq) and DIPEA (77.5 mg, 99.1 µL, 600 µmol, 3 eq) were added. The reaction solution was allowed to react at 25°C for 2 hours. LCMS analysis showed that the reaction ended. The reaction solution was purified by preparative high-performance liquid chromatography (preparative chromatograph manufacturer: Orienda, model: BRIX-2860, column: Phenomenex Luna C18 250 × 50 mm × 10 µm, mobile phase: water (0.225% HCOOH)-acetonitrile, with an elution ratio of acetonitrile increased from 50% to 80%), to afford LA-26-c (16 mg, 12.1% yield) as a white solid.

LCMS (ESI): m/z, 685.3[M+Na]⁺, Rt = 3.889 min.

### Step 3: Preparation of compound LA-26-d

Compound LA-26-c (3.98 mg, 6 µmol, 1 eq) was dissolved in DMAC (500 µL), the mixture was cooled to 0°C, and then DIPEA (2.33 mg, 2.97 µL, 18 µmol, 3 eq) and eribulin mesylate (4.96 mg, 6 µmol, 1 eq) were added. The reaction solution was allowed to react at 25°C for 3 hours. LCMS analysis showed that the reaction ended. The reaction solution was purified by preparative high-performance liquid chromatography (preparative chromatograph manufacturer: Orienda, model: BRIX-2860, column: Phenomenex Luna C18 250 × 50 mm × 10 µm, mobile phase: water (0.225% HCOOH)-acetonitrile, with an elution ratio of acetonitrile increased from 50% to 80%), to afford LA-26-d (7 mg, 93.1% yield) as a white solid.

LCMS (ESI): m/z, 276.7[M+Na]⁺, Rt = 3.023 min.

### Step 4: Preparation of compound LA-26-e

Compound LA-26-d (7 mg, 5.58 µmol, 1 eq) was dissolved in DMF (500 µL), the mixture was cooled to 0°C, and then DBU (2.55 mg, 2.50 µL, 16.75 µmol, 3 eq) was added. The reaction solution was allowed to react at 25°C for 1 hour. LCMS analysis showed that the reaction ended. TsOH (2.88 mg, 16.75 µmol, 3 eq) was added to the reaction solution, and the mixture was allowed to react for 2 minutes. The reaction solution was directly used for the next step without further treatment.

LCMS (ESI): m/z, 1031.7[M+H]⁺, Rt = 1.705 min.

### Step 5: Preparation of compound LA-26

Compound LA-26-e (5 mg, 4.85 µmol, 1 eq) was dissolved in DMF (500 µL), the mixture was cooled to 0°C, and then LY-22CD-3-d (3.86 mg, 4.85 µmol, 1 eq), DIPEA (1.88 mg, 2.40 µL, 14.55 µmol, 3 eq) and HATU (1.84 mg, 4.85 µmol, 1 eq) were added. The reaction solution was allowed to react at 25°C for 1 hour. LCMS analysis showed that the reaction ended. The reaction solution was purified by preparative high-performance liquid chromatography (preparative chromatograph manufacturer: Orienda, model: BRIX-2860, column: Phenomenex Luna C18 250 × 50 mm × 10 µm, mobile phase: water (0.225% HCOOH)-acetonitrile, with an elution ratio of acetonitrile increased from 30% to 60%), to afford a white solid LA-26 (5.37 mg, 61.2% yield, 98.57% purity).

LCMS (ESI): m/z, 1832.1[M+Na]⁺, Rt = 2.142 min.

### Example 25: Preparation of Compound LA-26B

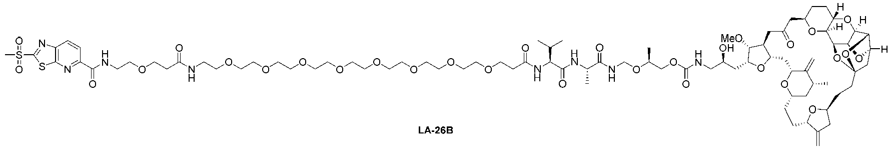

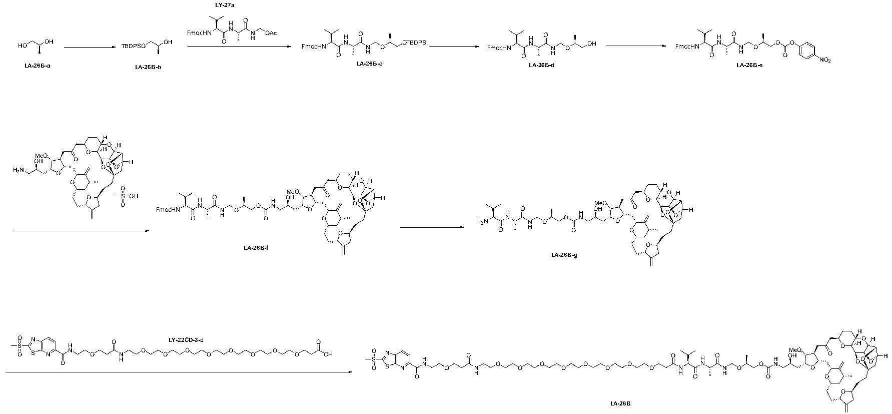

### Step 1: Preparation of compound LA-26B-b

1H-imidazole (1.07g, 976.07 µL, 15.77 mmol, 1.2 eq) and TBDPS-Cl (3.79g, 3.58 mL, 13.80 mmol, 1.05 eq) were added to a solution of compound S-1,2-propanediol (50 mg, 63.79 µmol, 1 eq) in THF (66 mL). The mixture was allowed to react at room temperature for 20 hours. The LCMS analysis indicated the reaction ended. The reaction solution was extracted with EA (30 mL × 3). The organic phases were combined, washed with H₂O (20 mL × 2), and dried over anhydrous sodium sulfate. The residue was purified by silica gel column chromatography (eluents: petroleum ether/ethyl acetate = 1:1) to afford (2S)-1-((tert-butyldiphenylsilyl)oxy)propan-2-ol (3.70g, 89.52% yield) as a colorless oil.

¹H NMR (400 MHz, Chloroform-d) δ 7.69 (dt, *J =* 8.0, 2.1 Hz, 4H), 7.49-7.35 (m, 6H), 3.96-3.93 (m, 1H), 3.64 (dd, *J =* 10.1, 3.5 Hz, 1H), 3.47 (dd, *J =* 10.1, 7.8 Hz, 1H), 1.12 (d, *J =* 6.3 Hz, 3H), 1.09 (s, 9H).

### Step 2: Preparation of compound LA-26B-c

TsOH (715.19 mg, 4.15 mmol, 1 eq) was added to a solution of (2S)-1-[(tert-butyldiphenyl silyl)oxy]propan-2-ol (2.61g, 8.31 mmol, 2 eq) and methyl [(2S)-2-[(2S)-2-({[(9H-fluoren-9-yl) methoxy]carbonyl}amino)-3-methylbutanamido]propanamido]acetate (2g, 4.15 mmol, 1 eq) in THF (30 mL). The mixture was allowed to react at 30°C for 2 hours. The LCMS analysis indicated the reaction ended. The reaction solution was purified by preparative high-performance liquid chromatography (preparative chromatograph manufacturer: ISCO, model: LC-20AP, column: Welch Xtimate C18 150 × 30 mm × 10 µm, mobile phase: water (0.225% HCOOH)-acetonitrile, with an elution ratio of acetonitrile increased from 65% to 95%), to afford compound LA-26B-c (233 mg, 7.62% yield) as a white solid. LCMS (ESI): m/z, 758[M+Na]⁺.

### Step 3: Preparation of compound LA-26B-d

Et₃N.3HF (2.43g, 2.46 mL, 15.07 mmol, 40 eq) was added to a solution of compound LA-26B-c (277.20 mg, 376.63 µmol, 1 eq) in THF (20 mL). The mixture was allowed to react at 25°C to 70°C for 2 hours. The LCMS analysis indicated the reaction ended. The residue was purified by silica gel column chromatography (C18, ISCO, R-120 g SepaFlash Silica Flash Column, eluents: 10%-40% water/CH₃CN, 100 mL/min) to afford compound LA-26B-d (130 mg, 69.37% yield, 100% purity) as a white solid. LCMS (ESI): m/z, 520[M+Na]⁺.

### Step 4: Preparation of compound LA-26B-e

Bis(4-nitrophenyl) carbonate (326.23 mg, 217.48 µL, 1.07 mmol, 4 eq) and DIPEA (207.89 mg, 265.84 µL, 1.61 mmol, 6 eq) were added to a solution of compound LA-26B-d (133.40 mg, 100%, 268.09 µmol, 1 eq) in DMF (2 mL). The mixture was allowed to react at 33°C for 1 hour. The LCMS analysis indicated the reaction ended. The residue was purified by silica gel column chromatography (PE/EA = 10:1 to 4:1, DCM/MeOH = 10:1) to afford compound LA-26B-e (197 mg, crude, 82% purity) as a white solid. LCMS (ESI): m/z 685 [M+Na]⁺.

### Step 5: Preparation of compound LA-26B-f

DIPEA (5.31 mg, 6.79 µL, 41.10 µmol, 3 eq) and HOBT (1.85 mg, 13.70 µmol, 1 eq) were added to the solution of LA-26B-e (12.18 mg, 82%, 15.07 µmol, 1.1 eq) and eribulin mesylate (10 mg, 13.70 µmol, 1 eq) in DMF (1 mL), and after a resulting mixture allowed to react for 2 hours at room temperature, the LCMS analysis indicated complete consumption of the starting material, and the reaction solution was purified by preparative high-performance liquid chromatography (preparative chromatograph manufacturer: ISCO, model: Lab311-DJ, column: Welch Xtimate C18 150 × 30 mm × 10 µm, mobile phase: water (0.225% HCOOH)-acetonitrile, with an elution ratio of acetonitrile increased from 50% to 80%), to afford the compound LA-26B-f (14 mg, 81.52% yield) as a white solid. LCMS (ESI): m/z, 1276[M+Na]⁺.

### Step 6: Preparation of compound LA-26B-g

DBU (5.10 mg, 5.01 µL, 33.51 µmol, 3 eq) was added to a solution of compound LA-26B-f (14 mg, 11.17 µmol, 1 eq) in DMF (1 mL). After the mixture was allowed to react at room temperature for 40 minutes, the LCMS analysis indicated complete consumption of the starting material. TsOH (5.77 mg, 33.51 µmol, 3 eq) was added. The reaction solution was used directly in the next step (a theoretical mass was 11.52 mg, and the yield was not calculated for the crude product). LCMS (ESI): m/z, 1053[M+Na]⁺.

### Step 7: Preparation of compound LA-26B

DIPEA (2.89 mg, 3.69 µL, 22.34 µmol, 2 eq) and HATU (5.10 mg, 13.41 µmol, 1.2 eq) were added to the solution of compound LA-26B-g (11.52 mg, 11.17 µmol, 1 eq) and compound LY-22CD-3-d (8.90 mg, 11.17 µmol, 1 eq) in DMF (1 mL) at 0°C. After addition, the reaction solution was allowed to react at room temperature for 1 hour, and the LCMS analysis indicated complete consumption of the starting material. The reaction solution was purified by preparative high-performance liquid chromatography (preparative chromatograph manufacturer: ISCO, model: BRIX-2860, column: Synergi Max-RP 200 × 30mm × 10 µm, mobile phase: water (0.225% HCOOH)-acetonitrile, with an elution ratio of acetonitrile increased from 30% to 60%) and lyophilized, to afford product LA-26B (4 mg, 18.79% yield, 95% purity) as a white solid. LCMS (ESI): m/z, 1832[M+Na]⁺.

### Example 26: Preparation of Compound LA-27A

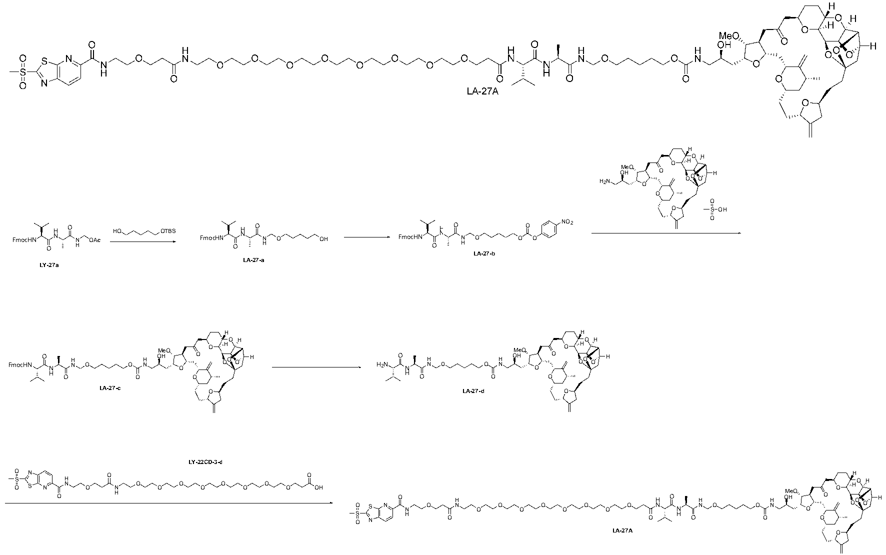

### Step 1: Preparation of compound LA-27-a

5-((tert-butyldimethylsilyl)oxy)pent-1-ol (181.42 mg, 830.65 µmol, 2 eq) and TsOH (21.46 mg, 20.05 µL, 124.60 µmol, 0.3 eq) were added to a solution of compound LY-27a (200 mg, 415.33 µmol, 1 eq) in THF (8 mL) at room temperature, and then the resulting mixture allowed to react at 33°C for 3 hours. After LCMS showed that the reaction ended, the reaction solution was concentrated to obtain a crude product, and the crude product was prepared by high-performance liquid chromatography (preparative chromatograph manufacturer: Shimadzu, model: LC-20AP, column: Phenomenex Luna C18 150 × 25 mm × 10 µm, mobile phase: water (0.225% FA)-acetonitrile, with an elution ratio of acetonitrile increased from 30% to 60%) and lyophilized, to obtain a yellow solid compound LA-27-a (102 mg, 46.72% yield).

LCMS (ESI): m/z, 548.4[M+Na]⁺. Rt = 2.009 min.

### Step 2: Preparation of compound LA-27-b

Bis(p-nitrobenzene)carbonate (118.06 mg, 388.09 µmol, 2 eq) and DIPEA (75.24 mg, 96.21 µL, 582.14 µmol, 3 eq) were added to the solution of compound LA-27-a (102 mg, 194.05 µmol, 1 eq) in DMF (5 mL) at 0°C, and then the reaction solution allowed to react at 33°C to 35°C for 3 hours. After LCMS showed that the reaction ended, the reaction solution was added into 50 mL of ice water and extracted with ethyl acetate (40 mL × 3), the organic phases were combined, washed with water (60 mL × 2) and brine (50 mL) in sequence, dried with anhydrous sodium sulfate, and filtered, the filtrate was concentrated to obtain a crude product, and the crude product was purified by silica gel column chromatography (eluents: methanol/dichloromethane = 1:20-1:10) to obtain a yellow solid compound LA-27-b (95 mg, yield of the crude product: 56.70%, purity: 80%).

LCMS (ESI): m/z, 713.4[M+Na]⁺. Rt = 2.783 min.

### Step 3: Preparation of compound LA-27-c

DIPEA (1.56 mg, 2 µL, 12.11 µmol, 1 eq) was added to a solution of the compound Eribulin mesylate (10 mg, 12.11 µmol, 1 eq) in DMAC (2 mL) at 0°C, then a resulting mixture was stirred at 25°C for 10 minutes, and then LA-27-b (10.45 mg, 80%, 12.11 µmol, 1 eq), HOBt (817.91 µg, 6.05 µmol, 0.5 eq), and pyridine (4.79 mg, 4.90 µL, 60.53 µmol, 5 eq) were added to the reaction solution. After addition, the reaction solution was allowed to react at 25°C for 16 hours, and the LCMS analysis showed that the reaction ended. The reaction solution was purified by preparative high-performance liquid chromatography (preparative chromatograph manufacturer: Orienda, model: BRIX-2860, column: Welch Xtimate C18 250 × 30 mm × 10 µm, mobile phase: water (0.225% HCOOH)-acetonitrile, with an elution ratio of acetonitrile increased from 50% to 80%) and lyophilized, to obtain a yellow solid compound LA-27-c (11 mg, 70.90% yield).

LCMS (ESI): m/z, 1303.8[M+Na]⁺. Rt = 3.103 min.

### Step 4: Preparation of compound LA-27-d

DBU (3.92 mg, 3.85 µL, 25.75 µmol, 3 eq) was added to the solution of compound LA-27-c (11 mg, 8.58 µmol, 1 eq) in DMF (2 mL) at 0°C, and then the reaction solution allowed to react at 25°C for half an hour. The LCMS showed that the reaction ended, TsOH (4.43 mg, 25.75 µmol, 3 eq) was added to the reaction solution, and then the reaction solution was directly used for the next step.

LCMS (ESI): m/z, 1059.8[M+H]⁺. Rt = 1.700 min.

### Step 5: Preparation of compound LA-27A

DIPEA (4.39 mg, 5.62 µL, 33.98 µmol, 4 eq) and HATU (3.88 mg, 10.20 µmol, 1.2 eq) were added to the solution of compounds LA-27-d (9 mg, 8.50 µmol, 1 eq) and LY-22CD-3-d (8.12 mg, 10.20 µmol, 1.2 eq) in DMF (2 mL) at 0°C. After addition, the reaction solution was allowed to react at 25°C for 20 minutes, and the LCMS analysis showed that the reaction ended. The reaction solution was purified by preparative high-performance liquid chromatography (preparative chromatograph manufacturer: Orienda, model: BRIX-2860, column: Welch Xtimate C18 150 × 30 mm × 10 µm, mobile phase: water (0.225% HCOOH)-acetonitrile, with an elution ratio of acetonitrile increased from 30% to 60%) and lyophilized, to afford compound LA-27A (8.92 mg, 57.12% yield, 98.54% purity) as a white solid.

LCMS (ESI): m/z, 1861.1[M+Na]⁺. Rt = 2.237 min.

### Example 27: Preparation of Compound LA-27AA

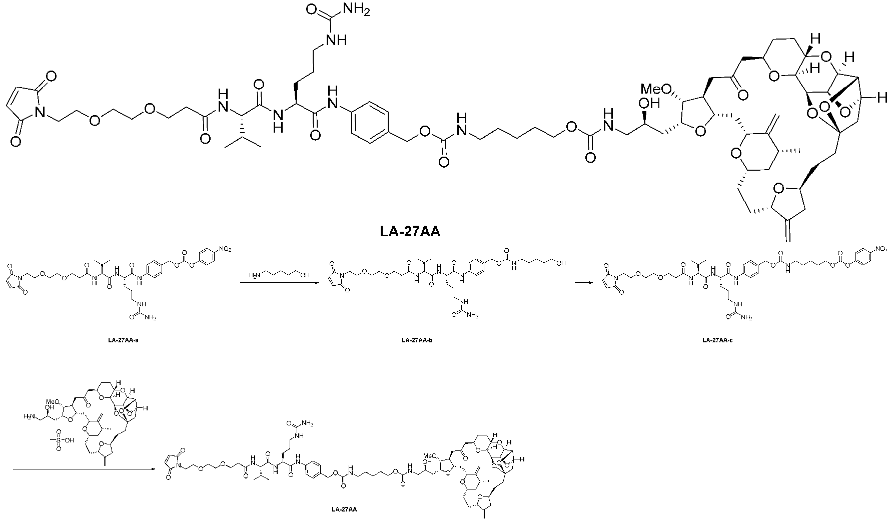

### Step 1: Preparation of compound LA-27AA-b

Compound DIPEA (8.24 mg, 63.79 µmol, 1 eq) was added to a solution of compound LA-27AA-a (50 mg, 63.79 µmol, 1 eq; for details, refer to the preparation of BA-1-c in Example 1) and 5-amino-1-pentanol (6.58 mg, 63.79 µmol, 1 eq) in DMF (2 mL) at 0°C. The reaction solution was stirred at room temperature for 1 hour. LCMS analysis showed that the reaction ended. The mixture was purified by preparative high-performance liquid chromatography (preparative chromatograph manufacturer: Orienda, model: BRIX-2860-R1, column: Welch Xtimate C18 250 × 30 mm × 10 µm, mobile phase: water (0.225% HCOOH)-acetonitrile, with an elution ratio of acetonitrile increased from 20% to 50%), to afford LA-27AA-b (28 mg, 58.69% yield) as a white solid.

### Step 2: Preparation of compound LA-27AA-c

NPC (68.34 mg, 224.64 µmol, 6 eq) and DIPEA (29.03 mg, 224.64 µmol, 6 eq) were added to the solution of the compound LA-27AA-b (28 mg, 37.44 µmol, 1 eq) in DMF (2 mL), and the reaction solution was stirred at 25°C for 1 hour. LCMS analysis showed that the reaction ended. The reaction solution was evaporated to dryness under reduced pressure. The residue was purified by silica gel column chromatography (eluents: methanol/dichloromethane = 0:1 to 1:10) to afford the product LA-27AA-c (21 mg, 61.44% yield) as a white solid.

### Step 3: Preparation of compound LA-27AA

N,N-diisopropylethylamine (2.82 mg, 21.79 µmol, 3 eq) was added to a solution of compound LA-27AA-c(7.29 mg, 7.99 µmol, 1.1 eq) and eribulin mesylate (6 mg, 7.26 µmol, 1 eq) in DMF (2 mL) at 0°C. The mixture was stirred for 2 hours at room temperature, and the LCMS analysis indicated complete consumption of the starting material. The mixture was purified by preparative high-performance liquid chromatography (preparative chromatograph manufacturer: Orienda, model: BRIX-2860, column: Welch Xtimate C18 150 × 30 mm × 10 µm, mobile phase: water (0.225% HCOOH)-acetonitrile, with an elution ratio of acetonitrile increased from 30% to 60%), to obtain LA-27AA (3.20 mg, 28.37% yield) as a white solid.

LCMS (ESI): m/z, 1526.9[M+Na]⁺.

### Example 28: Preparation of Compound LA-27AA-2

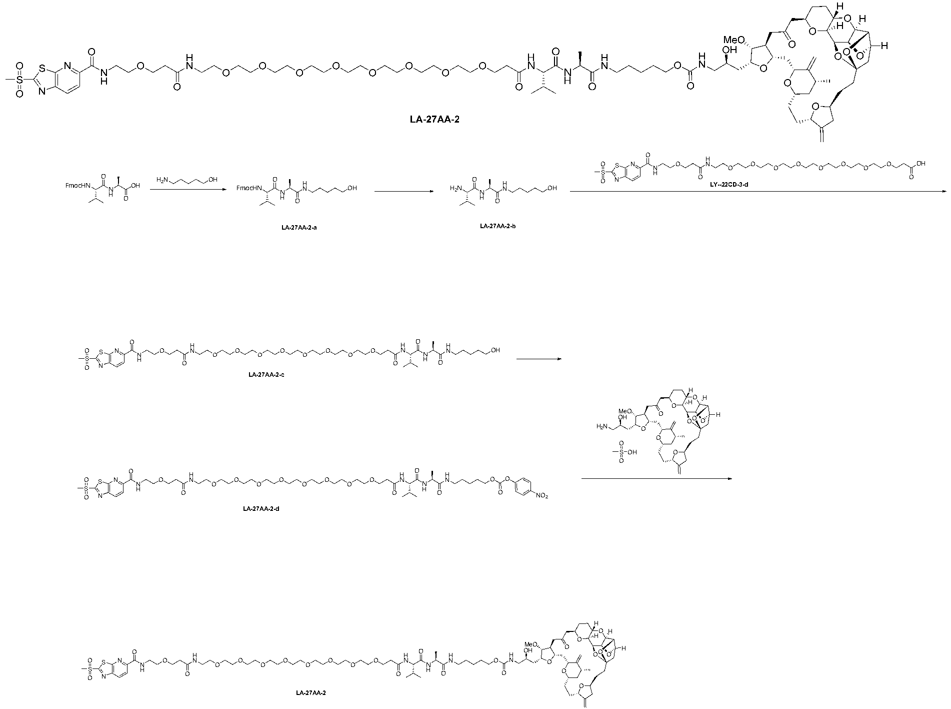

### Step 1: Preparation of compound LA-27AA-2-a

DIPEA (157.43 mg, 1.22 mmol, 2.5 eq) and HATU (222.33 mg, 584.71 µmol, 1.2 eq) were added to a solution of compound 5-amino-1-pentanol (55.29 mg, 535.98 µmol, 1.1 eq) and N-[fluorenylmethyloxycarbonyl]-L-valyl-L-alanine (200 mg, 487.26 µmol, 1 eq) in DMF (8 mL), the resulting mixture was stirred at room temperature for 2 hours, and the LCMS analysis indicated complete consumption of the starting material. The residue was purified by silica gel column chromatography (C18, ISCO, R-330g SepaFlash Silica Flash Column, eluents: 10%-50% water/CH3CN, 100 mL/min) to afford LA-27AA-2-a (227.4 mg, 94.16% yield) as a white solid.

LCMS (ESI): m/z, 496[M+H]⁺, Rt=2.049 min.

### Step 2: Preparation of compound LA-27AA-2-b

DBU (92.15 mg, 605.30 µmol, 3 eq) was added to the solution of LA-27AA-2-a (100 mg, 201.77 µmol, 1 eq) in DMF (5 mL) under an ice bath, the reaction solution was stirred at room temperature for 1 hour, LCMS analysis indicated complete consumption of the starting material, then p-toluenesulfonic acid (104.22 mg, 604 µmol, 3 eq) was added under an ice bath and a resulting mixture was directly used for the next step. A theoretical mass of LA-27AA-2-b was 55.16 mg.

LCMS (ESI): m/z, 274[M+H]⁺, Rt=0.198 min.

### Step 3: Preparation of compound LA-27AA-2-c

DIPEA (26.57 mg, 205.58 µmol, 4 eq) and HATU (23.45 mg, 61.67 µmol, 1.2 eq) were added to the solution of compounds LA-27AA-2-b (45.05 mg, 56.53 µmol, 1.1 eq) and LY-22CD-3-d (14.05 mg, 51.39 µmol, 1 eq) in DMF (1 mL) under the ice bath, the reaction solution was stirred for 1 hour at room temperature, and the LCMS analysis showed complete consumption of the starting material. The reaction solution was purified by preparative high-performance liquid chromatography (preparative chromatograph manufacturer: Orienda, model: BRIX-2860, column: Welch Xtimate C18 150 × 30 mm × 10 µm, mobile phase: water (0.225% HCOOH)-acetonitrile, with an elution ratio of acetonitrile increased from 10%-40%), to afford LA-27AA-2-c (21.30 mg, 39.39% yield) as a yellow oil.

LCMS (ESI): m/z, 1074[M+Na]⁺, Rt=1.241 min.

### Step 4: Preparation of compound LA-27AA-2-d

DIPEA (20.93 mg, 161.94 µmol, 8 eq) and NPC (36.95 mg, 121.45 µmol, 6 eq) were added to the solution of compound LA-27AA-2-c (21.30 mg, 20.24 µmol, 1 eq) in DMF (2 mL), the reaction solution was stirred at 35°C for 1.5 hours, the solution was concentrated by an oil pump, and the residue was purified by silica gel column chromatography (eluents: ethyl acetate/petroleum ether = 0%-50%, methanol/dichloromethane = 0%-8%), to afford LA-27AA-2-d (12.30 mg, 50% purity) as a yellow oil.

LCMS (ESI): m/z, 1217[M+H]⁺, Rt=1.937 min.

### Step 5: Preparation of compound LA-27AA-2

DIPEA (2.61 mg, 20.21 µmol, 4 eq) was added to the solution of compound LA-27AA-2-d (12.30 mg, 50% purity, 5.05 µmol, 1 eq) and eribulin mesylate (4.17 mg, 5.05 µmol, 1 eq) in DMF (1 mL), the reaction solution was stirred at room temperature for 1 hour, and the LCMS analysis indicated complete consumption of the starting material. The reaction solution was purified by preparative high-performance liquid chromatography (preparative chromatograph manufacturer: Orienda, model: BRIX-2860, column: Welch Xtimate C18 150 × 30 mm × 10 µm, mobile phase: water (0.225% HCOOH)-acetonitrile, with an elution ratio of acetonitrile increased from 30%-60%), to afford LA-27AA-2 (700 µg, 7.66% yield) as a white solid.

LCMS (ESI): m/z, 1831[M+Na]⁺. Rt = 2.18 min.

### Example 29: Preparation of Compound LA-27AA-3

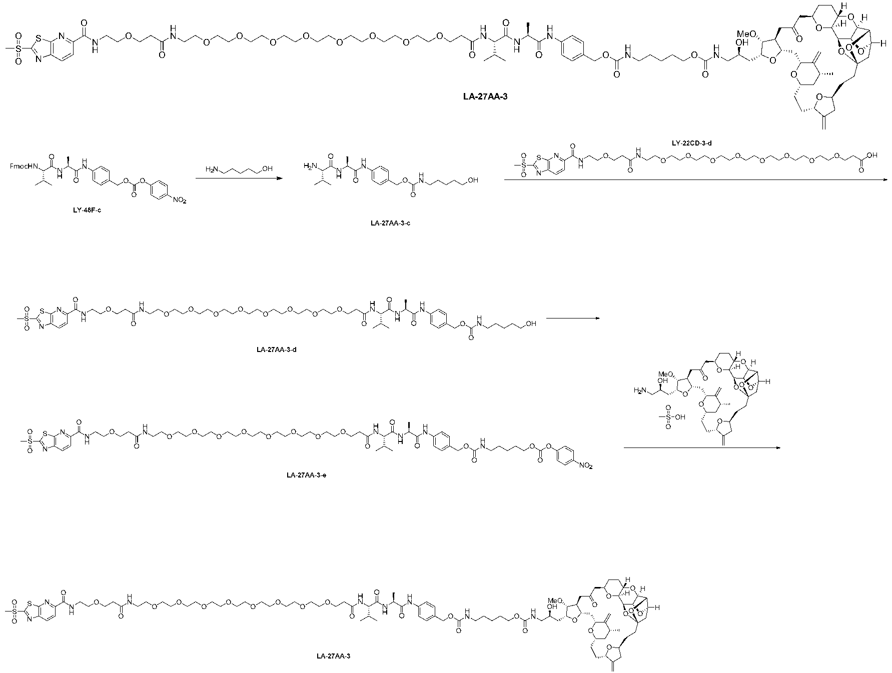

### Step 1: Preparation of compound LA-27AA-3-c

5-amino-1-pentanol (31.64 mg, 32.06 µL, 306.74 µmol, 1.2 eq) and DIPEA (66.07 mg, 84.49 µL, 511.24 µmol, 2 eq) were added to a solution of compound LY-46F-c (200 mg, 87%, 255.62 µmol, 1 eq) in DMF (4 mL), and the reaction solution was stirred at room temperature for 2 hours. The LCMS analysis indicated complete consumption of the starting material. The reaction solution was purified by preparative high-performance liquid chromatography to afford LA-27AA-3-c (60 mg, 48.33% yield) as a white solid.

Conditions are as follows: Preparative chromatograph manufacturer: Shimadzu, model: LC-20AP, column: YMC-Triart Prep C18 250 × 50 mm × 7 µm, mobile phase: water (0.225% HCOOH)-acetonitrile, with an elution ratio of acetonitrile increased from 10% to 40%.

LCMS (ESI): m/z, 445.3 [M+Na]⁺.

### Step 2: Preparation of compound LA-27AA-3-d

LY-22CD-3-d (75.44 mg, 94.67 µmol, 1 eq) and DIPEA (24.47 mg, 31.29 µL, 189.34 µmol, 2 eq) were added to the solution of compound LA-27AA-3-c (40 mg, 94.67 µmol, 1 eq) in DMF (2 mL), HATU (54 mg, 142 µmol, 1.5 eq) was added at 0°C, and the reaction solution was stirred at room temperature for 3 hours. The LCMS analysis indicated complete consumption of the starting material. The reaction solution was purified by preparative high-performance liquid chromatography to afford product LA-27AA-3-d (40 mg, 35.17% yield) as a yellow solid.

Conditions are as follows: Preparative chromatograph manufacturer: Shimadzu, model: LC-20AP, column: Phenomenex Luna C18 150 × 25mm × 10 µm, mobile phase: water (0.225% HCOOH)-acetonitrile, with an elution ratio of acetonitrile increased from 20% to 50%.

LCMS (ESI): m/z, 1223.7[M+Na]⁺.

### Step 3: Preparation of compound LA-27AA-3-e

NPC (25.32 mg, 83.24 µmol, 5 eq) and DIPEA (10.76 mg, 13.76 µL, 83.24 µmol, 5 eq) were added to the solution of compound LA-27AA-3-d (20 mg, 16.65 µmol, 1 eq) in DMF (1 mL), the reaction solution was stirred at room temperature for 6 hours, and the LCMS analysis indicated complete consumption of the starting material. The reaction solution was evaporated to dryness. The residue was purified by silica gel column chromatography (eluents: ethyl acetate/hexane = 1:20 to 1: 5) and preparative high-performance liquid chromatography (PE:EA=1:1 and DCM: MeOH=10:1, R_{f}=0.4) to afford LA-27AA-3-e (10 mg, 43.96% yield, 60% purity) as a yellow oil.

LCMS (ESI): m/z, 1366.5[M+H]⁺.

### Step 4: Preparation of compound LA-27AA-3

Eribulin mesylate (3.63 mg, 4.39 µmol, 1 eq) and DIPEA (1.13 mg, 1.45 µL, 8.78 µmol, 2 eq) were added to the solution of compound LA-27AA-3-d (10 mg, 60%, 4.39 µmol, 1 eq) in DMF (1 mL), and the reaction solution was stirred at 25°C for 3 hours. The LCMS analysis indicated complete consumption of the starting material. The reaction solution was subjected to preparative high-performance liquid chromatography to obtain a white solid product (1.81 mg, 21.06% yield, 90% purity).

The conditions of the preparative high-performance liquid chromatography were as follows:
preparative chromatograph manufacturer: Orienda, model: BRIX-2860 (R1, 4, 5, 6), column: Welch Xtimate C18 150 × 30 mm × 10 µm, mobile phase: water (0.225% HCOOH)-acetonitrile, with an elution ratio of acetonitrile increased from 20% to 50%.

LCMS (ESI): m/z, 978.9[(M+2H)/2]⁺.

### Example 30: Preparation of Compound LA-27A0

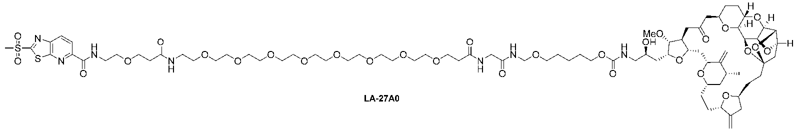

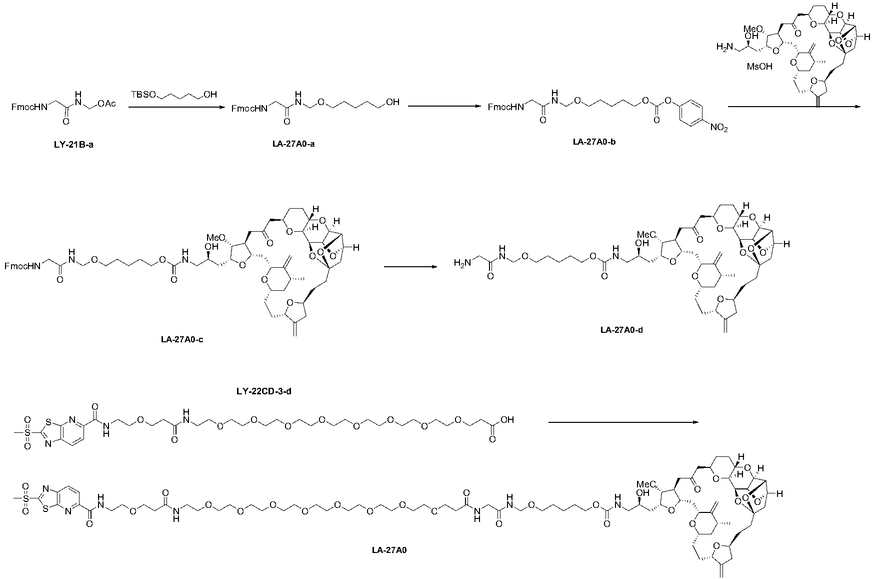

### Step 1: Preparation of compound (9H-fluoren-9-yl)methyl (2-((((5-hydroxypentyl)oxy) methyl)amino)-2-oxoethyl)carbamate

Methyl (2-((((9H-fluoren-9-yl)methoxy)carbonyl)amino)acetamido)acetate (300 mg, 814.38 µmol, 1 eq) was dissolved in THF (6 mL). 5-((tert-butyldimethylsilyl)oxy)pentan-1-ol (544.49 mg, 2.44 mmol, 3 eq) and TsOH (42.50 mg, 244.31 µmol, 0.3 eq) were added., and the reaction solution was stirred at 25°C for 1 hour. The LCMS showed that the product was generated. The reaction solution was subjected to preparative high-performance liquid chromatography to afford the product LA-27A0-a (200 mg, 59.54% yield) as a white solid.

The conditions of the preparative high-performance liquid chromatography were as follows:
preparative chromatograph manufacturer: Orienda, model: BRIX-2860, column: Synergi Max-RP 200 × 30 mm × 10 µm, mobile phase: water (0.225% HCOOH)-acetonitrile, with an elution ratio of acetonitrile increased from 20% to 50%.

LCMS (ESI): m/z, 435.3[M+Na]⁺.

### Step 2: Preparation of (9H-fluoren-9-yl)methyl (2-(((5-(((4-nitrophenoxy)carbonyl)oxy) pentyl)oxymethyl)amino)-2-oxoethyl)carbamate

(9H-fluoren-9-yl)methyl ((2-(((5-hydroxypentyl)oxy)methyl)amino)-2-oxoethyl)carbamate (200 mg, 484.86 µmol, 1 eq) was dissolved in DMF (5 mL), DIPEA (253.19 mg, 1.94 mmol, 4 eq) and NPC (304.13 mg, 969.73 µmol, 2 eq) were added, and the reaction solution was stirred at 25°C for 3 hours. TLC showed the disappearance of the starting material and formation of a new point with small polarity. The reaction solution was concentrated, and the residue was purified by silica gel column chromatography (DCM: MeOH = 10:1) to afford LA-27A0-b (260 mg, 92.84% yield) as a yellow oil.

LCMS (ESI): m/z, 578.2[M+H]⁺.

### Step 3: Preparation of compound LA-27A0-c

Eribulin mesylate was dissolved in DMF (2 mL). DIPEA (20.34 mg, 155.82 µmol, 3 eq) was added, followed by the compound (9H-fluoren-9-yl)methyl (2-(((5-(((4-nitrophenoxy)carbonyl) oxy)pentyl)oxymethyl)amino)-2-oxoethyl)carbamate (30 mg, 51.94 µmol, 1 eq). The reaction solution was stirred at 25°C for 1 hour. The LCMS showed that the product was generated. The reaction solution was subjected to preparative high-performance liquid chromatography to afford the product (200 mg, 84.36% yield) as a white solid.

The conditions of the preparative high-performance liquid chromatography were as follows:
preparative chromatograph manufacturer: Orienda, model: BRIX-2860, column: Synergi Max-RP 200 × 30 mm × 10 µm, mobile phase: water (0.225% HCOOH)-acetonitrile, with an elution ratio of acetonitrile increased from 50% to 80%.

LCMS (ESI): m/z, 1190.7[(M+Na]⁺.

### Step 4: Preparation of compound LA-27A0-d

Et₂NH (9.48 mg, 128.38 µmol, 5 eq) was added to the solution of compound LA-27A0-c (30 mg, 25.68 µmol, 1 eq) in DMF (3 mL) and the reaction solution was stirred at 25°C for 1 hour. The LCMS showed that the product was generated. The reaction solution was concentrated to afford the product (24 mg, 98.79% yield) as a yellow oil, which was used directly in the next step according to the theoretical amount.

LCMS (ESI): m/z, 946.6[(M+H)]⁺.

### Step 5: Preparation of compound LA-27A0

Compound LA-27A0-d (16 mg, 16.91 µmol, 1 eq) was dissolved in DMF (1 mL), LY-22CD-3-d (for synthesis of the compound, refer to patent application WO2024175069A1) (17.71 mg, 90%, 20 µmol, 1 eq) and DIPEA (4.42 mg, 33.82 µmol, 2 eq) were added, HATU (9.65 mg, 25.37 µmol, 1.5 eq) was added at 0°C, and the reaction solution was stirred at 0-10°C for 1 hour. The LCMS analysis showed the target product. The reaction solution was subjected to preparative high-performance liquid chromatography to obtain a white solid product (22.42 mg, 76.86% yield).

The conditions of the preparative high-performance liquid chromatography were as follows:
Preparative chromatograph manufacturer: Shimadzu, model: LC-20AP, column: Welch Xtimate C18 150 × 30 mm × 10 µm, mobile phase: water (0.225% HCOOH)-acetonitrile, with an elution ratio of acetonitrile increased from 30% to 60%.

LCMS (ESI) : m/z, 1724.8[(M+H]⁺.

¹H NMR (400 MHz, DMSO-*d6*) δ 9.06 (t, *J =* 5.7 Hz, 1H), 8.86 (d, *J =* 8.6 Hz, 1H), 8.51 (t, *J= 6.6* Hz, 1H), 8.38 (d, *J* = 8.6 Hz, 1H), 8.12 (t, *J =* 5.7 Hz, 1H), 7.90 (t, *J =* 5.6 Hz, 1H), 6.93 (d, *J* = 5.9 Hz, 1H), 5.05 (s, 1H), 4.99 (s, 1H), 4.82 (s, 1H), 4.75 (s, 1H), 4.63 (s, 1H), 4.58 - 4.46 (m, 4H), 4.25 (d, *J =* 10.8 Hz, 1H), 4.21 - 4.12 (m, 1H), 4.10 (s, 3H), 4.01 (t, *J =* 7.1 Hz, 2H), 3.89 (t, *J* = 6.6 Hz, 2H), 3.84 - 3.72 (m, 2H), 3.69 (d, J = 5.8 Hz, 2H), 3.65 (s, 3H), 3.63 - 3.59 (m, 3H), 3.53 (d, *J =* 5.2 Hz, 3H), 3.48 (d, *J =* 4.9 Hz, 31H), 3.37 (d, *J =* 5.9 Hz, 3H), 3.25 (s, 4H), 3.18 (q, *J =* 5.7 Hz, 2H), 2.93 (s, 2H), 2.83 (d, *J =* 9.5 Hz, 1H), 2.80 - 2.63 (m, 3H), 2.60 - 2.51 (m, 2H), 2.38 (t, *J* = 6.5 Hz, 2H), 2.36 - 2.29 (m, 4H), 2.25 (d, *J =* 11.9 Hz, 3H), 2.12 (t, *J =* 11.8 Hz, 1H), 2.05 - 1.83 (m, 7H), 1.75 - 1.59 (m, 5H), 1.48 (dq, *J =* 21.0, 6.8 Hz, 8H), 1.30 (q, *J =* 8.7, 8.1 Hz, 5H), 1.19 - 1.15 (m, 1H), 1.03 (d, *J =* 6.5 Hz, 3H), 0.99 - 0.92 (m, 1H).

### Example 31: Preparation of Compound LA-27AA-20

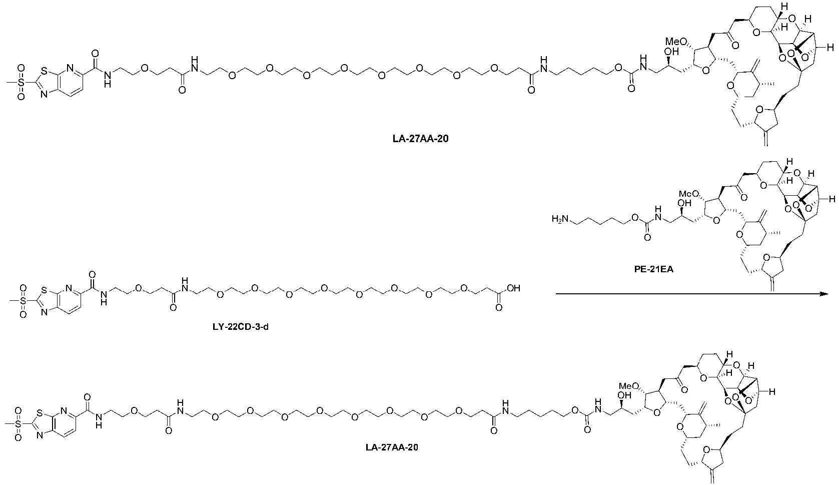

### Step 1: Preparation of compound LA-27AA-20

DIPEA (6.09 mg, 46.5 µmol, 3.6 eq) and HATU (14.77 mg, 38.83 µmol, 3 eq) were added to a solution of compound PE-21EA (11.12 mg, 12.94 µmol, 1 eq) (for the synthesis of the compound, refer to the Preparation Example of PE-21EA) and compound LY-22CD-3-d (11.46 mg, 90%, 12.94 µmol, 1 eq) (for the synthesis of the compound, refer to patent application WO2024175069A1) in DMF (1 mL) at 0°C. The reaction solution was stirred at 25°C for 30 minutes. The LCMS analysis showed that the product was generated. The reaction solution was filtered and the residue was then purified by preparative high-performance liquid chromatography (preparative chromatograph manufacturer: Orienda, model: BRIX-2860, column: Welch Xtimate C18 150 × 30 mm × 10 µm, mobile phase: water (0.225% FA)-acetonitrile, with an elution ratio of acetonitrile increased from 20% to 50%) and lyophilized, to afford compound LA-27AA-20 (3.25 mg, 5.33% yield, 100% purity) as a white solid.

LCMS (ESI): m/z, 1638.9[M+H]⁺.

¹H NMR (400 MHz, DMSO-*d*₆) δ 9.05 (t, *J =* 5.8 Hz, 1H), 8.87 (d, *J =* 8.6 Hz, 1H), 8.38 (d, *J* = 8.6 Hz, 1H), 7.88 (s, 1H), 7.77 (s, 1H), 6.90 (s, 1H), 5.05 (s, 1H), 4.99 (s, 1H), 4.83 (s, 1H), 4.75 (s, 1H), 4.63 (s, 1H), 4.55 (t, *J =* 4.2 Hz, 1H), 4.51 (d, *J =* 5.1 Hz, 1H), 4.26 (d, *J =* 10.6 Hz, 1H), 4.18 (d, *J =* 10.5 Hz, 1H), 4.12 - 4.08 (m, 3H), 4.02 (s, 1H), 3.89 (t, *J =* 6.7 Hz, 2H), 3.79 (dd, *J* = 20.8, 9.5 Hz, 2H), 3.65 (s, 3H), 3.62 (d, *J =* 6.4 Hz, 1H), 3.58 (d, *J* = 6.5 Hz, 1H), 3.56 - 3.53 (m, 3H), 3.49 (d, *J =* 1.5 Hz, 22H), 3.48 - 3.46 (m, 8H), 3.37 (t, *J =* 5.9 Hz, 2H), 3.26 (d, *J =* 4.7 Hz, 4H), 3.18 (d, *J =* 5.8 Hz, 2H), 3.01 (d, *J =* 6.3 Hz, 2H), 2.94 (s, 2H), 2.84 (d, *J =* 9.5 Hz, 1H), 2.72 (d, *J* = 11.6 Hz, 2H), 2.69 - 2.65 (m, 2H), 2.56 (d, *J =* 6.5 Hz, 1H), 2.35 - 2.29 (m, 6H), 2.29 - 2.24 (m, 4H), 2.20 - 2.12 (m, 2H), 2.04 - 1.95 (m, 2H), 1.92 (s, 4H), 1.73 - 1.62 (m, 5H), 1.51 (q, *J* = 6.9 Hz, 5H), 1.38 (q, *J =* 7.3 Hz, 3H), 1.28 (dd, *J =* 15.0, 8.0 Hz, 4H), 1.17 (d, *J =* 12.3 Hz, 1H), 1.03 (d, *J =* 6.4 Hz, 3H), 0.99 - 0.93 (m, 1H).

### Example 32: Preparation of Compound LA-27AAVC

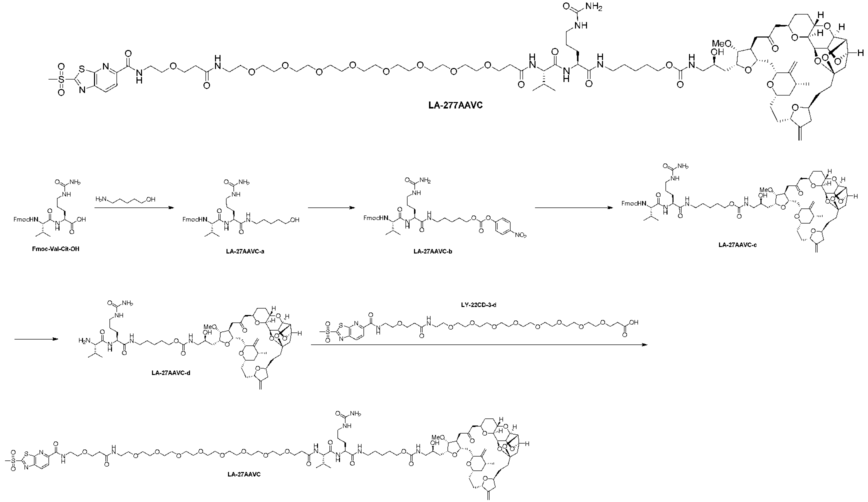

### Step 1: Preparation of compound LA-27AAVC-a

DIEA (2.996 mL, 18.125 mmol, 5 eq), 5-amino-1-pentanol (0.81g, 7.854 mmol, 1.3 eq), and HATU (2.99g, 7.854 mmol, 1.3 eq) were added to a solution of compound Fmoc-Val-Cit-OH (3g, 6.042 mmol, 1 eq) in DMF (10 mL) at room temperature. The mixture was then stirred at 25°C for 2 hours. LCMS analysis showed that the reaction ended. The reaction solution was concentrated under reduced pressure, to afford a crude product, and the crude product was triturated with ethyl acetate (30 mL) to afford LA-27AAVC-a (1.1g, 31.30% yield) as a yellow solid.

LCMS (ESI): m/z, 582.2[M+1]⁺, Rt=1.336 min.

### Step 2: Preparation of compound LA-27AAVC-b

DIEA (0.781 mL, 4.727 mmol, 2.5 eq) and methyl 4-nitrophenyl carbonate (1.73g, 5.673 mmol, 3 eq) were added to the solution of compound LA-27AAVC-a (1.1g, 1.891 mmol, 1 eq) in DMF (15 mL) at 25°C, the reaction solution was stirred at 25°C for 1 hour, and the LCMS analysis indicated complete consumption of the starting material. The reaction solution was purified by reverse-phase C18 chromatography (0.025% TFA, H₂O:ACN = 95%:5% to 46%:54%) to afford LA-27AAVC-b (770 mg, 54.52% yield) as a white solid.

LCMS (ESI): m/z, 746.6 [M]⁺, Rt=1.598 min.

### Step 3: Preparation of compound LA-27AAVC-c

DIEA (47 mg, 0.363 mmol, 3 eq), LEC-27AAVC-b (108.50 mg, 0.145 mmol, 1.2 eq), and HOBt (16.36 mg, 0.121 mmol, 1 eq) were added to the solution of the compound Eribulin mesylate (100 mg, 0.121 mmol, 1 eq) in DMF (3 mL) at room temperature. The mixture was then stirred at 25°C for 2 hours. LCMS analysis showed that the reaction ended. The reaction solution was purified by high-performance liquid chromatography (C18, 0.025% TFA, H₂O:ACN = 95%:5% to 18%:82%) to afford LA-27AAVC-c (67 mg, 41.37% yield) as a white solid.

LCMS (ESI): m/z, 1337.4[M]⁺, Rt=1.692 min.

### Step 4: Preparation of compound LA-27AAVC-d

Diethylamine (0.022 mL, 0.217 mmol, 10 eq) was added to the solution of compound LA-27AAVC-c (29 mg, 0.022 mmol, 1 eq) in DMF (1 mL) at 25°C, the reaction solution was stirred at 25°C for 1.5 hours, and the LCMS analysis indicated complete consumption of the starting material. The reaction solution was concentrated under low temperature in vacuo to afford crude LA-27AAVC-d (24 mg, crude) as a pale yellow oil.

LCMS (ESI): m/z, 1114.1[M-1]⁺, Rt=1.310 min.

### Step 5: Preparation of compound LA-27AAVC

DIEA (0.011 mL, 0.065 mmol, 3 eq), LY-22CD-3-d (for synthesis of the compound, refer to patent application WO2024175069A1) (18.86 mg, 0.024 mmol, 1.1 eq) and HATU (10.64 mg, 0.028 mmol, 1.3 eq) were added to the solution of the compound LA-27AAVC-d (24 mg, 0.022 mmol, 1 eq) in DMF (2 mL) at 0°C. The mixture was then stirred at 25°C for 1 hour. LCMS analysis showed that the reaction ended. The reaction solution was purified by high-performance liquid chromatography (Bonnasil-BS C18, 20 × 250 mm × 8 µm, H₂O (0.1%FA): ACN=70%:30% to 40%:60%) to afford LA-27AAVC (18.57 mg, 45.56% yield) as a white solid.

LCMS (ESI): m/z, 1894.3 [M]⁺, Rt=1.402 min.

¹H NMR (400 MHz, DMSO) δ 9.05 (t, *J =* 5.5 Hz, 1H), 8.89 - 8.85 (m, 1H), 8.38 (d, *J* = 8.6 Hz, 1H), 7.87 (dd, *J =* 13.5, 6.5 Hz, 2H), 7.78 (t, *J* = 5.2 Hz, 1H), 6.92 (t, *J =* 5.6 Hz, 1H), 5.96 (d, *J =* 14.9 Hz, 1H), 5.05 (s, 1H), 5.00 (s, 1H), 4.83 (s, 1H), 4.75 (s, 1H), 4.63 (s, 1H), 4.55 (t, *J* = 4.0 Hz, 1H), 4.26 (d, *J =* 10.8 Hz, 1H), 4.21 - 3.97 (m, 7H), 3.89 (t, *J =* 6.4 Hz, 2H), 3.79 (dd, *J* = 21.5, 9.5 Hz, 3H), 3.70 - 3.61 (m, 8H), 3.55 - 3.49 (m, 38H), 3.37 (d, *J =* 5.8 Hz, 5H), 3.25 (s, 3H), 3.23 - 3.15 (m, 3H), 3.02 (d, *J =* 6.0 Hz, 2H), 2.94 (d, *J =* 5.4 Hz, 4H), 2.84 (d, *J =* 9.5 Hz, 1H), 2.79 - 2.66 (m, 2H), 2.66 - 2.53 (m, 2H), 2.46 (dd, *J =* 13.6, 6.5 Hz, 1H), 2.35 (dt, *J* = 19.9, 6.4 Hz, 4H), 2.28 - 2.18 (m, 3H), 2.17 - 2.07 (m, 1H), 2.06 - 1.85 (m, 7H), 1.66 (dt, *J* = 22.6, 11.2 Hz, 5H), 1.48 (dd, *J =* 14.6, 8.1 Hz, 5H), 1.41 - 1.16 (m, 12H), 1.02 (t, *J =* 10.0 Hz, 3H), 0.82 (t, *J =* 7.6 Hz, 5H).

### Example 33: Preparation of Compound LA-27AAVCP

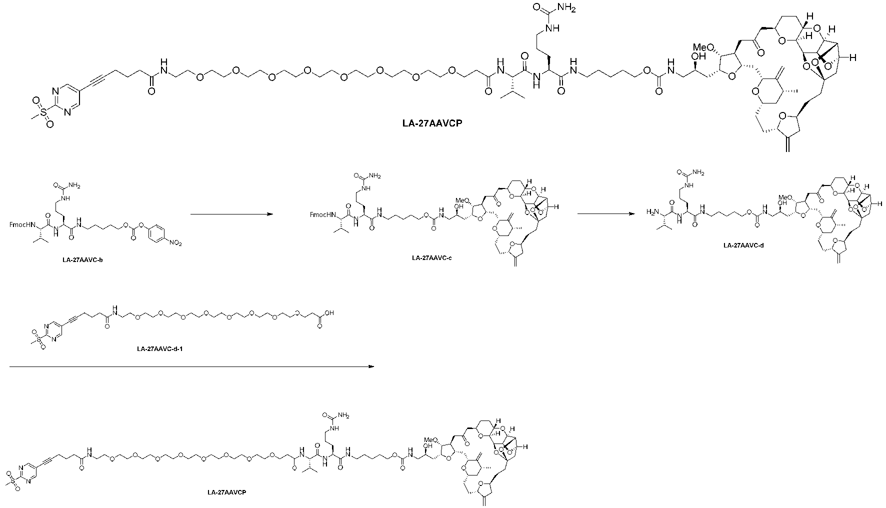

### Step 1: Preparation of compound LA-27AAVC-c

LA-27AAVC-b (for details, refer to the Example of LA-27AAVC) (45.56 mg, 0.061 mmol, 1.0 eq) and HOBt (8.18 mg, 0.061 mmol, 1.0 eq) were added to a solution of eribulin mesylate (purchased from a supplier) (50.0 mg, 0.061 mmol, 1.0 eq) and DIEA (23.47 mg, 0.182 mmol, 3.0 eq) in DMF (1.0 mL) at room temperature. The mixture was stirred at room temperature for 1 hour. LCMS analysis indicated the reaction ended. The reaction solution was filtered, and the filtrate was purified by C18 reverse-phase column chromatography by using a mobile phase of water (0.25% TFA)-acetonitrile, with the elution ratio of acetonitrile increased from 0% to 60%, to afford LA-27AAVC-c (61.0 mg, 75.35% yield) as a white solid.

LCMS (ESI): m/z, 1337.1[(M+H)]⁺.

### Step 2: Preparation of compound LA-27AAVC-d

DEA (32.81 mg, 0.449 mmol, 10.0 eq) was added to a solution of compound LA-27AAVC-c (60 mg, 0.045 mmol, 1.0 eq) in DMF (2.0 mL) at room temperature. The mixture was stirred at room temperature for 0.5 hours. The LCMS analysis indicated the reaction ended. The reaction solution was concentrated under reduced pressure to afford crude LA-27AAVC-d (60 mg, crude) as a brown oil.

LCMS (ESI): m/z, 1115.0 [(M+H)]⁺.

### Step 3: Preparation of compound LA-27AAVCP

HATU (25.67 mg, 0.067 mmol, 1.5 eq) was added to a solution of compounds LA-27AAVC-d (60 mg, crude, 1.0 eq) and LA-27AAVC-d-1 (for the synthesis of the compound, refer to patent application WO2024175069A1) (37.21 mg, 0.054 mmol, 1.2 eq) in DMF (2.0 mL) at room temperature. The mixture was stirred at room temperature for 1 hour. LCMS analysis indicated the reaction ended. The reaction solution was filtered, and the filtrate was purified by preparative high-performance liquid chromatography (model: JB-C235-04, column: Bonnasil-BS C18, 20×250 mm×8 µm, mobile phase: water (0.1% TFA)-acetonitrile, with the elution ratio of acetonitrile increased from 20% to 60%) to afford product LA-27AAVCP (55.9 mg, 69.70% yield) as a white solid.

LCMS (ESI): m/z, 895.6 [(M/2+H)]⁺.

¹HNMR(400 MHz, DMSO) δ 9.12 (s, 2H), 8.00 - 7.72 (m, 5H), 6.92 (s, 1H), 5.98 (s, 2H), 5.03 (d,J = 23.7 Hz, 2H), 4.80 (d,J = 30.9 Hz, 2H), 4.64 (s, 1H), 4.56 (t,J = 4.1 Hz, 1H), 4.27 (d,J = 10.2 Hz, 1H), 4.21 - 4.14 (m, 3H), 4.11 (s, 3H), 4.02 (s, 1H), 3.89 (t,J = 6.6 Hz, 2H), 3.79 (dd,J = 22.6, 10.1 Hz, 3H), 3.68 (s, 2H), 3.50 (d,J = 2.5 Hz, 28H), 3.26 (s, 3H), 3.21 (dd,J = 11.6, 5.8 Hz, 4H), 3.06 - 2.82 (m, 9H), 2.80 - 2.53 (m, 6H), 2.41 - 1.59 (m, 28H), 1.57 - 1.11 (m, 19H), 1.04 (d,J = 6.4 Hz, 3H), 0.86 - 0.79 (m, 6H).

### Example 34: Preparation of Compound LA-27BCN

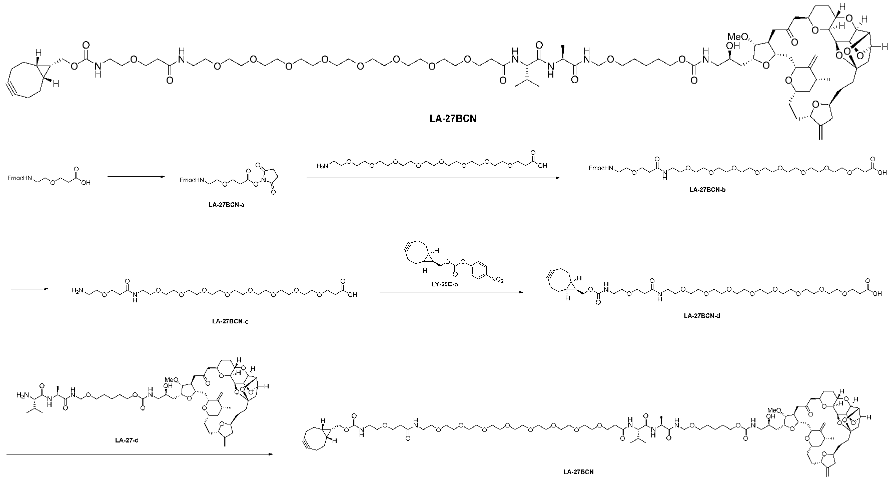

### Step 1: Preparation of compound LA-27BCN-a

N-hydroxysuccinimide (194.30 mg, 1.69 mmol, 1.2 eq) and N,N'-dicyclohexylcarbo diimide (580.56 mg, 716.74 µL, 2.81 mmol, 2 eq) were added to a solution of N-Fmoc-ethylene glycol-carboxylic acid (500 mg, 1.41 mmol, 1 eq) in dichloromethane (8 mL) under an ice bath, the reaction solution was stirred at room temperature for 2 hours, and the LCMS analysis indicated complete consumption of the starting material. After the insoluble material was filtered out, the filtrate was concentrated to afford crude LA-27BCN-a (1.19g, crude; the yield was not calculated for the crude product) as a colorless oil.

LCMS (ESI): m/z, 475 [M+Na]⁺, Rt=0.944 min.

### Step 2: Preparation of compound LA-27BCN-b

Amino-PEG(8)-acid (351.88 mg, 773.11 µmol, 1.1 eq) and DIPEA (232.31 µL, 1.41 mmol, 2 eq) were added to a solution of LA-27BCN-a (600 mg, 53%, 702.82 µmol, 1 eq) in DMF (5 mL) at 0°C. The mixture was stirred at room temperature for 1 hour. LCMS analysis indicated complete consumption of the starting material. The residue was purified by silica gel column chromatography (C18, ISCO, R-330g SepaFlash Silica Flash Column, eluents: 5%-25% water/CH₃CN, 100 mL/min) to afford LA-27BCN-b (456.80 mg, 83.45% yield) as a yellow oil.

LCMS (ESI): m/z, 779 [M+H]⁺, Rt=1.833 min.

### Step 3: Preparation of compound LA-27BCN-c

Et₂NH (305.13 µL, 2.93 mmol, 5 eq) was added to a solution of compound LA-27BCN-b (456.80 mg, 586.48 µmol, 1 eq) in DMF (8 mL). The mixture was stirred at room temperature for 1 hour. LCMS analysis indicated complete consumption of the starting material. The mixture was dried via an oil pump and used directly in the next step. A theoretical mass of LA-27BCN-c was 326.46 mg, and the yield was not calculated for the crude product.

LCMS (ESI): m/z, 557 [M+H]⁺, Rt=0.600.

### Step 4: Preparation of compound LA-27BCN-d

Compound LA-27BCN-c (163 mg, 292.82 µmol, 1 eq) and DIPEA (232.31 µL, 1.41 mmol, 2 eq) were added to a solution of compound LY-29C-b (101.57 mg, 322.11 µmol, 1.1 eq; for the synthesis of the compound, refer to the Preparation Example of LY-29C-c) in DMF (3 mL) at 0°C. The mixture was stirred at room temperature for 1 hour. LCMS analysis indicated complete consumption of the starting material (2 batches, same scale). The residue was purified by preparative high-performance liquid chromatography (preparative chromatograph manufacturer: Shimadzu, model: LC-20AP, column: Welch Xtimate C18 250 × 30 mm × 10 µm, mobile phase: water (0.225% HCOOH)-acetonitrile, with an elution ratio of acetonitrile increased from 20% to 50%), to afford LA-27BCN-d (232.2 mg, 30% yield, 70% purity) as a yellow oil.

LCMS (ESI): m/z, 733 [M+H]⁺, Rt=1.511.

### Step 5: Preparation of compound LA-27BCN

DIPEA (6.16 mg, 99%, 7.88 µL, 47.20 µmol, 5 eq) and HATU (4.31 mg, 11.33 µmol, 1.2 eq) were added to the solution of compounds LA-27BCN-d (8.99 mg, 12.27 µmol, 1.3 eq) and LA-27-d (8.99 mg, 12.27 µmol, 1.3 eq; for synthesis of the compound, refer to the Example of LA-27) in DMF (3 mL) at 0°C. After addition, the reaction solution was stirred at room temperature for 40 minutes, and the LCMS analysis indicated complete consumption of the starting material. The reaction solution was purified by preparative high-performance liquid chromatography (preparative chromatograph manufacturer: Orienda, model: BRIX-2860, column: Welch Xtimate C18 150 × 21.2 mm × 5 µm, mobile phase: water (0.225% HCOOH)-acetonitrile, with an elution ratio of acetonitrile increased from 30% to 60%), to obtain compound LA-27BCN (14.75 mg, 88.07% yield) as a white solid.

LCMS (ESI): m/z, 1797.1 [M+H]⁺, Rt=2.459 min.

¹H NMR (400 MHz, DMSO-*d*₆) δ 8.53 (t, *J* = 6.6 Hz, 1H), 8.04 (d, *J* = 7.2 Hz, 1H), 7.92-7.81 (m, 2H), 7.04 (s, 1H), 6.92 (s, 1H), 5.02 (d, *J* = 22.6 Hz, 2H), 4.79 (d, *J* = 31.5 Hz, 2H), 4.63 (p, *J=* 2.3 Hz, 1H), 4.58-4.43 (m, 4H), 4.30-4.13 (m, 4H), 4.12 - 3.96 (m, 5H), 3.89 (t, *J =* 6.6 Hz, 2H), 3.84-3.72 (m, 2H), 3.68 (s, 1H), 3.57 (dt, *J =* 8.5, 6.7 Hz, 4H), 3.50 (d, *J* = 3.9 Hz, 24H), 3.39 (t, *J* = 5.9 Hz, 2H), 3.36 (d, *J* = 6.0 Hz, 22H), 3.25 (s, 4H), 3.19 (q, *J* = 5.8 Hz, 2H), 3.09 (q, *J* = 5.9 Hz, 2H), 2.98-2.90 (m, 2H), 2.87-2.65 (m, 4H), 2.62-2.54 (m, 1H), 2.45-2.10 (m, 13H), 2.02-1.87 (m, 6H), 1.75-1.59 (m, 5H), 1.57-1.42 (m, 7H), 1.38-1.25 (m, 5H), 1.21 (d, *J* = 7.1 Hz, 3H), 1.03 (d, *J* = 6.4 Hz, 3H), 0.83 (dd, *J* = 13.4, 6.8 Hz, 6H).

### Example 35: Preparation of Compound LA-27C

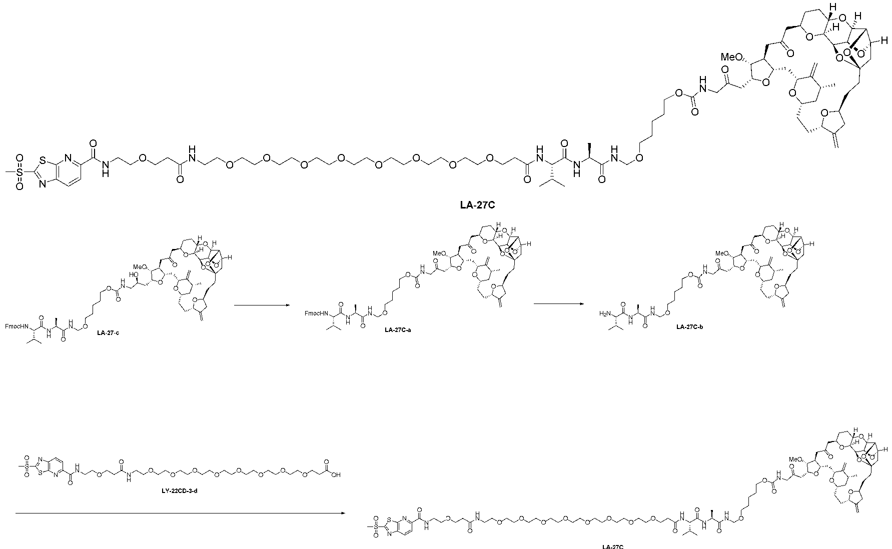

### Step 1: Preparation of compound LA-27C-a

Dess-Martin oxidant (21.48 mg, 98%, 15.77 µL, 49.63 µmol, 6 eq) was added to the solution of compound LA-27-c (for the synthesis of the compound, refer to the Example of LA-27) (10.60 mg, 8.27 µmol, 1 eq) in DCM (3 mL) at 0°C, then the resulting mixture was allowed to react at 25°C for 16 hours, and LCMS analysis showed that the reaction ended. The reaction solution was combined with the reaction solution from another small-scale batch and the mixture was added into a mixed solution of ice water, saturated sodium bicarbonate, and saturated sodium sulfite (1V/1V/1V, total volume: 60mL), the resulting mixture was stirred for 5 minutes, and extracted 3 times with DCM, 40 mL of DCM was used each time, the organic phases were combined, then washed with a mixed solution of saturated sodium bicarbonate and saturated sodium sulfite (1V/1V, total volume: 30 mL), dried with anhydrous sodium sulfate, and filtered, and the filtrate was concentrated to afford the compound LA-27C-a (14 mg, crude product, batch 1.28, the product from another small-scale trial at 0.28× scale was combined with this batch) as a yellow solid.

LCMS (ESI): m/z, 1279.9[M+H]⁺. Rt = 3.257 min.

### Step 2: Preparation of compound LA-27C-b

Diethylamine (4 mg, 54.71 µmol, 5 eq) was added to the solution of compound LA-27C-a (14 mg, 10.94 µmol, 1 eq) in DMF (1 mL) at 0°C, then the reaction solution allowed to react at 25°C for 1 hour, the LCMS showed that the reaction ended, and the reaction solution was directly evaporated to dryness to obtain the yellow solid compound LA-27C-b (12 mg, crude product).

LCMS (ESI): m/z, 1057.9[M+H]⁺.

### Step 3: Preparation of compound LA-27C

DIPEA (11.73 mg, 90.80 µmol, 8 eq) and HATU (8.63 mg, 22.70 µmol, 2 eq) were added to the solution of compounds LA-27C-b (12 mg, 11.35 µmol, 1 eq) and LY-22CD-3-d (18.09 mg, 22.70 µmol, 2 eq) in DMF (2 mL) at 0°C. After the addition, the reaction solution was allowed to react at 25°C for 2 hour. The LCMS analysis indicated the reaction ended. The reaction solution was purified by preparative high-performance liquid chromatography (preparative chromatograph manufacturer: Orienda, model: BRIX-2860, column: Welch Xtimate C18 150 × 30 mm × 10 µm, mobile phase: water (0.225% HCOOH)-acetonitrile, with an elution ratio of acetonitrile increased from 30% to 60%) and lyophilized, to afford compound LA-27C (5.12 mg, 24.57% yield, 98% purity) as a white solid.

LCMS (ESI): m/z, 1859.1[M+Na]⁺. Rt = 2.329 min.

¹H NMR (400 MHz, DMSO-*d*₆) δ 9.06 (t, *J* = 5.7 Hz, 1H), 8.87 (d, *J* = 8.6 Hz, 1H), 8.54 (t, *J =* 6.5 Hz, 1H), 8.38 (d, *J* = 8.6 Hz, 1H), 8.04 (d, *J* = 7.2 Hz, 1H), 7.92-7.83 (m, 2H), 7.28 (t, *J* = 5.9 Hz, 1H), 5.07-4.99 (m, 2H), 4.82 (s, 1H), 4.75 (s, 1H), 4.63 (dt, *J* = 4.5, 2.5 Hz, 1H), 4.59-4.37 (m, 4H), 4.30-4.15 (m, 4H), 4.12-3.99 (m, 5H), 3.92 (t, *J =* 6.6 Hz, 2H), 3.86-3.76 (m, 3H), 3.71 (s, 1H), 3.66-3.52 (m, 8H), 3.51-3.43 (m, 24H), 3.39-3.34 (m, 24H), 3.23 (s, 3H), 3.18 (q, *J =* 5.8 Hz, 3H), 2.87-2.71 (m, 4H), 2.67 (td, *J =* 4.0, 2.0 Hz, 2H), 2.57 (dd, *J =* 16.5, 9.7 Hz, 2H), 2.45-2.36 (m, 2H), 2.33-2.11 (m, 8H), 2.03-1.86 (m, 7H), 1.66 (p, *J* = 13.6, 12.7 Hz, 5H), 1.48 (dq, *J* = 21.9, 7.5 Hz, 6H), 1.37-1.17 (m, 9H), 1.00 (dd, *J* = 22.7, 9.1 Hz, 4H), 0.82 (dd, *J* = 13.5, 6.8 Hz, 6H).

### Example 36: Preparation of Compound LA-27CBCN

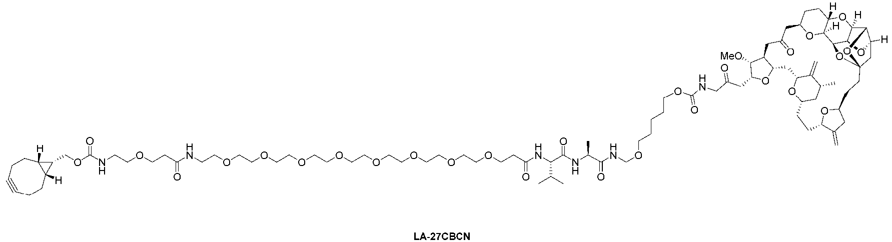

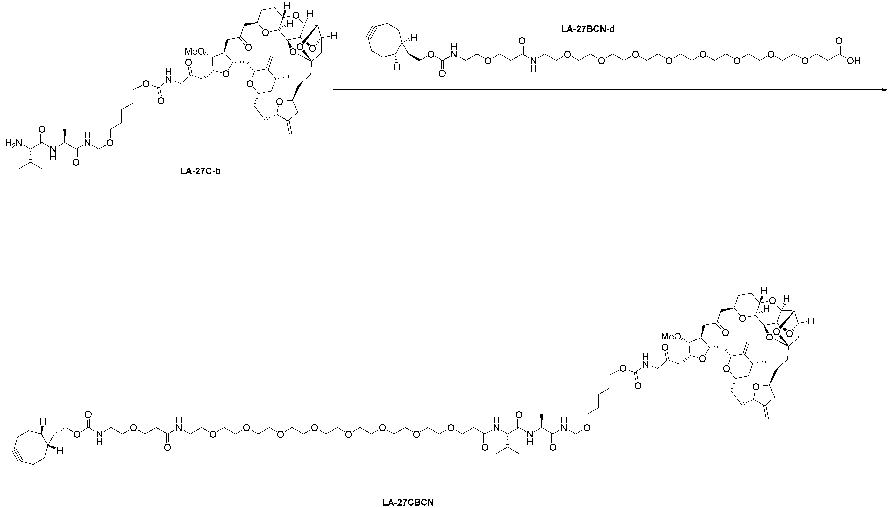

DIPEA (5.19 mg, 39.72 µmol, 4 eq) and HATU (4.53 mg, 11.92 µmol, 1.2 eq) were added to a solution of compound LA-27C-b (for the synthesis of this compound, refer to the Example of LA-27C; 10.50 mg, 9.93 µmol, 1 eq) and compound LA-27BCN-d (for the synthesis of the compound, refer to the Example of LA-27BCN; 14.56 mg, 60%, 11.92 µmol, 1.2 eq) in DMF (8 mL) under the ice bath. After the addition, the reaction solution was allowed to react at 25°C for 1 hour. The reaction solution was purified by preparative high-performance liquid chromatography (preparative chromatograph manufacturer: Orienda, model: BRIX-2860, column: Welch Xtimate C18 150 × 21.2 mm × 5 µm, mobile phase: water (0.225% HCOOH)-acetonitrile, with an elution ratio of acetonitrile increased from 30% to 60%), to afford compound LA-27CBCN (7.57 mg, 43.01% yield, 96% purity) as a white solid.

LCMS (ESI): m/z, 1795.1 [M+Na]⁺, Rt=2.603 min.

¹H NMR (400 MHz, DMSO-*d*₆) δ 8.53 (t, *J* = 7.0 Hz, 1H), 8.04 (d, *J* = 7.3 Hz, 1H), 7.92-7.82 (m, 2H), 7.28 (t, *J* = 5.9 Hz, 1H), 7.03 (d, *J* = 5.6 Hz, 1H), 5.04 (d, *J* = 11.0 Hz, 2H), 4.82 (s, 1H), 4.75 (s, 1H), 4.63 (q, *J =* 2.9 Hz, 1H), 4.58-4.44 (m, 3H), 4.29-4.07 (m, 7H), 4.03 (dd, *J* = 9.0, 4.7 Hz, 4H), 3.92 (t, *J* = 6.6 Hz, 2H), 3.87-3.64 (m, 5H), 3.63-3.54 (m, 4H), 3.54-3.43 (m, 24H), 3.39 (t, *J* = 5.9 Hz, 2H), 3.34 (d, *J* = 6.4 Hz, 6H), 3.25-3.13 (m, 5H), 3.09 (q, *J* = 6.0 Hz, 2H), 2.87-2.53 (m, 7H), 2.48-2.25 (m, 11H), 2.17 (dd, *J* = 27.8, 11.8 Hz, 8H), 1.94 (d, *J=* 13.0 Hz, 7H), 1.68 (q, *J =* 12.3 Hz, 4H), 1.50 (dt, *J =* 24.5, 7.1 Hz, 8H), 1.29 (dq, *J =* 16.3, 8.6 Hz, 6H), 1.21 (d, *J* = 7.1 Hz, 5H), 1.00 (dd, *J =* 22.6, 9.2 Hz, 4H), 0.83 (dd, *J =* 13.3, 6.8 Hz, 7H).

### Example 37: Preparation of compound LA-27C-2

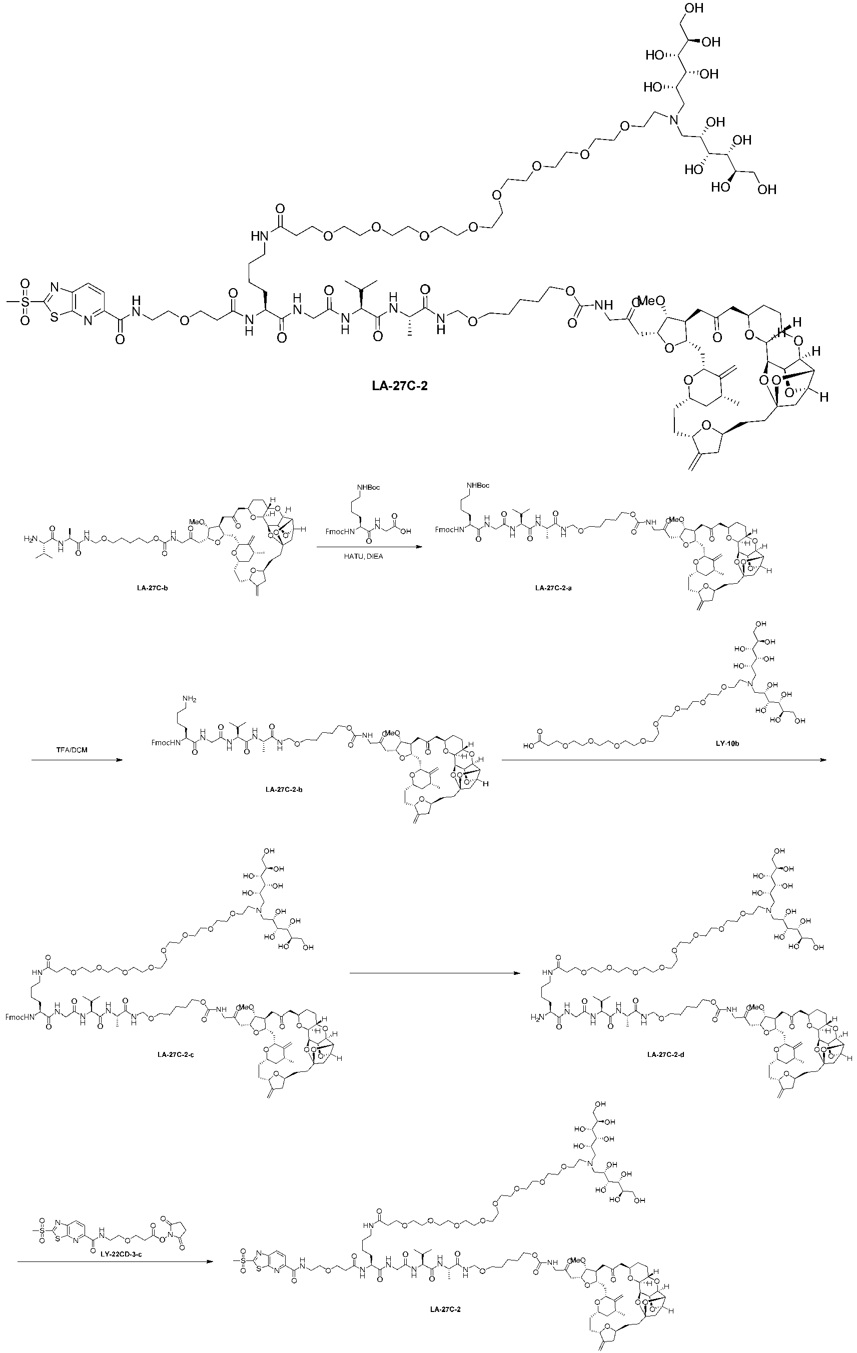

### Step 1: Preparation of compound LA-27C-2-a

The compound glycine (66.36 mg, 41.61 µL, 883.99 µmol, 1 eq) and NaHCO₃ (74.26 mg, 40.36 µL, 883.99 µmol, 1 eq) were dissolved in H₂O (5 mL), the mixture solution was stirred for 15 minutes at room temperature, and a mixture solution of compound 2,5-dioxopyrrolidin-1-ylN²-(((9H-fluoren-9-yl)methoxy)carbonyl)-N⁶-(tert-butoxycarbonyl)-L-lysine (500 mg, 883.99 µmol, 1 eq) in THF (5 mL) was added to the above mixture solution, the resulting solution was allowed to react for 2 hours at room temperature, and the LCMS analysis showed that the reaction ended. The resulting solution was evaporated to dryness, adjusted with 2N HCl to adjust pH to 2, and extracted with EA (20 mL × 3), the organic phases were combined, washed with water (20 mL × 3) and saturated brine (20 mL × 1), dried with anhydrous sodium sulfate, and evaporated to dryness under reduced pressure to afford the compound N²-(((9H-fluoren-9-yl)methoxy)carbonyl)-N⁶-(tert-butoxy carbonyl)-L-lysylglycine (427 mg, yield: 91.90%) as a yellow solid. LCMS (ESI): m/z, 548[M+ Na]⁺.

DIPEA (12.24 mg, 15.66 µL, 93.78 µmol, 3 eq) and HATU (14.26 mg, 37.51 µmol, 1.2 eq) were added to a solution of compound LA-27C-b (33.05 mg, 31.26 µmol, 1 eq) (for the synthesis of the compound, refer to the Preparation Example of LA-27C) and compound N²-(((9H-fluoren-9-yl)methoxy)carbonyl)-N⁶-(tert-butoxycarbonyl)-L-lysylglycine (18.67 mg, 88%, 31.26 µmol, 1 eq) in DMF (1 mL) at 0°C. After addition, the reaction solution was allowed to react at 20°C for 1 hour. The LCMS analysis showed that the reaction ended. After filtration, the reaction solution was purified by preparative high-performance liquid chromatography (preparative chromatograph manufacturer: Orienda, model: BRIX-2860, column: Welch Xtimate C18 150 × 30 mm × 10 µm, mobile phase: water (0.225% FA)-acetonitrile, with an elution ratio of acetonitrile increased from 50% to 80%), to afford the compound LA-27C-2-a (27.10 mg, 55.40% yield) as a white solid.

LCMS (ESI): m/z, 1587.8[M+Na]⁺. Rt = 3.278 min.

### Step 2: Preparation of compound LA-27C-2-b

TFA (0.5 mL, 6.66 mmol, 384.8 eq) was added to a solution of compound LA-27C-2-a (27.10 mg, 17.32 µmol, 1 eq) in DCM (3 mL) at 0°C. The mixture was allowed to react at 0°C for 30 minutes. The LCMS analysis showed that the reaction ended. The mixture was evaporated to dryness to afford the compound LA-27C-2-b (25.37 mg, where the yield was not calculated for the crude product) as a yellow oil, which was used directly in the next step.

LCMS (ESI): m/z, 1465.8[M+H]⁺. Rt = 2.421 min.

### Step 3: Preparation of compound LA-27C-2-c

D-glucose (502 mg, 2.79 mmol, 10 eq) and NaBH₃CN (294 mg, 1.39 mmol, 5 eq) were added to a solution of 1-amino-3,6,9,12,15,18,21,24-octaoxaheptacosan-27-oic acid (123 mg, 0.28 mmol, 1.0 eq) in methanol (10 mL)/acetic acid (1 mL), and the reaction solution was allowed to react at 40°C for 40 hours. The reaction mixture was concentrated to approximately 5 mL, 5 mL of methanol and 3 mL of water were added, and the reaction mixture was purified by preparative high-performance liquid chromatography (column: Phenomenex Luna C18 250× 50 mm× 10 µm; mobile phase: water (0.225% HCOOH)-acetonitrile, with an elution ratio increased from 45% to 85%) and lyophilized, to afford compound **LY-10b** (124 mg, 50.4% yield, TFA salt) as a colorless oil.

LCMS (ESI): m/z, 770.7[M+1]⁺.

¹H NMR (400 MHz, D₂O) δ 4 26 (m, 2H), 3.99-3.94 (m, 2H), 3.90-3.78 (m, 12H), 3.78-3.71 (m, 26H), 3.70-3.65 (m, 4H), 3.60-3.56 (m, 5H), 2.70 (t, *J =* 8.0 Hz, 2H).

DIPEA (9.04 mg, 11.57 µL, 69.28 µmol, 4 eq) and HATU (9.88 mg, 25.98 µmol, 1.5 eq) were added to the solution of compounds LA-27C-2-b (25.37 mg, 17.32 µmol, 1 eq) and LY-10b (20 mg, 25.98 µmol, 1.5 eq) in DMF (1 mL) at 0°C. After addition, the reaction solution was allowed to react at 20°C for 2 hours. LCMS analysis showed that the reaction ended. The reaction solution was filtered and the residue was then purified by preparative high-performance liquid chromatography (preparative chromatograph manufacturer: Orienda, model: BRIX-2860, column: YMC-Triart Prep C18 150 × 30 mm × 10 µm, mobile phase: water (0.1% TFA)-acetonitrile, with an elution ratio of acetonitrile increased from 30% to 60%) and lyophilized, to afford the compound LA-27C-2-c (20.40 mg, 53.14% yield) as a white solid.

LCMS (ESI): m/z, 1130.8[1/2M+Na]⁺. Rt = 2.263 min.

### Step 4: Preparation of compound LA-27C-2-d

Et₂NH (6.80 mg, 92.03 µmol, 10 eq) was added to a solution of compound LA-27C-2-c (20.40 mg, 9.20 µmol, 1 eq) in DMF (1 mL) at 0°C. After addition, the reaction solution was allowed to react at 0°C for 1 hour. The LCMS analysis showed that the reaction ended. The reaction solution was evaporated to dryness to afford the compound LA-27C-2-d (18.35 mg, where the yield was not calculated for the crude product) as a yellow oil, which was used directly in the next step.

LCMS (ESI): m/z, 998.5[1/2M+H]⁺, Rt=1.635min.

### Step 5: Preparation of compound LA-27C-2

A solution of LY-22CD-3-c (for the synthesis of the compound, refer to the Preparation Example of LY-22CD-3-d) (6.49 mg, 13.80 µmol, 1.5 eq) in DMF (0.5 mL) was added to a solution of compounds LA-27C-2-d (18.35 mg, 9.20 µmol, 1 eq) and DIPEA (3.60 mg, 27.60 µmol, 3 eq) in DMF (0.5 mL) at 0°C. After addition, the reaction solution was allowed to react at 20°C for 1 hour. LCMS analysis showed that the reaction ended. The reaction solution was filtered and the residue was then purified by preparative high-performance liquid chromatography (preparative chromatograph manufacturer: Orienda, model: BRIX-2860, column: YMC-Triart Prep C18 150 × 30 mm × 10 µm, mobile phase: water (0.1% TFA)-acetonitrile, with an elution ratio of acetonitrile increased from 30% to 60%) and lyophilized, to afford compound LA-27C-2 (8.28 mg, 38.30% yield, 100% purity) as a white solid.

LCMS (ESI): m/z, 1197.0[1/2M+Na]⁺. Rt = 1.980 min.

¹H NMR (400 MHz, DMSO-*d*₆) δ 9.10 (t, *J=* 5.7 Hz, 1H), 8.87 (d, *J =* 8.6 Hz, 1H), 8.52 (t, *J* = 6.6 Hz, 1H), 8.38 (d, *J* = 8.6 Hz, 1H), 8.17-8.08 (m, 3H), 8.04 (d, *J* = 7.6 Hz, 1H), 7.78 (d, *J* = 5.9 Hz, 1H), 7.72 (d, *J =* 8.7 Hz, 1H), 7.29 (t, *J =* 6.0 Hz, 1H), 6.19 (d, *J =* 3.0 Hz, 1H), 5.94 (d, *J* = 3.0 Hz, 1H), 5.42 (s, 2H), 4.94 (s, 1H), 4.86 (s, 1H), 4.80 (s, 1H), 4.75 (s, 1H), 4.55 (dd, *J =* 10.2, 6.7 Hz, 4H), 4.45 (dd, *J* = 10.2, 6.3 Hz, 2H), 4.33 (s, 1H), 4.29 (d, *J* = 9.6 Hz, 1H), 4.25-4.15 (m, 3H), 4.08 (d, *J =* 4.0 Hz, 2H), 4.00 (s, 3H), 3.92 (t, *J =* 6.6 Hz, 2H), 3.84 (d, *J =* 5.9 Hz, 2H), 3.77 (t, *J* = 5.0 Hz, 3H), 3.71 (t, *J =* 9.5 Hz, 5H), 3.65 (s, 4H), 3.62 (dd, *J =* 7.3, 2.5 Hz, 4H), 3.57 (d, *J =* 6.5 Hz, 5H), 3.55 (d, *J =* 2.3 Hz, 3H), 3.53 (s, 1H), 3.52 (s, 4H), 3.51 - 3.49 (m, 13H), 3.49 (s, 5H), 3.47-3.46 (m, 4H), 3.44 (d, *J* = 4.8 Hz, 4H), 3.41 (d, *J* = 5.2 Hz, 3H), 3.17 (s, 4H), 2.98 (d, *J* = 8.0 Hz, 3H), 2.81-2.73 (m, 2H), 2.71 - 2.66 (m, 3H), 2.59 (t, *J =* 8.5 Hz, 2H), 2.42 (s, 2H), 2.41-2.36 (m, 3H), 2.35-2.32 (m, 3H), 2.28 (t, *J =* 6.5 Hz, 3H), 2.15-2.07 (m, 2H), 2.00-1.90 (m, 4H), 1.84 (d, *J =* 11.1 Hz, 2H), 1.70 (s, 4H), 1.62 (s, 4H), 1.49 (dt, *J* = 26.5, 7.7 Hz, 8H), 1.31 (t, *J* = 8.6 Hz, 7H), 1.23 (s, 2H), 1.21 (d, *J* = 7.1 Hz, 4H), 1.03 (d, *J* = 6.4 Hz, 3H), 0.94 (d, *J* = 11.9 Hz, 2H), 0.81 (dd, *J =* 18.2, 6.8 Hz, 7H).

### Example 38: Preparation of compound LA-27C-22

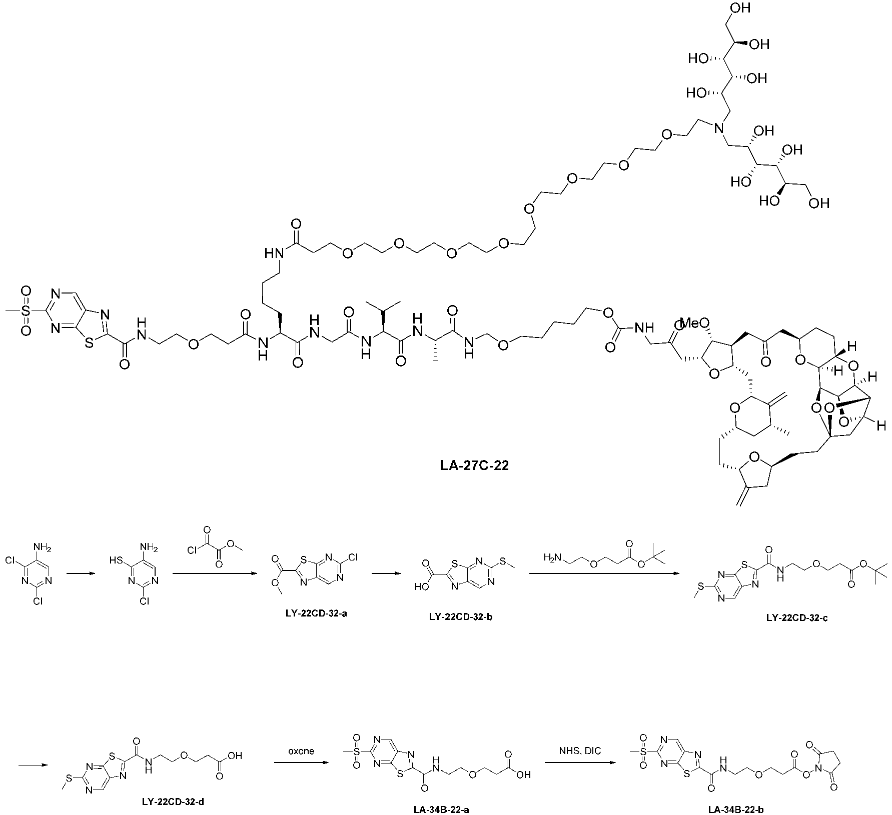

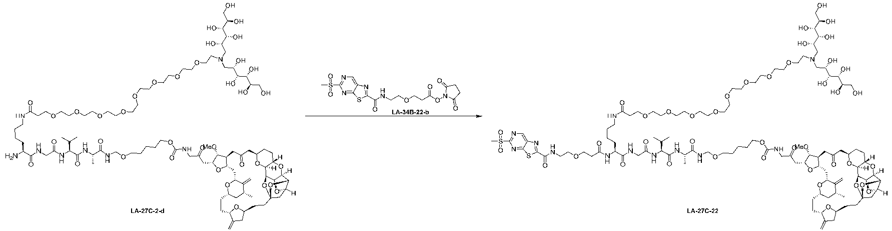

### Step 1: Preparation of 2-chloro-4-mercapto-5-aminopyrimidine

Sodium hydrosulfide hydrate (31.25g, 68%, 286.85 mmol, 3 eq) was added to a solution of 5-amino-2,4-dichloropyrimidine (16g, 95.62 mmol, 1 eq) in DMF (100 mL) portionwise at 0°C. The reaction solution was stirred at room temperature overnight. LCMS analysis showed that the reaction ended. The solvent was removed from the reaction solution under reduced pressure. Tetrahydrofuran (150 mL) was added to the residue. After filtration, the filter cake was washed with tetrahydrofuran (2 × 20 mL), and the filtrate was evaporated to dryness under reduced pressure. The residue was triturated with dichloromethane/tetrahydrofuran (90 mL, 2:1) to afford the product 2-chloro-4-mercapto-5-aminopyrimidine (14.27g; the yield was not calculated for the crude product) as an off-white solid. LCMS (ESI): m/z, 162.1[M+H]⁺.

### Step 2: Preparation of compound LY-22CD-32-a

DIPEA (15.69g, 120.17 mmol, 2 eq) and methyl oxalyl chloride (15.02g, 120.17 mmol, 2 eq) were added to a solution of 2-chloro-4-mercapto-5-aminopyrimidine (9.71g, 60.08 mmol, 1 eq) in DMF (100 mL). The reaction solution was stirred at 35°C for 2 hours. LCMS analysis showed that the reaction ended. The reaction solution (the previous two batches were combined for treatment, 500 mg and 5g respectively, with the same feeding procedure as the reaction) was evaporated to dryness under reduced pressure. DCM/MeOH (300 mL, 10:1) and water (200 mL) were added to the residue, and the pH was adjusted to below 6 with diluted hydrochloric acid (2 N). The mixture was extracted with DCM (3 × 100 mL). The organic phases were combined, washed with saturated brine (100 mL), dried over anhydrous Na₂SO₄, filtered, and evaporated to dryness under reduced pressure. The residue was triturated with PE/EA (250 mL, 4:1) and purified to afford the product LY-22CD-32-a (12.56g, 58.13% yield) as a white solid. LCMS (ESI): m/z, 230.0[M+H]⁺.

¹H NMR (400 MHz, DMSO-d6) δ 9.68 (s, 1H), 4.03 (s, 3H).

### Step 3: Preparation of compound LY-22CD-32-b

Sodium thiomethoxide hydrate (12.11g, 164.08 mmol, 3 eq) was added to a solution of compound LY-22CD-32-a (12.56g, 54.69 mmol, 1 eq) in MeOH (300 mL). The reaction solution was stirred at 70°C for 1 hour. LCMS analysis showed that the reaction ended. The reaction solution (combined with the previous batch for treatment, 4.81g, with the same feeding procedure as the reaction) was evaporated to dryness under reduced pressure. H₂O (200 mL) was added to the residue, and the pH was adjusted to below 4 with HCl (2N). The mixture was filtered and the filter cake was collected to afford the product LY-22CD-32-b (16.45g, 95.69% yield) as a white solid. LCMS (ESI): m/z, 228.2[M+H]⁺.

¹H NMR (400 MHz, DMSO-d6) δ 9.47 (s, 1H), 2.63 (s, 3H).

### Step 4: Preparation of compound LY-22CD-32-c

Pyridine (8.75g, 110.01 mmol, 5 eq) and POCl₃ (10.33g, 66 mmol, 3 eq) were added to a solution of compound LY-22CD-32-b (5.09g, 22 mmol, 1 eq) and amino-PEG-propanoic acid tert-butyl ester (4.29g, 22 mmol, 1 eq) in DCM (100 mL) at 0°C. The reaction solution was stirred at room temperature for half an hour. LCMS analysis showed that the reaction ended. DCM (100 mL) and water (100 mL) were added to the reaction solution. The mixture was extracted with DCM (3 × 100 mL). The organic phases were combined, washed with saturated brine (100 mL), dried over anhydrous Na₂SO₄, filtered, and then evaporated to dryness under reduced pressure. The mixture was triturated with PE/EA (100 mL, 2:1) and purified to afford the product LY-22CD-32-c as a white solid (5.61g, yield: 63.99%). LCMS (ESI): m/z, 421.1[M+Na]⁺.

¹H NMR (400 MHz, DMSO-d6) δ 9.38 (s, 1H), 9.20 (t, J = 5.8 Hz, 1H), 3.61 (t, J = 6.2 Hz, 2H), 3.56 (t, J = 6.0 Hz, 2H), 3.46 (q, J = 6.0 Hz, 2H), 2.63 (s, 3H), 2.42 (t, J = 6.0 Hz, 2H), 1.36 (s, 9H).

### Step 5: Preparation of compound LY-22CD-32-d

TFA (5 mL) was added to a solution of compound LY-22CD-32-c (5.61g, 14.08 mmol, 1 eq) in DCM (20 mL) at 0°C. The reaction solution was stirred at 30°C overnight. LCMS analysis showed that the reaction ended. The reaction solution was evaporated to dryness under reduced pressure to afford the product LY-22CD-32-d (7.13g; the yield was not calculated for the crude product; 65% purity) as a yellow oil. LCMS (ESI): m/z, 343.2[M+H]⁺.

### Step 6: Preparation of compound LA-34B-22-a

Oxone (10.83g, 63.09 mmol, 40 eq) was added to a solution of compound LY-22CD-32-d (540 mg, 1.58 mmol, 1 eq) in THF (5 mL) and H₂O (5 mL) under the ice bath. The mixture was stirred at room temperature overnight, and LCMS analysis indicated complete consumption of the starting material. After filtration, the mixture was concentrated. Water (5 mL) and dichloromethane (5 mL) were added, and the aqueous phase was extracted with dichloromethane (3 × 5 mL). The organic phases were combined, washed with saturated brine (10 mL) once, dried over anhydrous sodium sulfate, filtered and concentrated. The residue was then purified by silica gel column chromatography (eluents: dichloromethane/methanol = 0%-15%) to afford LA-34B-22-a (276.10 mg, 46.76% yield) as a yellow oil.

LCMS (ESI): m/z, 375[M+H]⁺, Rt=0.432 min.

### Step 7: Preparation of compound LA-34B-22-b

DIC (703.46 µL, 4.42 mmol, 6 eq) was added to a solution of compound LA-34B-22-a (276.10 mg, 737.47 µmol, 1 eq) and N-hydroxysuccinimide (519.64 mg, 4.42 mmol, 6 eq) in DMF (5 mL). The reaction solution was stirred at 50°C for 1 hour. The LCMS analysis indicated complete consumption of the starting material. The reaction solution was purified by preparative high-performance liquid chromatography (preparative chromatograph manufacturer: Orienda, model: BRIX-2860, column: Welch Xtimate C18 150 × 30mm × 10 µm, mobile phase: water (0.225% HCOOH)-acetonitrile, with an elution ratio of acetonitrile increased from 10% to 40%) and lyophilized to afford the compound LA-34B-22-b (233.30 mg, 46.97% yield, 70% purity) as a white solid.

LCMS (ESI): m/z, 472 [M+H]⁺. Rt = 0.987 min.

### Step 8: Preparation of compound LA-27C-22

A solution of LA-34B-22-b (8.91 mg, 70%, 13.23 µmol, 1.5 eq) in DMF (0.5 mL) was added to a solution of compound LA-27C-2-d (for the synthesis of the compound, refer to the Preparation Example of LA-27C-2) (17.59 mg, 8.82 µmol, 1 eq) and DIPEA (3.45 mg, 4.42 µL, 26.46 µmol, 3 eq) in DMF (0.5 mL) at 0°C. After addition, the reaction solution was allowed to react at 20°C for 40 minutes. LCMS analysis showed that the reaction ended. The reaction solution was filtered and the residue was then purified by preparative high-performance liquid chromatography (preparative chromatograph manufacturer: Orienda, model: BRIX-2860, column: Synergi Max-RP 200 × 30mm × 10 µm, mobile phase: water (0.1% TFA)-acetonitrile, with an elution ratio of acetonitrile increased from 20% to 50%) and lyophilized, to afford compound LA-27C-22 (10.91 mg, 52.62% yield, 100% purity) as a white solid.

LCMS (ESI): m/z, 1198.0[1/2M+Na]⁺. Rt = 1.946 min.

¹H NMR (400 MHz, DMSO-d6) δ 9.83 (s, 1H), 9.44 (s, 1H), 8.51 (s, 1H), 8.16-8.07 (m, 3H), 8.03 (d, J = 7.6 Hz, 1H), 7.77 (s, 1H), 7.72 (d, J = 8.6 Hz, 1H), 7.28 (s, 1H), 6.19 (d, J = 3.1 Hz, 1H), 5.96 (dd, J = 12.8, 3.0 Hz, 1H), 5.41 (d, J = 4.7 Hz, 2H), 4.97 (d, J = 21.7 Hz, 1H), 4.86 (s, 2H), 4.81-4.70 (m, 4H), 4.54 (dd, J = 18.1, 10.1 Hz, 6H), 4.45 (dd, J = 10.0, 6.4 Hz, 3H), 4.33 (s, 1H), 4.29 (d, J = 9.7 Hz, 1H), 4.20 (dt, J = 14.9, 7.6 Hz, 3H), 4.08 (d, J = 5.5 Hz, 2H), 4.00 (s, 3H), 3.92 (t, J = 6.6 Hz, 2H), 3.84 (d, J = 5.7 Hz, 2H), 3.78 (s, 3H), 3.73 (d, J = 5.8 Hz, 3H), 3.70-3.65 (m, 4H), 3.63 (d, J = 8.2 Hz, 4H), 3.57 (t, J = 6.4 Hz, 11H), 3.52 (s, 7H), 3.50 (d, J = 2.2 Hz, 15H), 3.49 (s, 5H), 3.47 (d, J = 1.9 Hz, 4H), 3.43 (dd, J = 9.7, 3.8 Hz, 7H), 3.18 (d, J = 6.1 Hz, 3H), 3.03-2.90 (m, 4H), 2.82-2.73 (m, 2H), 2.71-2.66 (m, 3H), 2.60 (d, J = 7.7 Hz, 3H), 2.41 (d, J = 10.3 Hz, 3H), 2.33 (p, J = 1.9 Hz, 3H), 2.28 (t, J = 6.6 Hz, 3H), 2.15-2.08 (m, 1H), 1.95 (t, J = 6.8 Hz, 3H), 1.84 (d, J = 10.4 Hz, 1H), 1.71 (d, J = 4.1 Hz, 3H), 1.63 (d, J = 6.8 Hz, 4H), 1.55-1.42 (m, 8H), 1.32 (t, J = 8.7 Hz, 7H), 1.21 (d, J = 7.1 Hz, 4H), 1.03 (d, J = 6.4 Hz, 3H), 1.00-0.93 (m, 1H), 0.84 (d, J = 6.8 Hz, 3H), 0.79 (d, J = 6.7 Hz, 3H).

### Example 39: Preparation of Compound LA-28

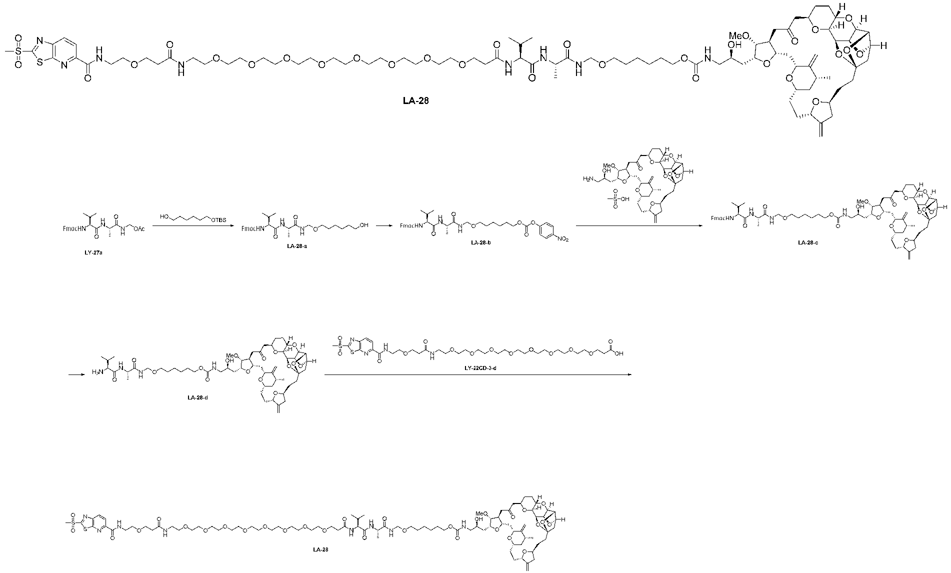

### Step 1: Preparation of compound LA-28-a

TsOH (21 mg, 124.6 µmol, 0.3 eq) was added to the solution of 6-((tert-butyldimethylsilyl) oxy)hexane-1-ol (193 mg, 830.65 µmol, 2 eq) and LY-27a (200 mg, 415.33 µmol, 1 eq) in THF (6 mL) and a resulting solution was stirred at 33°C for 3 hours. LCMS analysis showed that the reaction ended. HCl (4M, 0.5 mL) was added to the mixture, and the reaction solution was distilled and evaporated to dryness under reduced pressure. The residue was purified by silica gel column chromatography (eluents: methanol/dichloromethane = 0:1 to 1:10) to afford LA-28-a (277 mg; the yield was not calculated for the crude product) as a white solid.

### Step 2: Preparation of compound LA-28-b

DIPEA (254.49 µL) was added to a solution of compound LA-28-a (277 mg) and NPC (312.29 mg, 1.03 mmol, 2 eq) in DMF (6 mL) and the reaction solution was stirred overnight at room temperature. LCMS analysis showed that the reaction ended. The mixture was purified by preparative high-performance liquid chromatography (preparative chromatograph manufacturer: Orienda, model: BRIX-2860, column: Welch Xtimate C18 250 × 30 mm × 10 µm, mobile phase: water (0.225% HCOOH)-acetonitrile, with an elution ratio of acetonitrile increased from 50% to 80%), to afford LA-28-b (16.90 mg, 4.67% yield) as a white solid.

### Step 3: Preparation of compound LA-28-c

DIPEA (1.56 mg, 12.11 µmol, 1 eq) was added to a solution of eribulin mesylate (10 mg, 12.11 µmol, 1 eq) in DMF (2 mL) at 0°C, and then LA-28-b (11 mg, 15.74 µmol, 1.3 eq), HOBt (818 µg, 6.05 µmol, 0.5 eq), and pyridine (5 mg, 60.53 µmol, 5 eq) were added to the mixture. The reaction solution was stirred overnight at room temperature. LCMS analysis showed that the reaction ended. The mixture was purified by preparative high-performance liquid chromatography (preparative chromatograph manufacturer: Orienda, model: BRIX-2860, column: Welch Xtimate C18 250 × 30 mm × 10 µm, mobile phase: water (0.225% HCOOH)-acetonitrile, with an elution ratio of water increased from 30% to 60%), to afford LA-28-c (14 mg, 89.26% yield) as a white solid.

### Step 4: Preparation of compound LA-28-d

The compound LA-28-c (14 mg, 10.81 µmol, 1 eq) was dissolved in DMF (2 mL), DBU (4.94 mg, 32.42 µmol, 3 eq) was then added, and the reaction solution was stirred at room temperature for half an hour. LCMS analysis showed that the reaction ended. P-toluenesulfonic acid (5 mg, 32.42 µmol, 3 eq) was added to the reaction solution to afford product LA-28-d (11.60 mg, crude product in 2 mL of DMF), which was used directly in the next step without further treatment.

### Step 5: Preparation of compound LA-28

LY-22CD-3-d (9.47 mg, 11.89 µmol, 1.1 eq), N,N-diisopropylethylamine (6.98 mg, 54.04 µmol, 5 eq), and HATU (4.93 mg, 12.97 µmol, 1.2 eq) were added to a solution of compound LA-28-d(11.60 mg) in DMF (2 mL) at 0°C. The mixture was stirred at room temperature for 1 hour, and the LCMS analysis indicated complete consumption of the starting material. The mixture was purified by preparative high-performance liquid chromatography (preparative chromatograph manufacturer: Orienda, model: BRIX-2860, column: Welch Xtimate C18 150 × 30 mm × 10 µm, mobile phase: water (0.225% HCOOH)-acetonitrile, with an elution ratio of acetonitrile increased from 30% to 60%), to afford LA-28 (4 mg, 19.58% yield) as a white solid.

LCMS (ESI): m/z, 1875.1[M+Na]⁺.

### Example 40: Synthesis of Compound LA-28AA

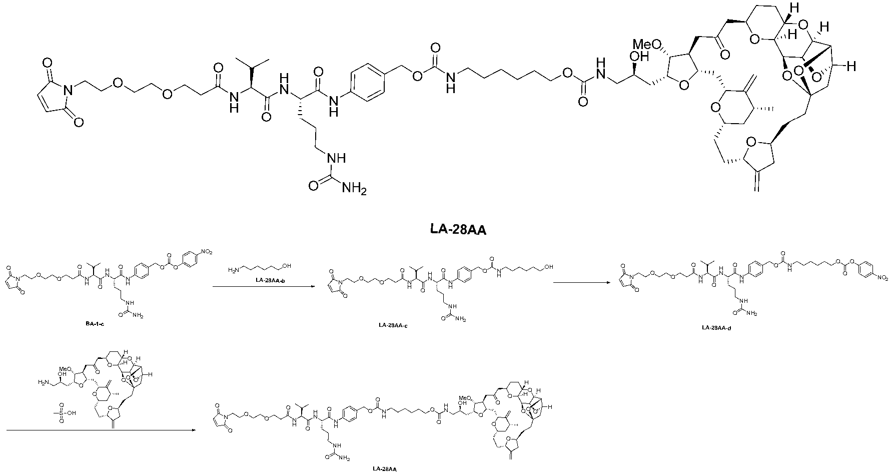

### Step 1: Preparation of compound LA-28AA-c

DIPEA (20.61 mg, 159.48 µmol, 2.5 eq) was added to a solution of compound BA-1-c (for the synthesis of the compound, refer to the Example of BA-1) (50 mg, 63.79 µmol, 1 eq) and 6-aminohexanol (7.48 mg, 63.79 µmol, 1 eq) in DMF (2 mL). The resulting solution was allowed to react at room temperature for 2 hours, and the LCMS analysis showed that the reaction ended. After filtration of the reaction solution, the filtrate was purified by preparative high-performance liquid chromatography (preparative chromatograph manufacturer: ISCO, model: ISCO, column: Welch Xtimate C18 250 × 30 mm × 10 µm, mobile phase: water (0.225% HCOOH)-acetonitrile, with an elution ratio of acetonitrile increased from 20% to 50%), to afford compound LA-28AA-c (35.60 mg, 73.25% yield) as a colorless oil. LCMS (ESI): m/z, 762[M+H]⁺.

### Step 2: Preparation of compound LA-28AA-d

Bis(4-nitrophenyl) carbonate (85.29 mg, 56.86 µL, 280.36 µmol, 6 eq) and DIPEA (54.35 mg, 69.50 µL, 420.54 µmol, 9 eq) were added to a solution of compound LA-28AA-c (35.60 mg, 46.73 µmol, 1 eq) in DMF (2 mL). The resulting solution was allowed to react at room temperature for 1 hour, and the LCMS analysis showed that the reaction ended. The residue was purified by silica gel column chromatography (eluents: ethyl acetate/hexane = 1:20 to 1:5; and dichloromethane /methanol = 20:1 to 10:1) to afford compound LA-28AA-d (34.80 mg, 80.34% yield) as a white solid. LCMS (ESI): m/z, 949 [M+Na]⁺.

### Step 3: Preparation of compound LA-28AA

DIPEA (4.69 mg, 6 µL, 36.32 µmol, 3 eq) and HOBT (1.64 mg, 12.11 µmol, 1 eq) were added to the solution of compound LA-28AA-d (12.34 mg, 13.32 µmol, 1.1 eq) and eribulin mesylate (10 mg, 12.11 µmol, 1 eq) in DMF (1 mL), and after a resulting mixture allowed to react for 2 hours at room temperature, LCMS analysis indicated complete consumption of the starting material, and the residue was purified by preparative high-performance liquid chromatography (preparative chromatograph manufacturer: ISCO, model: ISCO-R5, column: YMC-Triart Prep C18 150 × 30 mm × 10 µm, mobile phase: water (0.225% HCOOH)-acetonitrile, with an elution ratio of acetonitrile increased from 30% to 60%), to afford product LA-28AA (7.20 mg, 39.18% yield, 95% purity) as a white solid. LCMS (ESI): m/z, 1540[M+Na]⁺.

### Example 41: Preparation of Compound LA-28AA-2

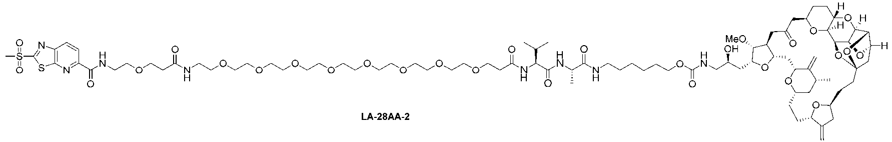

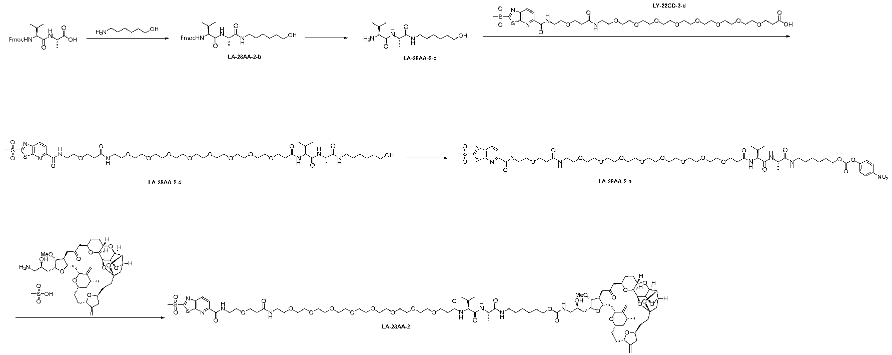

### Step 1: Preparation of compound LA-28AA-2-b

N-(fluorenylmethoxycarbonyl)-L-valyl-L-alanine (205.23 mg, 500 µmol, 1 eq) was dissolved in DMF (3 mL), the mixture was cooled to 0°C, then 6-amino-1-hexanol (58.59 mg, 500 µmol, 1 eq), and DIPEA (193.86 mg, 247.90 µL, 1.50 mmol, 3 eq) and HATU (190.12 mg, 500 µmol, 1 eq) were added. The reaction solution was allowed to react at 25°C for 3 hours. LCMS analysis showed complete consumption of the starting material. The residue was purified by preparative high-performance liquid chromatography (preparative chromatograph manufacturer: Shimadzu, model: LC-20AP, column: YMC-Triart Prep C18 150 × 30 mm × 10 µm, mobile phase: water (0.225% HCOOH)-acetonitrile, with an elution ratio of acetonitrile increased from 30% to 60%), to afford LA-28AA-2-b (150 mg, 58.86% yield) as a white solid.

LCMS (ESI): m/z, 532.4 [M+Na]⁺, Rt = 2.061 min.

### Step 2: Preparation of compound LA-28AA-2-c

Compound LA-28AA-2-b (71.35 mg, 140 µmol, 1 eq) was dissolved in DMF (3 mL), the mixture was cooled to 0°C, and then DBU (1,8-diazabicyclo[5.4.0]undec-7-ene, 63.94 mg, 62.75 µL, 420 µmol, 3 eq) was added. The reaction solution as allowed to react at 25°C for 1 hour. The LCMS analysis indicated complete consumption of the starting material. The reaction solution was neutralized with TsOH (72.32 mg, 420 µmol, 3 eq) and used directly in the next step.

LCMS (ESI): m/z, 288.4 [M+H]⁺, Rt = 0.206 min.

### Step 3: Preparation of compound LA-28AA-2-d

Compound LA-28AA-2-c (17.24 mg, 60 µmol, 1 eq) was dissolved in DMF (1 mL), the mixture was cooled to 0°C, and then LY-22CD-3-d (47.81 mg, 60 µmol, 1 eq), DIPEA (23.26 mg, 29.75 µL, 180 µmol, 3 eq) and HATU (22.81 mg, 60 µmol, 1 eq) were added. The reaction solution was allowed to react at 25°C for 30 minutes. LCMS analysis showed that the reaction ended. The reaction solution was purified by high-performance liquid chromatography (preparative chromatograph manufacturer: Shimadzu, model: LC-20AP, column: YMC-Triart Prep C18 250 × 30 mm × 10 µm, mobile phase: water (0.225% HCOOH)-acetonitrile, with an elution ratio of acetonitrile increased from 20% to 50%), to afford LA-28AA-2-d (60 mg, 93.78% yield) as a yellow oil.

LCMS (ESI): m/z, 1066.6 [M+H]⁺, Rt = 1.307 min.

### Step 4: Preparation of compound LA-28AA-2-e

Compound LA-28AA-2-d (31.99 mg, 30 µmol, 1 eq) was dissolved in DMF (2 mL), and then NPC (45.63 mg, 30.42 µL, 150 µmol, 5 eq) and DIPEA (23.26 mg, 29.75 µL, 180 µmol, 6 eq) were added. The reaction solution was allowed to react at 35°C for 1 hour. LCMS analysis showed that the reaction ended. The reaction solution was concentrated, and the residue was purified by silica gel column chromatography (DCM/MeOH = 10/1) to afford LA-28AA-2-e (20 mg, 27.07% yield, 50% purity) as a yellow oil.

LCMS (ESI): m/z, 1253.7 [M+Na]⁺, Rt = 2.038 min.

### Step 5: Preparation of compound LA-28AA-2

Compound LA-28AA-2-e (17.89 mg, 50%, 7.26 µmol, 1 eq) was dissolved in DMAC (500 µL) and the mixture was cooled to 0°C. Then eribulin mesylate (6 mg, 7.26 µmol, 1 eq) and DIPEA (2.82 mg, 3.60 µL, 21.79 µmol, 3 eq) were added. The reaction solution was allowed to react at 25°C for 1 hour. LCMS analysis showed that the reaction ended. The reaction solution was purified by preparative high-performance liquid chromatography (preparative chromatograph manufacturer: Orienda, model: BRIX-2860(R7), column: Phenomenex Luna C18 200 × 30 mm × 10 µm, mobile phase: water (0.225% HCOOH)-acetonitrile, with an elution ratio of acetonitrile increased from 30% to 60%), to afford LA-28AA-2 (2.61 mg, 19.72% yield, 96.37% purity) as a white solid.

LCMS (ESI): m/z, 1823.0 [M+H]⁺, Rt = 2.236 min.

### Example 42: Preparation of Compound LA-29

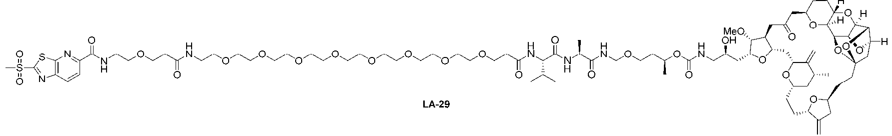

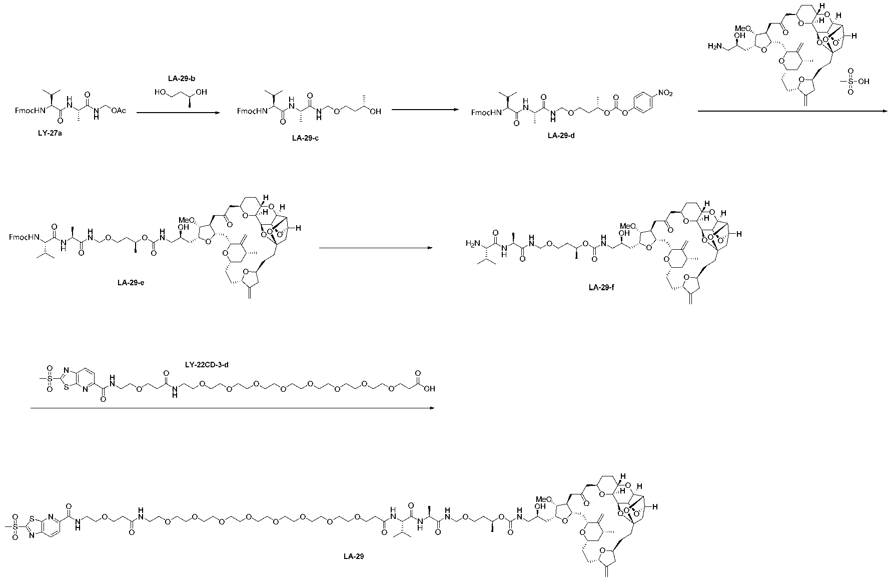

### Step 1: Preparation of compound LA-29-c

The compound LY-27a (250 mg, 519 µmol, 1 eq) was dissolved in THF (3 mL), a resulting mixture was cooled to 0°C, and then TsOH (89.4 mg, 83.5 µL, 519 µmol, 1 eq) and (S)-butane-1,3-diol (140 mg, 1.56 mmol, 3 eq) were added. The reaction solution was allowed to react at 25°C for 3 hours. The LCMS analysis indicated complete consumption of the starting material. The reaction solution was concentrated to dryness and then subjected to preparative high-performance liquid chromatography (preparative chromatograph manufacturer: Orienda, model: BRIX-2860, column: Phenomenex Luna C18 250 × 50 mm × 10 µm, mobile phase: water (0.225% HCOOH)-acetonitrile, with an elution ratio of acetonitrile increased from 30% to 60%), to afford compound LA-29-c (100 mg, 37.65% yield) as a white solid.

LCMS (ESI): m/z, 534.3 [M+Na]⁺. Rt = 1.971 min.

### Step 2: Preparation of compound LA-29-d

Bis(4-nitrophenyl)carbonate (237.84 mg, 781.83 µmol, 4 eq) and DIPEA (151.57 mg, 193.82 µL, 1.17 mmol, 6 eq) were added to the solution of compound LA-29-c (100 mg, 195.46 µmol, 1 eq) in DMF (5 mL), a resulting mixture was allowed to react at 33°C for 1 hour, LCMS analysis showed that the reaction ended, and the reaction solution was purified by preparative high-performance liquid chromatography (preparative chromatograph manufacturer: ISCO, model: ISCO, column: Welch Xtimate C18 250 × 30 mm × 10 µm, mobile phase: water (0.225% HCOOH)-acetonitrile, with an elution ratio of acetonitrile increased from 60% to 90%), to afford the compound LA-29-d (30 mg, 22.68% yield) as a white solid. LCMS (ESI): m/z, 699[M+Na]⁺.

### Step 3: Preparation of compound LA-29-e

DIPEA (4.69 mg, 6 µL, 36.32 µmol, 3 eq) and HOBT (1.64 mg, 12.11 µmol, 1 eq) were added to the solution of compound LA-29-d (9.01 mg, 13.32 µmol, 1.1 eq) and eribulin mesylate (10 mg, 12.11 µmol, 1 eq) in DMF (1 mL), and after a resulting mixture was allowed to react for 2 hours at room temperature, LCMS analysis showed that the reaction ended, and the reaction solution was purified by preparative high-performance liquid chromatography (preparative chromatograph manufacturer: ISCO, model: ISCO, column: Welch Xtimate C18 150 × 30mm × 10 µm, mobile phase: water (0.225% HCOOH)-acetonitrile, with an elution ratio of water increased from 50% to 80%), to afford the product LA-29-e (5.80 mg, 37.80% yield) as a white solid. LCMS (ESI): m/z, 1289[M+Na]⁺.

### Step 4: Preparation of compound LA-29-f

DBU (2.09 mg, 2.05 µL, 13.73 µmol, 3 eq) was added to the solution of the compound LA-29-e (5.80 mg, 4.58 µmol, 1 eq) in DMF (1 mL) and after a resulting mixture was allowed to react for 40 minutes at room temperature, LCMS analysis indicated complete consumption of the starting material, and TsOH (2.36 mg, 13.73 µmol, 3 eq) was added to afford the compound LA-29-f (4.78 mg; the yield was not calculated for the crude product) as a yellow oil. LCMS (ESI): m/z, 1067[M+Na]⁺.

### Step 5: Preparation of compound LA-29

DIPEA (1.18 mg, 1.51 µL, 9.15 µmol, 2 eq) and HATU (2.09 mg, 5.49 µmol, 1.2 eq) were added to the solution of compounds LA-29-f (4.78 mg, 4.57 µmol, 1 eq) and LY-22CD-3 (3.64 mg, 4.57 µmol, 1 eq) in DMF (1 mL), and after a resulting mixture was allowed to react for 1 hour at room temperature, LCMS analysis showed that the reaction ended, and the reaction solution was purified by preparative high-performance liquid chromatography (preparative chromatograph manufacturer: ISCO, model: ISCO, column: Welch Xtimate C18 250 × 30mm × 10 µm, mobile phase: water (0.225% HCOOH)-acetonitrile, with an elution ratio of acetonitrile increased from 30% to 60%), to afford product LA-29 (4.38 mg, 52.51% yield, 97% purity) as a white solid. LCMS (ESI): m/z, 1847[M+Na]⁺.

### Example 43: Preparation of Compound LA-29AR

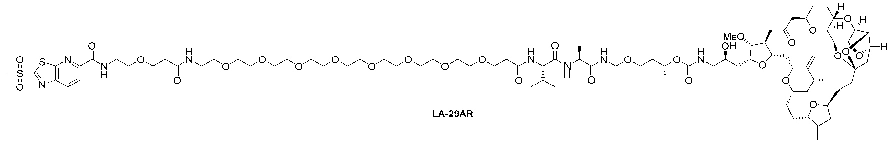

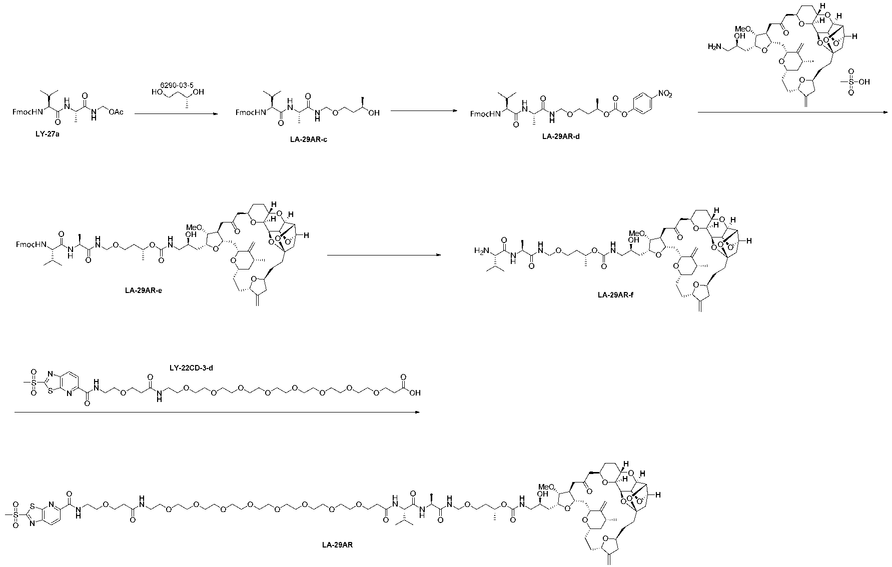

### Step 1: Preparation of compound LA-29AR-c

The compound LY-27a (500 mg 1.04 mmol, 1.2 eq) was dissolved in THF (5 mL), a resulting mixture was cooled to 0°C, and then TsOH (149 mg, 865.26 µL, 1 eq) and (R)-(-)-1,3-butanediol (233.93 mg, 2.60 mmol, 3 eq) were added. The reaction solution was allowed to react at 35°C for 2 hours. The LCMS analysis indicated complete consumption of the starting material. The reaction solution was concentrated to dryness and then subjected to preparative high-performance liquid chromatography (preparative chromatograph manufacturer: Orienda, model: BRIX-2860, column: YMC-Triart Prep C18 150 × 30 mm × 10 µm, mobile phase: water (0.225% HCOOH)-acetonitrile, with an elution ratio of acetonitrile increased from 30% to 60%), to afford compound LA-29AR-c (204.30 mg, 46.15% yield) as a white solid.

LCMS (ESI): m/z, 534.3 [M+Na]⁺. Rt = 1.970 min.

### Step 2: Preparation of compound LA-29AR-d

Bis(4-nitrophenyl) carbonate (485.91 mg, 323.94 µL, 1.60 mmol, 4 eq) and DIPEA (309.65 mg, 395.97 µL, 2.40 mmol, 6 eq) were added to a solution of compound LA-29AR-c (204.30 mg, 399.32 µmol, 1 eq) in DMF (6 mL). The resulting solution was allowed to react at 33°C for 4 hours, and the LCMS analysis showed that the reaction ended. The residue was purified by silica gel column chromatography (eluents: petroleum ether/ethyl acetate = 10:1 to 4:1; and dichloromethane/methanol = 10:1) to afford compound LA-29AR-d (191.40 mg, 70.83% yield) as a white solid. LCMS (ESI): m/z, 699 [M+Na]⁺. Rt = 2.879 min.

### Step 3: Preparation of compound LA-29-e

DIPEA (2.35 mg, 3 µL, 18.16 µmol, 3 eq) and HOBT (817.91 µg, 6.05 µmol, 1 eq) were added to the solution of compound LA-29AR-d (4.92 mg, 7.26 µmol, 1.2 eq) and eribulin mesylate (5 mg, 6.05 µmol, 1 eq) in DMF (0.5 mL), and after a resulting mixture was allowed to react at 40°C for 3 hours, LCMS analysis showed that the reaction ended, and the reaction solution was purified by preparative high-performance liquid chromatography (preparative chromatograph manufacturer: Orienda, model: BRIX-2860, column: Welch Xtimate C18 250 × 30mm × 10 µm, mobile phase: water (0.225% HCOOH)-acetonitrile, with an elution ratio of acetonitrile increased from 60% to 90%), to afford the product LA-29AR-e (5.60 mg, 72.99% yield) as a white solid. LCMS (ESI): m/z, 1289[M+Na]⁺. Rt = 3.113 min.

### Step 4: Preparation of compound LA-29AR-f

Et₂NH (2.19 mg, 3.09 µL, 29.98 µmol, 5 eq) was added to the solution of the compound LA-29AR-e (7.60 mg, 6 µmol, 1 eq) in DMF (0.5 mL) and after a resulting mixture was allowed to react at room temperature for 2 hours, LCMS analysis indicated complete consumption of the starting material. The reaction solution was evaporated to dryness to afford the compound LA-29AR-f (6.27 mg; the yield was not calculated for the crude product) as a white solid. LCMS (ESI): m/z, 1067[M+Na]⁺. Rt = 1.742 min.

### Step 5: Preparation of compound LA-29AR

DIPEA (1.55 mg, 1.98 µL, 12 µmol, 2 eq) and HATU (2.74 mg, 7.20 µmol, 1.2 eq) were added to the solution of compounds LA-29AR-f (6.27 mg, 6 µmol, 1 eq) and LY-22CD-3-d (4.78 mg, 6 µmol, 1 eq) in DMF (1 mL), and after a resulting mixture was allowed to react for 1 hour at room temperature, LCMS analysis showed that the reaction ended, and the reaction solution was purified by preparative high-performance liquid chromatography (preparative chromatograph manufacturer: Shimadzu, model: LC-20AP, column: Synergi Max-RP 250 × 40 mm × 10 µm, mobile phase: water (0.225% HCOOH)-acetonitrile, with an elution ratio of acetonitrile increased from 60% to 90%), to afford product LA-29AR (3.76 mg, 33.33% yield, 97% purity) as a white solid. LCMS (ESI): m/z, 1847[M+Na]⁺. Rt = 2.185 min.

### Example 44: Preparation of Compound LA-29B

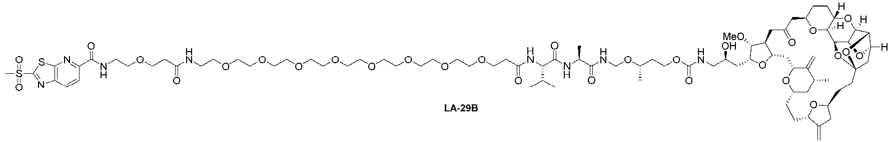

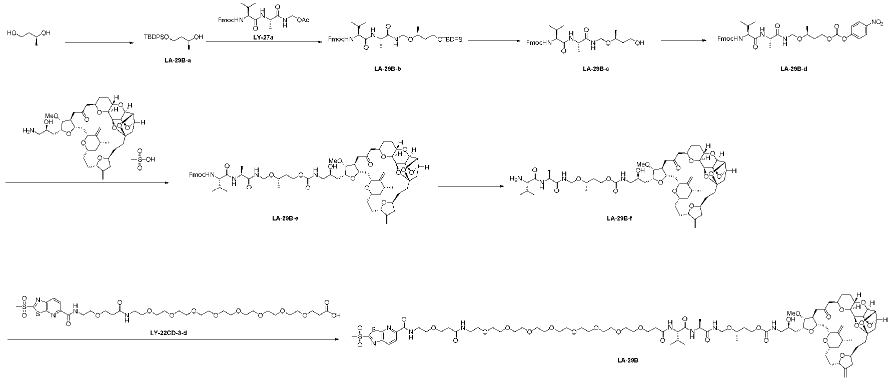

### Step 1: Preparation of (2S)-4-{[tert-butyl(diphenyl)silyl]oxy}butan-2-ol (LA-29B-a)

Imidazole (453.26 mg, 6.66 mmol, 1.2 eq) and TBDPSCl (1.52g, 5.55 mmol, 1 eq) were added to a solution of (S)-butane-1,3-diol (500 mg, 5.55 mmol, 1 eq) in THF (5 mL). The mixture was stirred at room temperature overnight, and TLC indicated complete consumption of the starting material. Water (10 mL) and ethyl acetate (10 mL) were added. The aqueous phase was extracted with ethyl acetate (10 mL × 3). The organic phase was washed with saturated brine (10 mL), dried over anhydrous sodium sulfate, filtered, and concentrated. The residue was purified by silica gel column chromatography (eluents: ethyl acetate/petroleum ether = 6%-8%) to afford the product LA-29B-a (1.33g, 73.16% yield).

¹H NMR (400 MHz, Chloroform-d) δ 7.73-7.66 (m, 4H), 7.47-7.37 (m, 6H), 4.10 (dqd, *J* = 9.0, 6.2, 2.8 Hz, 1H), 3.92-3.79 (m, 2H), 1.74 (dtd, *J=* 13.6, 8.4, 5.0 Hz, 1H), 1.66-1.60 (m, 1H), 1.21 (d, *J =* 6.2 Hz, 3H), 1.06 (d, *J* = 7.4 Hz, 9H).

### Step 2: Preparation of compound LA-29B-b

P-toluenesulfonic acid (178.80 mg, 1.04 mmol, 1 eq) was added to a solution of compound LA-29B-a (500 mg, 1.04 mmol, 1 eq) and LY-27a (682.24 mg, 2.08 mmol, 2 eq) in tetrahydrofuran (10 mL). The mixture was stirred at 30°C for 2 hours. After the reaction solution was concentrated, the residue was dissolved in N,N-dimethylformamide, the resulting solution was filtered, and the filtrate was purified by high-performance liquid chromatography (preparative chromatograph manufacturer: Orienda, model: BRIX-2860, column: Synergi Max-RP 200 × 30mm × 10 µm, mobile phase: water (0.225% HCOOH)-acetonitrile, with an elution ratio of acetonitrile increased from 60% to 90%), to afford the product LA-29B-b (167.7 mg, 22% yield).

LCMS (ESI): m/z, 772[M+Na]⁺.

### Step 3: Preparation of compound LA-29B-c

TEA.3HF (1.09 mL, 6.71 mmol, 30 eq) was added to the solution of compound LA-29B-b (167.70 mg, 223.59 µmol, 1 eq) in THF (3 mL), the reaction solution was stirred at 65°C for 2 hours, and the LCMS analysis indicated complete consumption of the starting material. The reaction solution was concentrated, and the residue was purified by preparative high-performance liquid chromatography (preparative chromatograph manufacturer: Orienda, model: BRIX-2860 (R6), column: Welch Xtimate C18 250 × 30mm × 10 µm, mobile phase: water (0.225% HCOOH)-acetonitrile, with an elution ratio of acetonitrile increased from 30%-60%), to afford the product LA-29B-c (92.70 mg, 81.04% yield).

LCMS (ESI): m/z, 534[M+Na]⁺.

### Step 4: Preparation of compound LA-29B-d

DIPEA (239.56 µL, 1.45 mmol, 8 eq) and NPC (330.72 mg, 1.09 mmol, 6 eq) were added to the solution of compound LA-29B-c (92.70 mg, 181.19 µmol, 1 eq) in DMF (10 mL), the reaction solution was stirred at 35°C for 3 hours and concentrated by an oil pump, and the residue was purified by silica gel column chromatography (eluents: ethyl acetate/petroleum ether = 0%-50%, methanol/dichloromethane = 0%-8%), to afford the product LA-29B-d (132.50 mg, 71% purity).

LCMS (ESI): m/z, 699[M+Na]⁺.

### Step 5: Preparation of compound LA-29B-e

DIPEA (6.26 mg, 48.43 µmol, 4 eq) and HOBT (1.64 mg, 12.11 µmol, 1 eq) were added to the solution of compound LA-29B-d (13.85 mg, 71%, 14.53 µmol, 1.2 eq) and eribulin mesylate (10 mg, 12.11 µmol, 1 eq) in DMF (1 mL), the reaction solution was stirred at room temperature for 2 hours, and LCMS analysis indicated complete consumption of the starting material. The reaction solution was purified by preparative high-performance liquid chromatography (preparative chromatograph manufacturer: Orienda, model: BRIX-2860, column: Synergi Max-RP 200 × 30mm × 10 µm, mobile phase: water (0.225% HCOOH)-acetonitrile, with an elution ratio of acetonitrile increased from 50%-80%), to afford the product LA-29B-e (6.40 mg, 41.71% yield).

LCMS (ESI): m/z, 1290[M+Na]⁺.

### Step 6: Preparation of compound LA-29B-f

DBU (2.31 mg, 15.15 µmol, 3 eq) was added to the solution of LA-29B-e (6.40 mg, 5.05 µmol, 0.5 eq) in DMF (1 mL) under an ice bath, the reaction solution was stirred at room temperature for 1 hour, LCMS analysis indicated complete consumption of the starting material, then p-toluenesulfonic acid (2.61 mg, 15.15 µmol, 3 eq) was added under an ice bath and a resulting mixture was directly used for the next step. A theoretical mass of LA-29B-f was 5.28 mg.

LCMS (ESI): m/z, 1045[M+H]⁺.

### Step 7: Preparation of compound LA-29B

DIPEA (2.61 mg, 3.34 µL, 20.21 µmol, 4 eq) and HATU (2.30 mg, 6.06 µmol, 1.2 eq) were added to the solution of compounds LA-29B-f (5.28 mg, 5.05 µmol, 1 eq) and LY-22CD-3-d (4.43 mg, 5.56 µmol, 1.1 eq) in DMF (1 mL) under the ice bath, the reaction solution was stirred for 1 hour at room temperature, and the LCMS analysis showed complete consumption of the starting material. The reaction solution was purified by preparative high-performance liquid chromatography (preparative chromatograph manufacturer: Orienda, model: BRIX-2860, column: Welch Xtimate C18 150 × 30 mm × 10 µm, mobile phase: water (0.225% HCOOH)-acetonitrile, with an elution ratio of acetonitrile increased from 30%- 60%), to afford the product LA-29B (3.59 mg, 38.96% yield).

LCMS (ESI): m/z, 1846[M+Na]⁺. Rt = 2.177 min.

### Example 45: Preparation of compounds LA-30A-1 and LA-30A-2

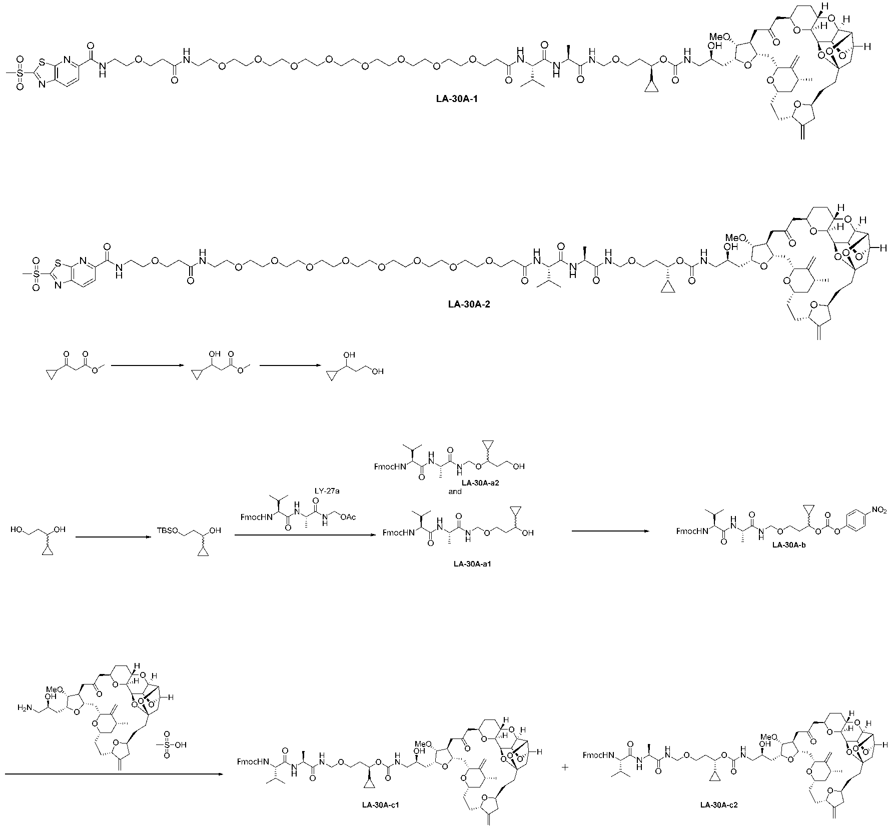

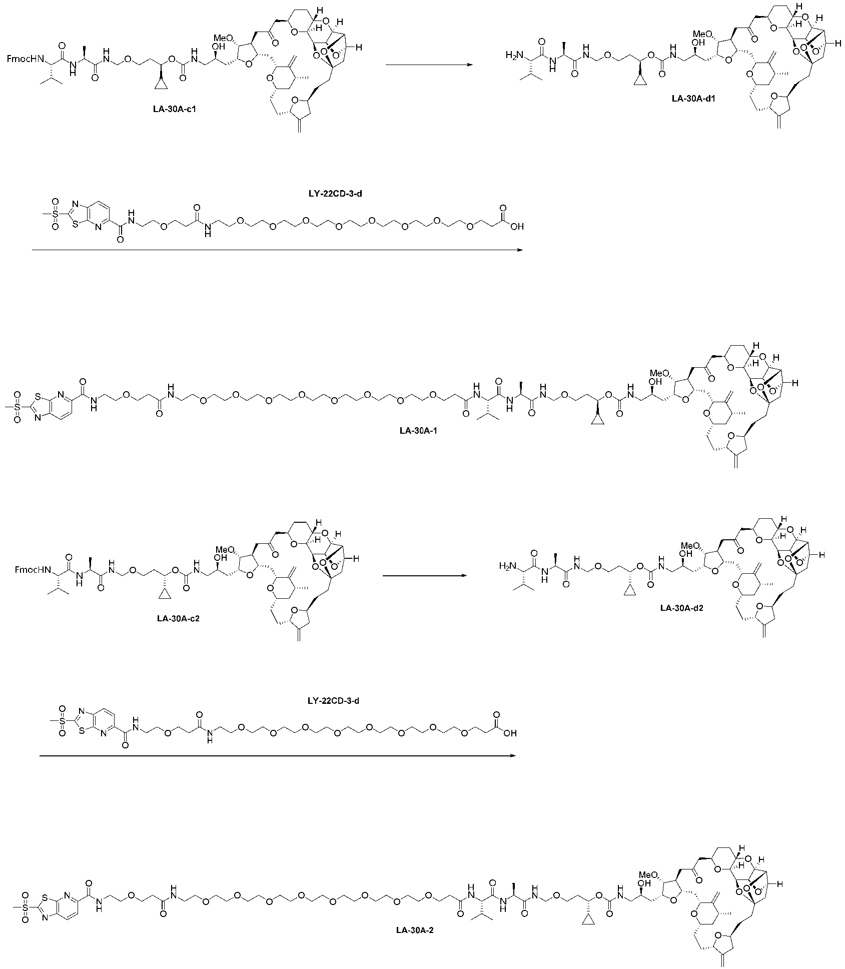

### Step 1: Preparation of methyl 3-cyclopropyl-3-hydroxypropanoate

NaBH₄ (3.99g, 4.22 mL, 105.52 mmol, 3 eq) was added to a solution of methyl 3-cyclopropyl-3-oxopropanoate (5g, 35.17 mmol, 1 eq) in MeOH (100 mL) at 0°C. After the addition, the reaction solution was allowed to react at 15°C for 3 hours. The bulk of methanol was then removed by evaporation. The residue was diluted with 100 mL of ethyl acetate. The organic phase was washed with water (100 mL × 2), dried over anhydrous sodium sulfate, and filtered, and the filtrate was concentrated to afford methyl 3-cyclopropyl-3-hydroxypropanoate (3.80g, 74.94% yield) as a colorless oil.

¹H NMR (400 MHz, Chloroform-d) *δ* 4.72 (s, 2H), 3.95-3.75 (m, 2H), 3.11 (td, *J* = 8.1, 4.5 Hz, 1H), 2.41 (d, *J* = 74.4 Hz, 2H), 1.95-1.75 (m, 2H), 1.05-0.86 (m, 1H), 0.53 (dq, *J* = 8.3, 2.2 Hz, 2H), 0.36-0.28 (m, 1H), 0.19-0.22 (m, 1H).

### Step 2: Preparation of compound 1-cyclopropylpropane-1,3-diol

LiAlH₄ (197.42 mg, 203.53 µL, 5.20 mmol, 1.5 eq) was added to a solution of methyl 3-cyclopropyl-3-hydroxypropanoate (500 mg, 3.47 mmol, 1 eq) in THF (10 mL) at 0°C. After the addition, the reaction solution was allowed to react at 5°C for 3 hours. TLC indicated the reaction ended. The reaction was quenched sequentially with 0.2 mL of water, 0.2 mL of 15% aqueous NaOH solution, and 0.6 mL of water at 0°C. Then 5g of anhydrous sodium sulfate was added to the reaction solution, the resulting mixture was then diluted with 30 mL of ethyl acetate and filtered, and the filtrate was concentrated to afford 1-cyclopropylpropane-1,3-diol as a colorless oil (300 mg, 74.47% yield).

¹H NMR (400 MHz, Methanol-*d⁴*) δ 3.71 (t, *J* = 6.6 Hz, 2H), 3.00 (td, *J* = 8.3, 4.6 Hz, 1H), 1.88-1.71 (m, 2H), 0.86 (dt, *J* = 8.1, 4.9 Hz, 1H), 0.48 (dq, *J* = 8.6, 1.7 Hz, 2H), 0.36-0.27 (m, 1H), 0.24-0.16 (m, 1H).

### Step 3: Preparation of 3-((tert-butyldimethylsilyl)oxy)-1-cyclopropylpropan-1-ol

TBSCl (135.59 mg, 155.85 µL, 899.62 µmol, 1.1 eq) and imidazole (55.68 mg, 50.62 µL, 817.84 µmol, 1 eq) were added to a solution of 1-cyclopropylpropane-1,3-diol (95 mg, 817.84 µmol, 1 eq) in DCM (8 mL) at 0°C. After the addition, the mixture was allowed to react at 25°C for 16 hours. The reaction ended as indicated by NMR. The reaction solution was added into ice-cold aqueous sodium bicarbonate solution (60 mL) and the mixture was then stirred for 2 minutes and extracted with dichloromethane (60 mL × 3). The organic phases were combined, washed with brine, dried over anhydrous sodium sulfate, and filtered. The filtrate was concentrated to afford the compound 3-((tert-butyldimethylsilyl)oxy)-1-cyclopropylpropan-1-ol (147 mg, 78.01% yield) as a colorless oil.

¹H NMR (400 MHz, Chloroform-*d*) δ 3.96-3.87 (m, 1H), 3.80 (ddd, *J* = 10.1, 7.0, 6.0 Hz, 1H), 3.11 (dt, *J* = 8.3, 5.8 Hz, 1H), 1.85-1.78 (m, 2H), 0.89 (s, 9H), 0.89-0.86 (m, 1H), 0.57-0.41 (m, 2H), 0.41-0.33 (m, 1H), 0.21-0.13 (m, 1H), 0.07 (d, *J=* 2.9 Hz, 6H).

### Step 4: Preparation of compounds LA-30A-al and LA-30A-a2

LY-27a (200 mg, 415.33 µmol, 1 eq) and TsOH (21.46 mg, 124.60 µmol, 0.3 eq) were added to a solution of 3-((tert-butyldimethylsilyl)oxy)-1-cyclopropylpropan-1-ol (143.55 mg, 622.99 µmol, 1.5 eq) in THF (3 mL) at room temperature. After the addition, the reaction solution was allowed to react at 33°C for 2 hours. LCMS analysis showed that the reaction ended. The reaction solution was evaporated to dryness to afford the crude product, and the crude product was purified by high-performance liquid chromatography (preparative chromatograph manufacturer: Shimadzu, model: LC-20AP, column: Welch Xtimate C18 150 × 21.2 mm × 5 µm, mobile phase: water (0.225% FA)-acetonitrile, with an elution ratio of acetonitrile increased from 40% to 70%) and lyophilized, to afford compound LA-30A-a1 (the structure was confirmed by 2D NMR) (51 mg, 22.84% yield) and LA-30A-a2 (10 mg, 4.48% yield) as a white solid.

LA-30A-a1: LCMS (ESI): m/z, 560.4[M+Na]⁺. Rt = 2.132 min.

¹H NMR (400 MHz, Acetonitrile-*d³*) δ 7.84 (d, *J* = 7.6 Hz, 2H), 7.70 (d, *J =* 7.6 Hz, 2H), 7.46-7.31 (m, 5H), 6.99 (s, 1H), 5.98 (d, *J =* 7.9 Hz, 1H), 4.58-4.51 (m, 2H), 4.39 (dd, *J =* 10.3, 7.1 Hz, 1H), 4.34-4.21 (m, 3H), 3.90 (t, *J =* 6.7 Hz, 1H), 3.50 (q, *J =* 5.8 Hz, 2H), 2.90 (q, *J =* 7.5, 7.0 Hz, 1H), 2.11-2.02 (m, 2H), 1.78-1.57 (m, 3H), 1.29 (t, *J =* 7.7 Hz, 4H), 0.92 (dd, *J =* 15.3, 6.8 Hz, 6H), 0.77 (dd, *J =* 8.2, 5.0 Hz, 1H), 0.38 (dd, *J =* 8.2, 1.8 Hz, 2H), 0.25-0.19 (m, 1H), 0.12 (d, *J =* 7.0 Hz, 1H).

LA-30A-a2: LCMS (ESI): m/z. 560.4[M+Na]⁺. Rt = 2.156 min.

¹H NMR (400 MHz, Acetonitrile-*d³*) δ 7.88-7.79 (m, 2H), 7.70 (d, *J* = 7.5 Hz, 2H), 7.42 (t, *J* = 7.4 Hz, 3H), 7.34 (td, *J* = 7.4, 1.2 Hz, 2H), 7.04 (s, 1H), 6.02 (d, *J* = 7.5 Hz, 1H), 4.73 (dd, *J* = 10.3, 7.0 Hz, 1H), 4.61 (dt, *J =* 10.3, 6.3 Hz, 1H), 4.39 (dd, *J =* 10.1, 6.9 Hz, 1H), 4.33-4.21 (m, 3H), 3.89 (q, *J =* 6.1 Hz, 1H), 3.51 (q, *J =* 6.9, 5.4 Hz, 2H), 2.93 (td, *J =* 8.9, 4.5 Hz, 2H), 2.12-2.04 (m, 1H), 1.74-1.55 (m, 2H), 1.29 (dd, *J =* 7.1, 1.3 Hz, 3H), 1.00-0.83 (m, 6H), 0.75 (dtd, *J =* 8.2, 5.2, 2.3 Hz, 1H), 0.52-0.43 (m, 2H), 0.42-0.33 (m, 1H), 0.01 (dq, *J* = 7.8, 4.7 Hz, 1H).

### Step 5: Preparation of compound LA-30A-b

DIPEA (98.07 mg, 125.41 µL, 758.84 µmol, 8 eq) and NPC (173.14 mg, 115.42 µL, 569.13 µmol, 6 eq) were added to a solution of compound LA-30A-a1 (51 mg, 94.86 µmol, 1 eq) in DMF (2 mL) at 0°C. After the addition, the mixture was allowed to react at 25°C for 16 hours. LCMS analysis showed that the reaction ended. The reaction solution was evaporated to dryness to afford the crude product, and the crude product was purified by silica gel column chromatography (eluents: ethyl acetate/n-hexane = 1:20 to 1:4; and dichloromethane/methanol =1:20 to 1:10) to afford compound LA-30A-b as a yellow solid (17 mg; the yield was not calculated for the crude product).

LCMS (ESI): m/z, 725.5[M+Na]⁺. Rt = 3.002 min.

### Step 6: Preparation of compounds LA-30A-c1 and LA-30A-c2

DIPEA (3.13 mg, 4 µL, 24.21 µmol, 4 eq) and HOBt (817.91 µg, 5.45e⁻¹ µL, 6.05 µmol, 1 eq) were added to a solution of compound LA-30A-b (5.10 mg, 7.26 µmol, 1.2 eq) and eribulin mesylate (5 mg, 6.05 µmol, 1 eq) in DMF (2 mL) at 0°C. After the addition, the reaction solution was allowed to react at 25°C for 16 hours. LCMS analysis showed that the reaction ended. The reaction solution and another batch of the reaction solution at the same scale were combined and purified by preparative high-performance liquid chromatography (preparative chromatograph manufacturer: Shimadzu, model: LC-20AP, column: Welch Xtimate C18 150 × 21.2 mm × 5 µm, mobile phase: water (0.225% FA)-acetonitrile, with an elution ratio of acetonitrile increased from 60% to 90%) and lyophilized, to afford compounds LA-30A-c1 (5 mg, 31.93% yield) and LA-30A-c2 (5 mg, 31.93% yield) as white solids (compounds LA-30A-c1 and LA-30A-c2 are diastereomers; and it is assumed that LA-30A-c1 has the R-configuration (at the three-membered ring) and LA-30A-c2 has the S-configuration (at the three-membered ring)).

LA-30A-c1: LCMS (ESI): m/z, 1315.8[M+Na]⁺. Rt = 3.205 min.

LA-30A-c2: LCMS (ESI): m/z, 1315.8[M+Na]⁺. Rt = 3.248 min.

### Step 7: Preparation of compound LA-30A-d1

DBU (1.77 mg, 1.73 µL, 11.60 µmol, 3 eq) was added to a solution of compound LA-30A-c1 (5 mg, 3.87 µmol, 1 eq) in DMF (1 mL) at 0°C. After the addition, the reaction solution was allowed to react at 25°C for 1 hour. LCMS analysis showed that the reaction ended. The reaction was quenched by adding TsOH (2 mg, 11.60 µmol, 3 eq) to the reaction solution at 0°C, and the mixture was used directly in the next step.

LCMS (ESI): m/z, 1071.7[M+H]⁺. Rt = 1.855 min.

### Step 8: Preparation of compound LA-30A-1

DIPEA (2.03 mg, 2.59 µL, 15.68 µmol, 4 eq) and HATU (1.79 mg, 4.70 µmol, 1.2 eq) were added to the solution of compounds LA-30A-d1 (4.20 mg, 3.92 µmol, 1 eq) and LY-22CD-3-d (3.12 mg, 3.92 µmol, 1 eq) in DMF (2mL) at 0°C. After addition, the reaction solution was allowed to react at 25°C for 30 minutes. LCMS analysis showed that the reaction ended. The reaction solution was purified by preparative high-performance liquid chromatography (preparative chromatograph manufacturer: Orienda, model: BRIX-2860, column: Welch Xtimate C18 150 × 21.2 mm × 5 µm, mobile phase: water (0.225% HCOOH)-acetonitrile, with an elution ratio of acetonitrile increased from 30% to 60%) and lyophilized, to afford compound LA-30A-1 (2.20 mg, 30.33% yield, 98% purity) as a white solid.

LCMS (ESI): m/z, 1873.1[M+Na]⁺. Rt = 2.304 min. HPLC: Rt=2.660 min, 98.91%.

### Step 9: Preparation of compound LA-30A-d2

DBU (1.77 mg, 1.73 µL, 11.60 µmol, 3 eq) was added to a solution of compound LA-30A-c2 (5 mg, 3.87 µmol, 1 eq) in DMF (1 mL) at 0°C. After the addition, the reaction solution was allowed to react at 25°C for 1 hour. LCMS analysis showed that the reaction ended. The reaction was quenched by adding TsOH (2 mg, 11.60 µmol, 3 eq) to the reaction solution at 0°C, and the mixture was used directly in the next step.

LCMS (ESI): m/z, 1071.8[M+H]⁺. Rt = 1.876 min.

### Step 10: Preparation of compound LA-30A-2

DIPEA (2.03 mg, 2.59 µL, 15.68 µmol, 4 eq) and HATU (1.79 mg, 4.70 µmol, 1.2 eq) were added to the solution of compounds LA-30A-d2 (4.20 mg, 3.92 µmol, 1 eq) and LY-22CD-3-d (3.44 mg, 4.31 µmol, 1.1 eq) in DMF (2mL) at 0°C. After addition, the reaction solution was allowed to react at 25°C for 30 minutes. LCMS analysis showed that the reaction ended. The reaction solution was purified by preparative high-performance liquid chromatography (preparative chromatograph manufacturer: Orienda, model: BRIX-2860, column: Welch Xtimate C18 150 × 21.2 mm × 5 µm, mobile phase: water (0.225% HCOOH)-acetonitrile, with an elution ratio of acetonitrile increased from 30% to 60%) and lyophilized, to afford compound LA-30A-2 (1.75 mg, 24.13% yield, 98% purity) as a white solid.

LCMS (ESI): m/z, 1873.2[M+Na]⁺. Rt = 2.317 min. HPLC: Rt=2.680 min, 100.0%.

### Example 46: Preparation of Compounds LA-30B-P1 and LA-30B-P2

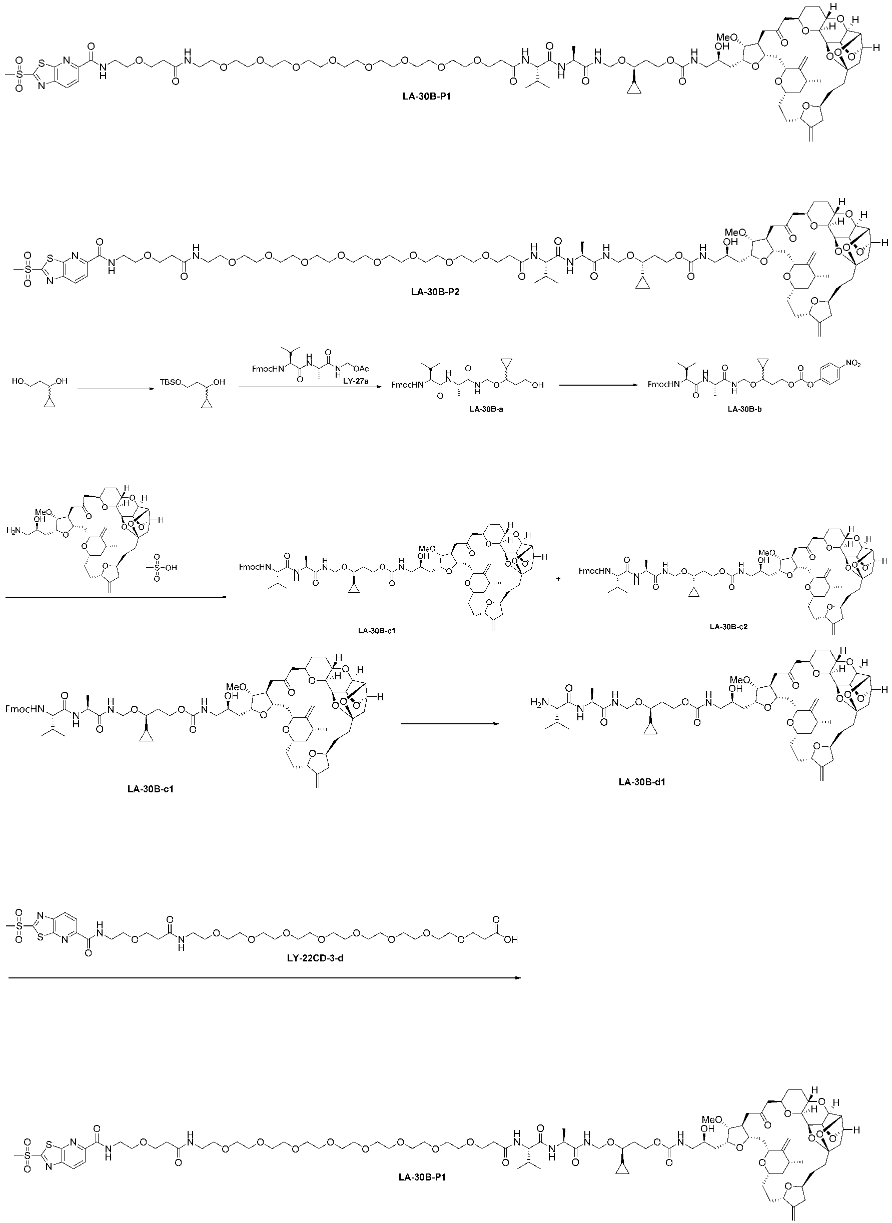

### Step 1: Preparation of 3-((tert-butyldimethylsilyl)oxy)-1-cyclopropylpropan-1-ol

1-cyclopropylpropane-1,3-diol (580 mg, 5 mmol, 1 eq) was dissolved in DCM (8 mL), the mixture was cooled to 0°C, then imidazole (340 mg, 5 mmol, 1 eq) and TBSCl (829 mg, 953 µL, 5.50 mmol, 1.1 eq) were added. The reaction solution was allowed to react at 25 C for 16 hours. The reaction solution was added into 100 mL of water at 0°C, then 100 mL of saturated aqueous NaHCO₃ solution was added, and the mixture was stirred for 5 minutes. The aqueous phase was extracted with DCM (100 mL × 3). The organic phases were combined, washed with brine, dried over anhydrous Na₂SO₄, filtered, and concentrated to afford 3-((tert-butyldimethylsilyl)oxy)-1-cyclopropylpropan-1-ol (620 mg, 53.8% yield) as a yellow oil.

¹H NMR (400 MHz, Chloroform-*d*): δ 3.91 (m, 1H), 3.80 (m, 1H), 3.11 (m, 1H), 1.81 (q, *J =* 6.4, 5.6 Hz, 2H), 0.89 (s, 9H), 0.86 (s, 1H), 0.57-0.48 (m, 1H), 0.48-0.43 (m, 1H), 0.40-0.33 (m, 1H), 0.16 (ddd, *J* = 8.8, 3.6, 1.2 Hz, 1H), 0.07 (s, 6H).

### Step 2: Preparation of compound LA-30B-a

Compound LY-27a (866 mg, 1.80 mmol, 1 eq) was dissolved in THF (10 mL), and then 3-((tert-butyldimethylsilyl)oxy)-1-cyclopropylpropan-1-ol (620 mg, 2.70 mmol, 1.5 eq) and TsOH (93 mg, 86.9 µL, 540 µmol, 0.3 eq) were added. The reaction solution was allowed to react at 33°C for 1 hour. LCMS analysis showed that the reaction ended. The reaction solution was concentrated to dryness and subjected to preparative high-performance liquid chromatography (preparative chromatograph manufacturer: Orienda, model: BRIX-2860, column: Phenomenex Luna C18 250 × 50 mm × 10 µm, mobile phase: water (0.225% HCOOH)-acetonitrile, with an elution ratio of acetonitrile increased from 10% to 50%), to afford LA-30B-a (60 mg, 6.2% yield) as a white solid.

LCMS (ESI): m/z, 560.4 [M+Na]⁺, Rt = 2.165 min.

### Step 3: Preparation of compound LA-30B-b

Compound LA-30B-a (60 mg, 111 µmol, 1 eq) was dissolved in DMF (2 mL), and NPC (169 mg, 557 µmol, 5 eq) and DIPEA (86.5 mg, 110 µL, 669 µmol, 6 eq) were then added. The reaction solution was allowed to react at 35°C for 2 hours. LCMS analysis showed that the reaction ended. The reaction solution was concentrated to dryness to afford the crude product, and the crude product was purified by silica gel column chromatography (eluents: ethyl acetate/petroleum ether = 1:20 to 1:4; and methanol/dichloromethane = 1:20 to 1:10) to obtain LA-30B-b (45 mg, 57.4% yield) as a yellow oil.

LCMS(ESI): m/z, 725.4 [M+Na]⁺, Rt = 3.029 min.

### Step 4: Preparation of compounds LA-30B-c1 and LA-30B-c2

Compound LA-30B-b (12.6 mg, 18 µmol, 1.5 eq) was dissolved in DMAC (1 mL), the mixture was cooled to 0°C, and then eribulin mesylate (9.9 mg, 12 µmol, 1 eq) and DIPEA (4.6 mg, 5.9 µL, 36 µmol, 3 eq) were added. The reaction solution was allowed to react at 25°C for 30 minutes. LCMS analysis showed that the reaction ended. The reaction solution was purified by preparative high-performance liquid chromatography (preparative chromatograph manufacturer: Orienda, model: BRIX-2860, column: Phenomenex Luna C18 250 × 50 mm × 10 µm, mobile phase: water (0.225% HCOOH)-acetonitrile, with an elution ratio of acetonitrile increased from 60% to 90%), to afford LA-30B-c1 (3 mg, 19.3% yield) and LA-30B-c2 (3 mg, 19.3% yield) as white solids (LA-30B-c1 and LA-30B-c2 are diastereomers; and it is assumed that LA-30B-c1 has the S-configuration at the three-membered ring, and LA-30B-c2 has the R-configuration at the three-membered ring).

LA-30B-c1: LCMS (ESI): m/z, 1315.7 [M+Na]⁺, Rt = 3.220 min.

LA-30B-c2: LCMS (ESI): m/z, 1315.7 [M+Na]⁺, Rt = 3.264 min.

### Step 5: Preparation of compound LA-30B-d1

Compound LA-30B-c1 (3 mg, 2.32 µmol, 1 eq) was dissolved in DMF (500 µL), the mixture was cooled to 0°C, and then DBU (1.06 mg, 6.96 µL, 3 eq) was added. The reaction solution was allowed to react at 25°C for 30 minutes. The LCMS analysis showed that the reaction ended. TsOH (1.20 mg, 6.96 µmol, 3 eq) was added to the reaction solution, and the mixture was allowed to react for 2 minutes. The reaction solution was directly used in the next step without further treatment.

LCMS (ESI): m/z, 1071.7 [M+H]⁺, Rt = 1.850 min.

### Step 6: Preparation of compound LA-30B-P1

Compound LY-22CD-3-d (3.72 mg, 4.67 µmol, 1 eq) was dissolved in DMF (500 µL), the mixture was cooled to 0°C, and then compound LA-30B-d1 (5 mg, 4.67 µmol, 1 eq), DIPEA (1.81 mg, 2.31 µL, 14 µmol, 3 eq) and HATU (1.77 mg, 4.67 µmol, 1 eq) were added. The reaction solution was allowed to react at 25°C for 30 minutes. The LCMS analysis showed that the reaction ended. The reaction solution was purified by preparative high-performance liquid chromatography (preparative chromatograph manufacturer: Orienda, model: BRIX-2860, column: Phenomenex Luna C18 250 × 50 mm × 10 µm, mobile phase: water (0.225% HCOOH)-acetonitrile, with an elution ratio of acetonitrile increased from 30% to 60%), to afford a white solid LA-30B-P1 (1.0 mg, 10.6% yield, 91.27% purity).

LCMS (ESI): m/z, 1873.0 [M+Na]⁺, Rt = 2.280 min.

### Step 7: Preparation of compound LA-30B-d2

Compound LA-30B-c2 (3 mg, 2.32 µmol, 1 eq) was dissolved in DMF (500 µL), the mixture was cooled to 0°C, and then DBU (1.06 mg, 6.96 µL, 3 eq) was added. The reaction solution was allowed to react at 25°C for 30 minutes. The LCMS analysis showed that the reaction ended. TsOH (1.20 mg, 6.96 µmol, 3 eq) was added to the reaction solution, and the mixture was allowed to react for 2 minutes. The reaction solution was directly used in the next step without further treatment.

LCMS (ESI): m/z, 1071.7 [M+H]⁺, Rt = 1.863 min.

### Step 8: Preparation of compound LA-30B-P2

Compound LY-22CD-3-d (2.98 mg, 3.73 µmol, 1 eq) was dissolved in DMF (500 µL), the mixture was cooled to 0°C, and then compound LA-30B-d2 (4 mg, 3.73 µmol, 1 eq), DIPEA (1.45 mg, 1.85 µL, 11.20 µmol, 3 eq) and HATU (1.42 mg, 3.73 µmol, 1 eq) were added. The reaction solution was allowed to react at 25°C for 30 minutes. The LCMS analysis showed that the reaction ended. The reaction solution was purified by preparative high-performance liquid chromatography (preparative chromatograph manufacturer: Orienda, model: BRIX-2860, column: Phenomenex Luna C18 250 × 50 mm × 10 µm, mobile phase: water (0.225% HCOOH)-acetonitrile, with an elution ratio of acetonitrile increased from 30% to 60%), to afford a white solid LA-30B-P2 (1.24 mg, 17.9% yield, 90.37% purity).

LCMS (ESI): m/z, 1872.2 [M+Na]⁺, Rt = 2.288 min.

### Example 47: Preparation of Compound LA-31A

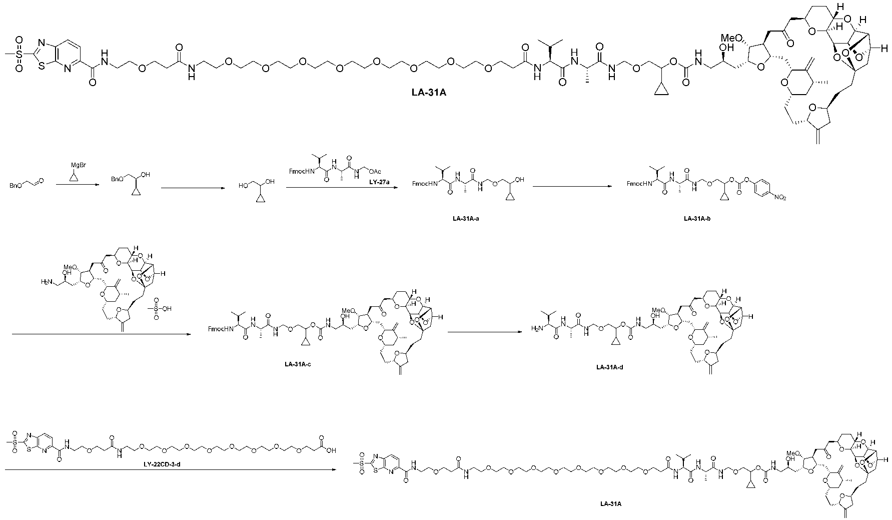

### Step 1: Preparation of Compound 2-(benzyloxy)-1-cyclopropyl-1-ethanol

Cyclopropylmagnesium bromide (47.95 mL, 23.97 mmol, 1.2 eq, 0.5 M) was added to a solution of the compound benzyloxyacetaldehyde (3g, 19.98 mmol, 1 eq) in THF (25 mL) at room temperature, the reaction solution was stirred at 70°C for 2 hours, and LCMS analysis indicated complete consumption of the starting material. A saturated ammonium chloride solution (10 mL) and water (20 mL) were slowly added to the reaction solution under an ice bath, the aqueous phase was extracted with ethyl acetate (20 mL) three times, the organic phases were combined, washed with saturated brine (20 mL) once, dried with anhydrous sodium sulfate, filtered and concentrated, and the residue was purified by silica gel column chromatography (eluents: ethyl acetate/petroleum ether = 1:5-1:3) to obtain colorless oily 2-(benzyloxy)-1-cyclopropyl-1-ethanol (1.2g, yield: 31.22%).

LCMS (ESI): m/z, 216[M+Na]⁺, Rt=1.442. ¹H NMR (400 MHz, Chloroform-*d*) δ 7.39-7.28 (m, 5H), 4.58 (s, 2H), 3.64 (dd, *J =* 9.6, 3.0 Hz, 1H), 3.48 (dd, *J =* 9.6, 8.2 Hz, 1H), 3.12 (td, *J* = 8.2, 3.0 Hz, 1H), 2.36 (s, 1H), 0.87 (qt, *J* = 8.2, 4.8 Hz, 1H), 0.60-0.43 (m, 2H), 0.38 (dtd, *J* = 9.2, 5.2, 4.2 Hz, 1H), 0.25-0.18 (m, 1H).

### Step 2: Preparation of Compound 1-cyclopropylethane-1,2-diol

Palladium on carbon (60 mg) was added to the solution of 2-(benzyloxy)-1-cyclopropyl-1-ethanol (600 mg, 3.12 mmol, 1 eq) in MeOH (6 mL) and concentrated hydrochloric acid HCl (56 µL), hydrogen displacement was performed three times, and the reaction solution was stirred at room temperature for 2 hours. TLC indicated complete consumption of the starting material. Palladium on carbon was filtered out with diatomaceous earth and the filter cake was washed with methanol (3 mL). The solution was concentrated to afford 1-cyclopropylethane-1,2-diol (320 mg, yield: 99%) as a colorless oil.

¹H NMR (400 MHz, Chloroform-d) δ 3.77 (d, *J* = 10.8 Hz, 1H), 3.61 (dd, *J* = 11.2, 7.2 Hz, 1H), 3.02 (t, *J =* 8.0 Hz, 1H), 1.01 - 0.84 (m, 1H), 0.55 (d, *J* = 7.8 Hz, 2H), 0.36 (d, *J* = 9.6 Hz, 1H), 0.26 (dd, *J* = 9.6, 4.6 Hz, 1H).

### Step 3: Preparation of compound LA-31A-a

P-toluenesulfonic acid (359.70 mg, 2.09 mmol, 1 eq) was added to the solution of 1-cyclopropylethane-1,2-diol (320 mg, 3.13 mmol, 1.5 eq) and LY-27a (1.01g, 2.09 mmol, 1 eq) in THF (20 mL), a resulting solution was stirred at 30°C for 2 hours and concentrated, the residue was dissolved in DMF, the mixture was filtered, and the filter cake was subjected to preparative high-performance liquid chromatography (preparative chromatograph manufacturer: Orienda, model: BRIX-2860, column: Welch Xtimate C18 150 × 30 mm × 10 µm, mobile phase: water (0.225% HCOOH)-acetonitrile, with an elution ratio of acetonitrile increased from 30% to 60%), to afford LA-31A-a (515.70 mg, 47.15% yield) as a white solid.

LCMS (ESI): m/z, 546[M+Na]⁺, Rt=2.058 min.

### Step 4: Preparation of compound LA-31A-b

DIPEA (1.10g, 1.41 mL, 8.50 mmol, 8 eq) and NPC (1.94g, 6.38 mmol, 6 eq) were added to the solution of compound LA-31A-a (556.50 mg, 1.06 mmol, 1 eq) in DMF (8 mL), the reaction solution was stirred at 35°C for 2 hours, the solution was concentrated by an oil pump, and the residue was purified by silica gel column chromatography (eluents: ethyl acetate/petroleum ether = 0%-50%; and methanol/dichloromethane = 0%-8%), to afford LA-31A-b (161.40 mg, 50% purity) as a yellow oil.

LCMS (ESI): m/z, 711[M+Na]⁺, Rt=1.953 min.

### Step 5: Preparation of compound LA-31A-c

DIPEA (6.26 mg, 48.43 µmol, 4 eq) and HOBT (1.64 mg, 12.11 µmol, 1 eq) were added to the solution of compound LA-31A-b (20.01 mg, purity: 50%, 14.53 µmol, 1.2 eq) and Eribulin mesylate (10 mg, 12.11 µmol, 1 eq) in DMF (1 mL), the reaction solution was stirred for 1 hour at room temperature, and the LCMS analysis indicated complete consumption of the starting material. The reaction solution was purified by preparative high-performance liquid chromatography (preparative chromatograph manufacturer: Orienda, model: BRIX-2860, column: Welch Xtimate C18 150 × 30 mm × 10 µm, mobile phase: water (0.225% HCOOH)-acetonitrile, with an elution ratio of acetonitrile increased from 50% to 80%), to afford LA-31A-c (6.40 mg, 41.32% yield) as a white solid.

LCMS (ESI): m/z, 1301[M+Na]⁺. Rt = 3.168 min.

### Step 6: Preparation of compound LA-31A-d

DBU (2.28 mg, 15.01 µmol, 3 eq) was added to the solution of compound LA-31A-c (6.40 mg, 5 µmol, 1 eq) in DMF (1 mL) under an ice bath, the reaction solution was stirred at room temperature for 1 hour, LCMS analysis indicated complete consumption of the starting material, then p-toluenesulfonic acid (2.58 mg, 15.01 µmol, 3 eq) was added under an ice bath and a resulting mixture was directly used for the next step. A theoretical mass of LA-31A-d was 5.29 mg.

LCMS (ESI): m/z, 1057[M+H]⁺, Rt=1.799 min.

### Step 7: Preparation of compound LA-31A

DIPEA (2.59 mg, 20.01 µmol, 4 eq) and HATU (2.28 mg, 6 µmol, 1.2 eq) were added to the solution of compounds LA-31A-d (5.29 mg, 5 µmol, 1 eq) and LY-22CD-3-d (4.78 mg, 6 µmol, 1.2 eq) in DMF (1 mL) under the ice bath, the reaction solution was stirred for 1 hour at room temperature, and the LCMS analysis showed complete consumption of the starting material. The reaction solution was purified by preparative high-performance liquid chromatography (preparative chromatograph manufacturer: Orienda, model: BRIX-2860, column: Phenomenex Luna C18 150 × 25mm × 10 µm, mobile phase: water (0.225% HCOOH)-acetonitrile, with an elution ratio of acetonitrile increased from 30% to 60%), to afford LA-31A (4.32 mg, 47.0% yield) as a white solid.

LCMS (ESI): m/z, 1859[M+Na]⁺, Rt=2.230 min.

### Example 48: Preparation of Compound LA-31B

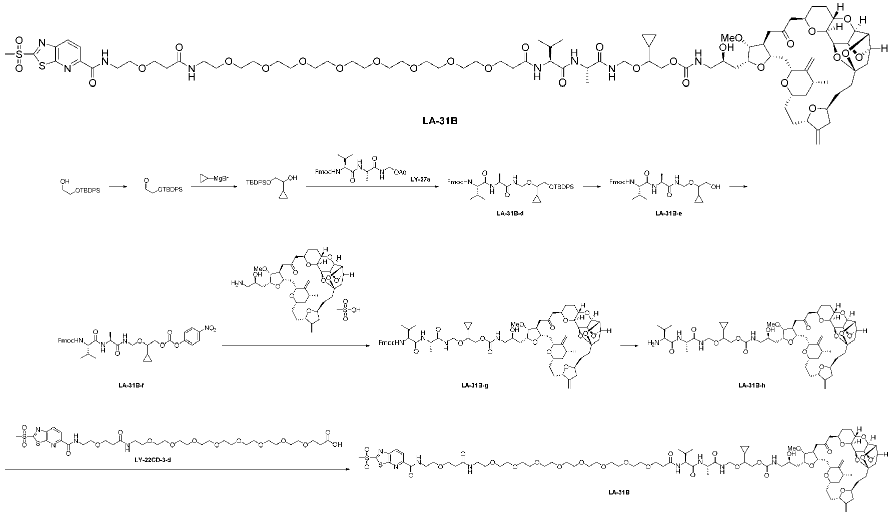

### Step 1: Preparation of 2-((tert-butyldiphenylsilyl)oxy)acetaldehyde

A solution of DMSO (1.68g, 21.45 mmol, 2.2 eq) in DCM (30 mL) was added to a solution of oxalyl chloride (2.72g, 21.45 mmol, 2.2 eq) in DCM (90 mL) dropwise at -78°C. After 5 minutes, a solution of 2-((tert-butyldiphenylsilyl)oxy)ethan-1-ol (2.93g, 9.75 mmol, 1 eq) in DCM (30 mL) was added dropwise, and the mixture was stirred at -65°C for 20 minutes. Triethylamine (5.92g, 58.51 mmol, 6 eq) was added dropwise at -65°C, and the mixture was stirred at room temperature for 30 minutes. TLC analysis showed that the reaction ended. Saturated aqueous NH₄Cl solution (30 mL) and water (30 mL) were added to the reaction solution dropwise at 0°C. The mixture was extracted with DCM (100 mL × 3). The organic phases were combined, washed with saturated brine (50 mL), dried over Na₂SO₄, filtered, and evaporated to dryness under reduced pressure to afford the product 2-((tert-butyldiphenylsilyl)oxy)acetaldehyde (3g; the yield was not calculated for the crude product) as a colorless oil.

¹H NMR (400 MHz, DMSO-*d*₆) δ 9.55 (s, 1H), 7.64-7.61 (m, 4H), 7.49-7.42 (m, 6H), 4.41 (s, 2H), 1.02 (s, 9H).

### Step 2: Preparation of 2-((tert-butyldiphenylsilyl)oxy)-1-cyclopropylethan-1-ol

Cyclopropylmagnesium bromide (50.26 mL, 1 M) was added to a solution of 2-((tert-butyldiphenylsilyl)oxy)acetaldehyde (3g, crude) in THF (60 mL) dropwise at 0°C. The reaction solution was stirred at room temperature for 2 hours under nitrogen atmosphere. TLC analysis showed that the reaction ended. Saturated aqueous NH₄Cl solution (20 mL) was slowly added to the mixture dropwise at 0°C to quench the reaction, followed by water (100 mL). The mixture was extracted with ethyl acetate (100 mL × 3). The organic phases were combined, washed with saturated brine (50 mL), dried over Na₂SO₄, filtered, and evaporated to dryness under reduced pressure to afford the product 2-((tert-butyldiphenylsilyl)oxy)-1-cyclopropylethan-1-ol (3.56g; the yield was not calculated for the crude product) as a colorless oil.

### Step 3: Preparation of compound LA-31B-d

TsOH (151.70 mg, 880.95 µmol, 0.3 eq) was added to a solution of compound LA-31B-c (1.80g, crude) and LY-27a (1.41g, 2.94 mmol, 1 eq) in THF (30 mL), and the mixture was stirred at 33°C for 1 hour. LCMS analysis showed that the reaction ended. The mixture was filtered and evaporated to dryness under reduced pressure. The residue was purified by preparative high-performance liquid chromatography (preparative chromatograph manufacturer: Orienda, model: BRIX-2860-R1, column: R-220, mobile phase: water (0.225% HCOOH)-acetonitrile, with an elution ratio of acetonitrile increased from 10% to 90%), to afford product LA-31B-d (405 mg, 18.10% yield) as a white solid. LCMS (ESI): m/z, 784.5[M+Na]⁺, Rt=3.241 min.

### Step 4: Preparation of compound LA-31B-e

Et₃N.3HF (3.43g, 21.26 mmol, 40 eq) was added to a solution of compound LA-31B-d (405 mg, 531.47 µmol, 1 eq) in THF (10 mL), and the mixture was stirred at 65°C for 2 hours. LCMS analysis showed that the reaction ended. The residue was purified by silica gel column chromatography (C18, ISCO, R-120g SepaFlash Silica Flash Column, eluents: 10%-50% water/CH₃CN, 100 mL/min) and lyophilized to afford the product LA-31B-e (163 mg, 58.57% yield) as a white solid. LCMS (ESI): m/z, 546.4[M+Na]⁺, Rt=2.097min.

### Step 5: Preparation of compound LA-31B-f

DIPEA (123.65 mg, 956.78 µmol, 3 eq) was added to a solution of compound LA-31B-e (167 mg, 318.93 µmol, 1 eq) and NPC (194.04 mg, 637.85 µmol, 2 eq) in DMF (3 mL) and the reaction solution was stirred at 35°C for 2 hours. LCMS analysis showed that the reaction ended. The reaction solution was evaporated to dryness under reduced pressure. The residue was purified by silica gel column chromatography (eluents: methanol/dichloromethane = 0:1 to 1:10) to afford the product LA-31B-f (106 mg, 48.26% yield) as a white solid. LCMS (ESI): m/z, 711.4 [M+Na]⁺, Rt=2.941min.

### Step 6: Preparation of compound LA-31B-g

DIPEA (4.69 mg, 36.32 µmol, 3 eq) was added to a solution of compound LA-31B-f (10 mg, 12.11 µmol, 1 eq) and eribulin mesylate (10.01 mg, 14.53 µmol, 1.2 eq) in DMF (2 mL) at 0°C. The reaction solution was stirred at room temperature under nitrogen atmosphere for 2 hours. LCMS analysis showed that the reaction ended. The mixture was purified by preparative high-performance liquid chromatography (preparative chromatograph manufacturer: Orienda, model: BRIX-2860, column: Welch Xtimate C18 150 × 30 mm × 10 µm, mobile phase: water (0.225% HCOOH)-acetonitrile, with an elution ratio of acetonitrile increased from 50% to 80%), to afford product LA-31B-g (7.80 mg, 50.35% yield) as a white solid. LCMS (ESI): m/z, 1301.8 [M+Na]⁺, Rt=3.169min.

### Step 7: Preparation of compound LA-31B-h

DBU (2.78 mg, 18.29 µmol, 3 eq) was added to a solution of compound LA-31B-g (7.80 mg, 6.10 µmol, 1 eq) in DMF (2 mL) at 0°C, and the mixture was stirred at room temperature for 1 hour. LCMS analysis showed that the reaction ended. Methanesulfonic acid (3.15 mg, 18.29 µmol, 3 eq) was added to the reaction solution to quench the reaction, to afford LA-31B-h (6.45 mg, crude in DMF solution), which was used directly in the next step.

### Step 8: Preparation of compound LA-31B

DIPEA (3.94 mg, 30.50 µmol, 5 eq) and HATU (2.78 mg, 7.32 µmol, 1.2 eq) were added to a solution of compound LA-31B-h (6.45 mg, crude) and LY-22CD-3-d (5.35 mg, 6.71 µmol, 1.1 eq) in DMF (2 mL) at 0°C, the reaction solution was stirred for 2 hours at room temperature, and the LCMS analysis showed complete consumption of the starting material. The mixture was purified by preparative high-performance liquid chromatography (preparative chromatograph manufacturer: Orienda, model: BRIX-2860, column: YMC-Triart Prep C18 200 × 30 mm × 10 µm, mobile phase: water (0.225% HCOOH)-acetonitrile, with an elution ratio of acetonitrile increased from 30% to 60%), to obtain LA-31B (4.10 mg, 36.60% yield) as a white solid. LCMS (ESI): m/z, 1859.0[M+Na]⁺, Rt=2.228 min.

### Example 49: Preparation of Compound LA-32AS

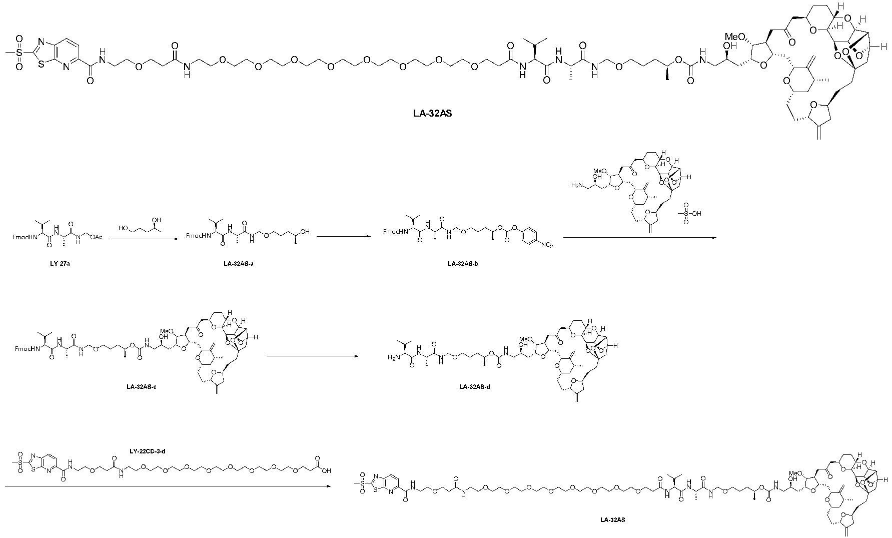

### Step 1: Preparation of compound LA-32AS-a

P-toluenesulfonic acid (107.28 mg, 622.99 µmol, 1 eq) was added to the solution of (2S)-2-methylbutane-1,4-diol (194.65 mg, 1.87 mmol, 3 eq) and LY-27a (300 mg, 622.99 µmol, 1 eq) in THF (5 mL), a resulting solution was stirred at 30°C for 2 hours and concentrated, the residue was dissolved in DMF, the mixture was filtered, and the filter cake was subjected to preparative high-performance liquid chromatography (preparative chromatograph manufacturer: Shimadzu, model: LC-20AP, column: Welch Xtimate C18 150 × 30 mm × 10 µm, mobile phase: water (0.225% HCOOH)-acetonitrile, with an elution ratio of acetonitrile increased from 30% to 60%), to afford LA-32AS-a (300 mg, 91.61% yield) as a white solid.

LCMS (ESI): m/z, 548[M+Na]⁺, Rt=2.023.

### Step 2: Preparation of compound LA-32AS-b

DIPEA (442.56 mg, 565.93 µL, 3.42 mmol, 6 eq) and NPC (694.48 mg, 2.28 mmol, 4 eq) were added to the solution of compound LA-32AS-a (300 mg, 570.72 µmol, 1 eq) in DMF (8 mL), the reaction solution was stirred at 35°C for 2 hours, the solution was concentrated by an oil pump, and the residue was purified by silica gel column chromatography (eluents: ethyl acetate/petroleum ether = 0%-50%; and methanol/dichloromethane = 0%-8%), to afford LA-32AS-b (175.80 mg, 44.59% purity) as a yellow oil.

LCMS (ESI): m/z, 713[M+Na]⁺, Rt=2.943.

### Step 3: Preparation of compound LA-32AS-c

DIPEA (4.69 mg, 6 µL, 36.32 µmol, 3 eq) and HOBT (1.64 mg, 12.11 µmol, 1 eq) were added to the solution of compound LA-32AS-b (10.03 mg, 14.53 µmol, 1.2 eq) and eribulin mesylate (10 mg, 12.11 µmol, 1 eq) in DMF (1 mL), the reaction solution was stirred for 1 hour at room temperature, and the LCMS analysis indicated complete consumption of the starting material. The reaction solution was purified by preparative high-performance liquid chromatography (preparative chromatograph manufacturer: Orienda, model: BRIX-2860, column: Welch Xtimate C18 250 × 30 mm × 10 µm, mobile phase: water (0.225% HCOOH)-acetonitrile, with an elution ratio of acetonitrile increased from 60% to 90%), to afford LA-32AS-c (11.20 mg, 72.19% yield) as a white solid.

LCMS (ESI): m/z, 1303[M+Na]⁺, Rt=3.113.

### Step 4: Preparation of compound LA-32AS-d

Et₂NH (3.20 mg, 4.50 µL, 43.70 µmol, 5 eq) was added to the solution of compound LA-32AS-c (11.20 mg, 8.74 µmol, 1 eq) in DMF (1 mL) under an ice bath, the reaction solution was stirred at room temperature for 1 hour, LCMS analysis indicated complete consumption of the starting material, the reaction solution was dried via an oil pump and the residue was directly used in the next step. A theoretical mass of LA-32AS-d was 9.26 mg.

LCMS (ESI): m/z, 1059[M+H]⁺, Rt=1.782.

### Step 5: Preparation of compound LA-32AS

DIPEA (2.26 mg, 2.89 µL, 17.48 µmol, 2 eq) and HATU (3.99 mg, 10.49 µmol, 1.2 eq) were added to the solution of compounds LA-32AS-d (9.26 mg, 8.74 µmol, 1 eq) and LY-22CD-3-d (8.36 mg, 10.49 µmol, 1.2 eq) in DMF (1 mL) under the ice bath, the reaction solution was stirred for 1 hour at room temperature, and the LCMS analysis showed complete consumption of the starting material. The reaction solution was purified by preparative high-performance liquid chromatography (preparative chromatograph manufacturer: Orienda, model: BRIX-2860, column: Welch Xtimate C18 250 × 30mm × 10 µm, mobile phase: water (0.225% HCOOH)-acetonitrile, with an elution ratio of acetonitrile increased from 30% to 60%), to afford LA-32AS (7.22 mg, 44.93% yield) as a white solid.

LCMS (ESI): m/z, 1860[M+Na]⁺, Rt=2.206. ¹H NMR (400 MHz, DMSO-*d*₆) δ 9.06 (t, *J* = 5.8 Hz, 1H), 8.87 (d, *J =* 8.6 Hz, 1H), 8.54 (t, *J =* 6.6 Hz, 1H), 8.38 (d, *J =* 8.6 Hz, 1H), 8.04 (d, *J* = 6.8 Hz, 1H), 7.93-7.83 (m, 2H), 6.85 (s, 1H), 5.02 (d, *J =* 23.2 Hz, 2H), 4.83 (s, 1H), 4.75 (s, 1H), 4.63 (s, 2H), 4.58-4.42 (m, 4H), 4.30-4.14 (m, 4H), 4.10 (s, 3H), 4.02 (s, 1H), 3.89 (s, 1H), 3.84-3.72 (m, 2H), 3.71-3.52 (m, 10H), 3.52-3.40 (m, 27H), 3.25 (s, 7H), 3.22-3.13 (m, 4H), 2.94 (d, *J=* 7.2 Hz, 3H), 2.84 (d, *J =* 9.4 Hz, 1H), 2.72 (d, *J* = 11.8 Hz, 2H), 2.56 (dd, *J* = 14.4, 7.2 Hz, 2H), 2.46-2.34 (m, 4H), 2.28-2.11 (m, 5H), 2.08-1.83 (m, 8H), 1.68 (dd, *J* = 25.2, 12.2 Hz, 6H), 1.56-1.41 (m, 6H), 1.31 (s, 4H), 1.21 (d, *J =* 7.2 Hz, 4H), 1.11 (d, *J =* 6.2 Hz, 3H), 1.08-0.94 (m, 5H), 0.83 (dd, *J* = 13.8, 6.8 Hz, 6H).

### Example 50: Preparation of Compound LA-32ASBCN

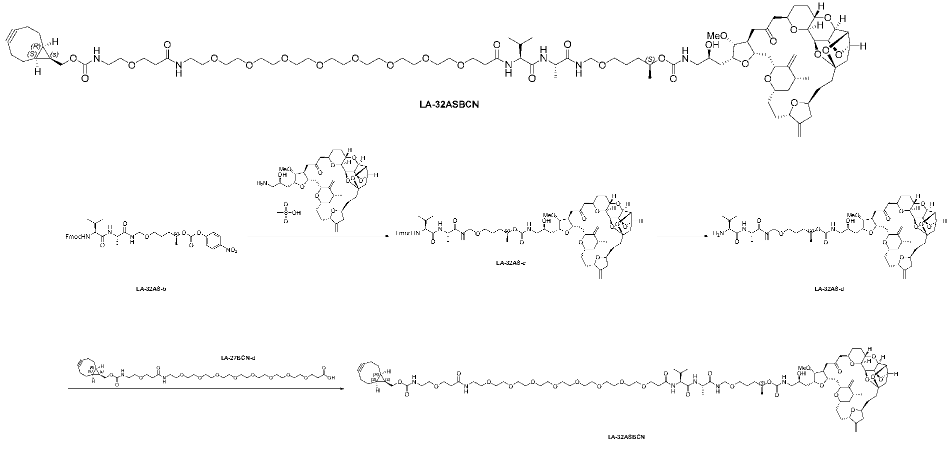

### Step 1: Preparation of compound LA-32AS-c

Eribulin mesylate (23.92 mg, 28.95 µmol, 1 eq), HOBT (3.91 mg, 28.95 µmol, 1 eq) and DIPEA (7.56 mg, 9.67 µL, 57.91 µmol, 2 eq) were added to the solution of compound (9H-fluoren-9-yl)methyl((3S,11S,14S)-3,11,15-trimethyl-1-(4-nitrophenoxy)-1,0,13-trioxy-2,7-dioxy-9,12-diaza hexadecan-14-yl)carbamate (for the synthesis of the compound, refer to the Example of LA-32AS) (20 mg, 28.95 µmol, 1 eq) in DMF (1 mL), and the reaction solution was stirred at 25°C for 2 hours. The LCMS showed that the product was generated. The reaction solution was subjected to preparative high-performance liquid chromatography to obtain a white solid product (15 mg, 40.42% yield).

The conditions of the preparative high-performance liquid chromatography were as follows:
preparative chromatograph manufacturer: Orienda, model: BRIX-2860, column: Welch Xtimate C18 250 × 30 mm × 10 µm, mobile phase: water (0.225% HCOOH)-acetonitrile, with an elution ratio of acetonitrile increased from 50% to 80%.

LCMS (ESI): m/z, 1281.7[M+H]⁺.

### Step 2: Preparation of compound LA-32AS-d

LA-32AS-c (16 mg, 12.48 µmol, 1 eq) was dissolved in DMF (2 mL), diethylamine (4.61 mg, 62.42 µmol, 5 eq) was added, and the reaction solution was stirred at 25°C for 1 hour. LCMS analysis showed that the reaction ended. The reaction solution was directly evaporated to dryness to afford the product (12 mg, 90.73% yield) as a yellow oil to be directly used in the next step.

LCMS (ESI): m/z, 1059.6[M+H]⁺.

### Step 3: Preparation of compound LA-32ASBCN

LA-32AS-d (10 mg, 9.44 µmol, 1 eq) was dissolved in DMF (1 mL), LA-27BCN-d (for synthesis of the compound, refer to Example of LA-27BCN) (8.65 mg, 80%, 9.44 µmol, 1 eq) and DIPEA (1.23 mg, 9.44 µmol, 1 eq) were added, HATU (3.59 mg, 9.44 µmol, 1 eq) was added at 0°C, the reaction solution was stirred at 25°C for 1 hour, and the LCMS analysis indicated complete consumption of the starting material. The reaction solution was purified by high-performance liquid chromatography to afford the product (7.89 mg, 47.11% yield, 95% purity) as a white solid.

The conditions of the preparative high-performance liquid chromatography were as follows:
preparative chromatograph manufacturer: Orienda, model: BRIX-2860, column: Welch Xtimate C18 250 × 30 mm × 10 µm, mobile phase: water (0.225% HCOOH)-acetonitrile, with an elution ratio of acetonitrile increased from 30% to 60%.

LCMS (ESI): m/z, 1796.1[(M+H)]⁺.

¹H NMR (400 MHz, DMSO-*d*₆) δ 8.54 (t, *J* = 6.6 Hz, 1H), 8.04 (d, *J* = 6.9 Hz, 1H), 7.95-7.78 (m, 2H), 7.05 (d, *J* = 5.6 Hz, 1H), 6.85 (t, *J=* 5.8 Hz, 1H), 5.11-4.96 (m, 2H), 4.79 (d, *J* = 31.0 Hz, 2H), 4.68-4.58 (m, 2H), 4.58-4.44 (m, 3H), 4.29-4.07 (m, 6H), 4.03 (d, *J* = 8.0 Hz, 2H), 3.92-3.72 (m, 3H), 3.68 (s, 1H), 3.63-3.55 (m, 4H), 3.50 (d, *J* = 3.9 Hz, 24H), 3.39 (q, *J =* 7.1, 6.5 Hz, 27H), 3.26 (d, *J =* 3.3 Hz, 3H), 3.19 (q, *J* = 5.8 Hz, 2H), 3.09 (q, *J* = 6.0 Hz, 2H), 2.99-2.81 (m, 3H), 2.80-2.63 (m, 2H), 2.56 (dd, *J* = 16.8, 10.3 Hz, 2H), 2.44 (t, *J* = 6.8 Hz, 1H), 2.38 (t, *J* = 6.6 Hz, 1H), 2.34-2.09 (m, 10H), 2.07-1.82 (m, 6H), 1.78-1.59 (m, 4H), 1.57-1.41 (m, 6H), 1.40-1.08 (m, 9H), 1.07-0.94 (m, 4H), 0.83 (dd, *J* = 13.6, 6.8 Hz, 6H).

### Example 51: Preparation of Compound LA-32AR

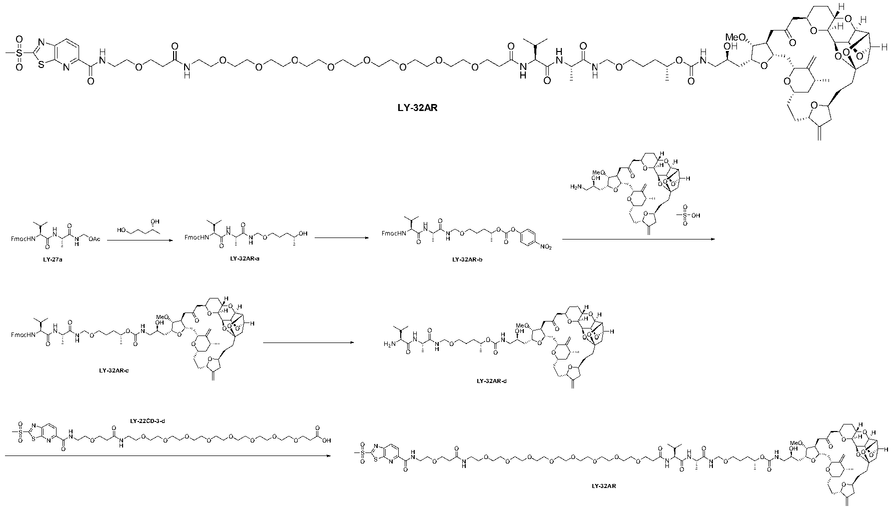

### Step 1: Preparation of compound LA-32AR-a

P-toluenesulfonic acid (103.32 mg, 96.56 µL, 600 µmol, 1 eq) was added to the solution of (2R)-2-methylbutane-1,4-diol (187.47 mg, 1.80 mmol, 3 eq) and LY-27a (187.47mg, 1.80 mmol, 3 eq) in THF (5 mL), a resulting solution was stirred at 30°C for 2 hours and concentrated, the residue was dissolved in DMF, the mixture was filtered, and the filter cake was subjected to preparative high-performance liquid chromatography (preparative chromatograph manufacturer: Orienda, model: BRIX-2860, column: Synergi Max-RP 200 × 30mm × 10 µm, mobile phase: water (0.225% HCOOH)-acetonitrile, with an elution ratio of acetonitrile increased from 30% to 60%), to afford LA-32AR-a (100 mg, 31.71% yield) as a white solid.

LCMS (ESI): m/z, 548[M+Na]⁺, Rt=2.031.

### Step 2: Preparation of compound LA-32AR-b

DIPEA (147.33 mg, 188.41 µL, 1.14 mmol, 6 eq) and NPC (231.20 mg, 154.13 µL, 760 µmol, 4 eq) were added to the solution of compound LA-32AR-a (99.87 mg, 190 µmol, 1 eq) in DMF (2 mL), the reaction solution was stirred at 35°C for 3 hours, the solution was concentrated by an oil pump, and the residue was purified by silica gel column chromatography (eluents: ethyl acetate/petroleum ether = 0%-50%; and methanol/dichloromethane = 0%-8%), to afford LA-32AR -b (50 mg, 29% yield, 67% purity) as a yellow oil.

LCMS (ESI): m/z, 713[M+Na]⁺, Rt=2.914.

### Step 3: Preparation of compound LA-32AR-c

DIPEA (4.69 mg, 6 µL, 36.32 µmol, 3 eq) and HOBT (1.64 mg, 1.09 µL, 12.11 µmol, 1 eq) were added to the solution of compound LA-32AR-b (14.98 mg, 67%, 14.53 µmol, 1.2 eq) and eribulin mesylate (10 mg, 12.11 µmol, 1 eq) in DMF (1 mL), the reaction solution was stirred for 1 hour at room temperature, and the LCMS analysis indicated complete consumption of the starting material. The residue was purified by preparative high-performance liquid chromatography (preparative chromatograph manufacturer: Shimadzu, model: LC-20AP, column: Synergi Max-RP 250 × 40 mm × 10 µm, mobile phase: water (0.225% HCOOH)-acetonitrile, with an elution ratio of acetonitrile increased from 60% to 90%), to afford LA-32AR-c (9.20 mg, 59.3% yield) as a white solid.

LCMS (ESI): m/z, 1303[M+Na]⁺, Rt=2.966.

### Step 4: Preparation of compound LA-32AR-d

Et₂NH (2.63 mg, 3.70 µL, 35.89 µmol, 5 eq) was added to the solution of compound LA-32AR-c (9.20 mg, 7.18 µmol, 1 eq) in DMF (1 mL) under an ice bath, the reaction solution was stirred at room temperature for 1 hour, LCMS analysis indicated complete consumption of the starting material, the reaction solution was dried via an oil pump and the residue was directly used in the next step. A theoretical mass of LA-32AR-d was 7.60 mg.

LCMS (ESI): m/z, 1059[M+H]⁺, Rt=1.802.

### Step 5: Preparation of compound LA-32AR

DIPEA (1.85 mg, 2.37 µL, 14.35 µmol, 2 eq) and HATU (3.27 mg, 8.61 µmol, 1.2 eq) were added to the solution of compounds LA-32AR-d (7.60 mg, 7.17 µmol, 1 eq) and LY-22CD-3-d (6.86 mg, 8.61 µmol, 1.2 eq) in DMF (1 mL) under the ice bath, the reaction solution was stirred for 1 hour at room temperature, and the LCMS analysis showed complete consumption of the starting material. The reaction solution was purified by preparative high-performance liquid chromatography (preparative chromatograph manufacturer: Orienda, model: BRIX-2860, column: Synergi Max-RP 200 × 30mm × 10 µm, mobile phase: water (0.225% HCOOH)-acetonitrile, with an elution ratio of acetonitrile increased from 30% to 60%), to afford LA-32AR (4.47 mg, 31.80% yield, 93.81% purity) as a white solid.

LCMS (ESI): m/z, 1860[M+Na]⁺, Rt=2.188. ¹H NMR (400 MHz, DMSO-*d*₆) δ 9.06 (s, 1H), 8.87 (d, *J* = 8.6 Hz, 1H), 8.58-8.51 (m, 1H), 8.38 (d, *J* = 8.6 Hz, 1H), 8.03 (t, *J* = 6.4 Hz, 1H), 7.92-7.82 (m, 2H), 6.87 (d, *J* = 26.6 Hz, 1H), 5.02 (d, *J* = 22.8 Hz, 2H), 4.79 (d, *J* = 31.4 Hz, 2H), 4.63 (s, 1H), 4.57-4.44 (m, 4H), 4.29-4.15 (m, 4H), 4.14-4.06 (m, 3H), 4.02 (s, 1H), 3.88 (d, *J =* 7.0 Hz, 1H), 3.79 (dd, *J =* 21.6, 9.8 Hz, 2H), 3.71-3.52 (m, 10H), 3.51-3.44 (m, 26H), 3.37 (t, *J* = 7.2 Hz, 4H), 3.25 (s, 4H), 3.18 (q, *J =* 5.8 Hz, 3H), 2.94 (t, *J =* 6.0 Hz, 2H), 2.84 (d, *J =* 9.6 Hz, 1H), 2.80-2.66 (m, 3H), 2.61-2.55 (m, 1H), 2.41 (dt, *J* = 24.4, 6.8 Hz, 2H), 2.33 (t, *J* = 6.4 Hz, 4H), 2.23 (t, *J =* 15.0 Hz, 4H), 2.14 (d, *J =* 12.0 Hz, 1H), 2.06-1.80 (m, 8H), 1.69 (dt, *J =* 25.4, 12.0 Hz, 5H), 1.56-1.41 (m, 6H), 1.39-1.29 (m, 3H), 1.27-1.17 (m, 5H), 1.17-1.08 (m, 3H), 1.00 (dd, *J =* 23.4, 9.0 Hz, 6H), 0.83 (dd, *J =* 13.6, 6.8 Hz, 6H).

### Example 52: Preparation of Compound LA-33A

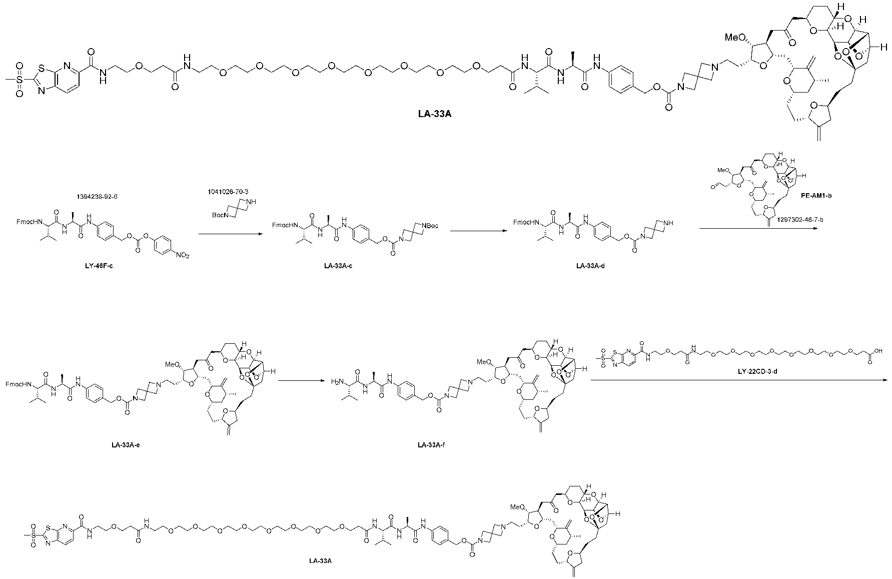

### Step 1: Preparation of compound LA-33A-c

DIPEA (157.67 mg, 199.61 µL, 1.21 mmol, 3 eq), compound LY-46F-c (315 mg, 87%, 402.60 µmol, 1 eq) and HOBT (55.51 mg, 402.60 µmol, 1 eq) were added to a solution of tert-butyl 2,6-diazaspiro[3.3]heptane-2-carboxylate (92.42 mg, 95%, 442.86 µmol, 1.1 eq) in DMF (5 mL). The reaction solution was allowed to react at room temperature for 1 hour. LCMS analysis indicated the reaction ended. The reaction solution was filtered and the residue was purified by preparative high-performance liquid chromatography (preparative chromatograph manufacturer: Shimadzu, model LC-20AP, column: Synergi Max-RP 250 × 40 mm × 10 µm, mobile phase: water (0.225% HCOOH)-acetonitrile, with an elution ratio of acetonitrile increased from 50% to 80%), to afford compound LA-33A-c (92.20 mg, 30.95% yield) as a white solid. LCMS (ESI): m/z, 762[M+Na]⁺.

### Step 2: Preparation of compound LA-33A-d

TFA (620.20 mg, 404.04 µL, 5.39 mmol, 43.2127 eq) was added to a solution of compound LA-33A-c (92.20 mg, 124.62 µmol, 1 eq) in DCM (2 mL). The reaction solution was allowed to react at 0°C for 1 hour. LCMS analysis indicated the reaction ended. The mixture was evaporated to dryness to afford compound LA-33A-d (79.72 mg; the yield was not calculated for the crude product) as a colorless oil, which was used directly in the next step. LCMS (ESI): m/z, 640[M+H]⁺.

### Step 3: Preparation of compound LA-33A-e

AcOH (2.88 mg, 99.5%, 2.73 µL, 47.70 µmol, 2 eq) was added to a solution of compound LA-33A-d (15.26 mg, 23.85 µmol, 1 eq) and compound PE-AM1-b (20 mg, 28.62 µmol, 1.2 eq; for the synthesis of the compound, refer to the Preparation Example of PE-AM1) in DCE (3 mL). The reaction solution was allowed to react at room temperature for 20 minutes, and sodium triacetoxyborohydride (10.32 mg, 98%, 47.70 µmol, 2 eq) was then added. The reaction solution was further allowed to react at room temperature for 100 minutes. LCMS analysis indicated the reaction ended. The reaction solution was evaporated to dryness, the residue was dissolved in acetonitrile, the mixture was filtered, and the filter cake was purified by preparative high-performance liquid chromatography (preparative chromatograph manufacturer: Orienda, model: BRIX-2860, column: Welch Xtimate C18 250 × 30mm × 10 µm, mobile phase: water (0.225% HCOOH)-acetonitrile, with an elution ratio of acetonitrile increased from 30% to 60%), to afford the compound LA-33A-e (8.20 mg, 26% yield) as a white solid. LCMS (ESI): m/z, 1344[M+Na]⁺.

### Step 4: Preparation of compound LA-33A-f

Et₂NH (32.27 mg, 436.83 µmol, 70.458 eq) was added to the solution of the compound LA-33A-e (8.20 mg, 6.20 µmol, 1 eq) in DMF (2 mL) and after a resulting mixture was allowed to react at room temperature for 1 hour, LCMS analysis indicated the reaction ended. The reaction solution was evaporated to dryness to afford the compound LA-33A-f (6.82 mg; the yield was not calculated for the crude product) as a white solid, which was used directly in the next step. LCMS (ESI): m/z, 1122[M+Na]⁺.

### Step 5: Preparation of compound LA-33A

DIPEA (1.60 mg, 2.05 µL, 12.40 µmol, 2 eq) and HATU (2.83 mg, 7.44 µmol, 1.2 eq) were added to the solution of compounds LA-33A-f (6.82 mg, 6.20 µmol, 1 eq) and LY-22CD-3-d (5.93 mg, 7.44 µmol, 1.2 eq) in DMF (1 mL), and after a resulting mixture was allowed to react for 1 hour at room temperature, LCMS analysis showed that the reaction ended, the reaction solution was filtered and the residue was purified by preparative high-performance liquid chromatography (preparative chromatograph manufacturer: Orienda, model: BRIX-2860, column: Welch Xtimate C18 250 × 30mm × 10 µm, mobile phase: water (0.225% HCOOH)-acetonitrile, with an elution ratio of acetonitrile increased from 30% to 60%), to afford compound LA-33A (6.87 mg, 58.98% yield, 95% purity) as a white solid.

LCMS (ESI): m/z, 940[1/2M+Na]⁺. ¹H NMR (400 MHz, DMSO-d6) δ 9.94 (s, 1H), 9.07 (s, 1H), 8.87 (d, *J* = 8.6 Hz, 1H), 8.38 (d, *J =* 8.6 Hz, 1H), 8.19 (d, *J =* 7.1 Hz, 1H), 7.89 (d, *J =* 8.6 Hz, 2H), 7.58 (d, *J =* 8.5 Hz, 2H), 7.27 (d, *J =* 8.4 Hz, 2H), 5.05 (s, 1H), 4.95 (s, 3H), 4.81 (s, 1H), 4.76 (s, 1H), 4.63 (s, 1H), 4.56 (s, 1H), 4.37 (t, *J* = 6.9 Hz, 1H), 4.27 (s, 2H), 4.19 (d, *J* = 8.4 Hz, 2H), 4.10 (s, 7H), 4.03 (s, 2H), 3.79 (s, 2H), 3.66-3.57 (m, 8H), 3.53 (d, *J =* 5.0 Hz, 3H), 3.51-3.44 (m, 34H), 3.27 (s, 4H), 3.22-3.15 (m, 5H), 2.84 (d, *J =* 9.4 Hz, 1H), 2.73 (s, 2H), 2.67 (s, 2H), 2.30 (d, *J =* 21.8 Hz, 9H), 1.96 (d, *J =* 28.0 Hz, 6H), 1.75-1.62 (m, 5H), 1.30 (d, *J =* 7.2 Hz, 6H), 1.24 (s, 2H), 1.03 (d, *J =* 6.4 Hz, 3H), 0.85 (dd, *J =* 15.6, 6.7 Hz, 6H).

### Example 53: Preparation of Compound LA-33B

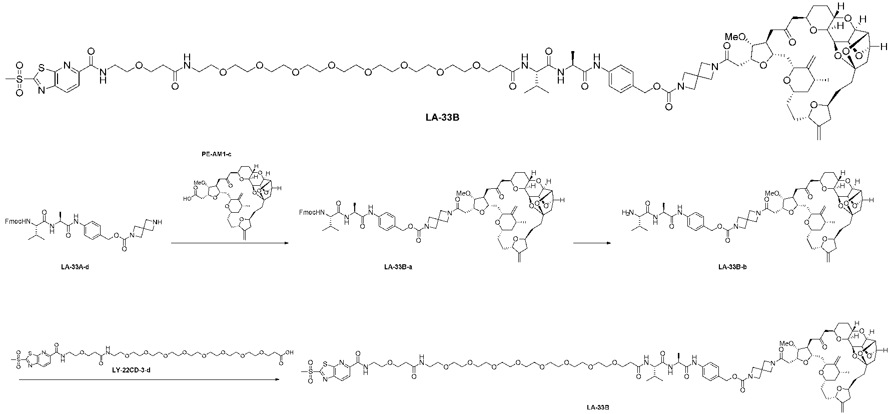

### Step 1: Preparation of compound LA-33B-a

DIPEA (7.23 mg, 9.25 µL, 55.96 µmol, 2 eq) and HATU (12.77 mg, 33.57 µmol, 1.2 eq) were added to the solution of compound LA-33A-d (17.90 mg, 27.98 µmol, 1 eq; for the synthesis of the compound, refer to the Example of LA-33A) and compound PE-AM1-c (20 mg, 27.98 µmol, 1 eq; for the synthesis of the compound, refer to the Preparation Example of PE-AM1) in DMF (2 mL), a resulting mixture was allowed to react for 1 hour at room temperature, LCMS analysis showed that the reaction ended, the reaction solution was filtered and the filter cake was purified by preparative high-performance liquid chromatography (preparative chromatograph manufacturer: Orienda, model: BRIX-2860, column: Synergi Max-RP 200 × 30mm × 10 µm, mobile phase: water (0.225% HCOOH)-acetonitrile, with an elution ratio of acetonitrile increased from 50% to 80%), to afford the compound LA-33B-a (4.70 mg, 12.57% yield) as a white solid. LCMS (ESI): m/z, 1359[M+Na]⁺.

### Step 2: Preparation of compound LA-33B-b

Et₂NH(18.19 mg, 99%, 25.61 µL, 246.15 µmol, 70 eq) was added to the solution of the compound LA-33B-a (4.70 mg, 3.52 µmol, 1 eq) in DMF (1 mL) and after a resulting mixture was allowed to react at room temperature for 1 hour, LCMS analysis indicated the reaction ended. The reaction solution was evaporated to dryness to afford the compound LA-33B-b (3.92 mg; the yield was not calculated for the crude product) as a white solid, which was used directly in the next step. LCMS (ESI): m/z, 1136[M+Na]⁺.

### Step 3: Preparation of compound LA-33B

DIPEA (909.28 µg, 1.16 µL, 7.04 µmol, 2 eq) and HATU (1.61 mg, 4.22 µmol, 1.2 eq) were added to the solution of compounds LA-33B-b (3.92 mg, 3.52 µmol, 1 eq) and LY-22CD-3-d (3.36 mg, 4.22 µmol, 1.2 eq) in DMF (1 mL), and after a resulting mixture was allowed to react for 1 hour at room temperature, LCMS analysis showed that the reaction ended, the reaction solution was filtered and the residue was purified by preparative high-performance liquid chromatography (preparative chromatograph manufacturer: Orienda, model: BRIX-2860, column: Welch Xtimate C18 150 × 30mm × 10 µm, mobile phase: water (0.225% HCOOH)-acetonitrile, with an elution ratio of acetonitrile increased from 30% to 60%), to afford compound LA-33B (2.94 mg, 42.82% yield, 97% purity) as a white solid. LCMS (ESI): m/z, 1917[M+Na]⁺.

### Example 54: Preparation of Compound LA-34A

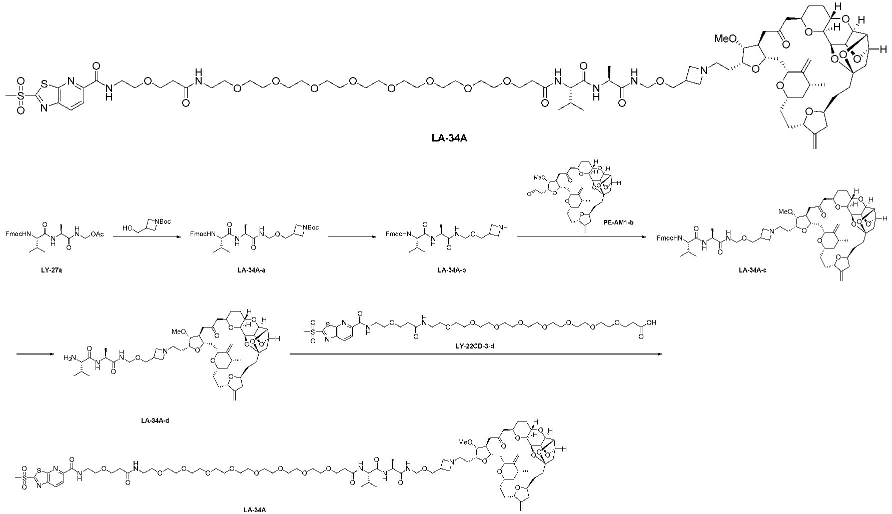

### Step 1: Preparation of compound LA-34A-a

P-toluenesulfonic acid (108.36 mg, 622.99 µmol, 1 eq) was added to the solution of compound 3-hydroxymethylazetidine-1-carboxylate (238.06 mg, 1.25 mmol, 2 eq) and LY-27a (300 mg, 622.99 µmol, 1 eq) in THF (10 mL) and a resulting mixture was left standing for reaction at 30°C for 2 hours. LCMS analysis indicated the reaction ended. The residue was purified by silica gel column chromatography (C18, ISCO, R-330g SepaFlash Silica Flash Column, eluents: 10%-60% water/CH₃CN, 100 mL/min) (medium pressure), to afford the compound LA-34A-a (252 mg, 66.4% yield) as a white solid.

LCMS (ESI): m/z, 631[M+Na]⁺, Rt=2.589 min.

### Step 2: Preparation of compound LA-34A-b

TFA (3.0 mL, 41.40 mmol, 100 eq) was added to the solution of compound LA-34A-a (252 mg, 413.97 µmol, 1 eq) in DCM (12 mL) and a resulting mixture allowed to react at 0°C for 30 minutes. The LCMS analysis showed that the reaction ended. After the resulting mixture was evaporated to dryness, the residue was purified (under medium pressure) by silica gel column chromatography (C18, ISCO, R-330g SepaFlash Silica Flash Column, eluents: 5%-40% water/CH₃CN, 100 mL/min), to obtain a white solid LA-34A-b (95.40 mg, yield: 45.31%).

LCMS (ESI): m/z, 531[M+Na]⁺, Rt=1.613 min.

### Step 3: Preparation of compound LA-34A-c

AcOH (1.65 µL, 28.62 µmol, 2 eq) was added to a solution of compound LA-34A-b (8.73 mg, 17.17 µmol, 1.2 eq) and compound PE-AM1-b (10 mg, 14.31 µmol, 1 eq; for the synthesis of the compound, refer to the Preparation Example of PE-AM1) in DCE (2 mL). The reaction solution was allowed to react at room temperature for 20 minutes, and sodium triacetoxyborohydride (6.19 mg, 28.62 µmol, 2 eq) was then added. The reaction solution was further allowed to react at room temperature for 40 minutes. LCMS analysis indicated the reaction ended. The reaction solution was evaporated to dryness, and the residue was purified by preparative high-performance liquid chromatography (preparative chromatograph manufacturer: Orienda, model: BRIX-2860, column: Welch Xtimate C18 250 × 30mm × 10 µm, mobile phase: water (0.225% HCOOH)-acetonitrile, with an elution ratio of acetonitrile increased from 30% to 60%), to afford compound LA-34A-c (11 mg, 64.52% yield) as a white solid.

LCMS (ESI): m/z, 1191[M+H]⁺, Rt=2.647 min.

### Step 4: Preparation of compound LA-34A-d

Et₂NH (4.80 µL, 46.16 µmol, 5 eq) was added to a solution of compound LA-34A-c (11 mg, 9.23 µmol, 1 eq) in DCM (2 mL), the resulting mixture allowed to react for 1 hour at room temperature, and the LCMS analysis showed that the reaction ended. The reaction solution was evaporated to dryness, to obtain LA-34A-d (8.95 mg; the yield was not calculated for the crude product) as a white solid, which was directly used in the next step.

LCMS (ESI): m/z, 969 [M+H]⁺, Rt=1.514 min.

### Step 5: Preparation of compound LA-34A

DIPEA (3.08 µL, 18.47 µmol, 2 eq) and HATU (4.21 mg, 11.08 µmol, 1.2 eq) were added to the solution of compounds LA-34A-d (8.95 mg, 9.23 µmol, 1 eq) and LY-22CD-3-d (8.83 mg, 11.08 µmol, 1.2 eq) in DMF (1 mL), and after a resulting mixture was allowed to react for 1 hour at room temperature, LCMS analysis showed that the reaction ended, the reaction solution was filtered and the residue was purified by preparative high-performance liquid chromatography (preparative chromatograph manufacturer: Orienda, model: BRIX-2860, column: Welch Xtimate C18 250 × 30mm × 10 µm, mobile phase: water (0.225% HCOOH)-acetonitrile, with an elution ratio of acetonitrile increased from 30% to 60%), to afford compound LA-34A (3.28 mg, 19.53% yield, 96.14% purity) as a white solid.

LCMS (ESI): m/z, 1748[M+H]⁺, Rt=2.022 min.

### Example 55: Preparation of Compound LA-34B

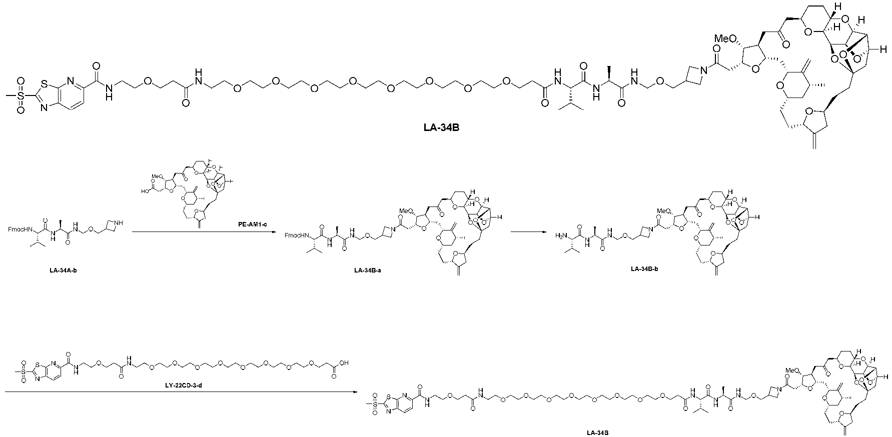

### Step 1: Preparation of compound LA-34B-a

DIPEA (7.01 µL, 41.97 µmol, 3 eq) and HATU (6.38 mg, 16.79 µmol, 1.2 eq) were added to the solution of compound LA-34A-d (8.54 mg, 16.79 µmol, 1.2 eq) (for the synthesis of the compound, refer to the Example of LA-34A) and compound PE-AM1-c (for the synthesis of the compound, refer to the Example of PE-AM1) (10 mg, 13.99 µmol, 1 eq) in DMF (2 mL), a resulting mixture was allowed to react for 1 hour at room temperature, LCMS analysis showed that the reaction ended, the reaction solution was filtered and the filter cake was purified by preparative high-performance liquid chromatography (preparative chromatograph manufacturer: Orienda, model: BRIX-2860, column: Welch Xtimate C18 150 × 30mm × 10 µm, mobile phase: water (0.225% HCOOH)-acetonitrile, with an elution ratio of acetonitrile increased from 50% to 80%), to obtain a white solid compound LA-34B-a (9 mg, yield: 53.37%).

LCMS (ESI): m/z, 1205[M+H]⁺, Rt=2.983 min.

### Step 2: Preparation of compound LA-34B-b

Et₂NH (3.88 µL, 37.33 µmol, 5 eq) was added to a solution of compound LA-34B-a (9 mg, 7.47 µmol, 1 eq) in DCM (1 mL), the resulting mixture allowed to react for 1 hour at room temperature, and the LCMS analysis showed that the reaction ended. The reaction solution was evaporated to dryness, to obtain the white solid compound LA-34B-b (7.34 mg, no yield was calculated for the crude product) to be directly used for the next step. LCMS (ESI): m/z, 983[M+H]⁺, Rt=1.677 min.

### Step 3: Preparation of compound LA-34B

DIPEA (2.49 µL, 14.93 µmol, 2 eq) and HATU (3.41 mg, 8.96 µmol, 1.2 eq) were added to the solution of compounds LA-34B-b (7.34 mg, 7.47 µmol, 1 eq) and LY-22CD-3-d (7.14 mg, 8.96 µmol, 1.2 eq) in DMF (1 mL), and after a resulting mixture was allowed to react for 1 hour at room temperature, LCMS analysis showed that the reaction ended, the reaction solution was filtered and the residue was purified by preparative high-performance liquid chromatography (preparative chromatograph manufacturer: Orienda, model: BRIX-2860, column: Welch Xtimate C18 150 × 30mm × 10 µm, mobile phase: water (0.225% HCOOH)-acetonitrile, with an elution ratio of acetonitrile increased from 30% to 60%), to afford compound LA-34B (4.84 mg, 35.48% yield, 96.42% purity) as a white solid.

LCMS (ESI): m/z, 1761[M+H]⁺, Rt=2.119 min.

### Example 56: Preparation of Compound LA-34B-2

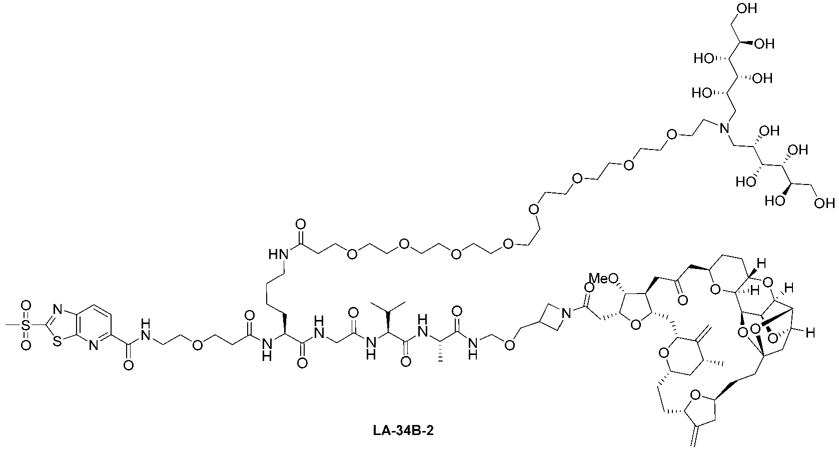

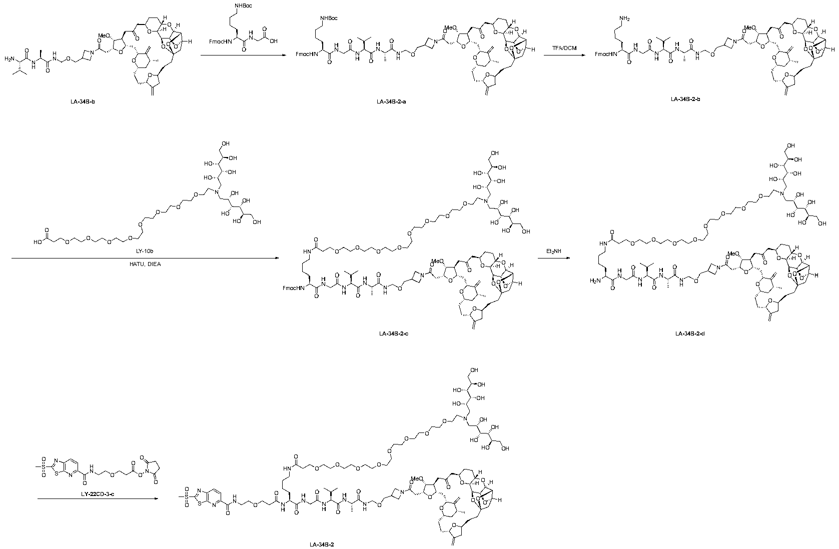

### Step 1: Preparation of compound LA-34B-2-a

DIPEA (52.11 µL, 315.31 µmol, 3 eq) and HATU (47.96 mg, 126.13 µmol, 1.2 eq) were added to a solution of compound LA-34B-b (103.34 mg, 105.10 µmol, 1 eq) (for the synthesis of the compound, refer to the Preparation Example of LA-34B) and compound N²-(((9H-fluoren-9-yl) methoxy)carbonyl)-N⁶-(tert-butoxycarbonyl)-L-lysylglycine (75.33 mg, 88%, 126.13 µmol, 1.2 eq) (for the synthesis of the compound, refer to the Example of LA-27C-2) in DMF (5 mL) under an ice bath. The reaction solution was allowed to react at room temperature for 0.5 hours. The LCMS analysis showed that the reaction ended. The reaction solution was filtered and the residue was purified by preparative high-performance liquid chromatography (preparative chromatograph manufacturer: Orienda, model: BRIX-2860, column: Welch Xtimate C18 150 × 30mm × 10 µm, mobile phase: water (0.225% HCOOH)-acetonitrile, with an elution ratio of acetonitrile increased from 50% to 80%), to afford compound LA-34B-2-a (137.50 mg, 87.75% yield) as a white solid.

LCMS (ESI): m/z, 1490[M+H]⁺. Rt = 3.031 min.

### Step 2: Preparation of compound LA-34B-2-b

TFA (1 mL, 13.33 mmol, 155.8 eq) was added to a solution of compound LA-34B-2-a (137.50 mg, 92.23 µmol, 1 eq) in DCM (5 mL) under an ice bath. The mixture was allowed to react under an ice bath for 1 hour. The LCMS analysis showed that the reaction ended. The mixture was evaporated to dryness to afford the compound LA-34B-2-b (127 mg; the yield was not calculated for the crude product) as a yellow oil, which was used directly in the next step.

LCMS (ESI): m/z, 1390[M+H]⁺. Rt = 2.340 min.

### Step 3: Preparation of compound LA-34B-2-c

DIPEA (70.67 µL, 427.63 µmol, 5 eq) and HATU (39.82 mg, 102.63 µmol, 1.2 eq) were added to the solution of compounds LA-34B-2-b (118.94 mg, 85.53 µmol, 1 eq) and LY-10b (for synthesis of the compound, refer to the example of LA-27C-2) (98.76 mg, 128.29 µmol, 1.5 eq) in DMF (5 mL) under an ice bath. After addition, the reaction solution was allowed to react at 25°C for 20 minutes. LCMS analysis showed that the reaction ended. The reaction solution was purified by preparative high-performance liquid chromatography (preparative chromatograph manufacturer: Orienda, model: BRIX-2860, column: Welch Xtimate C18 150 × 30mm × 10 µm, mobile phase: water (0.225% HCOOH)-acetonitrile, with an elution ratio of acetonitrile increased from 30% to 60%) and lyophilized, to afford compound LA-34B-2-c (112.50 mg, 56.95% yield) as a white solid.

LCMS (ESI): m/z, 1071[1/2M+H]⁺. Rt = 2.188 min.

### Step 4: Preparation of compound LA-34B-2-d

Et₂NH (27.32 µL, 262.54 µmol, 5 eq) was added to a solution of compound LA-34B-2-c (112.50 mg, 52.51 µmol, 1 eq) in DCM (3 mL), the resulting mixture was allowed to react for 1 hour at room temperature, and the LCMS analysis showed that the reaction ended. The reaction solution was evaporated to dryness, to afford compound LA-34B-2-d (100.83 mg; the yield was not calculated for the crude product) as a yellow oil, which was directly used in the next step.

LCMS (ESI): m/z, 961[1/2M+H]⁺, Rt=1.523 min.

### Step 5: Preparation of compound LA-34B-2

DIPEA (6.52 µL, 39.06 µmol, 1.5 eq) was added to the solution of compounds LA-34B-2-d (50 mg, 26.04 µmol, 1 eq) and LY-22CD-3-c (for synthesis of the compound, refer to Preparation Example of LY-22CD-3-d) (19.42 mg, 82%, 33.85 µmol, 1.3 eq) in DMF (1.5 mL) under an ice bath. After addition, the reaction solution was allowed to react at 25°C for 1 hour. LCMS analysis showed that the reaction ended. The reaction solution was purified by preparative high-performance liquid chromatography (preparative chromatograph manufacturer: Orienda, model: BRIX-2860, column: Waters Xbridge C18 150 × 30 mm × 5 µm, mobile phase: water (0.225% HCOOH)-acetonitrile, with an elution ratio of acetonitrile increased from 30% to 60%) and lyophilized, to afford compound LA-34B-2 (19.83 mg, 33.03% yield, 98.7% purity) as a white solid.

LCMS (ESI): m/z, 1060[1/2M+Na]⁺. Rt = 1.871 min. ¹H NMR (400 MHz, DMSO-d6) δ 9.03 (t, J = 5.8 Hz, 1H), 8.80 (d, J = 8.6 Hz, 1H), 8.51 (t, J = 6.6 Hz, 1H), 8.31 (d, J = 8.6 Hz, 1H), 8.10-8.03 (m, 3H), 7.98 (d, J = 7.6 Hz, 1H), 7.72 (t, J = 5.6 Hz, 1H), 7.66 (d, J = 8.6 Hz, 1H), 6.13 (d, J = 3.0 Hz, 1H), 5.88 (d, J = 3.0 Hz, 1H), 5.36 (s, 2H), 4.88 (s, 1H), 4.80 (s, 1H), 4.73 (s, 1H), 4.68 (s, 1H), 4.51 (q, J = 5.6, 5.0 Hz, 2H), 4.45 (d, J = 8.4 Hz, 2H), 4.27 (s, 2H), 4.22 (d, J = 9.6 Hz, 2H), 4.13 (dd, J = 12.4, 7.0 Hz, 4H), 4.08-3.89 (m, 10H), 3.79-3.39 (m, 86H), 3.14 (d, J = 2.6 Hz, 4H), 2.95 - 2.86 (m, 3H), 2.75 - 2.63 (m, 3H), 2.52 (d, J = 7.6 Hz, 3H), 2.39 - 2.26 (m, 4H), 2.04 (dd, J = 15.8, 7.4 Hz, 1H), 1.88 (t, J = 6.8 Hz, 3H), 1.78 (d, J = 11.4 Hz, 1H), 1.70 - 1.50 (m, 7H), 1.38 (d, J = 12.2 Hz, 3H), 1.26 (q, J = 12.6, 9.6 Hz, 5H), 1.15 (d, J = 7.2 Hz, 4H), 0.97 (d, J = 6.4 Hz, 3H), 0.75 (dd, J = 18.2, 6.6 Hz, 6H).

### Example 57: Preparation of Compound LA-34B-22

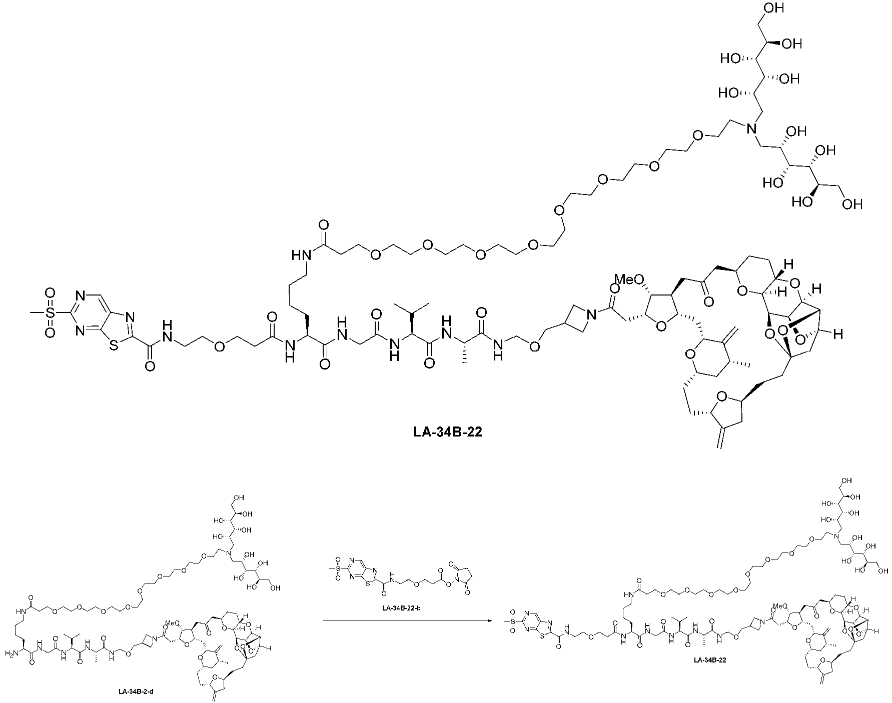

DIPEA (6.52 µL, 39.06 µmol, 1.5 eq) was added to the solution of compounds LA-34B -2-d (50 mg, 26.04 µmol, 1 eq) (for synthesis of the compound, refer to Preparation Example of LA-34B-2) and LA-34B-22-b (22.80 mg, 70%, 33.85 µmol, 1.3 eq) in DMF (1.5 mL) under an ice bath. After addition, the reaction solution was allowed to react at 25°C for 1 hour. LCMS analysis showed that the reaction ended. The reaction solution was purified by preparative high-performance liquid chromatography (preparative chromatograph manufacturer: Orienda, model: BRIX-2860, column: Waters Xbridge C18 150 × 30 mm × 5 µm, mobile phase: water (0.225% HCOOH)-acetonitrile, with an elution ratio of acetonitrile increased from 30% to 60%) and lyophilized, to afford compound LA-34B-22 (6.85 mg, 11.56% yield, 100% purity) as a white solid.

LCMS (ESI): m/z, 1038[1/2M+H]⁺. Rt = 1.822 min.

¹H NMR (400 MHz, DMSO-d6) δ 9.83 (s, 1H), 9.45 (t, J = 5.6 Hz, 1H), 8.58 (t, J = 6.8 Hz, 1H), 8.19 - 8.02 (m, 4H), 7.79 (t, J = 5.6 Hz, 1H), 7.74 (d, J = 8.8 Hz, 1H), 6.20 (d, J = 3.0 Hz, 1H), 5.94 (d, J = 3.0 Hz, 1H), 5.42 (s, 2H), 4.95 (s, 1H), 4.87 (s, 1H), 4.78 (d, J = 20.0 Hz, 3H), 4.56 (t, J = 15.2 Hz, 6H), 4.38 - 4.26 (m, 3H), 4.20 (q, J = 9.8, 8.6 Hz, 3H), 4.15 - 3.96 (m, 6H), 3.87 - 3.42 (m, 55H), 3.21 (d, J = 2.6 Hz, 3H), 2.98 (d, J = 8.4 Hz, 4H), 2.82 - 2.69 (m, 4H), 2.63 - 2.56 (m, 3H), 2.46 - 2.38 (m, 4H), 2.28 (t, J = 6.6 Hz, 5H), 2.11 (dd, J = 15.4, 7.2 Hz, 2H), 2.02 - 1.79 (m, 6H), 1.78 - 1.55 (m, 8H), 1.46 (d, J = 13.2 Hz, 4H), 1.33 (d, J = 10.0 Hz, 5H), 1.26 - 1.19 (m, 4H), 1.04 (d, J = 6.4 Hz, 3H), 0.82 (dd, J = 18.0, 6.8 Hz, 6H).

### Example 58: Preparation of Compound LA-34B-2C

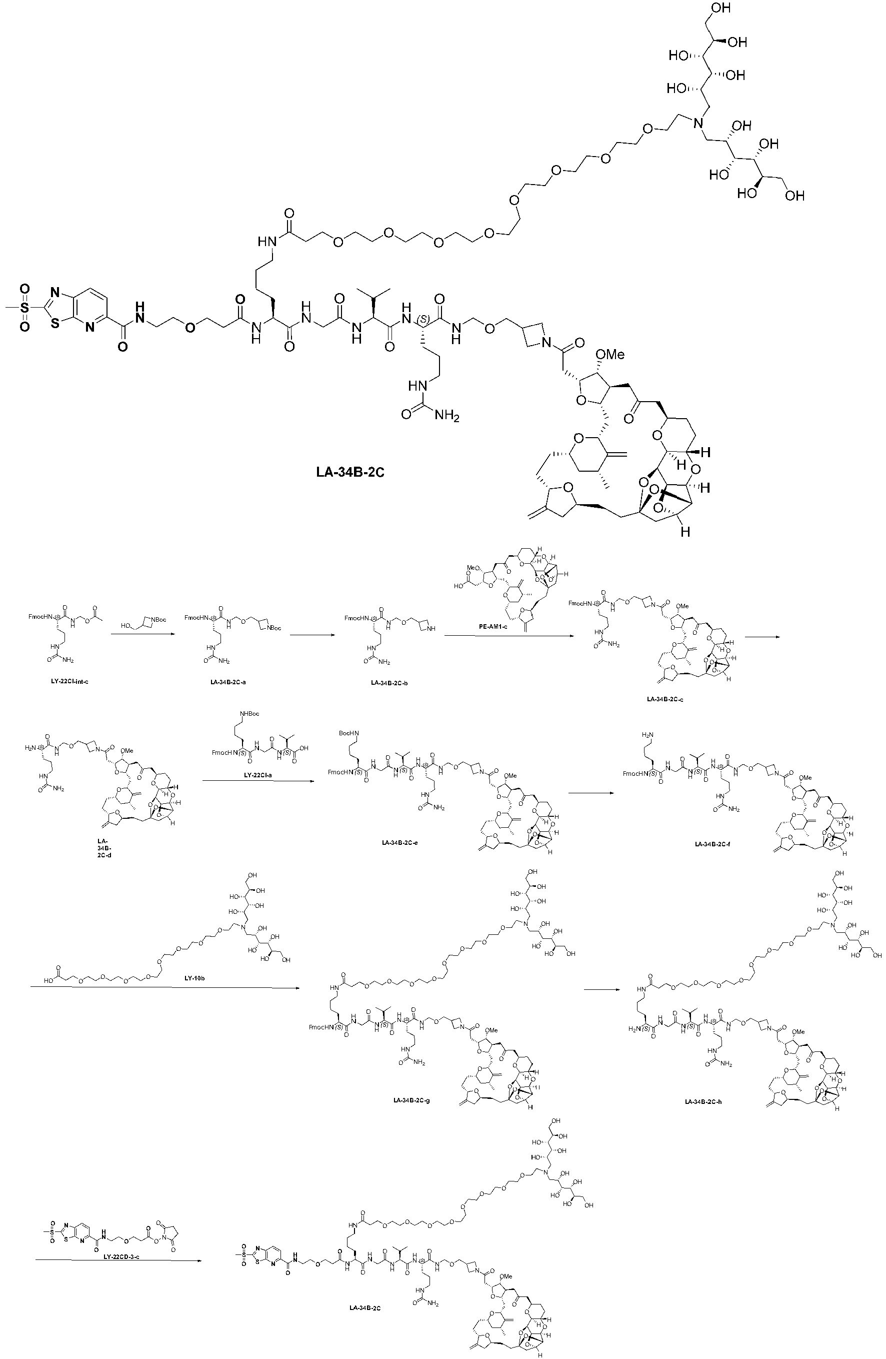

### Step 1: Preparation of compound LA-34B-2C-a

TsOH (177 mg, 1.01 mmol, 1 eq) was added to a solution of compound LY-22CI-int-c (472 mg, 1.01 mmol, 1 eq) (for the synthesis of the compound, refer to patent application WO2024175069A1) and tert-butyl 3-(hydroxymethyl)azetidine-1-carboxylate (385 mg, 2.01 mmol, 2 eq) in THF (3 mL) and DMF (1.5 mL). The mixture was allowed to react at 30°C for 2 hours. LCMS analysis indicated the reaction ended. The bulk of THF was removed under concentration. The residue was purified by silica gel column chromatography (C18, ISCO, R-120g SepaFlash Silica Flash Column, eluted with water/CH₃CN, at an elution ratio of acetonitrile: 5%-50%; 60 mL/min) to afford compound LA-34B-2C-a as a white solid (547 mg, 91.15% yield).

LCMS (ESI): m/z, 496[M-100+H]⁺.

### Step 2: Preparation of compound LA-34B-2C-b

TFA (1.55g, 1.01 mL) was added to a solution of compound LA-34B-2C-a (547 mg, 918.25 µmol, 1 eq) in DCM (4 mL) at 0°C. The mixture was allowed to react at 0°C for 1 hour. LCMS analysis indicated the reaction ended. DCM was removed under concentration. The residue was purified by silica gel column chromatography (C18, ISCO, R-120g SepaFlash Silica Flash Column, eluted with water/CH₃CN, at an elution ratio of acetonitrile: 5%-35%; 60 mL/min) to afford compound LA-34B-2C-b as a white solid (19.80 mg, 4.35% yield, 85% purity).

LCMS (ESI): m/z, 496[M+H]⁺.

### Step 3: Preparation of compound LA-34B-2C-c

DIPEA (13.34 µL, 79.91 µmol, 2 eq) and HATU (18.23 mg, 47.94 µmol, 1.2 eq) were added to the solution of compound LA-34B-2C-b (19.80 mg, 39.95 µmol, 1 eq) and compound PE-AM1-c (for the synthesis of the compound, refer to the Preparation Example of PE-AM1) (31.42 mg, 43.95 µmol, 1.1 eq) in DMF (1 mL) at 0°C, a resulting mixture was allowed to react at 25°C for 1 hour, LCMS analysis showed that the reaction ended, the reaction solution was filtered and the filter cake was purified by preparative high-performance liquid chromatography (preparative chromatograph manufacturer: Orienda, model: BRIX-2860, column: Welch Xtimate C18 250 × 30 mm × 10 µm, mobile phase: water (0.225% HCOOH)-acetonitrile, with an elution ratio of acetonitrile increased from 40% to 70%), to afford compound LA-34B-2C-c (20.70 mg, 43.45% yield) as a white solid.

LCMS (ESI): m/z, 1192[M+H]⁺, Rt=2.415 min.

### Step 4: Preparation of compound LA-34B-2C-d

Et₂NH (9.03 µL, 86.80 µmol, 5 eq) was added to a solution of compound LA-34B-2C-c (20.70 mg, 17.36 µmol, 1 eq) in DCM (1 mL) at 0°C, the resulting mixture was allowed to react at 25°C for 1 hour, and the LCMS analysis showed that the reaction ended. The reaction solution was concentrated, to afford LA-34B-2C-d (16.8 mg; the yield was not calculated for the crude product) as a yellow oil, which was directly used in the next step.

LCMS (ESI): m/z, 970[M+H]⁺, Rt=1.557 min.

### Step 5: Preparation of compound LA-34B-2C-e

DIPEA (5.78 µL, 34.63 µmol, 2 eq) and HATU (7.90 mg, 20.78 µmol, 1.2 eq) were added to the solution of compound LA-34B-2C-d (16.80 mg, 17.32 µmol, 1 eq) and compound LY-22CI-a (for the synthesis of the compound, refer to patent application WO2024175069A1) (13.91 mg, 85.58%, 19.05 µmol, 1.1 eq) in DMF (1 mL) at 0°C, a resulting mixture was allowed to react at 25°C for 1 hour, LCMS analysis showed that the reaction ended, the reaction solution was filtered and the filter cake was purified by preparative high-performance liquid chromatography (preparative chromatograph manufacturer: Orienda, model: BRIX-2860, column: YMC-Triart Prep C18 150 × 30 mm × 10 µm, mobile phase: water (0.225% HCOOH)-acetonitrile, with an elution ratio of acetonitrile increased from 50% to 80%), to afford compound LA-34B-2C-e in (29.40 mg, 86.13% yield, 80% purity) as a white solid.

LCMS (ESI): m/z, 1576[M+H]⁺, Rt=2.800 min.

### Step 6: Preparation of compound LA-34B-2C-f

TFA (0.6 mL) was added to a solution of compound LA-34B-2C-e (29.4 mg, 14.91 µmol, 1 eq) in DCM (3 mL) at 0°C. The mixture was stirred for 20 minutes. The LCMS analysis indicated complete consumption of the starting material. The mixture was concentrated and used directly in the next step. A theoretical mass of LA-34B-2C-f was 21.5 mg.

LCMS (ESI): m/z, 1476 [M+H]⁺, Rt=2.094 min.

### Step 7: Preparation of compound LA-34B-2C-g

DIPEA (1.3 mg, 9.48 µmol, 2 eq) and HATU (2.16 mg, 5.69 µmol, 1.2 eq) were added to a solution of compound LA-34B-2C-f (7 mg, 4.74 µmol, 1 eq) and LY-10b (4.38 mg, 5.69 µmol, 1.2 eq; for synthesis of the compound, refer to patent application WO2024175069A1) in DMF (1 mL) at 0°C. The mixture was stirred at 25°C for 1 hour. The LCMS analysis indicated complete consumption of the starting material. The reaction solution was purified by preparative high-performance liquid chromatography (preparative chromatograph manufacturer: Orienda, model: BRIX-2860, column: Welch Xtimate C18 150 × 30 mm × 10 µm, mobile phase: water (0.225% HCOOH)-acetonitrile, with an elution ratio of acetonitrile increased from 30%-60%), to afford LA-34B-2C-g (15.70 mg; combined with other batches for purification) as a white solid.

### LCMS (ESI): m/z, 1114[1/2M+H]⁺, Rt=2.118 min.

Step 8: Preparation of compound LA-34B-2C-h

Et₂NH (3.67 µL, 35.22 µmol, 5 eq) was added to the solution of the compound LA-34B-2C-g (15.70 mg, 7.04 µmol, 1 eq) in DMF (1 mL) at 0°C, the reaction solution was stirred at 25 C for 1 hour, and the LCMS analysis indicated complete consumption of the starting material. The mixture was concentrated by an oil pump and used directly in the next step. A theoretical mass of compound LA-34B-2C-h was 14 mg.

LCMS (ESI): m/z, 1003 [1/2M+H]⁺, Rt=1.620 min.

### Step 9: Preparation of compound LA-34B-2C

DIPEA (2.33 µL, 13.96 µmol, 2 eq) was added to the solution of compounds LA-34B-2C-h (14 mg, 6.98 µmol, 1 eq) and LY-22CD-3-c (for synthesis of the compound, refer to patent application WO2024175069A1) (3.61 mg, 7.68 µmol, 1.1 eq) in DMF (1 mL) at 0°C. After addition, the reaction solution was allowed to react at 25°C for 1 hour. LCMS analysis showed that the reaction ended. The reaction solution was purified by preparative high-performance liquid chromatography (preparative chromatograph manufacturer: Orienda, model: BRIX-2860, column: YMC-Triart Prep C18 150 × 30 mm × 10 µm, mobile phase: water (0.225% HCOOH)-acetonitrile, with an elution ratio of acetonitrile increased from 30% to 60%) and lyophilized, to afford compound LA-34B-2C (3.03 mg, 18.39% yield, 100% purity) as a white solid.

LCMS (ESI): m/z, 1181[1/2M+H]⁺. Rt=1.848 min.

### Example 59: Preparation of Compound LA-34B-2C-B

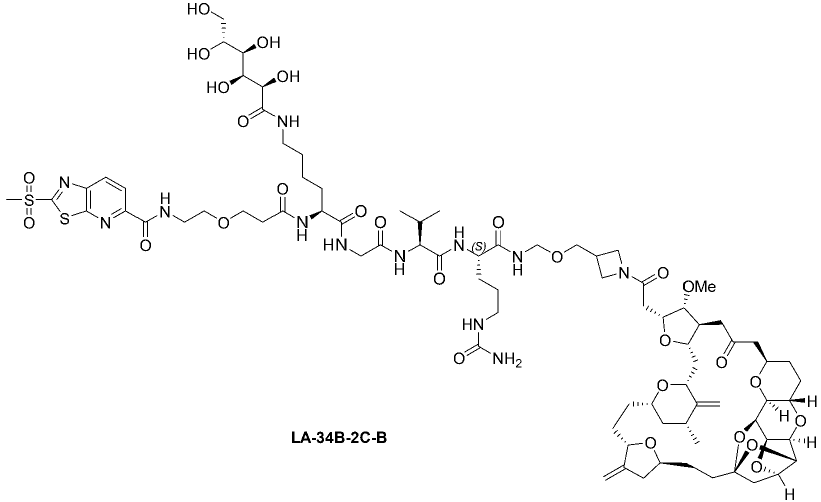

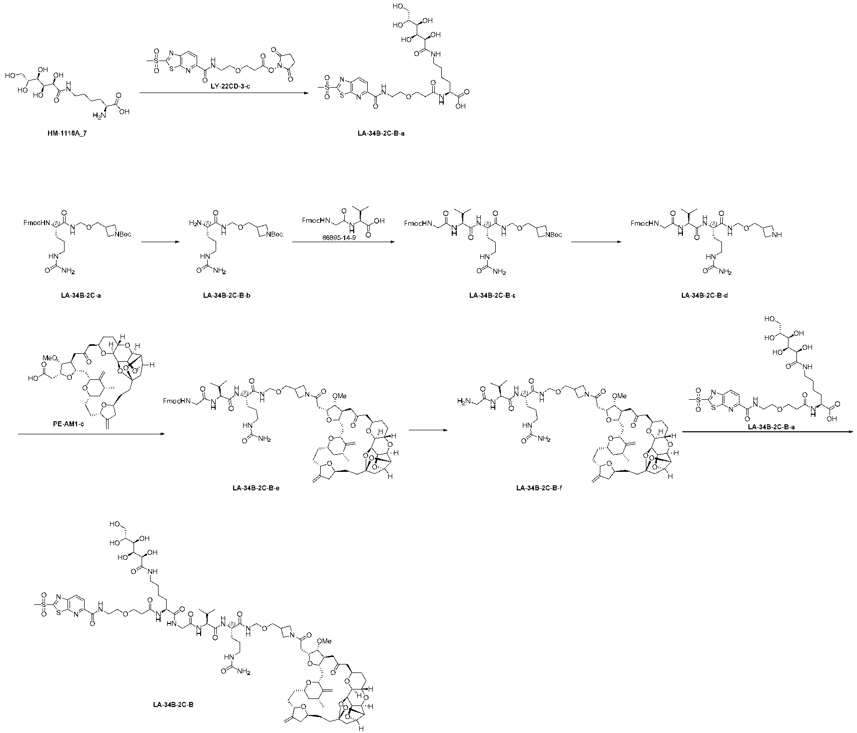

### Step 1: Preparation of compound LA-34B-2C-B-a

DIPEA (272 mg, 2.11 mmol, 6 eq) and LY-22CD-3-c (for the synthesis of the compound, refer to patent application WO2024175069A1) (165.37 mg, 351.49 µmol, 1 eq) were added to a solution of compound HM-1118A-7 (for the synthesis of the compound, refer to the Preparation Example of LA-3) (114 mg, 351.49 µmol, 1 eq) in DMF (5 mL) at 0°C. After addition, the reaction solution was allowed to react at 25°C for 1 hour. LCMS analysis showed that the reaction ended. The residue was purified by silica gel column chromatography (C18, ISCO, R-330g SepaFlash Silica Flash Column, eluted with water/CH₃CN, at an elution ratio of acetonitrile: 5%-25%; 100 mL/min) and lyophilized, to afford compound LA-34B-2C-B-a (99.10 mg, 41.48% yield) as a white solid.

LCMS (ESI): m/z, 680[M+H]⁺. Rt=0.492 min.

### Step 2: Preparation of compound LA-34B-2C-B-b

Et₂NH (226.91 µL, 2.18 mmol, 5 eq) was added to a solution of compound LA-34B-2C-a (309.30 mg, 84%, 436.15 µmol, 1 eq) in DCM (3 mL), the resulting mixture was allowed to react at 25°C for 1 hour, and the LCMS analysis showed that the reaction ended. The reaction solution was concentrated via an oil pump, to afford LA-34B-2C-B-b (162 mg; the yield was not calculated for the crude product) as a yellow oil, which was directly used in the next step.

LCMS (ESI): m/z 374[M+H]⁺, Rt=0.696 min.

### Step 3: Preparation of compound LA-34B-2C-B-c

DIPEA (112 mg, 867.59 µmol, 2 eq) and HATU (198 mg, 520.55 µmol, 1.2 eq) were added to a solution of compounds LA-34B-2C-B-b (162 mg, 433.79 µmol, 1 eq) and Fmoc-Gly-Val-OH (193.01 mg, 477.17 µmol, 1.1 eq) in DMF (6 mL) at 0°C. The mixture was allowed to react at 25°C for 1 hour. LCMS analysis indicated the reaction ended. The residue was purified by silica gel column chromatography (C18, ISCO, R-330g SepaFlash Silica Flash Column, eluted with water/CH₃CN, at an elution ratio of acetonitrile: 5%-25%; 100 mL/min) (medium pressure) and lyophilized, to afford compound LA-34B-2C-B-c (160 mg, 49.06% yield) as a white solid.

LCMS (ESI): m/z, 752[M+H]⁺, Rt=1.877 min.

### Step 4: Preparation of compound LA-34B-2C-B-d

TFA (0.6 mL) was added to a solution of compound LA-34B-2C-B-c (40 mg, 53.20 µmol, 1 eq) in DCM (3 mL) at 0°C. The mixture was stirred for 30 minutes. The LCMS analysis indicated complete consumption of the starting material. The mixture was concentrated via an oil pump and used directly in the next step. A theoretical mass of LA-34B-2C-B-d was 34 mg.

LCMS (ESI): m/z, 652 [M+H]⁺, Rt=1.363 min.

### Step 5: Preparation of compound LA-34B-2C-B-e

DIPEA (40 mg, 312.99 µmol, 6 eq) and HATU (23.80 mg, 62.60 µmol, 1.2 eq) were added to the solution of compound LA-34B-2C-B-d (34 mg, 52.17 µmol, 1 eq) and compound PE-AM1-c (for the synthesis of the compound, refer to the Preparation Example of PE-AM1) (37.29 mg, 52.17 µmol, 1 eq) in DMF (1.5 mL) at 0°C, a resulting mixture was allowed to react at 25°C for 1 hour, LCMS analysis showed that the reaction ended, the reaction solution was filtered and the filter cake was purified by preparative high-performance liquid chromatography (preparative chromatograph manufacturer: Orienda, model: BRIX-2860, column: Welch Xtimate C18 250 × 30 mm × 10 µm, mobile phase: water (0.225% HCOOH)-acetonitrile, with an elution ratio of acetonitrile increased from 40% to 70%), to afford compound LA-34B-2C-B-e (43.30 mg, 61.55% yield) as a white solid.

LCMS (ESI): m/z, 1348[M+H]⁺, Rt=2.526 min.

### Step 6: Preparation of compound LA-34B-2C-B-f

Et₂NH (12 mg, 160.54 µmol, 5 eq) was added to a solution of compound LA-34B-2C-B-e (43.30 mg, 32.11 µmol, 1 eq) in DCM (2 mL) at 0°C, the resulting mixture was allowed to react at 25°C for 1 hour, and the LCMS analysis showed that the reaction ended. The reaction solution was concentrated, to afford LA-34B-2C-B-f (36 mg; the yield was not calculated for the crude product) as a yellow oil, which was directly used in the next step.

LCMS (ESI): m/z, 1126[M+H]⁺, Rt=1.583 min.

### Step 7: Preparation of compound LA-34B-2C-B

HOBt (6.60 mg, 48.83 µmol, 2.5 eq) and EDCI (5.67 mg, 29.30 µmol, 1.5 eq) were added to the solution of compound LA-34B-2C-B-f (22 mg, 19.53 µmol, 1 eq) and LA-34B-2C-B-a (12 mg, 17.58 µmol, 0.9 eq) in DMF (1.5 mL) at 0°C, a resulting mixture was stirred for reaction at 25°C for 1 hour, LCMS analysis showed that the reaction ended, the reaction solution was filtered and the filter cake was purified by preparative high-performance liquid chromatography (preparative chromatograph manufacturer: Orienda, model: BRIX-2860, column: Welch Xtimate C18 250 × 30 mm × 10 µm, mobile phase: water (0.225% HCOOH)-acetonitrile, with an elution ratio of acetonitrile increased from 30% to 50%), to afford compound LA-34B-2C-B (3.1 mg; combined with another small-scale batch; 7% yield) as a white solid.

LCMS : m/z = 1810[M+Na]⁺; Rt = 1.797 min.

¹H NMR (400 MHz, DMSO-*d*₆) δ 9.11 (t, *J* = 5.6 Hz, 1H), 8.87 (d, *J* = 8.6 Hz, 1H), 8.62 (t, *J* = 6.8 Hz, 1H), 8.38 (d, *J=* 8.6 Hz, 1H), 8.16 (t, *J* = 5.8 Hz, 1H), 8.06 (t, *J* = 8.4 Hz, 2H), 7.72 (d, *J =* 8.6 Hz, 1H), 7.62 (t, *J =* 5.8 Hz, 1H), 5.94 (s, 1H), 5.44 - 5.34 (m, 3H), 5.04 (d, *J =* 10.6 Hz, 2H), 4.82 (s, 1H), 4.75 (s, 1H), 4.63 (d, *J =* 3.6 Hz, 1H), 4.55 (q, *J =* 3.2, 1.6 Hz, 3H), 4.48 (d, *J =* 5.2 Hz, 1H), 4.41 (d, *J =* 7.2 Hz, 1H), 4.33 (t, *J =* 5.6 Hz, 1H), 4.26 (d, *J =* 10.6 Hz, 1H), 4.22 - 4.16 (m, 3H), 4.09 (d, *J* = 4.4 Hz, 3H), 3.98 (q, *J* = 5.6, 4.6 Hz, 2H), 3.90 (d, *J* = 5.6 Hz, 1H), 3.83 - 3.71 (m, 5H), 3.65 (s, 4H), 3.54 (d, *J =* 5.4 Hz, 3H), 3.52 - 3.45 (m, 6H), 3.26 (d, *J =* 3.0 Hz, 3H), 3.04 (dt, *J =* 14.2, 7.0 Hz, 2H), 2.94 (d, *J =* 5.6 Hz, 2H), 2.86 - 2.81 (m, 1H), 2.80 - 2.69 (m, 3H), 2.54 (s, 2H), 2.46 - 2.34 (m, 5H), 2.31 - 2.11 (m, 9H), 2.04 - 1.86 (m, 8H), 1.71 - 1.57 (m, 6H), 1.57 - 1.42 (m, 5H), 1.29 (dd, *J* = 27.4, 15.0 Hz, 10H), 1.03 (d, *J* = 6.4 Hz, 3H), 0.81 (dd, *J* = 14.6, 6.8 Hz, 6H).

### Example 60: Preparation of Compound LA-34BVC

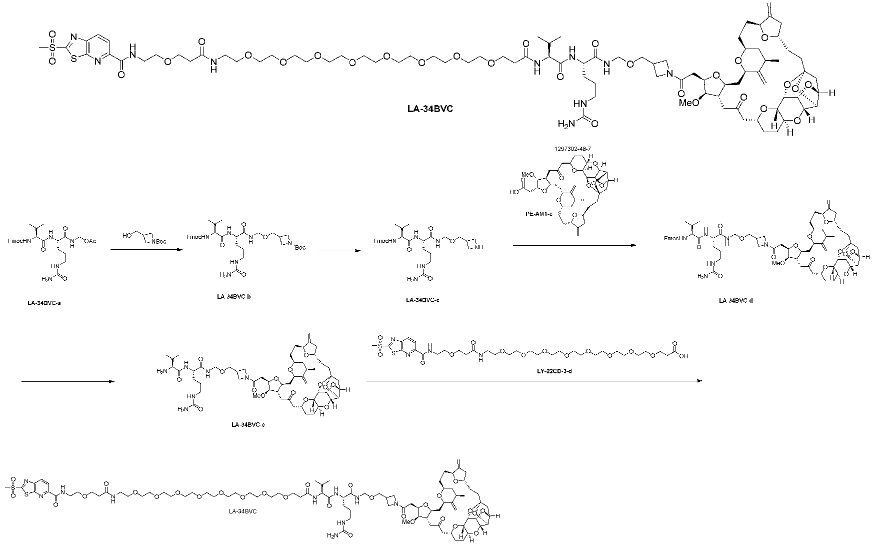

### Step 1: Preparation of compound LA-34BVC-b

TsOH (60.67 mg, 0.352 mmol, 1 eq) was added to the solution of compound (5S,8S)-1-(9H-fluoren-9-yl)-5-isopropyl-3,6,9-trioxo-8-(3-ureapropyl)-2-oxo-4,7,10-triazaundecan-11-yl acetate (commercially purchased) (200 mg, 0.352 mmol, 1 eq) and 3-(hydroxymethyl) azetidine-1-formic acid-2-methylpropyl-2-ylester (263.89 mg, 1.409 mmol, 4 eq) in DMF (5 mL) and THF (5 mL). The reaction solution was allowed to react at 20°C for 18 hours, the LCMS showed that the reaction ended, and the reaction solution was evaporated to dryness and purified by C18 to afford the product LA-34BVC-b (150 mg, 61.27%).

LCMS (ESI): m/z, 695.3[M+H]⁺, Rt=1.676 min.

### Step 2: Preparation of compound LA-34BVC-c

TFA (0.5 mL) was added to the solution of compound LA-34BVC-b (150 mg, 0.054 mmol, 1 eq) in DCM (2.5 mL) at 0°C, the resulting mixture was allowed to react at 0°C for 1 hour, the LCMS showed that the reaction ended, and the reaction solution was evaporated to dryness and purified by C18 (a solution of 40% ACN in H₂O), to afford LA-34BVC-b (102.71 mg, 79.99 %).

LCMS (ESI): m/z, 594.7[M+H]⁺, Rt=1.229 min.

### Step 3: Preparation of compound LA-34BVC-d

DIPEA (54.24 mg, 0.420 mmol, 3 eq) and HATU (63.83 mg, 0.168 mmol, 1.2 eq) were added to a solution of the compound LA-34AVC-b (for the synthesis of the compound, refer to the Preparation Example of LA-34A) (91.59 mg, 0.154 mmol, 1.1 eq) and the compound PE-AM1-c (for the synthesis of the compound, refer to the Preparation Example of PE-AM1) (100.00 mg, 0.140 mmol, 1 eq) in DMF (5 mL) at 0°C, and then the resulting mixture allowed to react for 1 hour at 20°C. The LCMS analysis showed that the reaction ended. The reaction solution was filtered, then purified by reversed-phase C18 (water and acetonitrile; and an elution ratio of acetonitrile increased from 50% to 80%) and lyophilized, to obtain the white solid compound LA-34BVC-d (144.54 mg, 80.03% yield).

LCMS (ESI): m/z, 1291.9[M+H]⁺, Rt=1.657 min.

### Step 4: Preparation of compound LA-34BVC-e

DEA (20 mg, 0.271 mmol, 1.5 eq) was added to a solution of compound LA-34BVC-a (70 mg, 0.054 mmol, 1 eq) in DMF (3 mL) at 0°C. The mixture was allowed to react at 20°C for 1 hour. The LCMS analysis showed that the reaction ended. The mixture was evaporated to dryness to afford LA-34BVC-b (100 mg, where the yield was not calculated for the crude product) as a crude product, which was used directly in the next step.

LCMS (ESI): m/z, 1069.1[M+H]⁺, Rt=1.266 min.

### Step 5: Preparation of compound LA-34BVC

DIPEA (0.031 mL, 0.187 mmol, 2 eq) and HATU (42.67 mg, 0.112 mmol, 1.2 eq) were added to the solution of compound LA-34BVC-b (100 mg, 0.094 mmol, 1 eq) and compound LY-22CD-3-d (for the synthesis of the compound, refer to patent application WO2024175069A1) (89.43 mg, 0.112 mmol, 1.2 eq) in DMF (3 mL) at 0°C. After addition, a resulting mixture was allowed to react at 20°C for 1 hour, LCMS analysis showed that the reaction ended, the reaction solution was filtered and the filter cake was purified by preparative high-performance liquid chromatography (preparative chromatograph manufacturer: Orienda, model: BRIX-2860, column: Welch Xtimate C18 250 × 30 mm × 10 µm, mobile phase: water (0.225% FA)-acetonitrile, with an elution ratio of acetonitrile increased from 20% to 50%) and lyophilized, to afford compound LA-34BVC (16.46 mg, 9.26% yield, 92.35% purity) as a white solid.

LCMS (ESI): m/z, 1848.1[M+H]⁺, Rt=1.358 min.

### Example 61: Preparation of Compound LA-35A

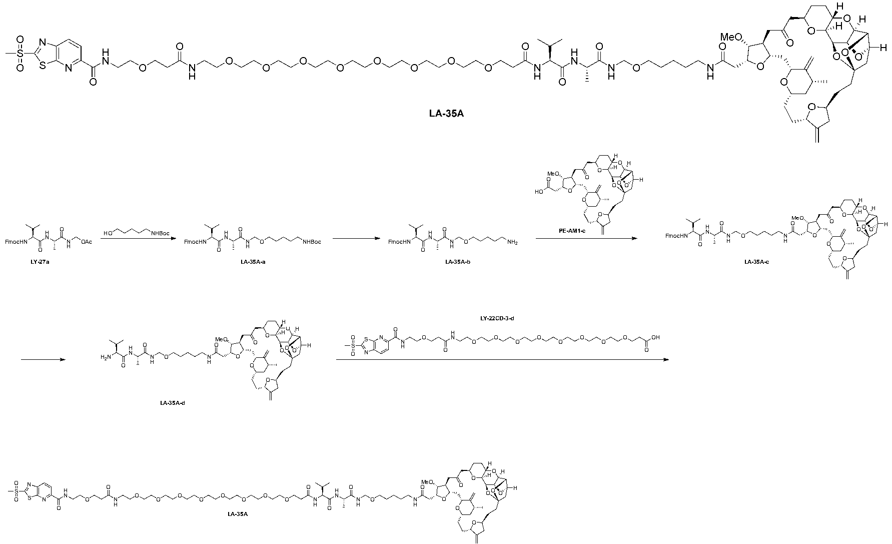

### Step 1: Preparation of compound LA-35A-a

P-toluenesulfonic acid (108.36 mg, 622.99 µmol, 1 eq) was added to the solution of compound LY-27a (300 mg, 622.99 µmol, 1 eq) and 5-(N-tert-butoxycarbonylamino)-1-pentanol (258.45 mg, 1.25 mmol, 2 eq) in tetrahydrofuran (10 mL), then the reaction solution was stirred at 30°C for 1 hour, and LCMS analysis showed complete consumption of the starting material. After concentration, the reaction solution was purified by silica gel column chromatography (C18, ISCO, R-120g SepaFlash Silica Flash Column; eluents: 10%-65% water (0.225% FA)/CH₃CN; 60 mL/min) and lyophilized, to afford compound LA-35A-a (222 mg, yield: 57.04%, purity: 97.76%) as a white solid.

LCMS (ESI): m/z, 647.3[M+Na]⁺.

### Step 2: Preparation of compound LA-35A-b

Trifluoroacetic acid (3.27g, 99%, 2.13 mL, 28.43 mmol, 80 eq) was added to the solution of compound LA-35A-a (222 mg, 355.33 µmol, 1 eq) in dichloromethane (10 mL) at 0°C and then a reaction solution was stirred at 0°C for 1 hour. The LCMS analysis showed complete consumption of the starting material. After concentration, the reaction solution was purified by silica gel column chromatography (C18, ISCO, R-40g SepaFlash Silica Flash Column; eluents: 5%-40% water (0.225% FA)/CH₃CN; 40 mL/min) and lyophilized, to afford compound LA-35A-b (132 mg, yield: 70.81%, purity: 93.11%) as a white solid.

LCMS (ESI): m/z, 525.3[M+H]⁺, 547.3[M+Na]⁺.

### Step 3: Preparation of compound LA-35A-c

DIPEA (13.70 mg, 99%, 17.51 µL, 104.92 µmol, 3 eq) and HATU (16.45 mg, 97%, 41.97 µmol, 1.2 eq) were added to the solution of compounds LA-35A-b (18.35 mg, 34.97 µmol, 1 eq) and PE-AM1-c (for the synthesis of the compound, refer to the Preparation Example of PE-AM1) (25 mg, 34.97 µmol, 1 eq) in DMF (0.5 mL). The resulting mixture was stirred at 25°C for 1 hour, and the LCMS analysis showed that the reaction ended. After the reaction solution was filtered, the filter cake was purified by preparative high-performance liquid chromatography (preparative chromatograph manufacturer: Orienda, model: BRIX-2860, column: Synergi Max-RP 200 × 30mm × 10 µm, mobile phase: water (0.225% HCOOH)-acetonitrile, with an elution ratio of acetonitrile increased from 50% to 80%) and lyophilized, to afford compound LA-35A-c (27 mg, 63.20% yield, 90.13% purity) as a white solid.

LCMS (ESI): m/z, 1221.6[M+H]⁺, 1243.5[M+Na]⁺.

### Step 4: Preparation of compound LA-35A-d

Diethylamine (8.17 mg, 110.52 µmol, 5 eq) was added to the solution of compound LA-35A-c (27 mg, 22.10 µmol, 1 eq) in DMF (0.5 mL), and then a resulting mixture was stirred at 25°C for 1 hour. LCMS showed that the reaction ended, and the reaction solution was concentrated to afford the compound LA-35A-d (22 mg, 99.60% purity) as a white oil.

LCMS (ESI): m/z, 999.7[M+H]⁺, 1021.8[M+Na]⁺.

### Step 5: Preparation of compound LA-35A

DIPEA (8.62 mg, 66.05 µmol, 3 eq) and HATU (10.36 mg, 26.42 µmol, 1.2 eq) were added to the solution of compound LA-35A-d (22 mg, 22.02 µmol, 1 eq) and compound LY-22CD-3-d (17.54 mg, 22.02 µmol, 1 eq) in DMF (1 mL). The resulting mixture was stirred at 25°C for 1 hour, and the LCMS analysis showed that the reaction ended. After the reaction solution was filtered, the residue was purified by preparative high-performance liquid chromatography (preparative chromatograph manufacturer: Orienda, model: BRIX-2860, column: Phenomenex Luna C18 150 × 25 mm × 10 µm, mobile phase: water (0.225% HCOOH)-acetonitrile, with an elution ratio of acetonitrile increased from 30% to 60%) and lyophilized, to afford compound LA-35A (9.47 mg, 24.19% yield, 96.65% purity) as a white solid.

LCMS (ESI): m/z, 1778.8[M+H]⁺, 890.5[(M+2)/2]⁺.

¹H NMR (400 MHz, DMSO-*d*₆) δ 9.06 (t, *J =* 5.7 Hz, 1H), 8.87 (d, *J =* 8.6 Hz, 1H), 8.53 (t, *J =* 6.6 Hz, 1H), 8.38 (d, *J =* 8.6 Hz, 1H), 8.04 (d, *J =* 7.2 Hz, 1H), 7.93 - 7.82 (m, 2H), 7.78 (t, *J* = 5.6 Hz, 1H), 5.03 (d, *J* = 14.9 Hz, 2H), 4.78 (d, *J* = 25.2 Hz, 2H), 4.63 (d, *J* = 4.9 Hz, 1H), 4.57 - 4.44 (m, 3H), 4.29-4.14 (m, 4H), 4.09 (d, *J* = 4.7 Hz, 3H), 4.00 (t, *J* = 5.1 Hz, 2H), 3.79 (t, *J* = 10.6 Hz, 1H), 3.69 (m, 1H), 3.65 (s, 3H), 3.62 (t, *J =* 6.4 Hz, 2H), 3.58 (t, *J =* 6.7 Hz, 2H), 3.53 (d, *J =* 5.2 Hz, 3H), 3.51-3.46 (m, 30H), 3.37 (t, *J =* 5.9 Hz, 2H), 3.31-3.28 (m, 3H), 3.25 (s, 3H), 3.18 (q, *J* = 5.8 Hz, 2H), 2.99 (dp, *J* = 19.8, 6.4 Hz, 2H), 2.86-2.71 (m, 3H), 2.61-2.52 (m, 1H), 2.48-2.37 (m, 3H), 2.33 (t, *J =* 6.3 Hz, 5H), 2.26 (d, *J =* 10.8 Hz, 3H), 2.20 (s, 1H), 2.12 (t, *J =* 12.1 Hz, 1H), 2.06-1.95 (m, 2H), 1.95-1.85 (m, 5H), 1.67 (q, *J =* 11.6, 11.1 Hz, 4H), 1.52 (dd, *J* = 12.8, 5.5 Hz, 1H), 1.48-1.40 (m, 3H), 1.34 (p, *J =* 6.7, 5.9 Hz, 4H), 1.22 (dd, *J =* 13.6, 6.6 Hz, 7H), 1.00 (dd, *J* = 22.3, 9.2 Hz, 4H), 0.82 (dd, *J =* 13.3, 6.8 Hz, 6H).

### Example 62: Preparation of Compound LA-35B

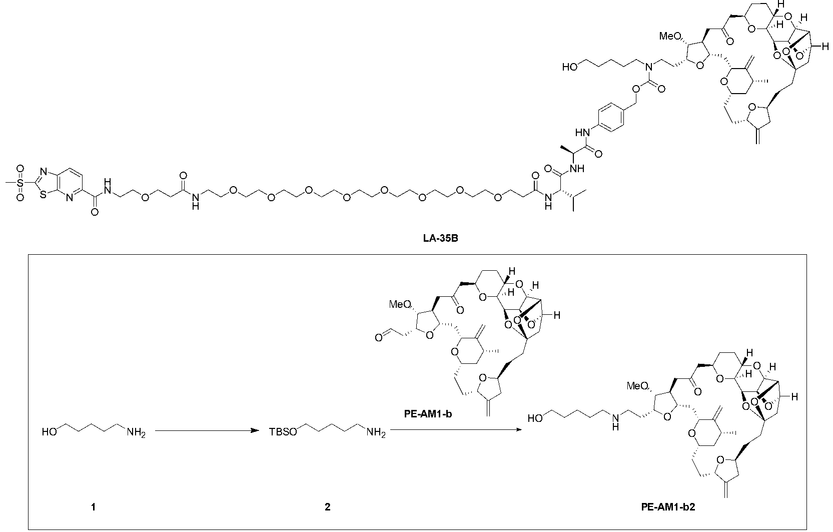

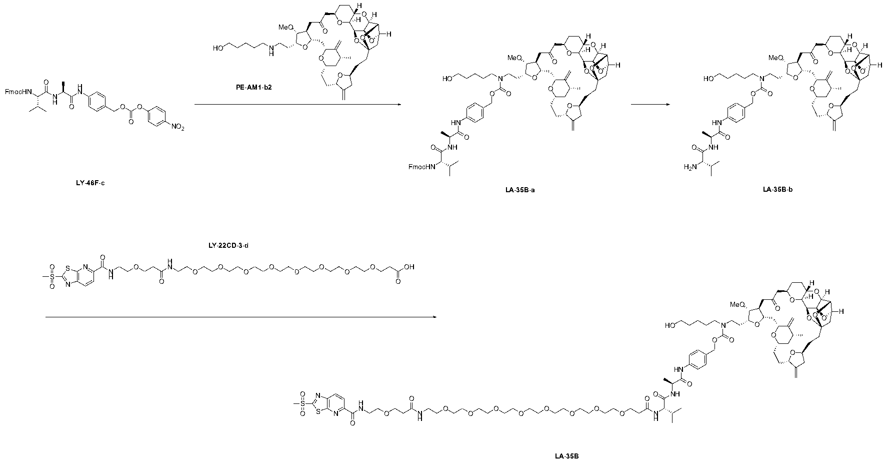

### Step 1: Preparation of compound 5-((tert-butyldimethylsilyl)oxy)pentan-1-amine

5-aminopentan-1-ol (500 mg, 95%, 506.59 µL, 4.60 mmol, 1 eq) was dissolved in DCM (20 mL). Imidazole (791.60 mg, 99%, 712.44 µL, 11.51 mmol, 2.5 eq) was added. TBSCl (779.92 mg, 97.88%, 877.46 µL, 5.06 mmol, 1.1 eq) was added at 0°C, and the mixture was stirred at 25°C for 3 hours. TLC showed the formation of a new spot. The reaction solution was purified by silica gel column chromatography (eluents: DCM:MeOH = 10:1, visualized with phosphomolybdic acid) to afford the product as a yellow oil (1g, 94.89% yield).

¹H NMR (400 MHz, Chloroform-d) *δ* 3.55 (t, *J =* 6.4 Hz, 2H), 2.78 (dd, *J =* 8.2, 6.7 Hz, 2H), 1.57 (p, *J* = 7.6 Hz, 2H), 1.52-1.42 (m, 2H), 1.39-1.25 (m, 2H), 0.84 (s, 9H), 0.40-0.00 (s, 6H).

### Step 2: Preparation of compound PE-AM1-b2

Compound PE-AM1-b (for the synthesis of the compound, refer to the Preparation Example of PE-AM1; 50 mg, 71.55 µmol, 1 eq) was dissolved in DCE (3 mL), and 5-((tertbutyldimethylsilyl)oxy)pentan-1-amine (31.11 mg, 143.09 µmol, 2 eq) and AcOH (4.32 mg, 99.5%, 4.10 µL, 71.55 µmol, 1 eq) were added. The mixture was stirred at 25°C for 20 minutes, then sodium triacetoxyborohydride (30.95 mg, 143.09 µmol, 2 eq) was added, and the mixture was further stirred at 25°C for 40 minutes. The LCMS showed the target product. The reaction solution was subjected to preparative high-performance liquid chromatography to obtain a white solid product (20 mg, 35.56% yield).

The conditions of the preparative high-performance liquid chromatography were as follows:
Preparative chromatograph manufacturer: Shimadzu, model: LC-20AP, column: YMC-Triart Prep C18 250 × 50 mm × 7 µm, mobile phase: water (0.225% HCOOH)-acetonitrile, with an elution ratio of acetonitrile increased from 30% to 60%.

¹H NMR (400 MHz, DMSO-*d⁶*) *δ* 8.33 (s, 1H), 5.03 (s, 1H), 4.96 (s, 1H), 4.81 (s, 1H), 4.73 (s, 1H), 4.61 (dt, *J =* 4.5, 2.7 Hz, 1H), 4.53 (t, *J* = 4.2 Hz, 1H), 4.24 (d, *J* = 10.8 Hz, 1H), 4.19-3.96 (m, 6H), 3.78 (t, *J* = 10.3 Hz, 2H), 3.67 (s, 4H), 3.57-3.46 (m, 9H), 3.25 (s, 3H), 2.82 (dd, *J* = 9.6, 1.9 Hz, 1H), 2.78-2.60 (m, 7H), 2.54 (dd, *J* = 16.3, 9.7 Hz, 1H), 2.34-2.06 (m, 7H), 2.02-1.83 (m, 6H), 1.81-1.55 (m, 6H), 1.54-1.36 (m, 6H), 1.34-1.09 (m, 7H), 0.98 (dd, *J* = 23.4, 9.1 Hz, 4H), 0.84 (s, 5H), 0.01 (s, 3H).

### Step 3: Preparation of compound LA-35B-a

The compound LY-46F-c (14.93 mg, 87%, 19.08 µmol, 1 eq) was dissolved in DMF (1 mL), and PE-AM1-b2 (15 mg, 19.08 µmol, 1 eq) and DIPEA (4.98 mg, 99%, 6.31 µL, 38.17 µmol, 2 eq) were added. The reaction solution was stirred at 25°C for 2 hours. The LCMS showed that the product was generated. The reaction solution was subjected to preparative high-performance liquid chromatography to obtain a white solid product (5 mg, 19.74% yield).

The conditions of the preparative high-performance liquid chromatography were as follows:
preparative chromatograph manufacturer: Orienda, model: BRIX-2860, column: Phenomenex Luna C18 250 × 50 mm × 10 µm, mobile phase: water (0.225% HCOOH)-acetonitrile, with an elution ratio of acetonitrile increased from 50% to 80%.

LCMS (ESI): m/z, 1349.7 [M+Na]⁺.

### Step 4: Preparation of compound LA-35B-b

Compound LA-35B-a (5 mg, 3.77 µmol, 1 eq) was dissolved in DMF (1 mL), Et₂NH (1.38 mg, 18.83 µmol, 5 eq) was added, and the reaction solution was stirred at 25°C for 2 hours. LCMS analysis showed that the reaction ended. The reaction solution was directly evaporated to dryness, and the residue was used for the next step.

LCMS (ESI): m/z, 1105.5[M+H]⁺.

### Step 5: Preparation of compound LA-35B

The LA-35B-b (4 mg, 3.62 µmol, 1 eq) was dissolved in DMF (1 mL), LY-22CD-3-d (2.88 mg, 3.62 µmol, 1 eq) and DIPEA (944.80 µg, 7.24 µmol, 2 eq) were added, finally HATU (1.65 mg, 4.34 µmol, 1.2 eq) was added, and the reaction solution was stirred at 25°C for 1 hour. The LCMS analysis indicated complete consumption of the starting material. The reaction solution was combined with the product from another small-scale trial at one-fourth of the scale, and the mixture was purified by high-performance liquid chromatography to afford the product (4.22 mg, 45.09% yield, 97% purity) as a white solid.

LCMS (ESI): m/z, 1905.9[M+Na]⁺.

### Example 63: Preparation of Compound LA-35C

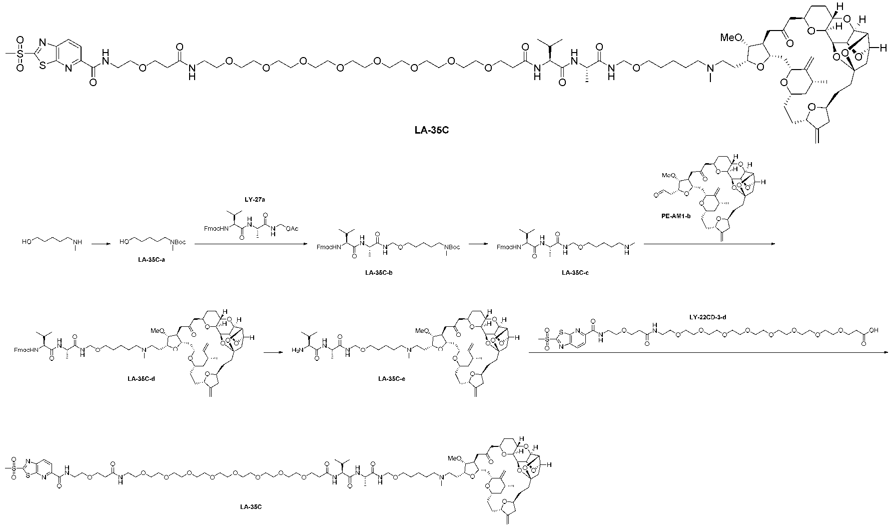

### Step 1: Preparation of compound LA-35C-a

Compound (Boc)₂O (486.54 mg, 2.18 mmol, 1.1 eq) was added to a solution of 5-(methylamino)pentan-1-ol (245mg, 1.99 mmol, 1 eq) in MeOH (5 mL). The reaction solution was stirred at room temperature overnight. TLC analysis showed that the reaction ended. The reaction solution was evaporated to dryness under reduced pressure. The residue was purified by silica gel column chromatography (eluents: ethyl acetate/petroleum ether = 0:1 to 1: 2) to afford the product LA-35C-a (352 mg, 81.56% yield) as a colorless oil.

¹H NMR (400 MHz, DMSO-*d*₆) δ 4.35 (t, *J* = 5.2 Hz, 1H), 3.41-3.34 (m, 2H), 3.33 (s, 3H), 3.12 (t, *J* = 7.2 Hz, 2H), 1.47-1.39 (m, 4H), 1.39-1.37 (m, 9H), 1.27-1.17 (m, 2H).

### Step 2: Preparation of compound LA-35C-b

TsOH (140.87 mg, 809.90 µmol, 1 eq) was added to a solution of compounds LA-35C-a (352 mg, 1.62 mmol, 2 eq) and compound LY-27a (390.01 mg, 809.90 µmol, 1 eq) in THF (5 mL) and the mixture was stirred at 35°C for 2 hours. LCMS analysis showed that the reaction ended. The reaction solution was purified by silica gel column chromatography (C18, ISCO, R-120g SepaFlash Silica Flash Column, eluents: 10%-65% water (0.225%FA)/CH₃CN, 100 mL/min), to afford the product LA-35C-b (517 mg, the yield was calculated for the crude product) as a white solid. LCMS (ESI): m/z, 661.3[M+Na]⁺.

### Step 3: Preparation of compound LA-35C-c

TFA (4 mL) was added to a solution of compound LA-35C-b (517 mg, crude) in DCM (20 mL) at 0°C. The reaction solution was stirred at room temperature for 1 hour. LCMS analysis showed that the reaction ended. The reaction solution was evaporated to dryness under reduced pressure. The residue was purified by silica gel column chromatography (C18, ISCO, R-120g SepaFlash Silica Flash Column, eluents: 5%-40% water (0.225%FA)/CH₃CN, 100 mL/min), to afford the product LA-35C-c (158.80 mg, 36.42% yield) as a white solid. LCMS (ESI): m/z, 539.3[M+H]⁺.

### Step 4: Preparation of compound LA-35C-d

Acetic acid (73.45 mg, 57.24 µmol, 2 eq) was added to a solution of compound LA-35C-c (15.42 mg, 28.62 µmol, 1 eq) and PE-AM1-b (20 mg, 28.62 µmol, 1 eq; for its synthesis, refer to the Preparation Example of PE-AM1) in DCE (5 mL). The reaction solution was stirred at room temperature for 20 minutes. Sodium triacetoxyborohydride (12.38 mg, 57.24 µmol, 2 eq) was added to the reaction solution. The reaction solution was stirred at room temperature for 40 minutes. LCMS analysis showed that the reaction ended. The reaction solution was evaporated to dryness under reduced pressure. The mixture was purified by preparative high-performance liquid chromatography (preparative chromatograph manufacturer: Orienda, model: BRIX-2860, column: Synergi Max-RP 250 × 40 mm × 10 µm, mobile phase: water (0.225% HCOOH)-acetonitrile, with an elution ratio of acetonitrile increased from 50% to 80%), to afford product LA-35C-d (13.60 mg, 38.90% yield) as a white solid. LCMS (ESI): m/z, 1221.6[M+H]⁺.

### Step 5: Preparation of compound LA-35C-e

Diethylamine (4.11 mg, 55.67 µmol, 5 eq) was added to the solution of compound LA-35C-d (13.60 mg, 11.13 µmol, 1 eq) in DMF (2 mL). The reaction solution was stirred at 20°C for 1 hour. LCMS analysis showed that the reaction ended. The reaction solution was evaporated to dryness under reduced pressure to afford the product LA-35C-e (11.13 mg, where the yield was not calculated for the crude product) as a colorless oil. LCMS (ESI): m/z, 999.8[M+Na]⁺.

### Step 6: Preparation of compound LA-35C

DIPEA (4.36 mg, 33.41 µmol, 3 eq) and HATU (4.24 mg, 11.14 µmol, 1 eq) were added to a solution of compound LA-35C-e (11.13 mg, crude) and LY-22CD-3-d (8.88 mg, 11.14 µmol, 1 eq) in DMF (2 mL), and the reaction solution was stirred at room temperature for 2 hours. LCMS analysis showed that the reaction ended. The reaction solution was purified by preparative high-performance liquid chromatography (preparative chromatograph manufacturer: Orienda, model: BRIX-2860-R1, column: Welch Xtimate C18 250 × 30 mm × 10 µm, mobile phase: water (0.1% TFA)-acetonitrile, with an elution ratio of acetonitrile increased from 30% to 60%), to obtain product LA-35C (8.30 mg, 41.17% yield) as a white solid. LCMS (ESI): m/z, 1778.8[M+H]⁺.

¹H NMR (400 MHz, DMSO-*d*₆) δ 9.09-8.96 (m, 2H), 8.87 (d, *J* = 8.4 Hz, 1H), 8.58-8.50 (m, 1H), 8.38 (d, *J* = 8.4 Hz, 1H), 8.03 (d, *J* = 7.2 Hz, 1H), 7.94-7.82 (m, 2H), 5.01 (d, *J* = 33.2 Hz, 3H), 4.78 (d, *J* = 18.0 Hz, 2H), 4.67-4.61 (m, 1H), 4.59-4.43 (m, 3H), 4.30-3.98 (m, 8H), 3.84-3.43 (m, 37H), 3.40-3.36 (m, 3H), 3.31-2.94 (m, 13H), 2.90-2.68 (m, 6H), 2.61-2.54 (m, 1H), 2.47-2.06 (m, 12H), 2.04-1.78 (m, 9H), 1.75-1.40 (m, 10H), 1.38-1.14 (m, 9H), 1.06-0.92 (m, 4H), 0.87-0.79 (m, 5H).

### Example 64: Preparation of Compound LA-36

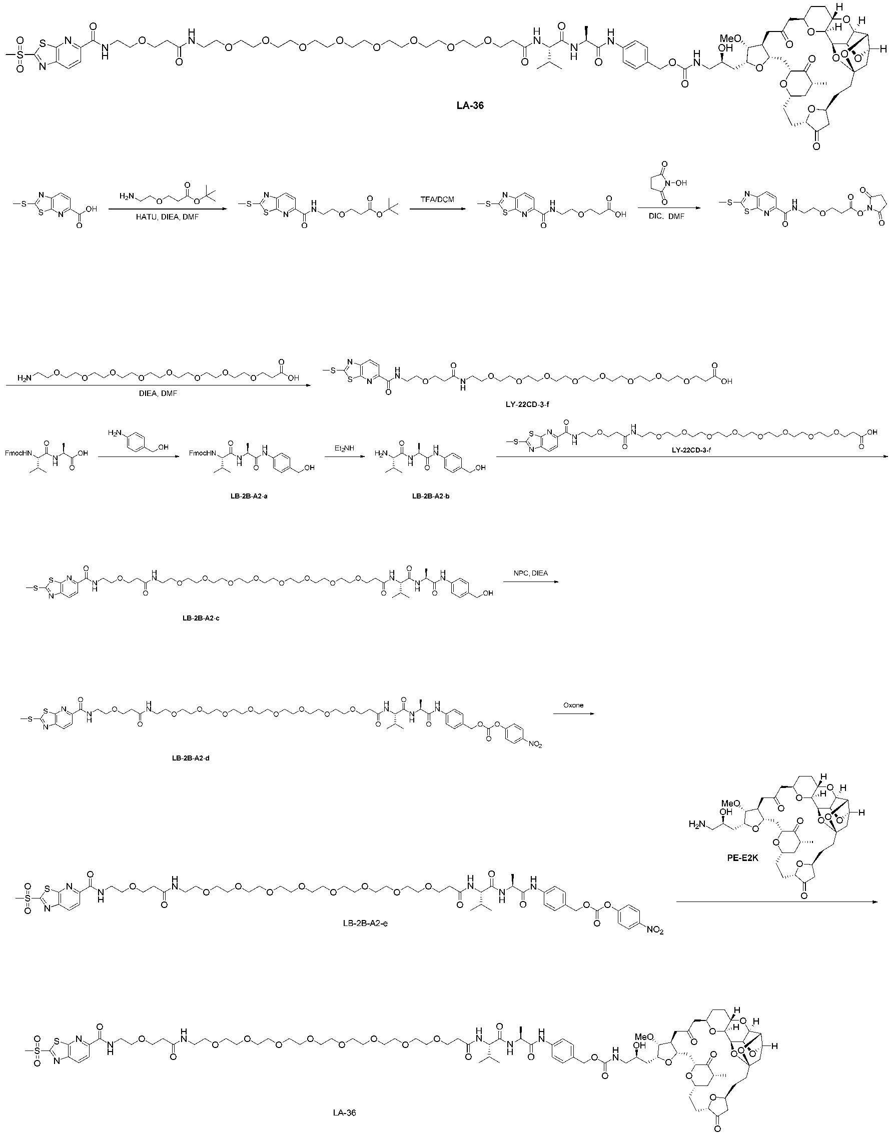

### Step 1: Preparation of compound LB-2B-A2-a

EEDQ (1.27g, 4.87 mmol, 2 eq) was added to a mixture of Fmoc-Val-Ala-OH (1.03g, 2.44 mmol, 1 eq) and 4-aminobenzyl alcohol (618.62 mg, 4.87 mmol, 2 eq) in dichloromethane (30 mL) and methanol (15 mL) at 0°C. The reaction solution was stirred at 40°C for 2 hours. LCMS analysis indicated complete consumption of the starting material. After concentration, the residue was triturated with (petroleum ether:ethyl acetate = 2:1, 90 mL). After filtration, the filter cake was afforded as a white solid, LB-2B-A2-a (1.01g, 80.40% yield).

LCMS (ESI): m/z, 516[M+H]+, Rt=1.673 min.

### Step 2: Preparation of LB-2B-A2-b

Et₂NH (146.31 µL, 1.41 mmol, 5 eq) was added to a solution of compound LB-2B-A2-a (145 mg, 281.23 µmol, 1 eq) in DCM (3 mL), the resulting mixture was allowed to react at 25°C for 1 hour, and the LCMS analysis showed that the reaction ended. The reaction solution was concentrated, to afford LB-2B-A2-b (82.50 mg; the yield was not calculated for the crude product) as a pale yellow solid, which was directly used in the next step.

LCMS (ESI): m/z, 294[M+H]⁺, Rt=0.265 min.

### Step 3: Preparation of compound LB-2B-A2-c

### Preparation of compound LY-22CD-3-f

DIPEA (571.18 mg, 730.40 µL, 4.42 mmol, 2 eq) was added to a solution of 2-(methylsulfanyl)thiazolo[5,4-b]pyridine-5-carboxylic acid (for the synthesis of the compound, refer to the Preparation Example of LY-22CD-3-d) (500 mg, 2.21 mmol, 1 eq) and tert-butyl 3-(2-aminoethoxy)propanoate (418.20 mg, 2.21 mmol, 1 eq) in DMF (7 mL) at 0°C, and HATU (1.01g, 2.65 mmol, 1.2 eq) was then added. The mixture was stirred at 20°C for 2 hours. LCMS analysis showed nearly complete consumption of the starting material. The reaction solution was added into a solution of ethyl acetate/water/ice (1V/1V/1V, 100 mL), and then the layers were separated. The aqueous phase was extracted with EA (50 mL × 2). The organic phases were combined, washed with water (50 mL × 2) and brine (50 mL), dried over anhydrous sodium sulfate, and filtered to obtain the filtrate. The filtrate was concentrated under reduced pressure to afford the crude product. The crude product was purified by silica gel column chromatography (eluents: dichloromethane/methanol = 20:1) to afford tert-butyl 3-(2-(2-(methylsulfanyl)thiazolo[5,4-b] pyridine-5-carboxamido)ethoxy)propanoate (800 mg, crude, 77.41% yield, 85% purity).

LCMS (ESI): m/z, 420.2[M+Na]⁺.

1H NMR (400 MHz, Chloroform-d) δ 8.27 (d, J = 8.4 Hz, 1H), 8.13 (d, J = 8.4 Hz, 2H), 3.74 (t, J = 6.2 Hz, 2H), 3.70-3.64 (m, 4H), 2.82 (s, 3H), 2.52 (t, J = 6.3 Hz, 2H), 1.45 (s, 9H).

TFA (2.5 mL) was added to a solution of tert-butyl 3-(2-(2-(methylsulfanyl)thiazolo[5,4-b] pyridine-5-carboxamido)ethoxy)propanoate (800 mg, 85%, 1.71 mmol, 1 eq) in DCM (10 mL) at 0°C. After the addition, the mixture was stirred at 25°C for 2 hours. LCMS analysis showed that the reaction ended. The reaction solution was concentrated to afford compound 3-(2-(2-(methylsulfanyl) thiazolo[5,4-b]pyridine-5-carboxamido)ethoxy)propanoic acid as a yellow oil (590 mg, crude).

LCMS (ESI): m/z, 342[M+H]⁺.

DIC (1.31g, 1.62 mL, 10.37 mmol, 6 eq) was added to a solution of 3-(2-(2-(methyl sulfanyl)thiazolo[5,4-b]pyridine-5-carboxamido)ethoxy)propanoic acid (590 mg, 1.73 mmol, 1 eq) and NHS (1.19g, 745.86 µL, 10.37 mmol, 6 eq) in DMF (8 mL) at room temperature. The reaction solution was stirred at 50°C for 2 hours. LCMS analysis indicated complete consumption of the starting material. The reaction solution was added into a solution of ethyl acetate/water/ice (1V/1V/1V, 100 mL), and then the layers were separated. The aqueous phase was extracted with EA (50 mL × 2). The organic phases were combined, washed with water (50 mL × 2) and brine (50 mL), dried over anhydrous sodium sulfate, and filtered to obtain the filtrate. The filtrate was concentrated under reduced pressure to afford the crude product. The crude product was purified by silica gel column chromatography (eluents: dichloromethane/methanol = 20:1) to afford 2,5-dioxopyrrolidin -1-yl 3-(2-(2-(methylsulfanyl)thiazolo[5,4-b]pyridine-5-carboxamido)ethoxy)propanoate (1.47g, crude, 97% yield, 50% purity, containing DIC byproduct, which does not affect the subsequent reaction).

LCMS (ESI): m/z, 439.2[M+H]⁺.

DIPEA (117.09 mg, 149.73 µL, 906 µmol, 4 eq) was added to a solution of 2,5-dioxo pyrrolidin-1-yl 3-(2-(2-(methylsulfanyl)thiazolo[5,4-b]pyridine-5-carboxamido)ethoxy)propanoate (198.63 mg, 50%, 226.50 µmol, 1 eq) and 27-amino-4,7,10,13,16,19,22,25-octaoxa heptacosanoic acid (185 mg, 168.18 µL, 419.03 µmol, 1.85 eq) in DMF (2 mL) at 0°C. After the addition, the mixture was stirred at 20°C for 2 hours. LCMS analysis indicated complete consumption of the starting material. The reaction solution was purified by C18 silica gel column chromatography (eluents: water/CH₃CN = 60%/40%) and lyophilized to afford compound LY-22CD-3-f (135 mg, 77.92% yield) as a yellow oil.

LCMS (ESI): m/z, 765.5[M+H]⁺.

DIPEA (85.36 µL, 511.32 µmol, 2 eq) and HATU (116.65 mg, 306.79 µmol, 1.2 eq) were added to a solution of compounds LB-2B-A2-b (82.50 mg, 281.22 µmol, 1.1 eq) and LY-22CD-3-f (226.47 mg, 296.08 µmol, 0.9 eq) in DMF (5 mL) at 0°C. The mixture was allowed to react at room temperature for 1 hour. LCMS analysis indicated the reaction ended. The residue was purified by silica gel column chromatography (C18, ISCO, R-330g SepaFlash Silica Flash Column, eluents: 5%-40% H₂O/CH₃CN; 100 mL/min) (medium pressure), to afford LB-2B-A2-c (236.60 mg, 71.17% yield, 80% purity) as a yellow oil.

LCMS (ESI): m/z, 1040[M+H]⁺, Rt=1.530 min.

### Step 4: Preparation of compound LB-2B-A2-d

DIPEA (182.25 µL, 1.09 mmol, 6 eq) and NPC (228.26 mg, 727.82 µmol, 4 eq) were added to the solution of compound LB-2B-A2-c (236.60 mg, 181.95 µmol, 1 eq) in DMF (6 mL), the reaction solution was stirred at 35°C for 2 hours, the solution was concentrated by an oil pump, and the residue was purified by silica gel column chromatography (eluents: ethyl acetate/petroleum ether = 0%-35%; and methanol/dichloromethane = 0%-10%), to afford LB-2B-A2-d as a pale yellow oil (212.60 mg, yield: 96.94%).

LCMS (ESI): m/z, 1205[M+H]+, Rt=2.252 min.

### Step 5: Preparation of compound LB-2B-A2-e

Oxone (1.21g, 7.06 mmol, 40 eq) was added to a solution of compound LB-2B-A2-d (212.60 mg, 176.38 µmol, 1 eq) in THF (5 mL) and H₂O (5 mL) at 0°C. The mixture was stirred at 25°C overnight, and LCMS analysis indicated complete consumption of the starting material. After filtration, the mixture was concentrated. Water (5 mL) and dichloromethane (5 mL) were added, and the aqueous phase was extracted with dichloromethane (3 × 5 mL). The organic phases were combined, washed with saturated brine (10 mL) once, dried over anhydrous sodium sulfate, filtered and concentrated. The residue was then purified by silica gel column chromatography (eluents: methanol/dichloromethane = 0%-10%) to afford LB-2B-A2-e (175.50 mg, 80.41% yield) as a yellow oil.

LCMS (ESI): m/z, 1237[M+H]⁺, Rt=2.004 min.

### Step 6: Preparation of compound LA-36

DIPEA (7.65 mg, 58.60 µmol, 4 eq) and HOBt (2 mg, 14.65 µmol, 1 eq) were added to a solution of compounds PE-E2K (10.75 mg, 14.65 µmol, 1 eq) and LB-2B-A2-e (12 mg, 9.70 µmol, 0.66 eq) in DMF (2 mL) at 0°C. The reaction solution was stirred at room temperature for 2 hours. LCMS analysis showed that the reaction ended. The reaction solution was evaporated to dryness under reduced pressure. The mixture was purified by preparative high-performance liquid chromatography (preparative chromatograph manufacturer: Orienda, model: BRIX-2860, column: Welch Xtimate C18 150 × 30 mm × 10 µm, mobile phase: water (0.225% FA)-acetonitrile, with an elution ratio of acetonitrile increased from 20% to 50%), to obtain product LA-36 (11.50 mg, 42.85% yield) as a white solid. LCMS (ESI): m/z, 1832.6[M+H]⁺.

¹H NMR (400 MHz, DMSO-*d*₆) δ 9.91 (s, 1H), 9.05 (t, *J =* 5.6 Hz, 1H), 8.87 (d, *J =* 8.8 Hz, 1H), 8.38 (d, *J =* 8.8 Hz, 1H), 8.17 (d, *J =* 7.2 Hz, 1H), 7.92-7.84 (m, 2H), 7.60-7.53 (m, 2H), 7.32-7.25 (m, 2H), 7.08 (t, *J =* 5.6 Hz, 1H), 4.93 (d, *J =* 2.4 Hz, 2H), 4.64 (t, *J =* 4.4 Hz, 1H), 4.56 (t, *J* = 3.6 Hz, 2H), 4.44-4.34 (m, 2H), 4.25-4.06 (m, 5H), 3.89-3.71 (m, 5H), 3.67-3.57 (m, 6H), 3.56-3.43 (m, 25H), 3.37 (t, *J* = 6.0 Hz, 3H), 3.29-3.13 (m, 8H), 2.96 (t, *J* = 6.0 Hz, 2H), 2.89-2.70 (m, 4H), 2.69-2.53 (m, 3H), 2.46-2.29 (m, 8H), 2.28-1.87 (m, 11H), 1.83-1.37 (m, 13H), 1.35-1.10 (m, 6H), 1.01-0.80 (m, 8H).

### Example 65: Preparation of Compound LA-36dPAB

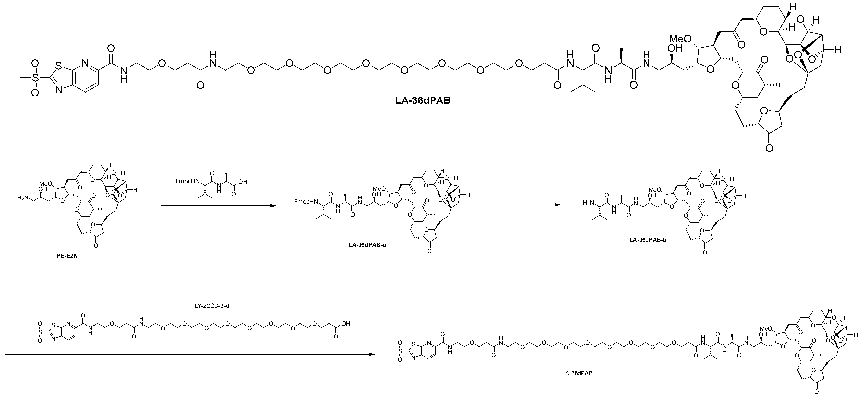

### Step 1: Preparation of compound LA-36dPAB

Diethylamine (0.012 mL, 0.120 mmol, 10 eq) was added to the solution of compound LA-36dPAB-a (13 mg, purity: 82% purity 0.012 mmol, 1 eq) in DMF (1 mL) at room temperature, the reaction solution was stirred at 25°C for 15 minutes, and the LCMS analysis indicated complete consumption of the starting material. The reaction solution was concentrated under reduced pressure to afford the crude product LA-36dPAB-b as a yellow oil. The crude product was directly used for the next step without purification.

LCMS (ESI): m/z, 903.9 [(M+H)]⁺.

### Step 2: Preparation of compound LA-36dPAB

DIEA (3 mg, 0.022 mmol, 2 eq) and HATU (4.2 mg, 0.011 mmol, 1.0 eq) were added to the solution of compounds LA-36dPAB-b (10.0 mg, 0.011 mmol, 1.0 eq) and LY-22CD-3-d (for the synthesis of the compound, refer to patent application WO2024175069A1) (9.70 mg, 0.012 mmol, 1.1 eq) in DMF (1.0 mL) at 0°C, the reaction solution was stirred for 30 minutes at 25°C, and the LCMS analysis indicated complete consumption of the starting material. The reaction solution was purified by prep-HPLC (mobile phase: water (0.1% TFA)/acetonitrile; with an elution ratio of acetonitrile increased from 25% to 55%), to afford product LA-36dPAB (4.21 mg, 22.39% yield) as a white solid.

LCMS (ESI): m/z, 1682.6 [(M+H)]⁺.

¹H NMR (400 MHz, DMSO-*d*₆) δ 9.05 (s, 1H), 8.87 (d, *J =* 8.6 Hz, 1H), 8.38 (d, *J =* 8.6 Hz, 1H), 7.95 (d, *J* = 7.4 Hz, 1H), 7.88 (dd, *J =* 16.4, 7.2 Hz, 2H), 7.72 (t, *J =* 5.6 Hz, 1H), 4.60 (d, *J= 32.8* Hz, 3H), 4.26 (s, 4H), 4.19 - 4.08 (m, 7H), 3.76 (dd, *J* = 18.4, 6.0 Hz, 9H), 3.66 - 3.50 (m, 29H), 3.26 - 3.15 (m, 9H), 3.09 - 2.62 (m, 9H), 2.32 (dd, *J =* 12.4, 5.6 Hz, 4H), 2.02 - 1.85 (m, 6H), 1.80 - 1.37 (m, 15H), 1.20 (t, *J =* 10.8 Hz, 6H), 0.94 (d, *J =* 6.4 Hz, 3H), 0.84 - 0.80 (m, 6H).

### Example 66: Preparation of Compound LA-36O2VC

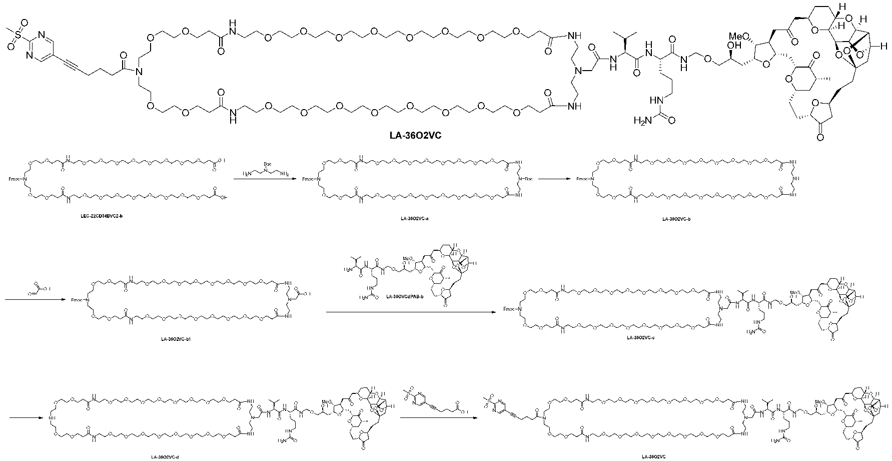

### Step 1: Preparation of compound LA-36O2VC-a

HATU (261.86 mg, 0.689 mmol, 2.0 eq) and DIEA (178 mg, 1.377 mmol, 4.0 eq) were added to a solution of compound LEC-22CD34BVC2-b (484.0 mg, 0.344 mmol, 1.0 eq) and tert-butyl bis(2-aminoethyl)carbamate (70.0 mg, 0.344 mmol, 1.0 eq) in DMF (70.0 mL) at room temperature, and the mixture was stirred at room temperature overnight. The LCMS analysis indicated the reaction ended. The reaction solution was concentrated under reduced pressure to afford a crude product, the crude product was purified by C18 reversed-phase column chromatography (mobile phase: water (0.1% TFA)-acetonitrile, with the elution ratio of acetonitrile increased from 5% to 60%), to afford the product LA-36O2VC-a (360.0 mg, 66.43% yield) as a yellow oil.

LCMS (ESI): m/z, [M]⁺ = 1573.9.

### Step 2: Preparation of compound LA-36O2VC-b

TFA (2 mL) was added to a solution of compound LA-36O2VC-a (360 mg, 0.229 mmol, 1.0 eq) in dichloromethane (6.0 mL) at room temperature. The mixture was stirred at room temperature overnight. LCMS analysis indicated the reaction ended. The reaction solution was concentrated under reduced pressure to afford a crude product, the crude product was purified by C18 reversed-phase column chromatography (mobile phase: water (0.05% NH₃)-acetonitrile, with the elution ratio of acetonitrile increased from 5% to 60%), to afford the product LA-36O2VC-b (240.0 mg, 71.20% yield) as a yellow oil.

LCMS (ESI): m/z, [M]⁺ = 1573.3.

### Step 3: Preparation of compound LA-36O2VC-b1

Acetic acid (43 mg, 0.712 mmol, 5.0 eq) was added to a solution of compound LA-36O2VC-b (210 mg, 0.142 mmol, 1.0 eq) and glyoxylic acid (50% aqueous solution, 0.162 mL, 10.0 eq) in ethanol (14.0 mL) at room temperature. The mixture was stirred at room temperature for 20 minutes. Then NaBH₃CN (89.54 mg, 1.425 mmol, 10.0 eq) was added, and the mixture was stirred at room temperature overnight. LCMS analysis showed that the reaction ended. The reaction solution was purified by C18 reversed-phase column chromatography (mobile phase: water (0.1% TFA)-acetonitrile, with the elution ratio of acetonitrile increased from 5% to 60%), to afford the product LA-36O2VC-b1 (130 mg, 59.56% yield) as a yellow oil.

LCMS (ESI): m/z, [M]⁺ = 1531.2.

### Step 4: Preparation of compound LA-36O2VC-c

HATU (11.48 mg, 0.030 mmol, 1.1 eq) and DIEA (11 mg, 0.082 mmol, 3.0 eq) were added to a solution of compound LA-36O2VC-b1 (42.0 mg, 0.027 mmol, 1.0 eq) and LA-36OVCdPAB-b (28 mg, 0.027 mmol, 1.0 eq) in DMF (1.5 mL) at room temperature, and the mixture was stirred at room temperature overnight. The LCMS analysis indicated the reaction ended. The reaction solution was purified by preparative high-performance liquid chromatography (model: JB-C235-04, column: Bonnasil-BS C18, 20 × 250 mm × 8 µm; mobile phase: water (0.1% TFA)-acetonitrile, with an elution ratio of acetonitrile increased from 20% to 60%), to afford product LA-36O2VC-c (17.0 mg, 24.44% yield) as a white solid.

LCMS (ESI): m/z, [M/2]⁺ = 1266.7.

### Step 5: Preparation of compound LA-36O2VC-d

Diethylamine (5 mg, 0.067 mmol, 10.0 eq) was added to a solution of compound LA-36O2VC-c (17.0 mg, 0.007 mmol, 1.0 eq) in DMF (1.0 mL) at room temperature. The mixture was stirred at room temperature for 1 hour. The LCMS analysis indicated the reaction ended. The reaction solution was concentrated under reduced pressure, to afford the crude product LA-36O2VC-d (the yield was not calculated for the crude product), which was directly used in the next step without purification.

LCMS (ESI): m/z, [M/2]⁺ = 1155.7.

### Step 6: Preparation of compound LA-36O2VC

HATU (3.19 mg, 0.008 mmol, 1.2 eq) and DIEA (2.7 mg, 0.021 mmol, 3.0 eq) were added to the solution of the crude product LA-36O2VC-d and 6-(2-(methylsulfonyl)pyrimidin-5-yl)hex-5-ynoic acid (2.25 mg, 0.008 mmol, 1.2 eq) in DMF (1.0 mL) at room temperature, and the reaction solution was stirred at room temperature for 1 hour. LCMS analysis showed that the reaction ended. The reaction solution was purified by preparative high-performance liquid chromatography (model: JB-C235-04, column: Bonnasil-BS C18, 20 × 250 mm × 8 µm; mobile phase: water (0.1% FA)-acetonitrile, with an elution ratio of acetonitrile increased from 30% to 50%), to afford product **LA-36O2VC** (7.82 mg, 46.61% yield) as a transparent gelatinous granular solid.

LCMS (ESI): m/z, [M/2]⁺ = 1280.8.

¹H NMR (400 MHz, DMSO-*d₆*) δ 9.11 (s, 2H), 8.62 (t, *J =* 6.5 Hz, 1H), 8.20 (d, *J =* 7.0 Hz, 1H), 7.89 - 7.80 (m, 4H), 7.73 (d, *J* = 9.1 Hz, 1H), 5.92 (t, *J* = 6.0 Hz, 1H), 5.38 (s, 2H), 4.65 - 4.52 (m, 4H), 4.49 - 4.37 (m, 2H), 4.29 - 4.20 (m, 2H), 4.17 - 4.09 (m, 4H), 3.88 - 3.74 (m, 5H), 3.59 - 3.39 (m, 99H), 3.25 (s, 3H), 3.21 - 3.16 (m, 6H), 3.14 - 3.05 (m, 5H), 2.97 - 2.92 (m, 2H), 2.88 - 2.83 (m, 2H), 2.78 - 2.72 (m, 1H), 2.57 (d, *J =* 5.8 Hz, 4H), 2.35 - 2.28 (m, 10H), 2.18 - 2.11 (m, 2H), 2.09 - 1.89 (m, 9H), 1.85 - 1.77 (m, 4H), 1.75 - 1.69 (m, 2H), 1.66 - 1.58 (m, 4H), 1.56 - 1.41 (m, 7H), 1.25 (d, *J=* 15.8 Hz, 8H), 0.94 (d, *J* = 6.4 Hz, 3H), 0.87 - 0.79 (m, 7H).

### Example 67: Compound LA-36OdPAB

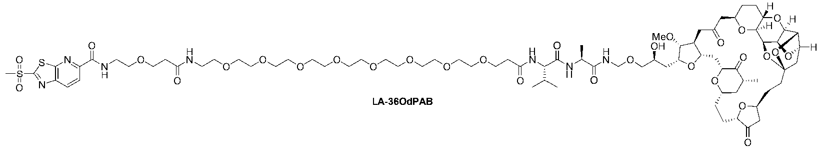

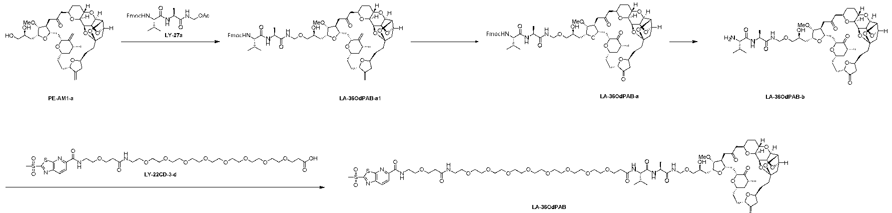

### Step 1: Preparation of compound LA-36OdPAB-a1

The compound PE-AM1-a (200 mg, 0.274 mmol, 1 eq) (commercially purchased) was dissolved in DMF/THF (4 mL/4 mL), LY-27a (197.66 mg, 0.410 mmol, 1.5 eq) was added, TsOH (70.68 mg, 0.410 mmol, 1.5 eq) was added at room temperature, and the reaction solution was stirred at 25°C for 1 hour. The LCMS analysis showed the target product. The reaction solution was purified by C18 reversed-phase column chromatography (mobile phase: water (0.25% TFA)-acetonitrile (0.25% TFA), with the elution ratio of acetonitrile increased from 0% to 70%), to afford the product LA-36OdPAB-a1 (125 mg, 39.64% yield) as a yellow solid.

LCMS (ESI): m/z, 1151.7 [M]⁺.

### Step 2: Preparation of compound LA-36OdPAB-a

K₂OsO₄.2H₂O (3.24 mg, 0.010 mmol, 0.1 eq) and NaIO₄ (446.0 mg, 2.082 mmol, 20 eq) were added to the compound LA-36OdPAB-a1 (125.0 mg, 0.108 mmol, 1.0 eq) at 0°C, a resulting mixture was stirred at 25°C for 18 hours, and the LCMS analysis showed complete consumption of the starting material. The reaction solution was purified by C18 reversed-phase column chromatography (mobile phase: water (0.25% TFA)-acetonitrile (0.25% TFA), with the elution ratio of acetonitrile increased from 0% to 70%), to afford the product LA-36OdPAB-a (32.5 mg, 26.02% yield) as a yellow solid.

LCMS (ESI): m/z, 1155.7 [M]⁺.

### Step 3: Preparation of compound LA-36OdPAB-b

Compound LA-36OdPAB-a (32.5 mg, 0.028 mmol, 1 eq) was dissolved in DMF (2.0 mL), diethylamine (21 mg, 0.281 mmol, 10 eq) was added, and the reaction solution was stirred at 25°C for 1 hour. LCMS analysis showed complete consumption of the starting material. The reaction solution was concentrated under reduced pressure to afford a crude product LA-36OdPAB-b (26.25 mg; the yield was not calculated for the crude product).

LCMS (ESI): m/z, 933.7 [M]⁺.

### Step 4: Preparation of compound LA-36OdPAB

Compound LA-36OdPAB-b (26 mg, 0.028 mmol, 1 eq) and compound LY-22CD-3-d (27 mg, 0.034 mmol, 1.2 eq) (for the synthesis of the compound, refer to patent application WO2024175069A1) were dissolved in DMF (2.0 mL). HATU (14 mg, 0.037 mmol, 1.3 eq) and DIEA (11 mg, 0.084 mmol, 3.0 eq) were added, and the reaction solution was stirred at 25°C for 1 hour. The LCMS analysis indicated complete consumption of the starting material. The reaction solution was purified by preparative high-performance liquid chromatography (model: JB-C235-04, column: Bonnasil-BS C18, 20 × 250 mm × 8 µm; mobile phase: water (0.1% TFA)-acetonitrile, with an elution ratio of acetonitrile increased from 20% to 60%), to afford product LA-36OdPAB (12.17 mg, 25.28% yield) as a white solid.

LCMS (ESI): m/z, 1712.7 [(M+H)]⁺.

¹H NMR (400 MHz, DMSO) δ 9.05 (t, *J* = 5.6 Hz, 1H), 8.87 (d, *J =* 8.6 Hz, 1H), 8.54 (t, *J* = 6.6 Hz, 1H), 8.38 (d, *J =* 8.6 Hz, 1H), 8.02 (d, *J =* 7.2 Hz, 1H), 7.89 (t, *J =* 5.6 Hz, 1H), 7.86 - 7.81 (m, 1H), 4.66 - 4.55 (m, 3H), 4.51 - 4.44 (m, 1H), 4.39 (t, *J =* 9.6 Hz, 1H), 4.27 - 4.05 (m, 7H), 3.87 - 3.73 (m, 5H), 3.65 (s, 3H), 3.63 - 3.53 (m, 11H), 3.48 - 3.46 (m, 16H), 3.39 - 3.35 (m, 4H), 3.29 - 3.24 (m, 6H), 3.20 - 3.16 (m, 2H), 2.88 - 2.81 (m, 2H), 2.79 - 2.72 (m, 1H), 2.67 - 2.59 (m, 1H), 2.46 - 2.31 (m, 7H), 2.29 - 2.07 (m, 5H), 2.03 - 1.88 (m, 6H), 1.84 - 1.41 (m, 14H), 1.33 - 1.14 (m, 10H), 0.94 (d, *J =* 6.5 Hz, 3H), 0.83 (dd, *J =* 13.7, 6.8 Hz, 6H).

### Example 68: Preparation of Compound LA-36OVCdPAB

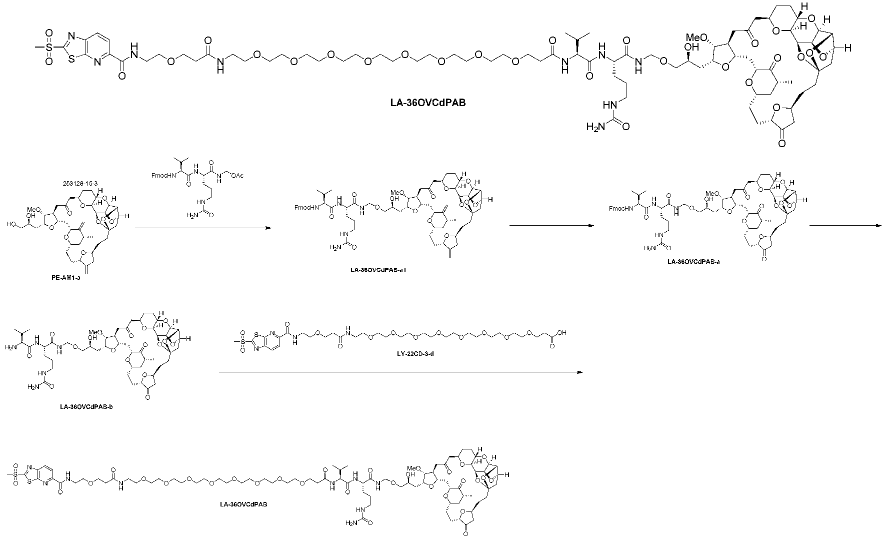

### Step 1: Preparation of compound LA-36OVCdPAB-a1

TsOH (106 mg, 0.62 mmol) was added to a solution of compound PE-AM1 (purchased from ChemExpress) (300 mg, 0.41 mmol) and (SS,8S)-1-(9H-fluoro-9-yl)-5-isopropyl-3,6,9-trioxo -8-(3-ureapropyl)-2-oxo-4,7,10-triazaundecan-11-yl acetate (350 mg, 0.62 mmol) in DMF/THF (5 mL/5mL) at 0°C. Then the mixture was stirred at 30°C for 1 hour. The reaction solution was purified by C18 reverse-phase column chromatography (mobile phase: water (0.1% TFA)-acetonitrile, with the elution ratio of acetonitrile increased from 0% to 60%), to afford LA-36OVCdPAB (350 mg, crude) as a yellow solid.

LCMS (ESI): m/z, 1238.3[M]⁺, Rt=2.62 min.

### Step 2: Preparation of compound LA-36OVCdPAB-a

Potassium osmate dihydrate (11 mg, 0.028 mmol) and sodium periodate (604 mg, 2.8 mmol) were added to a solution of compound LA-36OVCdPAB-a1 (350 mg, 0.28 mmol) in THF/H₂O (5 mL/5 mL) at 0°C. Then the mixture was stirred at 30°C for 1 hour. The reaction solution was extracted with EtOAc (50 mL × 3), the organic phases were combined, washed with brine (50 mL), and dried, and the reaction solution was filtered and evaporated to dryness to afford the crude product. The crude product was purified by C18 reversed-phase column chromatography (mobile phase: water (0.1% TFA)-acetonitrile, with the elution ratio of acetonitrile increased from 0% to 65%), to afford compound LA-36OVCdPAB (200 mg, crude product) as a yellow oil.

LCMS (ESI): m/z, 1242.2[M+1]⁺, Rt=2.304 min.

### Step 3: Preparation of compound LA-36OVCdPAB-b

Diethylamine (59 mg, 0.805 mmol) was added to the solution of compound LA-36OVCdPAB-a (100 mg, 0.08 mmol) in DMF (5 mL) at 0°C. Then the mixture was stirred at 25°C for 2 hour. The reaction solution was concentrated under vacuum to afford the compound LA-36OVCdPAB-b (50 mg, 60% yield) as a brown solid.

LCMS (ESI): m/z, 1019.7[M+1]⁺, Rt=1.08 min.

### Step 4: Preparation of compound LA-36OVCdPAB

Compound LY-22CD-3-d (for the synthesis of the compound, refer to patent application WO2024175069A1) (23.4 mg, 0.029 mmol), 2-(7-azabenzotriazole)-N,N,N',N'-tetramethyluronium hexafluorophosphate (13.4 mg, 0.035 mmol) and DIEA (12 mg, 0.088 mmol) were added to a solution of compound LA-36OVCdPAB-b (30 mg, 0.029 mmol) in N,N-dimethylformamide (5 mL) at 25°C, and the mixture was then stirred at 30°C for 1 hour. The reaction solution was concentrated under vacuum and the organic phase was separated to afford a crude product. The crude product was purified by preparative high-performance liquid chromatography (preparative chromatograph manufacturer: Orienda, model: BRIX-2860, Phenomenex Luna C18 (250×50 mm×10 µm) was used as a chromatographic column, mobile phase: water (0.225% CF₃COOH)-acetonitrile, with an elution ratio of acetonitrile increased from 12% to 42%) and the eluent was lyophilized to afford product LA-36OVCdPAB (4.0 mg, 7.6% yield) as a white solid.

LCMS (ESI): m/z, 899.5 [M/2+H]⁺, Rt=1.15min.

### Example 69: Preparation of Compound LA-36VCMC

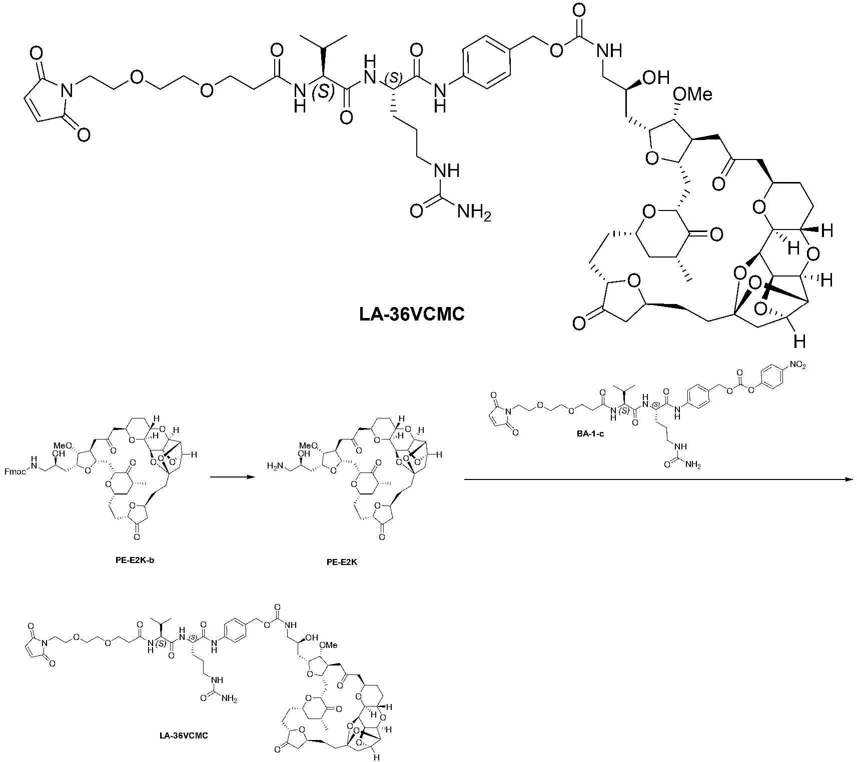

### Step 1: Preparation of compound PE-E2K

Diethylamine (12 mg, 0.160 mmol, 10 eq) was added to the solution of compound PE-E2K-b (for synthesis of the compound, refer to Preparation Example of PE-E2K) (15.0 mg, 0.016 mmol, 1 eq) in DMF (1 mL) at room temperature, the reaction solution was stirred at 25°C for 1 hour, and the LCMS analysis indicated complete consumption of the starting material. The reaction solution was concentrated under reduced pressure, to afford a crude product PE-E2K as a yellow solid, which was directly used for the next step without purification.

LCMS (ESI): m/z, 734.3 [(M+H)]⁺.

### Step 2: Preparation of compound LA-36VCMC

DIEA (4 mg, 0.032 mmol, 2.0 eq), BA-1-c (for synthesis of the compound, refer to the Example of BA-1) (15.05 mg, 0.019 mmol, 1.2 eq) and HOBt (2.59 mg, 0.019 mmol, 1.2 eq) were added to the solution of the foregoing crude product PE-E2K (0.016 mmol, 1 eq) in DMF (1 mL) at 0°C, the reaction solution was stirred at 25°C for 1 hour, and LCMS analysis indicated complete consumption of the starting material. The reaction solution was purified by preparative high-performance liquid chromatography (model: JB-C235-04, column: Bonnasil-BS C18, 20 × 250 mm × 8 µm; mobile phase: water (0.1% TFA)-acetonitrile, with an elution ratio of acetonitrile increased from 20% to 60%), to afford product LA-36VCMC (5.29 mg, 24.49% yield) as a white solid.

LCMS (ESI): m/z, 1378.8 [(M+H)]⁺.

¹H NMR (400 MHz, DMSO-*d₆*) δ 9.97 (s, 1H), 8.10 (d, *J* = 7.4 Hz, 1H), 7.85 (d, *J* = 8.8 Hz, 1H), 7.58 (d, *J* = 8.8 Hz, 2H), 7.27 (d, *J* = 8.8 Hz, 2H), 7.12 - 7.04 (m, 1H), 7.02 (s, 2H), 5.96 (t, *J =* 5.5 Hz, 1H), 5.40 (s, 1H), 4.98 - 4.89 (m, 2H), 4.64 (t, *J =* 4.4 Hz, 1H), 4.58 - 4.55 (m, 1H), 4.42 - 4.34 (m, 2H), 4.22 (dd, *J* = 8.4, 6.8 Hz, 1H), 4.17 - 4.08 (m, 4H), 3.88 - 3.73 (m, 4H), 3.58 - 3.41 (m, 11H), 2.98 - 2.65 (m, 12H), 2.35 - 1.91 (m, 15H), 1.75 - 1.15 (m, 21H), 0.94 (d, *J* = 6.4 Hz, 3H), 0.84 (dd, *J* = 13.6, 6.8 Hz, 6H).

### Example 70: Preparation of Compound LA-36VCdPAB

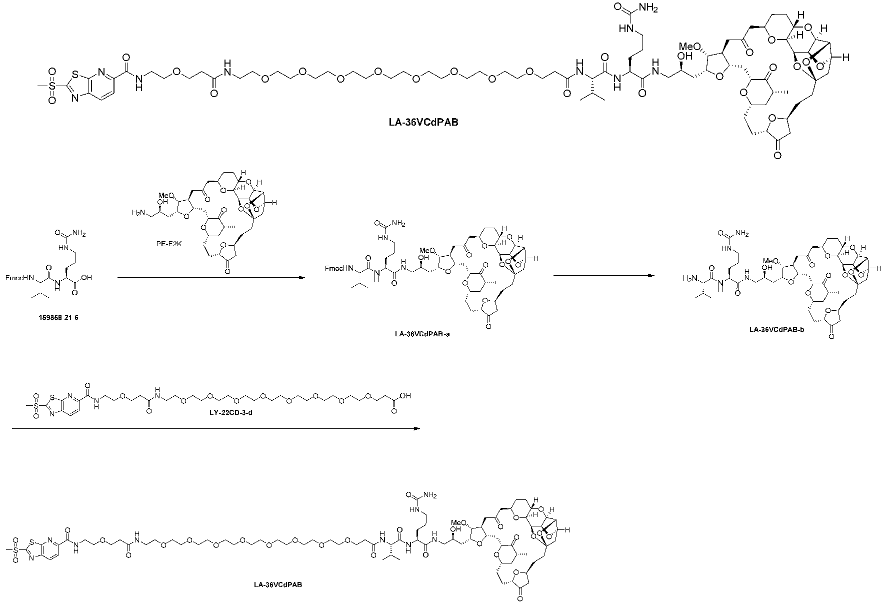

### Step 1: Preparation of compound LA-36VCdPAB-a

DIEA (40 mg, 0.307 mmol, 3 eq), a compound (CAS: 159858-21-6) (60.90 mg, 0.123 mmol, 1.2 eq) and HATU (46.63 mg, 0.123 mmol, 1.3 eq) were added to a solution of the compound PE-E2K (for synthesis of the compound, refer to Preparation Example of PE-E2K) (75 mg, 0.102 mmol, 1 eq) in DMF (3 mL) at 0°C. The mixture was then stirred at 25°C for 2 hours. LCMS analysis showed that the reaction ended. The reactant was purified by silica gel column chromatography (C18, H₂O (0.25% TFA): ACN = 34%: 64%), to afford LA-36VCdPAB-a (60 mg, 48.42% yield) as a white solid.

LCMS (ESI): m/z, 1212.8[M]⁺, Rt=1.508 min.

### Step 2: Preparation of compound LA-36VCdPAB-b

Diethylamine (5 mg, 0.070 mmol, 5 eq) was added to the solution of compound LA-36VCdPAB-a (17 mg, 0.014 mmol, 1 eq) in DMF (1 mL), the reaction solution was stirred at 25°C for 1 hour, and LCMS analysis indicated complete consumption of the starting material. The reaction solution was concentrated under low temperature in vacuo to afford crude LA-36VCdPAB-b (13.88 mg, crude) as a pale yellow oil.

LCMS (ESI): m/z, 989.7[M-1]⁺, Rt=1.151min.

### Step 3: Preparation of compound LA-36VCdPAB

DIEA (0.006 mL, 0.039 mmol, 3 eq), LY-22CD-3-d (for synthesis of the compound, refer to patent application WO2024175069A1) (11.51 mg, 0.014 mmol, 1.1 eq) and HATU (6.43 mg, 0.017 mmol, 1.3 eq) were added to the solution of the compound LA-36VCdPAB-b (13 mg, 0.013 mmol, 1 eq) in DMF (1.5 mL) at 0°C. The mixture was then stirred at 25°C for 1 hour. LCMS analysis showed that the reaction ended.

The reaction solution was purified by high-performance liquid chromatography (Xtimate C18, 21.2 × 250 mm × 5 µm, H₂O (0.1% FA): ACN=78%:22% to 48%:52%), to afford LA-36VCdPAB (1.3 mg, 5.60% yield) as a white solid.

LCMS (ESI): m/z, 1768.1 [M-1]⁺, Rt=1.252min.

¹H NMR (400 MHz, DMSO-*d₆*) δ 9.06 (t, *J =* 5.6 Hz, 1H), 8.87 (d, *J =* 8.6 Hz, 1H), 8.38 (d, *J =* 8.6 Hz, 1H), 7.88 (dd, *J =* 18.0, 8.4 Hz, 2H), 7.75 (s, 1H), 7.20 (s, 1H), 6.65 (s, 1H), 5.89 (s, 1H), 5.32 (t, *J =* 4.7 Hz, 2H), 4.65 (t, *J =* 4.4 Hz, 1H), 4.56 (s, 1H), 4.39 (s, 1H), 4.27 - 4.09 (m, 5H), 3.90 - 3.72 (m, 4H), 3.66 - 3.62 (m, 4H), 3.62 - 3.57 (m, 2H), 3.55 (dd, *J =* 9.6, 4.0 Hz, 3H), 3.49 (d, *J =* 6.2 Hz, 28H), 3.24 (t, *J =* 3.4 Hz, 5H), 3.18 (dd, *J =* 11.6, 6.0 Hz, 3H), 3.04 (s, 2H), 2.92 (s, 2H), 2.88 - 2.81 (m, 2H), 2.79 - 2.72 (m, 1H), 2.65 - 2.59 (m, 1H), 2.46 - 2.35 (m, 3H), 2.25 (d, *J* = 17.6 Hz, 2H), 2.19 - 2.04 (m, 3H), 1.96 (ddd, *J* = 18.2, 13.8, 5.9 Hz, 7H), 1.77 (dd, *J* = 21.8, 11.1 Hz, 2H), 1.71 - 1.54 (m, 7H), 1.49 - 1.42 (m, 3H), 1.23 (s, 12H), 0.94 (d, *J* = 6.4 Hz, 2H), 0.83 (dt, *J =* 8.8, 6.8 Hz, 7H).

### Example 71: Preparation of Compound LA-36VCP

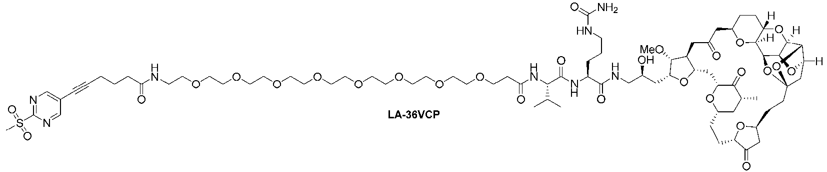

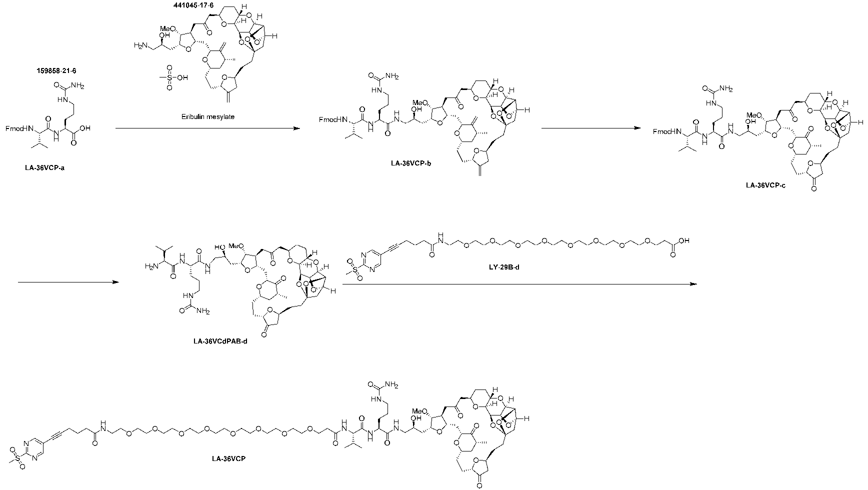

### Step 1: Preparation of compound LA-36VCP-b

2,4,6-collidine (220 mg, 1.816 mmol), eribulin mesylate (500 mg, 0.605 mmol) and HATU (299 mg, 0.787 mmol) were added to a solution of compound LA-36VCP-a (361 mg, 0.726 mmol) in DMF (20 mL) at room temperature. Then the mixture was stirred at 25°C for 1 hour. LCMS analysis showed that the reaction ended. The reactant was purified by silica gel column chromatography (C18, H₂O (0.025% TFA): ACN = 37%: 63%), to afford compound LA-36VCP-b (660 mg, 90% yield) as a white solid. LCMS (ESI): m/z, 1208.1[m]⁺, Rt=1.566min.

### Step 2: Preparation of compound LA-36VCP-c

Potassium osmate (8 mg, 0.023 mmol) and NaIO₄ (177 mg, 0.827 mmol) were added to a solution of compound LA-36VCP-b (50 mg, 0.041 mmol) in THF (8 mL) and H₂O (2 mL) at 0°C. The mixture was stirred at 25°C for 2 hours. Potassium osmate (8.29 mg, 0.023 mmol) and NaIO₄ (177 mg, 0.827 mmol) were then added to the mixture at 0°C. Then the mixture was stirred at 25°C for 18 hour. LCMS analysis showed that the reaction ended. The reaction solution was diluted with water (20 mL), and extracted with dichloromethane (50 mL × 2). The organic phase was concentrated in vacuo to afford the crude product. The crude product was purified by silica gel column chromatography (C18, H₂O (0.1% TFA): ACN = 52%: 48%), to afford compound LA-36VCP-c (40 mg, 80% yield) as a white solid. LCMS (ESI): m/z, 1212.9[m-1]⁺, Rt=1.404min.

### Step 3: Preparation of compound LA-36VCP-d

Diethylamine (24 mg, 0.330 mmol) was added to the solution of compound LA-36VCP-c (50 mg, 0.033 mmol) in DMF (2 mL) at 15°C. The mixture was then stirred at 15°C for 30 minutes. LCMS analysis showed that the reaction ended. The reaction solution was concentrated under vacuum, to afford compound LA-36VCdPAB-d (32 mg, crude product) as a brown oil.

LCMS (ESI): m/z, 989.9[M-1]⁺, Rt=1.057min.

### Step 4: Preparation of compound LA-36VCP

N,N-diisopropylethylamine (11 mg, 0.082 mmol), compound LY-29B-d (for the synthesis of the compound, refer to patent application WO2024175069A1) (25 mg, 0.036 mmol) and HATU (16 mg, 0.043 mmol) were added to a solution of compound LA-36VCdPAB-d (41 mg, 0.033 mmol) in DMF at 15°C. The mixture was then stirred at 15°C for 30 minutes. LCMS analysis showed that the reaction ended. The reaction solution was purified by JB-C235-04 (Prime C18, 30 × 150 mm, 5µm, H₂O (0.1% TFA): ACN=70%:30% to 56%:44%), to afford LA-36VCP (12.83 mg, 0.008 mmol, 23.37%) as a white solid. LCMS (ESI): m/z, 833.0[m/2+1]⁺, Rt=1.21min.

¹H NMR (400 MHz, DMSO-*d₆*) δ 9.12 (d, *J* = 3.0 Hz, 2H), 7.97 - 7.84 (m, 3H), 7.75 (t, *J* = 5.3 Hz, 1H), 5.93 (s, 1H), 4.65 (t, *J =* 4.2 Hz, 1H), 4.57 (s, 1H), 4.40 (s, 1H), 4.29 - 4.08 (m, 8H), 3.80 (ddd, *J =* 19.8, 18.0, 7.0 Hz, 9H), 3.59 (dd, *J =* 11.2, 6.2 Hz, 7H), 3.41 (d, *J =* 6.1 Hz, 10H), 3.28 - 3.18 (m, 8H), 3.06 (d, *J =* 4.9 Hz, 3H), 2.94 (s, 2H), 2.86 (dd, *J =* 17.9, 7.6 Hz, 3H), 2.75 (dt, *J* = 23.7, 8.6 Hz, 2H), 2.64 (dd, *J =* 18.1, 11.0 Hz, 2H), 2.56 (t, *J =* 7.0 Hz, 3H), 2.48 - 2.44 (m, 1H), 2.43 - 2.31 (m, 4H), 2.26 (dd, *J =* 15.4, 8.0 Hz, 5H), 2.20 - 1.90 (m, 9H), 1.81 (dt, *J =* 20.8, 10.3 Hz, 5H), 1.75 - 1.12 (m, 20H), 1.09 - 0.92 (m, 4H), 0.89 - 0.76 (m, 7H).

### Example 72: Preparation of Compound LA-37A

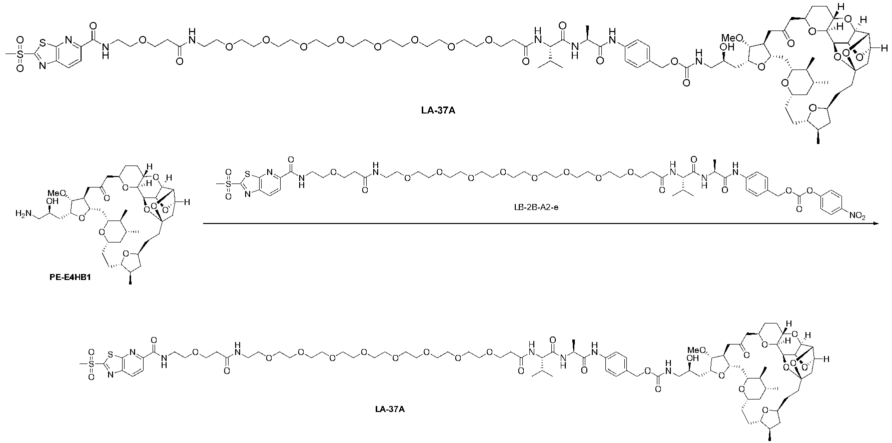

DIPEA (12.69 mg, 97.24 µmol, 3 eq) and HOBt (4.47 mg, 32.41 µmol, 1 eq) were added to a solution of compounds PE-E4HB1 (23.79 mg, 32.41 µmol, 1 eq) and LB-2B-A2-e (32.74 mg, 25.93 µmol, 0.8 eq; for the synthesis of the compound, refer to Example 50 or the Example of LA-36) in DMF (2 mL) at 0°C. The reaction solution was stirred at room temperature for 2 hours. LCMS analysis showed that the reaction ended. The reaction solution was evaporated to dryness under reduced pressure. The mixture was purified by preparative high-performance liquid chromatography (preparative chromatograph manufacturer: Orienda, model: BRIX-2860, column: Welch Xtimate C18 150 × 30 mm × 10 µm, mobile phase: water (0.225% FA)-acetonitrile, with an elution ratio of acetonitrile increased from 40% to 70%), to afford product LA-37A (3.50 mg, 5.89% yield) as a white solid. LCMS (ESI): m/z, 1834.0[M+H]⁺.

### Example 73: Preparation of Compound LA-37B

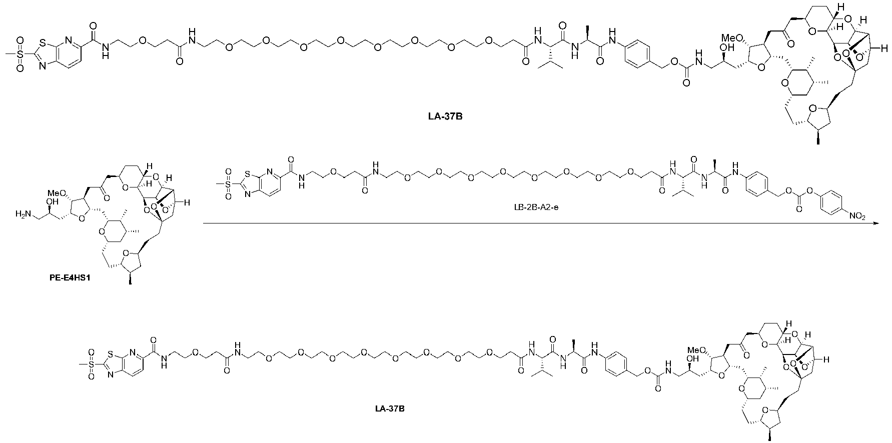

DIPEA (9.83 mg, 75.29 µmol, 3 eq) and HOBt (3.46 mg, 25.10 µmol, 1 eq) were added to a solution of compounds PE-E4HS1 (18.42 mg, 25.10 µmol, 1 eq) and LB-2B-A2-e (25.35 mg, 20.08 µmol, 0.8 eq; for the synthesis of the compound, refer to Example 50 or the Example of LA-36) in DMF (2 mL) at 0°C. The reaction solution was stirred at room temperature for 2 hours. LCMS analysis showed that the reaction ended. The reaction solution was evaporated to dryness under reduced pressure. The mixture was purified by preparative high-performance liquid chromatography (preparative chromatograph manufacturer: Orienda, model: BRIX-2860, column: Welch Xtimate C18 150 × 30 mm × 10 µm, mobile phase: water (0.225% FA)-acetonitrile, with an elution ratio of acetonitrile increased from 40% to 70%), to afford product LA-37B (1.80 mg, 3.91% yield) as a white solid. LCMS (ESI): m/z, 1872.8.0[M+MeCN]⁺.

### Example 74: Preparation of Compound LA-38

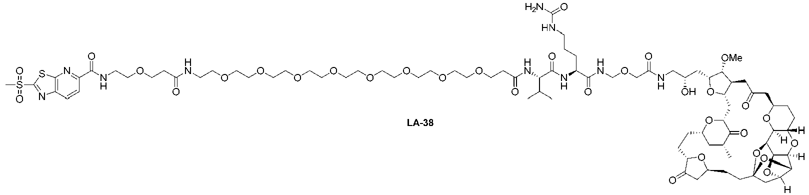

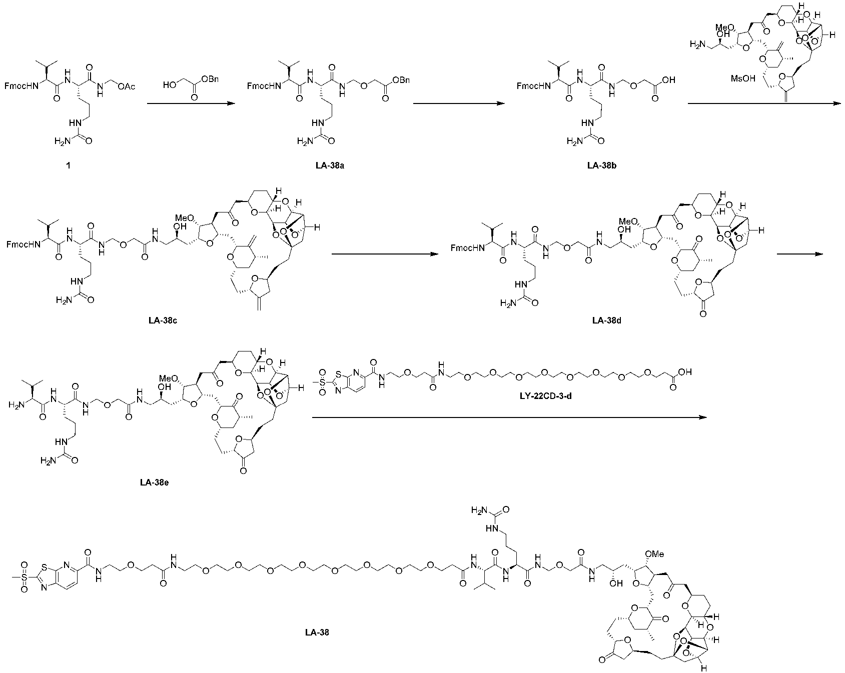

### Step 1: Preparation of compound LA-38a

P-toluenesulfonic acid (91 mg, 0.52 mmol) and benzyl glycolate (88 mg, 0.52 mmol) were added to a solution of compound 1 (Fmoc-Vc-OAc) (200 mg, 0.35 mmol) in tetrahydrofuran/N,N-dimethylformamide (5 mL) at 0°C. The mixture was stirred at 30°C for 4 hours. The reaction solution was purified by reverse-phase column chromatography (40g, ACN: H₂O = 10:90 to 80:20) to afford the crude product of compound LA-38a as a colorless oil (60 mg, 25.7% yield).

LCMS (ESI): m/z 696.2[M+Na]⁺, Rt=1.25min.

### Step 2: Preparation of compound LA-38b

Palladium on carbon (10%, 10 mg) was added to a solution of compound LA-38a (40 mg, 0.065 mmol) in methanol (5 mL) at 25°C. The mixture was then stirred at 30°C for 16 hours. The reaction solution was concentrated to afford the crude product of LA-38b as a white solid (40 mg, 76.5% yield).

LCMS (ESI): m/z, 606.4 [M+H]⁺, Rt=1.05 min.

### Step 3: Preparation of compound LA-38c

2-(7-azabenzotriazol)-N,N,N',N'-tetramethyluronium hexafluorophosphate (40 mg, 0.10 mmol), eribulin mesylate (56 mg, 0.07 mmol), and N,N-diisopropylethylamine (26 mg, 0.21 mmol) were added to a solution of compound LA-38b (40 mg, 0.07 mmol) in N,N-dimethylformamide (5 mL) at 25°C. The mixture was then stirred at 30°C for 1 hour. The reaction solution was purified by reverse-phase column chromatography (40g, ACN:H₂O = 10:90 to 80:20) to afford the product LA-38c (45 mg, 50.5% yield) as a white solid.

LCMS (ESI): m/z, 648.2 [M+H]⁺, Rt=1.18min.

### Step 4: Preparation of compound LA-38d

Sodium periodate (82 mg, 0.38 mmol) and potassium osmate dihydrate (2 mg, 0.004 mmol) were added to a solution of compound LA-38c (40 mg, 0.04 mmol) in tetrahydrofuran/water (5 mL) at 25°C. The mixture was then stirred at 30°C for 16 hours. The reaction solution was purified by reverse-phase column chromatography (40g, ACN:H₂O = 10:90 to 80:20) to afford the product LA-38c (20 mg, 40% yield) as a white solid.

LCMS (ESI): m/z, 1299.6 [M+H]⁺, Rt=1.61min.

### Step 5: Preparation of compound LA-38e

Diethylamine (11 mg, 0.15 mmol) was added to a solution of compound LA-38d (20 mg, 0.015 mmol) in N,N-dimethylformamide (5 mL) at 25°C. The mixture was then stirred at 30°C for 1 hour. The reaction solution was concentrated to afford the crude product of LA-38e (15 mg, 90.5% yield) as a solid.

LCMS (ESI): m/z, 1077.4 [M+H]⁺, Rt=1.52min.

### Step 6: Preparation of compound LA-38

2-(7-azabenzotriazol)-N,N,N',N'-tetramethyluronium hexafluorophosphate (8 mg, 0.021 mmol), LY-22CD-3-d (12 mg, 0.014 mmol), and N,N-diisopropylethylamine (6 mg, 0.042 mmol) were added to a solution of compound LA-38e (15 mg, 0.014 mmol) in N,N-dimethylformamide (5 mL) at 25°C. The mixture was then stirred at 30°C for 1 hour. The crude product was subjected to preparative high-performance liquid chromatography (preparative chromatograph manufacturer: Orienda, model: BRIX-2860, Phenomenex Luna C18 (250×50 mm×10 µm) was used as a chromatographic column, mobile phase: water (0.225% HCOOH)-acetonitrile, with an elution ratio of acetonitrile increased from 12% to 42%) and the eluent was lyophilized to afford product LA-38 (6.5 mg, 25% yield) as a white solid.

LCMS (ESI): m/z, 928.6 [M/2+H]⁺, Rt=0.98min.

### Preparation Examples of ADCs:

### Preparation Example 1 of ADCs: Synthesis of T-LA25AA (Procedure 1A, DAR 8)

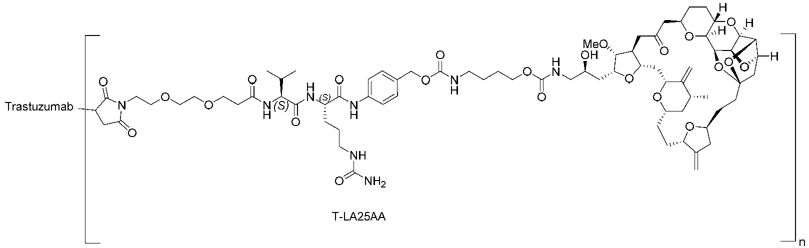

An aqueous solution of tris(2-carboxyethyl)phosphine (TCEP) (40 mM, 23.3 µL, 931 nmol) was added to the solution of trastuzumab (Herceptin, Henlius, 10.0 mg/mL, 2 mL, 133 nmol) in the aqueous solution of PBS buffer (0.04M aqueous solution of PBS buffer, pH = 7.4) at 37°C. The mixture was allowed to react in a water bath at 37°C for 2 hours, and then the reaction was quenched. The reaction solution was cooled to 25°C in the water bath.

The compound LA-25AA (1.98 mg, 1330 nmol) was dissolved in 83.3 µL of DMSO, a resulting solution was added to the above reaction solution, the reaction solution was allowed to react in the water bath at 25°C for 30 minutes, and then 1% (mass ratio) N-ethylmaleimide (NEM) was added to quench the reaction. The reaction solution was purified by dialysis via a dialysis card (Slide-A-Lyzer^{™} dialysis kit, 10K MWCO) to afford a PBS buffer (5.198 mg/mL, 0.28 mL) of T-LA25AA, and the PBS buffer was stored at 4°C. For long-term storage, the PBS buffer was kept at -80°C.

The mean drug load n calculated by RP was equal to 7.61.

### Preparation Example 2 of ADCs: Synthesis of T-LA27C (Procedure 1B, DAR 8)

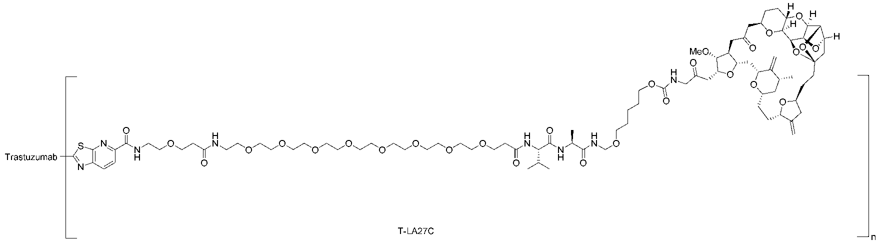

A prepared aqueous solution of tris(2-carboxyethyl)phosphine (TCEP) (40 mM, 23.3 µL, 931 nmol) was added to the solution of antibody trastuzumab (Herceptin, Henlius, 10.0 mg/mL, 2 mL, 133 nmol) in the aqueous solution of PBS buffer (0.04M aqueous solution of PBS buffer, pH = 7.4) at 37°C. The mixture was allowed to react in a water bath at 37°C for 2 hours, and then the reaction was quenched. The reaction solution was cooled to 25°C in the water bath.

The compound LA-27C (3.66 mg, 1995 nmol) was dissolved in 125 µL of DMSO, a resulting solution was added to the above reaction solution, the reaction solution was allowed to react in the water bath at 25°C for 30 minutes, and then 1% (molar ratio) N-ethylmaleimide (NEM) was added to quench the reaction. The reaction solution was purified by dialysis via a dialysis card (Slide-A-Lyzer^{™} dialysis kit, 10K MWCO) to afford a PBS buffer (5.198 mg/mL, 0.28 mL) of T-LA27C, and after aliquoting, the PBS buffer was stored at 4°C. For long-term storage, the PBS buffer was kept at -80°C.

The mean drug load n calculated via RP was equal to 7.14.

### Preparation Example 3 of ADC: Preparation of T-LA-3 (Procedure 2, Site-specific, DAR4)

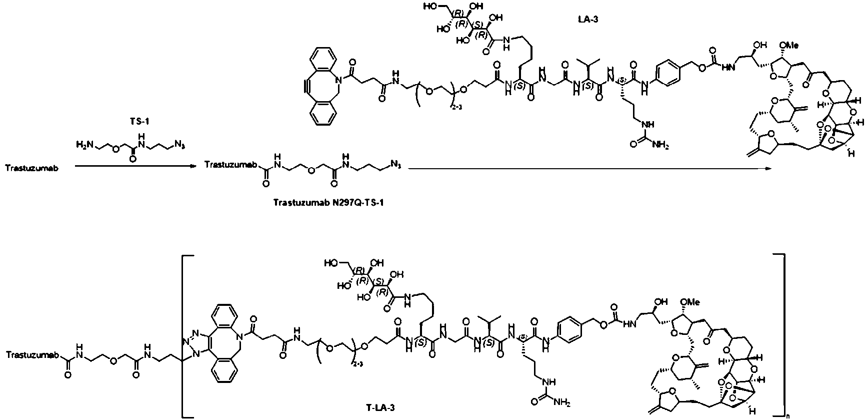

Azide-modified trastuzumab mutant (Trastuzumab N297Q-TS-1) (1.0 mg/mL, 1 mL, 6.9 µM) was prepared according to the method described in the literature *(*Angew. Chem. Int. Ed. 2010, 49, 9995-9997).

Compound LA-3 was dissolved in DMSO (2 mM), a resulting solution was added to the solution of trastuzumab mutant antibody in the aqueous solution of PBS (69 µM), and the reaction solution was allowed to react in the water bath at 25°C for 6 hours. The reaction solution was purified by dialysis via a dialysis kit (brand: Thermo Fisher; model: Slide-A-Lyzer^{™}) to afford PBS buffer of T-LA-3, and the PBS buffer was stored at 4°C. For long-term storage, the PBS buffer was kept at -80°C.

The mean drug load n calculated via RP was equal to 4.0.

| Procedure 2 | Structure | DAR |
|---|---|---|
| T-LA-3 | 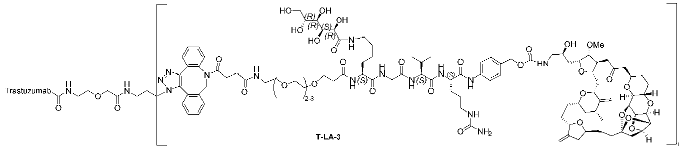 | 4 |
| T-LA-25B | 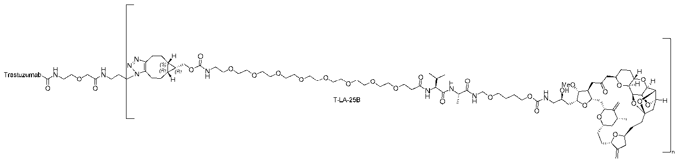 | 3.78 |
| T-LA-27CB CN | 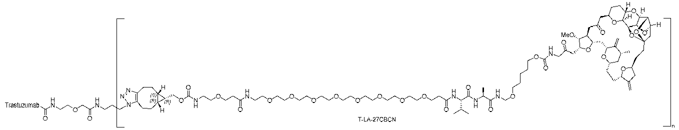 | 3.82 |
| T-LY-22CD3 | 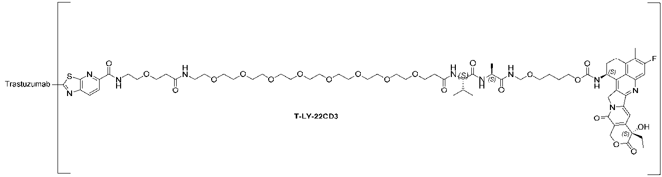 | 3.92 |
| T-LA-36VC dPAB | 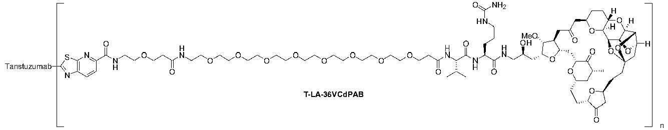 | 3.79 |
| T-LA-36O2 VC | 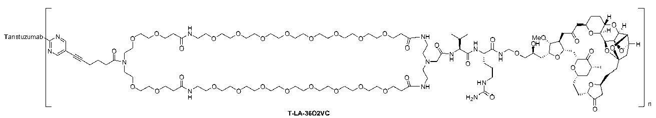 | 3.85 |

| Procedure 1A | Structure | DAR |
|---|---|---|
| T-BA-1 | 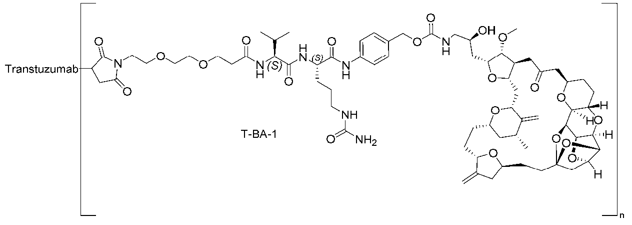 | 7.6 |
| T-BA-2 | 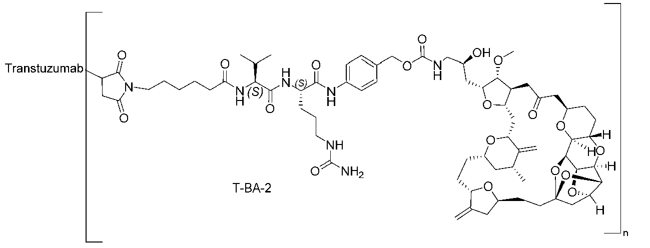 | 3.89 |
| T-BA-3 | 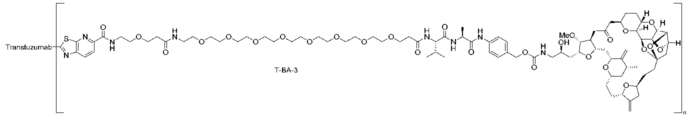 | 7.79 |
| T-BA-4 | 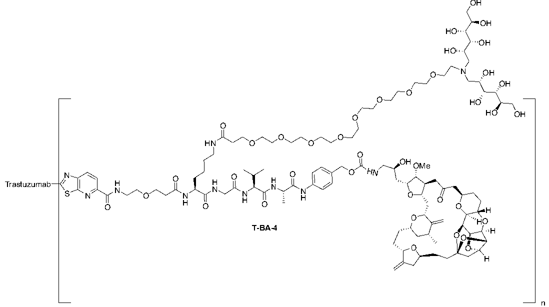 | 7.85 |
| T-BA-5 | 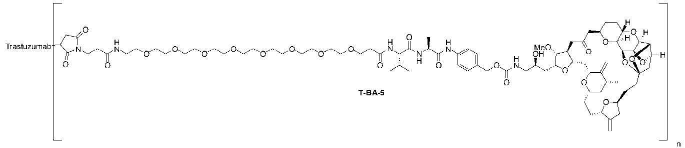 | 7.84 |
| T-BA-6 | 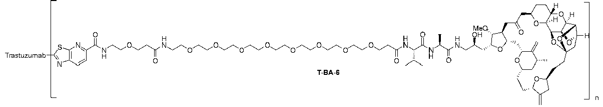 | 7.92 |
| T-LA-1 | 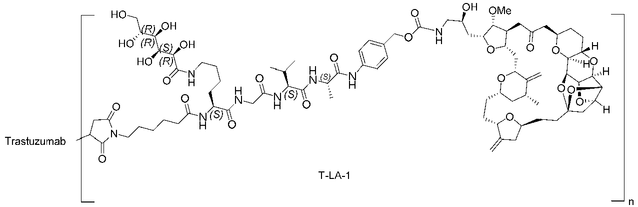 | 8 |
| T-LA-2 | 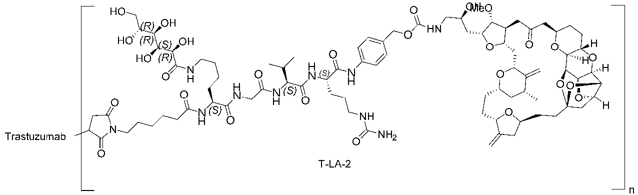 | 8 |
| T-LA-4 | 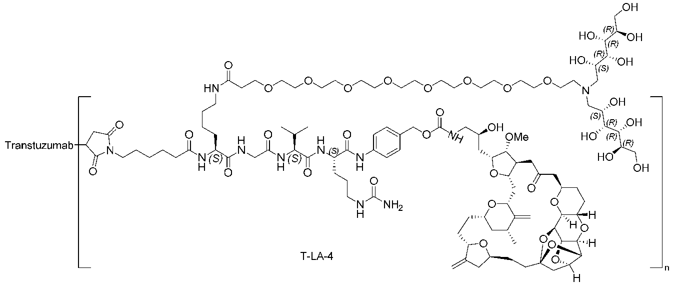 | 8 |
| T-LA-6 | 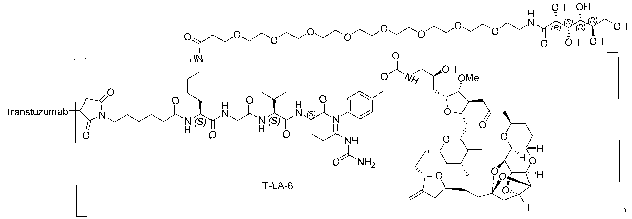 | 7.89 |
| T-LA-9 | 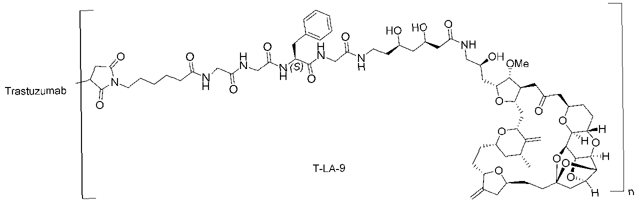 | 7.23 |
| T-LA-10 | 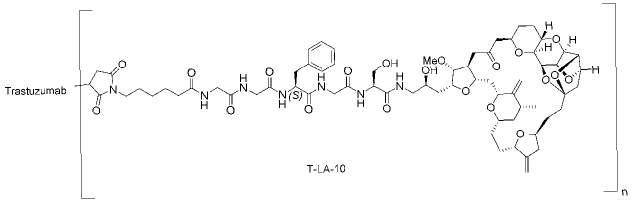 | 7.35 |
| T-LA-11 | 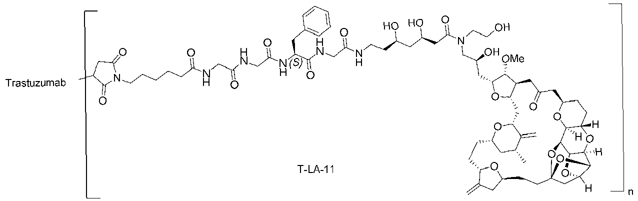 | 7.95 |
| T-LA-14 | 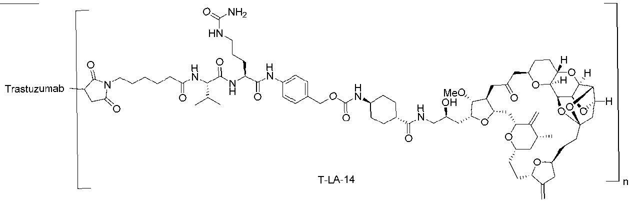 | 3.97 |
| T-LA-21 | 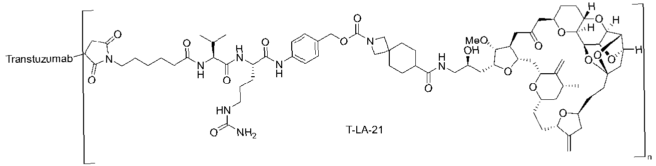 | 4.02 |
| T-LA-24 | 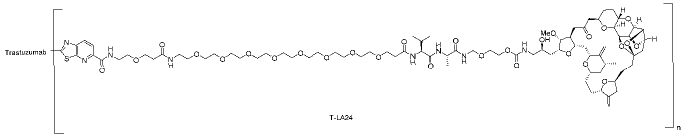 | 4.6 |
| T-LA-25A | 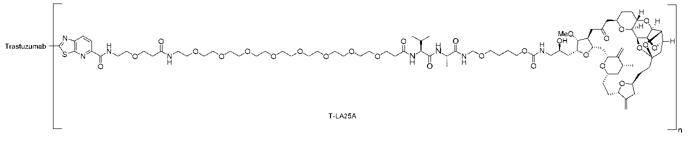 | 4.49 |
| T-LA-25A A | 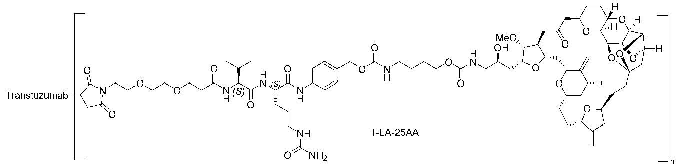 | 7.600 |
| T-LA-26 | 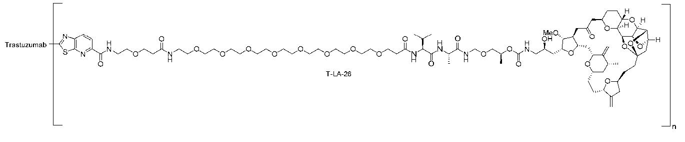 | 6.08 |
| T-LA26B | 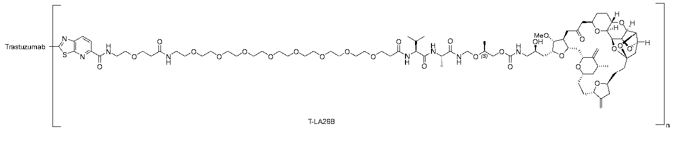 | 4.25 |
| T-LA-27A 0 | 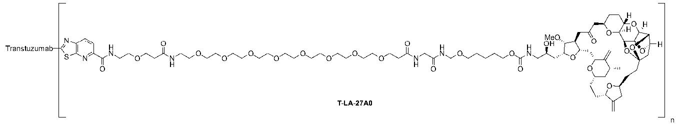 | 7.65 |
| T-LA-27A | 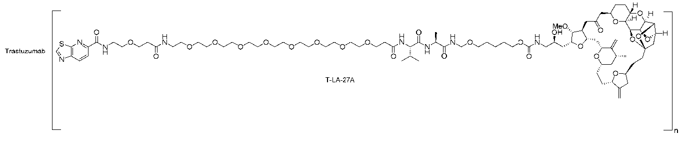 | 7.44 |
| T-LA-27A A | 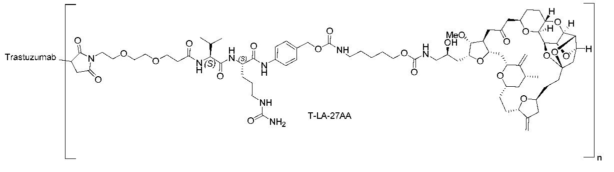 | 7.60 |
| T-LA-27A A-20 | 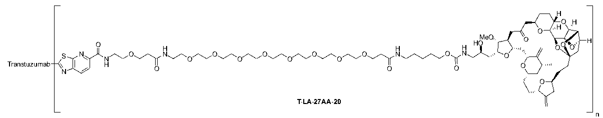 | 7.72 |
| T-LA-27A A-2 | 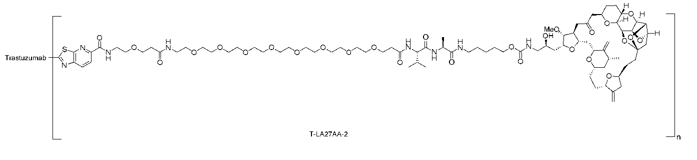 | 2.85 |
| T-LA-27A A-3 | 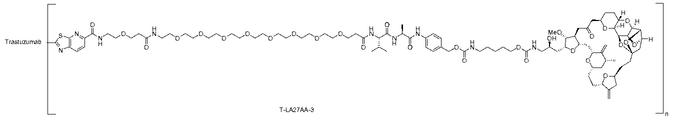 | 0.6 |
| T-LA-27A AVC | 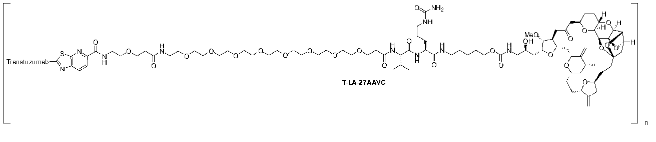 | 7.74 |
| T-LA-27A AVCP | 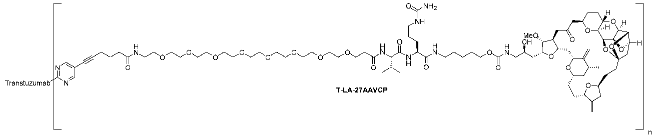 | 7.28 |
| T-LA28 | 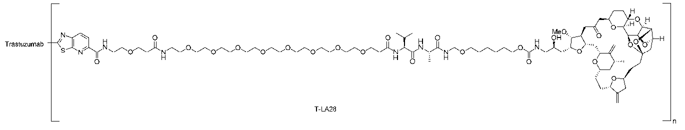 | 5.78 |
| T-LA28A A-2 | 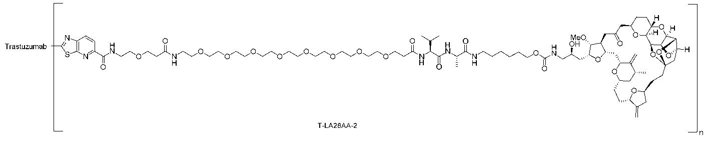 | 5.49 |
| T-LA29 | 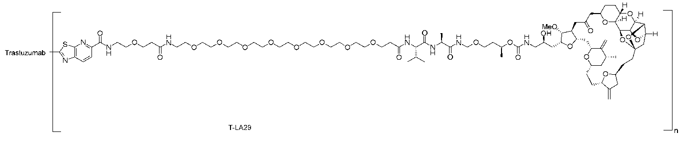 | 6.28 |
| T-LA-29B | 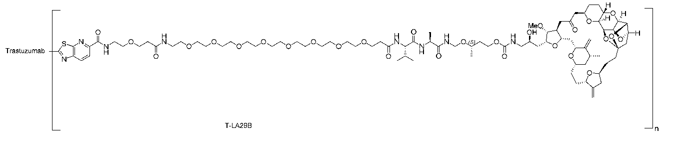 | 6.45 |
| T-LA-29A R | 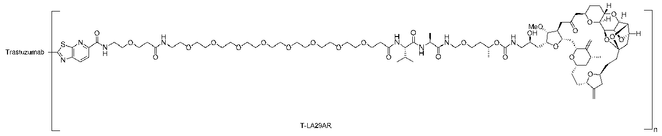 | 7.35 |
| T-LA-30B -P1 | 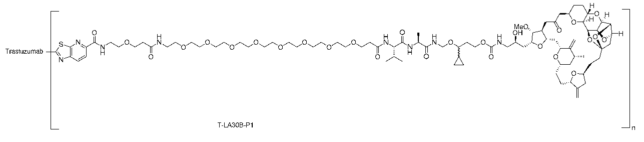 | 4.75 |
| T-L30B-P 2 | 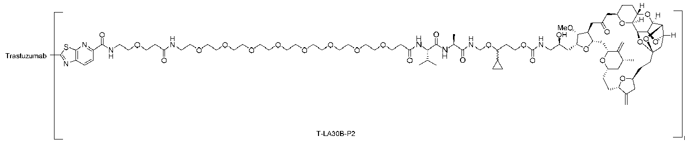 | 6.55 |
| T-L31B | 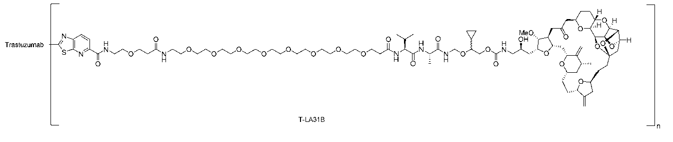 | 5.02 |
| T-L32AR | 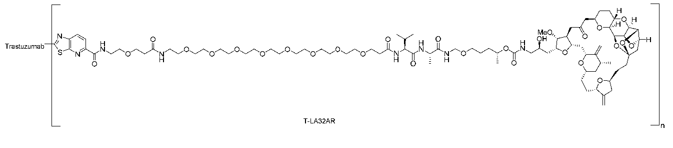 | 6.4 |

| Procedure 1B | Structure | DAR |
|---|---|---|
| T-LA-11B | 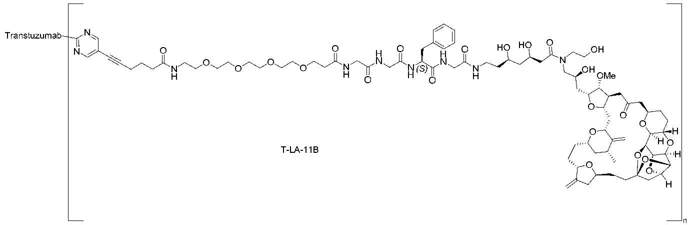 | 7.82 |
| T-LA-15 | 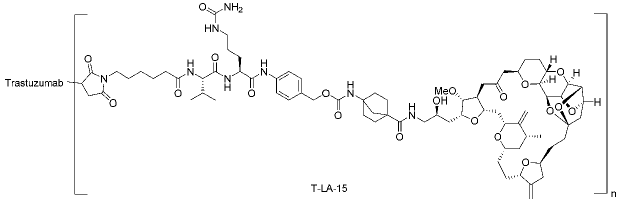 | 7.65 |
| T-LA-22 | 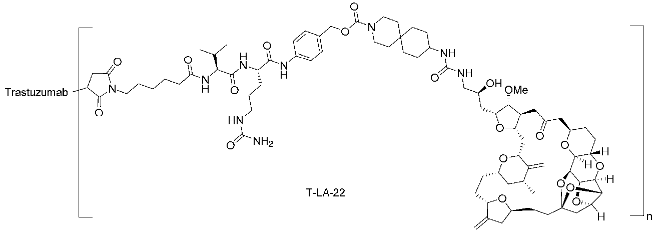 | 3.85 |
| T-LA-23 | 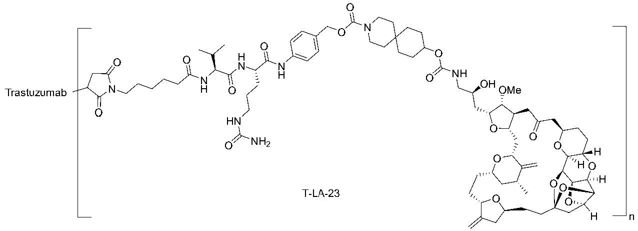 | 0.6 |
| T-LA-27A | 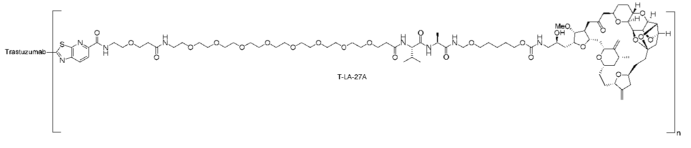 | 7.48 |
| T-LA-27C | 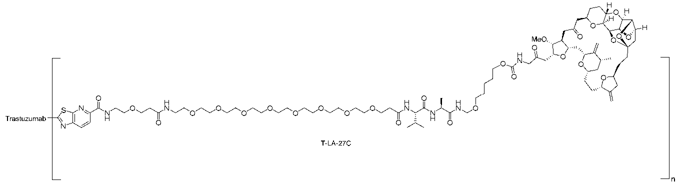 | 7.51 |
| T-LA-28A A | 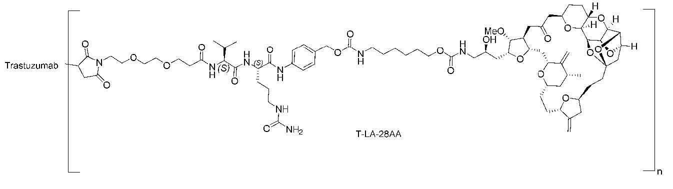 | 7.77 |
| T-LA-30A 1 | 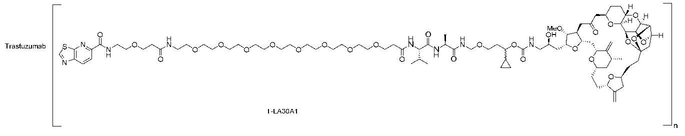 | 7.44 |
| T-LA-30A 2 | 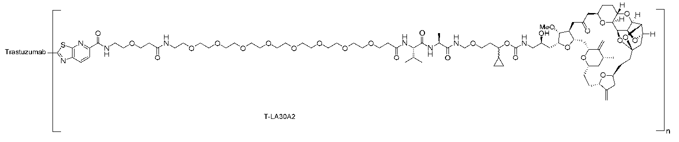 | 7.31 |
| T-LA-31A | 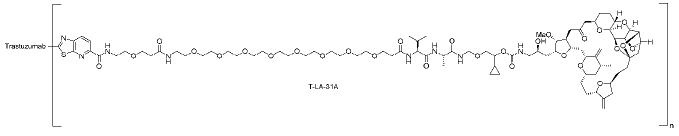 | 7.4 |
| T-LA-32A S | 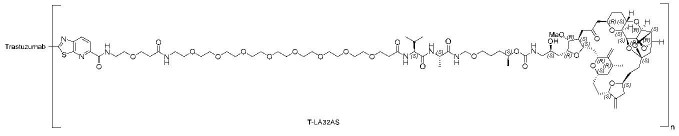 | 7.31 |
| T-LA-33A | 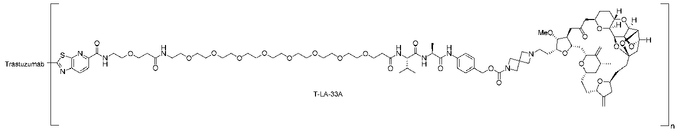 | 7.38 |
| T-LA-33B | 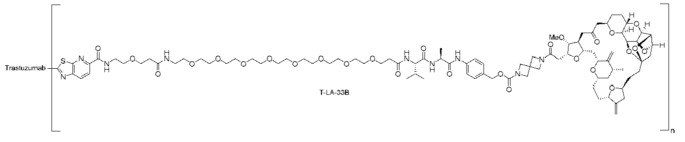 | 7.24 |
| T-LA-34A | 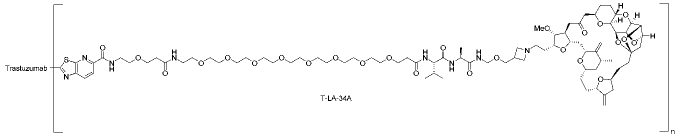 | 7.28 |
| T-LA-34B | 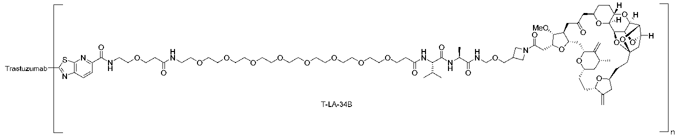 | 6.61 |
| T-LA-34B -2C | 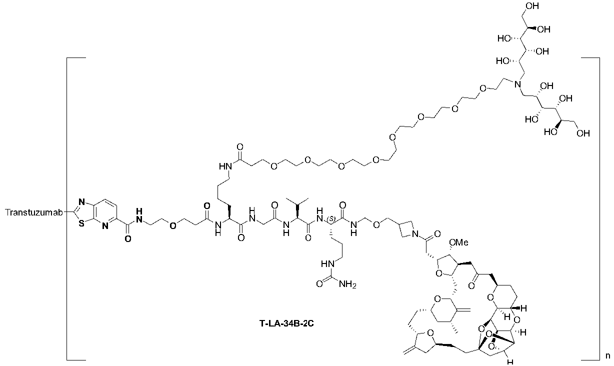 | 7.45 |
| T-LA-34B -2C-B | 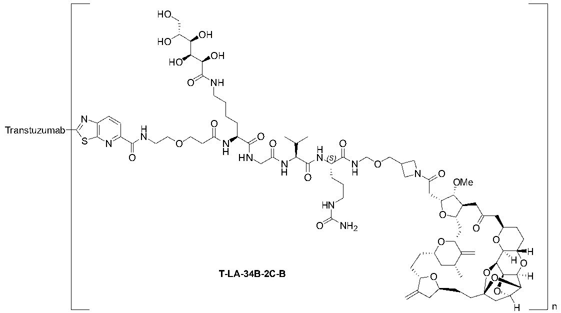 | 7.37 |
| LA-34BV C | 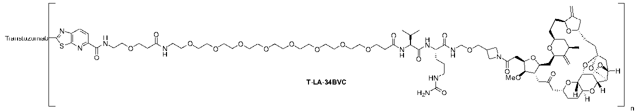 | 7.56 |
| T-LA-35A | 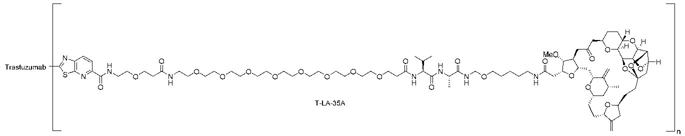 | 7.26 |
| T-LA-35B | 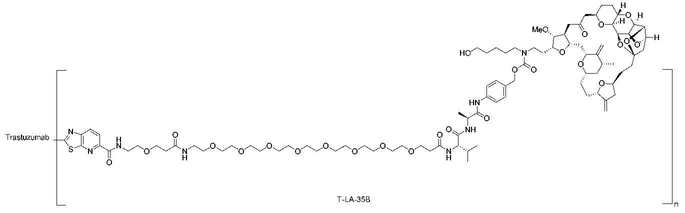 | 7.92 |
| T-LA-35C | 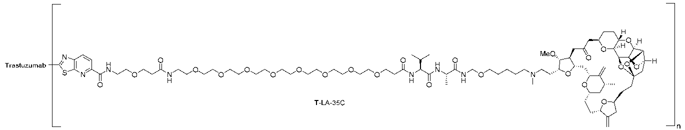 | 6.65 |
| T-LA-36 | 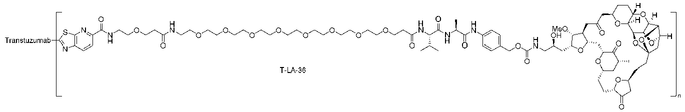 | 7.65 |
| T-LA-36d PAB | 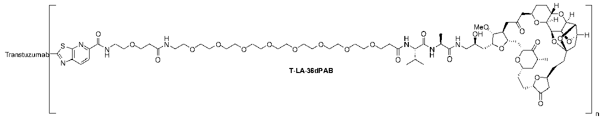 | 7.78 |
| T-LA-36V CMC | 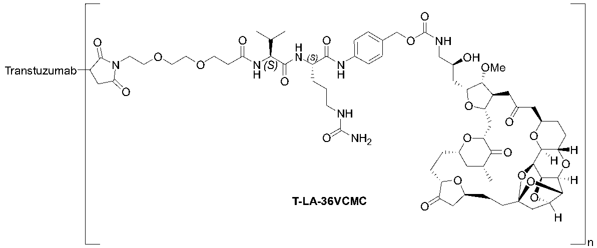 | 7.82 |
| T-LA-36V CdPAB | 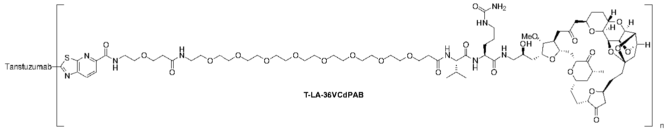 | 7.68 |
| T-LA-36V CP | 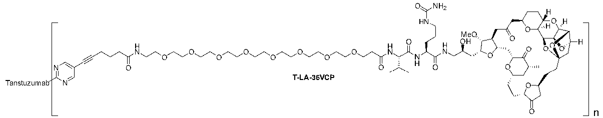 | 7.54 |
| T-LA-36O dPAB | 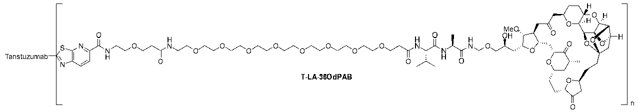 | 7.73 |
| T-LA-36O 2VC | 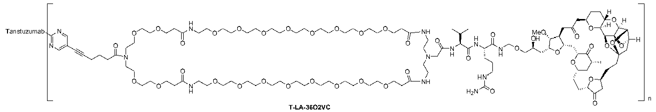 | 7.82 |
| T-LA-36O VCdPAB | 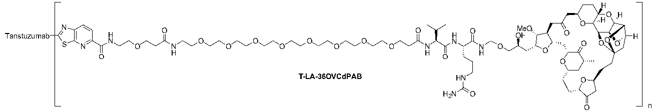 | 7.69 |
| T-LA-37A | 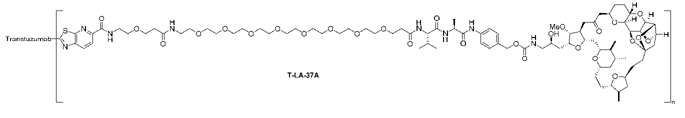 | 7.44 |
| T-LA-37B | 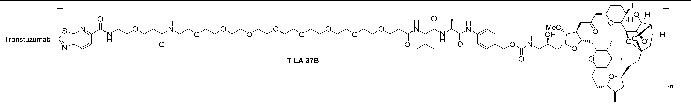 | 7.82 |
| T-LA-38 | 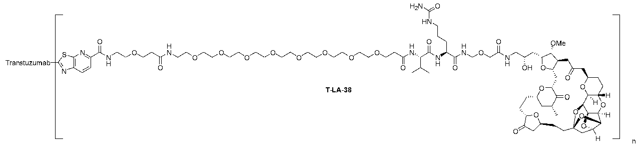 | 7.84 |

### Bioassay

### Test Example 1: Inhibitory Activity of Compounds in This Application on Cancer Cells

N87 gastric cancer cells (Shanghai Cell Bank of Chinese Academy of Sciences, SCSP-534) were cultured via RPMI640 medium (Gibco, 22400-105) containing 10% FBS (Gibco, 10099-141). On the day of the test, the cells were digested with 0.25% trypsin (Gibco, 25200-072), filtered with a sieve with a pore size of 40 µm, diluted with the corresponding medium until a cell density reached 1 × 10⁴ cells/mL, and inoculated onto a 96-well white cell culture plate (Beyotime, FCP968), 100 µL of cells were added per well, the culture plate was put in a 37°C incubator (Thermo 311) containing 5% CO₂, and the cells were further incubated for 24 hours. On Day 2, to-be-tested samples were prepared into a stock solution at a final concentration of 1M with DMSO (Sigma, D1650), then diluted with the corresponding medium until a final concentration reached 200 nM, and then subjected to four-fold serial dilutions, to prepare solutions at a total of 9 concentrations. 100 µL of the diluted sample was added to a 96-well plate, the plate was put in a 37°C incubator containing 5% CO₂, and the cells were further incubated for 144 hours. The cell culture plate was removed, 100 µL of cell viability assay agent (CellTiter-Glo^{®} Luminescent Cell Viability Assay, Promega, G7571) was added to each well, and the reaction solution allowed to react in the dark for 5 minutes at room temperature. Luminescence values were read by a microplate reader (TECAN SPARK) and data were processed with Graphpad Prism 7 software.

The inhibitory IC₅₀ values of the compounds in the present invention against N87 gastric cancer cells, MDA-MB-468 (Shanghai Cell Bank of Chinese Academy of Sciences, TCHu136) breast cancer cells, and JIMT-1 breast cancer cells (Cobioer, CBP60378) are shown in Table 1 below. The IC₅₀ value of PE-E (eribulin mesylate) was set to 1, and IC₅₀ values of other eribulin mesylate derivatives were multiples of eribulin. Inhibitory IC₅₀ values of different toxins PE-21EK and PE-AM4 on different cell lines are also shown in Table 2.

The structures of PE-E and PE-ALOH are as follows:

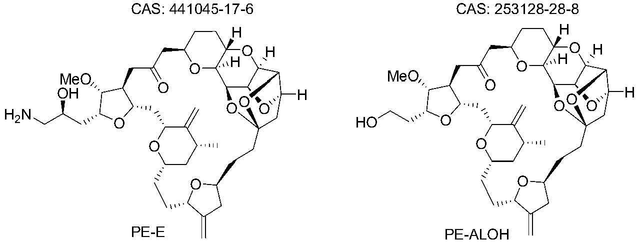

**Table 1: Inhibitory IC₅₀ Values of the Compounds in the Present Invention Against N87 Gastric Cancer Cells and JIMT-1 and MDA-MB-468 Breast Cancer Cells**

| No. | PE-E | PE-1 | PE-2 | PE-5 | PE-10 | PE-21C | PE-21EMS |
|---|---|---|---|---|---|---|---|
| N87 cell | 1 | 13.82 | 1.157 | 0.8925 | 7.746 | 0.3316 | 0.0549 |
| JIMT-1 cell | 1 | | | | | 0.1019 | 0.0959 |
| 468' cell | 1 | 47.35 | 5.234 | 1.398 | 43.19 | 0.2702 | 0.333 |

| No. | PE-21EMR | PE-21EK | PE-21G | PE-AM4 | PE-AM5 | PE-AL2H5 | PE-AL4 |
|---|---|---|---|---|---|---|---|
| N87 cell | 0.0411 | 0.7828 | 0.2097 | 0.8242 | 0.48 | 0.0841 | 0.1788 |
| JIMT-1 cell | 0.0568 | 0.1155 | 0.0561 | 0.6014 | 0.2946 | 0.07713 | 0.1085 |
| 468' cell | 0.2534 | 1.4948 | 0.1705 | 2.2506 | 1.737 | 0.4832 | 0.5057 |

| No. | PE-ALH5 | PE-AL5 | PE-ALOH | PE-E2K | PE-E4HB1 | PE-E4HS1 | PE-E2H |
|---|---|---|---|---|---|---|---|
| N87 cell | 0.1077 | 0.03422 | 1.52 | 14.1 | 15.5 | 4.5 | 0.0351 |
| JIMT-1 cell | 0.084 | 0.0413 | | | | | |
| 468' cell | 0.4939 | 0.3169 | 0.3682 | 71.51 | 80.39 | 23.24 | 0.1471 |

| No. | PE-21EA | PE-O2K | | | | | |
|---|---|---|---|---|---|---|---|
| N87 cell | 3.1266 | >100 | | | | | |
| JIMT-1 cell | 2.2109 | >100 | | | | | |
| 468' cell | 1.62413+ | 24.06 | | | | | |

The inhibitory IC₅₀ values of the compounds in the present invention against different cell lines measured according to the above similar methods are shown in Table 2 below.

**Table 2: Inhibitory IC₅₀ Values of Compounds PE-21EK and PE-AM4 in the Present Invention Against Other Cancer Cell Lines**

| Cell name | Cell source | Inhibitory IC₅₀ values | | |
|---|---|---|---|---|
| | | **PE-E** | **PE-21EK** | **PE-AM4** |
| SW620 | Colon cancer | 0.4188 | 0.02206 | 1.299 |
| MIA PaCa-2 | Pancreatic cancer | 0.6683 | 0.1577 | 1.225 |
| Renal cancer | ccRCC | 0.7546 | 0.1016 | 3.470 |
| IGROV-1 | Ovarian cancer | 0.4389 | 0.2892 | 0.6228 |
| H1975 | NSCLC | 0.5048 | 0.0708 | 0.7275 |
| MDA-MB-453 | Breast cancer | 19.27 | 0.0846 | 0.1511 |
| NSCLC | SCLC | 0.6492 | 0.1841 | 3.131 |
| A549 | NSCLC | 4.166 | 0.1938 | 4.9220 |
| NCI-H1650 | NSCLC | 2.35 | 0.2623 | 1.56 |
| MDAMB231 | Breast cancer | 0.8051 | 0.2179 | 1.085 |
| HS578T | Breast cancer | 0.1879 | 0.02066 | 0.2759 |
| HCC1937 | Breast cancer | 2.851 | 0.1044 | 0.6467 |
| Lncap cell | Prostate cancer | 1.296 | 0.1226 | 0.4573 |
| DU145 | Prostate cancer | 1.677 | 0.501 | 0.6857 |
| 22Rv1 | Prostate cancer | 0.618 | 0.1251 | 0.7409 |

As the results indicates, all the eribulin derivatives in the present application exhibit anticancer activity, and PE-21EK and PE-AM4 have comparable activity to eribulin at the small-molecule level.

### Test Example 2: Inhibitory Activity of ADCs in the Present Invention Against Cancer Cells

### Test Steps

Day 0: Cells in good growth states were digested, sifted and counted, 2000 cells and 100 µL of medium (N87 cells) were added to each well in a 96-well plate, 1000 cells and 100 µL of medium (MDA-MB-468 cells or JIMT-1) were added to each well in a 96-well plate, 200 µL of sterile water or PBS was added to the outer ring, and the cells were incubated for 24 hours.

Day 1: Samples were diluted in a medium gradient (9 gradients + 1 negative control group), N87 cells were plated and the corresponding ADC was added. Two parallel control groups were provided for each sample, an initial concentration was 100 nM, each of the 8 wells was then subjected to a 4-fold dilution, 100 µL of the corresponding sample was added to each well of the corresponding plate plated on Day 0, and the medium was added to the 11^{th} column of wells used as the control group. Two parallel control groups were provided for each sample, an initial concentration was 1000 nM, each of the 8 wells was then subjected to a 4-fold dilution, 100 µL of the corresponding sample was added to each well of the corresponding plate plated on Day 0, and the medium was added to the 11^{th} column of wells used as the control group.

Day 7: After 144 hours of cell treatment, a chromogenic agent CellTiter-Glo^{®} was dissolved 30 minutes in advance, 100 µL of the sample was added to each well and the corresponding plate was left standing in the dark for coloration for 2-5 minutes, readings were read twice, and a mean was selected for calculation.

**Table 3: Inhibitory IC₅₀ Values of ADCs in This Application Against N87 Gastric Cancer Cells and MDA-MB-468 and JIMT-1 Breast Cancer Cells**

| ADC No. | Inhibitory IC₅₀ values (nM) | | |
|---|---|---|---|
| | N87 | JIMT-1 | 468 |
| T-BA-1 | 0.01917 | 0.06162 | 6.378 |
| T-BA-2 | 0.0121 | 0.05371 | 6.487 |
| T-BA-3 | 0.005374 | 0.01867 | 3.814 |
| T-LA-1 | 0.01215 | 0.04525 | 7.682 |
| T-LA-2 | 0.01378 | 0.03784 | 8.546 |
| T-LA-3 | 0.007942 | | 2.143 |
| T-LA-9 | 0.008079 | | 5.172 |
| T-LA-11 | 0.01426 | | 4.289 |
| T-LA-24 | 0.0251 | 0.0768 | 3.36 |
| T-LA-25A | 0.02382 | 0.4321 | 21.72 |
| T-LA-25AA | 0.01051 | 0.04771 | 7.602 |
| T-LA-26 | 0.02587 | 0.3608 | 32.82 |
| T-LA-26B | 0.02333 | 0.4 | 15.33 |
| T-LA-27A | 0.0252 | 0.9732 | 11.13 |
| T-LA-27AA-2 | 0.009148 | 0.02625 | 17.28 |
| T-LA-27C | 0.08346 | 3 | 16.18 |
| T-LA-27A0 | 0.08938 | | |
| T-LA-27AA-20 | 0.07433 | | |
| T-LA-27AAVC | | 0.04353 | |
| T-LA-28AA-2 | 0.022 | 0.1107 | 10.68 |
| T-LA-28 | 0.03039 | 0.7 | 7.915 |
| T-LA-29 | 0.0346 | 0.6329 | 28.61 |
| T-LA-29AR | 0.04759 | 1.415 | 90 |
| T-LA-29B | 0.03092 | 0.3906 | 26.92 |
| T-LA-30A-1 | 0.07169 | 0.8 | 12.07 |
| T-LA-30A-2 | 0.06187 | 1 | 10.69 |
| T-LA-30B-P1 | 0.0401 | 8 | 19.8 |
| T-LA-30B-P2 | 0.0506 | 5 | 22.28 |
| T-LA-31A | 0.02991 | 0.5687 | 34.59 |
| T-LA-31B | 0.02483 | 3 | 19.36 |
| T-LA-32AS | 0.02296 | 0.4863 | 22.52 |
| T-LA-32AR | 0.02041 | 1.087 | 8.389 |
| T-LA-33A | 0.01606 | 0.1267 | 6.21 |
| T-LA-33B | 0.003705 | 0.02633 | 3.336 |
| T-LA-34A | 0.006712 | 0.03167 | 30.75 |
| T-LA-34B | 0.04068 | 0.2818 | 27.27 |
| T-LA-34B-2C-B | 55.79 | | |
| T-LA-35A | 0.04409 | 1 | 88 |
| T-LA-35B | 0.009978 | 0.2098 | 5.978 |
| T-LA-35C | 0.02821 | 0.1076 | 10.4 |
| T-LA-36 | 0.04318 | 0.1757 | >100 |
| T-LA-36dPAB | 0.0612 | 0.4208 | >100 |
| T-LA-36OdPAB | 0.2969 | 0.1423 | >100 |
| T-LA-36OVCdPAB | 0.07662 | 0.1942 | 30.51 |
| T-LA-36VCdPAB | 0.07433 | 0.3701 | >100 |
| T-LA-36VCP | 0.07522 | 0.4327 | >100 |

| | | | |
|---|---|---|---|
| Note: DAR values were corrected to DAR8 during cell inhibition experiments. | | | |

The results show that the ADCs of the eribulin derivatives in the present application have good anticancer activity.

### Test Example 3A: Efficacy Experiment of ADCs in This Application in a Human Breast Cancer JIMT-1 Xenograft Model

Human breast cancer JIMT-1 cell line (Cobioer, CBP60378) was cultured in DMEM medium (Gibco, catalog number: 11995-065) and 10% FBS (Excell Bio, catalog number: FCS500) in a 37°C incubator containing 5% CO₂. Cells were digested with 0.25% trypsin-EDTA, washed with PBS twice, counted by a cell counter, and diluted with PBS until a cell concentration reached 1x10⁸ cells/mL.

CB17 SCID mice (female, 4-6 weeks of age, Beijing Vital River Laboratory Animal Technology Co., Ltd.) were used. CB17 SCID mice were acclimated in the laboratory environment for 2-5 days, JIMT-1 cells were inoculated subcutaneously at the right rib, the amount of inoculated cells was 5x10⁶ cells/mouse, the inoculation volume was 0.1 mL (containing 50% Matrigel), and when sizes of the tumors reached about 150-200 mm³, the mice were dosed according to the schedule shown in Table 4. Tumor volumes and body weight were measured twice a week, and the tumor proliferation rate and relative tumor inhibition rate were calculated.

**Table 4: Dosing Schedule of ADCs in Mice Bearing Human Breast Cancer JIMT-1 Xenograft Tumors**

| No. | Group | Number of animals | Dosage | Route and cycle of administration |
|---|---|---|---|---|
| 1 | PBS | 6 | 10 mL/kg | *i.v.* twice |
| 2 | T-BA-1 | 6 | 3mg/kg | *i.v.* twice |
| 3 | T-BA-3 | 6 | 3mg/kg | *i.v.* twice |
| 4 | T-LA-27A | 6 | 3mg/kg | *i.v*. twice |
| 5 | T-LA-27C | 6 | 3mg/kg | *i.v.* twice |
| 6 | T-LA-34B | 6 | 3mg/kg | *i.v.* twice |
| 7 | T-LA-35A | 6 | 3mg/kg | *i.v.* twice |
| 8 | T-LA-32AS | 6 | 3mg/kg | *i.v.* twice |
| 9 | A023-LA-34B | 6 | 3mg/kg | *i.v.* twice |
| 10 | A023-LA-27C | 6 | 3mg/kg | *i.v.* twice |

At the end of the experiment, the mice were sacrificed and the tumors were cut off and weighed. Tumor volumes and body weight were measured twice a week, and the tumor proliferation rate and relative tumor inhibition rate were calculated. A calculation formula is as follows: Relative tumor growth rate: $T / C \left(%\right) = {T}_{RTV} / {C}_{RTV} \times 100 %$

(T_{RTV}: mean RTV of the treatment group; C_{RTV}: mean RTV of the control group; RTV = Vₜ/Vₒ, Vₒ is the tumor volume of the animal during grouping, and Vₜ is the tumor volume of the animal after treatment). Relative tumor inhibition rate: $TGI % = \left(1-T/C\right) \times 100 %$

(T and C are respectively relative tumor volumes (RTV) of the treatment and control groups at a specific time point).

Formula for calculating the tumor volume (V): V = l/2 × L_{L}× L_{S}²
L_{L} and L_{S} respectively denote the long and short diameters of the tumor.

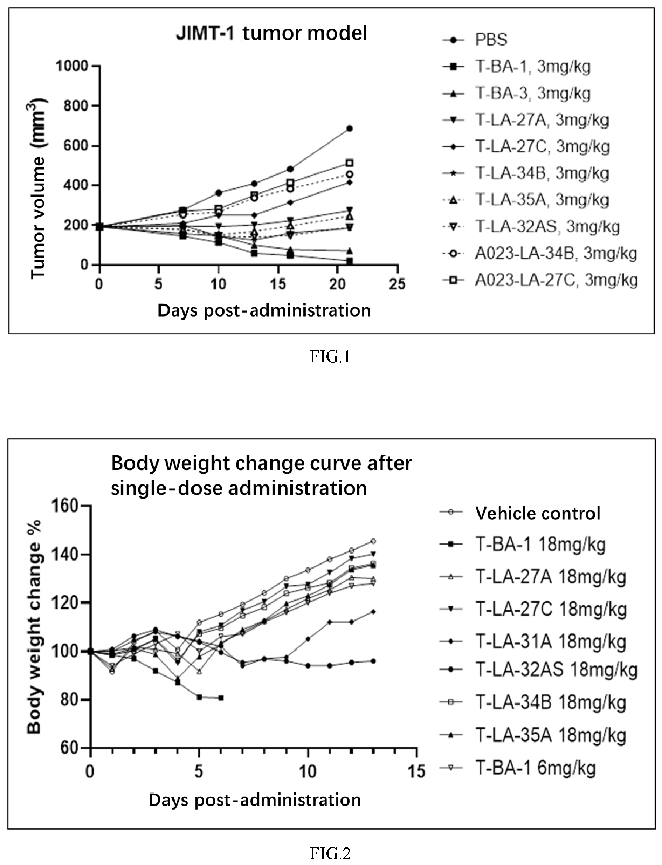

For experiment results, refer to Table 5 and FIG. 1.

**Table 5: Tumor Volume Data in Tumor-Bearing Mouse Model of JIMT-1 Breast Cancer**

| Sample | Tumor volume (mm³) | | | | | | T/C (%) | TGI (%) |
|---|---|---|---|---|---|---|---|---|
| | Day 0 of treatment | Day 7 of treatment | Day 10 of treatment | Day 13 of treatment | Day 16 of treatment | Day 21 of treatment | Day 21 of treatment | Day 21 of treatment |
| PBS | 192.86 | 276.22 | 362.57 | 409.27 | 482.90 | 687.50 | | |
| T-BA-1 | 195.53 | 147.25 | 114.01 | 60.39 | 49.49 | 20.85 | 3.57 | 96.43 |
| T-BA-3 | 191.86 | 158.37 | 148.77 | 100.01 | 78.61 | 73.24 | 12.50 | 87.50 |
| T-LA-2 7A | 192.06 | 197.24 | 194.82 | 203.04 | 224.27 | 273.55 | 39.50 | 60.50 |
| T-LA-2 7C | 192.39 | 211.98 | 251.27 | 251.43 | 313.57 | 416.57 | 61.50 | 38.50 |
| T-LA-3 4B | 194.76 | 199.25 | 146.22 | 126.89 | 161.98 | 185.57 | 29.04 | 70.96 |
| T-LA-3 5A | 193.06 | 178.29 | 152.90 | 169.55 | 197.90 | 247.18 | 35.64 | 64.36 |
| T-LA-3 2AS | 193.71 | 181.51 | 147.21 | 143.06 | 150.11 | 191.00 | 29.81 | 70.19 |
| A023-L A-34B | 192.41 | 254.99 | 266.62 | 337.73 | 382.98 | 457.05 | 68.66 | 31.34 |
| A023-L A-27C | 194.88 | 271.29 | 281.71 | 350.25 | 415.60 | 513.76 | 72.53 | 27.47 |

Results: On Day 21, the average tumor volume of the vehicle group (PBS) reached 687 mm³. Both control groups T-BA1 and T-BA3 exhibited favorable tumor inhibition rates: 96.43% and 87.5%, respectively. In addition, both eribulin derivatives T-LA-34B and T-32-AS exhibited favorable tumor inhibition rates, all exceeding 70%. T-LA-27A and T-LA-35A exhibited secondary tumor inhibition rates. T-LA-27C only exhibited a relatively low tumor inhibition rate: 38.5%. The two irrelevant control antibodies ADC A023-LA-34B and A023-LA-27C exhibited lowest tumor inhibition rates. During the administration period, each group of animals showed no significant weight loss and drug toxicity.

Conclusion: The ADCs of the eribulin derivatives in the present invention exhibit favorable antitumor activity.

### Test Example 3B: Efficacy Experiment of ADCs in This Application in a Human Breast Cancer JIMT-1 Xenograft Model

Human breast cancer JIMT-1 cell line (Cobioer, CBP60378) was cultured in DMEM medium (Gibco, catalog number: 11995-065) and 10% FBS (Excell Bio, catalog number: FCS500) in a 37°C incubator containing 5% CO₂. Cells were digested with 0.25% trypsin-EDTA, washed with PBS twice, counted by a cell counter, and diluted with PBS until a cell concentration reached 1x10⁸ cells/mL.

CB17 SCID mice (female, 4-6 weeks of age, Beijing Vital River Laboratory Animal Technology Co., Ltd.) were used. CB17 SCID mice were acclimated in the laboratory environment for 2-5 days, JIMT-1 cells were inoculated subcutaneously at the right rib, the amount of inoculated cells was 5x10⁶ cells/mouse, the inoculation volume was 0.1 mL (containing 50% Matrigel), and when sizes of the tumors reached about 150-200 mm³, the mice were dosed according to the schedule shown in Table 4. Tumor volumes and body weight were measured twice a week, and the tumor proliferation rate and relative tumor inhibition rate were calculated.

**Table 6: Dosing Schedule of ADCs in Mice Bearing Human Breast Cancer JIMT-1 Xenograft Tumors**

| No . | Group | Number of animals | Dosage | Route and cycle of administration |
|---|---|---|---|---|
| 1 | PBS | 6 | 10 mL/kg | *i.v.* twice |
| 2 | TJ106A028-LA36VCdPA B | 6 | 3mg/kg | *i.v.* twice |
| 3 | TJ106A028-LA36VCdPA B | 6 | 6mg/kg | *i.v.* twice |
| 4 | TJ106A028-LA36VCdPA B | 6 | 12mg/kg | *i*.*v.* twice |
| 5 | TJ106A028-LA36O2VC | 6 | 6mg/kg | *i.v.* twice |
| 6 | TJ106A028-LY22CD3 | 6 | 6mg/kg | *i.v.* twice |

At the end of the experiment, the mice were sacrificed and the tumors were cut off and weighed. Tumor volumes and body weight were measured twice a week, and the tumor proliferation rate and relative tumor inhibition rate were calculated. A calculation formula is as follows: Relative tumor growth rate: $T / C \left(%\right) = {T}_{RTV} / {C}_{RTV} \times 100 %$

(T_{RTV}: mean RTV of the treatment group; C_{RTV}: mean RTV of the control group; RTV = Vₜ/Vₒ, Vₒ is the tumor volume of the animal during grouping, and Vt is the tumor volume of the animal after treatment). Relative tumor inhibition rate: $TGI % = \left(1-T/C\right) \times 100 %$

(T and C are respectively relative tumor volumes (RTV) of the treatment and control groups at a specific time point).
Formula for calculating the tumor volume (V): V = 1/2 × L_{L}× L_{S}²

L_{L} and L_{S} respectively denote the long and short diameters of the tumor.

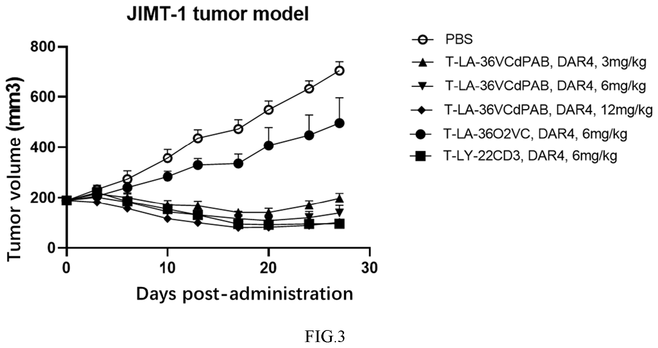

For experiment results, refer to Table 7 and FIG. 3.

**Table 7: Tumor Volume Data in Tumor-Bearing Mouse Model of JIMT-1 Breast Cancer**

| Sample | Tumor volume (mm³) | | | | | | | | | T/C (%) | TGI (%) |
|---|---|---|---|---|---|---|---|---|---|---|---|
| Days of treatment | Day 0 | Day 3 | Day 6 | Day 10 | Day 13 | Day 17 | Day 20 | Day 24 | Day 27 | Day 27 | Day 27 |
| PBS | 188. 98 | 232. 95 | 273. 79 | 357. 12 | 436. 61 | 472. 89 | 548. 92 | 632. 58 | 704. 78 | N/A | N/A |
| T-LA-36VCd PAB 3mg/kg | 188. 75 | 215. 33 | 200. 60 | 170. 66 | 167. 62 | 140. 73 | 140. 81 | 170. 69 | 197. 63 | 27.42 | 72.58 |
| T-LA-36VCd PAB 6mg/kg | 189. 04 | 201. 47 | 181. 88 | 143. 36 | 131. 70 | 116.0 8 | 108. 25 | 120. 40 | 138. 84 | 18.46 | 81.54 |
| T-LA-36VCd PAB 12mg/kg | 189. 14 | 181. 80 | 156. 85 | 116.1 8 | 100. 13 | 80.8 0 | 82.5 1 | 89.4 2 | 101. 97 | 14.64 | 85.36 |
| T-LA-36O2V C | 189.11 | 206.13 | 240.47 | 283.84 | 329.57 | 335.89 | 407.20 | 448.17 | 496.12 | 67.48 | 32.52 |
| T-LY-22CD3 | 188.91 | 220.99 | 184.63 | 154.05 | 131.26 | 95.27 | 92.51 | 95.81 | 96.40 | 13.41 | 86.59 |

Results: On Day 27, the average tumor volume of the vehicle group (PBS) reached 704 mm³. The eribulin derivative T-LA-36VCdPAB exhibited favorable tumor inhibition rates at different doses, all exceeding 70%. The 12 mg/kg dose group did not show a dose-dependent effect in the tumor inhibition rate compared to the 6 mg/kg dose group, indicating that the ADC approached target saturation in the tumor at 6 mg/kg. In addition, the tumor inhibition effects of the medium and high dose groups were similar to those of T-LY-22CD3. During the administration period, each group of animals showed no significant weight loss and drug toxicity.

Conclusion: The ADC T-LA-36VCdPAB, an eribulin derivative in the present invention, exhibits favorable antitumor activity.

### Test Example 4: Toxicity Test of the ADC in the Present Invention in Rats

1. Test method: SD rats were purchased from Beijing Vital River Laboratory Animal Technology Co., Ltd. and housed in SPF animal facilities. Rats with body weight of 200g-250g were randomly divided into 10 groups, with 5 rats in each group, and the rats were injected with corresponding doses of ADCs or the vehicle as a control group in the tail veins, and the day of injection was viewed as Day 1. The body weight and food intake of the rats was measured daily, activity statuses, fur and other abnormal appearance of the animals were observed, and it was observed whether there was diarrhea or soft stool. Blood samples were collected on Day 5 and Day 15 after administration for tests of blood biochemistry and routine blood indexes. At the end of the test on Day 22 after administration, the rats were euthanized and dissected to observe main organs (heart, liver, spleen, lungs, kidneys, and so on) to detect abnormal changes visible to the naked eye.
2. Test Results

From Day 1 to Day 10 post-administration, all rats in the T-BA-1 group at the dose of 18 mg/kg died on Day 6. On Day 6 post-administration, at the dose of 6 mg/kg, although all rats survived, they had lower body weights than rats receiving triple-dose derivatives T-LA-27A (18 mg/kg), T-LA-27C (18 mg/kg), T-LA-34B (18 mg/kg), and T-LA-35A (18 mg/kg). However, as shown in FIG. 2, the body weights of rats in all groups were lower than those of the vehicle control group. On Day 10 post-administration, one rat died in the T-LA-31A (18 mg/kg) group, and one rat died in the T-LA-32AS (18 mg/kg) group on Day 15. In addition, the rats in these two groups had significantly lower body weights than rats in other eribulin derivative groups.

Conclusion: The tolerance ranking of the ADCs in the present invention at the same dose (18 mg/kg) is as follows: T-LA-27C > T-LA-34B > T-LA-35A ≈ T-LA-27A ≈ T-BA-1 (6 mg/kg) > T-LA-31A > T-LA-32AS. It also demonstrates that rats receiving the eribulin derivative ADCs in this present application, T-LA-27C, T-LA-34B, T-LA-35A, and T-LA-27A, exhibit threefold tolerance relative to the rats receiving the eribulin ADC, T-BA-1, thereby providing a broader therapeutic window than T-BA-1.

## Claims

1. An eribulin derivative, which is a compound of formula (I) or (II), or an isotope-labeled compound, an optical isomer, a geometric isomer, a tautomer, an isomer mixture, or a pharmaceutically acceptable salt thereof; 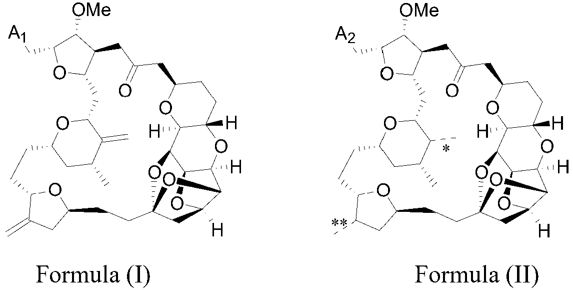 wherein:
A₁ in formula (I) is selected from 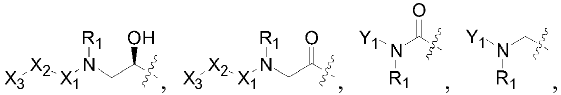 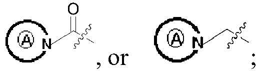
A₂ in formula (II) is selected from 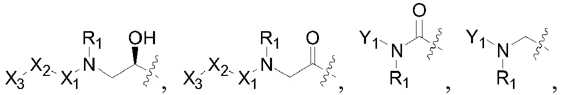 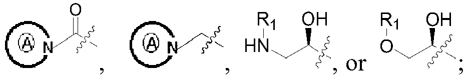 each of 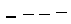 marked by * and ** is independently a single bond, a double bond, or an oxo bond (=O), and at least one of 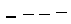 is not a double bond;
R₁ is selected from H, an alkyl group, a hydroxyl-substituted alkyl group, an alkyloxy-substituted alkyl group, an amino-substituted alkyl group, a haloalkyl group or a cyano-substituted alkyl group;
X₁ is selected from CO-, -CH₂, -S(O)-, or -S(O)₂-;
X₂ is selected from the direct bond, -O-, or -NR₁-;
X₃ is selected from -(CR₂R₃)ₘ₁-NH₂, -(CR₂R₃)ₘ₁-OH, -R₄-(CR₂R₃)ₘ₂-NH₂, -R₄-(CR₂R₃)ₘ₂-OH, or 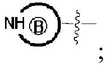
Yi is selected from -(CR₂R₃)ₘ₁-NH₂, -(CR₂R₃)ₘ₁-OH, -R₄-(CR₂R₃)ₘ₂-NH₂, -R₄-(CR₂R₃)ₘ₂-OH, or 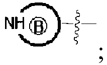
R₂ and R₃ are each independently selected from H, an alkyl group, a hydroxyl group, an alkyloxy group, an amino group, halogen, a cyano group, a hydroxyl-substituted alkyl group, an alkyloxy-substituted alkyl group, an amino-substituted alkyl group, a haloalkyl group, a cyano-substituted alkyl group, an alkyl group substituted by a substituted or unsubstituted carbon ring, an alkyl group substituted by a substituted or unsubstituted heterocyclic substituent, a substituted or unsubstituted carbon ring group or a substituted or unsubstituted heterocyclic group; and the substituted or unsubstituted carbon ring group and the substituted or unsubstituted heterocyclic group are optionally substituted by one or more substituents selected from an oxo group, an alkyl group, a hydroxyl group, an alkyloxy group, an amino group, halogen, a cyano group, a hydroxyl-substituted alkyl group, an alkyloxy-substituted alkyl group, an amino-substituted alkyl group, a haloalkyl group, and a cyano-substituted alkyl group;
R₄ is selected from a substituted or unsubstituted carbon ring group or a substituted or unsubstituted heterocyclic group; and the substituted or unsubstituted carbon ring group and the substituted or unsubstituted heterocyclic group are optionally substituted by one or more substituents selected from an oxo group, an alkyl group, a hydroxyl group, an alkyloxy group, an amino group, halogen, a cyano group, a hydroxyl-substituted alkyl group, an alkyloxy-substituted alkyl group, an amino-substituted alkyl group, a haloalkyl group, and a cyano-substituted alkyl group;
Ⓑ ring is a substituted or unsubstituted heterocyclic group; and a ring of the heterocyclic group contains at least one -NH-, and the substitution refers to substitution with one or more substituents selected from an oxo group, an alkyl group, an alkyloxy group, halogen, a cyano group, an alkyloxy-substituted alkyl group, a haloalkyl group, and a cyano-substituted alkyl group;
Ⓐ ring is the heterocyclic group; and a ring of the heterocyclic group contains at least one -NH-, and/or the heterocyclic group is substituted by an amino group, a hydroxyl group, an amino-substituted alkyl group, or a hydroxyl-substituted alkyl group, and the heterocyclic group is optionally substituted by one or more substituents selected from an oxo group, an alkyl group, an alkyloxy group, halogen, a cyano group, an alkyloxy-substituted alkyl group, a haloalkyl group, and a cyano-substituted alkyl group;
m1 is selected from 1, 2, 3, 4, 5, 6, 7, 8, 9, or 10; and
m2 is selected from 0, 1, 2, 3, 4, 5, 6, 7, 8, 9, or 10.

2. The eribulin derivative of claim 1, wherein the alkyl group is a C₁-C₁₂ linear or branched alkyl group; the carbon ring group is a saturated, partially unsaturated or completely unsaturated carbon ring group, and the number of carbon atoms is 3 to 20; and the heterocyclic group is a saturated, partially unsaturated or completely unsaturated heterocyclic group, containing 3 to 20 atoms and 1, 2, 3, or 4 heteroatoms selected from N, O, and S.

3. The eribulin derivative of claim 1 or 2, wherein R₁ is selected from H, a C₁-C₆ alkyl group, or a hydroxy-substituted C₁-C₆ alkyl group, or is preferably selected from H, a methyl group, an ethyl group, an n-propyl group, an isopropyl group, a hydroxymethyl group, a hydroxyethyl group, a hydroxy-n-propyl group, and a hydroxyisopropyl group.

4. The eribulin derivative of any one of claims 1 to 3, wherein X₁ is selected from -CO- and -CH₂-.

5. The eribulin derivative of any one of claims 1 to 4, wherein R₂ and R₃ are each independently selected from H, a C₁-C₆ alkyl group, a hydroxy group, a C₁-C₆ alkyloxy group, a hydroxy- substituted C₁-C₆ alkyl group, a C₁-C₆ alkyloxy-substituted C₁-C₆ alkyl group, a C₁-C₆ alkyl group substituted by a substituted or unsubstituted C₃-C₆ cycloalkyl group, a C₁-C₆ alkyl group substituted by a substituted or unsubstituted 3- to 6-membered heterocycloalkyl group containing 1 or 2 heteroatoms of N or O, a substituted or unsubstituted C₃-C₆ cycloalkyl group, and a substituted or unsubstituted 3- to 6-membered heterocycloalkyl group containing 1 or 2 heteroatoms of N or O; and the substituted or unsubstituted C₃-C₆ cycloalkyl group and the substituted or unsubstituted 3- to 6-membered heterocycloalkyl group containing 1 or 2 heteroatoms of N or O are optionally substituted with one or more substituents selected from oxo, a C₁-C₆ alkyl group, a hydroxy group, a C₁-C₆ alkyloxy group, a hydroxy-substituted C₁-C₆ alkyl group, and a C₁-C₆ alkyloxy-substituted C₁-C₆ alkyl group; or
preferably, R₂ and R₃ are each independently selected from H, a C₁-C₆ alkyl group, a hydroxy group, a hydroxy-substituted C₁-C₆ alkyl group, a C₁-C₆ alkyl group substituted by a substituted or unsubstituted C₃-C₆ cycloalkyl group, a C₁-C₆ alkyl group substituted by a substituted or unsubstituted 3- to 6-membered heterocycloalkyl group containing 1 or 2 heteroatoms of N or O, a substituted or unsubstituted C₃-C₆ cycloalkyl group, and a substituted or unsubstituted 3- to 6-membered heterocycloalkyl group containing 1 or 2 heteroatoms of N or O; and the substituted or unsubstituted C₃-C₆ cycloalkyl group and the substituted or unsubstituted 3- to 6-membered heterocycloalkyl group containing 1 or 2 heteroatoms of N or O are optionally substituted with one or more substituents selected from oxo, a C₁-C₆ alkyl group, a hydroxy group, and a hydroxy-substituted C₁-C₆ alkyl group.

6. The eribulin derivative of any one of claims 1 to 5, wherein R₄ is selected from a substituted or unsubstituted C₃-C₁₂ monocyclic, fused, bicyclic, spiro or bridged cycloalkyl group, a substituted or unsubstituted 3- to 12-membered monocyclic, fused, bicyclic, spiro or bridged heterocycloalkyl group containing 1, 2, or 3 heteroatoms of N or O, a substituted or unsubstituted C₆-C₁₀ aryl group, and a substituted or unsubstituted 5- to 10-membered heteroaryl group containing 1, 2, or 3 heteroatoms of N or O; and substitution means being substituted with one or more substituents selected from oxo, a C₁-C₆ alkyl group, a hydroxy group, a C₁-C₆ alkyloxy group, a hydroxy-substituted C₁-C₆ alkyl group, and a C₁-C₆ alkyloxy-substituted C₁-C₆ alkyl group; and
preferably, R₄ is selected from a substituted or unsubstituted C₄-C₁₂ monocyclic, fused, bicyclic, spiro or bridged cycloalkyl group, a substituted or unsubstituted 4- to 12-membered monocyclic, fused, bicyclic, spiro or bridged heterocycloalkyl group containing 1, 2, or 3 heteroatoms of N or O, a substituted or unsubstituted phenyl group, and a substituted or unsubstituted 5- to 10-membered heteroaryl group containing 1, 2, or 3 heteroatoms of N or O; and substitution means being substituted with one or more substituents selected from oxo and a C₁-C₆ alkyl group.

7. The eribulin derivative of any one of claims 1 to 6, wherein Ⓑ ring is a substituted or unsubstituted 3- to 12-membered monocyclic, fused, bicyclic, spiro or bridged heterocycloalkyl group containing 1, 2, or 3 heteroatoms of N or O, the heterocycloalkyl ring contains at least one -NH-, and substitution means being substituted with one or more substituents selected from oxo, a C₁-C₆ alkyl group, a C₁-C₆ alkyloxy group, and a C₁-C₆ alkyloxy-substituted C₁-C₆ alkyl group.

8. The eribulin derivative of any one of claims 1 to 4, wherein X₃ is selected from -(CR₂R₃)ₘ₁-NH₂, -(CR₂R₃)ₘ₁-OH, -R₄-(CR₂R₃)ₘ₂-NH₂, -R₄-(CR₂R₃)ₘ₂-OH, or 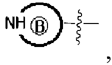 Ⓑ ring is a substituted or unsubstituted 4- to 12-membered monocyclic, fused, bicyclic, spiro or bridged heterocycloalkyl group containing 1, 2, or 3 heteroatoms of N or O, the substitution means being substituted with one or more substituents selected from oxo and a C₁-C₆ alkyl group; m1 is selected from 1, 2, 3, 4, 5, or 6; m2 is selected from 0, 1, 2, 3, 4, 5, or 6; R₂ and R₃ are each independently selected from H, a C₁-C₆ alkyl group, a hydroxy group, a hydroxy-substituted C₁-C₆ alkyl group, a C₁-C₆ alkyl group substituted by a substituted or unsubstituted C₃-C₆ cycloalkyl group, and a substituted or unsubstituted C₃-C₆ cycloalkyl group, wherein the substituted or unsubstituted C₃-C₆ cycloalkyl group is optionally substituted with one or more substituents selected from oxo and a C₁-C₆ alkyl group; and R₄ is selected from a C₄-C₁₂ monocyclic, fused, bicyclic, spiro or bridged cycloalkyl group, a 4- to 12-membered monocyclic, fused, bicyclic, spiro or bridged heterocycloalkyl group containing 1, 2, or 3 heteroatoms of N or O, and a phenyl group.

9. The eribulin derivative of claim 8, wherein X₃ is selected from the following groups: 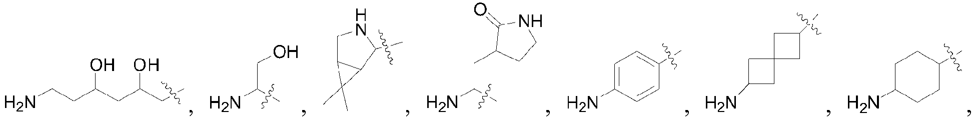 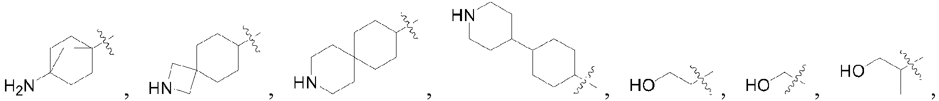 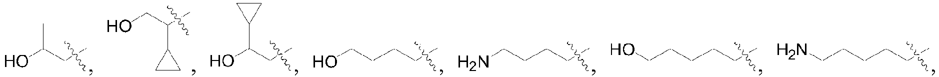 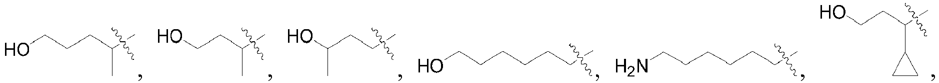 and 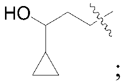 or
preferably, X₃ is selected from the following groups:

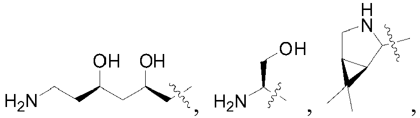

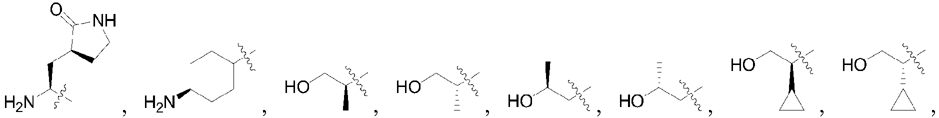

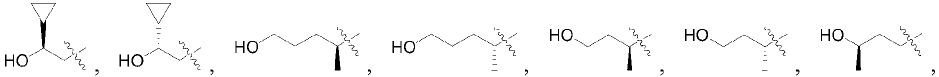

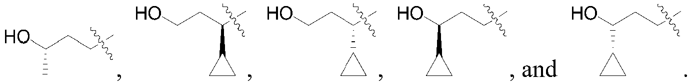

10. The eribulin derivative of any one of claims 1 to 4, wherein Yi is selected from -(CR₂R₃)ₘ₁-NH₂, -(CR₂R₃)ₘ₁-OH, -R₄-(CR₂R₃)ₘ₂-NH₂, -R₄-(CR₂R₃)ₘ₂-OH, or 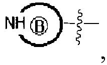 Ⓑ ring is a substituted or unsubstituted 4- to 12-membered monocyclic, fused, bicyclic, spiro or bridged heterocycloalkyl group containing 1, 2, or 3 heteroatoms of N or O, the substitution means being substituted with one or more substituents selected from oxo and a C₁-C₆ alkyl group; m1 is selected from 1, 2, 3, 4, 5, or 6; m2 is selected from 0, 1, 2, 3, 4, 5, or 6; R₂ and R₃ are each independently selected from H, a C₁-C₆ alkyl group, a hydroxy group, a hydroxy-substituted C₁-C₆ alkyl group, a C₁-C₆ alkyl group substituted by a substituted or unsubstituted C₃-C₆ cycloalkyl group, and a substituted or unsubstituted C₃-C₆ cycloalkyl group, wherein the substituted or unsubstituted C₃-C₆ cycloalkyl group is optionally substituted with one or more substituents selected from oxo and a C₁-C₆ alkyl group; and R₄ is selected from a C₄-C₁₂ monocyclic, fused, bicyclic, spiro or bridged cycloalkyl group, a 4- to 12-membered monocyclic, fused, bicyclic, spiro or bridged heterocycloalkyl group containing 1, 2, or 3 heteroatoms of N or O, and a phenyl group.

11. The eribulin derivative of claim 10, wherein Yi is selected from -(CR₂R₃)ₘ₁-NH₂ and -(CR₂R₃)ₘ₁-OH, m1 is selected from 1, 2, 3, 4, 5, or 6, and R₂ and R₃ are each independently selected from H, a C₁-C₆ alkyl group, a hydroxy group, or a hydroxy-substituted C₁-C₆ alkyl group; or preferably, Y₁ is

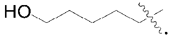

12. The eribulin derivative of claim 1, wherein Ⓐ ring is a 3- to 12-membered monocyclic, fused, bicyclic, spiro or bridged heterocycloalkyl group containing 1, 2, or 3 heteroatoms of N or O, the heterocycloalkyl ring contains at least one -NH- and/or the heterocycloalkyl group is substituted with an amino group, a hydroxy group, an amino-substituted C₁-C₆ alkyl group, or a hydroxy-substituted C₁-C₆ alkyl group, and the heterocycloalkyl group is optionally substituted with one or more substituents selected from oxo, a C₁-C₆ alkyl group, a C₁-C₆ alkyloxy group, or a C₁-C₆ alkyloxy-substituted C₁-C₆ alkyl group.

13. The eribulin derivative of claim 12, wherein Ⓐ ring is a 4- to 12-membered monocyclic, fused, bicyclic, spiro or bridged heterocycloalkyl group containing 1, 2, or 3 heteroatoms of N or O, and the heterocycloalkyl ring contains at least one -NH- and/or the heterocycloalkyl group is substituted with an amino group, a hydroxy group, an amino-substituted C₁-C₆ alkyl group, or a hydroxy-substituted C₁-C₆ alkyl group, and preferably, Ⓐ is selected from the following groups:

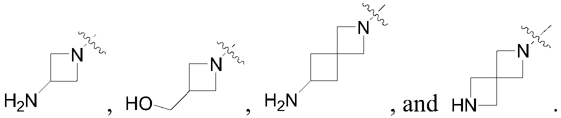

14. The eribulin derivative of claim 1, wherein A₁ and A₂ are selected from the following groups: 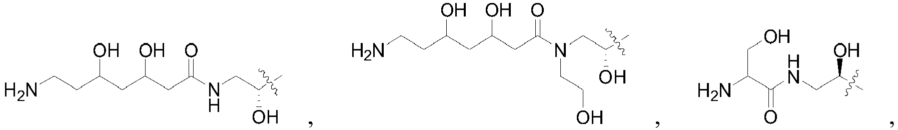 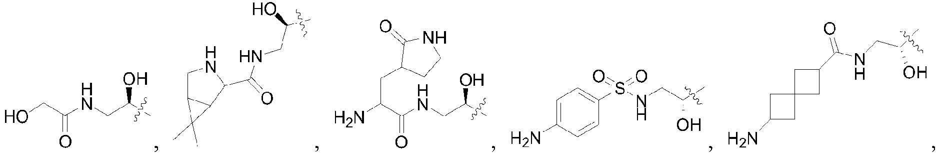 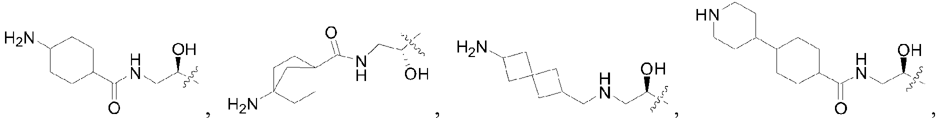 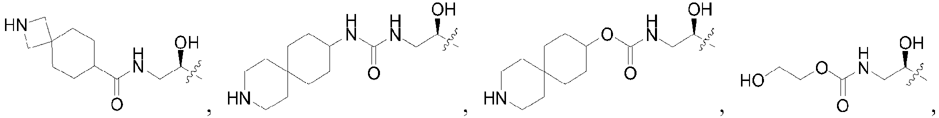 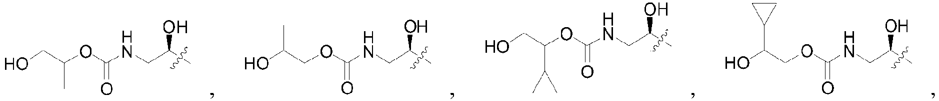 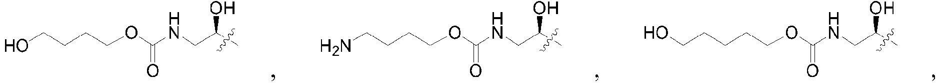 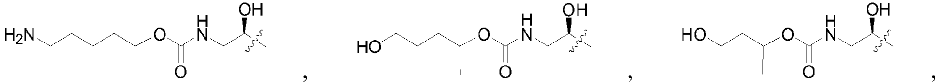 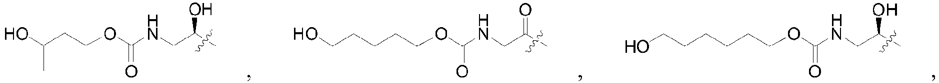 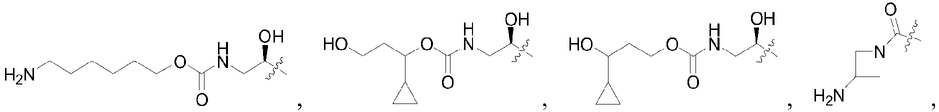 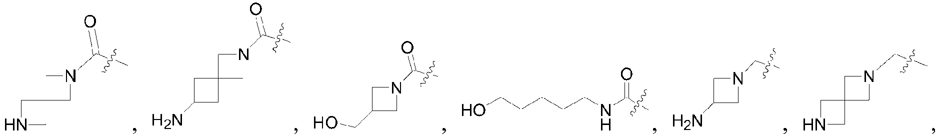 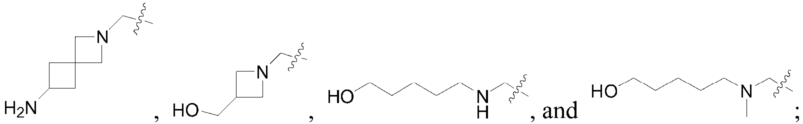 and
A₂ in formula (II) is alternatively selected from 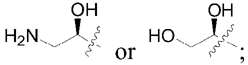 or
preferably, A₁ and A₂ are selected from the following groups:

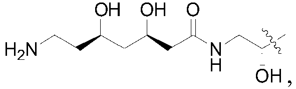

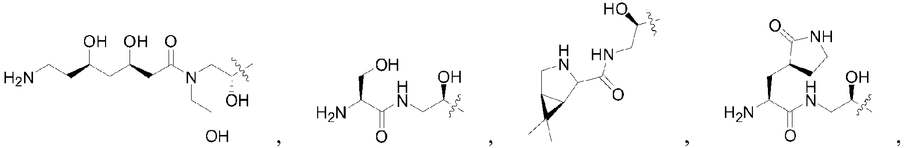

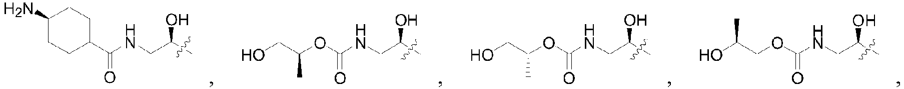

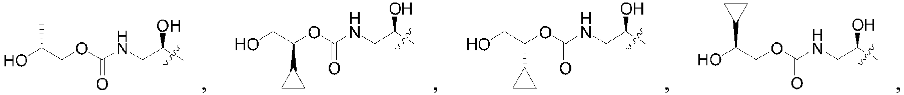

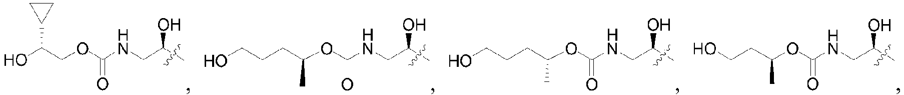

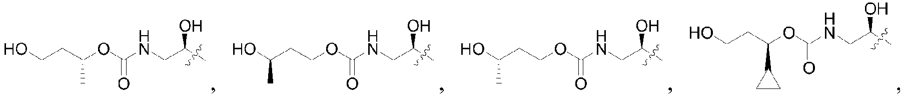

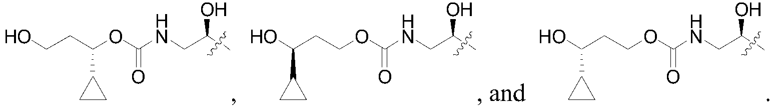

15. The eribulin derivative of claim 1, wherein the compound of formula (I) is one of the following: 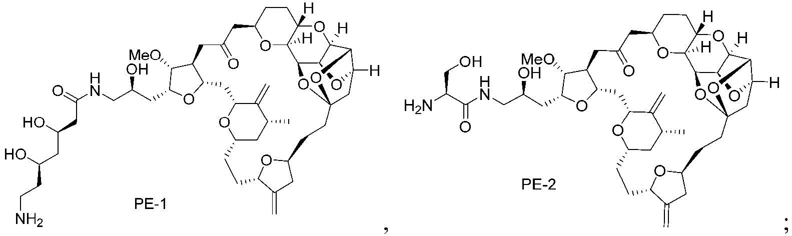 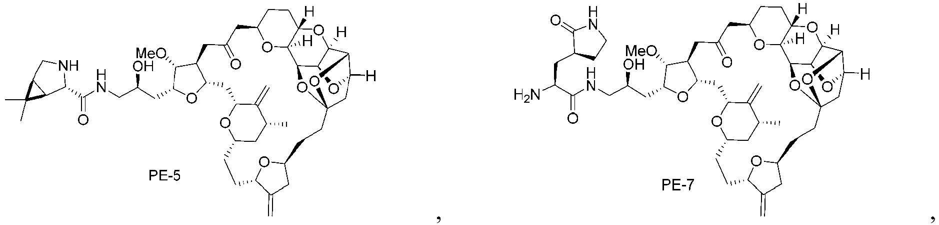 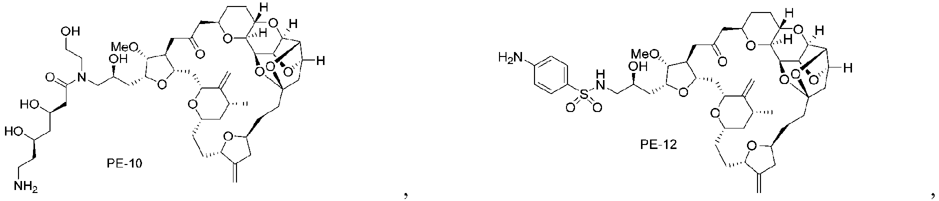 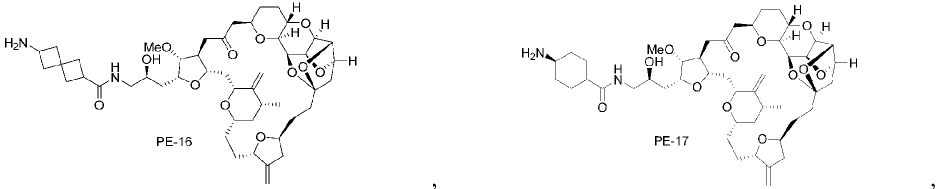 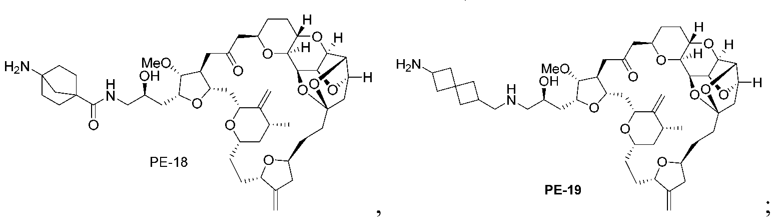 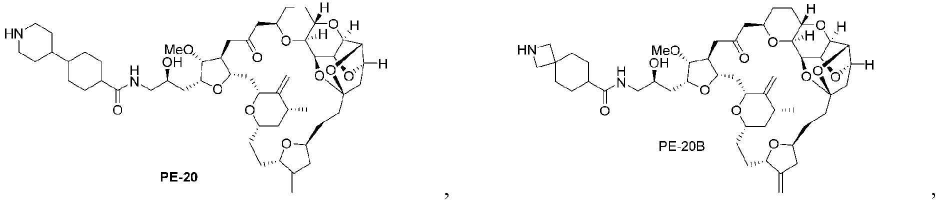 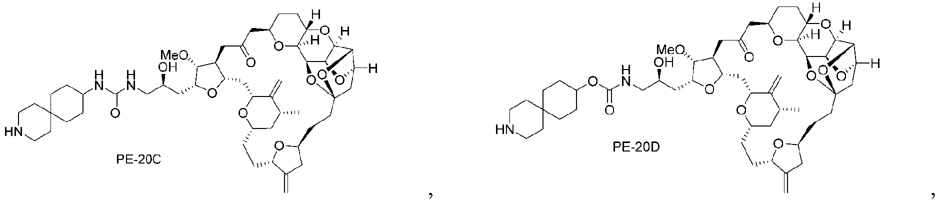 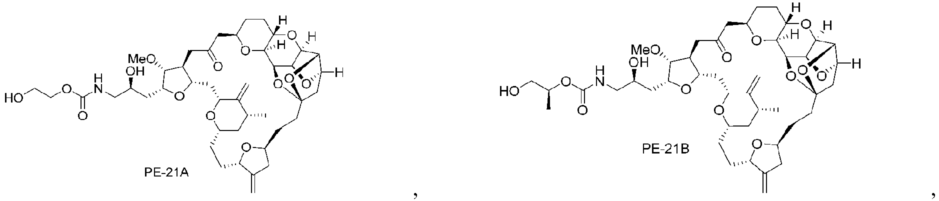 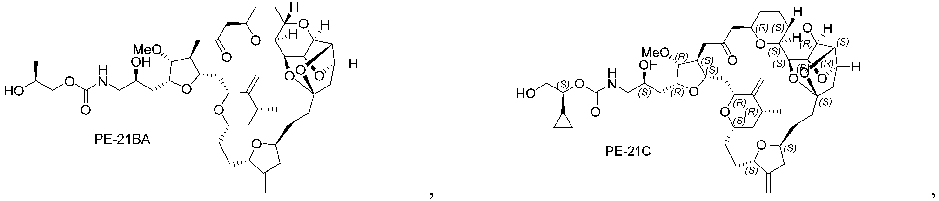 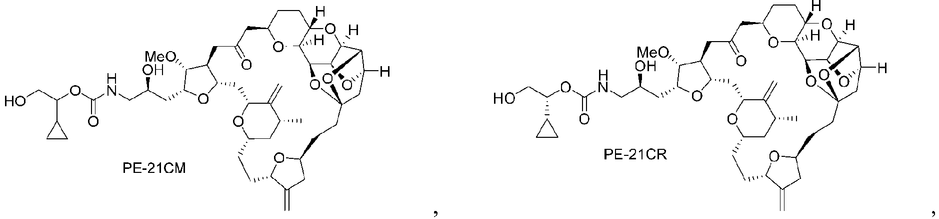 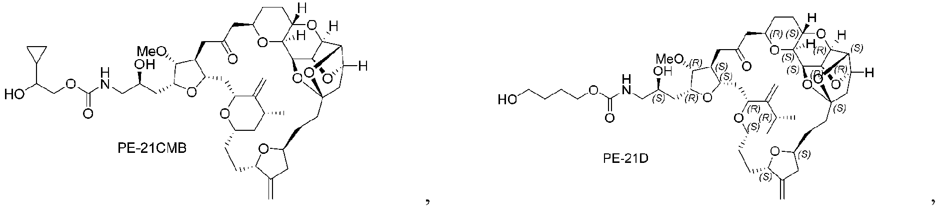 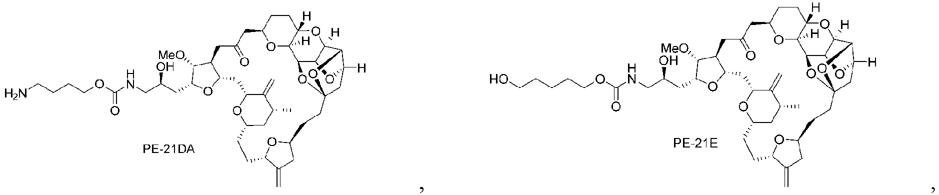 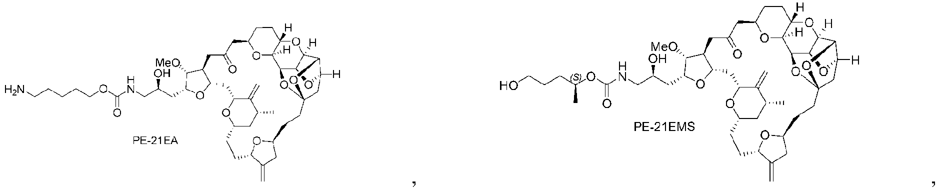 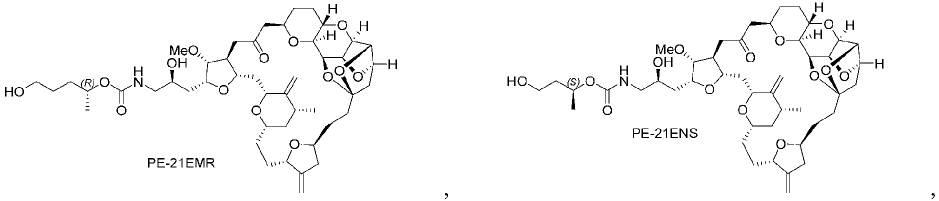 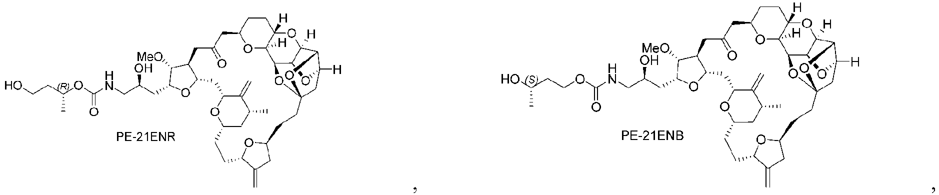 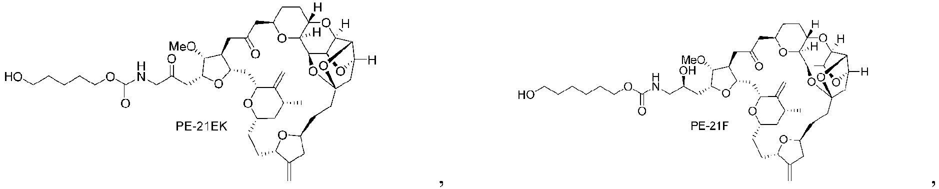 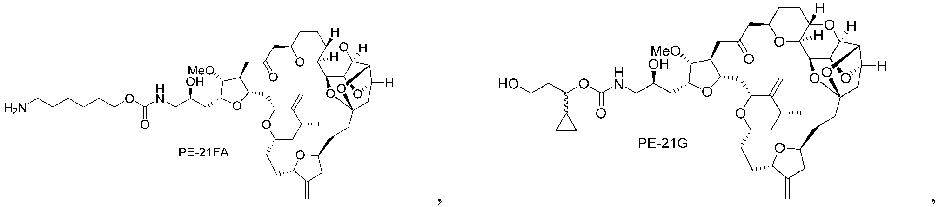 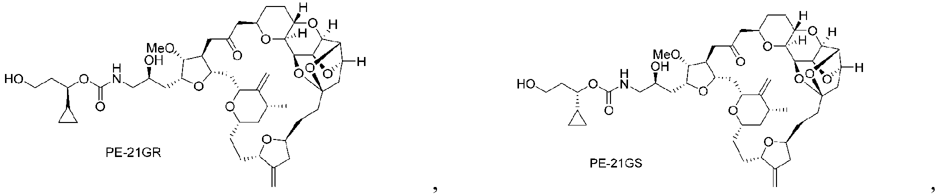 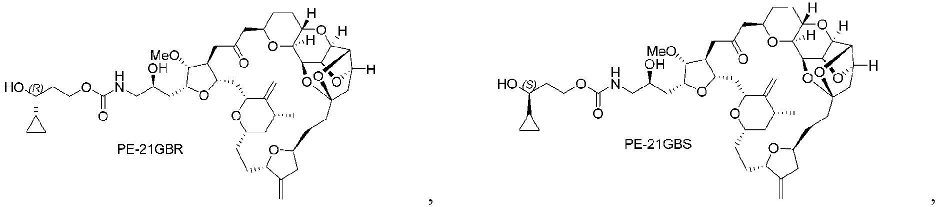 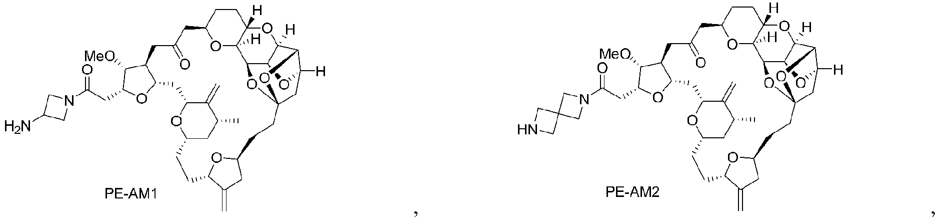 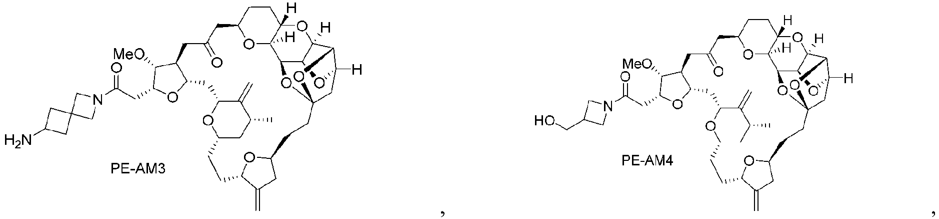 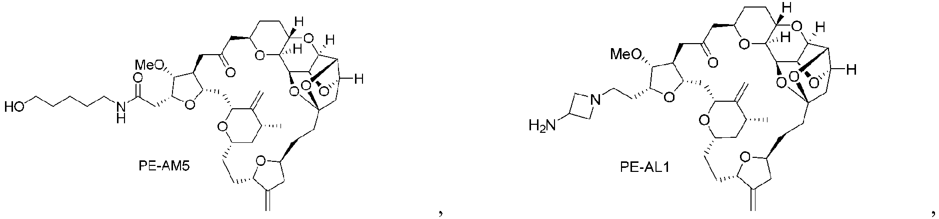 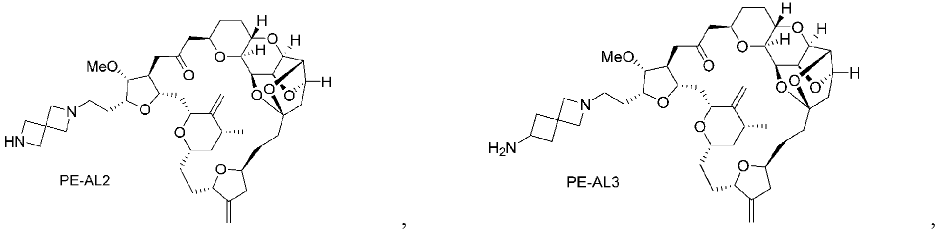 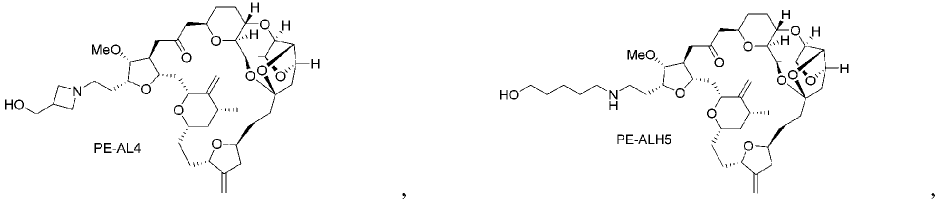 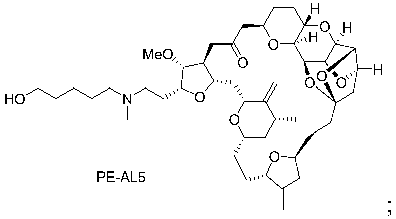 or
specifically, the compound of formula (II) is one of the following:

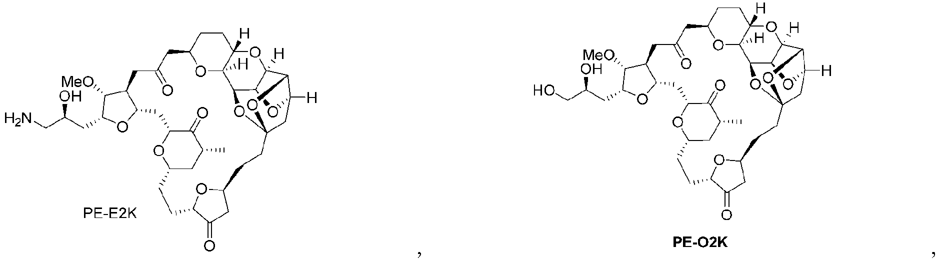

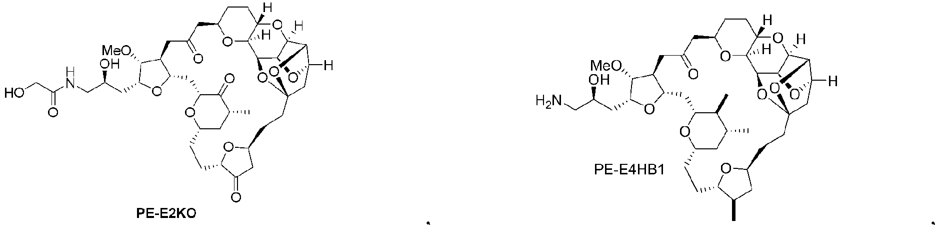

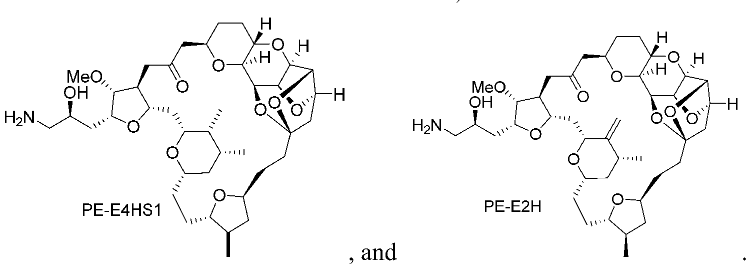

16. An eribulin derivative, which is a compound of formula (I') or (II'), or an isotope-labeled compound, an optical isomer, a geometric isomer, a tautomer, an isomer mixture, or a pharmaceutically acceptable salt thereof; 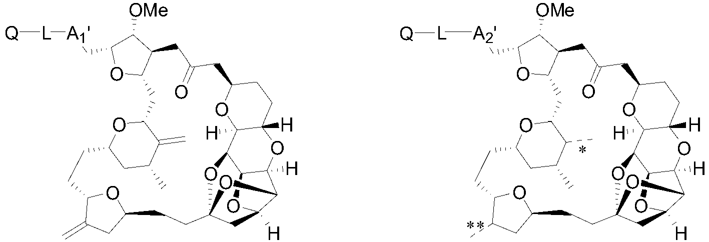 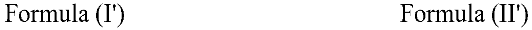 wherein:
Q is a linker group capable of conjugating to an antibody or antigen-binding fragment;
L is a linker group that connects Q to A₁' or Q to A₂';
A₁' is a group formed by connecting A₁ to L after removal of a hydrogen atom from -NH₂, -OH, -NH- on the chain, or -NH- on the ring of Ai;
A₂' is a group formed by connecting A₂ to L after removal of a hydrogen atom from -NH₂, -OH, -NH- on the chain, or -NH- on the ring of A₂; and
definitions of Ai, A₂, *, and ** are the same as those in formula (I) and formula (II) of any one of claims 1 to 15.

17. The eribulin derivative of claim 16, wherein when X₃ and Y₁ are each independently selected from -(CR₂R₃)ₘ₁-NH₂, -(CR₂R₃)ₘ₁-OH, -R₄-(CR₂R₃)ₘ₂-NH₂, and -R₄-(CR₂R₃)ₘ₂-OH, A₁' is a group formed by connecting A₁ to L after removal of the hydrogen atom from terminal -NH₂ or terminal -OH of X₃ or Y₁; and A₂' is a group formed by connecting A₂ to L after removal of the hydrogen atom from the terminal -NH₂ or terminal -OH of X₃ or Y₁;
when X₃ and Y₁ are each independently 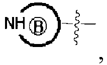 A₁' represents a group formed by connecting A₁ to L after removal of the hydrogen atom of -NH- on the Ⓑ ring, and A2' represents a group formed by connecting A₂ to L after removal of the hydrogen atom of -NH- on the Ⓑ ring; and
when A₁ and A₂ are each independently 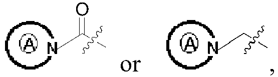 A₁' is a group formed by connecting A₁ to L after removal of the hydrogen atom of -NH- on the Ⓐ ring, or the hydrogen atom of -NH₂ or -OH on the Ⓐ ring substituent, and A₂' is a group formed by connecting A₂ to L after removal of the hydrogen atom of -NH- on the Ⓐ ring, or the hydrogen atom of -NH₂ or -OH on the Ⓐ ring substituent.

18. The eribulin derivative of claim 16, wherein R₁ is H, A₁' is a group formed by connecting A₁ to L after removal of R₁ from -NRi-, and A₂' is a group formed by connecting A₂ to L after removal of R₁ from -NR₁-.

19. The eribulin derivative of any one of claims 16 to 18, wherein L is -L₄-L₃-L₂-L₁-, and L₁ is connected to A₁' or A₂', and L₄ is connected to Q;
L₁ is selected from a direct bond, 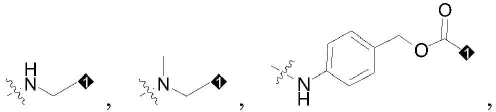 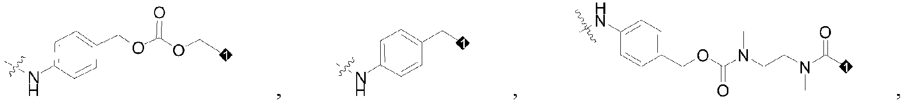 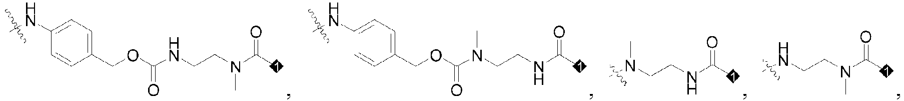 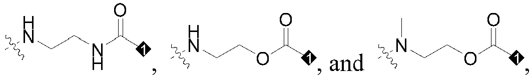 wherein 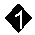 represents an attachment site to A₁' or A₂', and 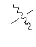 represents an attachment site to L₂; and
L₂ is a direct bond, an amino acid residue, a short peptide chain formed by two or more amino acid residues, the amino acid residues are selected from natural or unnatural amino acid residues, the amino acid residue or short peptide chain has N-terminus attached to L₃ and C-terminus attached to L₁, and L₂ optionally contains one or more structures selected from formula (III-1) to formula (III-9): 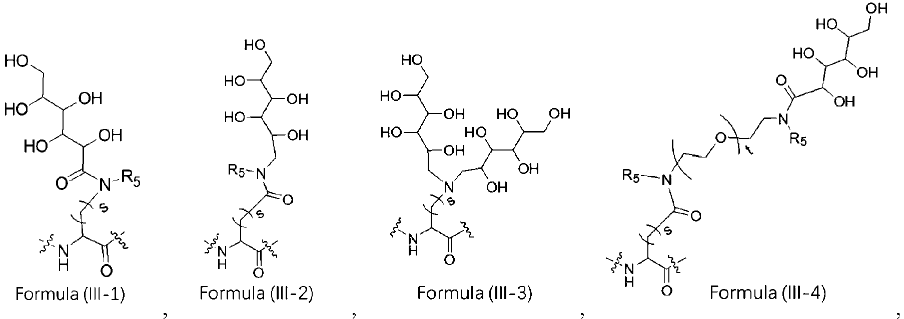 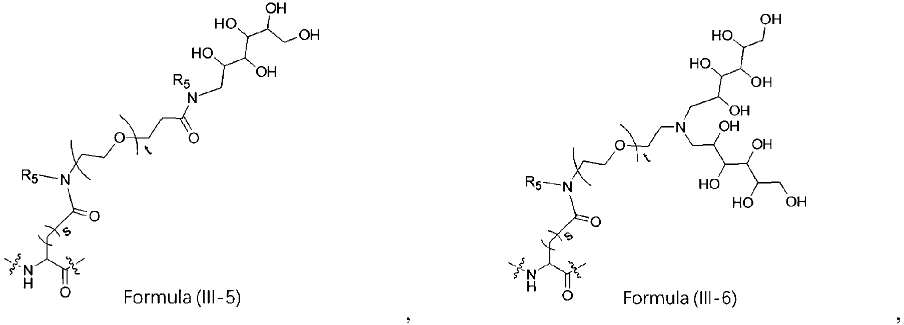 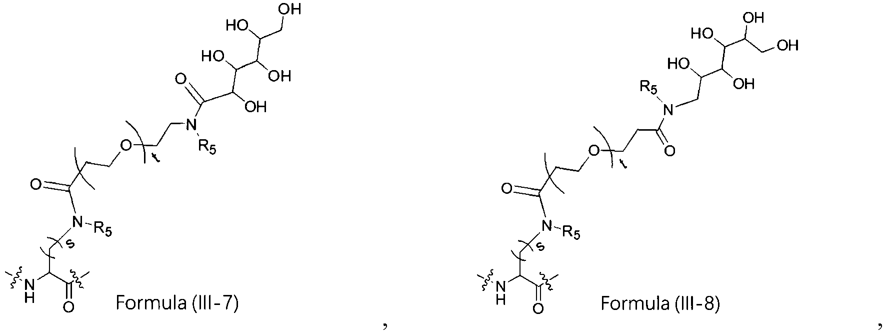 and 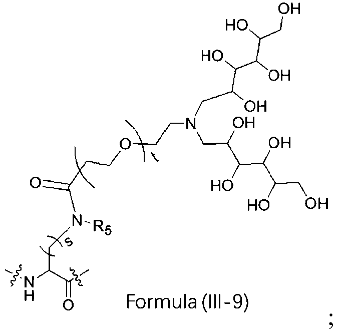
s is selected from 1, 2, 3, 4, 5, 6, 7, and 8, t is selected from 1, 2, 3, 4, 5, 6, 7, 8, 9, 10, 11, and 12, and R₅ is selected from H or a C₁-C₈ alkyl group;
L₃ is selected from a direct bond, -NR₆-(CH₂)ᵣ₁-(CH₂CH₂O)ᵣ₂-(CH₂)ᵣ₃-CO- 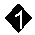 , -O-(CH₂)ᵣ₁- (CH₂CH₂O)ᵣ₂-(CH₂)ᵣ₃-CO- 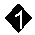 , -NR₆-(CH₂)ᵣ₄-(OCH₂CH₂)ᵣ₅-(CH₂)ᵣ₆-CO- 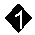 , -O-(CH₂)ᵣ₄-(OCH₂CH₂)ᵣ₅- (CH₂)ᵣ₆-CO- 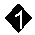 , or ; or L₃ is selected from: 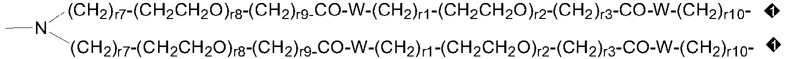 or 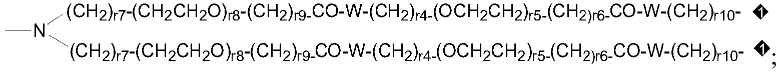 r1 is selected from 0, 1, 2, 3, 4, 5, or 6, r2 is selected from 1, 2, 3, 4, 5, 6, 7, 8, 9, 10, 11, or 12, r3 is selected from 1, 2, 3, 4, 5, or 6, r4 is selected from 1, 2, 3, 4, 5, or 6, r5 is selected from 1, 2, 3, 4, 5, 6, 7, 8, 9, 10, 11, or 12, r6 is selected from 0, 1, 2, 3, 4, 5, or 6, r7 is selected from 0, 1, 2, 3, 4, 5, or 6, r8 is selected from 1, 2, 3, 4, 5, 6, 7, 8, 9, 10, 11, or 12, r9 is selected from 1, 2, 3, 4, 5, or 6, r10 is selected from 1, 2, 3, 4, 5, or 6, W is selected from O or NR₆, R₆ is selected from H or a C₁-C₈ alkyl group, wherein 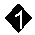 represents an attachment site to L₂, and when 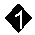 appears twice in the same L₃ group, it means that L₂ is attached at both sites; and
L₄ is selected from a direct bond, -CO-, -(CH₂)ₚ₁-CO-, -(CH₂CH₂O)ₚ₂-(CH₂)ₚ₃-CO-, -(CH₂)ₚ₄ -O-(CH₂)ₚ₅-C(O)-, -NR₇-(CH₂)ₚ₁-CO-, -NR₇-(CH₂CH₂O)ₚ₂-(CH₂)ₚ₃-CO-, -NR₇-(CH₂)ₚ₄-O-(CH₂)ₚ₅-C(O)-, -O-(CH₂)ₚ₁-CO-, -O-(CH₂CH₂O)ₚ₂-(CH₂)ₚ₃-CO-, and -O-(CH₂)ₚ₄-O-(CH₂)ₚ₅-C(O)-, p1, p2, p3, p4, and p5 are each independently selected from 1, 2, 3, 4, 5, 6, 7, and 8, and R₇ is selected from H or a C₁-C₈ alkyl group, wherein -CO- is attached to L₃.

20. The eribulin derivative of claim 19, wherein A₁' represents a group formed by connecting A₁ to L after removal of the hydrogen atom from -NH₂, -NH- on the chain, or -NH- on the ring, and A₂' represents a group formed by connecting A₂ to L after removal of the hydrogen atom from -NH₂, -NH- on the chain, or -NH- on the ring, and L₁ is selected from a direct bond, 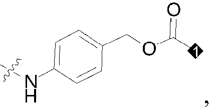 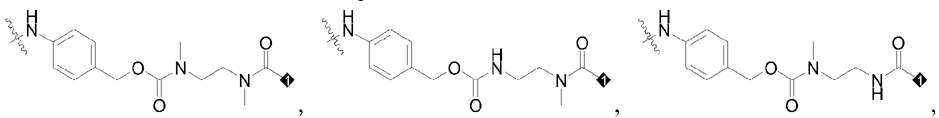 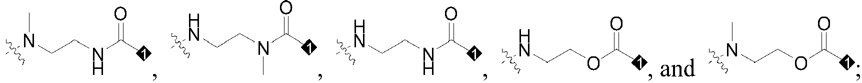 or
when A₁' represents a group formed by connecting A₁ to L after removal of the hydrogen atom of -OH-, or A2' represents a group formed by connecting A₂ to L after removal of the hydrogen atom of -OH-, L₁ is selected from 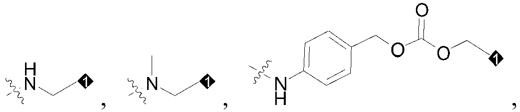 and

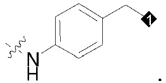

21. The eribulin derivative of claim 19, wherein L₁ is selected from the direct bond, 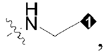 and

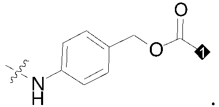

22. The eribulin derivative of any one of claims 19 to 21, wherein L₂ is an amino acid residue or a short peptide chain formed by 2 to 8 amino acid residues, and preferably, the amino acid residues are amino acid residues formed by amino acids selected from phenylalanine, alanine, glycine, valine, citrulline, leucine, isoleucine, tryptophan, tyrosine, histidine, lysine, serine, threonine, cysteine, glutamic acid, glutamine, aspartic acid, asparagine, methionine, and arginine, or more preferably, the amino acid residues are amino acid residues formed by amino acids selected from phenylalanine, alanine, glycine, valine, and citrulline.

23. The eribulin derivative of claim 22, wherein L₂ optionally comprises one or more structures selected from formula (III-1) and formula (III-9), or preferably, comprises one or more structures selected from the following groups: 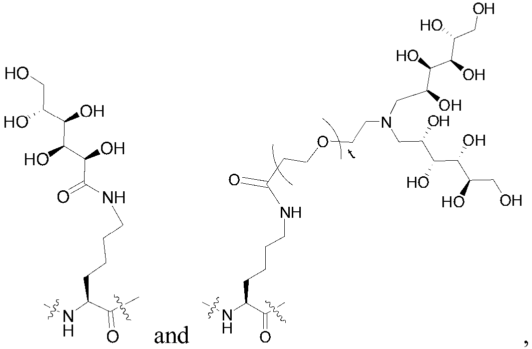 wherein t=8.

24. The eribulin derivative of claim 22 or 23, wherein L₂ is selected from -^{NH}Gly^{CO}-, or selected from the following short peptide chains: -^{NH}Gly-Gly-Phe-Gly^{CO}-, -^{NH}Val-Cit^{CO}-, -^{NH}Val-Ala^{CO}-, -^{NH}Gly-Gly-Val-Ala^{CO}-, -^{NH}Gly-Gly-Val-Cit^{CO}-, -^{NH}Lys-Gly-Val-Ala^{CO}-, -^{NH}Lys-Gly-Val-Cit^{CO}-, and -^{NH}Gly-Val-Cit^{CO}-.

25. The eribulin derivative of claim 24, wherein L₂ is selected from the following groups: 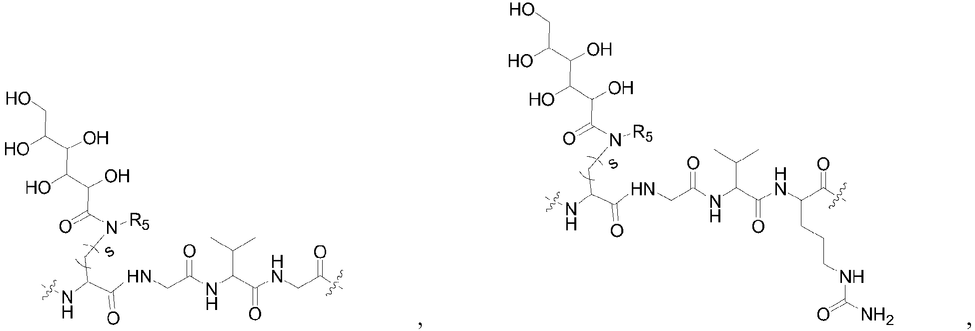 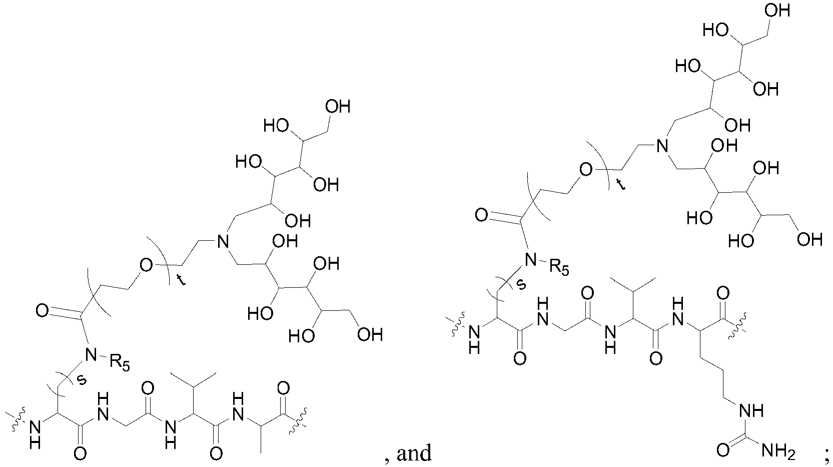 or
preferably, L₂ is selected from the following groups: 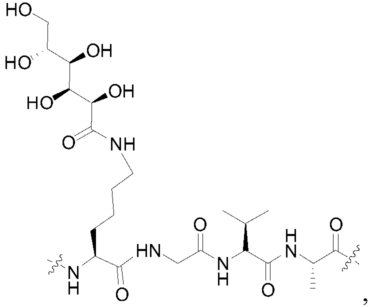 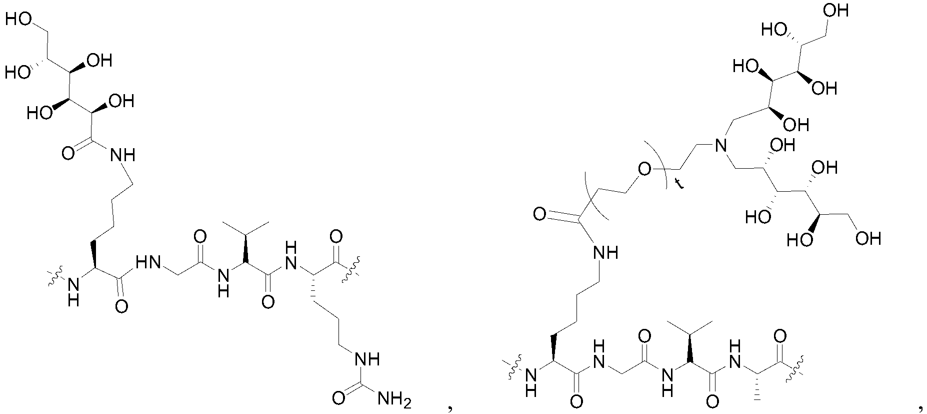 and 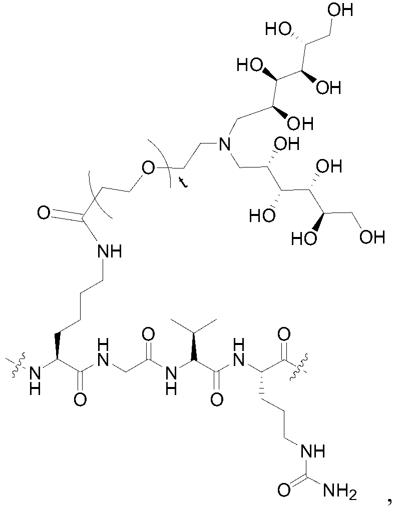 wherein t=8.

26. The eribulin derivative of any one of claims 19 to 25, wherein L₃ is selected from the direct bond, -NH-(CH₂CH₂O)ᵣ₂-(CH₂)ᵣ₃-CO- 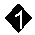 , -NH-(CH₂)ᵣ₄-(OCH₂CH₂)ᵣ₅-CO- 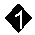 , -O-(CH₂CH₂O)ᵣ₂-(CH₂)ᵣ₃-CO- 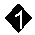 , or -O-(CH₂)ᵣ₄-(OCH₂CH₂)ᵣ₅-CO- 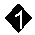 ; or L₃ is selected from 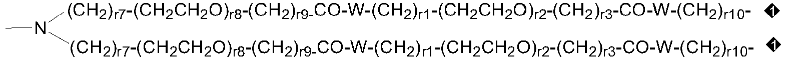 or 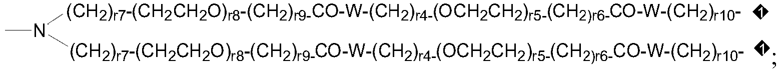 r1, r6, or r7 is 0, r2 is selected from 4, 5, 6, 7, or 8, r3 is selected from 1, 2, 3, or 4, r4 is selected from 1, 2, 3, or 4, r5 is selected from 4, 5, 6, 7, or 8, r8 is selected from 1, 2, 3, or 4, r9 is selected from 1, 2, 3, or 4, r10 is selected from 1, 2, 3, or 4, and W is NH; or
preferably, L₃ is selected from the direct bond, -O-(CH₂CH₂O)ᵣ₂-(CH₂)ᵣ₃-CO- 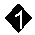 , -NH-(CH₂CH₂O)ᵣ₂-(CH₂)ᵣ₃-CO- 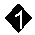 , and 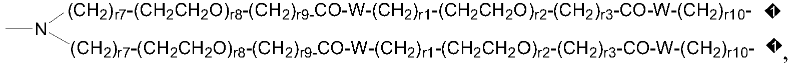 r1 and r7 are 0, r2 is selected from 4, 5, 6, 7, or 8, r3 is 2, r8 is selected from 2, 3, or 4, r9 is 2, and r10 is 2.

27. The eribulin derivative of any one of claims 19 to 26, wherein L₄ is selected from the direct bond, -(CH₂)ₚ₁-CO-, -(CH₂CH₂O)ₚ₂-(CH₂)ₚ₃-CO-, -(CH₂)ₚ₄-O-(CH₂)ₚ₅-C(O)-, -NR₇-(CH₂)ₚ₁-CO-, -NR₇-(CH₂CH₂O)ₚ₂-(CH₂)ₚ₃-CO-, or -NR₇-(CH₂)ₚ₄-O-(CH₂)ₚ₅-C(O)-, p1 is selected from 1, 2, 3, 4, 5, or 6, p2, p3, p4, and p5 are each independently selected from 1, 2, 3, or 4, and R₇ is selected from H or a C₁-C₆ alkyl group; or preferably, L₄ is selected from the following groups: the direct bond, -(CH₂)ₚ₁-CO-, -(CH₂CH₂O)ₚ₂-(CH₂)ₚ₃-CO-, -(CH₂)ₚ₄-O-(CH₂)ₚ₅-C(O)-, -NR₇-(CH₂)ₚ₁-CO-, -NR₇-(CH₂CH₂O)ₚ₂-(CH₂)ₚ₃-CO-, or -NR₇-(CH₂)ₚ₄-O-(CH₂)ₚ₅-C(O)-, p1 is selected from 3, 4, 5, or 6, p2, p3, p4, and p5 are each independently selected from 2 and 3, and R₇ is H.

28. The eribulin derivative of any one of claims 16 to 27, wherein Q is selected from a group comprising a maleimide linker, a group comprising a mesylpyrimidine linker, a group comprising a mesylpyridothiazole linker, a group comprising a mesylpyrimidothiazole linker, a group comprising a cycloalkyne linker, or a group comprising an oxime bond linker.

29. The eribulin derivative of claim 28, wherein Q is selected from 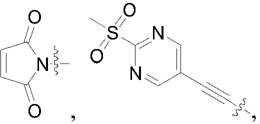 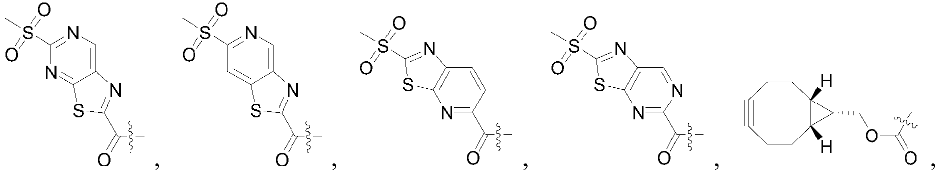 and

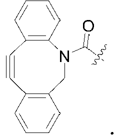

30. The eribulin derivative of claim 29, wherein Q-L₄ is selected from 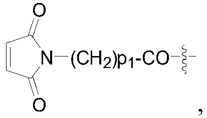 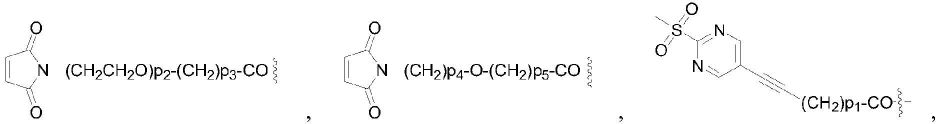 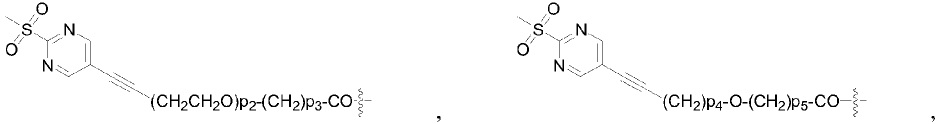 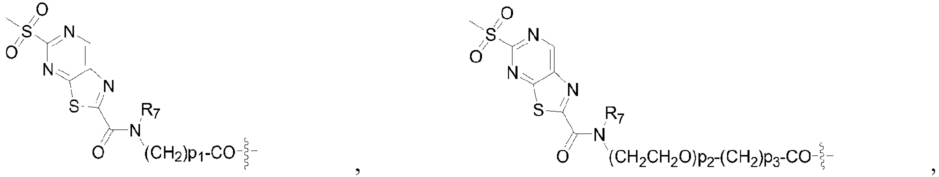 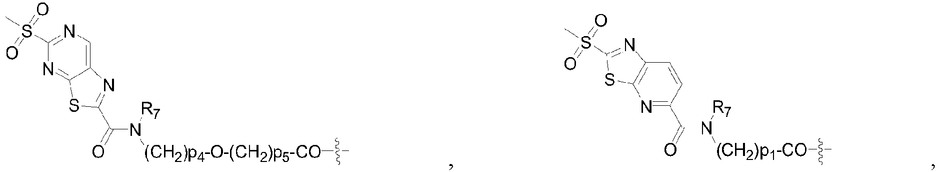 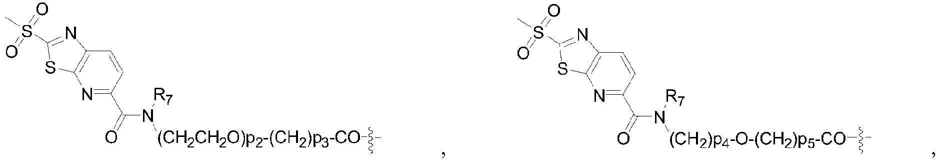 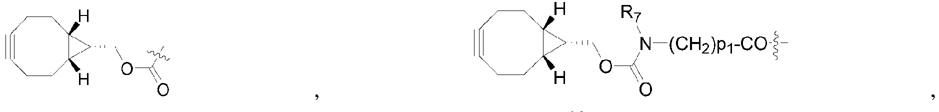 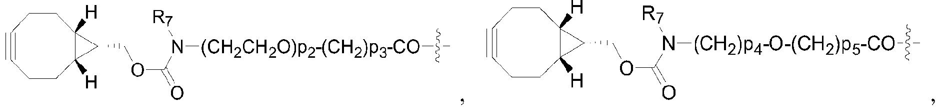 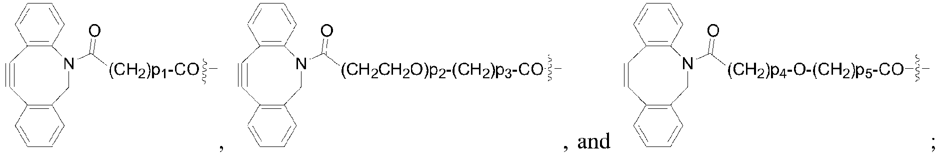 or
preferably, Q-L₄ is selected from

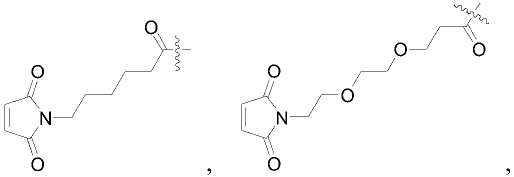

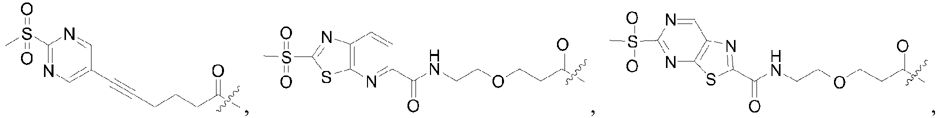

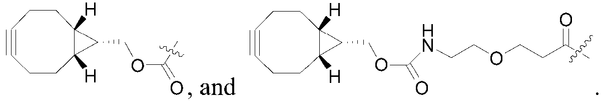

31. The eribulin derivative of claim 16, wherein formula (I') is one of the following: 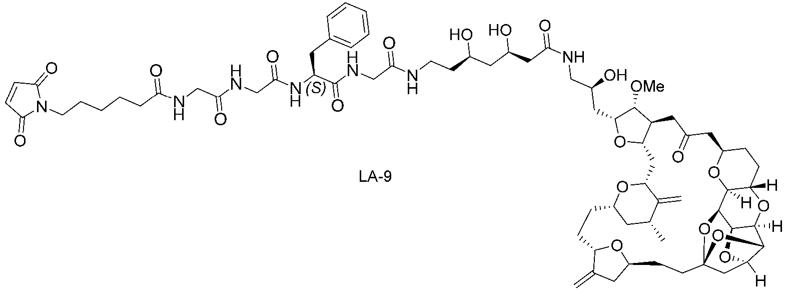 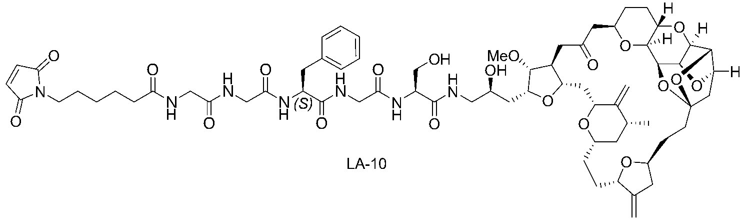 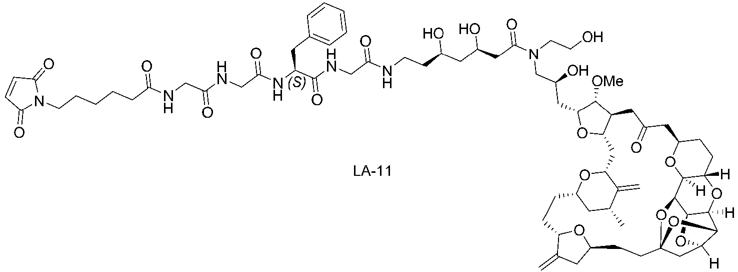 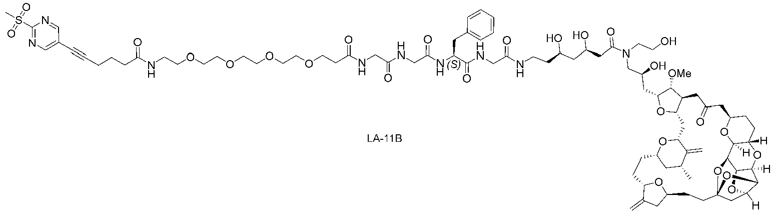 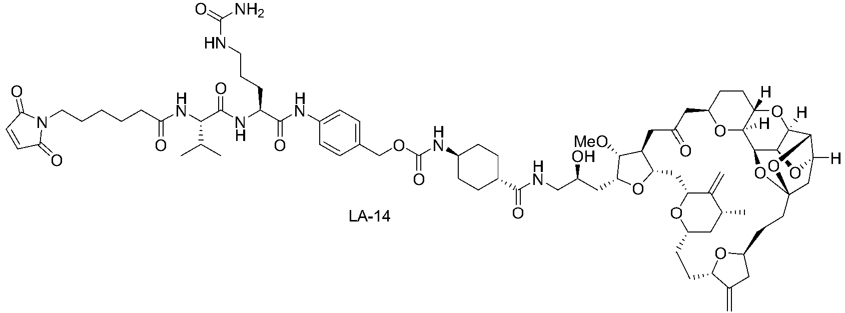 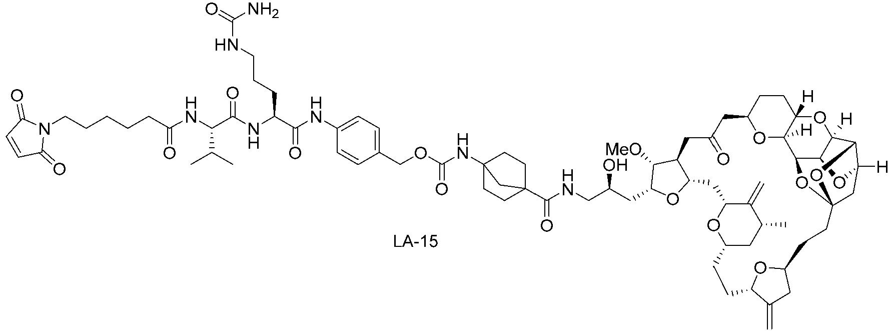 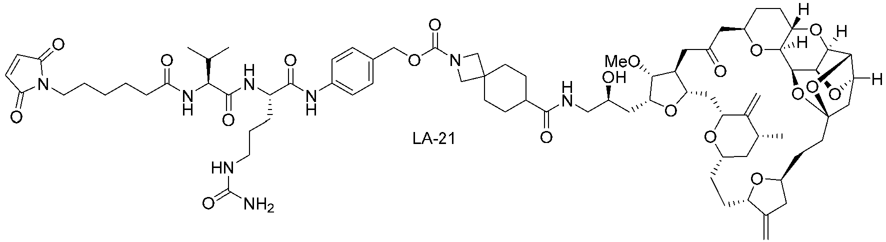 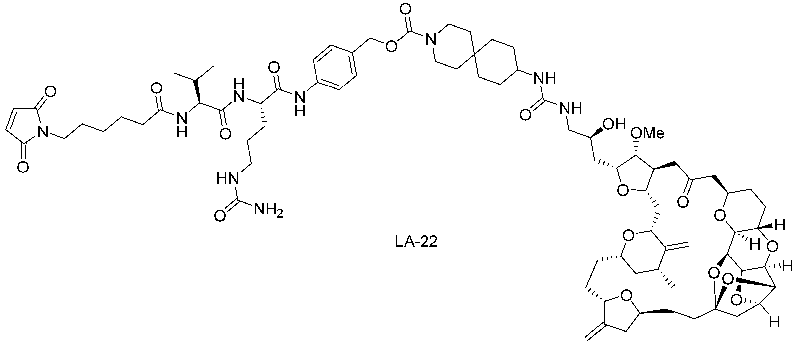 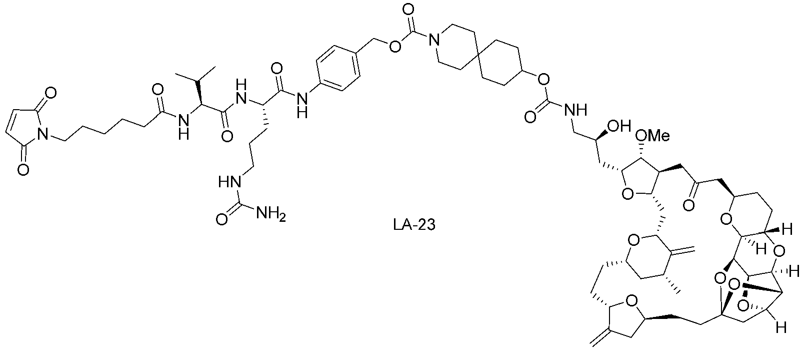 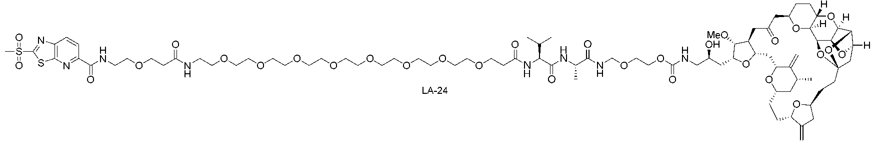 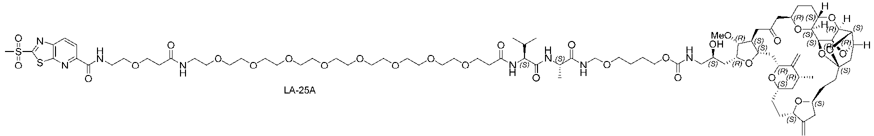 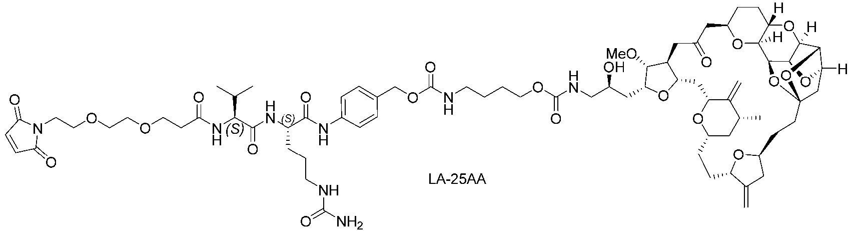 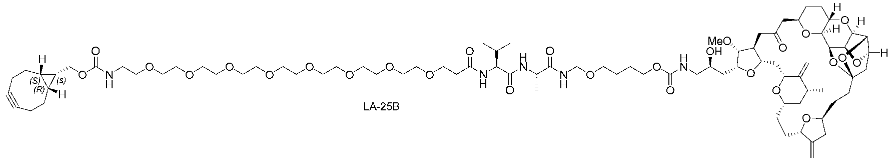 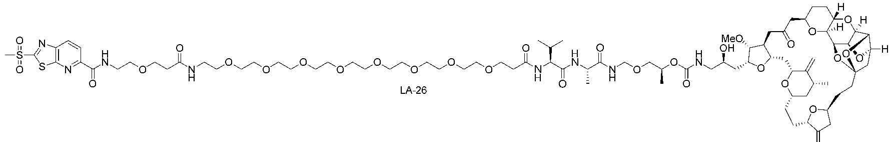 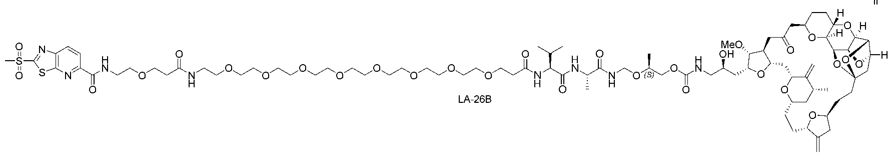 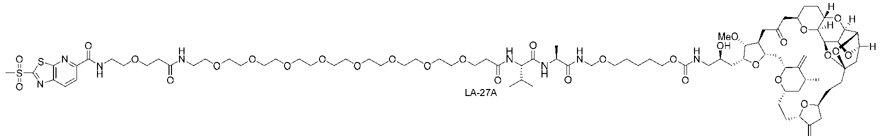 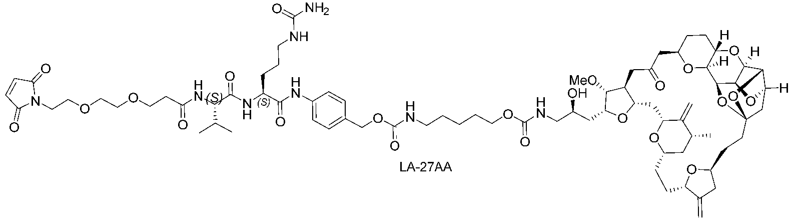 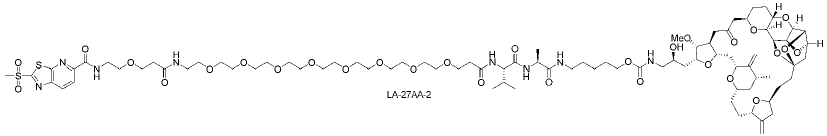 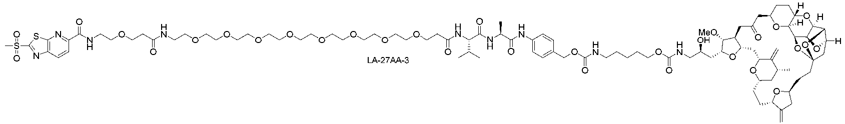 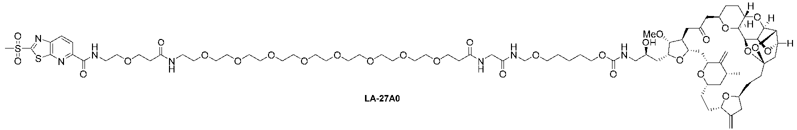 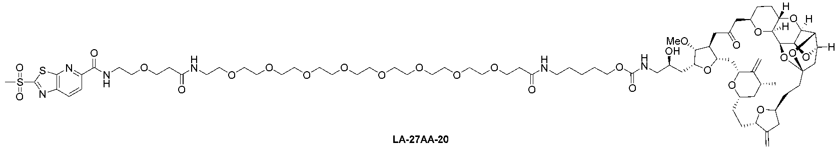 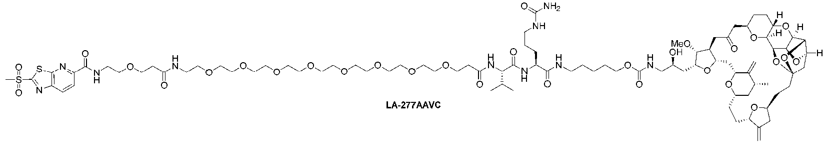 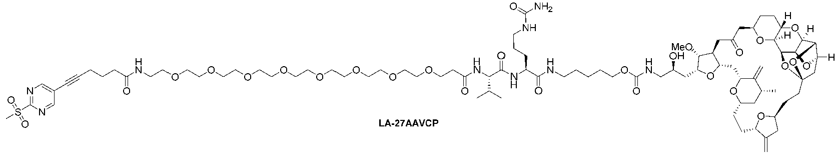 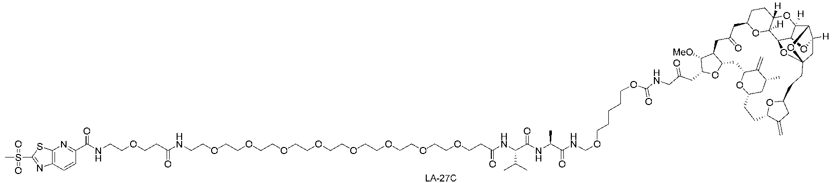 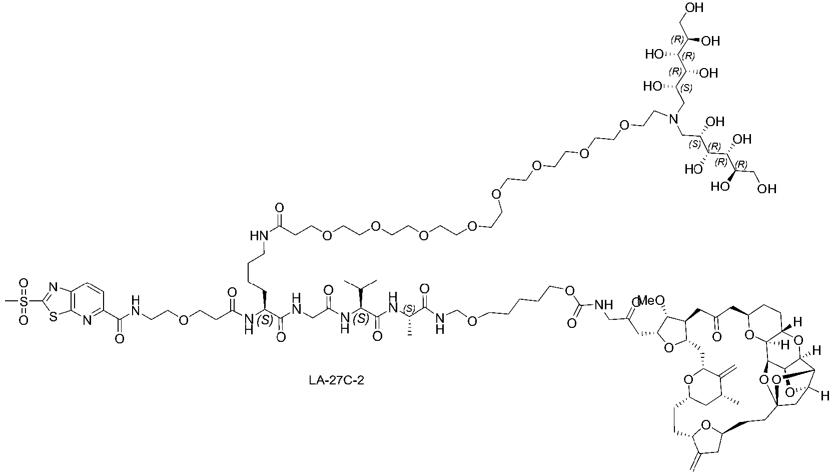 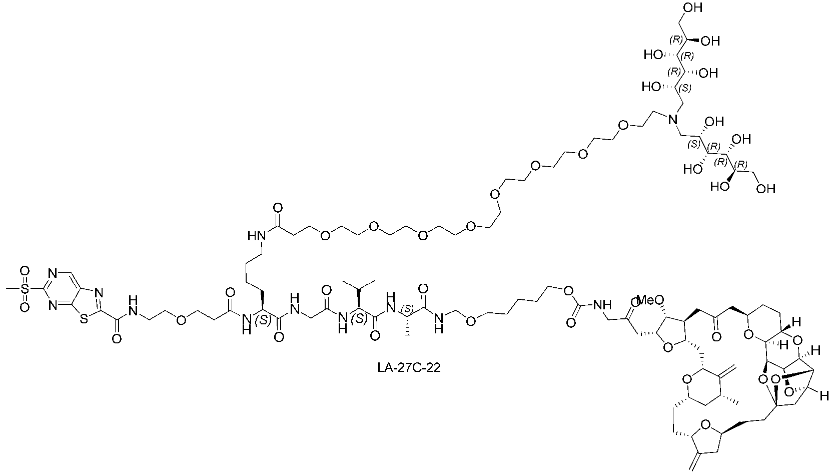 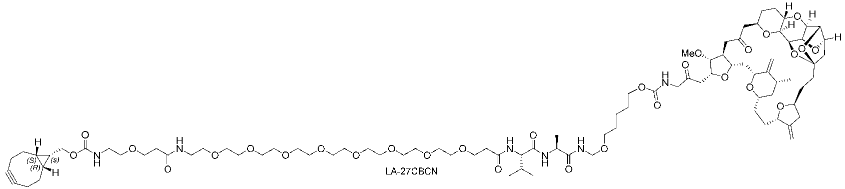 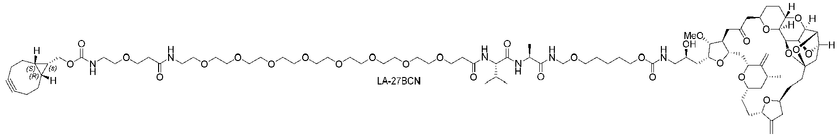 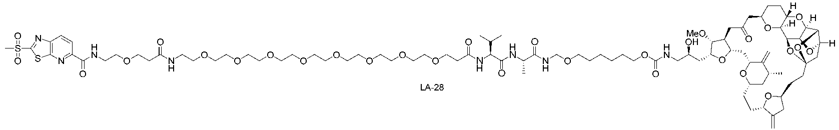 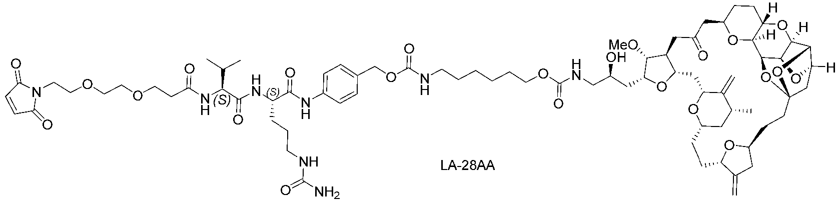 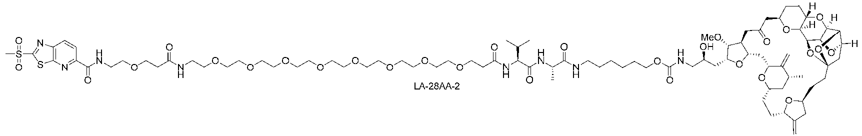 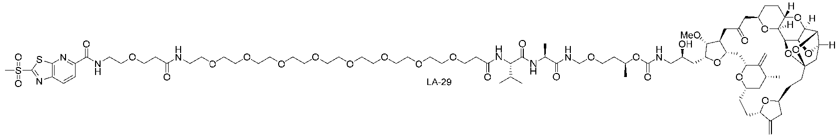 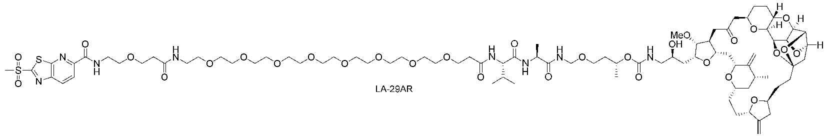 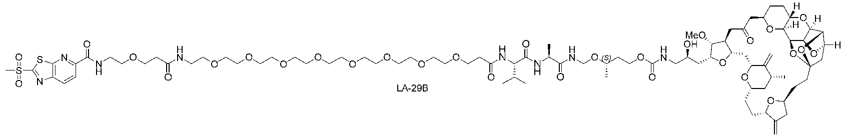 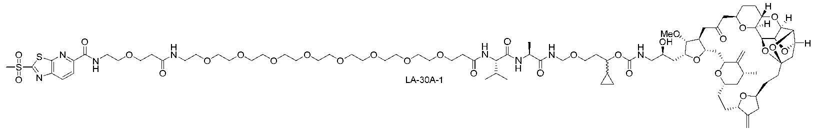 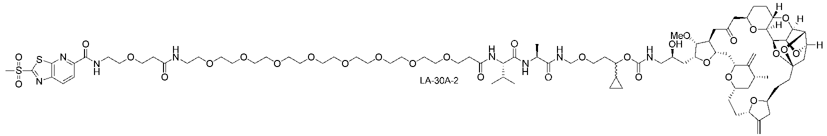 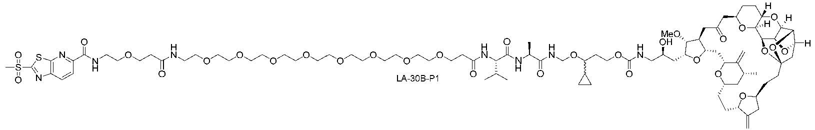 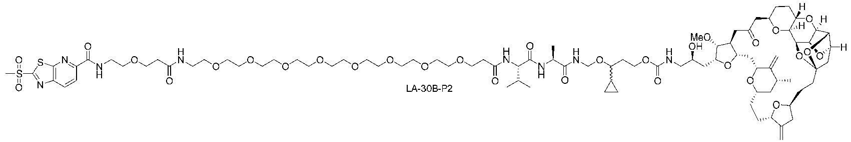 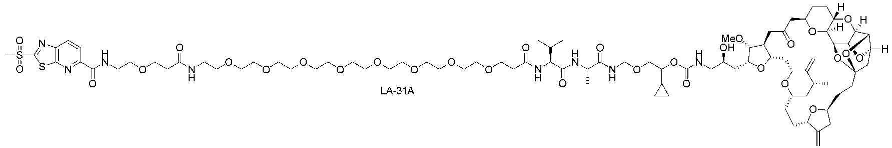 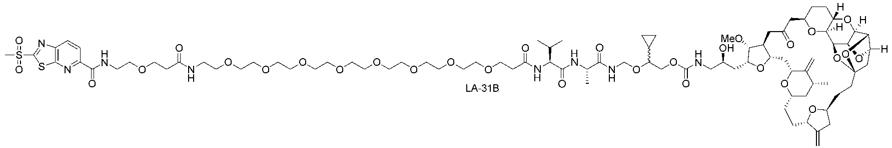 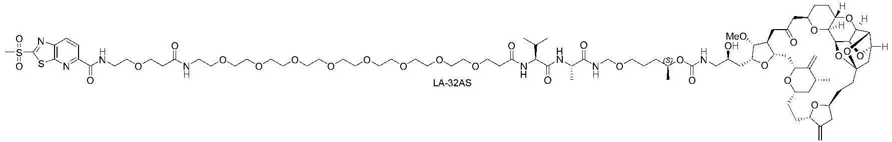 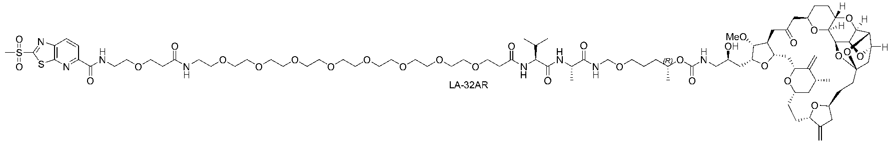 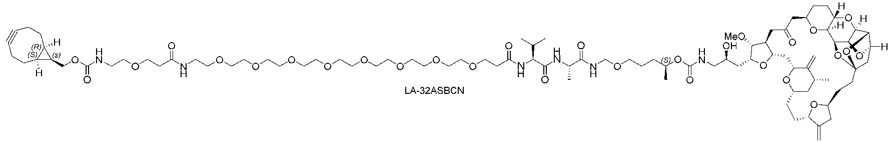 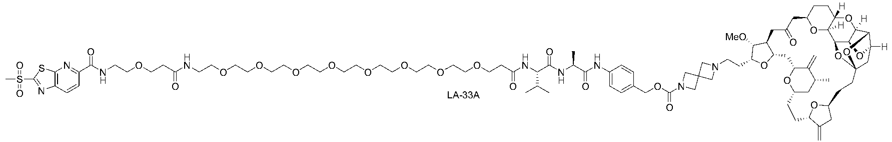 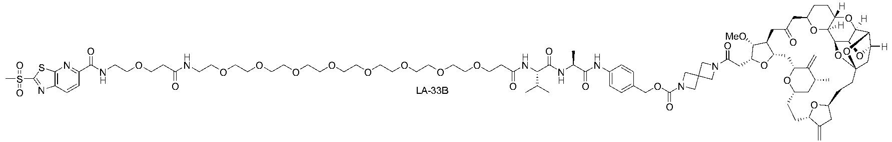 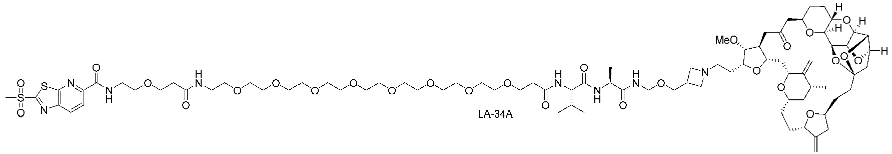 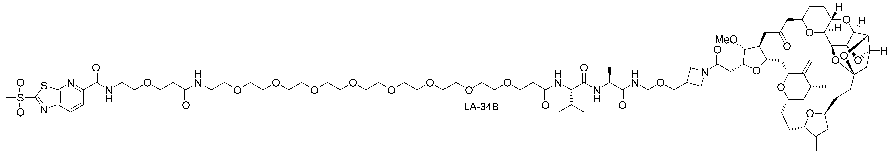 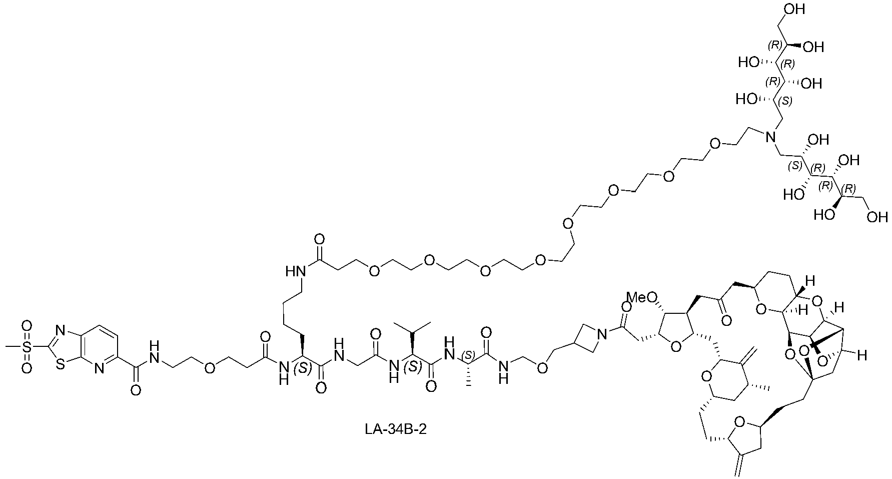 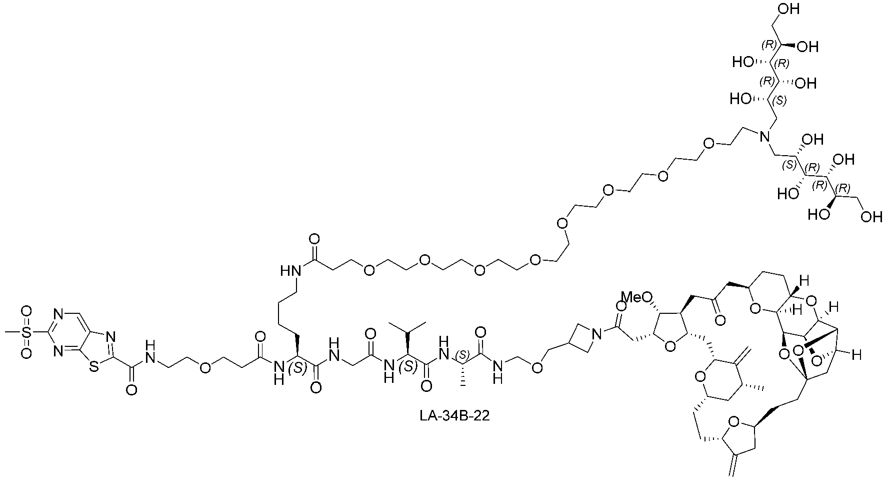 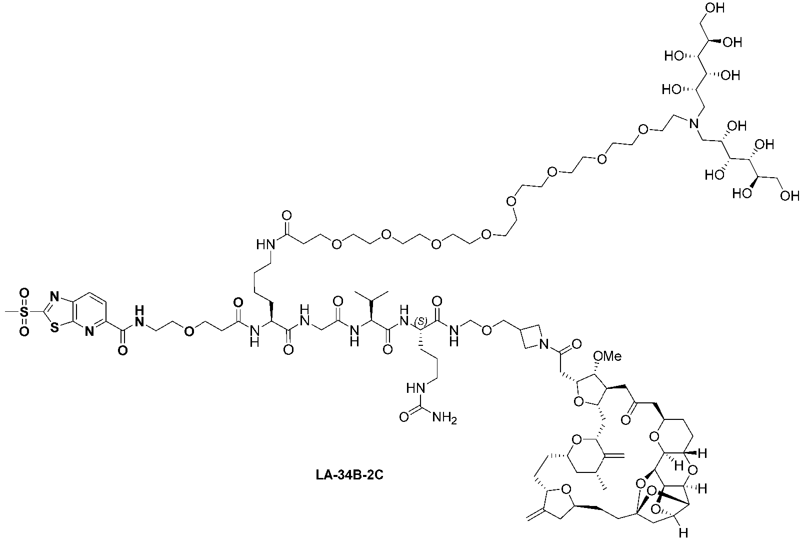 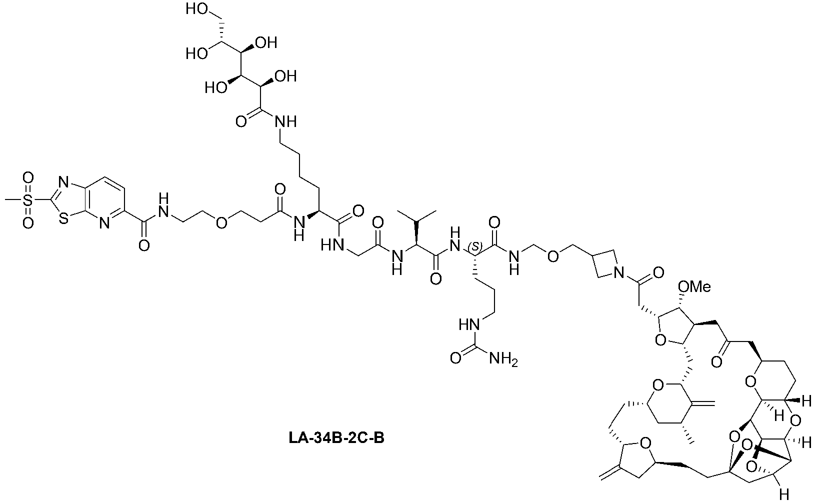 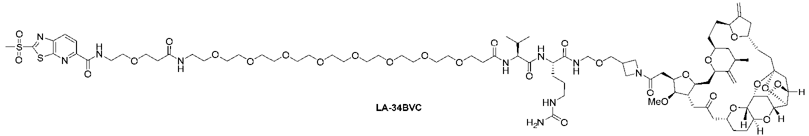 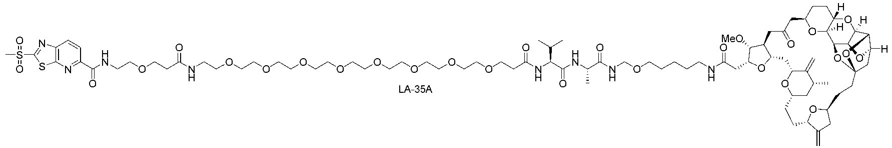 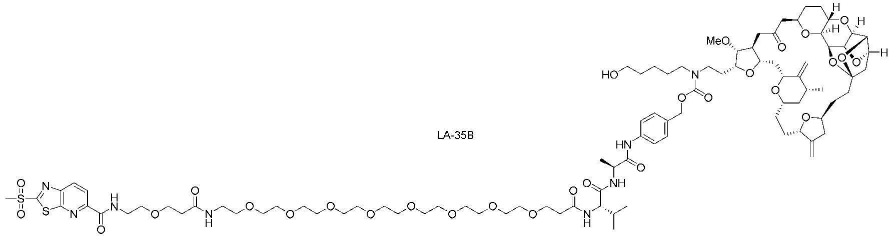 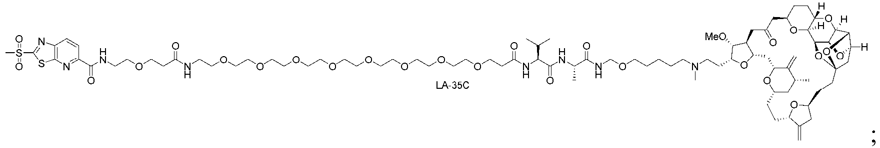 formula (II') is one of the following:

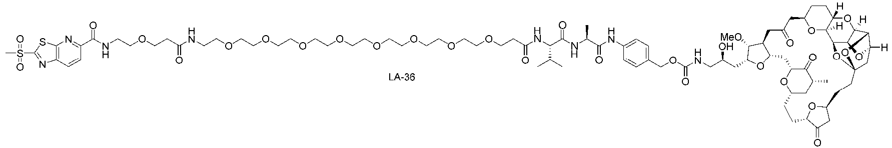

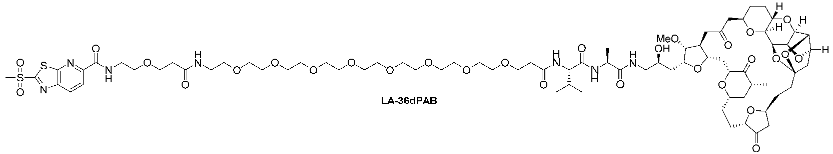

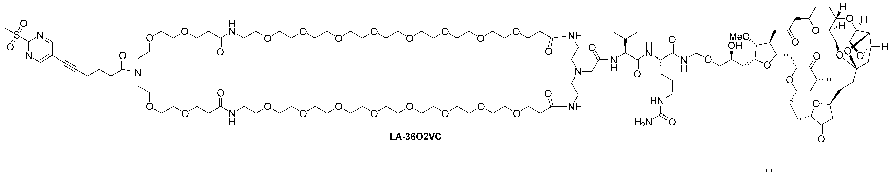

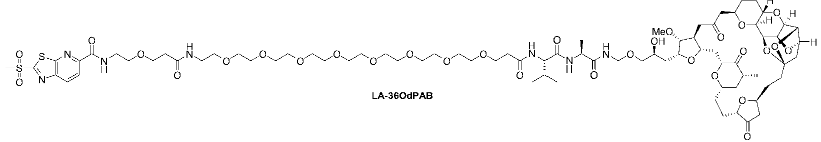

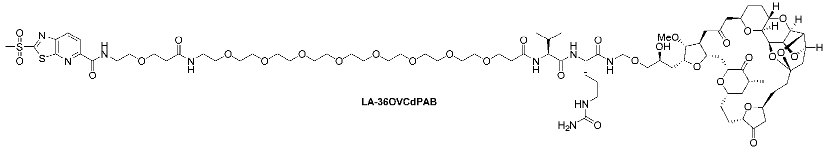

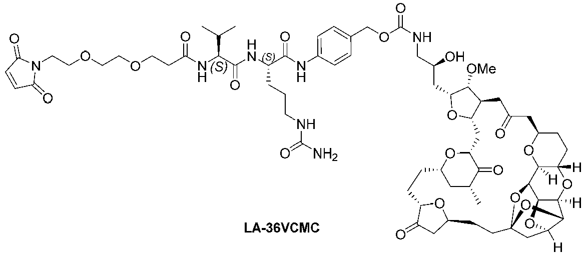

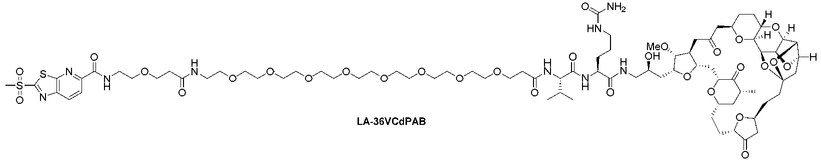

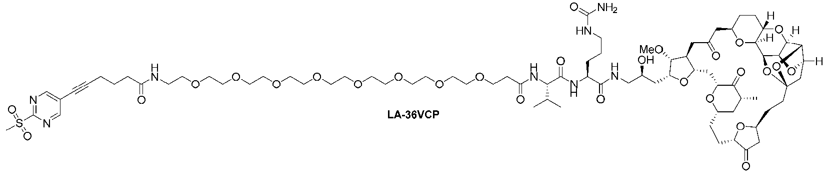

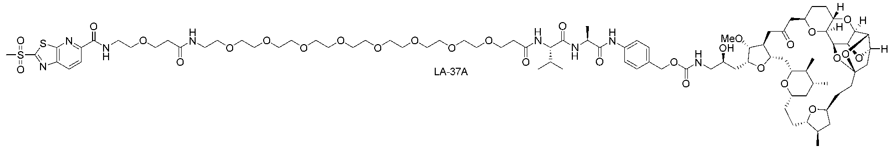

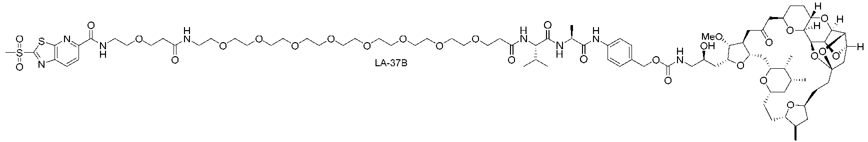

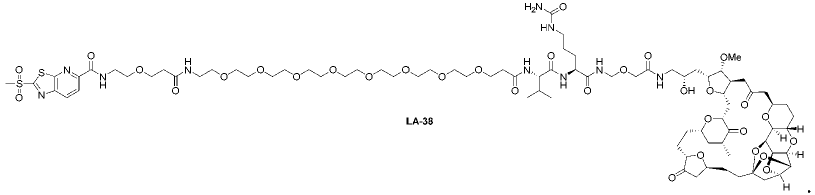

32. An antibody-drug conjugate, which is a compound of formula (I") or (II"), or an isotope-labeled compound, an optical isomer, a geometric isomer, a tautomer, an isomer mixture, or a pharmaceutically acceptable salt thereof; 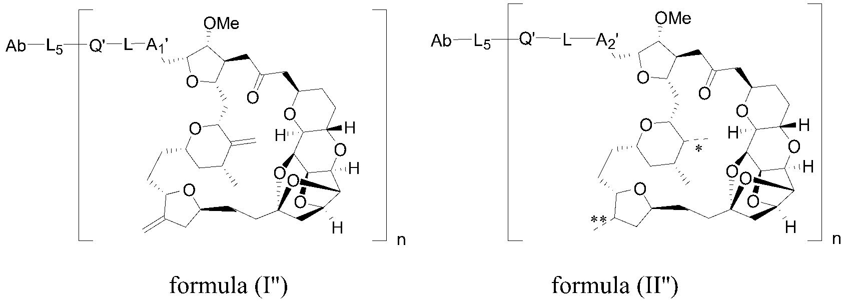 wherein:
Ab is an antibody or an antigen-binding fragment;
L₅ is a direct bond or a linking group connecting Ab and Q';
Q' is a group formed after Q is attached to Ab via L₅;
n is an integer or decimal between 0 and 20, or preferably, n is an integer or decimal between 0 and 8; and
definitions of Q, L, A₁', A₂', *, and ** are the same as those in formula (I') and formula (II') of any one of claims 16 to 31.

33. The antibody-drug conjugate of claim 32, wherein L₅ is the direct bond or 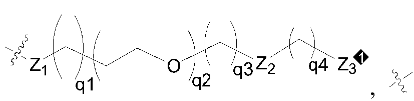 represents an attachment site to Ab, 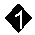 indicates that Z₃ is linked to Q', and Z₃ is a click probe, a sulfhydryl group, or a precursor thereof capable of binding to Q' after reaction;
Z₁ and Z₂ are each independently selected from -C(O)NH-, -C(O)O-, -C(O)-, -OC(O)-, -NHC(O)-, -NH-, -O-, -OC(O)NH-, or -NHC(O)O-;
q₁ is 0, 1, 2, 3, 4, 5, 6, 7, or 8;
q₂ is 1, 2, 3, 4, 5, 6, 7, or 8;
q₃ is 1, 2, 3, 4, 5, 6, 7, or 8; and
q₄ is 1, 2, 3, 4, 5, 6, 7, or 8.

34. The antibody-drug conjugate of claim 33, wherein Z₃ is a click probe or a sulfhydryl group or a precursor thereof that can be linked to Q' after a metal-free click reaction, and preferably, Z₃ is an azido group.

35. The antibody-drug conjugate of any one of claims 32 to 34, wherein L₅ is the direct bond or 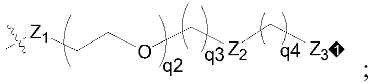 and preferably, L₅ is the direct bond or

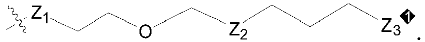

36. The antibody-drug conjugate of any one of claims 32 to 35, wherein Z₁ and Z₂ are each independently -C(O)NH-.

37. The antibody-drug conjugate of any one of claims 32 to 36, wherein Q' is selected from the following: 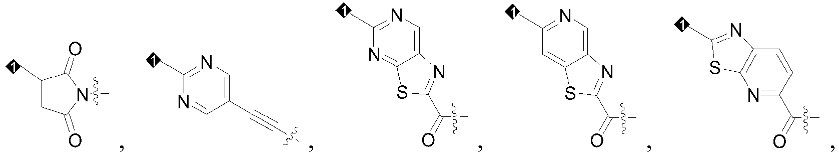 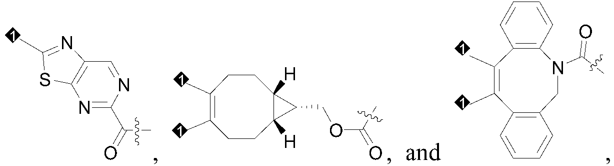 wherein 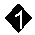 represents an attachment site to L₅, and 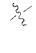 represents an attachment site to L₄.

38. The antibody-drug conjugate of claim 37, wherein L₅-Q' is selected from the following: 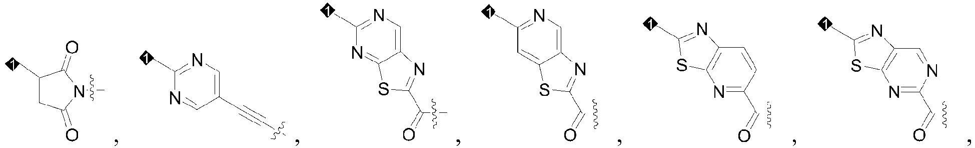 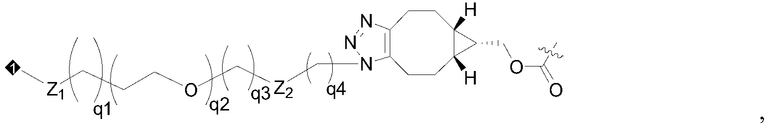 and 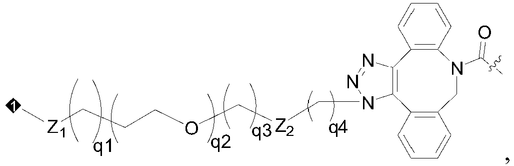 wherein 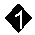 represents an attachment site to Ab, and 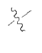 represents an attachment site to L₄.

39. The antibody-drug conjugate of claim 38, wherein L₅-Q' is selected from 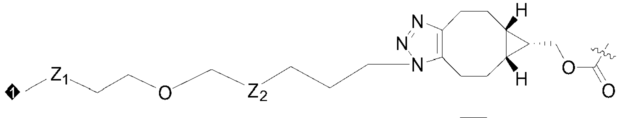 and 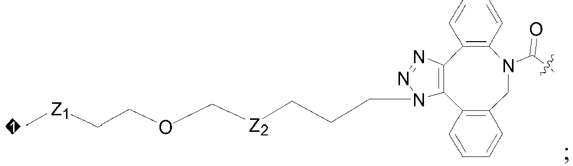 and preferably, Z₁ and Z₂ are each independently -C(O)NH-.

40. The antibody-drug conjugate of any one of claims 32 to 39, wherein Ab is selected from a mouse-derived antibody, a chimeric antibody, a humanized antibody, a fully human antibody, or an antigen-binding fragment thereof; and preferably, Ab is selected from anti-HER2 (ErbB2) antibody, anti-EGFR antibody, anti-B7-H3 antibody, anti-c-Met antibody, anti-HER3 (ErbB3) antibody, anti-HER4 (ErbB4) antibody, anti-ROR1 antibody, anti-CLDN6 antibody, anti-CLDN9 antibody, anti-CLDN18.2 antibody, anti-NaPi-2b antibody, anti-TNF-α antibody, anti-ENPP3 antibody, anti-DLL3 antibody, anti-CD20 antibody, anti-CD22 antibody, anti-CD28 antibody, anti-CD30 antibody, anti-CD33 antibody, anti-CD37 antibody, anti-CD38 antibody, anti-CD44 antibody, anti-CD45 antibody, anti-CD47 antibody, anti-CD48 antibody, anti-CD56 antibody, anti-CD70 antibody, anti-CD73 antibody, anti-CD98 antibody, anti-CD105 antibody, anti-CEA antibody, anti-EphA2 antibody, anti-MUCI antibody, anti-Lewis Y antibody, anti-VEGFR antibody, anti-GPNMB antibody, anti-Integrin antibody, anti-PSMA antibody, anti-Tenascin-C antibody, anti-SLC44A4 antibody, anti-CD79 antibody, anti-TROP-2 antibody, anti-CD79B antibody, anti-Mesothelin antibody, anti-Nectin-4 antibody, anti-TPBG antibody, or an antigen-binding fragment thereof.

41. The antibody-drug conjugate of claim 40, wherein Ab is the anti-HER2 (ErbB2) antibody or the antigen-binding fragment thereof.

42. The antibody-drug conjugate of claim 40, wherein Ab is selected from the monoclonal antibody or the antigen-binding fragment thereof, or preferably, selected from Trastuzumab, Cetuximab, Pertuzumab, Nimotuzumab, Enoblituzumab, Emibetuzumab, Inotuzumab, Pinatuzumab Vedotin, Brentuximab Vedotin, Gemtuzumab, Bivatuzumab, Lorvotuzumab, or an antigen-binding fragment thereof.

43. The antibody-drug conjugate of any one of claims 32 to 42, wherein formula (I") is one of the following: 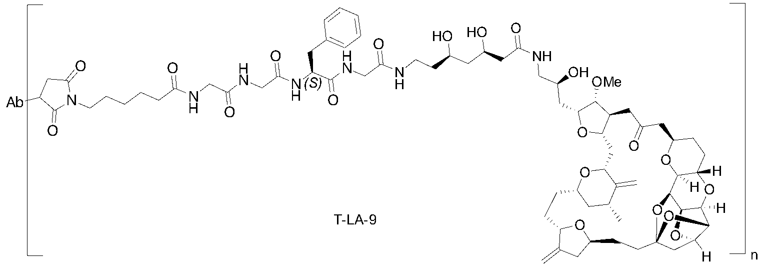 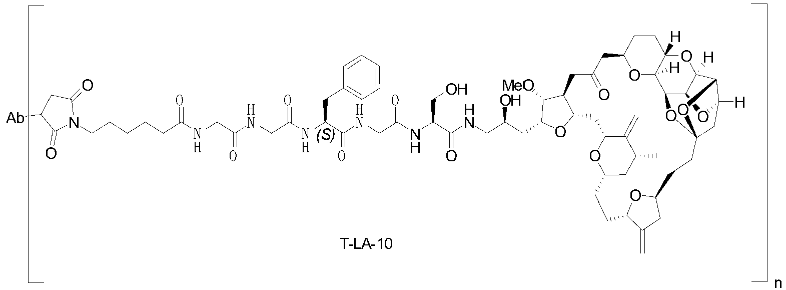 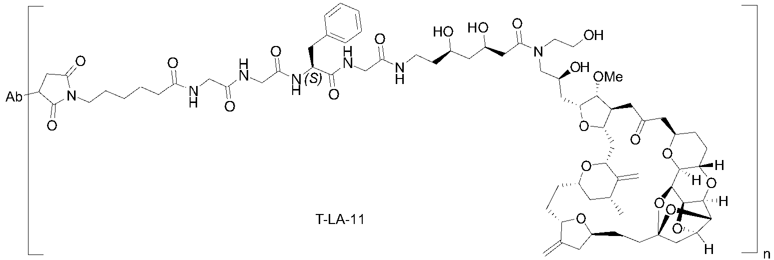 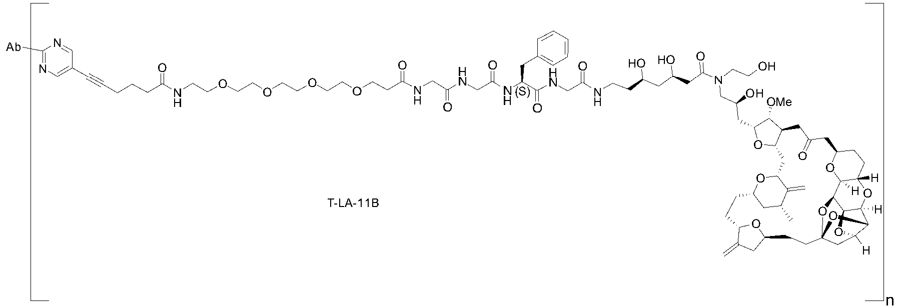 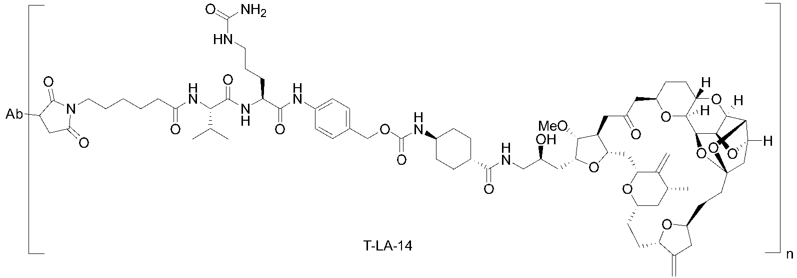 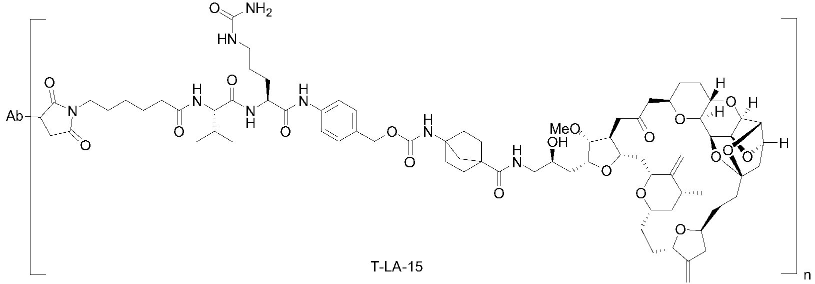 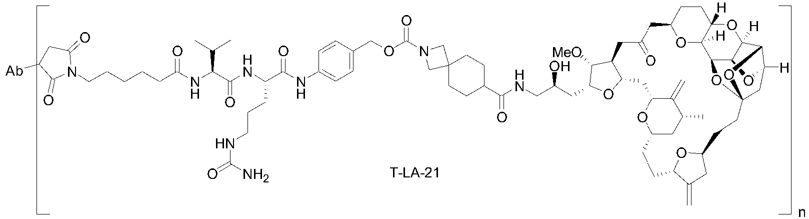 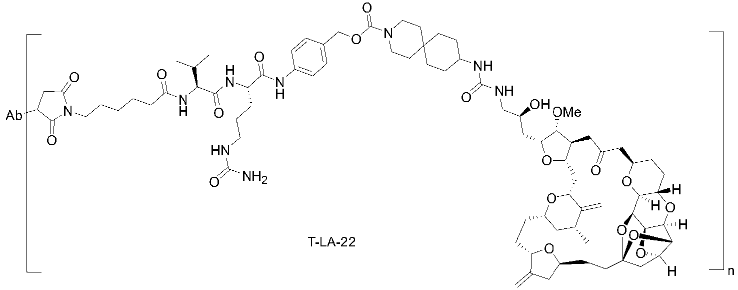 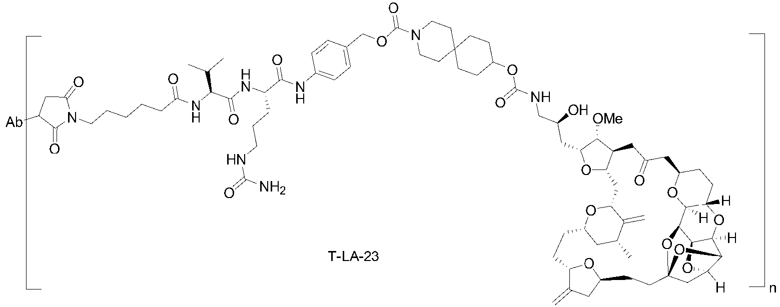 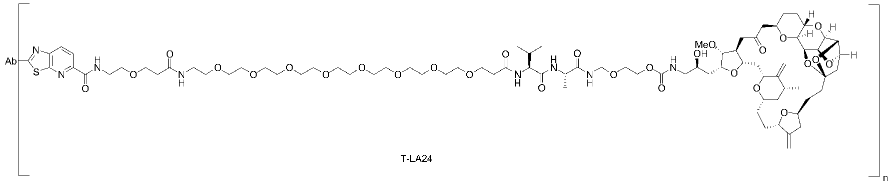 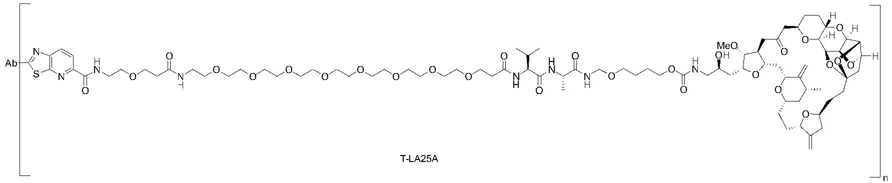 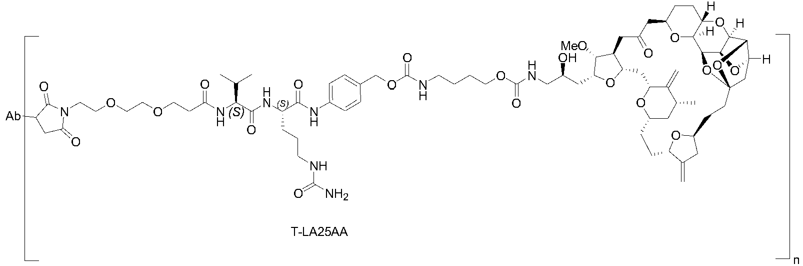 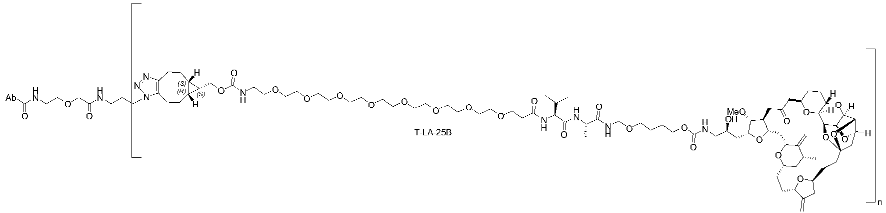 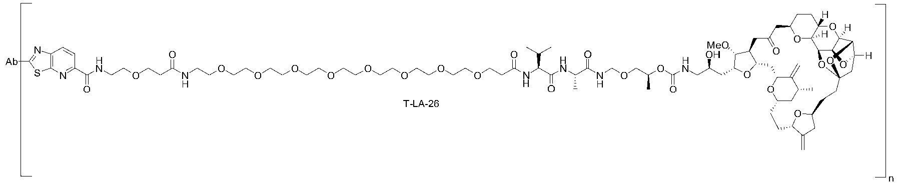 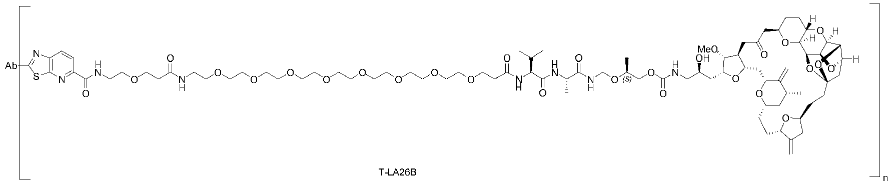 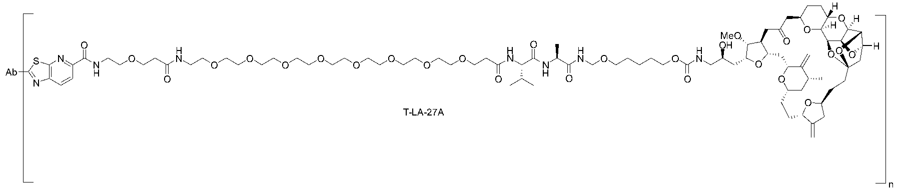 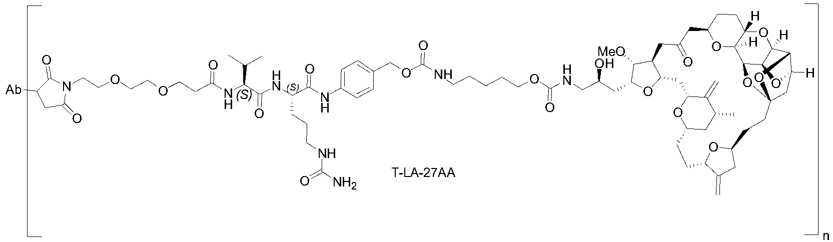 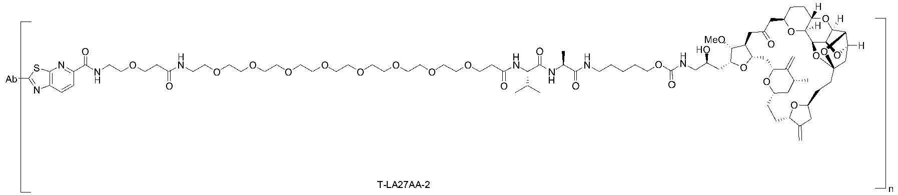 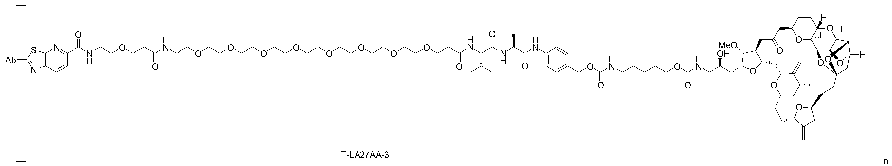 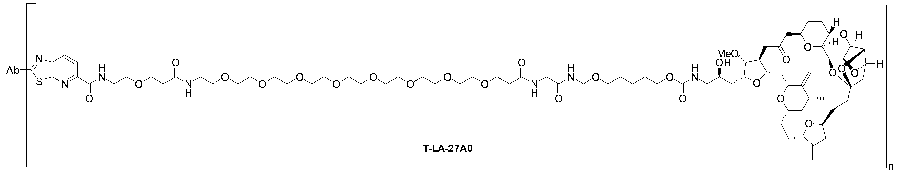 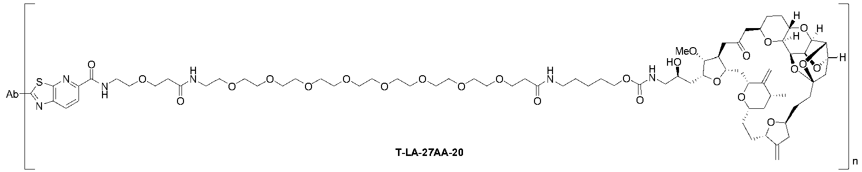 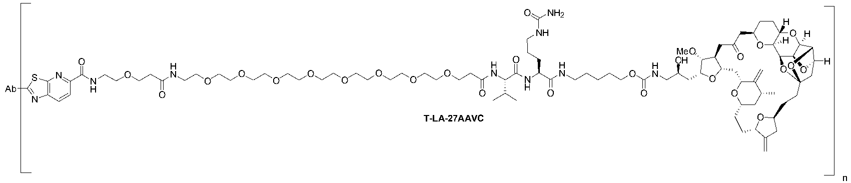 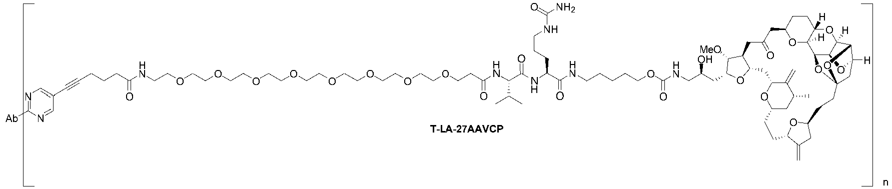 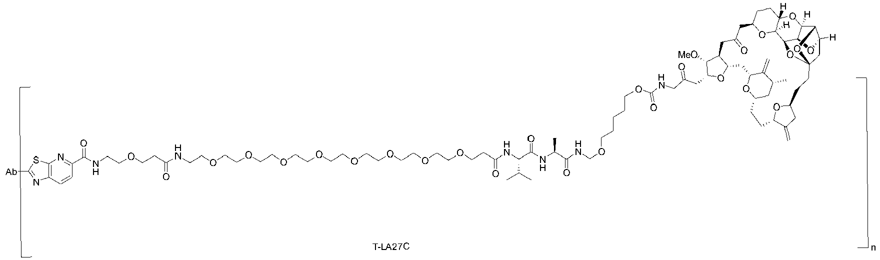 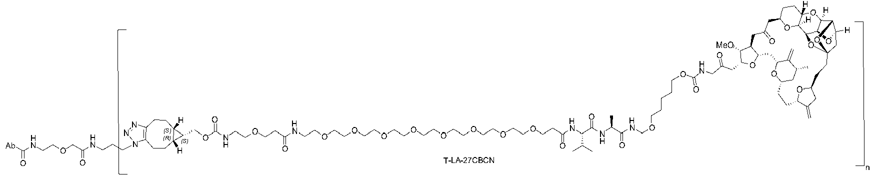 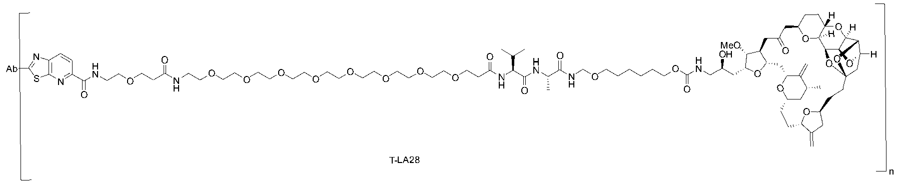 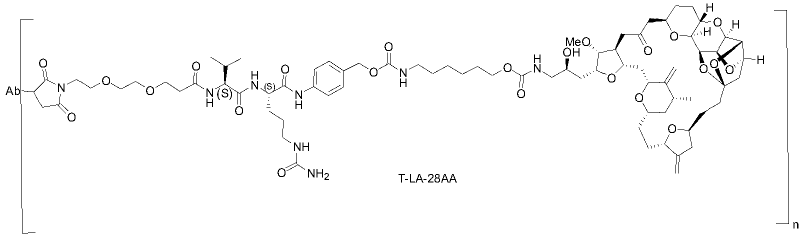 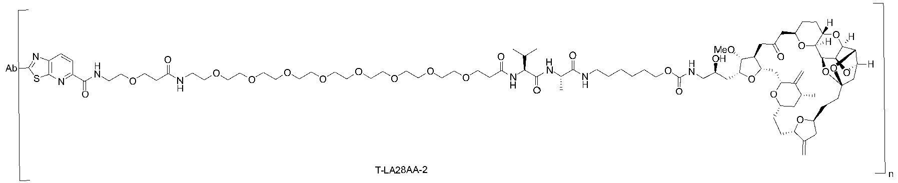 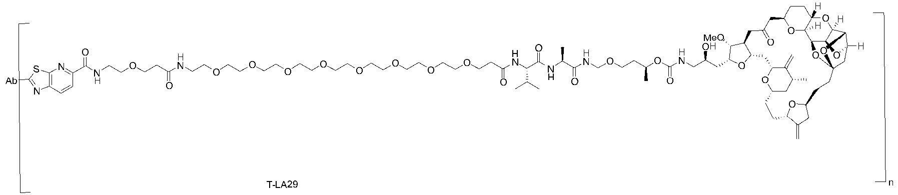 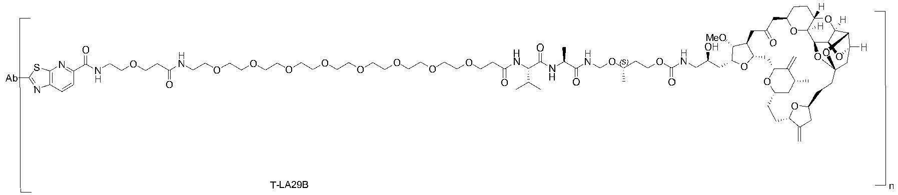 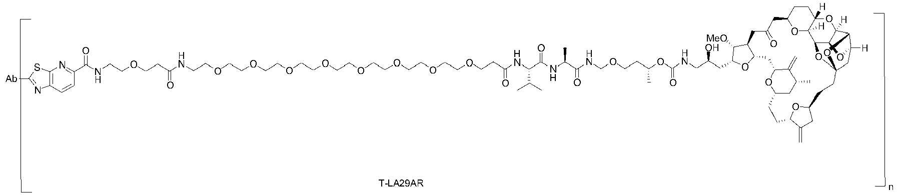 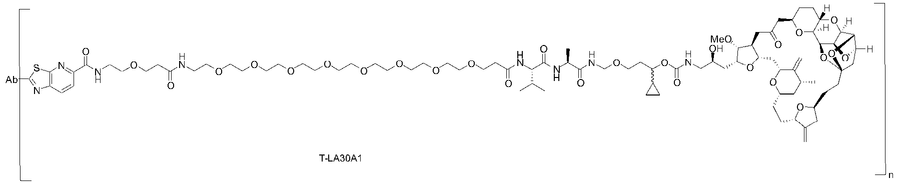 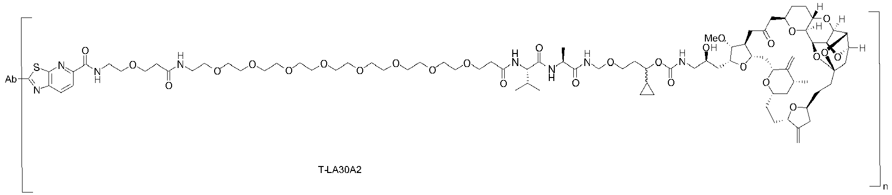 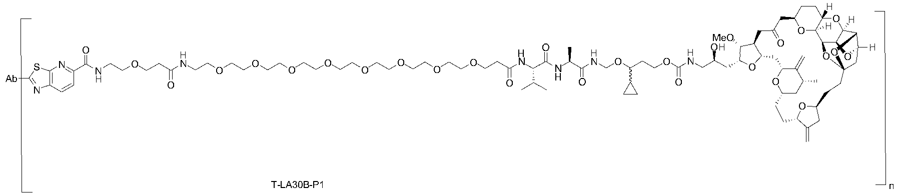 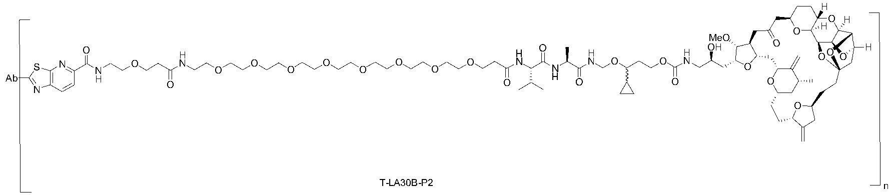 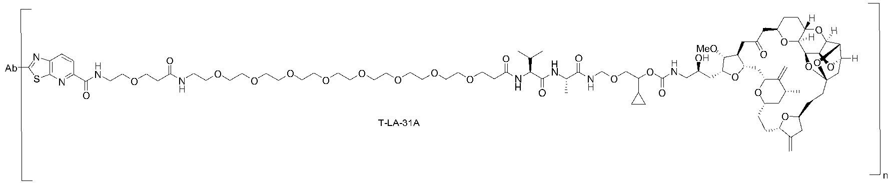 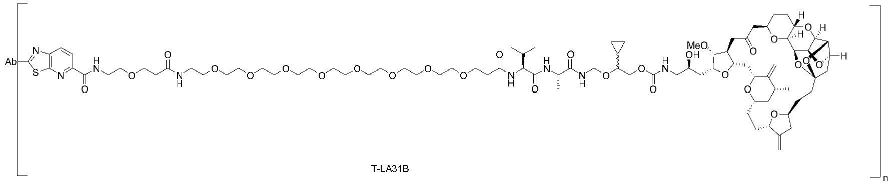 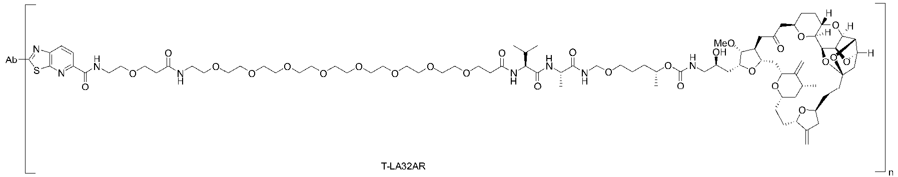 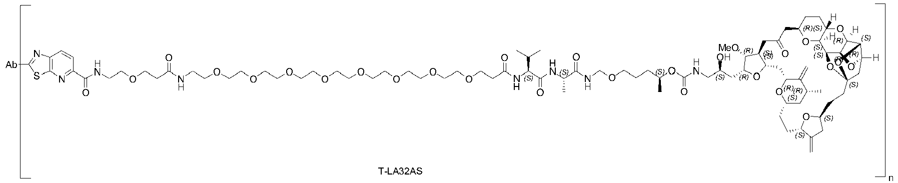 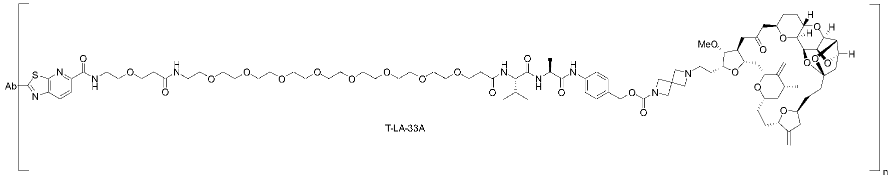 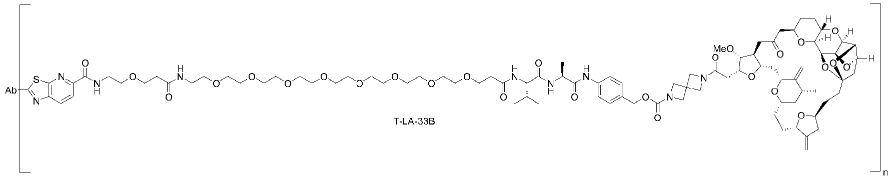 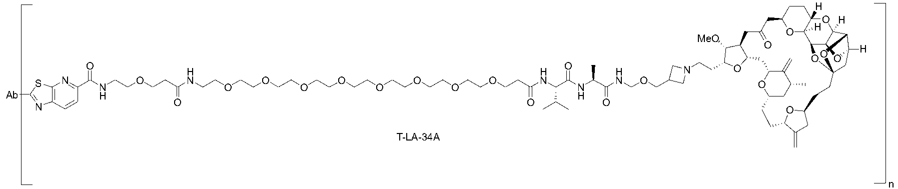 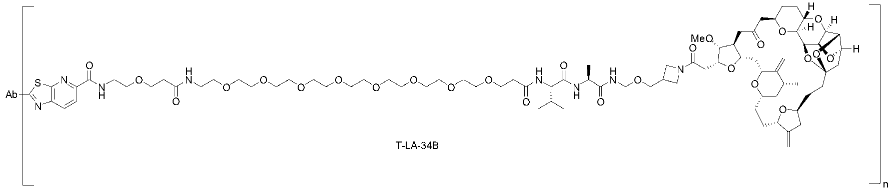 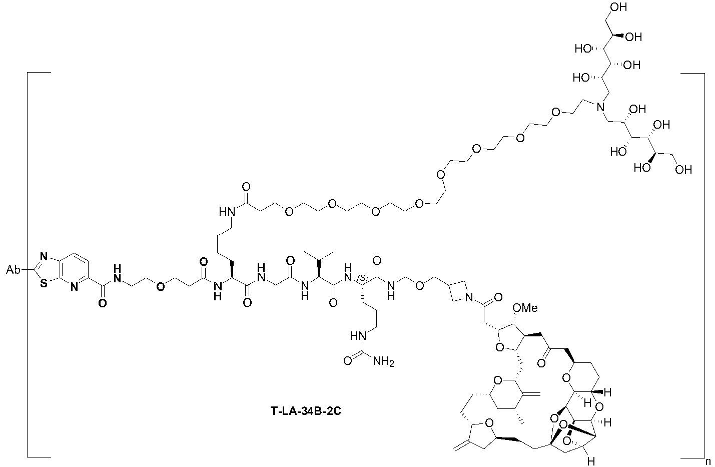 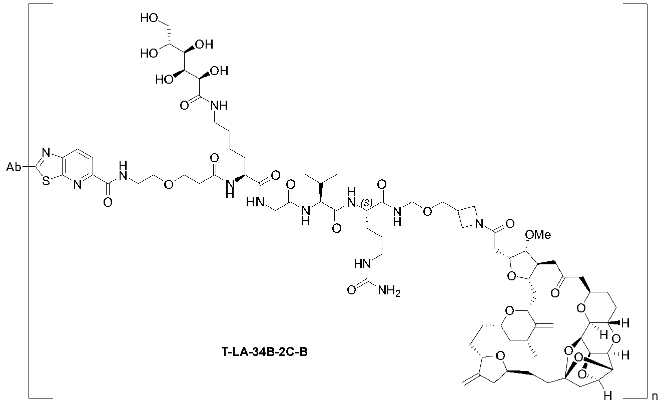 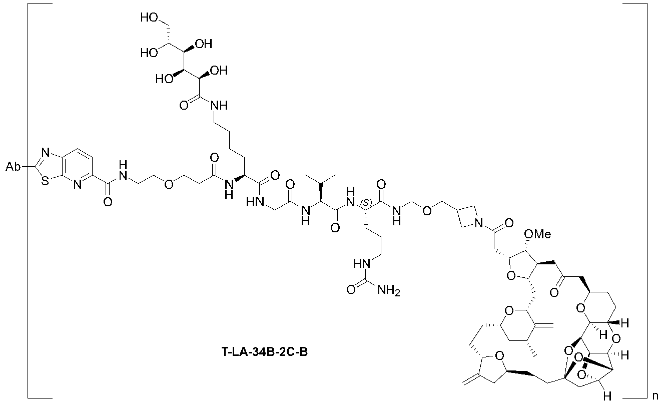 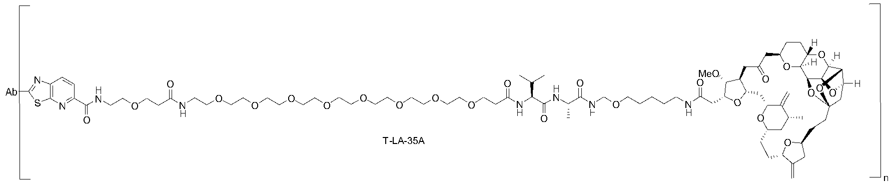 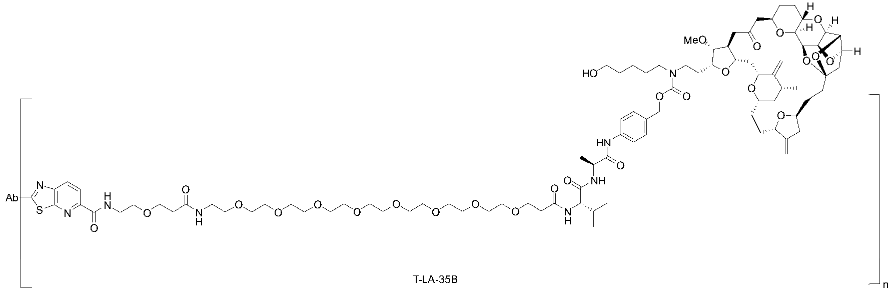 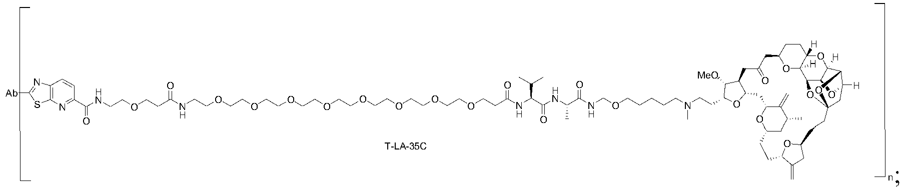
formula (II") is one of the following: 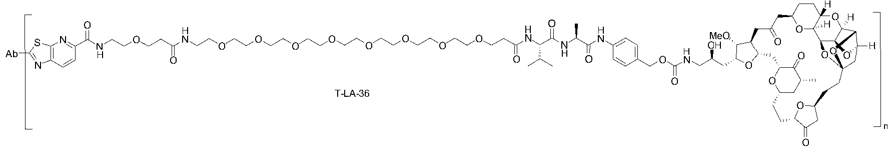 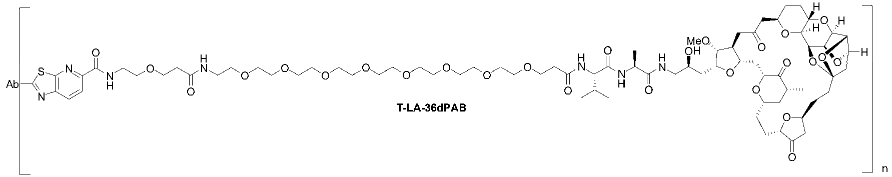 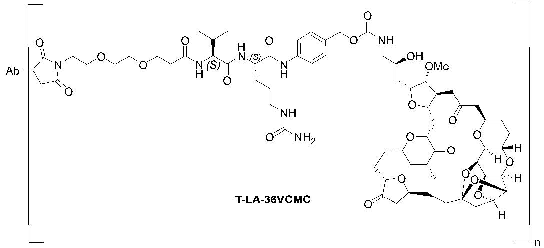 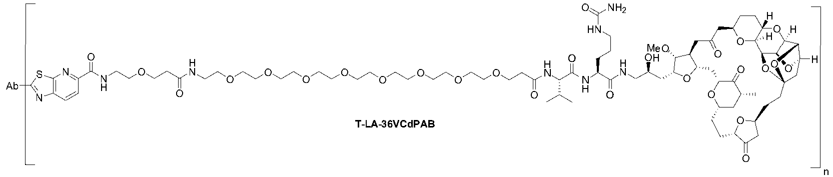 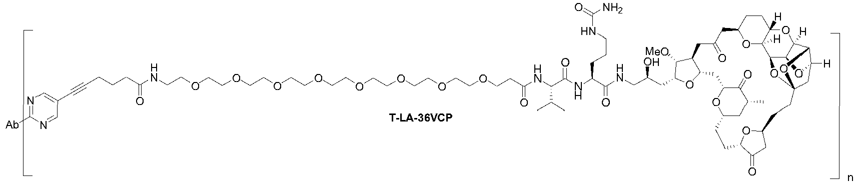 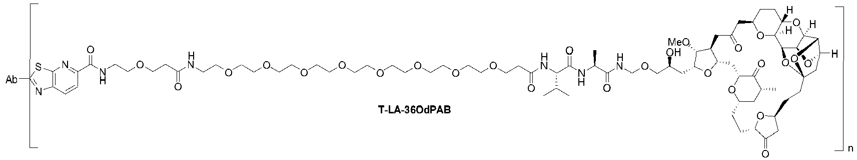 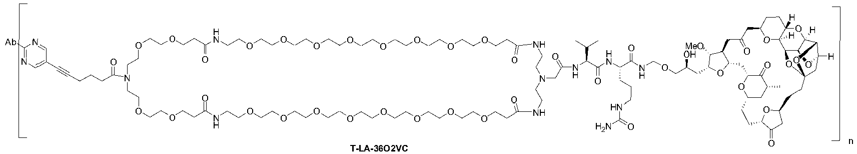 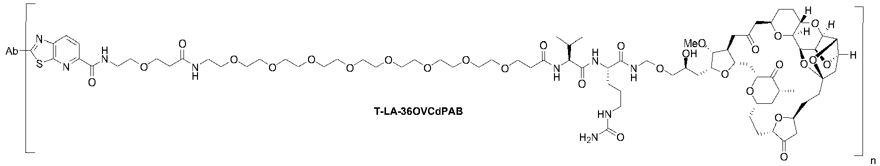 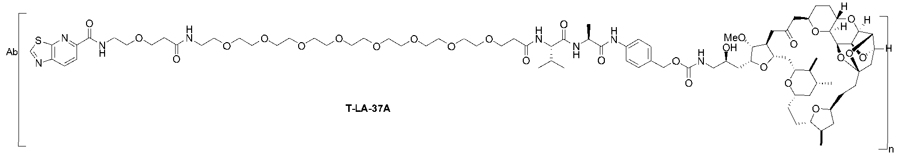 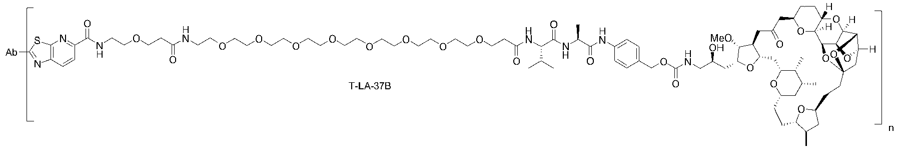 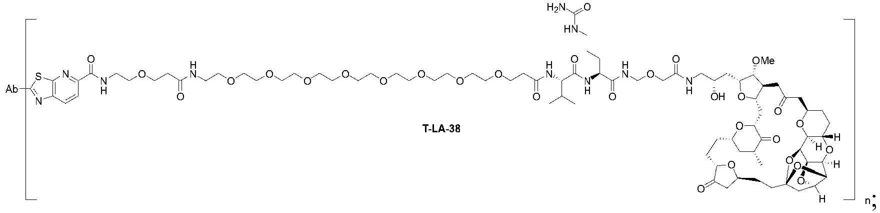
preferably, Ab is the anti-HER2 (ErbB2) antibody or the antigen-binding fragment thereof, and more preferably, Ab is Trastuzumab or an antigen-binding fragment thereof.

44. An eribulin derivative, wherein the eribulin derivative is a compound shown below, or an isotope-labeled compound, an optical isomer, a geometric isomer, a tautomer, an isomer mixture, or a pharmaceutically acceptable salt thereof;

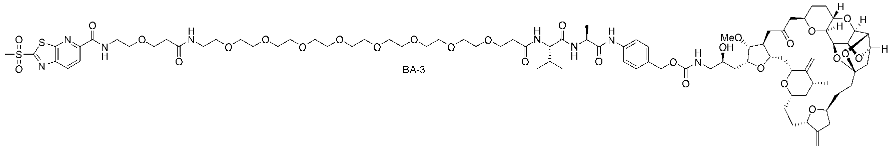

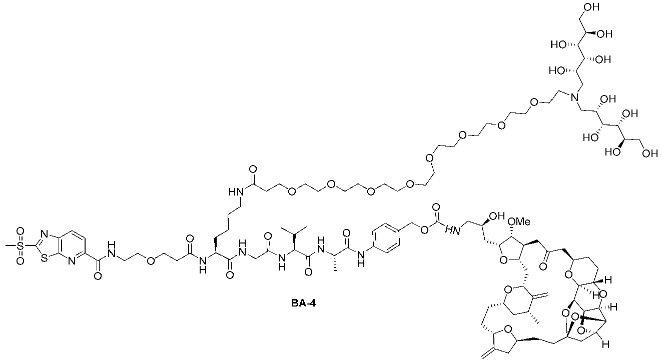

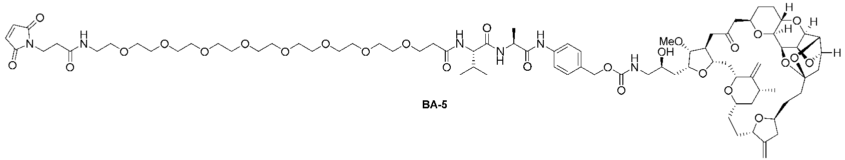

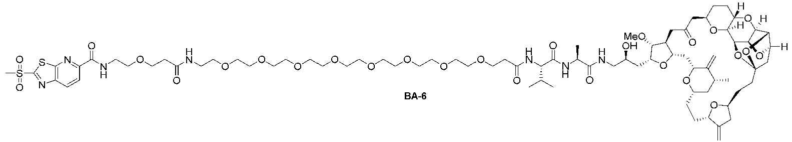

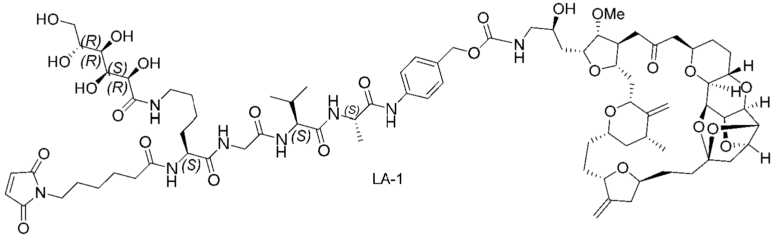

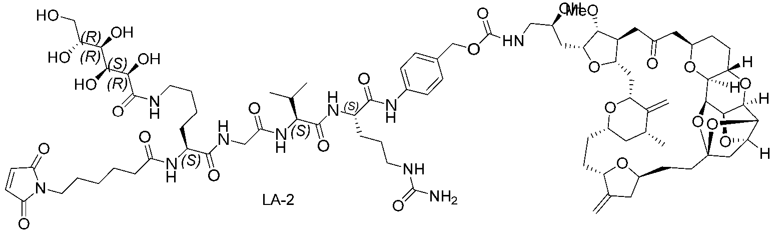

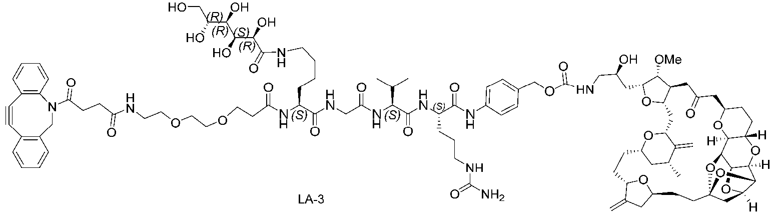

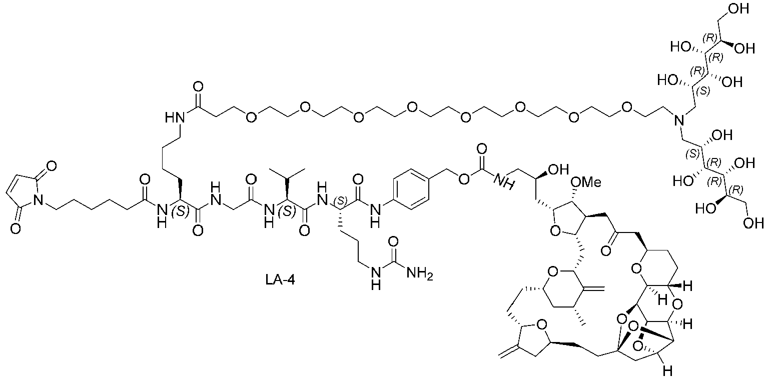

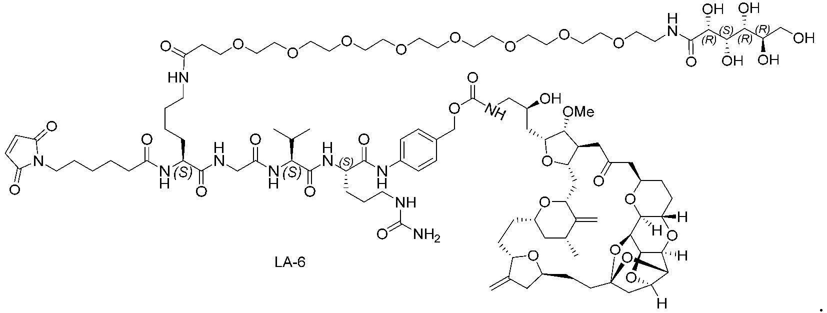

45. An antibody-drug conjugate, wherein the antibody-drug conjugate is a compound shown below, or an isotope-labeled compound, an optical isomer, a geometric isomer, a tautomer, an isomer mixture, or a pharmaceutically acceptable salt thereof; 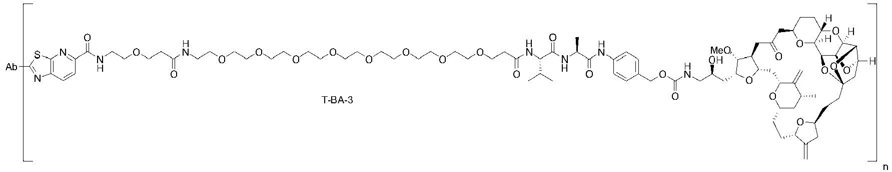 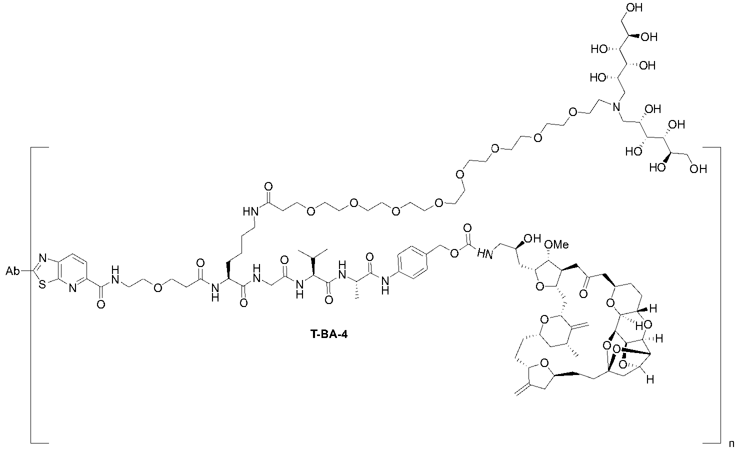 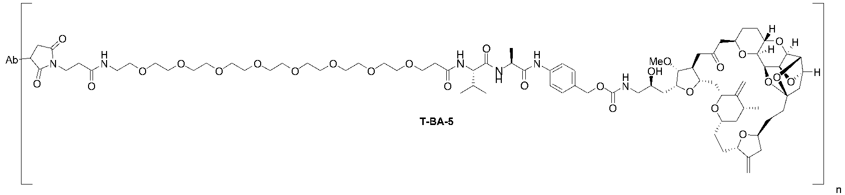 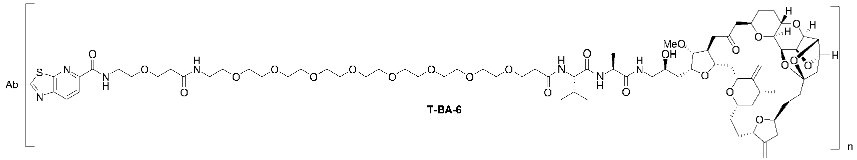 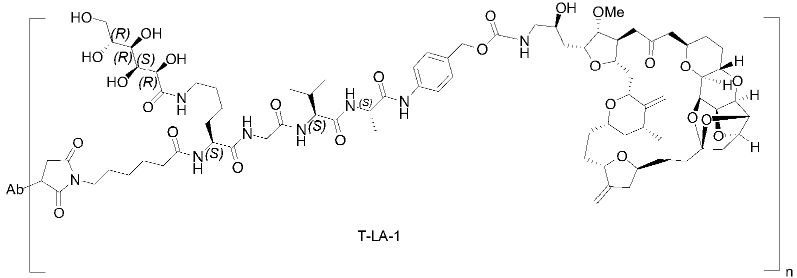 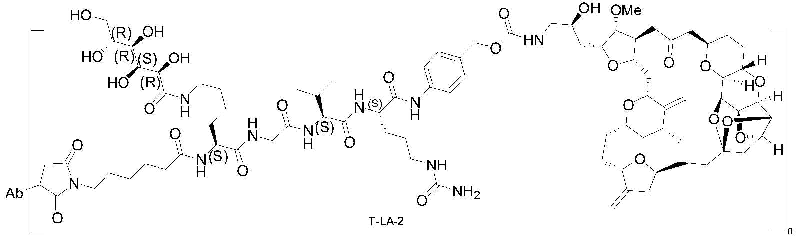 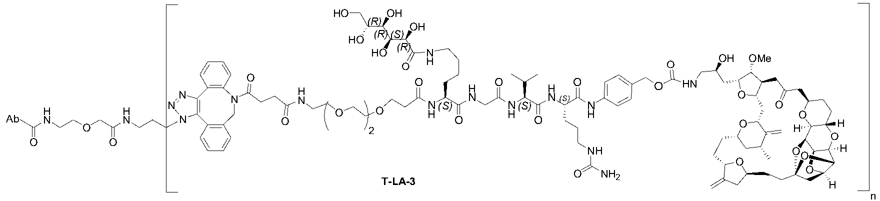 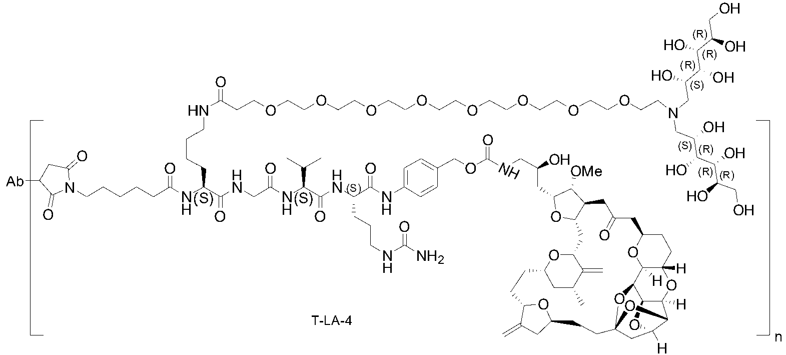 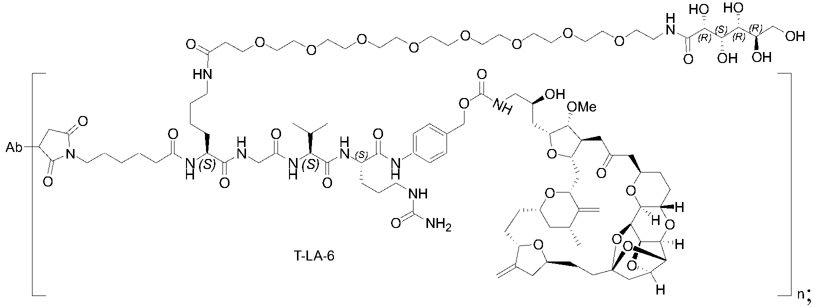 wherein Ab is an antibody or an antigen-binding fragment; and n is an integer or a decimal between 0 and 20, or preferably, n is an integer or a decimal between 0 and 8.

46. The antibody-drug conjugate of claim 45, wherein Ab is selected from a mouse-derived antibody, a chimeric antibody, a humanized antibody, a fully human antibody, or an antigen-binding fragment thereof; and preferably, Ab is selected from anti-HER2 (ErbB2) antibody, anti-EGFR antibody, anti-B7-H3 antibody, anti-c-Met antibody, anti-HER3 (ErbB3) antibody, anti-HER4 (ErbB4) antibody, anti-ROR1 antibody, anti-CLDN6 antibody, anti-CLDN9 antibody, anti-CLDN18.2 antibody, anti-NaPi-2b antibody, anti-TNF-α antibody, anti-ENPP3 antibody, anti-DLL3 antibody, anti-CD20 antibody, anti-CD22 antibody, anti-CD28 antibody, anti-CD30 antibody, anti-CD33 antibody, anti-CD37 antibody, anti-CD38 antibody, anti-CD44 antibody, anti-CD45 antibody, anti-CD47 antibody, anti-CD48 antibody, anti-CD56 antibody, anti-CD70 antibody, anti-CD73 antibody, anti-CD98 antibody, anti-CD105 antibody, anti-CEA antibody, anti-EphA2 antibody, anti-MUCI antibody, anti-Lewis Y antibody, anti-VEGFR antibody, anti-GPNMB antibody, anti-Integrin antibody, anti-PSMA antibody, anti-Tenascin-C antibody, anti-SLC44A4 antibody, anti-CD79 antibody, anti-TROP-2 antibody, anti-CD79B antibody, anti-Mesothelin antibody, anti-Nectin-4 antibody, anti-TPBG antibody, or an antigen-binding fragment thereof.

47. The antibody-drug conjugate of claim 46, wherein Ab is the anti-HER2 (ErbB2) antibody or the antigen-binding fragment thereof.

48. The antibody-drug conjugate of claim 46, wherein Ab is selected from the monoclonal antibody or the antigen-binding fragment thereof, or preferably, selected from Trastuzumab, Cetuximab, Pertuzumab, Nimotuzumab, Enoblituzumab, Emibetuzumab, Inotuzumab, Pinatuzumab Vedotin, Brentuximab Vedotin, Gemtuzumab, Bivatuzumab, Lorvotuzumab, or an antigen-binding fragment thereof, or preferably, Ab is Trastuzumab or an antigen-binding fragment thereof.

49. A pharmaceutical composition, comprising the eribulin derivative of any one of claims 1 to 31 and claim 44 or the antibody-drug conjugate of any one of claims 32 to 43 and claims 45 to 48, and one or more pharmaceutically acceptable carriers or excipients.

50. Use of the eribulin derivative of any one of claims 1 to 31 and claim 44 in preparation of an antibody-drug conjugate.

51. Use of the eribulin derivative of any one of claims 1 to 31 and claim 44, the antibody-drug conjugate of any one of claims 32 to 43 and claims 45 to 48, or the pharmaceutical composition of claim 49 in preparation of a medicament for treating tumors or cancers.

52. A method for treating a tumor or cancer, comprising administering an effective dose of the eribulin derivative of any one of claims 1 to 31 and claim 44, the antibody-drug conjugate of any one of claims 32 to 43 and claims 45 to 48, or the pharmaceutical composition of claim 49 to a subject with such need.

53. The use of claim 51 or the method of claim 52, wherein the tumor or cancer is selected from a solid tumor or hematological tumor, and is preferably selected from breast cancer, gastric cancer, colon cancer, pancreatic cancer, kidney cancer, ovarian cancer, lung cancer, soft tissue sarcoma, liposarcoma, non-small cell lung cancer, melanoma, head and neck cancer, cervical cancer, and prostate cancer, and is more preferably HER-2-positive breast cancer, triple-negative breast cancer, HER-2-positive triple-negative breast cancer, clear cell renal cell carcinoma, small cell lung cancer, non-small cell lung cancer, and large cell lung cancer.

54. The use or method of claim 53, wherein the tumor or cancer is selected from a cancer or tumor with high expression of HER2 (ErbB2) antibody.
